(19) Europäisches Patentamt
European Patent Office
Office européen des brevets

(11) EP 4 162 865 A1

(12) EUROPEAN PATENT APPLICATION

(43) Date of publication:
**12.04.2023 Bulletin 2023/15**

(21) Application number: **22200097.8**

(22) Date of filing: **06.10.2022**

(51) International Patent Classification (IPC):
***A61B 5/00*** *(2006.01)* ***G01S 7/41*** *(2006.01)*
***G01S 13/04*** *(2006.01)* ***G01S 13/50*** *(2006.01)*
***G01S 13/66*** *(2006.01)* ***G01S 13/88*** *(2006.01)*
*G01S 13/46* *(2006.01)*

(52) Cooperative Patent Classification (CPC):
**G01S 7/415; A61B 5/4806; G01S 13/343; G01S 13/42; G01S 13/50; G01S 13/88;**
G01S 2013/462

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**06.10.2021 US 202163253083 P**
**07.11.2021 US 202163276652 P**
**18.11.2021 US 202163281043 P**
**22.12.2021 US 202163293065 P**
**16.01.2022 US 202263300042 P**
**30.05.2022 US 202217827902**
**04.06.2022 US 202263349082 P**
**15.08.2022 US 202217888429**
**18.08.2022 US 202217891037**
**15.09.2022 US 202217945995**
**04.10.2022 US 202217960080**
**04.10.2022 US 202217959487**

(71) Applicants:
- **Origin Wireless, Inc.**
  **Greenbelt, MD 20770 (US)**
- **Wang, Beibei**
  **Clarksville, MD 21029 (US)**
- **Wu, Chenshu**
  **Hong Kong (HK)**
- **Zhu, Guozhen**
  **Greenbelt, MD 20770 (US)**
- **Ozturk, Muhammed Zahid**
  **Beltsville, MD 20740 (US)**
- **Wang, Fangyu**
  **Beijing Beijing 100876 (CN)**
- **Liu, K.J. Ray**
  **Potomac, MD 20854 (US)**
- **Lai, Hung-Quoc Duc**
  **Parkville, MD 21234 (US)**
- **Samaranayake Arachchige Dona**
  **College Park, MD 20740 (US)**
- **Regani, Sai Deepika**
  **Hyattsville, MD 20783 (US)**
- **Zeng, Xiaolu**
  **Beijing 100081 (CN)**
- **Wang, Wei-Hsiang**
  **College Park MD Maryland 20740 (US)**
- **Au, Oscar Chi-Lim**
  **San Jose, CA 95136 (US)**
- **Wu, Min**
  **Clarksville, MD 21029 (US)**

(72) Inventors:
- **WANG, Beibei**
  **Clarksville, MD Maryland 21029 (US)**
- **WU, Chenshu**
  **Hong Kong, Hong Kong SAR (China) (CN)**
- **ZHU, Guozhen**
  **Greenbelt, MD Maryland 20770 (US)**
- **OZTURK, Muhammed Zahid**
  **College Park, MD Maryland 20740 (US)**
- **WANG, Fengyu**
  **Beijing, Beijing 100876 (CN)**
- **LIU, K. J. Ray**
  **Potomac, MD Maryland 20854 (US)**
- **DUC LAI, Hung-Quoc**
  **Parkville, MD Maryland 21234 (US)**
- **SAMARANAYAKE ARACHCHIGE DONA, Sakila Sandeepani Jayaweera**
  **College Park, MD Maryland 20740 (US)**
- **REGANI, Sai Deepika**
  **Hyattsville, MD Maryland 20783 (US)**
- **ZENG, Xiaolu**
  **Beijing, Beijing (CN)**
- **WANG, Wei-Hsiang**
  **College Park, MD Maryland 20740 (US)**
- **AU, Oscar Chi-Lim**
  **San Jose, CA California 95136 (US)**
- **WU, Min**
  **Clarksville, MD Maryland 21029 (US)**

(74) Representative: **Boult Wade Tennant LLP**
**Salisbury Square House**
**8 Salisbury Square**
**London EC4Y 8AP (GB)**

(54) **SYSTEM AND METHOD FOR RADIO-BASED SLEEP MONITORING**

(57) Methods, apparatus and systems for wireless sensing, monitoring and tracking are described. In one example, a described system comprises: a transmitter configured to transmit a first wireless signal through a wireless multipath channel in a venue; a receiver configured to receive a second wireless signal through the wireless multipath channel, wherein the second wireless signal differs from the first wireless signal due to the wireless multipath channel which is impacted by a sleeping motion of an object in the venue; and a processor. The processor is configured for: obtaining a time series of channel information (TSCI) of the wireless multipath channel based on the second wireless signal, wherein each channel information (CI) of the TSCI comprises N1 components, wherein N1 is a positive integer larger than one, computing N1 component-wise analytics each associated with one of the N1 components of the TSCI, identifying N2 largest component-wise analytics among the N1 component-wise analytics, wherein N2 is a positive integer less than N1, computing at least one first motion statistics based on the N2 largest component-wise analytics of the TSCI, and monitoring the sleeping motion of the object based on the at least one first motion statistics.

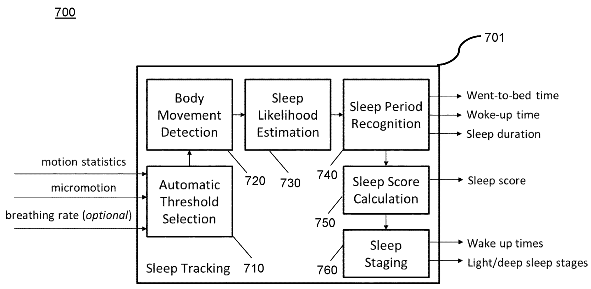


FIG. 7

## Description

### TECHNICAL FIELD

The present teaching generally relates to wireless sensing, monitoring and tracking. More specifically, the present teaching relates to: Wi-Fi-based sleep monitoring system that can work on any low-cost Internet-of-things (IoT) devices; heartbeat tracking and monitoring by processing wireless channel information and beamforming; detecting voice activity based on radio signals; map reconstruction based on wireless tracking; and wireless monitoring vital signs by processing, decomposing and enhancing wireless channel information.

### BACKGROUND

Sleep plays a vital role in an individual's health and well-being, both mentally and physically. It is well recognized that sleep quantity and quality is fundamentally related to health risks like cardiovascular decease, stroke, kidney failure, diabetes, and adverse mental conditions, etc. Unfortunately, in modern society, a number of people suffer from sleep disorders. As recently reported, 10% of the population suffers from chronic insomnia (which is even higher among elders), and 1/3 of Americans do not get sufficient sleep. Monitoring sleep emerges as an essential demand to help, manage, diagnose, and treat the growing group of sleep disorders as well as to keep regular tabs on personal health.

Sleep monitoring, however, is a challenging task that has drawn tremendous efforts for decades. Generally, it measures sleep time, recognizes different sleep stages, e.g., wake, REM (Rapid Eye Movement) and NREM (Non-REM), and accordingly assesses an individual's sleep quality. Various solutions have been proposed. The medical gold standard relies on Polysomnography (PSG), which monitors various physiological parameters such as brain activities, respirations, and body movements by a number of wired sensors attached to the patient. Albeit accurate and comprehensive, PSG is usually expensive and cumbersome with the invasive sensors that may cause sleep difficulties, limiting itself to clinical usage for confirmed patients. Other approaches including photoplethysmography (PPG) and actigraphy (ACT) require users to wear dedicated sensors during sleep. Ballistocardiogram (BCG) needs to instrument the mattress with an array of EMFi sensors to measure ballistic force. Despite of the costs, these approaches provide suitable solutions for those who need special cares but are less-than-ideal for the public. Recent efforts in mobile computing envision in-home sleep monitoring using smartphones and wearables. These methods, however, only provide coarse-grained, less accurate measurements and fail to monitor vital signs like respiratory rate. In addition, mobiles and wearables are undesirable for especially elders and those with dementia.

The rapid development of wireless sensing has transformed Wi-Fi from a pure communication platform to a ubiquitous sensing infrastructure. Many applications have been studied, including motion detection, sleep monitoring, gesture recognition, fall detection, gait monitoring, imaging, etc. In Wi-Fi sensing, more antennas and larger bandwidths are preferred for better performance, which, however, are not always available. Particularly, there is frequently only one single antenna with 20MHz bandwidth on 2.4GHz channels on low-cost, compact IoT devices, creating an extremely challenging environment for Wi-Fi sensing to be implemented. Many existing approaches would fail in such stringent conditions, since many of them rely on antenna arrays and/or larger bandwidths for channel parameter estimation or phase cleaning.

Heart Rate Variability (HRV), defined as the variation of the periods between consecutive heartbeats, i.e., Inter-Beat Intervals (IBI), is an important indicator of the overall health status of an individual. Analysis of the HRV has been proved to be a powerful tool to assess cardiac health and evaluate the state of the Autonomic Nervous System (ANS). High-accuracy HRV monitoring is required in numerous applications such as early diagnose of cardiovascular disease, stress evaluation, emotions recognition and anxiety treatment, etc.

Traditional measurements of the HRV are obtained by continuously measuring the IBIs using the electrocardiogram (ECG) or photoplethysmogram (PPG) sensors, both of which are dedicated medical devices and have to be physically contacted with the human skin. However, using ECG or PPG is uncomfortable for users and sometimes may cause skin allergies. To avoid the direct contact with users' skin, other wearable devices such as Inertial Measurement Units (IMUs) have been explored to measure the movements of the chest surfaces to determine the IBIs and then measure the HRV. Although some of the aforementioned methods are less invasive than ECG and PPG-based approaches, all of them require users to wear dedicated devices, which is cumbersome and usually expensive for daily usage. Therefore, it is desirable to monitor the HRV in a noncontact and accurate way with a robust system.

As automobiles have become an essential part to facilitate people's daily life, Advanced Driver Assistance Systems (ADAS) have been gaining more and more interest in assisting drivers to enhance both safety and convenience. To respond timely in case of an emergency, ADAS needs to keep track of the driver's health/consciousness, which is generally achieved by monitoring the driver's vital signs including Respiration Rate (RR), Heart Rate (HR) and Heart Rate Variability (HRV). However, most of existing solutions requires an assumption that the human is stationary, which does not hold in practical driving scenarios.

Humans are enormously capable of understanding a noisy speech (a.k.a. speech enhancement (SE)) or separating one speaker from another (a.k.a. speech separation (SS)), which may be collectively called SES, and is known as a cocktail party problem. SES capability for machines is of great demand for many applications, such as voice commands, live speech recording, etc., yet remains a challenging problem using microphones.

Monaural SES methods achieved remarkable progress in recent years with the help of deep learning, especially when there is not much background noise. However, fundamental problems still exist in estimating the number of sources in a mixture, associating output sources with the desired speakers (a.k.a. label permutation problem), and tracing the speakers for long periods of time. Although these problems can be solved for clean mixtures, by clustering-based methods and permutation invariant training (PIT), their performance can decrease with noisy mixtures. Overall, audio-only approaches suffer from these ill-posed problems inherently.

To overcome the problems and enhance SES, multimodal systems have been introduced to exploit readily available information beyond audio, such as video. Similar to human perception, which also uses lip motion and facial information, audiovisual systems are shown to improve SES performance, especially in challenging cases, such as same-speaker mixtures. Same and similar-speaker mixtures are especially difficult for audio-only methods, as the distinction between the two sources is minimal. Additional visual information about the speaker, e.g., videos or even a facial picture of the user, or other information, such as voice activity detection, or pitch improves the SES performance. However, camera-based methods require good lighting conditions and raise potential privacy concerns.

Voice interfaces have become one of the key elements of human-machine interaction in recent years, with the widespread availability of smart assistants. For most voice interfaces, whether a single microphone to record sound or a multi-microphone array to process and understand the user commands, voice activity detection (VAD) is the first essential processing block. However, existing VAD systems are not robust enough against interference and noise, and not computationally efficient.

Indoor location-based services rely on indoor maps, which are yet widely available despite numerous efforts from the industry. Existing solutions employ costly hardware (e.g., lidar) to achieve accurate mapping of indoor environments, or resort to crowdsourcing for floor plan generation at the cost of precision due to inaccurate inertial sensing.

Location-Based Services (LBS) are gaining increasing popularity, such as navigation, location-based searching, and social network services, etc. This is thanks to the ever-growing presence of smart devices with built-in location systems and maps. However, most of these services are only available in outdoor environments. One of the most critical constraints to the development of indoor LBS is the lack of digital indoor maps. Although projects like Google Indoor Maps, Point Inside, and Micello Indoor Map aim at collecting indoor maps, the availability of indoor maps is still very limited considering the huge number of buildings worldwide. Furthermore, most floor plans are manually generated and uploaded in these projects. The traditional manual methods require professional technicians to draw the floor plan using specialized measurement devices, which is time-consuming, costly, and thus unaffordable to cover all buildings. In addition, potential changes in the indoor environment would require much effort to update the maps.

Efforts have been taken to generate indoor maps automatically. Solutions based on lidar and cameras could achieve moderate accuracy but are very costly and/or privacy-intrusive. Systems based on WiFi fingerprinting require dense Access Points (APs) deployment, which may not always be available, especially in home environments. Map reconstruction based on crowdsourced trajectories is also extensively studied. Leveraging data contributed by mobile participants, crowdsourcing-based map reconstruction makes it possible to efficiently generate maps with less cost. However, the existing crowdsourcing based map reconstruction methods mainly rely on inertial sensors, which are known to have limited accuracy due to accumulative errors.

To overcome the significant accumulative errors in inertial tracking (e.g., pedestrian dead-reckoning), existing approaches resort to 1) various auxiliary calibration by discovering potential reference anchors (e.g., elevators) in the environments and 2) hundreds of various trajectories collected by a large number of users with a long time of efforts. However, in many environments, especially private environments such as homes, such anchors are not available. It is also not practical to collect plenty of trajectories with long-time efforts from a large number of users. In view of these erroneous inertial sensing, it is desirable to have systems and methods for map reconstruction based on a high-accuracy yet still low-cost trajectory tracking.

Continuous monitoring of respiration as well as heart rate is critical for early detection and prevention of potentially fatal diseases. Current solutions usually require users to wear dedicated devices such as wrist-worn sensors or chest straps, which require to contact human body during the monitoring, making them less convenient and comfortable. With the rapid development of Internet of Things (IoT), wireless sensing has received increasing attention in recent years because of the ubiquitous deployment of wireless devices. It has been proved that the presence of human will affect wireless signal propagation, enabling the functionality of wirelessly monitoring human subjects by analyzing the electromagnetic (EM) wave.

In addition, vehicle monitoring has become a very important application with the ubiquitous deployment of wireless devices in vehicles. For example, by occupant awareness, the vehicle monitoring may enable the identification of the motion and breathing of a pet (pet awareness), identification of subtle motion and breathing of a child (child awareness), detection of motion within the trunk (unknown awareness), detection of broken glass (window breakage) and a hit and run. Existing methods and systems related to vehicle monitoring are not entirely satisfactory in terms of effectiveness, security and safety concerns.

## SUMMARY

The present teaching generally relates to wireless sensing, monitoring and tracking. More specifically, the present teaching relates to: Wi-Fi-based sleep monitoring system that can work on any low-cost Internet-of-things (IoT) devices; heartbeat tracking and monitoring by processing wireless channel information and beamforming; detecting voice activity based on radio signals; map reconstruction based on wireless tracking; and wireless monitoring vital signs by processing, decomposing and enhancing wireless channel information.

In one embodiment, a system for radio-based sleep tracking is described. The system comprises: a transmitter configured to transmit a first wireless signal through a wireless multipath channel in a venue; a receiver configured to receive a second wireless signal through the wireless multipath channel, wherein the second wireless signal differs from the first wireless signal due to the wireless multipath channel which is impacted by a sleeping motion of an object in the venue; and a processor. The processor is configured for: obtaining a time series of channel information (TSCI) of the wireless multipath channel based on the second wireless signal, wherein each channel information (CI) of the TSCI comprises N1 components, wherein N1 is a positive integer larger than one, computing N1 component-wise analytics each associated with one of the N1 components of the TSCI, identifying N2 largest component-wise analytics among the N1 component-wise analytics, wherein N2 is a positive integer less than N1, computing at least one first motion statistics based on the N2 largest component-wise analytics of the TSCI, and monitoring the sleeping motion of the object based on the at least one first motion statistics.

In another embodiment, a wireless device of a system for radio-based sleep tracking is described. The wireless device comprises: a processor; a memory communicatively coupled to the processor; and a receiver communicatively coupled to the processor. An additional wireless device of the system is configured to transmit a first wireless signal through a wireless multipath channel in a venue. The receiver is configured to receive a second wireless signal through the wireless multipath channel. The second wireless signal differs from the first wireless signal due to the wireless multipath channel which is impacted by a sleeping motion of an object in the venue. The processor is configured for: obtaining a time series of channel information (TSCI) of the wireless multipath channel based on the second wireless signal, wherein each channel information (CI) of the TSCI comprises N1 components, wherein N1 is a positive integer larger than one, computing N1 component-wise analytics each associated with one of the N1 components of the TSCI, identifying N2 largest component-wise analytics among the N1 component-wise analytics, wherein N2 is a positive integer less than N1, computing at least one first motion statistics based on the N2 largest component-wise analytics of the TSCI, and monitoring the sleeping motion of the object based on the at least one first motion statistics.

In yet another embodiment, a method for radio-based sleep tracking is described. The method comprises: transmitting a first wireless signal through a wireless multipath channel in a venue; receiving a second wireless signal through the wireless multipath channel, wherein the second wireless signal differs from the first wireless signal due to the wireless multipath channel which is impacted by a sleeping motion of an object in the venue; obtaining a time series of channel information (TSCI) of the wireless multipath channel based on the second wireless signal, wherein each channel information (CI) of the TSCI comprises N1 components, wherein N1 is a positive integer larger than one; computing N1 component-wise analytics each associated with one of the N1 components of the TSCI; identifying N2 largest component-wise analytics among the N1 component-wise analytics, wherein N2 is a positive integer less than N1; computing at least one first motion statistics based on the N2 largest component-wise analytics of the TSCI; and monitoring the sleeping motion of the object based on the at least one first motion statistics.

In one embodiment, a system for wireless monitoring is described. The system comprises: a transmitter configured for transmitting, using N1 transmit antennas, a first wireless signal through a wireless channel of a venue; a receiver configured for receiving, using N2 receive antennas, a second wireless signal through the wireless channel; and a processor. N1 and N2 are positive integers. The second wireless signal comprises a reflection of the first wireless signal by at least one living being having at least one repetitive motion in the venue. The processor is configured for: obtaining a plurality of time series of channel information (TSCI) of the wireless channel based on the second wireless signal, wherein each of the plurality of TSCI is associated with a respective transmit antenna of the transmitter and a respective receive antenna of the receiver; generating, for each living being of the at least one living being, a vital signal representing all repetitive motions of the living being based on the plurality of TSCI; extracting, from the vital signal of each living being, a heartbeat signal; and monitoring, for each living being in the venue, a heart rate variability based on the heartbeat signal.

In another embodiment, a wireless device of a wireless monitoring system is described. The wireless device comprises: a processor; a memory communicatively coupled to the processor; and a receiver communicatively coupled to the processor. An additional wireless device of the wireless monitoring system is configured for transmitting a first wireless signal through a wireless channel of a venue. The receiver is configured for receiving a second wireless signal through the wireless channel. The second wireless signal comprises a reflection of the first wireless signal by at least one living being having at least one repetitive motion in the venue. The processor is configured for: obtaining a time series of channel information (TSCI) of the wireless channel based on the second wireless signal; generating, for each living being of the at least one living being, a vital signal representing all repetitive motions of the living being based on the TSCI; extracting, from the vital signal of each living being, a heartbeat signal; and monitoring, for each living being in the venue, a heart rate variability based on the heartbeat signal.

In yet another embodiment, a method of a wireless monitoring system is described. The method comprises: transmitting a first wireless signal through a wireless channel of a venue; receiving a second wireless signal through the wireless channel, wherein the second wireless signal comprises a reflection of the first wireless signal by a plurality of human beings in the venue; obtaining a time series of channel information (TSCI) of the wireless channel based on the second wireless signal, wherein each CI comprises at least one of: a channel state information (CSI), channel impulse response (CIR), channel frequency response (CFR), or received signal strength index (RSSI); generating, for each of the plurality of human beings, a vital signal representing all repetitive motions of the human being based on the TSCI; extracting, from the vital signal of each human being, a heartbeat signal; and simultaneously monitoring, for each of the plurality of human beings, a heart rate variability based on the heartbeat signal.

In a different embodiment, a system for vital sign monitoring based on wireless beamforming is described. The system comprises: a transmitter configured to transmit a wireless signal through a wireless channel of a venue; a receiver configured to receive the wireless signal through the wireless channel that is being impacted by an object motion of an object in the venue; and a processor. At least one of the transmitter or the receiver comprises an array of antennas used to transmit or receive the wireless signal. The object motion comprises at least one non-periodic body motion of the object and at least one periodic vital-sign motion of the object. The processor is configured for: segmenting space around the venue into a plurality of sectors based on a beamforming and the received wireless signal, wherein each sector of the plurality of sectors is associated with a spatial direction relative to the array of antennas, obtaining a plurality of time series of channel information (CI) of the wireless channel based on the beamforming, wherein each time series of CI (TSCI) of the plurality of TSCI is associated with a respective sector of the plurality of sectors, isolating the object motion of the object in the plurality of TSCI to generate a plurality of isolated TSCI, compensating for the at least one non-periodic body motion of the object in the plurality of isolated TSCI to generate a plurality of compensated TSCI, and monitoring the at least one periodic vital-sign motion of the object based on the plurality of compensated TSCI.

In another embodiment, a wireless device of a vital sign monitoring system is described. The wireless device comprises: a processor; a memory communicatively coupled to the processor; and a receiver communicatively coupled to the processor. An additional wireless device of the vital sign monitoring system is configured for transmitting a wireless signal through a wireless channel of a venue. The receiver is configured for receiving the wireless signal through the wireless channel that is being impacted by an object motion of an object in the venue. At least one of the transmitter or the receiver comprises an array of antennas used to transmit or receive the wireless signal. The object motion comprises at least one non-periodic body motion of the object and at least one periodic vital-sign motion of the object. The processor is configured for: segmenting space around the venue into a plurality of sectors based on a beamforming and the received wireless signal, wherein each sector of the plurality of sectors is associated with a spatial direction relative to the array of antennas, obtaining a plurality of time series of channel information (CI) of the wireless channel based on the beamforming, wherein each time series of CI (TSCI) of the plurality of TSCI is associated with a respective sector of the plurality of sectors, isolating the object motion of the object in the plurality of TSCI to generate a plurality of isolated TSCI, compensating for the at least one non-periodic body motion of the object in the plurality of isolated TSCI to generate a plurality of compensated TSCI, and monitoring the at least one periodic vital-sign motion of the object based on the plurality of compensated TSCI.

In yet another embodiment, a method of a vital sign monitoring system is described. The method comprises: transmitting, by a transmitter, a wireless signal through a wireless channel of a venue; receiving, by a receiver, the wireless signal through the wireless channel that is being impacted by an object motion of an object in the venue, wherein at least one of the transmitter or the receiver comprises an array of antennas used to transmit or receive the wireless signal, the object motion comprises at least one non-periodic body motion of the object and at least one periodic vital-sign motion of the object; segmenting space around the venue into a plurality of sectors based on a beamforming and the received wireless signal, wherein each sector of the plurality of sectors is associated with a spatial direction relative to the array of antennas; obtaining a plurality of time series of channel information (CI) of the wireless channel based on the beamforming, wherein each time series of CI (TSCI) of the plurality of TSCI is associated with a respective sector of the plurality of sectors; isolating the object motion of the object in the plurality of TSCI to generate a plurality of isolated TSCI; compensating for the at least one non-periodic body motion of the object in the plurality of isolated TSCI to generate a plurality of compensated TSCI; and monitoring the at least one periodic vital-sign motion of the object based on the plurality of compensated TSCI.

In one embodiment, a system for radio-based voice activity detection is described. The system comprises: a transmitter configured to transmit a radio signal through a wireless channel of a venue; a receiver configured to receive the radio signal through the wireless channel, wherein the wireless channel is impacted by a voice activity of a target voice source in the venue; and a processor. The processor is configured for: computing a time series of channel information (CI) of the wireless channel based on the radio signal, and detecting the voice activity of the target voice source based on the time series of CI (TSCI) of the wireless channel, without using any media signal.

In another embodiment, a method for radio-based voice activity detection is described. The method comprises: obtaining a radio signal transmitted from a transmitter to a receiver through a wireless channel of a venue, wherein the wireless channel is impacted by a voice activity of a target voice source in the venue; computing a time series of channel information (CI) of the wireless channel based on the radio signal; and detecting the voice activity of the target voice source based on the time series of CI (TSCI) of the wireless channel, without using any signal other than the radio signal.

In one embodiment, a system for map generation is described. The system comprises: a sensor configured to collect sensing data in a venue and obtain a plurality of trajectories, and a processor. Each trajectory is a time series of spatial coordinates (TSSC) representing a path traversed by a respective object in the venue. Each TSSC is accompanied by at least one respective time series of sensing data (TSSD) collected while the respective object traverses the path in the venue. The processor is configured for: segmenting each TSSC and its accompanying at least one TSSD into segments, bundling the plurality of trajectories based on similarity measures between pairs of the segments, fusing the bundled trajectories to generate fused trajectories, computing a shape of the fused trajectories, and generating a map of the venue based on the computed shape.

In another embodiment, an apparatus for map generation is described. The apparatus comprises: a sensor and a processor communicatively coupled to the sensor. The sensor is configured to collect sensing data in a venue and obtain a plurality of trajectories. Each trajectory is a time series of spatial coordinates (TSSC) representing a path traversed by a respective object in the venue. Each TSSC is accompanied by at least one respective time series of sensing data (TSSD) collected while the respective object traverses the path in the venue. The processor is configured for: segmenting each TSSC and its accompanying at least one TSSD into segments, bundling the plurality of trajectories based on similarity measures between pairs of the segments, fusing the bundled trajectories to generate fused trajectories, computing a shape of the fused trajectories, and generating a map of the venue based on the computed shape.

In yet another embodiment, a method for map generation is described. The method comprises: obtaining sensing data and a plurality of trajectories in a venue, wherein: each trajectory is a time series of spatial coordinates (TSSC) representing a path traversed by a respective object in the venue, each TSSC is accompanied by at least one respective time series of sensing data (TSSD) collected while the respective object traverses the path in the venue; segmenting each TSSC and its accompanying at least one TSSD into segments; bundling the plurality of trajectories based on similarity measures between pairs of the segments; fusing the bundled trajectories to generate fused trajectories; computing a shape of the fused trajectories; and generating a map of the venue based on the computed shape.

In one embodiment, a system for wireless vital sign monitoring is described. The system comprises: a transmitter configured to transmit a wireless signal through a wireless channel of a venue; a receiver configured to receive the wireless signal through the wireless channel; and a processor. The received wireless signal differs from the transmitted wireless signal due to the wireless channel that is impacted by a periodic motion of a vital sign of an object in the venue. The processor is configured for: obtaining a time series of channel information (CI) of the wireless channel based on the received wireless signal, computing a two dimensional (2D) decomposition of the time series of CI (TSCI), enhancing the 2D decomposition, and monitoring the periodic motion of the vital sign based on the enhanced 2D decomposition.

In another embodiment, a wireless device of a system for wireless vital sign monitoring is described. The wireless device comprises: a processor; a memory communicatively coupled to the processor; and a receiver communicatively coupled to the processor. An additional wireless device in the system is configured to transmit a wireless signal through a wireless channel of a venue. The receiver is configured to receive the wireless signal through the wireless channel. The received wireless signal differs from the transmitted wireless signal due to the wireless channel that is impacted by a periodic motion of a vital sign of an object in the venue. The processor is configured for: obtaining a time series of channel information (CI) of the wireless channel based on the received wireless signal, computing a two dimensional (2D) decomposition of the time series of CI (TSCI), enhancing the 2D decomposition, and monitoring the periodic motion of the vital sign based on the enhanced 2D decomposition.

In yet another embodiment, a method for wireless vital sign monitoring is described. The method comprises: transmitting a wireless signal through a wireless channel of a venue; receiving the wireless signal through the wireless channel, wherein the received wireless signal differs from the transmitted wireless signal due to the wireless channel that is impacted by a periodic motion of a vital sign of an object in the venue; obtaining a time series of channel information (CI) of the wireless channel based on the received wireless signal; computing a two dimensional (2D) decomposition of the time series of CI (TSCI); enhancing the 2D decomposition; and monitoring the periodic motion of the vital sign based on the enhanced 2D decomposition.

Other concepts relate to software for implementing the present teaching on wireless sensing, monitoring and tracking. Additional novel features will be set forth in part in the description which follows, and in part will become apparent to those skilled in the art upon examination of the following and the accompanying drawings or may be learned by production or operation of the examples. The novel features of the present teachings may be realized and attained by practice or use of various aspects of the methodologies, instrumentalities and combinations set forth in the detailed examples discussed below.

## BRIEF DESCRIPTION OF DRAWINGS

The methods, systems, and/or devices described herein are further described in terms of exemplary embodiments. These exemplary embodiments are described in detail with reference to the drawings. These embodiments are non-limiting exemplary embodiments, in which like reference numerals represent similar structures throughout the several views of the drawings.

FIG. 1 illustrates an example of sleep related motion and micromotion data, according to some embodiments of the present disclosure.
FIG. 2 illustrates an example of detecting body movements during sleep (BMS), according to some embodiments of the present disclosure.
FIG. 3 illustrates an example of calculated BMS score, according to some embodiments of the present disclosure.
FIG. 4 illustrates an example of estimated presence and activities throughout a whole day of 24 hours based on motion detection, according to some embodiments of the present disclosure.
FIG. 5 illustrates an example of estimated sleep likelihood and sleep tracking result, according to some embodiments of the present disclosure.
FIG. 6 illustrates an exemplary scenario where object motion or sleep motion is detected in a venue, according to some embodiments of the present disclosure.
FIG. 7 illustrates an exemplary method for radio-based sleep tracking, according to some embodiments of the present disclosure.
FIG. 8 illustrates an exemplary unified method for sleep monitoring and tracking, according to some embodiments of the present disclosure.
FIG. 9 illustrates a flow chart of an exemplary method for radio-based sleep tracking, according to some embodiments of the present disclosure.
FIG. 10A illustrates an exemplary setup for a wireless vital monitoring system, according to some embodiments of the present disclosure.
FIG. 10B illustrates an exemplary workflow for wirelessly monitoring heart rate variability, according to some embodiments of the present disclosure.
FIG. 11 illustrates exemplary basic concepts of a frequency-modulated continuous wave (FMCW) radar system, according to some embodiments of the present disclosure.
FIG. 12 illustrates exemplary antenna deployment of a wireless vital monitoring system, according to some embodiments of the present disclosure.
FIGS. 13A-13D illustrate exemplary performances of a reflecting object detector, according to some embodiments of the present disclosure.
FIGS. 14A-14D illustrate exemplary performances of a human subject detector, according to some embodiments of the present disclosure.
FIGS. 15A-15B illustrate exemplary performances of a heartbeat extractor, according to some embodiments of the present disclosure.
FIGS. 16A-16C illustrate an exemplary inter-beat intervals (IBI) estimation, according to some embodiments of the present disclosure.
FIGS. 17A-17B illustrate examples of IBI estimation error, according to some embodiments of the present disclosure.
FIG. 18 illustrates a flow chart of an exemplary method for wireless vital monitoring, according to some embodiments of the present disclosure.
FIG. 19 illustrates exemplary vital signals in different range-azimuth bins, according to some embodiments of the present disclosure.
FIG. 20 illustrates an exemplary processing flow of a vital sign monitoring system, according to some embodiments of the present disclosure.
FIG. 21 illustrates exemplary signal transmissions of a vital sign monitoring system using a Frequency-Modulated Continuous Wave (FMCW) radar, according to some embodiments of the present disclosure.
FIG. 22A illustrates an exemplary CIR amplitude before clutter removal, where the reflections from the driver are submerged in the background reflections, according to some embodiments of the present disclosure.
FIG. 22B illustrates an exemplary CIR amplitude after clutter removal, where the reflections corresponding to the driver can be easily identified, according to some embodiments of the present disclosure.

FIG. 23 illustrates an example of consecutive frame after clutter removal, according to some embodiments of the present disclosure.

FIG. 24 illustrates an example of large body movement compensation, according to some embodiments of the present disclosure.

FIG. 25A illustrates an average of the CIR amplitude over 1-min window after large body motion compensation during an exemplary target detection, according to some embodiments of the present disclosure.

FIG. 25B illustrates bins corresponding to the driver by using CFAR detector during an exemplary target detection, according to some embodiments of the present disclosure.

FIG. 26A illustrates an example of fine movement cancellation, where original unwrapped phase measurement from two different vital bins (in solid lines) and the corresponding estimated motion (in dashed lines), according to some embodiments of the present disclosure.

FIG. 26B illustrates phase measurement after an example of fine movement cancellation, according to some embodiments of the present disclosure.

FIG. 26C illustrates the ACF of the calibrated phase measurement during the example of fine movement cancellation, according to some embodiments of the present disclosure.

FIG. 27A illustrates a decomposition result in the time domain of an example of phase decomposition of 3 vital bins, according to some embodiments of the present disclosure.

FIG. 27B illustrates corresponding spectrum of each decomposed component of an example of phase decomposition of 3 vital bins, according to some embodiments of the present disclosure.

FIG. 28A illustrates exemplary RR and HR estimation result of a vital sign monitoring system, according to some embodiments of the present disclosure.

FIG. 28B illustrates estimated heartbeat signal of a vital sign monitoring system compared with the ECG sensor result, where the ground-truth from ECG sensor are marked as vertical dashed lines, according to some embodiments of the present disclosure.

FIG. 28C illustrates estimated IBI of a vital sign monitoring system compared with ground-truth from the ECG sensor, according to some embodiments of the present disclosure.

FIG. 29 illustrates an exemplary overall performance of a vital sign monitoring system based on a Bland-Altman plot, according to some embodiments of the present disclosure.

FIGS. 30A-30C illustrate an exemplary comparison of vital sign estimation performance between a disclosed system and another system, according to some embodiments of the present disclosure.

FIGS. 31A-31C illustrate vital sign estimation performance versus different device locations, according to some embodiments of the present disclosure.

FIGS. 32A-32C illustrate vital sign estimation performance versus different motion types, according to some embodiments of the present disclosure.

FIGS. 33A-33D illustrate an exemplary impact of window length to a vital sign monitoring system based on HRV metrics, according to some embodiments of the present disclosure.

FIG. 34 illustrates a flow chart of an exemplary method for wireless vital sign monitoring, according to some embodiments of the present disclosure.

FIG. 35 illustrates an overview of a speech enhancement and separation system using audio and radio signals, according to some embodiments of the present disclosure.

FIG. 36 illustrates an exemplary block diagram of a speech enhancement and separation system, according to some embodiments of the present disclosure.

FIG. 37 illustrates a constant false alarm rate (CFAR) window for radio feature extraction, according to some embodiments of the present disclosure.

FIG. 38 illustrates an amplitude map for radio feature extraction, according to some embodiments of the present disclosure.

FIG. 39 illustrates a variance map for radio feature extraction, according to some embodiments of the present disclosure.

FIG. 40 illustrates a detection map for radio feature extraction, according to some embodiments of the present disclosure.

FIG. 41 illustrates a clustering output for radio feature extraction, according to some embodiments of the present disclosure.

FIG. 42A and FIG. 42B illustrate a unimodal system and a multimodal system respectively, according to some embodiments of the present disclosure.

FIG. 43 illustrates a detailed structure of a speech enhancement and separation system, according to some embodiments of the present disclosure.

FIG. 44 illustrates a reshaping operation and a workflow of a dual-path recurrent neural network (DPRNN), according to some embodiments of the present disclosure.

FIG. 45 illustrates learning curves for audio-only (AO) and audio-radio (AR) systems, according to some embodiments of the present disclosure.

FIG. 46 illustrates a performance comparison between audio-only (AO) and audio-radio (AR) systems, according to some embodiments of the present disclosure.

FIG. 47 illustrates a differential gain for an audio-radio (AR) system compared to an audio-only (AO) system, according to some embodiments of the present disclosure.

FIGS. 48A-48C illustrate different experimental settings for a speech enhancement and separation system, according to some embodiments of the present disclosure.

FIG. 49 illustrates a flow chart of an exemplary method for radio-assisted signal estimation, according to some embodiments of the present disclosure.

FIG. 50 illustrates a system for radio-assisted signal estimation, according to some embodiments of the present disclosure.

FIG. 51 illustrates a first adaptive filter in a system for radio-assisted signal estimation, according to some embodiments of the present disclosure.

FIG. 52 illustrates a detailed diagram of a first adaptive filter in a system for radio-assisted signal estimation, according to some embodiments of the present disclosure.

FIGS. 53A-53C illustrate exemplary performances of different voice activity detection (VAD) systems, according to some embodiments of the present disclosure.

FIG. 54 illustrates an overview of a voice activity detection system, according to some embodiments of the present disclosure.

FIG. 55 illustrates an exemplary a neural network structure for voice activity detection, according to some embodiments of the present disclosure.

FIGS. 56A and 56B illustrate performance comparison of different voice activity detection (VAD) systems, according to some embodiments of the present disclosure.

FIG. 57 illustrates an exemplary voice activity detection system based on an audio-radio framework, according to some embodiments of the present disclosure.

FIG. 58 illustrates a flow chart of an exemplary method for radio-based voice activity detection, according to some embodiments of the present disclosure.

FIG. 59 illustrates an exemplary diagram of a map reconstruction system, according to some embodiments of the present disclosure.

FIG. 60 illustrates an exemplary structure of atomic segments in a trajectory, according to some embodiments of the present disclosure.

FIG. 61 illustrates exemplary errors accumulated at turns of a trajectory, according to some embodiments of the present disclosure.

FIG. 62 illustrates an exemplary matching process of a map reconstruction system, according to some embodiments of the present disclosure.

FIG. 63A illustrates an exemplary time series of magnetic field strength (MFS), according to some embodiments of the present disclosure.

FIG. 63B illustrates an exemplary time series of Received Signal Strength Indicator (RSSI), according to some embodiments of the present disclosure.

FIG. 64 illustrates an exemplary trajectory bundling process, according to some embodiments of the present disclosure.

FIG. 65 illustrates an exemplary trajectory fusion process, according to some embodiments of the present disclosure.

FIG. 66 illustrates an exemplary curved trajectory positioning process, according to some embodiments of the present disclosure.

FIG. 67A illustrates an exemplary office floor plan used for testing a map reconstruction system, according to some embodiments of the present disclosure.

FIG. 67B illustrates an exemplary home floor plan used for testing a map reconstruction system, according to some embodiments of the present disclosure.

FIG. 67C illustrates an exemplary campus court floor plan used for testing a map reconstruction system, according to some embodiments of the present disclosure.

FIGS. 68A-68D illustrate exemplary reconstruction results of a map reconstruction system, according to some embodiments of the present disclosure.

FIGS. 69A-69D illustrate other exemplary reconstruction results of a map reconstruction system, according to some embodiments of the present disclosure.

FIGS. 70A-70D illustrate additional exemplary reconstruction results of a map reconstruction system, according to some embodiments of the present disclosure.

FIGS. 71A-71E illustrate exemplary reconstructed hallway plans of a map reconstruction system, according to some embodiments of the present disclosure.

FIG. 72 illustrates a flow chart of an exemplary method for map reconstruction, according to some embodiments of the present disclosure.

FIG. 73A illustrates a side perspective view of a car including a wireless vital sign detection system, according to some embodiments of the present disclosure.

FIG. 73B illustrates a top perspective view of a car including a wireless vital sign detection system, according to some embodiments of the present disclosure.

FIG. 74 illustrates a flow chart showing an exemplary method of a wireless vital sign detection system, according to some embodiments of the present disclosure.

FIG. 75 illustrates an exemplary distribution of dynamic time warping (DTW) values during a breathing detection, according to some embodiments of the present disclosure.

FIG. 76 illustrates an exemplary block diagram of a first wireless device of a system for wireless vital sign monitoring, according to some embodiments of the present disclosure.

FIG. 77 illustrates an exemplary block diagram of a second wireless device of a system for wireless vital sign monitoring, according to some embodiments of the present disclosure.

FIG. 78 illustrates a flow chart of an exemplary method for radio-assisted signal estimation, according to some embodiments of the present disclosure.

## DETAILED DESCRIPTION

In one embodiment, the present teaching discloses a method, apparatus, device, system, and/or software (method/apparatus/device/system/software) of a wireless monitoring system. A time series of channel information (CI) of a wireless multipath channel (channel) may be obtained (e.g. dynamically) using a processor, a memory communicatively coupled with the processor and a set of instructions stored in the memory. The time series of CI (TSCI) may be extracted from a wireless signal (signal) transmitted between a Type 1 heterogeneous wireless device (e.g. wireless transmitter, TX) and a Type 2 heterogeneous wireless device (e.g. wireless receiver, RX) in a venue through the channel. The channel may be impacted by an expression (e.g. motion, movement, expression, and/or change in position/pose/shape/expression) of an object in the venue. A characteristics and/or a spatial-temporal information (STI, e.g. motion information) of the object and/or of the motion of the object may be monitored based on the TSCI. A task may be performed based on the characteristics and/or STI. A presentation associated with the task may be generated in a user-interface (UI) on a device of a user. The TSCI may be a wireless signal stream. The TSCI or each CI may be preprocessed. A device may be a station (STA). The symbol "A/B" means "A and/or B" in the present teaching.

The expression may comprise placement, placement of moveable parts, location, position, orientation, identifiable place, region, spatial coordinate, presentation, state, static expression, size, length, width, height, angle, scale, shape, curve, surface, area, volume, pose, posture, manifestation, body language, dynamic expression, motion, motion sequence, gesture, extension, contraction, distortion, deformation, body expression (e.g. head, face, eye, mouth, tongue, hair, voice, neck, limbs, arm, hand, leg, foot, muscle, moveable parts), surface expression (e.g. shape, texture, material, color, electromagnetic (EM) characteristics, visual pattern, wetness, reflectance, translucency, flexibility), material property (e.g. living tissue, hair, fabric, metal, wood, leather, plastic, artificial material, solid, liquid, gas, temperature), movement, activity, behavior, change of expression, and/or some combination.

The wireless signal may comprise: transmitted/received signal, EM radiation, RF signal/transmission, signal in licensed/unlicensed /ISM band, bandlimited signal, baseband signal, wireless/mobile/cellular communication signal, wireless/mobile/cellular network signal, mesh signal, light signal/communication, downlink/uplink signal, unicast/multicast/broadcast signal, standard (e.g. WLAN, WWAN, WPAN, WBAN, international, national, industry, defacto, IEEE, IEEE 802, 802.11/15/16, WiFi, 802.11n/ac/ax/be, 3G/4G/LTE/5G/6G/7G/8G, 3GPP, Bluetooth, BLE, Zigbee, RFID, UWB, WiMax) compliant signal, protocol signal, standard frame, beacon/pilot/probe/enquiry/acknowledgement/handshake/synchronization signal, management/control/data frame, management/control/data signal, standardized wireless/cellular communication protocol, reference signal, source signal, motion probe/detection/sensing signal, and/or series of signals. The wireless signal may comprise a line-of-sight (LOS), and/or a non-LOS component (or path/link). Each CI may be extracted/generated/computed/sensed at a layer (e.g. PHY/MAC layer in OSI model) of Type 2 device and may be obtained by an application (e.g. software, firmware, driver, app, wireless monitoring software/system).

The wireless multipath channel may comprise: a communication channel, analog frequency channel (e.g. with analog carrier frequency near 700/800/900MHz, 1.8/1.8/2.4/3/5/6/27/60 GHz), coded channel (e.g. in CDMA), and/or channel of a wireless network/system (e.g. WLAN, WiFi, mesh, LTE, 4G/5G, Bluetooth, Zigbee, UWB, RFID, microwave). It may comprise more than one channel. The channels may be consecutive (e.g. with adjacent/overlapping bands) or non-consecutive channels (e.g. non-overlapping WiFi channels, one at 2.4GHz and one at 5GHz).

The TSCI may be extracted from the wireless signal at a layer of the Type 2 device (e.g. a layer of OSI reference model, physical layer, data link layer, logical link control layer, media access control (MAC) layer, network layer, transport layer, session layer, presentation layer, application layer, TCP/IP layer, internet layer, link layer). The TSCI may be extracted from a derived signal (e.g. baseband signal, motion detection signal, motion sensing signal) derived from the wireless signal (e.g. RF signal). It may be (wireless) measurements sensed by the communication protocol (e.g. standardized protocol) using existing mechanism (e.g. wireless/cellular communication standard/network, 3G/LTE/4G/5G/6G/7G/8G, WiFi, IEEE 802.11/15/16). The derived signal may comprise a packet with at least one of: a preamble, a header and a payload (e.g. for data/control/management in wireless links/networks). The TSCI may be extracted from a probe signal (e.g. training sequence, STF, LTF, L-STF, L-LTF, L-SIG, HE-STF, HE-LTF, HE-SIG-A, HE-SIG-B, CEF) in the packet. A motion detection/sensing signal may be recognized/identified base on the probe signal. The packet may be a standard-compliant protocol frame, management frame, control frame, data frame, sounding frame, excitation frame, illumination frame, null data frame, beacon frame, pilot frame, probe frame, request frame, response frame, association frame, reassociation frame, disassociation frame, authentication frame, action frame, report frame, poll frame, announcement frame, extension frame, enquiry frame, acknowledgement frame, RTS frame, CTS frame, QoS frame, CF-Poll frame, CF-Ack frame, block acknowledgement frame, reference frame, training frame, and/or synchronization frame.

The packet may comprise a control data and/or a motion detection probe. A data (e.g. ID/parameters/ characteristics/ settings/ control signal/command/instruction/ notification/ broadcasting-related information of the Type 1 device) may be obtained from the payload. The wireless signal may be transmitted by the Type 1 device. It may be received by the Type 2 device. A database (e.g. in local server, hub device, cloud server, storage network) may be used to store the TSCI, characteristics, STI, signatures, patterns, behaviors, trends, parameters, analytics, output responses, identification information, user information, device information, channel information, venue (e.g. map, environmental model, network, proximity devices/networks) information, task information, class/category information, presentation (e.g. UI) information, and/or other information.

The Type 1/Type 2 device may comprise at least one of: electronics, circuitry, transmitter (TX)/receiver (RX)/transceiver, RF interface, "Origin Satellite"/"Tracker Bot", unicast/multicast/broadcasting device, wireless source device, source/destination device, wireless node, hub device, target device, motion detection device, sensor device, remote/wireless sensor device, wireless communication device, wireless-enabled device, standard compliant device, and/or receiver. The Type 1 (or Type 2) device may be heterogeneous because, when there are more than one instances of Type 1 (or Type 2) device, they may have different circuitry, enclosure, structure, purpose, auxiliary functionality, chip/IC, processor, memory, software, firmware, network connectivity, antenna, brand, model, appearance, form, shape, color, material, and/or specification. The Type 1/Type 2 device may comprise: access point, router, mesh router, internet-of-things (IoT) device, wireless terminal, one or more radio/RF subsystem/wireless interface (e.g. 2.4GHz radio, 5GHz radio, front haul radio, backhaul radio), modem, RF front end, RF/radio chip or integrated circuit (IC).

At least one of: Type 1 device, Type 2 device, a link between them, the object, the characteristics, the STI, the monitoring of the motion, and the task may be associated with an identification (ID) such as UUID. The Type 1/Type 2/another device may obtain/store/retrieve/ access/preprocess/condition/process/analyze/monitor/apply the TSCI. The Type 1 and Type 2 devices may communicate network traffic in another channel (e.g. Ethernet, HDMI, USB, Bluetooth, BLE, WiFi, LTE, other network, the wireless multipath channel) in parallel to the wireless signal. The Type 2 device may passively observe/monitor/receive the wireless signal from the Type 1 device in the wireless multipath channel without establishing connection (e.g. association/authentication) with, or requesting service from, the Type 1 device.

The transmitter (i.e. Type 1 device) may function as (play role of) receiver (i.e. Type 2 device) temporarily, sporadically, continuously, repeatedly, interchangeably, alternately, simultaneously, concurrently, and/or contemporaneously; and vice versa. A device may function as Type 1 device (transmitter) and/or Type 2 device (receiver) temporarily, sporadically, continuously, repeatedly, simultaneously, concurrently, and/or contemporaneously. There may be multiple wireless nodes each being Type 1 (TX) and/or Type 2 (RX) device. A TSCI may be obtained between every two nodes when they exchange/communicate wireless signals. The characteristics and/or STI of the object may be monitored individually based on a TSCI, or jointly based on two or more (e.g. all) TSCI.

The motion of the object may be monitored actively (in that Type 1 device, Type 2 device, or both, are wearable of/associated with the object) and/or passively (in that both Type 1 and Type 2 devices are not wearable of/associated with the object). It may be passive because the object may not be associated with the Type 1 device and/or the Type 2 device. The object (e.g. user, an automated guided vehicle or AGV) may not need to carry/install any wearables/fixtures (i.e. the Type 1 device and the Type 2 device are not wearable/attached devices that the object needs to carry in order perform the task). It may be active because the object may be associated with either the Type 1 device and/or the Type 2 device. The object may carry (or installed) a wearable/a fixture (e.g. the Type 1 device, the Type 2 device, a device communicatively coupled with either the Type 1 device or the Type 2 device).

The presentation may be visual, audio, image, video, animation, graphical presentation, text, etc. A computation of the task may be performed by a processor (or logic unit) of the Type 1 device, a processor (or logic unit) of an IC of the Type 1 device, a processor (or logic unit) of the Type 2 device, a processor of an IC of the Type 2 device, a local server, a cloud server, a data analysis subsystem, a signal analysis subsystem, and/or another processor. The task may be performed with/without reference to a wireless fingerprint or a baseline (e.g. collected, processed, computed, transmitted and/or stored in a training phase/survey/current survey/previous survey/recent survey/initial wireless survey, a passive fingerprint), a training, a profile, a trained profile, a static profile, a survey, an initial wireless survey, an initial setup, an installation, a re-training, an updating and a reset.

The Type 1 device (TX device) may comprise at least one heterogeneous wireless transmitter. The Type 2 device (RX device) may comprise at least one heterogeneous wireless receiver. The Type 1 device and the Type 2 device may be collocated. The Type 1 device and the Type 2 device may be the same device. Any device may have a data processing unit/apparatus, a computing unit/system, a network unit/system, a processor (e.g. logic unit), a memory communicatively coupled with the processor, and a set of instructions stored in the memory to be executed by the processor. Some processors, memories and sets of instructions may be coordinated.

There may be multiple Type 1 devices interacting (e.g. communicating, exchange signal/control/notification/other data) with the same Type 2 device (or multiple Type 2 devices), and/or there may be multiple Type 2 devices interacting with the same Type 1 device. The multiple Type 1 devices/Type 2 devices may be synchronized and/or asynchronous, with same/different window width/size and/or time shift, same/different synchronized start time, synchronized end time, etc. Wireless signals sent by the multiple Type 1 devices may be sporadic, temporary, continuous, repeated, synchronous, simultaneous, concurrent, and/or contemporaneous. The multiple Type 1 devices/Type 2 devices may operate independently and/or collaboratively. A Type 1 and/or Type 2 device may have/comprise/be heterogeneous hardware circuitry (e.g. a heterogeneous chip or a heterogeneous IC capable of generating/receiving the wireless signal, extracting CI from received signal, or making the CI available). They may be communicatively coupled to same or different servers (e.g. cloud server, edge server, local server, hub device).

Operation of one device may be based on operation, state, internal state, storage, processor, memory output, physical location, computing resources, network of another device. Difference devices may communicate directly, and/or via another device/server/hub device/cloud server. The devices may be associated with one or more users, with associated settings. The settings may be chosen once, pre-programmed, and/or changed (e.g. adjusted, varied, modified)/varied over time. There may be additional steps in the method. The steps and/or the additional steps of the method may be performed in the order shown or in another order. Any steps may be performed in parallel, iterated, or otherwise repeated or performed in another manner. A user may be human, adult, older adult, man, woman, juvenile, child, baby, pet, animal, creature, machine, computer module/software, etc.

In the case of one or multiple Type 1 devices interacting with one or multiple Type 2 devices, any processing (e.g. time domain, frequency domain) may be different for different devices. The processing may be based on locations, orientation, direction, roles, user-related characteristics, settings, configurations, available resources, available bandwidth, network connection, hardware, software, processor, co-processor, memory, battery life, available power, antennas, antenna types, directional/unidirectional characteristics of the antenna, power setting, and/or other parameters/characteristics of the devices.

The wireless receiver (e.g. Type 2 device) may receive the signal and/or another signal from the wireless transmitter (e.g. Type 1 device). The wireless receiver may receive another signal from another wireless transmitter (e.g. a second Type 1 device). The wireless transmitter may transmit the signal and/or another signal to another wireless receiver (e.g. a second Type 2 device). The wireless transmitter, wireless receiver, another wireless receiver and/or another wireless transmitter may be moving with the object and/or another object. The another object may be tracked.

The Type 1 and/or Type 2 device may be capable of wirelessly coupling with at least two Type 2 and/or Type 1 devices. The Type 1 device may be caused/controlled to switch/establish wireless coupling (e.g. association, authentication) from the Type 2 device to a second Type 2 device at another location in the venue. Similarly, the Type 2 device may be caused/controlled to switch/establish wireless coupling from the Type 1 device to a second Type 1 device at yet another location in the venue. The switching may be controlled by a server (or a hub device), the processor, the Type 1 device, the Type 2 device, and/or another device. The radio used before and after switching may be different. A second wireless signal (second signal) may be caused to be transmitted between the Type 1 device and the second Type 2 device (or between the Type 2 device and the second Type 1 device) through the channel. A second TSCI of the channel extracted from the second signal may be obtained. The second signal may be the first signal. The characteristics, STI and/or another quantity of the object may be monitored based on the second TSCI. The Type 1 device and the Type 2 device may be the same. The characteristics, STI and/or another quantity with different time stamps may form a waveform. The waveform may be displayed in the presentation.

The wireless signal and/or another signal may have data embedded. The wireless signal may be a series of probe signals (e.g. a repeated transmission of probe signals, a re-use of one or more probe signals). The probe signals may change/vary over time. A probe signal may be a standard compliant signal, protocol signal, standardized wireless protocol signal, control signal, data signal, wireless communication network signal, cellular network signal, WiFi signal, LTE/5G/6G/7G signal, reference signal, beacon signal, motion detection signal, and/or motion sensing signal. A probe signal may be formatted according to a wireless network standard (e.g. WiFi), a cellular network standard (e.g. LTE/5G/6G), or another standard. A probe signal may comprise a packet with a header and a payload. A probe signal may have data embedded. The payload may comprise data. A probe signal may be replaced by a data signal. The probe signal may be embedded in a data signal. The wireless receiver, wireless transmitter, another wireless receiver and/or another wireless transmitter may be associated with at least one processor, memory communicatively coupled with respective processor, and/or respective set of instructions stored in the memory which when executed cause the processor to perform any and/or all steps needed to determine the STI (e.g. motion information), initial STI, initial time, direction, instantaneous location, instantaneous angle, and/or speed, of the object.

The processor, the memory and/or the set of instructions may be associated with the Type 1 device, one of the at least one Type 2 device, the object, a device associated with the object, another device associated with the venue, a cloud server, a hub device, and/or another server.

The Type 1 device may transmit the signal in a broadcasting manner to at least one Type 2 device(s) through the channel in the venue. The signal is transmitted without the Type 1 device establishing wireless connection (e.g. association, authentication) with any Type 2 device, and without any Type 2 device requesting services from the Type 1 device. The Type 1 device may transmit to a particular media access control (MAC) address common for more than one Type 2 devices. Each Type 2 device may adjust its MAC address to the particular MAC address. The particular MAC address may be associated with the venue. The association may be recorded in an association table of an Association Server (e.g. hub device). The venue may be identified by the Type 1 device, a Type 2 device and/or another device based on the particular MAC address, the series of probe signals, and/or the at least one TSCI extracted from the probe signals.

For example, a Type 2 device may be moved to a new location in the venue (e.g. from another venue). The Type 1 device may be newly set up in the venue such that the Type 1 and Type 2 devices are not aware of each other. During set up, the Type 1 device may be instructed/guided/ caused/ controlled (e.g. using dummy receiver, using hardware pin setting/connection, using stored setting, using local setting, using remote setting, using downloaded setting, using hub device, or using server) to send the series of probe signals to the particular MAC address. Upon power up, the Type 2 device may scan for probe signals according to a table of MAC addresses (e.g. stored in a designated source, server, hub device, cloud server) that may be used for broadcasting at different locations (e.g. different MAC address used for different venue such as house, office, enclosure, floor, multi-storey building, store, airport, mall, stadium, hall, station, subway, lot, area, zone, region, district, city, country, continent). When the Type 2 device detects the probe signals sent to the particular MAC address, the Type 2 device can use the table to identify the venue based on the MAC address.

A location of a Type 2 device in the venue may be computed based on the particular MAC address, the series of probe signals, and/or the at least one TSCI obtained by the Type 2 device from the probe signals. The computing may be performed by the Type 2 device.

The particular MAC address may be changed (e.g. adjusted, varied, modified) over time. It may be changed according to a time table, rule, policy, mode, condition, situation and/or change. The particular MAC address may be selected based on availability of the MAC address, a pre-selected list, collision pattern, traffic pattern, data traffic between the Type 1 device and another device, effective bandwidth, random selection, and/or a MAC address switching plan. The particular MAC address may be the MAC address of a second wireless device (e.g. a dummy receiver, or a receiver that serves as a dummy receiver).

The Type 1 device may transmit the probe signals in a channel selected from a set of channels. At least one CI of the selected channel may be obtained by a respective Type 2 device from the probe signal transmitted in the selected channel.

The selected channel may be changed (e.g. adjusted, varied, modified) over time. The change may be according to a time table, rule, policy, mode, condition, situation, and/or change. The selected channel may be selected based on availability of channels, random selection, a pre-selected list, co-channel interference, inter-channel interference, channel traffic pattern, data traffic between the Type 1 device and another device, effective bandwidth associated with channels, security criterion, channel switching plan, a criterion, a quality criterion, a signal quality condition, and/or consideration.

The particular MAC address and/or an information of the selected channel may be communicated between the Type 1 device and a server (e.g. hub device) through a network. The particular MAC address and/or the information of the selected channel may also be communicated between a Type 2 device and a server (e.g. hub device) through another network. The Type 2 device may communicate the particular MAC address and/or the information of the selected channel to another Type 2 device (e.g. via mesh network, Bluetooth, WiFi, NFC, ZigBee, etc.). The particular MAC address and/or selected channel may be chosen by a server (e.g. hub device). The particular MAC address and/or selected channel may be signaled in an announcement channel by the Type 1 device, the Type 2 device and/or a server (e.g. hub device). Before being communicated, any information may be pre-processed.

Wireless connection (e.g. association, authentication) between the Type 1 device and another wireless device may be established (e.g. using a signal handshake). The Type 1 device may send a first handshake signal (e.g. sounding frame, probe signal, request-to-send RTS) to the another device. The another device may reply by sending a second handshake signal (e.g. a command, or a clear-to-send CTS) to the Type 1 device, triggering the Type 1 device to transmit the signal (e.g. series of probe signals) in the broadcasting manner to multiple Type 2 devices without establishing connection with any Type 2 device. The second handshake signals may be a response or an acknowledge (e.g. ACK) to the first handshake signal. The second handshake signal may contain a data with information of the venue, and/or the Type 1 device. The another device may be a dummy device with a purpose (e.g. primary purpose, secondary purpose) to establish the wireless connection with the Type 1 device, to receive the first signal, and/or to send the second signal. The another device may be physically attached to the Type 1 device.

In another example, the another device may send a third handshake signal to the Type 1 device triggering the Type 1 device to broadcast the signal (e.g. series of probe signals) to multiple Type 2 devices without establishing connection (e.g. association, authentication) with any Type 2 device. The Type 1 device may reply to the third special signal by transmitting a fourth handshake signal to the another device. The another device may be used to trigger more than one Type 1 devices to broadcast. The triggering may be sequential, partially sequential, partially parallel, or fully parallel. The another device may have more than one wireless circuitries to trigger multiple transmitters in parallel. Parallel trigger may also be achieved using at least one yet another device to perform the triggering (similar to what as the another device does) in parallel to the another device. The another device may not communicate (or suspend communication) with the Type 1 device after establishing connection with the Type 1 device. Suspended communication may be resumed. The another device may enter an inactive mode, hibernation mode, sleep mode, stand-by mode, low-power mode, OFF mode and/or power-down mode, after establishing the connection with the Type 1 device. The another device may have the particular MAC address so that the Type 1 device sends the signal to the particular MAC address. The Type 1 device and/or the another device may be controlled and/or coordinated by a first processor associated with the Type 1 device, a second processor associated with the another device, a third processor associated with a designated source and/or a fourth processor associated with another device. The first and second processors may coordinate with each other.

A first series of probe signals may be transmitted by a first antenna of the Type 1 device to at least one first Type 2 device through a first channel in a first venue. A second series of probe signals may be transmitted by a second antenna of the Type 1 device to at least one second Type 2 device through a second channel in a second venue. The first series and the second series may/may not be different. The at least one first Type 2 device may/may not be different from the at least one second Type 2 device. The first and/or second series of probe signals may be broadcasted without connection (e.g. association, authentication) established between the Type 1 device and any Type 2 device. The first and second antennas may be same/different.

The two venues may have different sizes, shape, multipath characteristics. The first and second venues may overlap. The respective immediate areas around the first and second antennas may overlap. The first and second channels may be same/different. For example, the first one may be WiFi while the second may be LTE. Or, both may be WiFi, but the first one may be 2.4GHz WiFi and the second may be 5GHz WiFi. Or, both may be 2.4GHz WiFi, but have different channel numbers, SSID names, and/or WiFi settings.

Each Type 2 device may obtain at least one TSCI from the respective series of probe signals, the CI being of the respective channel between the Type 2 device and the Type 1 device. Some first Type 2 device(s) and some second Type 2 device(s) may be the same. The first and second series of probe signals may be synchronous/asynchronous. A probe signal may be transmitted with data or replaced by a data signal. The first and second antennas may be the same.

The first series of probe signals may be transmitted at a first rate (e.g. 30Hz). The second series of probe signals may be transmitted at a second rate (e.g. 200Hz). The first and second rates may be same/different. The first and/or second rate may be changed (e.g. adjusted, varied, modified) over time. The change may be according to a time table, rule, policy, mode, condition, situation, and/or change. Any rate may be changed (e.g. adjusted, varied, modified) over time.

The first and/or second series of probe signals may be transmitted to a first MAC address and/or second MAC address respectively. The two MAC addresses may be same/different. The first series of probe signals may be transmitted in a first channel. The second series of probe signals may be transmitted in a second channel. The two channels may be same/different. The first or second MAC address, first or second channel may be changed over time. Any change may be according to a time table, rule, policy, mode, condition, situation, and/or change.

The Type 1 device and another device may be controlled and/or coordinated, physically attached, or may be of/in/of a common device. They may be controlled by/connected to a common data processor, or may be connected to a common bus interconnect/ network/ LAN/ Bluetooth network/ NFC network/ BLE network/ wired network/ wireless network/ mesh network/ mobile network/ cloud. They may share a common memory, or be associated with a common user, user device, profile, account, identity (ID), identifier, household, house, physical address, location, geographic coordinate, IP subnet, SSID, home device, office device, and/or manufacturing device.

Each Type 1 device may be a signal source of a set of respective Type 2 devices (i.e. it sends a respective signal (e.g. respective series of probe signals) to the set of respective Type 2 devices). Each respective Type 2 device chooses the Type 1 device from among all Type 1 devices as its signal source. Each Type 2 device may choose asynchronously. At least one TSCI may be obtained by each respective Type 2 device from the respective series of probe signals from the Type 1 device, the CI being of the channel between the Type 2 device and the Type 1 device.

The respective Type 2 device chooses the Type 1 device from among all Type 1 devices as its signal source based on identity (ID) or identifier of Type 1/Type 2 device, task to be performed, past signal source, history (e.g. of past signal source, Type 1 device, another Type 1 device, respective Type 2 receiver, and/or another Type 2 receiver), threshold for switching signal source, and/or information of a user, account, access info, parameter, characteristics, and/or signal strength (e.g. associated with the Type 1 device and/or the respective Type 2 receiver).

Initially, the Type 1 device may be signal source of a set of initial respective Type 2 devices (i.e. the Type 1 device sends a respective signal (series of probe signals) to the set of initial respective Type 2 devices) at an initial time. Each initial respective Type 2 device chooses the Type 1 device from among all Type 1 devices as its signal source.

The signal source (Type 1 device) of a particular Type 2 device may be changed (e.g. adjusted, varied, modified) when (1) time interval between two adjacent probe signals (e.g. between current probe signal and immediate past probe signal, or between next probe signal and current probe signal) received from current signal source of the Type 2 device exceeds a first threshold; (2) signal strength associated with current signal source of the Type 2 device is below a second threshold; (3) a processed signal strength associated with current signal source of the Type 2 device is below a third threshold, the signal strength processed with low pass filter, band pass filter, median filter, moving average filter, weighted averaging filter, linear filter and/or non-linear filter; and/or (4) signal strength (or processed signal strength) associated with current signal source of the Type 2 device is below a fourth threshold for a significant percentage of a recent time window (e.g. 70%, 80%, 90%). The percentage may exceed a fifth threshold. The first, second, third, fourth and/or fifth thresholds may be time varying.

Condition (1) may occur when the Type 1 device and the Type 2 device become progressively far away from each other, such that some probe signal from the Type 1 device becomes too weak and is not received by the Type 2 device. Conditions (2)-(4) may occur when the two devices become far from each other such that the signal strength becomes very weak.

The signal source of the Type 2 device may not change if other Type 1 devices have signal strength weaker than a factor (e.g. 1, 1.1, 1.2, or 1.5) of the current signal source.

If the signal source is changed (e.g. adjusted, varied, modified), the new signal source may take effect at a near future time (e.g. the respective next time). The new signal source may be the Type 1 device with strongest signal strength, and/or processed signal strength. The current and new signal source may be same/different.

A list of available Type 1 devices may be initialized and maintained by each Type 2 device. The list may be updated by examining signal strength and/or processed signal strength associated with the respective set of Type 1 devices. A Type 2 device may choose between a first series of probe signals from a first Type 1 device and a second series of probe signals from a second Type 1 device based on: respective probe signal rate, MAC addresses, channels, characteristics/properties/ states, task to be performed by the Type 2 device, signal strength of first and second series, and/or another consideration.

The series of probe signals may be transmitted at a regular rate (e.g. 100 Hz). The series of probe signals may be scheduled at a regular interval (e.g. 0.01s for 100 Hz), but each probe signal may experience small time perturbation, perhaps due to timing requirement, timing control, network control, handshaking, message passing, collision avoidance, carrier sensing, congestion, availability of resources, and/or another consideration.

The rate may be changed (e.g. adjusted, varied, modified). The change may be according to a time table (e.g. changed once every hour), rule, policy, mode, condition and/or change (e.g. changed whenever some event occur). For example, the rate may normally be 100Hz, but changed to 1000Hz in demanding situations, and to 1Hz in low power/standby situation. The probe signals may be sent in burst.

The probe signal rate may change based on a task performed by the Type 1 device or Type 2 device (e.g. a task may need 100 Hz normally and 1000 Hz momentarily for 20 seconds). In one example, the transmitters (Type 1 devices), receivers (Type 2 device), and associated tasks may be associated adaptively (and/or dynamically) to classes (e.g. classes that are: low-priority, high-priority, emergency, critical, regular, privileged, non-subscription, subscription, paying, and/or non-paying). A rate (of a transmitter) may be adjusted for the sake of some class (e.g. high priority class). When the need of that class changes, the rate may be changed (e.g. adjusted, varied, modified). When a receiver has critically low power, the rate may be reduced to reduce power consumption of the receiver to respond to the probe signals. In one example, probe signals may be used to transfer power wirelessly to a receiver (Type 2 device), and the rate may be adjusted to control the amount of power transferred to the receiver.

The rate may be changed by (or based on): a server (e.g. hub device), the Type 1 device and/or the Type 2 device. Control signals may be communicated between them. The server may monitor, track, forecast and/or anticipate the needs of the Type 2 device and/or the tasks performed by the Type 2 device, and may control the Type 1 device to change the rate. The server may make scheduled changes to the rate according to a time table. The server may detect an emergency situation and change the rate immediately. The server may detect a developing condition and adjust the rate gradually.

The characteristics and/or STI (e.g. motion information) may be monitored individually based on a TSCI associated with a particular Type 1 device and a particular Type 2 device, and/or monitored jointly based on any TSCI associated with the particular Type 1 device and any Type 2 device, and/or monitored jointly based on any TSCI associated with the particular Type 2 device and any Type 1 device, and/or monitored globally based on any TSCI associated with any Type 1 device and any Type 2 device. Any joint monitoring may be associated with: a user, user account, profile, household, map of venue, environmental model of the venue, and/or user history, etc.

A first channel between a Type 1 device and a Type 2 device may be different from a second channel between another Type 1 device and another Type 2 device. The two channels may be associated with different frequency bands, bandwidth, carrier frequency, modulation, wireless standards, coding, encryption, payload characteristics, networks, network ID, SSID, network characteristics, network settings, and/or network parameters, etc.

The two channels may be associated with different kinds of wireless system (e.g. two of the following: WiFi, LTE, LTE-A, LTE-U, 2.5G, 3G, 3.5G, 4G, beyond 4G, 5G, 6G, 7G, a cellular network standard, UMTS, 3GPP, GSM, EDGE, TDMA, FDMA, CDMA, WCDMA, TD-SCDMA, 802.11 system, 802.15 system, 802.16 system, mesh network, Zigbee, NFC, WiMax, Bluetooth, BLE, RFID, UWB, microwave system, radar like system). For example, one is WiFi and the other is LTE.

The two channels may be associated with similar kinds of wireless system, but in different network. For example, the first channel may be associated with a WiFi network named "Pizza and Pizza" in the 2.4GHz band with a bandwidth of 20MHz while the second may be associated with a WiFi network with SSID of "StarBud hotspot" in the 5GHz band with a bandwidth of 40MHz. The two channels may be different channels in same network (e.g. the "StarBud hotspot" network).

In one embodiment, a wireless monitoring system may comprise training a classifier of multiple events in a venue based on training TSCI associated with the multiple events. A CI or TSCI associated with an event may be considered/may comprise a wireless sample/characteristics/fingerprint associated with the event (and/or the venue, the environment, the object, the motion of the object, a state/ emotional state/ mental state/ condition/ stage/ gesture/ gait/ action/ movement/ activity/ daily activity/ history/ event of the object, etc.).

For each of the multiple known events happening in the venue in a respective training (e.g. surveying, wireless survey, initial wireless survey) time period associated with the known event, a respective training wireless signal (e.g. a respective series of training probe signals) may be transmitted by an antenna of a first Type 1 heterogeneous wireless device using a processor, a memory and a set of instructions of the first Type 1 device to at least one first Type 2 heterogeneous wireless device through a wireless multipath channel in the venue in the respective training time period.

At least one respective time series of training CI (training TSCI) may be obtained asynchronously by each of the at least one first Type 2 device from the (respective) training signal. The CI may be CI of the channel between the first Type 2 device and the first Type 1 device in the training time period associated with the known event. The at least one training TSCI may be preprocessed. The training may be a wireless survey (e.g. during installation of Type 1 device and/or Type 2 device).

For a current event happening in the venue in a current time period, a current wireless signal (e.g. a series of current probe signals) may be transmitted by an antenna of a second Type 1 heterogeneous wireless device using a processor, a memory and a set of instructions of the second Type 1 device to at least one second Type 2 heterogeneous wireless device through the channel in the venue in the current time period associated with the current event.

At least one time series of current CI (current TSCI) may be obtained asynchronously by each of the at least one second Type 2 device from the current signal (e.g. the series of current probe signals). The CI may be CI of the channel between the second Type 2 device and the second Type 1 device in the current time period associated with the current event. The at least one current TSCI may be preprocessed.

The classifier may be applied to classify at least one current TSCI obtained from the series of current probe signals by the at least one second Type 2 device, to classify at least one portion of a particular current TSCI, and/or to classify a combination of the at least one portion of the particular current TSCI and another portion of another TSCI. The classifier may partition TSCI (or the characteristics/STI or other analytics or output responses) into clusters and associate the clusters to specific events/ objects/ subjects/ locations/ movements/ activities. Labels/tags may be generated for the clusters. The clusters may be stored and retrieved. The classifier may be applied to associate the current TSCI (or characteristics/STI or the other analytics/output response, perhaps associated with a current event) with: a cluster, a known/specific event, a class/ category/ group/ grouping/ list/ cluster/ set of known events/subjects/locations/movements/activities, an unknown event, a class/ category/ group/ grouping/ list/ cluster/ set of unknown events/subjects/locations/movements/activities, and/or another event/subject/location/movement/activity/ class/ category/ group/ grouping/ list/ cluster/ set. Each TSCI may comprise at least one CI each associated with a respective timestamp. Two TSCI associated with two Type 2 devices may be different with different: starting time, duration, stopping time, amount of CI, sampling frequency, sampling period. Their CI may have different features. The first and second Type 1 devices may be at same location in the venue. They may be the same device. The at least one second Type 2 device (or their locations) may be a permutation of the at least one first Type 2 device (or their locations). A particular second Type 2 device and a particular first Type 2 device may be the same device.

A subset of the first Type 2 device and a subset of the second Type 2 device may be the same. The at least one second Type 2 device and/or a subset of the at least one second Type 2 device may be a subset of the at least one first Type 2 device. The at least one first Type 2 device and/or a subset of the at least one first Type 2 device may be a permutation of a subset of the at least one second Type 2 device. The at least one second Type 2 device and/or a subset of the at least one second Type 2 device may be a permutation of a subset of the at least one first Type 2 device. The at least one second Type 2 device and/or a subset of the at least one second Type 2 device may be at same respective location as a subset of the at least one first Type 2 device. The at least one first Type 2 device and/or a subset of the at least one first Type 2 device may be at same respective location as a subset of the at least one second Type 2 device.

The antenna of the Type 1 device and the antenna of the second Type 1 device may be at same location in the venue. Antenna(s) of the at least one second Type 2 device and/or antenna(s) of a subset of the at least one second Type 2 device may be at same respective location as respective antenna(s) of a subset of the at least one first Type 2 device. Antenna(s) of the at least one first Type 2 device and/or antenna(s) of a subset of the at least one first Type 2 device may be at same respective location(s) as respective antenna(s) of a subset of the at least one second Type 2 device.

A first section of a first time duration of the first TSCI and a second section of a second time duration of the second section of the second TSCI may be aligned. A map between items of the first section and items of the second section may be computed. The first section may comprise a first segment (e.g. subset) of the first TSCI with a first starting /ending time, and/or another segment (e.g. subset) of a processed first TSCI. The processed first TSCI may be the first TSCI processed by a first operation. The second section may comprise a second segment (e.g. subset) of the second TSCI with a second starting time and a second ending time, and another segment (e.g. subset) of a processed second TSCI. The processed second TSCI may be the second TSCI processed by a second operation. The first operation and/or the second operation may comprise: subsampling, resampling, interpolation, filtering, transformation, feature extraction, pre-processing, and/or another operation.

A first item of the first section may be mapped to a second item of the second section. The first item of the first section may also be mapped to another item of the second section. Another item of the first section may also be mapped to the second item of the second section. The mapping may be one-to-one, one-to-many, many-to-one, many-to-many. At least one function of at least one of: the first item of the first section of the first TSCI, another item of the first TSCI, timestamp of the first item, time difference of the first item, time differential of the first item, neighboring timestamp of the first item, another timestamp associated with the first item, the second item of the second section of the second TSCI, another item of the second TSCI, timestamp of the second item, time difference of the second item, time differential of the second item, neighboring timestamp of the second item, and another timestamp associated with the second item, may satisfy at least one constraint.

One constraint may be that a difference between the timestamp of the first item and the timestamp of the second item may be upper-bounded by an adaptive (and/or dynamically adjusted) upper threshold and lower-bounded by an adaptive lower threshold.

The first section may be the entire first TSCI. The second section may be the entire second TSCI. The first time duration may be equal to the second time duration. A section of a time duration of a TSCI may be determined adaptively (and/or dynamically). A tentative section of the TSCI may be computed. A starting time and an ending time of a section (e.g. the tentative section, the section) may be determined. The section may be determined by removing a beginning portion and an ending portion of the tentative section. A beginning portion of a tentative section may be determined as follows. Iteratively, items of the tentative section with increasing timestamp may be considered as a current item, one item at a time.

In each iteration, at least one activity measure/index may be computed and/or considered. The at least one activity measure may be associated with at least one of: the current item associated with a current timestamp, past items of the tentative section with timestamps not larger than the current timestamp, and/or future items of the tentative section with timestamps not smaller than the current timestamp. The current item may be added to the beginning portion of the tentative section if at least one criterion (e.g. quality criterion, signal quality condition) associated with the at least one activity measure is satisfied.

The at least one criterion associated with the activity measure may comprise at least one of: (a) the activity measure is smaller than an adaptive (e.g. dynamically adjusted) upper threshold, (b) the activity measure is larger than an adaptive lower threshold, (c) the activity measure is smaller than an adaptive upper threshold consecutively for at least a predetermined amount of consecutive timestamps, (d) the activity measure is larger than an adaptive lower threshold consecutively for at least another predetermined amount of consecutive timestamps, (e) the activity measure is smaller than an adaptive upper threshold consecutively for at least a predetermined percentage of the predetermined amount of consecutive timestamps, (f) the activity measure is larger than an adaptive lower threshold consecutively for at least another predetermined percentage of the another predetermined amount of consecutive timestamps, (g) another activity measure associated with another timestamp associated with the current timestamp is smaller than another adaptive upper threshold and larger than another adaptive lower threshold, (h) at least one activity measure associated with at least one respective timestamp associated with the current timestamp is smaller than respective upper threshold and larger than respective lower threshold, (i) percentage of timestamps with associated activity measure smaller than respective upper threshold and larger than respective lower threshold in a set of timestamps associated with the current timestamp exceeds a threshold, and (j) another criterion (e.g. a quality criterion, signal quality condition).

An activity measure/index associated with an item at time T1 may comprise at least one of: (1) a first function of the item at time T1 and an item at time T1-D1, wherein D1 is a pre-determined positive quantity (e.g. a constant time offset), (2) a second function of the item at time T1 and an item at time T1+D1, (3) a third function of the item at time T1 and an item at time T2, wherein T2 is a pre-determined quantity (e.g. a fixed initial reference time; T2 may be changed (e.g. adjusted, varied, modified) over time; T2 may be updated periodically; T2 may be the beginning of a time period and T1 may be a sliding time in the time period), and (4) a fourth function of the item at time T1 and another item.

At least one of: the first function, the second function, the third function, and/or the fourth function may be a function (e.g. F(X, Y, ...)) with at least two arguments: X and Y. The two arguments may be scalars. The function (e.g. F) may be a function of at least one of: X, Y, (X-Y), (Y-X), abs(X-Y), X^a, Y^b, abs(X^a - Y^b), (X-Y)^a, (X/Y), (X+a)/(Y+b), (X^a/Y^b), and ((X/Y)^a-b), wherein a and b are may be some predetermined quantities. For example, the function may simply be abs(X-Y), or (X-Y)^2, (X-Y)^4. The function may be a robust function. For example, the function may be (X-Y)^2 when abs (X-Y) is less than a threshold T, and (X-Y)+a when abs(X-Y) is larger than T. Alternatively, the function may be a constant when abs(X-Y) is larger than T. The function may also be bounded by a slowly increasing function when abs(X-y) is larger than T, so that outliers cannot severely affect the result. Another example of the function may be (abs(X/Y)-a), where a=1. In this way, if X=Y (i.e. no change or no activity), the function will give a value of 0. If X is larger than Y, (X/Y) will be larger than 1 (assuming X and Y are positive) and the function will be positive. And if X is less than Y, (X/Y) will be smaller than 1 and the function will be negative. In another example, both arguments X and Y may be n-tuples such that X=(x_1, x_2, ..., x_n) and Y=(y_1, y_2, ..., y_n). The function may be a function of at least one of: x_i, y_i, (x_i - y_i), (y_i- x_i), abs(x_i - y_i), x_i ^a, y_i ^b, abs(x_i ^ay_i ^b), (x_i - y_i)^a, (x_i/y_i), (x_i+a)/(y_i +b), (x_i ^a/ y_i ^b), and ((x_i / y_i)^a-b), wherein i is a component index of the n-tuple X and Y, and 1<=i<=n, e.g. component index of x_1 is i=1, component index of x_2 is i=2. The function may comprise a component-by-component summation of another function of at least one of the following: x_i, y_i, (x_i - y_i), (y_i- x_i), abs(x_i - y_i), x_i ^a, y_i ^b, abs(x_i ^a - y_i ^b), (x_i - y_i)^a, (x_i/y_i), (x_i+a)/(y_i +b), (x_i ^a/ y_i ^b), and ((x_i / y_i)^a-b) , wherein i is the component index of the n-tuple X and Y. For example, the function may be in a form of sum_{i=1 }^n (abs(x_i/y_i)-1)/n, or sum_{i=1 }^n w_i*(abs(x_i/y_i)-1), where w_i is some weight for component i.

The map may be computed using dynamic time warping (DTW). The DTW may comprise a constraint on at least one of: the map, the items of the first TSCI, the items of the second TSCI, the first time duration, the second time duration, the first section, and/or the second section. Suppose in the map, the i^{th} domain item is mapped to the j^{th} range item. The constraint may be on admissible combination of i and j (constraint on relationship between i and j). Mismatch cost between a first section of a first time duration of a first TSCI and a second section of a second time duration of a second TSCI may be computed.

The first section and the second section may be aligned such that a map comprising more than one links may be established between first items of the first TSCI and second items of the second TSCI. With each link, one of the first items with a first timestamp may be associated with one of the second items with a second timestamp. A mismatch cost between the aligned first section and the aligned second section may be computed. The mismatch cost may comprise a function of: an item-wise cost between a first item and a second item associated by a particular link of the map, and a link-wise cost associated with the particular link of the map.

The aligned first section and the aligned second section may be represented respectively as a first vector and a second vector of same vector length. The mismatch cost may comprise at least one of: an inner product, inner-product-like quantity, quantity based on correlation, correlation indicator, quantity based on covariance, discriminating score, distance, Euclidean distance, absolute distance, Lk distance (e.g. LI, L2, ... ), weighted distance, distance-like quantity and/or another similarity value, between the first vector and the second vector. The mismatch cost may be normalized by the respective vector length.

A parameter derived from the mismatch cost between the first section of the first time duration of the first TSCI and the second section of the second time duration of the second TSCI may be modeled with a statistical distribution. At least one of: a scale parameter, location parameter and/or another parameter, of the statistical distribution may be estimated.

The first section of the first time duration of the first TSCI may be a sliding section of the first TSCI. The second section of the second time duration of the second TSCI may be a sliding section of the second TSCI.

A first sliding window may be applied to the first TSCI and a corresponding second sliding window may be applied to the second TSCI. The first sliding window of the first TSCI and the corresponding second sliding window of the second TSCI may be aligned.

Mismatch cost between the aligned first sliding window of the first TSCI and the corresponding aligned second sliding window of the second TSCI may be computed. The current event may be associated with at least one of: the known event, the unknown event and/or the another event, based on the mismatch cost.

The classifier may be applied to at least one of: each first section of the first time duration of the first TSCI, and/or each second section of the second time duration of the second TSCI, to obtain at least one tentative classification results. Each tentative classification result may be associated with a respective first section and a respective second section.

The current event may be associated with at least one of: the known event, the unknown event, a class/category/ group/ grouping/list/ set of unknown events, and/or the another event, based on the mismatch cost. The current event may be associated with at least one of: the known event, the unknown event and/or the another event, based on a largest number of tentative classification results in more than one sections of the first TSCI and corresponding more than sections of the second TSCI. For example, the current event may be associated with a particular known event if the mismatch cost points to the particular known event for N consecutive times (e.g. N=10). In another example, the current event may be associated with a particular known event if the percentage of mismatch cost within the immediate past N consecutive N pointing to the particular known event exceeds a certain threshold (e.g. >80%).

In another example, the current event may be associated with a known event that achieves smallest mismatch cost for the most times within a time period. The current event may be associated with a known event that achieves smallest overall mismatch cost, which is a weighted average of at least one mismatch cost associated with the at least one first sections. The current event may be associated with a particular known event that achieves smallest of another overall cost. The current event may be associated with the "unknown event" if none of the known events achieve mismatch cost lower than a first threshold T1 in a sufficient percentage of the at least one first section. The current event may also be associated with the "unknown event" if none of the events achieve an overall mismatch cost lower than a second threshold T2. The current event may be associated with at least one of: the known event, the unknown event and/or the another event, based on the mismatch cost and additional mismatch cost associated with at least one additional section of the first TSCI and at least one additional section of the second TSCI. The known events may comprise at least one of: a door closed event, door open event, window closed event, window open event, multi-state event, on-state event, off-state event, intermediate state event, continuous state event, discrete state event, human-present event, human-absent event, sign-of-life-present event, and/or a sign-of-life-absent event.

A projection for each CI may be trained using a dimension reduction method based on the training TSCI. The dimension reduction method may comprise at least one of: principal component analysis (PCA), PCA with different kernel, independent component analysis (ICA), Fisher linear discriminant, vector quantization, supervised learning, unsupervised learning, self-organizing maps, auto-encoder, neural network, deep neural network, and/or another method. The projection may be applied to at least one of: the training TSCI associated with the at least one event, and/or the current TSCI, for the classifier.

The classifier of the at least one event may be trained based on the projection and the training TSCI associated with the at least one event. The at least one current TSCI may be classified/categorized based on the projection and the current TSCI. The projection may be re-trained using at least one of: the dimension reduction method, and another dimension reduction method, based on at least one of: the training TSCI, at least one current TSCI before retraining the projection, and/or additional training TSCI. The another dimension reduction method may comprise at least one of: principal component analysis (PCA), PCA with different kernels, independent component analysis (ICA), Fisher linear discriminant, vector quantization, supervised learning, unsupervised learning, self-organizing maps, auto-encoder, neural network, deep neural network, and/or yet another method. The classifier of the at least one event may be re-trained based on at least one of: the re-trained projection, the training TSCI associated with the at least one events, and/or at least one current TSCI. The at least one current TSCI may be classified based on: the re-trained projection, the re-trained classifier, and/or the current TSCI.

Each CI may comprise a vector of complex values. Each complex value may be preprocessed to give the magnitude of the complex value. Each CI may be preprocessed to give a vector of non-negative real numbers comprising the magnitude of corresponding complex values. Each training TSCI may be weighted in the training of the projection. The projection may comprise more than one projected components. The projection may comprise at least one most significant projected component. The projection may comprise at least one projected component that may be beneficial for the classifier.

Channel/channel information/venue/spatial-temporal info/motion/object

The channel information (CI) may be associated with/may comprise signal strength, signal amplitude, signal phase, spectral power measurement, modem parameters (e.g. used in relation to modulation/ demodulation in digital communication systems such as WiFi, 4G/LTE), dynamic beamforming information (including feedback or steering matrices generated by wireless communication devices, according to a standardized process, e.g., IEEE 802.11, or another standard), transfer function components, radio state (e.g. used in digital communication systems to decode digital data, baseband processing state, RF processing state, etc.), measurable variables, sensed data, coarse-grained/ fine-grained information of a layer (e.g. physical layer, data link layer, MAC layer, etc.), digital setting, gain setting, RF filter setting, RF front end switch setting, DC offset setting, DC correction setting, IQ compensation setting, effect(s) on the wireless signal by the environment (e.g. venue) during propagation, transformation of an input signal (the wireless signal transmitted by the Type 1 device) to an output signal (the wireless signal received by the Type 2 device), a stable behavior of the environment, a state profile, wireless channel measurements, received signal strength indicator (RSSI), channel state information (CSI), channel impulse response (CIR), channel frequency response (CFR), characteristics of frequency components (e.g. subcarriers) in a bandwidth, channel characteristics, channel filter response, timestamp, auxiliary information, data, meta data, user data, account data, access data, security data, session data, status data, supervisory data, household data, identity (ID), identifier, device data, network data, neighborhood data, environment data, real-time data, sensor data, stored data, encrypted data, compressed data, protected data, and/or another channel information. Each CI may be associated with a time stamp, and/or an arrival time. A CSI can be used to equalize/undo/ minimize/reduce the multipath channel effect (of the transmission channel) to demodulate a signal similar to the one transmitted by the transmitter through the multipath channel. The CI may be associated with information associated with a frequency band, frequency signature, frequency phase, frequency amplitude, frequency trend, frequency characteristics, frequency-like characteristics, time domain element, frequency domain element, time-frequency domain element, orthogonal decomposition characteristics, and/or non-orthogonal decomposition characteristics of the signal through the channel. The TSCI may be a stream of wireless signals (e.g. CI).

The CI may be preprocessed, processed, postprocessed, stored (e.g. in local memory, portable/mobile memory, removable memory, storage network, cloud memory, in a volatile manner, in a non-volatile manner), retrieved, transmitted and/or received. One or more modem parameters and/or radio state parameters may be held constant. The modem parameters may be applied to a radio subsystem. The modem parameters may represent a radio state. A motion detection signal (e.g. baseband signal, and/or packet decoded/demodulated from the baseband signal, etc.) may be obtained by processing (e.g. down-converting) the first wireless signal (e.g. RF/WiFi/LTE/5G signal) by the radio subsystem using the radio state represented by the stored modem parameters. The modem parameters/radio state may be updated (e.g. using previous modem parameters or previous radio state). Both the previous and updated modem parameters/radio states may be applied in the radio subsystem in the digital communication system. Both the previous and updated modem parameters/radio states may be compared/ analyzed/ processed/ monitored in the task.

The channel information may also be modem parameters (e.g. stored or freshly computed) used to process the wireless signal. The wireless signal may comprise a plurality of probe signals. The same modem parameters may be used to process more than one probe signals. The same modem parameters may also be used to process more than one wireless signals. The modem parameters may comprise parameters that indicate settings or an overall configuration for the operation of a radio subsystem or a baseband subsystem of a wireless sensor device (or both). The modem parameters may include one or more of: a gain setting, an RF filter setting, an RF front end switch setting, a DC offset setting, or an IQ compensation setting for a radio subsystem, or a digital DC correction setting, a digital gain setting, and/or a digital filtering setting (e.g. for a baseband subsystem). The CI may also be associated with information associated with a time period, time signature, timestamp, time amplitude, time phase, time trend, and/or time characteristics of the signal. The CI may be associated with information associated with a time-frequency partition, signature, amplitude, phase, trend, and/or characteristics of the signal. The CI may be associated with a decomposition of the signal. The CI may be associated with information associated with a direction, angle of arrival (AoA), angle of a directional antenna, and/or a phase of the signal through the channel. The CI may be associated with attenuation patterns of the signal through the channel. Each CI may be associated with a Type 1 device and a Type 2 device. Each CI may be associated with an antenna of the Type 1 device and an antenna of the Type 2 device.

The CI may be obtained from a communication hardware (e.g. of Type 2 device, or Type 1 device) that is capable of providing the CI. The communication hardware may be a WiFi-capable chip/IC (integrated circuit), chip compliant with a 802.11 or 802.16 or another wireless/radio standard, next generation WiFi-capable chip, LTE-capable chip, 5G-capable chip, 6G/7G/8G-capable chip, Bluetooth-enabled chip, NFC (near field communication)-enabled chip, BLE (Bluetooth low power)-enabled chip, UWB chip, another communication chip (e.g. Zigbee, WiMax, mesh network), etc. The communication hardware computes the CI and stores the CI in a buffer memory and make the CI available for extraction. The CI may comprise data and/or at least one matrices related to channel state information (CSI). The at least one matrices may be used for channel equalization, and/or beam forming, etc. The channel may be associated with a venue. The attenuation may be due to signal propagation in the venue, signal propagating/reflection/ refraction/ diffraction through/at/around air (e.g. air of venue), refraction medium/reflection surface such as wall, doors, furniture, obstacles and/or barriers, etc. The attenuation may be due to reflection at surfaces and obstacles (e.g. reflection surface, obstacle) such as floor, ceiling, furniture, fixtures, objects, people, pets, etc. Each CI may be associated with a timestamp. Each CI may comprise N1 components (e.g. N1 frequency domain components in CFR, N1 time domain components in CIR, or N1 decomposition components). Each component may be associated with a component index. Each component may be a real, imaginary, or complex quantity, magnitude, phase, flag, and/or set. Each CI may comprise a vector or matrix of complex numbers, a set of mixed quantities, and/or a multi-dimensional collection of at least one complex numbers.

Components of a TSCI associated with a particular component index may form a respective component time series associated with the respective index. A TSCI may be divided into N1 component time series. Each respective component time series is associated with a respective component index. The characteristics/STI of the motion of the object may be monitored based on the component time series. In one example, one or more ranges of CI components (e.g. one range being from component 11 to component 23, a second range being from component 44 to component 50, and a third range having only one component) may be selected based on some criteria/cost function/signal quality metric (e.g. based on signal-to-noise ratio, and/or interference level) for further processing.

A component-wise characteristic of a component-feature time series of a TSCI may be computed. The component-wise characteristics may be a scalar (e.g. energy) or a function with a domain and a range (e.g. an autocorrelation function, transform, inverse transform). The characteristics/STI of the motion of the object may be monitored based on the component-wise characteristics. A total characteristics (e.g. aggregate characteristics) of the TSCI may be computed based on the component-wise characteristics of each component time series of the TSCI. The total characteristics may be a weighted average of the component-wise characteristics. The characteristics/STI of the motion of the object may be monitored based on the total characteristics. An aggregate quantity may be a weighted average of individual quantities.

The Type 1 device and Type 2 device may support WiFi, WiMax, 3G/beyond 3G, 4G/beyond 4G, LTE, LTE-A, 5G, 6G, 7G, Bluetooth, NFC, BLE, Zigbee, UWB, UMTS, 3GPP, GSM, EDGE, TDMA, FDMA, CDMA, WCDMA, TD-SCDMA, mesh network, proprietary wireless system, IEEE 802.11 standard, 802.15 standard, 802.16 standard, 3GPP standard, and/or another wireless system.

A common wireless system and/or a common wireless channel may be shared by the Type 1 transceiver and/or the at least one Type 2 transceiver. The at least one Type 2 transceiver may transmit respective signal contemporaneously (or: asynchronously, synchronously, sporadically, continuously, repeatedly, concurrently, simultaneously and/or temporarily) using the common wireless system and/or the common wireless channel. The Type 1 transceiver may transmit a signal to the at least one Type 2 transceiver using the common wireless system and/or the common wireless channel.

Each Type 1 device and Type 2 device may have at least one transmitting/receiving antenna. Each CI may be associated with one of the transmitting antenna of the Type 1 device and one of the receiving antenna of the Type 2 device. Each pair of a transmitting antenna and a receiving antenna may be associated with a link, a path, a communication path, signal hardware path, etc. For example, if the Type 1 device has M (e.g. 3) transmitting antennas, and the Type 2 device has N (e.g. 2) receiving antennas, there may be MxN (e.g. 3x2=6) links or paths. Each link or path may be associated with a TSCI.

The at least one TSCI may correspond to various antenna pairs between the Type 1 device and the Type 2 device. The Type 1 device may have at least one antenna. The Type 2 device may also have at least one antenna. Each TSCI may be associated with an antenna of the Type 1 device and an antenna of the Type 2 device. Averaging or weighted averaging over antenna links may be performed. The averaging or weighted averaging may be over the at least one TSCI. The averaging may optionally be performed on a subset of the at least one TSCI corresponding to a subset of the antenna pairs.

Timestamps of CI of a portion of a TSCI may be irregular and may be corrected so that corrected timestamps of time-corrected CI may be uniformly spaced in time. In the case of multiple Type 1 devices and/or multiple Type 2 devices, the corrected timestamp may be with respect to the same or different clock. An original timestamp associated with each of the CI may be determined. The original timestamp may not be uniformly spaced in time. Original timestamps of all CI of the particular portion of the particular TSCI in the current sliding time window may be corrected so that corrected timestamps of time-corrected CI may be uniformly spaced in time.

The characteristics and/or STI (e.g. motion information) may comprise: location, location coordinate, change in location, position (e.g. initial position, new position), position on map, height, horizontal location, vertical location, distance, displacement, speed, acceleration, rotational speed, rotational acceleration, direction, angle of motion, azimuth, direction of motion, rotation, path, deformation, transformation, shrinking, expanding, gait, gait cycle, head motion, repeated motion, periodic motion, pseudo-periodic motion, impulsive motion, sudden motion, fall-down motion, transient motion, behavior, transient behavior, period of motion, frequency of motion, time trend, temporal profile, temporal characteristics, occurrence, change, temporal change, change of CI, change in frequency, change in timing, change of gait cycle, timing, starting time, initiating time, ending time, duration, history of motion, motion type, motion classification, frequency, frequency spectrum, frequency characteristics, presence, absence, proximity, approaching, receding, identity/identifier of the object, composition of the object, head motion rate, head motion direction, mouth-related rate, eye-related rate, breathing rate, heart rate, tidal volume, depth of breath, inhale time, exhale time, inhale time to exhale time ratio, airflow rate, heart heat-to-beat interval, heart rate variability, hand motion rate, hand motion direction, leg motion, body motion, walking rate, hand motion rate, positional characteristics, characteristics associated with movement (e.g. change in position/location) of the object, tool motion, machine motion, complex motion, and/or combination of multiple motions, event, signal statistics, signal dynamics, anomaly, motion statistics, motion parameter, indication of motion detection, motion magnitude, motion phase, similarity score, distance score, Euclidean distance, weighted distance, L_1 norm, L_2 norm, L_k norm for k>2, statistical distance, correlation, correlation indicator, auto-correlation, covariance, auto-covariance, cross-covariance, inner product, outer product, motion signal transformation, motion feature, presence of motion, absence of motion, motion localization, motion identification, motion recognition, presence of object, absence of object, entrance of object, exit of object, a change of object, motion cycle, motion count, gait cycle, motion rhythm, deformation motion, gesture, handwriting, head motion, mouth motion, heart motion ,internal organ motion, motion trend, size, length, area, volume, capacity, shape, form, tag, starting/initiating location, ending location, starting/initiating quantity, ending quantity, event, fall-down event, security event, accident event, home event, office event, factory event, warehouse event, manufacturing event, assembly line event, maintenance event, car-related event, navigation event, tracking event, door event, door-open event, door-close event, window event, window-open event, window-close event, repeatable event, one-time event, consumed quantity, unconsumed quantity, state, physical state, health state, well-being state, emotional state, mental state, another event, analytics, output responses, and/or another information. The characteristics and/or STI may be computed/monitored based on a feature computed from a CI or a TSCI (e.g. feature computation/extraction). A static segment or profile (and/or a dynamic segment/profile) may be identified /computed/analyzed/ monitored/ extracted/ obtained/ marked/ presented/ indicated/ highlighted/ stored/ communicated based on an analysis of the feature. The analysis may comprise a motion detection/movement assessment/presence detection. Computational workload may be shared among the Type 1 device, the Type 2 device and another processor.

The Type 1 device and/or Type 2 device may be a local device. The local device may be: a smart phone, smart device, TV, sound bar, set-top box, access point, router, repeater, wireless signal repeater/extender, remote control, speaker, fan, refrigerator, microwave, oven, coffee machine, hot water pot, utensil, table, chair, light, lamp, door lock, camera, microphone, motion sensor, security device, fire hydrant, garage door, switch, power adapter, computer, dongle, computer peripheral, electronic pad, sofa, tile, accessory, home device, vehicle device, office device, building device, manufacturing device, watch, glasses, clock, television, oven, air-conditioner, accessory, utility, appliance, smart machine, smart vehicle, internet-of-thing (IoT) device, internet-enabled device, computer, portable computer, tablet, smart house, smart office, smart building, smart parking lot, smart system, and/or another device.

Each Type 1 device may be associated with a respective identifier (e.g. ID). Each Type 2 device may also be associated with a respective identify (ID). The ID may comprise: numeral, combination of text and numbers, name, password, account, account ID, web link, web address, index to some information, and/or another ID. The ID may be assigned. The ID may be assigned by hardware (e.g. hardwired, via dongle and/or other hardware), software and/or firmware. The ID may be stored (e.g. in database, in memory, in server (e.g. hub device), in the cloud, stored locally, stored remotely, stored permanently, stored temporarily) and may be retrieved. The ID may be associated with at least one record, account, user, household, address, phone number, social security number, customer number, another ID, another identifier, timestamp, and/or collection of data. The ID and/or part of the ID of a Type 1 device may be made available to a Type 2 device. The ID may be used for registration, initialization, communication, identification, verification, detection, recognition, authentication, access control, cloud access, networking, social networking, logging, recording, cataloging, classification, tagging, association, pairing, transaction, electronic transaction, and/or intellectual property control, by the Type 1 device and/or the Type 2 device.

The object may be person, user, subject, passenger, child, older person, baby, sleeping baby, baby in vehicle, patient, worker, high-value worker, expert, specialist, waiter, customer in mall, traveler in airport/ train station/ bus terminal/ shipping terminals, staff/worker/customer service personnel in factory/ mall/ supermarket/ office/ workplace, serviceman in sewage/air ventilation system/lift well, lifts in lift wells, elevator, inmate, people to be tracked/ monitored, animal, plant, living object, pet, dog, cat, smart phone, phone accessory, computer, tablet, portable computer, dongle, computing accessory, networked devices, WiFi devices, IoT devices, smart watch, smart glasses, smart devices, speaker, keys, smart key, wallet, purse, handbag, backpack, goods, cargo, luggage, equipment, motor, machine, air conditioner, fan, air conditioning equipment, light fixture, moveable light, television, camera, audio and/or video equipment, stationary, surveillance equipment, parts, signage, tool, cart, ticket, parking ticket, toll ticket, airplane ticket, credit card, plastic card, access card, food packaging, utensil, table, chair, cleaning equipment/tool, vehicle, car, cars in parking facilities, merchandise in warehouse/ store/ supermarket/ distribution center, boat, bicycle, airplane, drone, remote control car/plane/ boat, robot, manufacturing device, assembly line, material/unfinished part/robot/wagon/ transports on factory floor, object to be tracked in airport/shopping mart/supermarket, non-object, absence of an object, presence of an object, object with form, object with changing form, object with no form, mass of fluid, mass of liquid, mass of gas/smoke, fire, flame, electromagnetic (EM) source, EM medium, and/or another object.

The object itself may be communicatively coupled with some network, such as WiFi, MiFi, 3G/ 4G/ LTE/ 5G/ 6G/ 7G, Bluetooth, NFC, BLE, WiMax, Zigbee, UMTS, 3GPP, GSM, EDGE, TDMA, FDMA, CDMA, WCDMA, TD-SCDMA, mesh network, adhoc network, and/or other network. The object itself may be bulky with AC power supply, but is moved during installation, cleaning, maintenance, renovation, etc. It may also be installed in moveable platform such as lift, pad, movable, platform, elevator, conveyor belt, robot, drone, forklift, car, boat, vehicle, etc. The object may have multiple parts, each part with different movement (e.g. change in position/location). For example, the object may be a person walking forward. While walking, his left hand and right hand may move in different direction, with different instantaneous speed, acceleration, motion, etc.

The wireless transmitter (e.g. Type 1 device), the wireless receiver (e.g. Type 2 device), another wireless transmitter and/or another wireless receiver may move with the object and/or another object (e.g. in prior movement, current movement and/or future movement. They may be communicatively coupled to one or more nearby device. They may transmit TSCI and/or information associated with the TSCI to the nearby device, and/or each other. They may be with the nearby device. The wireless transmitter and/or the wireless receiver may be part of a small (e.g. coin-size, cigarette box size, or even smaller), light-weight portable device. The portable device may be wirelessly coupled with a nearby device.

The nearby device may be smart phone, iPhone, Android phone, smart device, smart appliance, smart vehicle, smart gadget, smart TV, smart refrigerator, smart speaker, smart watch, smart glasses, smart pad, iPad, computer, wearable computer, notebook computer, gateway. The nearby device may be connected to a cloud server, local server (e.g. hub device) and/or other server via internet, wired internet connection and/or wireless internet connection. The nearby device may be portable. The portable device, the nearby device, a local server (e.g. hub device) and/or a cloud server may share the computation and/or storage for a task (e.g. obtain TSCI, determine characteristics/STI of the object associated with the movement (e.g. change in position/location) of the object, computation of time series of power (e.g. signal strength) information, determining/computing the particular function, searching for local extremum, classification, identifying particular value of time offset, de-noising, processing, simplification, cleaning, wireless smart sensing task, extract CI from signal, switching, segmentation, estimate trajectory/path/track, process the map, processing trajectory/path/track based on environment models/constraints/limitations, correction, corrective adjustment, adjustment, map-based (or model-based) correction, detecting error, checking for boundary hitting, thresholding) and information (e.g. TSCI). The nearby device may/ may not move with the object. The nearby device may be portable/ not portable/ moveable/ non-moveable. The nearby device may use battery power, solar power, AC power and/or other power source. The nearby device may have replaceable/non-replaceable battery, and/or rechargeable/non-rechargeable battery. The nearby device may be similar to the object. The nearby device may have identical (and/or similar) hardware and/or software to the object. The nearby device may be a smart device, network enabled device, device with connection to WiFi/ 3G/ 4G/ 5G/ 6G/ Zigbee/ Bluetooth/ NFC/ UMTS/ 3GPP/ GSM/ EDGE/ TDMA/ FDMA/ CDMA/ WCDMA/ TD-SCDMA/ adhoc network/ other network, smart speaker, smart watch, smart clock, smart appliance, smart machine, smart equipment, smart tool, smart vehicle, internet-of-thing (IoT) device, internet-enabled device, computer, portable computer, tablet, and another device. The nearby device and/or at least one processor associated with the wireless receiver, the wireless transmitter, the another wireless receiver, the another wireless transmitter and/or a cloud server (in the cloud) may determine the initial STI of the object. Two or more of them may determine the initial spatial-temporal info jointly. Two or more of them may share intermediate information in the determination of the initial STI (e.g. initial position).

In one example, the wireless transmitter (e.g. Type 1 device, or Tracker Bot) may move with the object. The wireless transmitter may send the signal to the wireless receiver (e.g. Type 2 device, or Origin Register) or determining the initial STI (e.g. initial position) of the object. The wireless transmitter may also send the signal and/or another signal to another wireless receiver (e.g. another Type 2 device, or another Origin Register) for the monitoring of the motion (spatial-temporal info) of the object. The wireless receiver may also receive the signal and/or another signal from the wireless transmitter and/or the another wireless transmitter for monitoring the motion of the object. The location of the wireless receiver and/or the another wireless receiver may be known. In another example, the wireless receiver (e.g. Type 2 device, or Tracker Bot) may move with the object. The wireless receiver may receive the signal transmitted from the wireless transmitter (e.g. Type 1 device, or Origin Register) for determining the initial spatial-temporal info (e.g. initial position) of the object. The wireless receiver may also receive the signal and/or another signal from another wireless transmitter (e.g. another Type 1 device, or another Origin Register) for the monitoring of the current motion (e.g. spatial-temporal info) of the object. The wireless transmitter may also transmit the signal and/or another signal to the wireless receiver and/or the another wireless receiver (e.g. another Type 2 device, or another Tracker Bot) for monitoring the motion of the object. The location of the wireless transmitter and/or the another wireless transmitter may be known.

The venue may be a space such as a sensing area, room, house, office, property, workplace, hallway, walkway, lift, lift well, escalator, elevator, sewage system, air ventilations system, staircase, gathering area, duct, air duct, pipe, tube, enclosed space, enclosed structure, semi-enclosed structure, enclosed area, area with at least one wall, plant, machine, engine, structure with wood, structure with glass, structure with metal, structure with walls, structure with doors, structure with gaps, structure with reflection surface, structure with fluid, building, roof top, store, factory, assembly line, hotel room, museum, classroom, school, university, government building, warehouse, garage, mall, airport, train station, bus terminal, hub, transportation hub, shipping terminal, government facility, public facility, school, university, entertainment facility, recreational facility, hospital, pediatric/neonatal wards, seniors home, elderly care facility, geriatric facility, community center, stadium, playground, park, field, sports facility, swimming facility, track and/or field, basketball court, tennis court, soccer stadium, baseball stadium, gymnasium, hall, garage, shopping mart, mall, supermarket, manufacturing facility, parking facility, construction site, mining facility, transportation facility, highway, road, valley, forest, wood, terrain, landscape, den, patio, land, path, amusement park, urban area, rural area, suburban area, metropolitan area, garden, square, plaza, music hall, downtown facility, over-air facility, semi-open facility, closed area, train platform, train station, distribution center, warehouse, store, distribution center, storage facility, underground facility, space (e.g. above ground, outer-space) facility, floating facility, cavern, tunnel facility, indoor facility, open-air facility, outdoor facility with some walls/ doors/ reflective barriers, open facility, semi-open facility, car, truck, bus, van, container, ship/boat, submersible, train, tram, airplane, vehicle, mobile home, cave, tunnel, pipe, channel, metropolitan area, downtown area with relatively tall buildings, valley, well, duct, pathway, gas line, oil line, water pipe, network of interconnecting pathways/alleys/roads/tubes/cavities/ caves/pipe-like structure/air space/fluid space, human body, animal body, body cavity, organ, bone, teeth, soft tissue, hard tissue, rigid tissue, non-rigid tissue, blood/body fluid vessel, windpipe, air duct, den, etc. The venue may be indoor space, outdoor space, The venue may include both the inside and outside of the space. For example, the venue may include both the inside of a building and the outside of the building. For example, the venue can be a building that has one floor or multiple floors, and a portion of the building can be underground. The shape of the building can be, e.g., round, square, rectangular, triangle, or irregular-shaped. These are merely examples. The disclosure can be used to detect events in other types of venue or spaces.

The wireless transmitter (e.g. Type 1 device) and/or the wireless receiver (e.g. Type 2 device) may be embedded in a portable device (e.g. a module, or a device with the module) that may move with the object (e.g. in prior movement and/or current movement). The portable device may be communicatively coupled with the object using a wired connection (e.g. through USB, microUSB, Firewire, HDMI, serial port, parallel port, and other connectors) and/or a connection (e.g. Bluetooth, Bluetooth Low Energy (BLE), WiFi, LTE, NFC, ZigBee). The portable device may be a lightweight device. The portable may be powered by battery, rechargeable battery and/or AC power. The portable device may be very small (e.g. at sub-millimeter scale and/or sub-centimeter scale), and/or small (e.g. coin-size, card-size, pocket-size, or larger). The portable device may be large, sizable, and/or bulky (e.g. heavy machinery to be installed). The portable device may be a WiFi hotspot, access point, mobile WiFi (MiFi), dongle with USB/micro USB/ Firewire/ other connector, smartphone, portable computer, computer, tablet, smart device, internet-of-thing (IoT) device, WiFi-enabled device, LTE-enabled device, a smart watch, smart glass, smart mirror, smart antenna, smart battery, smart light, smart pen, smart ring, smart door, smart window, smart clock, small battery, smart wallet, smart belt, smart handbag, smart clothing/garment, smart ornament, smart packaging, smart paper/ book/ magazine/ poster/ printed matter/ signage/ display/ lighted system/ lighting system, smart key/ tool, smart bracelet/ chain/ necklace/ wearable/ accessory, smart pad/ cushion, smart tile/ block/ brick/ building material/ other material, smart garbage can/ waste container, smart food carriage/ storage, smart ball/ racket, smart chair/ sofa/ bed, smart shoe/ footwear/ carpet/ mat/ shoe rack, smart glove/ hand wear/ ring/ hand ware, smart hat/ headwear/ makeup/ sticker/ tattoo, smart mirror, smart toy, smart pill, smart utensil, smart bottle/food container, smart tool, smart device, IoT device, WiFi enabled device, network enabled device, 3G/4G/5G/6G enabled device, UMTS devices, 3GPP devices, GSM devices, EDGE devices, TDMA devices, FDMA devices, CDMA devices, WCDMA devices, TD-SCDMA devices, embeddable device, implantable device, air conditioner, refrigerator, heater, furnace, furniture, oven, cooking device, television/ set-top box (STB)/ DVD player/ audio player/ video player/ remote control, hi-fi, audio device, speaker, lamp/ light, wall, door, window, roof, roof tile/ shingle/ structure/ attic structure/ device/ feature/ installation/ fixtures, lawn mower/ garden tools/ yard tools/ mechanics tools/ garage tools/, garbage can/ container, 20-ft/40-ft container, storage container, factory/ manufacturing/ production device, repair tools, fluid container, machine, machinery to be installed, vehicle, cart, wagon, warehouse vehicle, car, bicycle, motorcycle, boat, vessel, airplane, basket/ box/ bag/ bucket/ container, smart plate/ cup/ bowl/ pot/ mat/ utensils/ kitchen tools/ kitchen devices/ kitchen accessories/ cabinets/ tables/ chairs/ tiles/ lights/ water pipes/ taps/ gas range/ oven/ dishwashing machine/ etc. The portable device may have a battery that may be replaceable, irreplaceable, rechargeable, and/or non-rechargeable. The portable device may be wirelessly charged. The portable device may be a smart payment card. The portable device may be a payment card used in parking lots, highways, entertainment parks, or other venues/facilities that need payment. The portable device may have an identity (ID)/identifier as described above.

An event may be monitored based on the TSCI. The event may be an object related event, such as fall-down of the object (e.g. an person and/or a sick person), rotation, hesitation, pause, impact (e.g. a person hitting a sandbag, door, window, bed, chair, table, desk, cabinet, box, another person, animal, bird, fly, table, chair, ball, bowling ball, tennis ball, football, soccer ball, baseball, basketball, volley ball), two-body action (e.g. a person letting go a balloon, catching a fish, molding a clay, writing a paper, person typing on a computer), car moving in a garage, person carrying a smart phone and walking around an airport/mall/government building/office/etc., autonomous moveable object/machine moving around (e.g. vacuum cleaner, utility vehicle, car, drone, self-driving car).

The task or the wireless smart sensing task may comprise: object detection, presence detection, proximity detection, object recognition, activity recognition, object verification, object counting, daily activity monitoring, well-being monitoring, vital sign monitoring, health condition monitoring, baby monitoring, elderly monitoring, sleep monitoring, sleep stage monitoring, walking monitoring, exercise monitoring, tool detection, tool recognition, tool verification, patient detection, patient monitoring, patient verification, machine detection, machine recognition, machine verification, human detection, human recognition, human verification, baby detection, baby recognition, baby verification, human breathing detection, human breathing recognition, human breathing estimation, human breathing verification, human heart beat detection, human heart beat recognition, human heart beat estimation, human heart beat verification, fall-down detection, fall-down recognition, fall-down estimation, fall-down verification, emotion detection, emotion recognition, emotion estimation, emotion verification, motion detection, motion degree estimation, motion recognition, motion estimation, motion verification, periodic motion detection, periodic motion recognition, periodic motion estimation, periodic motion verification, repeated motion detection, repeated motion recognition, repeated motion estimation, repeated motion verification, stationary motion detection, stationary motion recognition, stationary motion estimation, stationary motion verification, cyclo-stationary motion detection, cyclo-stationary motion recognition, cyclo-stationary motion estimation, cyclo-stationary motion verification, transient motion detection, transient motion recognition, transient motion estimation, transient motion verification, trend detection, trend recognition, trend estimation, trend verification, breathing detection, breathing recognition, breathing estimation, breathing estimation, human biometrics detection, human biometric recognition, human biometrics estimation, human biometrics verification, environment informatics detection, environment informatics recognition, environment informatics estimation, environment informatics verification, gait detection, gait recognition, gait estimation, gait verification, gesture detection, gesture recognition, gesture estimation, gesture verification, machine learning, supervised learning, unsupervised learning, semi-supervised learning, clustering, feature extraction, featuring training, principal component analysis, eigen-decomposition, frequency decomposition, time decomposition, time-frequency decomposition, functional decomposition, other decomposition, training, discriminative training, supervised training, unsupervised training, semi-supervised training, neural network, sudden motion detection, fall-down detection, danger detection, life-threat detection, regular motion detection, stationary motion detection, cyclo-stationary motion detection, intrusion detection, suspicious motion detection, security, safety monitoring, navigation, guidance, map-based processing, map-based correction, model-based processing/correction, irregularity detection, locationing, room sensing, tracking, multiple object tracking, indoor tracking, indoor position, indoor navigation, energy management, power transfer, wireless power transfer, object counting, car tracking in parking garage, activating a device/system (e.g. security system, access system, alarm, siren, speaker, television, entertaining system, camera, heater/air-conditioning (HVAC) system, ventilation system, lighting system, gaming system, coffee machine, cooking device, cleaning device, housekeeping device), geometry estimation, augmented reality, wireless communication, data communication, signal broadcasting, networking, coordination, administration, encryption, protection, cloud computing, other processing and/or other task. The task may be performed by the Type 1 device, the Type 2 device, another Type 1 device, another Type 2 device, a nearby device, a local server (e.g. hub device), edge server, a cloud server, and/or another device. The task may be based on TSCI between any pair of Type 1 device and Type 2 device. A Type 2 device may be a Type 1 device, and vice versa. A Type 2 device may play/perform the role (e.g. functionality) of Type 1 device temporarily, continuously, sporadically, simultaneously, and/or contemporaneously, and vice versa. A first part of the task may comprise at least one of: preprocessing, processing, signal conditioning, signal processing, post-processing, processing sporadically/ continuously/ simultaneously/ contemporaneously/ dynamically/ adaptive/ on-demand/ as-needed, calibrating, denoising, feature extraction, coding, encryption, transformation, mapping, motion detection, motion estimation, motion change detection, motion pattern detection, motion pattern estimation, motion pattern recognition, vital sign detection, vital sign estimation, vital sign recognition, periodic motion detection, periodic motion estimation, repeated motion detection/estimation, breathing rate detection, breathing rate estimation, breathing pattern detection, breathing pattern estimation, breathing pattern recognition, heart beat detection, heart beat estimation, heart pattern detection, heart pattern estimation, heart pattern recognition, gesture detection, gesture estimation, gesture recognition, speed detection, speed estimation, object locationing, object tracking, navigation, acceleration estimation, acceleration detection, fall-down detection, change detection, intruder (and/or illegal action) detection, baby detection, baby monitoring, patient monitoring, object recognition, wireless power transfer, and/or wireless charging.

A second part of the task may comprise at least one of: a smart home task, smart office task, smart building task, smart factory task (e.g. manufacturing using a machine or an assembly line), smart internet-of-thing (IoT) task, smart system task, smart home operation, smart office operation, smart building operation, smart manufacturing operation (e.g. moving supplies/parts/raw material to a machine/an assembly line), IoT operation, smart system operation, turning on a light, turning off the light, controlling the light in at least one of: a room, region, and/or the venue, playing a sound clip, playing the sound clip in at least one of: the room, the region, and/or the venue, playing the sound clip of at least one of: a welcome, greeting, farewell, first message, and/or a second message associated with the first part of the task, turning on an appliance, turning off the appliance, controlling the appliance in at least one of: the room, the region, and/or the venue, turning on an electrical system, turning off the electrical system, controlling the electrical system in at least one of: the room, the region, and/or the venue, turning on a security system, turning off the security system, controlling the security system in at least one of: the room, the region, and/or the venue, turning on a mechanical system, turning off a mechanical system, controlling the mechanical system in at least one of: the room, the region, and/or the venue, and/or controlling at least one of: an air conditioning system, heating system, ventilation system, lighting system, heating device, stove, entertainment system, door, fence, window, garage, computer system, networked device, networked system, home appliance, office equipment, lighting device, robot (e.g. robotic arm), smart vehicle, smart machine, assembly line, smart device, internet-of-thing (IoT) device, smart home device, and/or a smart office device.

The task may include: detect a user returning home, detect a user leaving home, detect a user moving from one room to another, detect/control/lock/unlock/open/ close/partially open a window/door/garage door/blind/curtain/panel/solar panel/sun shade, detect a pet, detect/monitor a user doing something (e.g. sleeping on sofa, sleeping in bedroom, running on treadmill, cooking, sitting on sofa, watching TV, eating in kitchen, eating in dining room, going upstairs/downstairs, going outside/coming back, in the rest room), monitor/detect location of a user/pet, do something (e.g. send a message, notify/report to someone) automatically upon detection, do something for the user automatically upon detecting the user, turn on/off/dim a light, turn on/off music/radio/home entertainment system, turn on/off/adjust/control TV/HiFi/set-top-box (STB)/ home entertainment system/smart speaker/smart device, turn on/off/adjust air conditioning system, turn on/off/adjust ventilation system, turn on/off/adjust heating system, adjust/control curtains/light shades, turn on/off/wake a computer, turn on/off/pre-heat/control coffee machine/hot water pot, turn on/off/control/preheat cooker/oven/microwave oven/another cooking device, check/adjust temperature, check weather forecast, check telephone message box, check mail, do a system check, control/adjust a system, check/control/arm/disarm security system/baby monitor, check/control refrigerator, give a report (e.g. through a speaker such as Google home, Amazon Echo, on a display/screen, via a webpage/email/messaging system/notification system).

For example, when a user arrives home in his car, the task may be to, automatically, detect the user or his car approaching, open the garage door upon detection, turn on the driveway/garage light as the user approaches the garage, turn on air conditioner/ heater/ fan, etc. As the user enters the house, the task may be to, automatically, turn on the entrance light, turn off driveway/garage light, play a greeting message to welcome the user, turn on the music, turn on the radio and tuning to the user’s favorite radio news channel, open the curtain/blind, monitor the user’s mood, adjust the lighting and sound environment according to the user’s mood or the current/imminent event (e.g. do romantic lighting and music because the user is scheduled to eat dinner with girlfriend in 1 hour) on the user’s daily calendar, warm the food in microwave that the user prepared in the morning, do a diagnostic check of all systems in the house, check weather forecast for tomorrow’s work, check news of interest to the user, check user’s calendar and to-do list and play reminder, check telephone answer system/messaging system/email and give a verbal report using dialog system/speech synthesis, remind (e.g. using audible tool such as speakers/HiFi/ speech synthesis/sound/voice/ music/song/sound field/ background sound field/ dialog system, using visual tool such as TV/entertainment system/ computer/ notebook/smart pad/display/light/color/brightness/patterns/symbols, using haptic tool/virtual reality tool/gesture/ tool, using a smart device/appliance/material/furniture/fixture, using web tool/server/ hub device/cloud server/fog server/edge server/home network/mesh network, using messaging tool/notification tool/communication tool/scheduling tool/email, using user interface/GUI, using scent/smell/ fragrance/taste, using neural tool/nervous system tool, using a combination) the user of his mother’s birthday and to call her, prepare a report, and give the report (e.g. using a tool for reminding as discussed above). The task may turn on the air conditioner/heater/ventilation system in advance, or adjust temperature setting of smart thermostat in advance, etc. As the user moves from the entrance to the living room, the task may be to turn on the living room light, open the living room curtain, open the window, turn off the entrance light behind the user, turn on the TV and set-top box, set TV to the user’s favorite channel, adjust an appliance according to the user’s preference and conditions/states (e.g. adjust lighting and choose/play music to build a romantic atmosphere), etc.

Another example may be: When the user wakes up in the morning, the task may be to detect the user moving around in the bedroom, open the blind/curtain, open the window, turn off the alarm clock, adjust indoor temperature from night-time temperature profile to day-time temperature profile, turn on the bedroom light, turn on the restroom light as the user approaches the restroom, check radio or streaming channel and play morning news, turn on the coffee machine and preheat the water, turn off security system, etc. When the user walks from bedroom to kitchen, the task may be to turn on the kitchen and hallway lights, turn off the bedroom and restroom lights, move the music/message/reminder from the bedroom to the kitchen, turn on the kitchen TV, change TV to morning news channel, lower the kitchen blind and open the kitchen window to bring in fresh air, unlock backdoor for the user to check the backyard, adjust temperature setting for the kitchen, etc. Another example may be: When the user leaves home for work, the task may be to detect the user leaving, play a farewell and/or have-a-good-day message, open/close garage door, turn on/off garage light and driveway light, turn off/dim lights to save energy (just in case the user forgets), close/lock all windows/doors (just in case the user forgets), turn off appliance (especially stove, oven, microwave oven), turn on/arm the home security system to guard the home against any intruder, adjust air conditioning/heating/ventilation systems to "away-from-home" profile to save energy, send alerts/reports/updates to the user’s smart phone, etc.

A motion may comprise at least one of: a no-motion, resting motion, non-moving motion, movement, change in position/location, deterministic motion, transient motion, fall-down motion, repeating motion, periodic motion, pseudo-periodic motion, periodic/repeated motion associated with breathing, periodic/repeated motion associated with heartbeat, periodic/repeated motion associated with living object, periodic/repeated motion associated with machine, periodic/repeated motion associated with man-made object, periodic/repeated motion associated with nature, complex motion with transient element and periodic element, repetitive motion, non-deterministic motion, probabilistic motion, chaotic motion, random motion, complex motion with non-deterministic element and deterministic element, stationary random motion, pseudo-stationary random motion, cyclo-stationary random motion, non-stationary random motion, stationary random motion with periodic autocorrelation function (ACF), random motion with periodic ACF for period of time, random motion that is pseudo-stationary for a period of time, random motion of which an instantaneous ACF has a pseudo-periodic/repeating element for a period of time, machine motion, mechanical motion, vehicle motion, drone motion, air-related motion, wind-related motion, weather-related motion, water-related motion, fluid-related motion, ground-related motion, change in electro-magnetic characteristics, sub-surface motion, seismic motion, plant motion, animal motion, human motion, normal motion, abnormal motion, dangerous motion, warning motion, suspicious motion, rain, fire, flood, tsunami, explosion, collision, imminent collision, human body motion, head motion, facial motion, eye motion, mouth motion, tongue motion, neck motion, finger motion, hand motion, arm motion, shoulder motion, body motion, chest motion, abdominal motion, hip motion, leg motion, foot motion, body joint motion, knee motion, elbow motion, upper body motion, lower body motion, skin motion, below-skin motion, subcutaneous tissue motion, blood vessel motion, intravenous motion, organ motion, heart motion, lung motion, stomach motion, intestine motion, bowel motion, eating motion, breathing motion, facial expression, eye expression, mouth expression, talking motion, singing motion, eating motion, gesture, hand gesture, arm gesture, keystroke, typing stroke, user-interface gesture, man-machine interaction, gait, dancing movement, coordinated movement, and/or coordinated body movement.

The heterogeneous IC of the Type 1 device and/or any Type 2 receiver may comprise low-noise amplifier (LNA), power amplifier, transmit-receive switch, media access controller, baseband radio, 2.4 GHz radio, 3.65 GHz radio, 4.9 GHz radio, 5 GHz radio, 5.9GHz radio, below 6GHz radio, below 60 GHz radio and/or another radio. The heterogeneous IC may comprise a processor, a memory communicatively coupled with the processor, and a set of instructions stored in the memory to be executed by the processor. The IC and/or any processor may comprise at least one of: general purpose processor, special purpose processor, microprocessor, multi-processor, multi-core processor, parallel processor, CISC processor, RISC processor, microcontroller, central processing unit (CPU), graphical processor unit (GPU), digital signal processor (DSP), application specific integrated circuit (ASIC), field programmable gate array (FPGA), embedded processor (e.g. ARM), logic circuit, other programmable logic device, discrete logic, and/or a combination. The heterogeneous IC may support broadband network, wireless network, mobile network, mesh network, cellular network, wireless local area network (WLAN), wide area network (WAN), and metropolitan area network (MAN), WLAN standard, WiFi, LTE, LTE-A, LTE-U, 802.11 standard, 802.11a, 802.11b, 802.11g, 802.11n, 802.11ac, 802.11ad, 802.11af, 802,11ah, 802.11ax, 802.11ay, mesh network standard, 802.15 standard, 802.16 standard, cellular network standard, 3G, 3.5G, 4G, beyond 4G, 4.5G, 5G, 6G, 7G, 8G, 9G, UMTS, 3GPP, GSM, EDGE, TDMA, FDMA, CDMA, WCDMA, TD-SCDMA, Bluetooth, Bluetooth Low-Energy (BLE), NFC, Zigbee, WiMax, and/or another wireless network protocol.

The processor may comprise general purpose processor, special purpose processor, microprocessor, microcontroller, embedded processor, digital signal processor, central processing unit (CPU), graphical processing unit (GPU), multi-processor, multi-core processor, and/or processor with graphics capability, and/or a combination. The memory may be volatile, non-volatile, random access memory (RAM), Read Only Memory (ROM), Electrically Programmable ROM (EPROM), Electrically Erasable Programmable ROM (EEPROM), hard disk, flash memory, CD-ROM, DVD-ROM, magnetic storage, optical storage, organic storage, storage system, storage network, network storage, cloud storage, edge storage, local storage, external storage, internal storage, or other form of non-transitory storage medium known in the art. The set of instructions (machine executable code) corresponding to the method steps may be embodied directly in hardware, in software, in firmware, or in combinations thereof. The set of instructions may be embedded, preloaded, loaded upon boot up, loaded on the fly, loaded on demand, pre-installed, installed, and/or downloaded.

The presentation may be a presentation in an audio-visual way (e.g. using combination of visual, graphics, text, symbols, color, shades, video, animation, sound, speech, audio, etc.), graphical way (e.g. using GUI, animation, video), textual way (e.g. webpage with text, message, animated text), symbolic way (e.g. emoticon, signs, hand gesture), or mechanical way (e.g. vibration, actuator movement, haptics, etc.).

Basic computation

Computational workload associated with the method is shared among the processor, the Type 1 heterogeneous wireless device, the Type 2 heterogeneous wireless device, a local server (e.g. hub device), a cloud server, and another processor.

An operation, pre-processing, processing and/or postprocessing may be applied to data (e.g. TSCI, autocorrelation, features of TSCI). An operation may be preprocessing, processing and/or postprocessing. The preprocessing, processing and/or postprocessing may be an operation. An operation may comprise preprocessing, processing, postprocessing, scaling, computing a confidence factor, computing a line-of-sight (LOS) quantity, computing a non-LOS (NLOS) quantity, a quantity comprising LOS and NLOS, computing a single link (e.g. path, communication path, link between a transmitting antenna and a receiving antenna) quantity, computing a quantity comprising multiple links, computing a function of the operands, filtering, linear filtering, nonlinear filtering, folding, grouping, energy computation, lowpass filtering, bandpass filtering, highpass filtering, median filtering, rank filtering, quartile filtering, percentile filtering, mode filtering, finite impulse response (FIR) filtering, infinite impulse response (IIR) filtering, moving average (MA) filtering, autoregressive (AR) filtering, autoregressive moving averaging (ARMA) filtering, selective filtering, adaptive filtering, interpolation, decimation, subsampling, upsampling, resampling, time correction, time base correction, phase correction, magnitude correction, phase cleaning, magnitude cleaning, matched filtering, enhancement, restoration, denoising, smoothing, signal conditioning, enhancement, restoration, spectral analysis, linear transform, nonlinear transform, inverse transform, frequency transform, inverse frequency transform, Fourier transform (FT), discrete time FT (DTFT), discrete FT (DFT), fast FT (FFT), wavelet transform, Laplace transform, Hilbert transform, Hadamard transform, trigonometric transform, sine transform, cosine transform, DCT, power-of-2 transform, sparse transform, graph-based transform, graph signal processing, fast transform, a transform combined with zero padding, cyclic padding, padding, zero padding, feature extraction, decomposition, projection, orthogonal projection, non-orthogonal projection, over-complete projection, eigen-decomposition, singular value decomposition (SVD), principle component analysis (PCA), independent component analysis (ICA), grouping, sorting, thresholding, soft thresholding, hard thresholding, clipping, soft clipping, first derivative, second order derivative, high order derivative, convolution, multiplication, division, addition, subtraction, integration, maximization, minimization, least mean square error, recursive least square, constrained least square, batch least square, least absolute error, least mean square deviation, least absolute deviation, local maximization, local minimization, optimization of a cost function, neural network, recognition, labeling, training, clustering, machine learning, supervised learning, unsupervised learning, semi-supervised learning, comparison with another TSCI, similarity score computation, quantization, vector quantization, matching pursuit, compression, encryption, coding, storing, transmitting, normalization, temporal normalization, frequency domain normalization, classification, clustering, labeling, tagging, learning, detection, estimation, learning network, mapping, remapping, expansion, storing, retrieving, transmitting, receiving, representing, merging, combining, splitting, tracking, monitoring, matched filtering, Kalman filtering, particle filter, intrapolation, extrapolation, histogram estimation, importance sampling, Monte Carlo sampling, compressive sensing, representing, merging, combining, splitting, scrambling, error protection, forward error correction, doing nothing, time varying processing, conditioning averaging, weighted averaging, arithmetic mean, geometric mean, harmonic mean, averaging over selected frequency, averaging over antenna links, logical operation, permutation, combination, sorting, AND, OR, XOR, union, intersection, vector addition, vector subtraction, vector multiplication, vector division, inverse, norm, distance, and/or another operation. The operation may be the preprocessing, processing, and/or post-processing. Operations may be applied jointly on multiple time series or functions.

The function (e.g. function of operands) may comprise: scalar function, vector function, discrete function, continuous function, polynomial function, characteristics, feature, magnitude, phase, exponential function, logarithmic function, trigonometric function, transcendental function, logical function, linear function, algebraic function, nonlinear function, piecewise linear function, real function, complex function, vector-valued function, inverse function, derivative of function, integration of function, circular function, function of another function, one-to-one function, one-to-many function, many-to-one function, many-to-many function, zero crossing, absolute function, indicator function, mean, mode, median, range, statistics, histogram, variance, standard deviation, measure of variation, spread, dispersion, deviation, divergence, range, interquartile range, total variation, absolute deviation, total deviation, arithmetic mean, geometric mean, harmonic mean, trimmed mean, percentile, square, cube, root, power, sine, cosine, tangent, cotangent, secant, cosecant, elliptical function, parabolic function, hyperbolic function, game function, zeta function, absolute value, thresholding, limiting function, floor function, rounding function, sign function, quantization, piecewise constant function, composite function, function of function, time function processed with an operation (e.g. filtering), probabilistic function, stochastic function, random function, ergodic function, stationary function, deterministic function, periodic function, repeated function, transformation, frequency transform, inverse frequency transform, discrete time transform, Laplace transform, Hilbert transform, sine transform, cosine transform, triangular transform, wavelet transform, integer transform, power-of-2 transform, sparse transform, projection, decomposition, principle component analysis (PCA), independent component analysis (ICA), neural network, feature extraction, moving function, function of moving window of neighboring items of time series, filtering function, convolution, mean function, histogram, variance/standard deviation function, statistical function, short-time transform, discrete transform, discrete Fourier transform, discrete cosine transform, discrete sine transform, Hadamard transform, eigen-decomposition, eigenvalue, singular value decomposition (SVD), singular value, orthogonal decomposition, matching pursuit, sparse transform, sparse approximation, any decomposition, graph-based processing, graph-based transform, graph signal processing, classification, identifying a class/group/category, labeling, learning, machine learning, detection, estimation, feature extraction, learning network, feature extraction, denoising, signal enhancement, coding, encryption, mapping, remapping, vector quantization, lowpass filtering, highpass filtering, bandpass filtering, matched filtering, Kalman filtering, preprocessing, postprocessing, particle filter, FIR filtering, IIR filtering, autoregressive (AR) filtering, adaptive filtering, first order derivative, high order derivative, integration, zero crossing, smoothing, median filtering, mode filtering, sampling, random sampling, resampling function, downsampling, down-converting, upsampling, up-converting, interpolation, extrapolation, importance sampling, Monte Carlo sampling, compressive sensing, statistics, short term statistics, long term statistics, autocorrelation function, cross correlation, moment generating function, time averaging, weighted averaging, special function, Bessel function, error function, complementary error function, Beta function, Gamma function, integral function, Gaussian function, Poisson function, etc.

Machine learning, training, discriminative training, deep learning, neural network, continuous time processing, distributed computing, distributed storage, acceleration using GPU/DSP/coprocessor/multicore/multiprocessing may be applied to a step (or each step) of this disclosure.

A frequency transform may include Fourier transform, Laplace transform, Hadamard transform, Hilbert transform, sine transform, cosine transform, triangular transform, wavelet transform, integer transform, power-of-2 transform, combined zero padding and transform, Fourier transform with zero padding, and/or another transform. Fast versions and/or approximated versions of the transform may be performed. The transform may be performed using floating point, and/or fixed point arithmetic.

An inverse frequency transform may include inverse Fourier transform, inverse Laplace transform, inverse Hadamard transform, inverse Hilbert transform, inverse sine transform, inverse cosine transform, inverse triangular transform, inverse wavelet transform, inverse integer transform, inverse power-of-2 transform, combined zero padding and transform, inverse Fourier transform with zero padding, and/or another transform. Fast versions and/or approximated versions of the transform may be performed. The transform may be performed using floating point, and/or fixed point arithmetic.

A quantity/feature from a TSCI may be computed. The quantity may comprise statistic of at least one of: motion, location, map coordinate, height, speed, acceleration, movement angle, rotation, size, volume, time trend, pattern, one-time pattern, repeating pattern, evolving pattern, time pattern, mutually excluding patterns, related/correlated patterns, cause-and-effect, correlation, short-term/long-term correlation, tendency, inclination, statistics, typical behavior, atypical behavior, time trend, time profile, periodic motion, repeated motion, repetition, tendency, change, abrupt change, gradual change, frequency, transient, breathing, gait, action, event, suspicious event, dangerous event, alarming event, warning, belief, proximity, collision, power, signal, signal power, signal strength, signal intensity, received signal strength indicator (RSSI), signal amplitude, signal phase, signal frequency component, signal frequency band component, channel state information (CSI), map, time, frequency, time-frequency, decomposition, orthogonal decomposition, non-orthogonal decomposition, tracking, breathing, heart beat, statistical parameters, cardiopulmonary statistics/analytics (e.g. output responses), daily activity statistics/analytics, chronic disease statistics/analytics, medical statistics/analytics, an early (or instantaneous or contemporaneous or delayed) indication/suggestion/sign/indicator/verifier/ detection/symptom of a disease/condition/ situation, biometric, baby, patient, machine, device, temperature, vehicle, parking lot, venue, lift, elevator, spatial, road, fluid flow, home, room, office, house, building, warehouse, storage, system, ventilation, fan, pipe, duct, people, human, car, boat, truck, airplane, drone, downtown, crowd, impulsive event, cyclo-stationary, environment, vibration, material, surface, 3-dimensional, 2-dimensional, local, global, presence, and/or another measurable quantity/variable.

Sliding window/algorithm

Sliding time window may have time varying window width. It may be smaller at the beginning to enable fast acquisition and may increase over time to a steady-state size. The steady-state size may be related to the frequency, repeated motion, transient motion, and/or STI to be monitored. Even in steady state, the window size may be adaptively (and/or dynamically) changed (e.g. adjusted, varied, modified) based on battery life, power consumption, available computing power, change in amount of targets, the nature of motion to be monitored, etc.

The time shift between two sliding time windows at adjacent time instance may be constant/variable/locally adaptive/dynamically adjusted over time. When shorter time shift is used, the update of any monitoring may be more frequent which may be used for fast changing situations, object motions, and/or objects. Longer time shift may be used for slower situations, object motions, and/or objects.

The window width/size and/or time shift may be changed (e.g. adjusted, varied, modified) upon a user request/choice. The time shift may be changed automatically (e.g. as controlled by processor/ computer/server/hub device/cloud server) and/or adaptively (and/or dynamically).

At least one characteristics (e.g. characteristic value, or characteristic point) of a function (e.g. auto-correlation function, auto-covariance function, cross-correlation function, cross-covariance function, power spectral density, time function, frequency domain function, frequency transform) may be determined (e.g. by an object tracking server, the processor, the Type 1 heterogeneous device, the Type 2 heterogeneous device, and/or another device). The at least one characteristics of the function may include: a maximum, minimum, extremum, local maximum, local minimum, local extremum, local extremum with positive time offset, first local extremum with positive time offset, n^th local extremum with positive time offset, local extremum with negative time offset, first local extremum with negative time offset, n^th local extremum with negative time offset, constrained maximum, constrained minimum, constrained extremum, significant maximum, significant minimum, significant extremum, slope, derivative, higher order derivative, maximum slope, minimum slope, local maximum slope, local maximum slope with positive time offset, local minimum slope, constrained maximum slope, constrained minimum slope, maximum higher order derivative, minimum higher order derivative, constrained higher order derivative, zero-crossing, zero crossing with positive time offset, n^th zero crossing with positive time offset, zero crossing with negative time offset, n^th zero crossing with negative time offset, constrained zero-crossing, zero-crossing of slope, zero-crossing of higher order derivative, and/or another characteristics. At least one argument of the function associated with the at least one characteristics of the function may be identified. Some quantity (e.g. spatial-temporal information of the object) may be determined based on the at least one argument of the function.

A characteristics (e.g. characteristics of motion of an object in the venue) may comprise at least one of: an instantaneous characteristics, short-term characteristics, repetitive characteristics, recurring characteristics, history, incremental characteristics, changing characteristics, deviational characteristics, phase, magnitude, degree, time characteristics, frequency characteristics, time-frequency characteristics, decomposition characteristics, orthogonal decomposition characteristics, non-orthogonal decomposition characteristics, deterministic characteristics, probabilistic characteristics, stochastic characteristics, autocorrelation function (ACF), mean, variance, standard deviation, measure of variation, spread, dispersion, deviation, divergence, range, interquartile range, total variation, absolute deviation, total deviation, statistics, duration, timing, trend, periodic characteristics, repetition characteristics, long-term characteristics, historical characteristics, average characteristics, current characteristics, past characteristics, future characteristics, predicted characteristics, location, distance, height, speed, direction, velocity, acceleration, change of the acceleration, angle, angular speed, angular velocity, angular acceleration of the object, change of the angular acceleration, orientation of the object, angular of rotation, deformation of the object, shape of the object, change of shape of the object, change of size of the object, change of structure of the object, and/or change of characteristics of the object.

At least one local maximum and at least one local minimum of the function may be identified. At least one local signal-to-noise-ratio-like (SNR-like) parameter may be computed for each pair of adjacent local maximum and local minimum. The SNR-like parameter may be a function (e.g. linear, log, exponential function, monotonic function) of a fraction of a quantity (e.g. power, magnitude) of the local maximum over the same quantity of the local minimum. It may also be the function of a difference between the quantity of the local maximum and the same quantity of the local minimum. Significant local peaks may be identified or selected. Each significant local peak may be a local maximum with SNR-like parameter greater than a threshold T1 and/or a local maximum with amplitude greater than a threshold T2. The at least one local minimum and the at least one local minimum in the frequency domain may be identified/computed using a persistence-based approach.

A set of selected significant local peaks may be selected from the set of identified significant local peaks based on a selection criterion (e.g. a quality criterion, a signal quality condition). The characteristics/STI of the object may be computed based on the set of selected significant local peaks and frequency values associated with the set of selected significant local peaks. In one example, the selection criterion may always correspond to select the strongest peaks in a range. While the strongest peaks may be selected, the unselected peaks may still be significant (rather strong).

Unselected significant peaks may be stored and/or monitored as "reserved" peaks for use in future selection in future sliding time windows. As an example, there may be a particular peak (at a particular frequency) appearing consistently over time. Initially, it may be significant but not selected (as other peaks may be stronger). But in later time, the peak may become stronger and more dominant and may be selected. When it became "selected", it may be back-traced in time and made "selected" in the earlier time when it was significant but not selected. In such case, the back-traced peak may replace a previously selected peak in an early time. The replaced peak may be the relatively weakest, or a peak that appear in isolation in time (i.e. appearing only briefly in time).

In another example, the selection criterion may not correspond to select the strongest peaks in the range. Instead, it may consider not only the "strength" of the peak, but the "trace" of the peak - peaks that may have happened in the past, especially those peaks that have been identified for a long time.

For example, if a finite state machine (FSM) is used, it may select the peak(s) based on the state of the FSM. Decision thresholds may be computed adaptively (and/or dynamically) based on the state of the FSM.

A similarity score and/or component similarity score may be computed (e.g. by a server (e.g. hub device), the processor, the Type 1 device, the Type 2 device, a local server, a cloud server, and/or another device) based on a pair of temporally adjacent CI of a TSCI. The pair may come from the same sliding window or two different sliding windows. The similarity score may also be based on a pair of, temporally adjacent or not so adjacent, CI from two different TSCI. The similarity score and/or component similar score may be/comprise: time reversal resonating strength (TRRS), correlation, cross-correlation, auto-correlation, correlation indicator, covariance, cross-covariance, auto-covariance, inner product of two vectors, distance score, norm, metric, quality metric, signal quality condition, statistical characteristics, discrimination score, neural network, deep learning network, machine learning, training, discrimination, weighted averaging, preprocessing, denoising, signal conditioning, filtering, time correction, timing compensation, phase offset compensation, transformation, component-wise operation, feature extraction, finite state machine, and/or another score. The characteristics and/or STI may be determined/computed based on the similarity score.

Any threshold may be pre-determined, adaptively (and/or dynamically) determined and/or determined by a finite state machine. The adaptive determination may be based on time, space, location, antenna, path, link, state, battery life, remaining battery life, available power, available computational resources, available network bandwidth, etc.

A threshold to be applied to a test statistics to differentiate two events (or two conditions, or two situations, or two states), A and B, may be determined. Data (e.g. CI, channel state information (CSI), power parameter) may be collected under A and/or under B in a training situation. The test statistics may be computed based on the data. Distributions of the test statistics under A may be compared with distributions of the test statistics under B (reference distribution), and the threshold may be chosen according to some criteria. The criteria may comprise: maximum likelihood (ML), maximum aposterior probability (MAP), discriminative training, minimum Type 1 error for a given Type 2 error, minimum Type 2 error for a given Type 1 error, and/or other criteria (e.g. a quality criterion, signal quality condition). The threshold may be adjusted to achieve different sensitivity to the A, B and/or another event/condition/situation/state. The threshold adjustment may be automatic, semi-automatic and/or manual. The threshold adjustment may be applied once, sometimes, often, periodically, repeatedly, occasionally, sporadically, and/or on demand. The threshold adjustment may be adaptive (and/or dynamically adjusted). The threshold adjustment may depend on the object, object movement/location/ direction/action, object characteristics/ STI/size/ property/trait/habit/behavior, the venue, feature/ fixture/ furniture/barrier/ material/ machine/living thing/thing/object/boundary/ surface/ medium that is in/at/of the venue, map, constraint of the map (or environmental model), the event/state/ situation/condition, time, timing, duration, current state, past history, user, and/or a personal preference, etc.

A stopping criterion (or skipping or bypassing or blocking or pausing or passing or rejecting criterion) of an iterative algorithm may be that change of a current parameter (e.g. offset value) in the updating in an iteration is less than a threshold. The threshold may be 0.5, 1, 1.5, 2, or another number. The threshold may be adaptive (and/or dynamically adjusted). It may change as the iteration progresses. For the offset value, the adaptive threshold may be determined based on the task, particular value of the first time, the current time offset value, the regression window, the regression analysis, the regression function, the regression error, the convexity of the regression function, and/or an iteration number.

The local extremum may be determined as the corresponding extremum of the regression function in the regression window. The local extremum may be determined based on a set of time offset values in the regression window and a set of associated regression function values. Each of the set of associated regression function values associated with the set of time offset values may be within a range from the corresponding extremum of the regression function in the regression window.

The searching for a local extremum may comprise robust search, minimization, maximization, optimization, statistical optimization, dual optimization, constraint optimization, convex optimization, global optimization, local optimization an energy minimization, linear regression, quadratic regression, higher order regression, linear programming, nonlinear programming, stochastic programming, combinatorial optimization, constraint programming, constraint satisfaction, calculus of variations, optimal control, dynamic programming, mathematical programming, multi-objective optimization, multi-modal optimization, disjunctive programming, space mapping, infinite-dimensional optimization, heuristics, metaheuristics, convex programming, semidefinite programming, conic programming, cone programming, integer programming, quadratic programming, fractional programming, numerical analysis, simplex algorithm, iterative method, gradient descent, subgradient method, coordinate descent, conjugate gradient method, Newton's algorithm, sequential quadratic programming, interior point method, ellipsoid method, reduced gradient method, quasi-Newton method, simultaneous perturbation stochastic approximation, interpolation method, pattern search method, line search, non-differentiable optimization, genetic algorithm, evolutionary algorithm, dynamic relaxation, hill climbing, particle swarm optimization, gravitation search algorithm, simulated annealing, memetic algorithm, differential evolution, dynamic relaxation, stochastic tunneling, Tabu search, reactive search optimization, curve fitting, least square, simulation based optimization, variational calculus, and/or variant. The search for local extremum may be associated with an objective function, loss function, cost function, utility function, fitness function, energy function, and/or an energy function.

Regression may be performed using regression function to fit sampled data (e.g. CI, feature of CI, component of CI) or another function (e.g. autocorrelation function) in a regression window. In at least one iteration, a length of the regression window and/or a location of the regression window may change. The regression function may be linear function, quadratic function, cubic function, polynomial function, and/or another function.

The regression analysis may minimize at least one of: error, aggregate error, component error, error in projection domain, error in selected axes, error in selected orthogonal axes, absolute error, square error, absolute deviation, square deviation, higher order error (e.g. third order, fourth order), robust error (e.g. square error for smaller error magnitude and absolute error for larger error magnitude, or first kind of error for smaller error magnitude and second kind of error for larger error magnitude), another error, weighted sum (or weighted mean) of absolute/square error (e.g. for wireless transmitter with multiple antennas and wireless receiver with multiple antennas, each pair of transmitter antenna and receiver antenna form a link), mean absolute error, mean square error, mean absolute deviation, and/or mean square deviation. Error associated with different links may have different weights. One possibility is that some links and/or some components with larger noise or lower signal quality metric may have smaller or bigger weight.), weighted sum of square error, weighted sum of higher order error, weighted sum of robust error, weighted sum of the another error, absolute cost, square cost, higher order cost, robust cost, another cost, weighted sum of absolute cost, weighted sum of square cost, weighted sum of higher order cost, weighted sum of robust cost, and/or weighted sum of another cost.

The regression error determined may be an absolute error, square error, higher order error, robust error, yet another error, weighted sum of absolute error, weighted sum of square error, weighted sum of higher order error, weighted sum of robust error, and/or weighted sum of the yet another error.

The time offset associated with maximum regression error (or minimum regression error) of the regression function with respect to the particular function in the regression window may become the updated current time offset in the iteration.

A local extremum may be searched based on a quantity comprising a difference of two different errors (e.g. a difference between absolute error and square error). Each of the two different errors may comprise an absolute error, square error, higher order error, robust error, another error, weighted sum of absolute error, weighted sum of square error, weighted sum of higher order error, weighted sum of robust error, and/or weighted sum of the another error.

The quantity may be compared with a reference data or a reference distribution, such as an F-distribution, central F-distribution, another statistical distribution, threshold, threshold associated with probability/histogram, threshold associated with probability/histogram of finding false peak, threshold associated with the F-distribution, threshold associated the central F-distribution, and/or threshold associated with the another statistical distribution.

The regression window may be determined based on at least one of: the movement (e.g. change in position/location) of the object, quantity associated with the object, the at least one characteristics and/or STI of the object associated with the movement of the object, estimated location of the local extremum, noise characteristics, estimated noise characteristics, signal quality metric, F-distribution, central F-distribution, another statistical distribution, threshold, preset threshold, threshold associated with probability/histogram, threshold associated with desired probability, threshold associated with probability of finding false peak, threshold associated with the F-distribution, threshold associated the central F-distribution, threshold associated with the another statistical distribution, condition that quantity at the window center is largest within the regression window, condition that the quantity at the window center is largest within the regression window, condition that there is only one of the local extremum of the particular function for the particular value of the first time in the regression window, another regression window, and/or another condition.

The width of the regression window may be determined based on the particular local extremum to be searched. The local extremum may comprise first local maximum, second local maximum, higher order local maximum, first local maximum with positive time offset value, second local maximum with positive time offset value, higher local maximum with positive time offset value, first local maximum with negative time offset value, second local maximum with negative time offset value, higher local maximum with negative time offset value, first local minimum, second local minimum, higher local minimum, first local minimum with positive time offset value, second local minimum with positive time offset value, higher local minimum with positive time offset value, first local minimum with negative time offset value, second local minimum with negative time offset value, higher local minimum with negative time offset value, first local extremum, second local extremum, higher local extremum, first local extremum with positive time offset value, second local extremum with positive time offset value, higher local extremum with positive time offset value, first local extremum with negative time offset value, second local extremum with negative time offset value, and/or higher local extremum with negative time offset value.

A current parameter (e.g. time offset value) may be initialized based on a target value, target profile, trend, past trend, current trend, target speed, speed profile, target speed profile, past speed trend, the motion or movement (e.g. change in position/location) of the object, at least one characteristics and/or STI of the object associated with the movement of object, positional quantity of the object, initial speed of the object associated with the movement of the object, predefined value, initial width of the regression window, time duration, value based on carrier frequency of the signal, value based on subcarrier frequency of the signal, bandwidth of the signal, amount of antennas associated with the channel, noise characteristics, signal h metric, and/or an adaptive (and/or dynamically adjusted) value. The current time offset may be at the center, on the left side, on the right side, and/or at another fixed relative location, of the regression window.

In the presentation, information may be displayed with a map (or environmental model) of the venue. The information may comprise: location, zone, region, area, coverage area, corrected location, approximate location, location with respect to (w.r.t.) a map of the venue, location w.r.t. a segmentation of the venue, direction, path, path w.r.t. the map and/or the segmentation, trace (e.g. location within a time window such as the past 5 seconds, or past 10 seconds; the time window duration may be adjusted adaptively (and/or dynamically); the time window duration may be adaptively (and/or dynamically) adjusted w.r.t. speed, acceleration, etc.), history of a path, approximate regions/zones along a path, history/ summary of past locations, history of past locations of interest, frequently-visited areas, customer traffic, crowd distribution, crowd behavior, crowd control information, speed, acceleration, motion statistics, breathing rate, heart rate, presence/absence of motion, presence/absence of people or pets or object, presence/absence of vital sign, gesture, gesture control (control of devices using gesture), location-based gesture control, information of a location-based operation, identity (ID) or identifier of the respect object (e.g. pet, person, self-guided machine/device, vehicle, drone, car, boat, bicycle, self-guided vehicle, machine with fan, air-conditioner, TV, machine with movable part), identification of a user (e.g. person), information of the user, location/speed/acceleration/direction/motion/gesture/gesture control/ motion trace of the user, ID or identifier of the user, activity of the user, state of the user, sleeping/resting characteristics of the user, emotional state of the user, vital sign of the user, environment information of the venue, weather information of the venue, earthquake, explosion, storm, rain, fire, temperature, collision, impact, vibration, event, door-open event, door-close event, window-open event, window-close event, fall-down event, burning event, freezing event, water-related event, wind-related event, air-movement event, accident event, pseudo-periodic event (e.g. running on treadmill, jumping up and down, skipping rope, somersault, etc.), repeated event, crowd event, vehicle event, gesture of the user (e.g. hand gesture, arm gesture, foot gesture, leg gesture, body gesture, head gesture, face gesture, mouth gesture, eye gesture, etc.).

The location may be 2-dimensional (e.g. with 2D coordinates), 3-dimensional (e.g. with 3D coordinates). The location may be relative (e.g. w.r.t. a map or environmental model) or relational (e.g. halfway between point A and point B, around a corner, up the stairs, on top of table, at the ceiling, on the floor, on a sofa, close to point A, a distance R from point A, within a radius of R from point A, etc.). The location may be expressed in rectangular coordinate, polar coordinate, and/or another representation.

The information (e.g. location) may be marked with at least one symbol. The symbol may be time varying. The symbol may be flashing and/or pulsating with or without changing color/intensity. The size may change over time. The orientation of the symbol may change over time. The symbol may be a number that reflects an instantaneous quantity (e.g. vital sign/ breathing rate/heart rate/gesture/state/status/action/motion of a user, temperature, network traffic, network connectivity, status of a device/machine, remaining power of a device, status of the device, etc.). The rate of change, the size, the orientation, the color, the intensity and/or the symbol may reflect the respective motion. The information may be presented visually and/or described verbally (e.g. using pre-recorded voice, or voice synthesis). The information may be described in text. The information may also be presented in a mechanical way (e.g. an animated gadget, a movement of a movable part).

The user-interface (UI) device may be a smart phone (e.g. iPhone, Android phone), tablet (e.g. iPad), laptop (e.g. notebook computer), personal computer (PC), device with graphical user interface (GUI), smart speaker, device with voice/audio/speaker capability, virtual reality (VR) device, augmented reality (AR) device, smart car, display in the car, voice assistant, voice assistant in a car, etc.

The map (or environmental model) may be 2-dimensional, 3-dimensional and/or higher-dimensional. (e.g. a time varying 2D/3D map/environmental model) Walls, windows, doors, entrances, exits, forbidden areas may be marked on the map or the model. The map may comprise floor plan of a facility. The map or model may have one or more layers (overlays). The map/model may be a maintenance map/model comprising water pipes, gas pipes, wiring, cabling, air ducts, crawl-space, ceiling layout, and/or underground layout. The venue may be segmented/subdivided/zoned/grouped into multiple zones/ regions/geographic regions/ sectors/ sections/ territories/ districts/ precincts/ localities/ neighborhoods/ areas/ stretches/expanse such as bedroom, living room, storage room, walkway, kitchen, dining room, foyer, garage, first floor, second floor, rest room, offices, conference room, reception area, various office areas, various warehouse regions, various facility areas, etc. The segments/regions/areas may be presented in a map/model. Different regions may be color-coded. Different regions may be presented with a characteristic (e.g. color, brightness, color intensity, texture, animation, flashing, flashing rate, etc.). Logical segmentation of the venue may be done using the at least one heterogeneous Type 2 device, or a server (e.g. hub device), or a cloud server, etc.

Here is an example of the disclosed system, apparatus, and method. Stephen and his family want to install the disclosed wireless motion detection system to detect motion in their 2000 sqft two-storey town house in Seattle, Washington. Because his house has two storeys, Stephen decided to use one Type 2 device (named A) and two Type 1 devices (named B and C) in the ground floor. His ground floor has predominantly three rooms: kitchen, dining room and living room arranged in a straight line, with the dining room in the middle. The kitchen and the living rooms are on opposite end of the house. He put the Type 2 device (A) in the dining room, and put one Type 1 device (B) in the kitchen and the other Type 1 device (C) in the living room. With this placement of the devices, he is practically partitioning the ground floor into 3 zones (dining room, living room and kitchen) using the motion detection system. When motion is detected by the AB pair and the AC pair, the system would analyze the motion information and associate the motion with one of the 3 zones.

When Stephen and his family go out on weekends (e.g. to go for a camp during a long weekend), Stephen would use a mobile phone app (e.g. Android phone app or iPhone app) to turn on the motion detection system. When the system detects motion, a warning signal is sent to Stephen (e.g. an SMS text message, an email, a push message to the mobile phone app, etc.). If Stephen pays a monthly fee (e.g. $10/month), a service company (e.g. security company) will receive the warning signal through wired network (e.g. broadband) or wireless network (e.g. home WiFi, LTE, 3G, 2.5G, etc.) and perform a security procedure for Stephen (e.g. call him to verify any problem, send someone to check on the house, contact the police on behalf of Stephen, etc.). Stephen loves his aging mother and cares about her well-being when she is alone in the house. When the mother is alone in the house while the rest of the family is out (e.g. go to work, or shopping, or go on vacation), Stephen would turn on the motion detection system using his mobile app to ensure the mother is ok. He then uses the mobile app to monitor his mother's movement in the house. When Stephen uses the mobile app to see that the mother is moving around the house among the 3 regions, according to her daily routine, Stephen knows that his mother is doing ok. Stephen is thankful that the motion detection system can help him monitor his mother's well-being while he is away from the house.

On a typical day, the mother would wake up at around 7 AM. She would cook her breakfast in the kitchen for about 20 minutes. Then she would eat the breakfast in the dining room for about 30 minutes. Then she would do her daily exercise in the living room, before sitting down on the sofa in the living room to watch her favorite TV show. The motion detection system enables Stephen to see the timing of the movement in each of the 3 regions of the house. When the motion agrees with the daily routine, Stephen knows roughly that the mother should be doing fine. But when the motion pattern appears abnormal (e.g. there is no motion until 10 AM, or she stayed in the kitchen for too long, or she remains motionless for too long, etc.), Stephen suspects something is wrong and would call the mother to check on her. Stephen may even get someone (e.g. a family member, a neighbor, a paid personnel, a friend, a social worker, a service provider) to check on his mother.

At some time, Stephen feels like repositioning the Type 2 device. He simply unplugs the device from the original AC power plug and plug it into another AC power plug. He is happy that the wireless motion detection system is plug-and-play and the repositioning does not affect the operation of the system. Upon powering up, it works right away.

Sometime later, Stephen is convinced that our wireless motion detection system can really detect motion with very high accuracy and very low alarm, and he really can use the mobile app to monitor the motion in the ground floor. He decides to install a similar setup (i.e. one Type 2 device and two Type 1 devices) in the second floor to monitor the bedrooms in the second floor. Once again, he finds that the system set up is extremely easy as he simply needs to plug the Type 2 device and the Type 1 devices into the AC power plug in the second floor. No special installation is needed. And he can use the same mobile app to monitor motion in the ground floor and the second floor. Each Type 2 device in the ground floor/second floor can interact with all the Type 1 devices in both the ground floor and the second floor. Stephen is happy to see that, as he doubles his investment in the Type 1 and Type 2 devices, he has more than double the capability of the combined systems.

According to various embodiments, each CI (CI) may comprise at least one of: channel state information (CSI), frequency domain CSI, frequency representation of CSI, frequency domain CSI associated with at least one sub-band, time domain CSI, CSI in domain, channel response, estimated channel response, channel impulse response (CIR), channel frequency response (CFR), channel characteristics, channel filter response, CSI of the wireless multipath channel, information of the wireless multipath channel, timestamp, auxiliary information, data, meta data, user data, account data, access data, security data, session data, status data, supervisory data, household data, identity (ID), identifier, device data, network data, neighborhood data, environment data, real-time data, sensor data, stored data, encrypted data, compressed data, protected data, and/or another CI. In one embodiment, the disclosed system has hardware components (e.g. wireless transmitter/receiver with antenna, analog circuitry, power supply, processor, memory) and corresponding software components. According to various embodiments of the present teaching, the disclosed system includes Bot (referred to as a Type 1 device) and Origin (referred to as a Type 2 device) for vital sign detection and monitoring. Each device comprises a transceiver, a processor and a memory.

The disclosed system can be applied in many cases. In one example, the Type 1 device (transmitter) may be a small WiFi-enabled device resting on the table. It may also be a WiFi-enabled television (TV), set-top box (STB), a smart speaker (e.g. Amazon echo), a smart refrigerator, a smart microwave oven, a mesh network router, a mesh network satellite, a smart phone, a computer, a tablet, a smart plug, etc. In one example, the Type 2 (receiver) may be a WiFi-enabled device resting on the table. It may also be a WiFi-enabled television (TV), set-top box (STB), a smart speaker (e.g. Amazon echo), a smart refrigerator, a smart microwave oven, a mesh network router, a mesh network satellite, a smart phone, a computer, a tablet, a smart plug, etc. The Type 1 device and Type 2 devices may be placed in/near a conference room to count people. The Type 1 device and Type 2 devices may be in a well-being monitoring system for older adults to monitor their daily activities and any sign of symptoms (e.g. dementia, Alzheimer's disease). The Type 1 device and Type 2 device may be used in baby monitors to monitor the vital signs (breathing) of a living baby. The Type 1 device and Type 2 devices may be placed in bedrooms to monitor quality of sleep and any sleep apnea. The Type 1 device and Type 2 devices may be placed in cars to monitor well-being of passengers and driver, detect any sleeping of driver and detect any babies left in a car. The Type 1 device and Type 2 devices may be used in logistics to prevent human trafficking by monitoring any human hidden in trucks and containers. The Type 1 device and Type 2 devices may be deployed by emergency service at disaster area to search for trapped victims in debris. The Type 1 device and Type 2 devices may be deployed in an area to detect breathing of any intruders. There are numerous applications of wireless breathing monitoring without wearables.

Hardware modules may be constructed to contain the Type 1 transceiver and/or the Type 2 transceiver. The hardware modules may be sold to/used by variable brands to design, build and sell final commercial products. Products using the disclosed system and/or method may be home/office security products, sleep monitoring products, WiFi products, mesh products, TV, STB, entertainment system, HiFi, speaker, home appliance, lamps, stoves, oven, microwave oven, table, chair, bed, shelves, tools, utensils, torches, vacuum cleaner, smoke detector, sofa, piano, fan, door, window, door/window handle, locks, smoke detectors, car accessories, computing devices, office devices, air conditioner, heater, pipes, connectors, surveillance camera, access point, computing devices, mobile devices, LTE devices, 3G/4G/5G/6G devices, UMTS devices, 3GPP devices, GSM devices, EDGE devices, TDMA devices, FDMA devices, CDMA devices, WCDMA devices, TD-SCDMA devices, gaming devices, eyeglasses, glass panels, VR goggles, necklace, watch, waist band, belt, wallet, pen, hat, wearables, implantable device, tags, parking tickets, smart phones, etc.

The summary may comprise: analytics, output response, selected time window, subsampling, transform, and/or projection. The presenting may comprise presenting at least one of: monthly/weekly/daily view, simplified/detailed view, cross-sectional view, small/large form-factor view, color-coded view, comparative view, summary view, animation, web view, voice announcement, and another presentation related to the periodic/repetition characteristics of the repeating motion.

A Type 1/Type 2 device may be an antenna, a device with antenna, a device with a housing (e.g. for radio, antenna, data/signal processing unit, wireless IC, circuits), device that has interface to attach/connect to/link antenna, device that is interfaced to/attached to/connected to/linked to another device/system/computer/phone/network/data aggregator, device with a user interface(UI)/graphical UI/display, device with wireless transceiver, device with wireless transmitter, device with wireless receiver, internet-of-thing (IoT) device, device with wireless network, device with both wired networking and wireless networking capability, device with wireless integrated circuit (IC), Wi-Fi device, device with Wi-Fi chip (e.g. 802.11a/b/g/n/ac/ax standard compliant), Wi-Fi access point (AP), Wi-Fi client, Wi-Fi router, Wi-Fi repeater, Wi-Fi hub, Wi-Fi mesh network router/hub/AP, wireless mesh network router, adhoc network device, wireless mesh network device, mobile device (e.g. 2G/2.5G/3G/3.5G/4G/LTE/ 5G/6G/7G, UMTS, 3GPP, GSM, EDGE, TDMA, FDMA, CDMA, WCDMA, TD-SCDMA), cellular device, base station, mobile network base station, mobile network hub, mobile network compatible device, LTE device, device with LTE module, mobile module (e.g. circuit board with mobile-enabling chip (IC) such as Wi-Fi chip, LTE chip, BLE chip), Wi-Fi chip (IC), LTE chip, BLE chip, device with mobile module, smart phone, companion device (e.g. dongle, attachment, plugin) for smart phones, dedicated device, plug-in device, AC-powered device, battery-powered device, device with processor/memory/set of instructions, smart device/gadget/items: clock, stationary, pen, user-interface, paper, mat, camera, television (TV), set-top-box, microphone, speaker, refrigerator, oven, machine, phone, wallet, furniture, door, window, ceiling, floor, wall, table, chair, bed, nightstand, air-conditioner, heater, pipe, duct, cable, carpet, decoration, gadget, USB device, plug, dongle, lamp/light, tile, ornament, bottle, vehicle, car, AGV, drone, robot, laptop, tablet, computer, harddisk, network card, instrument, racket, ball, shoe, wearable, clothing, glasses, hat, necklace, food, pill, small device that moves in the body of creature (e.g. in blood vessels, in lymph fluid, digestive system), and/or another device. The Type 1 device and/or Type 2 device may be communicatively coupled with: the internet, another device with access to internet (e.g. smart phone), cloud server (e.g. hub device), edge server, local server, and/or storage. The Type 1 device and/or the Type 2 device may operate with local control, can be controlled by another device via a wired/wireless connection, can operate automatically, or can be controlled by a central system that is remote (e.g. away from home).

In one embodiment, a Type B device may be a transceiver that may perform as both Origin (a Type 2 device, a Rx device) and Bot (a Type 1 device, a Tx device), i.e., a Type B device may be both Type 1 (Tx) and Type 2 (Rx) devices (e.g. simultaneously or alternately), for example, mesh devices, a mesh router, etc. In one embodiment, a Type A device may be a transceiver that may only function as Bot (a Tx device), i.e., Type 1 device only or Tx only, e.g., simple IoT devices. It may have the capability of Origin (Type 2 device, Rx device), but somehow it is functioning only as Bot in the embodiment. All the Type A and Type B devices form a tree structure. The root may be a Type B device with network (e.g. internet) access. For example, it may be connected to broadband service through a wired connection (e.g. Ethernet, cable modem, ADSL/HDSL modem) connection or a wireless connection (e.g. LTE, 3G/4G/5G, WiFi, Bluetooth, microwave link, satellite link, etc.). In one embodiment, all the Type A devices are leaf node. Each Type B device may be the root node, non-leaf node, or leaf node.

Type 1 device (transmitter, or Tx) and Type 2 device (receiver, or Rx) may be on same device (e.g. RF chip/IC) or simply the same device. The devices may operate at high frequency band, such as 28GHz, 60GHz, 77GHz, etc. The RF chip may have dedicated Tx antennas (e.g. 32 antennas) and dedicated Rx antennas (e.g. another 32 antennas).

One Tx antenna may transmit a wireless signal (e.g. a series of probe signal, perhaps at 100Hz). Alternatively, all Tx antennas may be used to transmit the wireless signal with beamforming (in Tx), such that the wireless signal is focused in certain direction (e.g. for energy efficiency or boosting the signal to noise ratio in that direction, or low power operation when "scanning" that direction, or low power operation if object is known to be in that direction).

The wireless signal hits an object (e.g. a living human lying on a bed 4 feet away from the Tx/Rx antennas, with breathing and heart beat) in a venue (e.g. a room). The object motion (e.g. lung movement according to breathing rate, or blood-vessel movement according to heart beat) may impact/modulate the wireless signal. All Rx antennas may be used to receive the wireless signal.

Beamforming (in Rx and/or Tx) may be applied (digitally) to "scan" different directions. Many directions can be scanned or monitored simultaneously. With beamforming, "sectors" (e.g. directions, orientations, bearings, zones, regions, segments) may be defined related to the Type 2 device (e.g. relative to center location of antenna array). For each probe signal (e.g. a pulse, an ACK, a control packet, etc.), a channel information or CI (e.g. channel impulse response/CIR, CSI, CFR) is obtained/computed for each sector (e.g. from the RF chip). In breathing detection, one may collect CIR in a sliding window (e.g. 30 sec, and with 100Hz sounding/probing rate, one may have 3000 CIR over 30 sec).

The CIR may have many taps (e.g. N1 components/taps). Each tap may be associated with a time lag, or a time-of-flight (tof, e.g. time to hit the human 4 feet away and back). When a person is breathing in a certain direction at a certain distance (e.g. 4ft), one may search for the CIR in the "certain direction". Then one may search for the tap corresponding to the "certain distance". Then one may compute the breathing rate and heart rate from that tap of that CIR.

One may consider each tap in the sliding window (e.g. 30 second window of "component time series") as a time function (e.g. a "tap function", the "component time series"). One may examine each tap function in search of a strong periodic behavior (e.g. corresponds to breathing, perhaps in the range of 10bpm to 40bpm).

The Type 1 device and/or the Type 2 device may have external connections/links and/or internal connections/links. The external connections (e.g. connection 1110) may be associated with 2G/2.5G/3G/3.5G/4G/LTE/5G/6G/7G/NBIoT, UWB, WiMax, Zigbee, 802.16 etc. The internal connections (e.g., 1114A and 1114B, 1116, 1118, 1120) may be associated with WiFi, an IEEE 802.11 standard, 802.11a/b/g/n/ac/ad/af/ag/ah/ai/aj/aq/ax/ay, Bluetooth, Bluetooth 1.0/1.1/1.2/2.0/2.1/3.0/4.0/4.1/ 4.2/5, BLE, mesh network, an IEEE 802.16/1/1a/1b/2/2a/a/b/c/d/e1f/g/h/i/j/k/l/m/n/o/p/ standard.

The Type 1 device and/or Type 2 device may be powered by battery (e.g. AA battery, AAA battery, coin cell battery, button cell battery, miniature battery, bank of batteries, power bank, car battery, hybrid battery, vehicle battery, container battery, non-rechargeable battery, rechargeable battery, NiCd battery, NiMH battery, Lithium ion battery, Zinc carbon battery, Zinc chloride battery, lead acid battery, alkaline battery, battery with wireless charger, smart battery, solar battery, boat battery, plane battery, other battery, temporary energy storage device, capacitor, fly wheel).

Any device may be powered by DC or direct current (e.g. from battery as described above, power generator, power convertor, solar panel, rectifier, DC-DC converter, with various voltages such as 1.2V, 1.5V, 3V, 5V, 6V, 9V, 12V, 24V, 40V, 42V, 48V, 110V, 220V, 380V, etc.) and may thus have a DC connector or a connector with at least one pin for DC power.

Any device may be powered by AC or alternating current (e.g. wall socket in a home, transformer, invertor, shorepower, with various voltages such as 100V, 110V, 120V, 100-127V, 200V, 220V, 230V, 240V, 220-240V, 100-240V, 250V, 380V, 50Hz, 60Hz, etc.) and thus may have an AC connector or a connector with at least one pin for AC power. The Type 1 device and/or the Type 2 device may be positioned (e.g. installed, placed, moved to) in the venue or outside the venue.

For example, in a vehicle (e.g. a car, truck, lorry, bus, special vehicle, tractor, digger, excavator, teleporter, bulldozer, crane, forklift, electric trolley, AGV, emergency vehicle, freight, wagon, trailer, container, boat, ferry, ship, submersible, airplane, air-ship, lift, mono-rail, train, tram, rail-vehicle, railcar, etc.), the Type 1 device and/or Type 2 device may be an embedded device embedded in the vehicle, or an add-on device (e.g. aftermarket device) plugged into a port in the vehicle (e.g. OBD port/socket, USB port/socket, accessory port/socket, 12V auxiliary power outlet, and/or 12V cigarette lighter port/socket).

For example, one device (e.g. Type 2 device) may be plugged into 12V cigarette lighter/accessory port or OBD port or the USB port (e.g. of a car/truck/vehicle) while the other device (e.g. Type 1 device) may be plugged into 12V cigarette lighter/accessory port or the OBD port or the USB port. The OBD port and/or USB port can provide power, signaling and/or network (of the car/truck/vehicle). The two devices may jointly monitor the passengers including children/babies in the car. They may be used to count the passengers, recognize the driver, detect presence of passenger in a particular seat/position in the vehicle.

In another example, one device may be plugged into 12V cigarette lighter/accessory port or OBD port or the USB port of a car/truck/vehicle while the other device may be plugged into 12V cigarette lighter/accessory port or OBD port or the USB port of another car/truck/vehicle.

In another example, there may be many devices of the same type A (e.g. Type 1 or Type 2) in many heterogeneous vehicles/portable devices/smart gadgets (e.g. automated guided vehicle/AGV, shopping/luggage/moving cart, parking ticket, golf cart, bicycle, smart phone, tablet, camera, recording device, smart watch, roller skate, shoes, jackets, goggle, hat, eye-wear, wearable, Segway, scooter, luggage tag, cleaning machine, vacuum cleaner, pet tag/collar/wearable/implant), each device either plugged into 12V accessory port/OBD port/USB port of a vehicle or embedded in a vehicle. There may be one or more device of the other type B (e.g. B is Type 1 if A is Type 2, or B is Type 2 if A is Type 1) installed at locations such as gas stations, street lamp post, street corners, tunnels, multi-storey parking facility, scattered locations to cover a big area such as factory/ stadium/ train station/ shopping mall/construction site. The Type A device may be located, tracked or monitored based on the TSCI.

The area/venue may have no local connectivity, e.g., broadband services, WiFi, etc. The Type 1 and/or Type 2 device may be portable. The Type 1 and/or Type 2 device may support plug and play.

Pairwise wireless links may be established between many pairs of devices, forming the tree structure. In each pair (and the associated link), a device (second device) may be a non-leaf (Type B). The other device (first device) may be a leaf (Type A or Type B) or non-leaf (Type B). In the link, the first device functions as a bot (Type 1 device or a Tx device) to send a wireless signal (e.g. probe signal) through the wireless multipath channel to the second device. The second device may function as an Origin (Type 2 device or Rx device) to receive the wireless signal, obtain the TSCI and compute a "linkwise analytics" based on the TSCI.

One goal of the present teaching is to enable contactless sleep monitoring on any low-cost, Wi-Fi-enabled IoT devices, including those having only a single antenna and on a 20MHz 2.4GHz channel. The state-of-the-art sleep monitoring algorithm estimates motion and breathing rates to infer and stage sleep. However, sleep estimation only works reliably on 5GHz channels with 40MHz or more bandwidths, and barely works for a 20MHz 2.4GHz channel. In some embodiments, the present teaching resorts to motion detection alone and circumvents the need of breathing estimation for sleep monitoring. Motion detection has demonstrated to be extremely efficient and accurate and can run on any IoTs. The key insight is that people make involuntary body movements during sleep (BMS), such as poster changes, limb movements, head movements, etc. These movements exhibit distinct behavioral patterns that are differentiable from voluntary motions in the day time. Albeit being occasional, instantaneous, and weak, these BMS can still be captured precisely by Wi-Fi-based motion detection. Based on this observation, the present teaching discloses AnySleep, a sleep monitoring system based on body movements during sleep, which can run on any Wi-Fi IoT devices.

In some embodiments, AnySleep first introduces a boosted motion indicator, named micromotion, which can capture tiny BMS motion more reliably than the existing motion statistics, while causing negligible computation overhead. Based on the time series of the detected motions, AnySleep first analyzes the presence and activeness of the user. It then detects BMS by investigating the unique behavioral patterns of typical BMS during night. The system may then calculate a BMS score, which is further refined as a sleep likelihood based on the estimated presence and activeness information. Finally, an iterative searching algorithm is designed to recognize the most likely period of sleep and use a heuristic metric to assess the sleep quality.

Behavioral quiescence, i.e., the absence of voluntary motor behavior, is an essential characteristic of sleep. However, people do move (quite a lot) during sleep, a fact that is elusive and somewhat counter-intuitive. Not only do we change sleep postures, but we also move our head, hands/arms, and legs, etc. All these lead to a considerable amount of movements, which can be termed as Body Movements during Sleep (BMS). According to clinical studies, healthy adults usually experience, in average, 10 times of major posture changes and tens of or even a few hundreds of minor body movements per night, which certainly vary over nights and across subjects.

In some embodiments, the disclosed motion detection may be based on WiDetect, which is the most sensitive and robust motion detector using WiFi signals that has been commercialized as real products. To capture the tiny involuntary motion during sleep, however, the system has enhanced WiDetect for micro motion detection by optimizing the motion sensitivity.

Given a wireless channel between a pair of Wi-Fi devices, the ideal CSI estimation for the subcarrier with frequency f measured at time t can be denoted as: $H(t,f) = \sum_{l=1}^{L} a_l(t)\exp(-j2\pi f\tau_l(t))$, where $a_l(t)$ and $\tau_l(t)$ denote the complex amplitude and propagation delay of the l-th multipath component, respectively. Due to timing and frequency synchronization errors in real Wi-Fi systems, the measured CSI contains significant phase offsets and becomes $\tilde{H}(t,f) = \exp(-j(\alpha(t) + \beta(t)f))H(t,f) + n(t,f)$, where $\alpha(t)$ and $\beta(t)$ are the random initial and linear phase offsets at time t, respectively. For brevity, H(t,f) is still used for the real measurement $\tilde{H}(t,f)$ hereafter.

As the phase information is severely distorted and is non-trivial to calibrate, one can mainly exploit the measured CSI amplitude for motion detection. Nevertheless, the derived motion statistics can be directly extended to incorporate phase information if it is properly cleaned. Denote the power response of a CSI measurement H(t,f) as $G(t,f) \triangleq |H(t,f)|^2$. WiDetect defines a novel metric within the range of [0,1] to indicate the existence and strength of surrounding motion, which is termed as motion statistic and can be calculated from the auto-correlation function (ACF) of the CSI. Specifically, the motion statistic on subcarrier f is defined as (omitting the notion of time t for brevity):

$$\phi(f) = \lim_{\tau\to 0}\rho_G(\tau,f) \triangleq \rho_G\left(\tau = \frac{1}{F_s}, f\right).$$

Putting it simply, in presence of motion, one can have $\lim_{\tau\to 0}\rho_G(\tau,f) > 0$; while if there is no motion in the environment, $\lim_{\tau\to 0}\rho_G(\tau,f) = 0$. Therefore, $\lim_{\tau\to 0}\rho_G(\tau,f)$ provably, and also practically, embodies a good indicator for the presence of motion, which is only determined by the power response of the motion and the measurement noises and is independent of the environments, locations, orientations, and subjects, etc. In the above equation, one can use the first sample of the ACF, $\rho_G\left(\tau = \frac{1}{F_s}, f\right)$, as an approximation of $\lim_{\tau\to 0}\rho_G(\tau,f)$ since the channel sampling rate is limited in practice. In some embodiments, the average over all subcarriers is taken as the overall motion statistic

$\phi = \frac{1}{F}\sum_{f=1}^{F}\phi(f)$ so that a proper threshold for motion detection can be theoretically determined.

Depending on the placement of the devices and the distances from the subject to the transceivers, the averaged motion statistic may not be super sensitive to very tiny motion (e.g., small body movements during sleep) due to CSI measurement noises. Therefore, the system can maximize the motion signals so that the AnySleep system can reliably sense body motions during sleep. In some embodiments, maximal ratio combining (MRC) can be performed to optimally combine the motion statistics calculated on individual subcarriers, instead of the equal gain averaging. MRC is a general diversity fusion method that maximizes the SNR by combining multiple receiving signals in an optimal way, which has been widely used in wireless communication and, recently, wireless sensing. Particularly, the ACF on different subcarriers are inherently synchronized and independent from the time origin and that the noise variances for different subcarriers are the same, thus satisfying the conditions to apply MRC. Given a fixed number of F subcarriers, the motion signal will thus be maximized by combining the motion statistics as follows:

$$\varrho_G(\tau, f) =$$

$$\sum_{f=1}^{F} w(f)\rho_G(\tau, f),$$

where w(f) denotes the optimal weight for combining multiple subcarriers, which depends on the channel gain. The calculated motion statistic itself can serve as the channel gain for each subcarrier, and therefore can be used as the optimal weights for combining, i.e.,

$$\varrho_G(\tau, f) = \sum_{f=1}^{F} \rho_G\left(\tau = \frac{1}{F_s}, f\right)\rho_G(\tau, f).$$

Then the boosted motion statistic for micro motion detection can be, again, estimated as the first sample of the maximized ACF

$$\varphi = \varrho_G\left(\tau = \frac{1}{F_s}, f\right).$$

In practice, instead of fusing all F subcarriers, one may sort all $\rho_G(t, f)$ and select the K subcarriers of the largest motion statistics for MRC. This would further increase the values of the combined

$$\varrho_G(\tau, f).$$

One issue for the boosted signal is about the noise level in absence of motion. As mentioned earlier, the averaged motion statistic $\phi$ approximates 0 if no motion presents, and thus a theoretical threshold can be effectively obtained for motion detection. The optimized micro motion statistic $\varphi$, however, not only boosts the motion signal but also enlarges the noises (including environmental dynamics of non-interest) to some extent. Therefore, the value of $\varphi$ does not necessarily approximate 0 in empty environment, but will be at some level greater than 0 depending on the device noises and environmental conditions. Fortunately, one can devise a mechanism to automatically and adaptively estimate the noise level and determine an appropriate threshold for micro motion detection.

FIG. 1 illustrates an example of sleep data, which shows that the disclosed micromotion boosts the motion values compared to the regular motion statistics, underpinning a foundation for reliable and continuous BMS detection.

One disclosed approach to track sleep period is to identify the body movements during sleep. The body movements during sleep can exhibit distinct patterns that can be differentiated from activity motions in the daytime. Therefore, the system can recognize the sleep period by detecting the time period with the BMS patterns. The key challenge, however, is how to detect BMS accurately, robustly, and efficiently.

FIG. 2 illustrates an example of body movements during sleep. As seen, compared with daily motions, BMS appear to be (1) transient: motions occur due to BMS but usually only last for a very short time, and the environment is otherwise quiet with no motion; (2) significant: motions due to BMS mostly cause quite high motion statistics, outstanding in the mostly quiet sleep time and comparable to large motions in the daytime; and (3) sparse: the moments with body movements during sleep are sparse throughout the entire sleep period. Consequently, motions due to BMS exhibit spiky and sparse patterns, which turn out to be unique characteristics for BMS detection.

One can formulate BMS detection as a spiky peak detection problem. Accounting for the above properties of BMS, one can devise the following constraints for the BMS motion peak detection. First, a minimum peak height means the maximum motion is required to be larger than a minimum peak height. Second, a maximum peak width means the formed motion peak due to one time of BMS should be narrow and spiky. In other words, the peak should appear and then vanish within a short time of w seconds, where w denotes the maximum peak width. Third, an isolation means there should be only one peak at a time as BMS are sparse and noncontinuous. Fourth, the motion level beyond a potential peak should be low, ideally around noise level. This is to reflect the fact of motion absence except for body movements during sleep.

FIG. 2 illustrates possible peaks in the case of empty environment, sleep, and daily active motion, respectively. As seen, the BMS in sleep time exhibits a unique pattern compared with empty and motion cases.

To minimize computation, one can adopt an iterative approach for the disclosed BMS detection. Specifically, one can examine one condition at a time and will stop once a criterion is not met. The disclosed peak detection algorithm may not be perfect but is sufficient for BMS detection. First, not all possible BMS will be detected. For example, extremely small BMS (e.g., mouth movements) may be missing. Second, some motions caused by daytime activities may be detected as well. However, the goal is not to detect every single body movements of sleep but to detect the sleep period. The disclosed BMS detection underpins this goal even with some missing and false detections.

In some embodiments, the system can also perform presence detection and activity detection, both based on the (micro) motion statistics. The purpose is to eliminate the periods without human presence or with activities for sleep consideration. Apparently, sleep can only happen when a human subject stays quiet without voluntary activities.

Presence detection is to detect whether or not a user presents in the space. This can be done by examining the captured motion levels since a human subject cannot stay completely still for a long time. Whenever there are some voluntary motions, they will be reflected by the motion statistics. Evan when a user is sleeping, the potential unconscious BMS can trigger micro motion as well. This is different from the case when the environment is empty without any moving targets, in which case both the motion and micro motion estimation will be around zero.

One can employ a simple yet effective rule for presence detection. First, the system can perform motion detection by fusing motion and micro motion. Specifically, one can have $I_M(t) = \mathbb{1}[\phi(t) > \eta | \varphi(t) > \zeta]$ for any time t, where $\eta$ and $\zeta$ are the thresholds for motion and micro motion detection, respectively. Then presence indicator is a simple expansion of motion detection. One can apply a sliding window $W_P$ over time, and presence is claimed for the entire window if any motion is detected within it. This is based on the empirical observation that human cannot appear/disappear suddenly and $W_P$ is accordingly determined as a few minutes, e.g., 10 minutes.

Activity is defined when a user is being active, resulting in relatively intensive motion. Therefore, activity detection is based on the ratio of detection motion within a certain time window $W_A$. Specifically, activities are marked for a window as long as the motion ratio $\Sigma_{t \in W_A} I_M(t)/|W_A|$ is greater than a threshold ratio. $|W_A|$ denotes the window length, which can be normally chose as a few minutes (e.g., 5 minutes).

FIG. 4 is an example of the estimated presence and activities throughout a whole day of 24 hours.

In some embodiments, one can define a minimum unit of sleep as $W_U$ to indicate the shortest nap people would normally take. The value of $W_U$ can be determined empirically and quite arbitrarily as, for example, 15 minutes assuming a meaningful sleep lasts for at least such long. Then the system can break down the time into many of such units and examine the sleep likelihood of each of these units. The more BMS-like motions and the less high and consecutive motions, the more likely a sleep occurs. Correspondingly, the sleep likelihood estimation is also based on the motion statistics and the BMS detections.

Considering a unit time $W_U\langle T\rangle$ ending at time T, the system can first calculate a BMS score for the unit as $B_U(t) = N_{BMS}(W_U\langle T\rangle)/|W_U|$, where $|W_U|$ is the length in seconds of the window $W_U\langle T\rangle$ and $N_{BMS}(W_U\langle t\rangle)$ denotes the amount of detected BMS within the $N_U$ seconds of window $W_U\langle T\rangle$. FIG. 3 shows an example of the calculated BMS score. As seen, although there can be non-zero BMS scores in the day time, the BMS scores corresponding to the actual sleep period are outstandingly higher than other periods, thanks to the denser BMS.

Then the system can calculate a sleep likelihood $L(t) = (1 - M_I(t)) \times B(t)$, where $M_I(t)$ is the motion intensity at time t, which is the averaged value of the motion statistic $\Phi(t)$ over a certain window. The entire time window $W_U\langle t\rangle$ features the same BMS score $B_U(t)$ for every time point t. Importantly, the BMS score is set to zero for any time with activity or without presence, since, again, sleep and thus BMS are unlikely to happen if a user is voluntarily active or absent.

FIG. 5 illustrates an example of the estimated sleep likelihood. One can further incorporate two factors for a better estimation. First, one can quantize the sleep likelihood as binary values by using the minimum likelihood value as an adaptive threshold: $L^b(t) = \mathbb{1}[L(t) > l]$, where $\mathbb{1}[\cdot]$ is an indicator function. $1 = \min(L(t), \forall t \in W_O\langle T\rangle)$ denotes the sleep likelihood threshold for quantization, where $W_O\langle T\rangle$ is a larger observation window that ends at time T and potentially covers the entire actual sleep period. Second, one can define a motion penalty based on the motion detection results.

The motion penalty differs from the motion intensity as the former only applies to the time moments when motion is detected (i.e., $\phi(t) > \eta$), while the latter is the averaged motion statistic $\phi(t)$ that is always there for every single time point t. With the above two additional factors, the final sleep likelihood for each time point t then becomes $L'(t) = L(t) + L^b(t) - pI_M(t)$, where p is the constant motion penalty in presence of motion and $I_M(t) = \mathbb{1}[\phi(t) > \eta]$ is an indicator function of motion presence. FIG. 5 illustrates an example of the final sleep likelihood estimation. For brevity, L(t) is still used for L'(t) hereafter.

Given an observation window $W_O\langle T\rangle$, one can have a time series of sleep likelihood values $\{L(t), t \in W_O\langle T\rangle\}$. The window $W_O\langle T\rangle$ is a larger one, e.g., one day of motion data, such that it can cover the potential sleep period, if any. The remaining task is to recognize the entire sleep period based on the estimated sleep likelihood. The system can achieve so by finding the period $P^\star$ within $W_O\langle T\rangle$ that maximizes the integral sleep likelihood. One can first identify k initial periods, each of one-hour segments that feature the largest integral sleep likelihood values. Then the sleep recognition algorithm starts from each of the identified segment and iteratively expands to former and later time, with a step size s. The reason that the system initially chooses top-k segments for searching is because that the largest one, in rare cases, may be a false high value and fall out of the actual sleep period. The total sleep likelihood of a period $P_i$ is calculated as the sum of the sleep likelihood over time, i.e., $Q(P_i) = \Sigma_{t \in P_i} L(t)$.

The sleep durations of the majority of people fall in a common range, e.g., around 7 to 8 hours per day. Therefore, to avoid unrealistic long or short sleep periods, the system can adjust the original total sleep likelihood Q by considering the typical distribution of normal sleep. Specifically, the system can build a Gaussian distribution based on the normal sleep time reported in medical literature . Then if the period $P_i$ being considered is out of the 3-$\sigma$ zone of the normal distribution, an attenuation factor of the corresponding probability will be applied to the calculated total score $Q(P_i)$.

Finally, the most likely period $P^\star$ is found as the one that maximizes the total score $Q(P^\star)$. $P^\star$ will be claimed as a detected sleep period if the total score $Q(P^\star)$ is greater than a certain threshold. In some embodiments of AnySleep, at most one sleep period will be detected for a given window $W_O\langle T\rangle$. In case a user wakes up during a sleep and then sleeps again, the two periods of sleep will be automatically combined as one sleep and the wake-up time will be reflected in the corresponding sleep summary, as detailed next. An example of sleep recognition is shown in FIG. 5.

For the recognized sleep periods, several sleep-related properties are assessed and reported. These include start time (i.e., time to bed/sleep), end time (i.e., wake up time), sleep duration, awake time during sleep, wake-up times during a sleep, etc. Most properties are straight-forward to calculate based on the detected sleep period. Awake time is estimated as the durations which observe activities during sleep, while wake-up times are number of non-consecutive activity periods detected. One can also calculate a sleep score to indicate the sleep quality. Computing a sleep score is a complicated task, which is even more challenging when one only has motion information. Despite rich research on BMS, there lacks an established formula to calculate sleep score from the BMS. In AnySleep, based on the intuition that the longer one sleeps and the more quiet (i.e., less motion) one experiences, the higher sleep score one could have, the present teaching discloses an empirical formula as below:

$$S_{sleep} = \min\left(\left(\frac{100}{8}D - \alpha M_a\right) * (1 - M_w), 100\right)$$

where D is the sleep time in hours, $M_a = \sum_{t \in W_O} \mathbb{1}[\phi(t) > \eta_{low}]$ denotes the motion-indicated wakeness, and $M_w = \sum_{t \in W_O} \mathbb{1}[\phi(t) > \eta_{high}]/(D \times 3600)$ indicates the motion activeness. As the motion statistic is mostly independent of the environment, one can empirically set the low motion threshold $\eta_{low} = 0.35$ and the high one as $\eta_{high} = 0.5$. $\alpha$ is a constant weighting factor that is empirically determined such that the calculated sleep scores are mostly in a reasonable range especially for normal sleep of healthy subjects.

The above shows how to detect a possible sleep period, given a particular time window with motion observations. In practice, the motion data will enter in real-time, and thus the algorithm needs to run every once a while (e.g., every 10 minutes, depending on user preferences) to update the sleep tracking results. To do so, the system can slide the observation window $W_O$ over time and takes the latest data ending at the current time point as input. Given any window $W_O$, the disclosed sleep tracking algorithm will either detect and output a potential sleep period or announce no sleep therein. As the sliding step is much smaller than the window size, the sliding windows will overlap and lead to potential conflicting detection results. In other words, multiple time windows covering the same sleep period, entirely or partially, may output different sleep information.

To handle sleep tracking in real-time system and address the above issues, consider a window $W_O\langle T\rangle$ ending at time T, and assume a sleep period $P_{W_O}$ is detected. As mentioned earlier, $P_{WO}$ should contain the detected time to bed (i.e., starting time), time to wake up (i.e., end time), other sleep summary information, and particularly, a sleep likelihood score of the detected sleep period. The disclosed AnySleep system can maintain a list of the detected sleep periods, denoted as $\mathbb{P} = \{P_{W_O}(T_1), \cdots, P_{W_O}(T_N)\}$ where $P_{W_O}(T_i)$ is the detected sleep period given observation window $W_O\langle T_i\rangle$. In some embodiments, AnySleep then performs online updating to confirm one and only one most likely sleep period for a given time period.

Specifically, every time when a new sleep period $P_{W_O}(T_c)$ is detected, e.g., one for the latest time window ending at current time $T_c$, the sleep period will be compared with all previously detected periods that overlap with $P_{W_O}(T_c)$. $P_{W_O}(T_c)$ will be added into the history list $\mathbb{P}$ only if its sleep likelihood score is the maximum among those of all its overlapped sleep periods. In such cases, all the overlapped sleep periods of $P_{W_O}(T_c)$ will become outdated and thus removed from $\mathbb{P}$ . Otherwise, $P_{W_O}(T_c)$ will simply be discarded and $\mathbb{P}$ remains unchanged. By doing so, the periods maintained in $\mathbb{P}$ become the most likely detections to date and will be reflected to users upon real-time user queries. Finally, a sleep segment in $\mathbb{P}$ will be finally confirmed and no longer updated if the corresponding end time becomes earlier than the starting time of the current observations window, $W_O\langle T_c\rangle$, as no further sleep periods will be possible to be detected for the concerned time period.

In some embodiments, while AnySleep system involves quite some parameters and thresholds, the system can determine some key parameters automatically and adaptively.

First, for empty level of micromotion, one can safely assume that, over a sufficiently long time of observation (e.g., a full day), there will be at least some time the user is absent or still/quasi-still. Such periods can be treated as the empty case, and the corresponding motion measurements can be utilized to estimate the reference empty level for micromotion. To do so, the system will find a certain period throughout the day that experiences the lowest motion and none of the motion statistics exceed the threshold (i.e., no motion occurs). The system may then estimate the average motion within this period as the empty micromotion level, which will be then subtracted from the original micromotion estimates.

Second, for motion/micromotion threshold, the system can determine an adaptive motion/micromotion threshold with reference to the motion level when there is no user present. Therefore, the system can still find the most likely "empty" period throughout the day. Instead of using the average motion level, the system now detects the maximum motion statistics/micromotion of the empty window as the adaptive threshold for motion/micromotion.

Third, for leveraging history, although the motion/micromotion levels in empty environments may vary a little in different environments and on different devices, the values should be consistent once a specific system has been deployed at a certain site. Therefore, in practical deployment, the estimated empty levels, thresholds, and other parameters should not change too vastly over time. Inspired by this observation, one can update the parameters in a more conservative way by considering historical estimates. Specifically, for any parameter $\gamma$, the system can update its latest value as below: $\gamma_i = \beta_\gamma \gamma_{i-1} + (1 - \beta_\gamma)\hat{\gamma}_i$, where $\hat{\gamma}_i$ is the estimate based on the current observations, $\gamma_{i-1}$ denotes the previously determined value, and $\beta_\gamma$ is a low-pass averaging factor. By doing so, the system can also avoid outlying values produced by the automatic parameter selection (e.g., wrong motion threshold can be estimated if the users are highly active throughout a certain day).

FIG. 6 illustrates an exemplary scenario where object motion or sleep motion is detected in a venue, according to one embodiment of the present teaching. FIG. 6 shows a setup that can be used for WiDetect, AnySleep, or any motion detection system described herein. For example, as shown in FIG. 6, in a 2-bedroom apartment 600, Origin 601 may be placed in the living-room area 602, Bot 1 610 may be placed in a bedroom1-area 612, and Bot 2 620 may be placed in the dining-room area 622. Each of Bot 1 610 and Bot 2 620 can transmit a wireless signal to the Origin 601, which can obtain channel information of a wireless multipath channel based on the wireless signal. The Origin 601, by itself or through a third device like a motion detector, can compute motion information based on the channel information and detect object/user motion/activity based on the motion information. That is, the Origin 601, by itself or through a third device like a motion detector, can detect object/user motion/activity based on wireless signals transmitted by Bot 1 610 and/or Bot 2 620.

In some embodiments, if object motion is detected based on wireless signals transmitted by both Bot 1 610 and Bot 2 620, the activity/motion or the object (e.g. person/user) may be in the living-room area 602. If object motion, e.g. sleep motion, is detected based only on wireless signals transmitted by Bot 1 610, the activity/motion or the object (e.g. person/user) may be in the bedroom-1 area 612. If object motion/activity is detected based only on wireless signals transmitted by Bot 2 620, the activity/motion or the object (e.g. person/user) may be in the dining-room area 622. If object motion/activity cannot be detected based on wireless signals transmitted by either Bot 1 610 or Bot 2 620, then it may be determined that nobody and no object is in the apartment 600.

FIG. 7 illustrates an exemplary method 700 for radio-based sleep tracking, according to some embodiments of the present disclosure. As shown in FIG. 7, the method 700 is performed by a sleep tracking module 701, which includes: an automatic threshold selection module 710, a body movement detection module 720, a sleep likelihood estimation module 730, a sleep period recognition module 740, a sleep score calculation module 750, and a sleep staging module 760.

The automatic threshold selection module 710 can take inputs of motion statistics, micromotion data, and optionally breathing rate, based on a wireless motion detection, e.g. as shown in FIG. 6. In some embodiments, the sleep tracking module 701 can take inputs with arbitrary time lengths, but would produce the best results if the inputs cover the entire sleep period. In some embodiments, the disclosed method does not require any input (e.g., regular bedtime, # of people) from a user. After the body movement detection module 720 and the sleep likelihood estimation module 730, a sleep period can be recognized at the sleep period recognition module 740. In some embodiments, the sleep period recognition module 740 can generate outputs including: a went-to-bed time, a woke-up time, and a sleep duration of the monitored and tracked sleep motion. The sleep score calculation module 750 can calculate and generate a sleep score as an output, e.g. to indicate a quality of the sleep. In some embodiments, the sleep staging module 760 may determine sleep staging by generating outputs including: wake up times and light/deep sleep stages.

In some embodiments, a two-person sleeping can be treated indifferently, and the method 700 may just output one shared time-to-bed and wake-up time. In some embodiments, the method 700 can support both 5GHz and 2.4GHz wireless signals for motion detection, while 5GHz wireless signal has a better performance.

FIG. 8 illustrates an exemplary unified method 800 for sleep monitoring and tracking, according to some embodiments of the present disclosure. Compared to the method 700 in FIG. 7, the method 800 includes a determination step 802, where a breathing rate estimation (bratio) in a certain time period is calculated based on inputs of motion statistics, mocromotion data, and optionally breathing rate, and then compared to a threshold bratio_th. If bratio > bratio_th, which means there is a good amount of breathing rate, the method 800 goes to a sleep monitoring process 804. If not, the method 800 goes to the sleep tracking process which may be performed as described above with respect to the method 700 in FIG. 7.

FIG. 9 illustrates a flow chart of an exemplary method 900 for radio-based sleep tracking, according to some embodiments of the present disclosure. In various embodiments, the method 900 can be performed by the systems disclosed above and herein. At operation 902, a first wireless signal is transmitted through a wireless multipath channel in a venue. At operation 904, a second wireless signal is received through the wireless multipath channel, the second wireless signal differing from the first wireless signal due to the wireless multipath channel impacted by a sleeping motion of an object in the venue. At operation 906, a time series of channel information (TSCI) of the wireless multipath channel is obtained based on the second wireless signal, wherein each channel information (CI) of the TSCI comprises N1 components, wherein N1 is a positive integer larger than one. At operation 908, N1 component-wise analytics each associated with one of the N1 components of the TSCI are computed. At operation 910, N2 largest component-wise analytics are identified among the N1 component-wise analytics, wherein N2 is a positive integer less than N1. At operation 912, at least one first motion statistics is computed based on the N2 largest component-wise analytics of the TSCI. At operation 914, the sleeping motion of the object is monitored based on the at least one first motion statistics. The order of the operations in FIG. 9 may be changed according to various embodiments of the present teaching.

In some embodiments, the present teaching discloses systems and methods for sleep tracking using two motion statistics but without using breathing statistics. An exemplary method includes the following steps. First, the system can compute motion statistics (MS), for each pair of TX-RX. Then, the system can compute micro motion statistics (MM), for each TX-RX link. The system may perform automatic (e.g. dynamic, adaptive, or periodic) selection of thresholds for MS and MM. For any time instance, the system can compute MM-residue by subtracting LI from MM, where the LI or "MM empty-mrc-motion-level" may be an estimate of DC level of MM when the object (e.g. person or user) is absent and the venue is "quiet" in terms of MM. This may be used in a preliminary screen of the presence of the object. It may be min{ mean(MM[w]): all window w in a period}, where the window size may be 30, 60 or 90 minutes, and the "mean" here may be a weighted mean. In some embodiments, L1 may be updated periodically (e.g. once a day). It may be a weighted average of the current value and the past (or even future) few values (e.g. it may be low-pass filtered for continuity, perhaps to suppress noise).

In some embodiment, for any time instance, the system can detect motion if either MS>T1 or MM>T2. The T1 or "MS threshold" or "T_MS" may be an estimate of MS level when the object is absent. It may be max {MS[arg min(mean(MS[w]))]}. It may be to compute the mean of each sliding window (e.g. 5, 10, 15, 20, 25, or 30, etc. minutes) of MS in a period (e.g. 24 hours), identify the sliding window with the smallest mean which should be a "quiet" window with no person/user present, compute max of MS in the window. The T_MS may be adjusted by subtracting or adding a quantity. The T2 or "MM threshold" or "T_MM" may be an estimate of MM level when the object is absent. It may be max {MM[arg min(mean(MM[w]))]}.

For any sliding time window, the system can compute a presence of object in a sliding window if motion is detect in any time stance in the sliding window. For any sliding time window, the system can detect an activity in the sliding window if the amount of time instance in the sliding window with motion detected exceeds a threshold (e.g. 30% of all time instances in the sliding window). The system can identify peaks of MM such that (i) peak MM>T3, (ii) "right_base" - "left_base"<T4. (i.e. identify all peaks and then eliminate those that do not satisfy both (i) and (ii).) The goal is to find peaks corresponding to movements naturally occurring during sleep, or "body-motion-during-sleep" (i.e. body motion such as body rolling/turning or hand/head/body motion).

The T3 or "min_peak_height" is a threshold to qualify sleep motion. Basically a legitimate sleep motion cannot be too small. It may be min[mean(sorted_MM_res[1:k]), 0.5], wherein k may be 50. Within a period (e.g. 24 hours), the threshold may be a mean of the largest k (e.g. 50) MM. The "right_min" or "right_base" is the time of first minimum point on the right hand side for the neighboring MM below the T3. The "left_min" or "left_base" is the time of first minimum point on the left hand side for the neighboring MM below the T3. The T4 or "min_base_gap" is another threshold to qualify sleep motion. Basically a legitimate sleep motion cannot be too long in time duration (e.g. end within 2 seconds). The right_min - left_min is a measure of the "duration" of the motion associated with the MM peak.

Optionally, the system can eliminate peaks s.t. a peak-prominence measure (a measure of height of a peak relative to lowest contour) is larger than a threshold. Optionally, the system can eliminate or merge neighboring peaks that are close to each other (e.g. within 10 minutes). In some embodiments, the system can eliminate a peak if no presence of object (defined above) is detected in a (small) window around the peak. In some embodiments, the system can eliminate a peak if activity (defined above) is detected beyond the "left_base" and the "right_base" within a (larger) window around the peak, e.g. the window around the peak at time t, from (t-delta) to (t+delta). In some embodiments, the system can eliminate a peak if it is the only peak (e.g. singular peak) within a long time period (e.g. 90 minutes, 2 hours, 3 hours).

For each time instance of the remaining peak, the system can compute a body-movement-during-sleep indicator (BMS) as 1 if a remaining peak occurs at the time, and 0 if no remaining peak occur at the time. For each sliding window (e.g. 30 minute long), the system can count the remaining peaks in the sliding window and compute a BMS score ("BMS_score") as the percentage of time instances in the sliding window being the remaining peaks. It may be sum(BMS[w])/w. For each time instance, the system can compute a sleep likelihood score (SL) based on the BMS and a motion intensity measure. It may be (1-motion_intensity)*BMS_score. Likelihood should be higher if there are more BMS.

For each time instance, the system can compute a sleep indicator (SI) as 1 if SL >T5 and 0 otherwise, where T5 may be min(SL(SL>0)), i.e. a minimum among the non-zero SL). For each time instance, the system can compute a test score based on SL, and SI. It may be SL+SI-motion_penalty. Within a day, the system can find the 1-hour period with the highest sum of test score as an initial estimate of a sleep period.

The system may expand the sleep period to the left and to the right recursively (e.g. expand to right, then to left, then to right, and so on) with an increment step of Delta_T. When total duration of sleep period is approaching or beyond a normal range (e.g. 8/9 hours; the value may be adaptively adjusted based on a recognition of the user, e.g. 8/9/10 hours for a child, 7/8/9 hours for an adult, or 5/6 hours for an older person; the value may be adjusted based on the day of the week, e.g. longer for weekends or holidays and shorter for week days), the test score may be reduced s.t. it is e.g. Delta_T may be 5, 10, 15, or etc. (minutes). Recursion may stop when total sum of test score start to decrease (i.e. when test score is negative).

In some embodiments, the system can compute sleep analytics such as sleep start time (beginning time of sleep period), sleep end time (end time of sleep period), sleep duration (length of sleep period), interruption during sleep (number of times interruption occur during sleep period), interruption duration during sleep (or "awake_time_during_sleep", which is the amount of time during the sleep period that activity is detected), wakeup_times, and/or activity_times.

The following numbered clauses provide implementation examples for radio-based sleep tracking.

Clause 1. A method/device/system/software of a radio-based sleep tracking system, comprising: transmitting a wireless signal from a Type1 heterogeneous wireless device of the system through a wireless multipath channel in a venue, wherein the wireless multipath channel is impacted by a sleeping motion of an object in the venue; receiving the wireless signal by a Type2 heterogeneous wireless device of the system through the wireless multipath channel, wherein the received wireless signal differs from the transmitted wireless signal due to the wireless multipath channel and the sleeping motion of the object; obtaining a time series of channel information (TSCI) of the wireless multipath channel based on the received wireless signal using a process, a memory and a set of instructions, wherein each channel information (CI) of the TSCI comprises N1 components; computing N1 component-wise analytics each associated with one of the N1 components of the TSCI; identifying N2 largest component-wise analytics; computing a first motion statistics based on the N2 largest component-wise analytics of the TSCI; monitoring the sleeping motion of the object based on the first motion statistics.

In some embodiments, CI may be CSI, CFR, CIR. The N1 components may be subcarrier values of CFR, or tap values of CIR. The component-wise analytics may be term-by-term multiplication of corresponding component For example, let X(t1) and X(t2) be two CI at time t1 and t2, each CI being N1-tuples. The i^th component-wise analytics may be X1(i)*X2(i), where x(i) and y(i) are the i^{th} components of X(t1) and X(t2). It may be an estimate of component-wise auto-correlation function. The analytics may also be the magnitude, or a magnitude feature (e.g. a function of the magnitude), of the X1(i)*X2(i). It may be a weighted average of multiple pairs of (t1, t2), e.g. (1,2), (2,3), (3,4) with the same time difference (i.e. t2-t1). In some embodiments, the first motion analytics may be "micro motion" (MM). It may be an average or weighted average of a feature of N2 largest component-wise analytics. The feature may be absolute value, magnitude, magnitude square, etc. The object may be a person. The "sleep motion" may include motion when the person is sleep, and also motion when the person is not sleep, or even absent in the venue. When the person is absent, there may be no motion, i.e. the sleep motion may be NIL or zero.

Clause 2. The method/device/system/software of the radio-based sleep tracking system of clause 1, further comprising: identifying the N2 largest component-wise analytics by one of the following: (a) thresholding the N1 component-wise analytics based on a threshold; or (b) sorting N1 component-wise analytics to find the N2 largest component-wise analytics.

Clause 3. The method/device/system/software of the radio-based sleep tracking system of clause 1 or 2, further comprising: computing a time series of the first motion statistics, each first motion statistics associated with a time stamp; computing a set of potential body-motion-during sleep (BMS), each potential BMS (PBMS) being a local maximum point or a local peak of the time series of first motion statistics; monitoring the sleeping motion of the object based on the set of PBMS.

Clause 4. The method/device/system/software of the radio-based sleep tracking system of clause 3, further comprising: performing a BMS test on a PBMS and the time series of first motion statistics, removing a PBMS from the set of PBMS if the PBMS fails the BMS test.

Clause 5. The method/device/system/software of the radio-based sleep tracking system of clause 4, further comprising: performing the BMS test based on a magnitude feature of the PBMS, wherein the PBMS fails the BMS test if the local peak of the first motion statistics associated of the PBMS has the magnitude feature less than a threshold.

Clause 6. The method/device/system/software of the radio-based sleep tracking system of clause 5, further comprising: computing the threshold adaptively based on at least one of: a weighted mean of the magnitude feature of a number of largest first motion statistics in a period of time and a predefined quantity.

Clause 7. The method/device/system/software of the radio-based sleep tracking system of clause 4, further comprising: performing the BMS test based on a width measure associated with the PBMS, wherein the PBMS fails the BMS test if the width measure is larger than a threshold.

Clause 8. The method/device/system/software of the radio-based sleep tracking system of clause 6 or 7, further comprising: computing a left minimum point which is the nearest minimum point to the left of PBMS in the time series of first motion statistics; computing a right minimum point which is the nearest minimum point to the right of PBMS in the time series of first motion statistics; computing the width measure as a time difference between the left minimum point and the right minimum point.

Clause 9. The method/device/system/software of the radio-based sleep tracking system of clause 6 or 7, further comprising: computing a left drop point which is a nearest point to the left of PBMS in the time series of first motion statistics when the first motion statistics has a magnitude feature below a first target value; computing a right drop point which is a nearest point to the right of PBMS in the time series of first motion statistics when the first motion statistics has a magnitude feature below a second target value; computing the width measure as a time difference between the left drop point and the right drop point.

Clause 10. The method/device/system/software of the radio-based sleep tracking system of clauses 4 to 9: wherein at least one of: the first target value and the second target value is one of the following: a predefined threshold, or an adaptive threshold based on a peak magnitude feature of the first motion statistics at the PBMS.

Clause 11. The method/device/system/software of the radio-based sleep tracking system of clauses 4 to 10: wherein the adaptive threshold is one of: a difference of the peak magnitude feature and a predefined quantity, or a fraction of the peak magnitude feature.

Clause 12. The method/device/system/software of the radio-based sleep tracking system of clauses 4 to 11, further comprising: performing the BMS test based on a height measure associated with the PBMS, wherein the PBMS fails the BMS test if the height measure is less than a threshold.

Clause 13. The method/device/system/software of the radio-based sleep tracking system of clauses 4 to 12, further comprising: computing at least one of: a left minimum or a right minimum, wherein the left minimum point is a first adjacent minimum point to the left of the PBMS in the time series of first motion statistics, wherein the right minimum point is a first adjacent minimum point to the right of the PBMS in the time series of first motion statistics; computing the height measure based on at least one of: a difference of a magnitude feature of the first motion statistics between the PBMS and the left minimum point, a difference of the magnitude feature of the first motion statistics between the PBMS and the right minimum point, a quotient of another magnitude feature of the first motion statistics between the PBMS and the left minimum point, a quotient of the another magnitude feature of the first motion statistics between the PBMS and the right minimum point.

Clause 14. The method/device/system/software of the radio-based sleep tracking system of clauses 4 to 13, further comprising: computing a left drop point which is a point to the left of PBMS in the time series of first motion statistics at a first time difference from the PBMS; computing a right drop point which is a point to the right of PBMS in the time series of first motion statistics at a second time difference from the PBMS; computing the height measure based on at least one of: a difference of a magnitude feature of the first motion statistics between the PBMS and the left drop point, a difference of the magnitude feature of the first motion statistics between the PBMS and the right drop point, a quotient of another magnitude feature of the left drop point and the PBMS, or a quotient of the another magnitude feature of the right drop point and the PBMS.

Clause 15. The method/device/system/software of the radio-based sleep tracking system of clauses 4 to 14, further comprising: computing a width measure and a height measure associated with the PBMS; computing a prominence measure based on an increasing function of the height measure and a decreasing function of the width measure, wherein the PBMS fails the BMS test if the prominence measure is less than a threshold.

Clause 16. The method/device/system/software of the radio-based sleep tracking system of clause 15, further comprising: computing the prominence measure based on at least one of: a quotient, a difference or a comparison of the height measure and the width measure.

Clause 17. The method/device/system/software of the radio-based sleep tracking system of clauses 4 to 16, further comprising: computing a left drop point which is a point to the left of PBMS in the time series of first motion statistics at a first time difference from the PBMS; computing a left boundary point which is a point to the left of PBMS in the time series of first motion statistics at a second time difference from the PBMS; computing a right drop point which is a point to the right of PBMS in the time series of first motion statistics at a third time difference from the PBMS; computing a right boundary point which is a point to the right of PBMS in the time series of first motion statistics at a fourth time difference from the PBMS; performing the BMS test based on a neighborhood dominance measure computed based on the left drop point, the left boundary point, the right drop point and the right boundary point, wherein the PBMS fails the BMS test if the neighborhood dominance measure is larger than a threshold.

Clause 18. The method/device/system/software of the radio-based sleep tracking system of clause 17, further comprising: computing the neighborhood dominance measure based on at least one of: a maximum of a magnitude feature of the first motion statistics between the left drop point and the left boundary point, a maximum of the magnitude feature of the first motion statistics between the right drop point and the right boundary point, a percentile point of the magnitude feature of the first motion statistics between the left drop point and the left boundary point, a percentile point of the magnitude feature of the first motion statistics between the right drop point and the right boundary point, a weighted average of a number of percentile points of the magnitude feature of the first motion statistics between the left drop point and the left boundary point, a weighted average of a number of percentile points of the magnitude feature of the first motion statistics between the right drop point and the right boundary point.

Clause 19. The method/device/system/software of the radio-based sleep tracking system of clause 17 or 18: wherein at least one of the following is true: the second time difference is equal to the fourth time difference, the first time difference is equal to the third time difference, both the first time difference and the third time different are predefined quantities, both the second time difference and the fourth time different are predefined quantities, the left drop point is the nearest minimum point to the left of PBMS in the time series of first motion statistics, the right drop point is the nearest minimum point to the right of PBMS in the time series of first motion statistics, the left boundary point is the nearest minimum point to the left of PBMS in a time series of lowpass-filtered first motion statistics, the right boundary point is the nearest minimum point to the right of PBMS in the time series of lowpass-filtered first motion statistics.

Clause 20. The method/device/system/software of the radio-based sleep tracking system of clauses 4 to 19, further comprising: detecting a presence of the object in a time period around the PBMS based on the first motion statistics in the time period; performing the BMS test based on the presence detection of the object, wherein the PBMS fails the BMS test if presence of the object is not detected in the time period.

Clause 21. The method/device/system/software of the radio-based sleep tracking system of any previous clause, further comprising: detecting a motion of the object at each time in the time period based on the first motion statistics; wherein the presence of the object is detected in the time period if the motion of the object is detected at any time during the time period.

Clause 22. The method/device/system/software of the radio-based sleep tracking system of clause 21: wherein the motion of the object is detected at a time if the first motion statistics at that time is larger than a first threshold or a second motion statistics at that time computed based on the TSCI is larger than a second threshold.

Clause 23. The method/device/system/software of the radio-based sleep tracking system of any previous clause: for either of the first or second motion statistics, computing the respective threshold based on at least one of: a filtering of the time series of the motion statistics, a weighted averaging of the motion statistics, a minimization after the filtering, a minimization after the weighted averaging, a particular sliding time window associated with at least one of: the minimization after the filtering, or the minimization after the weighted averaging, a maximum of the motion statistics in the particular sliding time window.

Clause 24. The method/device/system/software of the radio-based sleep tracking system of clauses 4 to 23, further comprising: detecting a non-sleeping activity of the object in a time period around the PBMS based on the first motion statistics in the time period; performing the BMS test based on the non-sleeping activity detection of the object, wherein the PBMS fails the BMS test if non-sleeping activity of the object is detected in the time period.

Clause 25. The method/device/system/software of the radio-based sleep tracking system of any previous clause, further comprising: detecting a motion of the object at each time in the time period based on the first motion statistics, wherein the motion of the object is detected at a time if the first motion statistics at that time is larger than a first threshold or a second motion statistics at that time computed based on the TSCI is larger than a second threshold; computing a percentage of time in the time period at which the motion of the object is detected, wherein the non-sleeping activity of the object is detected in the time period if the percentage is larger than a threshold.

Clause 26. The method/device/system/software of the radio-based sleep tracking system of clauses 4 to 25, further comprising: performing the BMS test based on a time difference between the PBMS and an immediate past PBMS, wherein the PBMS fails the BMS test if the time difference is less than a threshold.

Clause 27. The method/device/system/software of the radio-based sleep tracking system of clauses 4 to 26, further comprising: performing the BMS test based on a time difference between the PBMS and a neighboring PBMS, merging the PBMS and the neighboring PBMS if the time difference is less than a threshold.

Clause 28. The method/device/system/software of the radio-based sleep tracking system of clauses 4 to 26, further comprising: computing a time series of sleep likelihood (TSSL) for a period of time, each sleep likelihood (SL) associated with a time, wherein each SL is computed based on a motion intensity at the time and a count of PBMS in a time period associated with the time; computing a time series of sleep indicator (TSSI) for the period of time, each sleep indicator (SI) based on a comparison of the respective SL with a threshold, monitoring the sleep motion of the object based on the TSSL and the TSSI.

Clause 29. The method/device/system/software of the radio-based sleep tracking system of clause 28, further comprising: computing a time series of testing score (TS) for the period of time, each TS based on the SL, the SI and a penalty for large motion intensity; partitioning the period of time into a number of non-overlapping time units; computing a sum of test scores for each time unit; among the time units, identifying a time unit with the largest sum of test scores and initializing a sleep period as the time unit and a total testing score (TTS) as the associated largest sum of test scores; iteratively expanding the sleep period by adding an adjoining incremental time window either to the right or to the left of the sleep period and updating the TTS by adding the TS associated with the increment time window to TTS; in each iteration, adding a penalty to the TTS if the sleep period has a duration approaching or exceeding a typical sleep duration of the object; stopping the iteration based on a stopping criterion; monitoring the sleeping motion of the object based on the sleep period, the TTS, and the TS, the PBMS and the first motion statistics in the sleep period.

Clause 30. The method/device/system/software of the radio-based sleep tracking system of clause 29, further comprising: computing a sleep analytics based on the sleep period, the TTS, and the TS, the PBMS and the first motion statistics in the sleep period, wherein the sleep analytics comprises at least one of: a sleep starting time, a beginning time of the sleep period, a sleep waking-up time, an ending time of the sleep period, a sleep duration, a total duration of the sleep period, an amount of interruption during sleep, a count of non-sleep activities during the sleep period, a count of toilet visits during sleep period, a total duration of interrupts during sleep, a total time duration of non-sleep activities during the sleep period, a total time duration of toilet visits during the sleep period, an amount of body motion, a count of PBMS during the sleep period, an amount of awake time during sleep, a duration of awake time during sleep, a sleep score, a sleep quality score, a motion factor, a motion wakeness, a percentage of time or an amount of time that a first motion analytics, a second motion analytics computed based on the TSCI, a SL, a SI, a TS, a derivative or an integration is greater than a threshold during the sleep period, a percentage of time or an amount of time that a first motion analytics, a second motion analytics, a SL, a SI, a TS, a derivative or an integration is less than a threshold.

Clause 31. The method/device/system/software of the radio-based sleep tracking system of any previous clause, comprising: wherein each of the N1 component-wise analytics is a pair-wise analytics based on a pair of CI of the TCSI; computing each of the N1 component-wise analytics based on the respective components of the pair of CI of the TSCI.

Clause 32. The method/device/system/software of the radio-based sleep tracking system of clause 31, comprising: computing each component-wise analytics based on a multiplication of the respective components of the pair of CI.

Clause 33. The method/device/system/software of the radio-based sleep tracking system of clause 32, comprising: wherein each component-wise analytics is an estimate of a component-wise correlation of the respective components of the pair of CI.

Clause 34. The method/device/system/software of the radio-based sleep tracking system of clause 31 to 33, comprising: wherein each of the N1 component-wise analytics is a pairwise analytics based on multiple pairs of CI of the TCSI; computing each of the N1 component-wise analytics based on the multiple pairs of CI.

Clause 35. The method/device/system/software of the radio-based sleep tracking system of clause 34, comprising: computing each component-wise analytics based on a weighted average of a number of multiplicative product of the respective components of each of the multiple pairs of CI.

Clause 36. The method/device/system/software of the radio-based sleep tracking system of clause 35, comprising: wherein all of the multiple pairs of CI have a common time difference between the pair of CI; wherein each component-wise analytics is an estimate of a component-wise correlation of the respective components of the pair of CI associated with the common time difference.

Clause 37. The method/device/system/software of the radio-based sleep tracking system of clause 34, comprising: wherein the multiple pairs of CI are consecutive or adjacent in time.

Clause 38. The method/device/system/software of the radio-based sleep tracking system of any previous clause, further comprising: computing a time series of the first motion statistics; computing a baseline value of the first motion statistics in a period of time based on the first motion statistics of the time series in the period of time; subtracting the baseline value from each first motion statistics in the period of time.

Clause 39. The method/device/system/software of the radio-based sleep tracking system of clause 38, further comprising: wherein there are more than one periods of time; computing more than one of the baseline value of the first motion statistics, one for each period of time; computing an aggregated baseline value associated with a particular time period based on an aggregation of the more than one baseline values; subtracting the aggregated baseline value from each first motion statistics in the particular period of time.

The following numbered clauses provide some examples for radio-based sleep tracking.

Clause A1. A system for radio-based sleep tracking, comprising: a transmitter configured to transmit a first wireless signal through a wireless multipath channel in a venue; a receiver configured to receive a second wireless signal through the wireless multipath channel, wherein the second wireless signal differs from the first wireless signal due to the wireless multipath channel which is impacted by a sleeping motion of an object in the venue; and a processor configured for: obtaining a time series of channel information (TSCI) of the wireless multipath channel based on the second wireless signal, wherein each channel information (CI) of the TSCI comprises N1 components, wherein N1 is a positive integer larger than one, computing N1 component-wise analytics each associated with one of the N1 components of the TSCI, identifying N2 largest component-wise analytics among the N1 component-wise analytics, wherein N2 is a positive integer less than N1, computing at least one first motion statistics based on the N2 largest component-wise analytics of the TSCI, and monitoring the sleeping motion of the object based on the at least one first motion statistics.

Clause A2. The system of Clause A1, wherein the N2 largest component-wise analytics are identified by one of the following: comparing each of the N1 component-wise analytics to a threshold; or sorting the N1 component-wise analytics to find the N2 largest component-wise analytics.

Clause A3. The system of Clause A1 or A2, wherein the processor is further configured for: computing a time series of first motion statistics, each first motion statistics associated with a time stamp; computing a set of potential body-motion-during sleep (BMS), each potential BMS (PBMS) being a local maximum point or a local peak of the time series of first motion statistics; and monitoring the sleeping motion of the object based on the set of PBMS.

Clause A4. The system of Clause A3, wherein the processor is further configured for: performing a BMS test on each PBMS in the set of PBMS and the time series of first motion statistics; removing a PBMS from the set of PBMS when the PBMS fails the BMS test 5. The system of Clause A4, wherein: the BMS test is performed based on a magnitude feature of each PBMS, wherein the PBMS fails the BMS test when the local peak of a first motion statistics associated of the PBMS has the magnitude feature less than a first threshold; and the first threshold is computed adaptively based on at least one of: a weighted mean of the magnitude feature of a number of largest first motion statistics in a period of time and a predefined quantity.

Clause A6. The system of Clause A5, wherein: the BMS test is performed based on a width measure associated with each PBMS, wherein the PBMS fails the BMS test when the width measure is larger than a second threshold.

Clause A7. The system of Clause A6, wherein the width measure is computed based on: computing a left drop point which is a nearest point to the left of PBMS in the time series of first motion statistics when the first motion statistics has a magnitude feature below a first target value; computing a right drop point which is a nearest point to the right of PBMS in the time series of first motion statistics when the first motion statistics has a magnitude feature below a second target value; and computing the width measure as a time difference between the left drop point and the right drop point.

Clause A8. The system of Clauses A3 to A7, wherein at least one of the first target value or the second target value is an adaptive threshold based on a peak magnitude feature of the first motion statistics at the PBMS.

Clause A9. The system of Clauses A4 to A8, wherein: the BMS test is performed based on a height measure associated with each PBMS, wherein the PBMS fails the BMS test when the height measure is less than a third threshold.

Clause A10. The system of Clause A9, wherein the height measure is computed based on: computing at least one of: a left minimum point or a right minimum point, wherein the left minimum point is a first adjacent minimum point to the left of the PBMS in the time series of first motion statistics, wherein the right minimum point is a first adjacent minimum point to the right of the PBMS in the time series of first motion statistics; and computing the height measure based on at least one of: a difference of a first magnitude feature of the first motion statistics between the PBMS and the left minimum point, a difference of the first magnitude feature of the first motion statistics between the PBMS and the right minimum point, a quotient of a second magnitude feature of the first motion statistics between the PBMS and the left minimum point, a quotient of the second magnitude feature of the first motion statistics between the PBMS and the right minimum point.

Clause A11. The system of Clause A9 or A10, wherein the processor is further configured for: computing a prominence measure based on an increasing function of the height measure and a decreasing function of the width measure, wherein the PBMS fails the BMS test when the prominence measure is less than a fourth threshold; 12. The system of Clause A11, wherein the processor is further configured for: computing a left dropping point which is a point to the left of PBMS in the time series of first motion statistics at a first time difference from the PBMS; computing a left boundary point which is a point to the left of PBMS in the time series of first motion statistics at a second time difference from the PBMS; computing a right dropping point which is a point to the right of PBMS in the time series of first motion statistics at a third time difference from the PBMS; computing a right boundary point which is a point to the right of PBMS in the time series of first motion statistics at a fourth time difference from the PBMS; and performing the BMS test based on a neighborhood dominance measure computed based on the left dropping point, the left boundary point, the right dropping point and the right boundary point, wherein the PBMS fails the BMS test when the neighborhood dominance measure is larger than a fifth threshold.

Clause A13. The system of Clauses A4 to A12, wherein the processor is further configured for: detecting a presence of the object in a time period around the PBMS based on a first motion statistics in the time period; and performing the BMS test based on the detecting, wherein the PBMS fails the BMS test when presence of the object is not detected in the time period.

Clause A14. The system of any previous A Clause, wherein the processor is further configured for: detecting a motion of the object at each time in the time period based on the first motion statistics, wherein the presence of the object is detected in the time period when the motion of the object is detected at any time during the time period, wherein the motion of the object is detected at a time when the first motion statistics at that time is larger than a sixth threshold or a second motion statistics at that time computed based on the TSCI is larger than a seventh threshold.

Clause A15. The system of any previous A Clause, wherein the processor is further configured for: detecting a non-sleeping activity of the object in a time period around the PBMS based on the first motion statistics in the time period, wherein the non-sleeping activity of the object is detected in the time period when a percentage of time in the time period at which the motion of the object is detected is larger than an eighth threshold; and performing the BMS test based on the non-sleeping activity detection of the object, wherein the PBMS fails the BMS test when non-sleeping activity of the object is detected in the time period.

Clause A16. The system of Clauses A4 to A15, wherein the processor is further configured for: performing the BMS test based on a time difference between the PBMS and a neighboring PBMS, wherein the PBMS fails the BMS test when the time difference is less than a ninth threshold; and merging the PBMS and the neighboring PBMS when the time difference is less than a tenth threshold.

Clause A17. The system of any previous A Clause, wherein the processor is further configured for: computing a time series of sleep likelihood (TSSL) for a period of time, each sleep likelihood (SL) associated with a time, wherein each SL is computed based on a motion intensity at the time and a count of PBMS in a period of time associated with the time; computing a time series of sleep indicator (TSSI) for the period of time, each sleep indicator (SI) is computed based on a comparison of a respective SL with a threshold; and monitoring the sleep motion of the object based on the TSSL and the TSSI.

Clause A18. The system of Clause A17, wherein the processor is further configured for: computing a time series of testing score (TS) for the period of time, each TS based on the SL, the SI and a penalty for large motion intensity; partitioning the period of time into a number of non-overlapping time units; computing a sum of test scores for each time unit; among the non-overlapping time units, identifying a time unit with the largest sum of test scores, initializing a sleep period as the time unit, and initializing a total testing score (TTS) as the associated largest sum of test scores; iteratively expanding the sleep period by adding an adjoining incremental time window either to the right or to the left of the sleep period and iteratively updating the TTS by adding the TS associated with the increment time window to TTS; in each iteration, adding a penalty to the TTS when the sleep period has a duration approaching or exceeding a typical sleep duration of the object; stopping the iteration based on a stopping criterion; and monitoring the sleeping motion of the object based on the sleep period, the TTS, the TS, the PBMS and the first motion statistics in the sleep period.

Clause A19. The system of Clause A18, wherein the processor is further configured for: computing a sleep analytics based on the sleep period, the TTS, the TS, the PBMS and the first motion statistics in the sleep period.

Clause A20. The system of any previous A Clause, wherein: each of the N1 component-wise analytics is a pair-wise analytics based on a pair of CI of the TCSI; and each of the N1 component-wise analytics is computed based on respective components of the pair of CI of the TSCI.

Clause A21. The system of Clause A20, wherein the processor is further configured for: computing each component-wise analytics based on a multiplication of the respective components of the pair of CI.

Clause A22. The system of Clause A21, wherein: each component-wise analytics is an estimate of a component-wise correlation of the respective components of the pair of CI.

Clause A23. The system of Clauses A20 to A22, wherein: each of the N1 component-wise analytics is a pair-wise analytics based on multiple pairs of CI of the TCSI; and each of the N1 component-wise analytics is computed based on the multiple pairs of CI.

Clause A24. The system of Clause A23, wherein the processor is further configured for: computing each component-wise analytics based on a weighted average of a number of multiplicative product of the respective components of each of the multiple pairs of CI.

Clause A25. The system of Clauses A23 to A24, wherein: all of the multiple pairs of CI have a common time difference between the pair of CI; each component-wise analytics is an estimate of a component-wise correlation of the respective components of the pair of CI associated with the common time difference; and the multiple pairs of CI are consecutive or adjacent in time.

Clause A26. The system of any previous A Clause, wherein the processor is further configured for: computing a baseline value for a first time period based on the time series of first motion statistics in the first time period; and subtracting the baseline value from each first motion statistics in the first time period.

Clause A27. The system of Clause A26, wherein the processor is further configured for: computing a plurality of baseline values, each of which is computed for a corresponding time period; computing an aggregated baseline value associated with a particular time period based on an aggregation of the plurality of baseline values; and subtracting the aggregated baseline value from each first motion statistics in the particular time period.

Clause A28. A wireless device of a system for radio-based sleep tracking, comprising: a processor; a memory communicatively coupled to the processor; and a receiver communicatively coupled to the processor, wherein: an additional wireless device of the system is configured to transmit a first wireless signal through a wireless multipath channel in a venue, the receiver is configured to receive a second wireless signal through the wireless multipath channel, the second wireless signal differs from the first wireless signal due to the wireless multipath channel which is impacted by a sleeping motion of an object in the venue, and the processor is configured for: obtaining a time series of channel information (TSCI) of the wireless multipath channel based on the second wireless signal, wherein each channel information (CI) of the TSCI comprises N1 components, wherein N1 is a positive integer larger than one, computing N1 component-wise analytics each associated with one of the N1 components of the TSCI, identifying N2 largest component-wise analytics among the N1 component-wise analytics, wherein N2 is a positive integer less than N1, computing at least one first motion statistics based on the N2 largest component-wise analytics of the TSCI, and monitoring the sleeping motion of the object based on the at least one first motion statistics.

Clause A29. The wireless device of Clause A28, wherein the processor is further configured for: computing a time series of first motion statistics, each first motion statistics associated with a time stamp; computing a set of potential body-motion-during sleep (BMS), each potential BMS (PBMS) being a local maximum point or a local peak of the time series of first motion statistics; monitoring the sleeping motion of the object based on the set of PBMS; performing a BMS test on each PBMS in the set of PBMS and the time series of first motion statistics; and removing a PBMS from the set of PBMS when the PBMS fails the BMS test.

Clause A30. A method for radio-based sleep tracking, comprising: transmitting a first wireless signal through a wireless multipath channel in a venue; receiving a second wireless signal through the wireless multipath channel, wherein the second wireless signal differs from the first wireless signal due to the wireless multipath channel which is impacted by a sleeping motion of an object in the venue; obtaining a time series of channel information (TSCI) of the wireless multipath channel based on the second wireless signal, wherein each channel information (CI) of the TSCI comprises N1 components, wherein N1 is a positive integer larger than one; computing N1 component-wise analytics each associated with one of the N1 components of the TSCI; identifying N2 largest component-wise analytics among the N1 component-wise analytics, wherein N2 is a positive integer less than N1; computing at least one first motion statistics based on the N2 largest component-wise analytics of the TSCI; and monitoring the sleeping motion of the object based on the at least one first motion statistics.

Because the presence of a human subject will affect the RF signal propagation, e.g., RF signals reflected from the human body will be modulated by the body movement such as the chest movement caused by respiration and heartbeat, vital information of the human subject can be unveiled by analyzing the channel propagation characteristics. Although RF signals can be used to estimate the Respiration Rate (RR) and the Heart Rate (HR), one cannot obtain the Heart Rate Variability (HRV) from RR and HR, without the precise timing of each heartbeat. An accurate HRV estimation is much more difficult than HR estimation. The HR estimating systems usually take multiple samples in the time domain to achieve higher HR estimation accuracy, which equals to averaging the heartbeats over a certain time window. However, they are not applicable for HRV estimation which needs the exact time of each heartbeat and entails the following challenges. First, RF signals reflected by human chests are modulated by both respiration and heartbeats in which the distance change caused by respiration is a magnitude greater than that caused by heartbeats. In signal process terminology, the Signal-to-Interference-plus-Noise Ratio (SINR) is very low to recover and separate the heartbeat wave from the compound signal. Second, the heart pumping motion has to reach the chest wall through bones and tissues first and then be detected by the RF signal. As a result, the bones and tissues of a human body act as a filter and thus dampen the signal. Therefore, the heartbeat wave captured by RF signals lacks sharp peaks as those in ECG signals, making it harder to identify IBIs. Furthermore, to provide a robust system for HRV estimation, it is necessary to determine the number of targets and their locations before estimating HRV for each human subject, which is non-trivial as well.

The present teaching discloses a multi-person HRV estimation system (referred to as "mmHRV" hereinafter) using a Commodity Off-The-Shelf (COTS) millimeter-Wave (mmWave) radio. In some embodiments, a target detector is devised to identify the number of users and their locations without any prior calibration. Due to the fast attenuation of the mmWave RF signal, the strength of the signal decreases as it traverses a longer distance. To detect human subjects at various distances, the mmHRV can employ a two-dimension constant false alarm detector in the range-azimuth plane to estimate the noise level, and thus provide an adaptive threshold for target detection. The phase information is further used to filter out the static objects (e.g., walls, furniture). There may be more than one reflecting point for a single human subject. As a result, to determine the number of targets, mmHRV can further employ a non-parametric clustering to identify the range-azimuth bins corresponding to each human subject.

In some embodiments, after target detection, to estimate the HRV, the heartbeat wave needs to be extracted from the composite received signal whose phase includes the whole chest motion including both the respiration and heartbeat movements. In some embodiments, the respiration movement ranges from 4 to 12 mm with a frequency of 6 to 30 Breaths Per Minute (BPM) while the heartbeat movement ranges from 0.2 to 0.5 mm with a frequency of 50 to 120 BPM, both of which are quasi-periodic signals. Leveraging this property, mmHRV can utilize a heartbeat wave extractor, which optimizes the decomposition of the composite signal to several band-limited signal components. Among the decomposed signal components, the heartbeat wave will be the one whose amplitude and frequency satisfy the requirement of a typical heartbeat signal. Compared with approaches about successively decomposing the composite signal, mmHRV can avoid the error propagation problem by concurrently decomposing the signal components. In addition, the mmHRV system can work in a multi-user case by target detection, to monitor HRV and/or other statistics of heartbeat signals of multiple persons at the same time.

The peaks of the estimated heartbeat wave are then recognized to identify the exact time of each heartbeat. Consequently, the IBIs can be further derived and used for calculating the commonly used HRV metrics such as the Root Mean Square of Successive Differences (RMSSD), the standard deviation of all the IBIs (SDRR), and the percentage of successive IBIs that differ by more than 50ms (pNN50).

FIG. 10A illustrates an exemplary setup for a wireless vital monitoring system, e.g. the mmHRV system, according to some embodiments of the present disclosure. As shown in FIG. 10A, the mmHRV system includes a device 1000 with a transmitter (Tx) antenna array 1001 and a receiver (Rx) antenna array 1002. In some embodiments, the device 1000 operates at high frequency band, such as 28GHz, 60GHz, 77GHz, with a bandwidth of 3 to 5GHz. To obtain channel information, the Tx 1001 can transmit, using one or more antennas, a wireless signal, which is received by different Rx antennas 1002 after reflected by the objects and human beings in a venue shown in FIG. 10A.

As shown in FIG. 10A, there may be multiple objects, including static object 1008 and human subjects 1005, 1006, 1007, in the venue where the device 1000 is located. The mmHRV system can monitor HRV for multiple persons 1005, 1006, 1007 in the venue at the same time, with or without other static objects 1008 in the venue, based on channel information obtained from the reflected signal by the Rx 1002. Different objects may be located at different directions from the device 1000, without impacting the effective operation of the mmHRV system. For example, human subject 1007, human subject 1005, and chair 1008 are located at different azimuth angles from the device 1000. Different objects may also be located at different distances from the device 1000, without impacting the effective operation of the mmHRV system. For example, human subject 1005 and human subject 1006 are located at different distance ranges (but at the same azimuth angle) from the device 1000. Different human subjects may face different directions in the venue, without impacting the effective operation of the mmHRV system. For example, human subjects 1005, 1006, 1007 are facing different directions as shown in FIG. 10A.

In some embodiments, the Tx 1001 is a Bot as described above; and the Rx 1002 is an Origin as described above. While the Tx 1001 and the Rx 1002 are physically coupled to each other in FIG. 10A, they may be separated in different devices in other embodiments. In some embodiments, the device 1000 serves like a radar.

In some embodiments, to evaluate the performance of mmHRV system, 11 participants aging from 20 to 60 are asked to perform extensive experiments under different settings, including different distances, orientation and incidental angles. The Non-Light-of-Sight (NLOS) scenario and multi-person scenario are also investigated. In some embodiments, experimental results show that the mmHRV achieves accurate estimations with a medium error of about 28ms for IBI estimations (w.r.t. 96.16% accuracy). The Root-Mean-Square-Error (RMSE) of the NLOS and the multi-user case are still within 32ms and 69ms respectively. The HRV metrics are also evaluated, which show a better performance compared with the state-of-art works. The mmHRV can achieve 3.89ms average error of mean IBI, 6.43ms average error of RMSSD, 6.44ms average error of SDRR, and 2.52% average error of the pNN50 when users sit 1 meter away from the device.

The mmHRV system is a wireless system that can accurately detect the heartbeat signal of human subjects and estimate their HRV by purely using the RF signals reflected off the users' bodies. FIG. 10B illustrates an exemplary processing workflow of the mmHRV system, according to some embodiments of the present disclosure. In some embodiments, the mmHRV system utilizes a Frequency-Modulated Continuous Wave (FMCW) radar to transmit the RF signal and capture the reflections of human subjects and static objects.

As shown in FIG. 10B, channel information is obtained at operation 1010 based on the captured reflections by the Rx. In order to detect human subjects at different locations, a beamforming is performed at operation 1020, e.g. by a Bartlett beamformer, to get the channel information at different azimuth-range bins. Then, target detection is performed at operation 1030, e.g. by a target detector to adaptively estimate the noise level at various distances and azimuth angles and thus detect the presence of reflecting objects. The variance of phase is further utilized to distinguish human subjects and static objects. To identify the number of target and their locations, a non-parametric clustering algorithm may be employed.

At operation 1040, to extract the heartbeat signal from the phase information that is modulated by both respiration and heartbeat, the mmHRV may devise a heartbeat signal extractor, which can decompose the phase signal into several narrow-band signals concurrently and give an estimate of heartbeat wave. The HRV of the detected human subject can be further analyzed at operation 1050 based on the Inter-Beat Intervals (IBIs) derived from the estimated heartbeat signals.

Signal Model: In some embodiments, a chirp signal is transmitted by the FMCW radar, where the instantaneous transmitting frequency is a periodic linearly-increasing signal as shown in FIG. 11, and it can be expressed as

$$f_t = f_c + \frac{B}{T_c} t, \qquad (1)$$

where $f_c$ is the chirp starting frequency, $T_c$ is the chirp duration and $B$ is the bandwidth. According to Frequency Modulation (FM), the transmitted signal $x_T(t)$ can be expressed as

$$\begin{aligned} x_T(t) &= A_T \exp\{-j[2\pi \int_0^t f_t(\tau) d\tau]\} \\ &= A_T \exp\{-j[2\pi f_c t + \pi \frac{B}{T_c} t^2]\}, \end{aligned} \qquad (2)$$

where $A_T$ is the transmitting power. When the electromagnetic (EM) wave is reflected by human chest at distance d(t), the reflected signal $x_R(t)$ can be expressed as

$$x_R(t) = A_R \exp\{-j[2\pi f_c (t - t_d) + \pi \frac{B}{T_c} (t - t_d)^2]\}, \qquad (3)$$

where $A_R$ is the amplitude of the receiving signal. $t_d$ stands for the round-trip delay and can be denoted as $t_d = \frac{2d(t)}{c}$, where c is the speed of light.

Mixing the received signal with a replica of the transmitted signal and following a low-pass filter, the channel information $h(t)$ can be expressed as

$$h(t) = A \exp\{-j(2\pi \frac{B t_d}{T_c} t + 2\pi f_c t_d - \pi \frac{B}{T_c} t_d^2)\}. \qquad (4)$$

Note that the term $\pi \frac{B}{T_c} t_d^2$ is negligible, especially in short-range scenarios. Therefore, the $h(t)$ can be written as

$$h(t) = A \exp\{-j(2\pi \frac{B t_d}{T_c} t + 2\pi f_c t_d)\}, \qquad (5)$$

which is a sinusoidal signal whose frequency $f_b \triangleq \frac{B t_d}{T_c} = \frac{2Bd(t)}{cT_c}$ depends on the target's distance. For each chirp, the baseband signal $h(t)$ is digitized by Analog-to-Digital Converter (ADC), producing N samples per chip, referred to as fast time. The time corresponding to the transmission of chirps is referred to as slow time, as shown in FIG. 11. Therefore, the digitized channel information for the $n^{th}$ ADC sample and $m^{th}$ chirp can be expressed as

$$h(n, m) = A \exp\{-j(2\pi f_b n T_f + \frac{4\pi d(nT_f + mT_s)}{\lambda_c})\}, \qquad (6)$$

where $T_f$ and $T_s$ are the time interval in fast time and slow time respectively. $\lambda_c$ denotes the wavelength of the chirp.

In some embodiments of mmHRV, one may take advantage of the multiple antennas of the chipset, and use 2 Tx antennas and 4 Rx antennas, as shown in FIG. 12. To increase the azimuth resolution, the chirps are transmitted in the time-division multiplexing (TDM) mode by transmitting sequentially through two Tx antennas. This is equivalent to the 8-element virtual array as shown in FIG. 12. Therefore, for channel $l$, the channel information can be rewritten as

$$h(l,n,m) \quad = A\exp\{-j(2\pi f_b n T_f + \frac{4\pi d(nT_f+mT_s)}{\lambda_c} + 2\pi\frac{d_l \sin\theta}{\lambda_c})\}, \tag{7}$$

where $d_l$ is the relative distance introduced by virtual antenna $l$. $\theta$ is the azimuth angle of the target as shown in FIG. 12.

The phase of the channel information changes periodically in slow time due to the periodic motions of respiration and heartbeat. FIG. 14A shows a typical phase signal containing vital signs collected by the system.

For practical application, target detection needs to be performed before vital sign detection. The target detection is hard to achieve, especially in the indoor scenario, where there are various objects (e.g., wall, desk, metal objects, etc.) with strong reflections of EM waves.

Range-FFT and Digital Beamforming: In some embodiments, the channel information for the case when there is a static object is:

$$h(l,n,m) = A\exp\{-j(2\pi f_b n T_f + \frac{4\pi d_0}{\lambda_c} + 2\pi\frac{d_l \sin\theta}{\lambda_c})\}, \tag{8}$$

where $d_0$ is the distance between the object and the device, which stays constant in slow time.

The channel information corresponding to the reflecting object is a periodic signal in fast time, and the periodicity is related to the distance as shown in Eqn. (6) and Eqn. (8). To determine the range information of reflecting objects, Fast Fourier Transform (FFT) may be performed over the fast time for each chirp, i.e., range-FFT, and the channel information can be written as $h_r(l, m)$, where r is the range tap index. The range taps corresponding to the reflecting objects would observe larger energy compared with that without reflecting objects.

To further determine the azimuth angles of the reflecting objects, digital beamforming is performed over all antenna elements for each range tap, and the channel information corresponding to range r and azimuth angle $\theta$ can be expressed as

$$h_{r,\theta}(m) = s^H(\theta) h_{r,l}(m) + \varepsilon(m), \tag{9}$$

where $s^H(\theta)$ is the steering vector towards angle $\theta$. In some embodiments of mmHRV, a Bartlett beamformer is adopted, where the coefficient of the $l$-th antenna is

$$s_l(\theta) = \exp(-j2\pi\frac{d_l \sin\theta}{\lambda_c}). \tag{10}$$

$\varepsilon(m)$ is the additive white Gaussian noise assumed to be independent and identically distributed (I.I.D) for different range-azimuth bins. $\mathbf{h}_{r,l}(m) = [h_{r,1}(m), h_{r,2}(m), ..., h_{r,L}(m)]$ is the channel information vector at range tap r overall all antenna elements. Therefore, for each sample $m$ in slow time, one will have a channel information matrix $h(r, \theta)$, which contains channel information at different location bins with range r and azimuth angle $\theta$. FIG. 13B shows the amplitude of the channel information at the range-azimuth plane.

Reflecting Object Detector: In some embodiments, to locate human subjects, one first needs to identify the range-angle bins with reflecting objects. The channel information for the bins without any reflecting object only contains noise, and thus, the energy of channel information for the bins with reflecting objects is larger than those without any reflecting objects, as shown in Eqn. (6) and Eqn. (8) respectively. However, it is difficult to find a universal predefined threshold for target detection. According to the propagation laws of EM wave, for the same reflecting objects, a shorter distance corresponding to a larger reflecting energy. In some embodiments of mmHRV, one may utilize the Constant False Alarm Rate (CFAR) detector, which can estimate the noise level by convolving the CFAR window (shown in FIG. 13A) with the channel information at the range-azimuth plane (shown in FIG. 13B), and the location bins with reflecting objects are those whose energy is above the noise level, as shown in FIG. 13C. FIG. 13D shows the example of CFAR detection in the range domain, where the threshold is shown in the dashed line.

Human Subjects Detector: In some embodiments, although reflecting object detector can filter out the empty taps, it cannot distinguish human subjects from static reflecting objects. Different from static objects, the distance between human subjects and the device will change over slow time due to motions (e.g., respiration and heartbeat), and thus result in a phase change as shown in FIG. 14A. Therefore, to further filter out the static reflecting objects, one may leverage the phase information of the candidate bins selected by the reflecting object detector.

FIGS. 14A-14D illustrate an example of the human subject detector. The ground truth is that there are 3 human subjects, one of which sits at 1.5m away from the device, with azimuth angle 0°, and the other two sit at 1m away from the device with azimuth angle 30° and -30° respectively. FIG. 14A is phase information corresponding to a human subject; FIG. 14B is phase information corresponding to a static reflecting object; FIG. 14C is the result of the human subject detector, where the black spots correspond to human subjects; and FIG. 14D shows the clustering result for each target.

When the EM wave is reflected by a human subject, the phase will change over slow time due to the modulation of human motions. Therefore, there is a large phase variance for the bins corresponding to human subjects. However, for bins corresponding to the static objects (e.g. desk, wall, etc.), the phase variance will be much smaller, as shown in FIG. 14A and FIG. 14B. So in some embodiments of mmHRV, to filter out the static objects, one may check the variance of the phase information over slow time, and the bins corresponding to a human subject are those whose phase variance above a certain threshold.

There will be more than one bin corresponding to a human subject considering the volume of a human subject, as shown in FIG. 14C. To identity the target number, the mmHRV utilizes a non-parametric clustering method, Density-Based Spatial Clustering of Applications with Noise (DBSCAN) algorithm, to cluster the candidate bins without prior knowledge of cluster number in some embodiments. The clustering result is shown in FIG. 14D. The representative of each cluster may be the bin with the best periodicity. For example, the bin with the highest peak for the first peak of the auto-correlation is selected, which corresponds to the bin with the highest SNR of the vital signs.

Heartbeat extraction and HRV estimation: in some embodiments, estimating HRV requires accurate estimation of Inter-Beat Intervals (IBIs). Therefore, the mmHRV may extract the displacement change caused by heartbeats (a.k.a., heartbeat wave) from the compound displacement change of chest wall and detect moments in which heartbeats occur.

Heartbeat Extraction Algorithm: the phase information reflects the distance change caused by vital signs. For simplicity, one can directly use the analog form of signals, and the distance change of the human chest can be written as

$$y(t) = s_m(t) + s_r(t) + s_h(t) + n(t), \tag{11}$$

where $s_m(t)$ denotes the distance change caused by body motion. $s_r(t)$ and $s_h(t)$ denote the distance change caused by respiration and heartbeat, respectively. $n(t)$ is the random phase offset introduced by noise, which is independent with the phase change caused by vital signs.

Both $s_r(t)$ and $s_h(t)$ are quasi-periodic signals, where the period can slightly change over time. Besides, one can assume the body motion introduces few oscillations, i.e., a base-band signal. Thus, the signals related with the human subject are sparse in the spectral domain and one can reconstruct these signals with a few band-limited signals. For example, each component $u_k$(t) is assumed to be compact around a center pulsation $\omega_k$, which is to be determined along with the decomposition. Moreover, the decomposition should achieve the spectrum sparsity and data fidelity at the same time, which is modeled as

$$\begin{aligned}\min_{u_k \in \mathcal{U}, \omega_k \in \Omega} \quad & \alpha \textstyle\sum_{k=1}^{K} \left\| \partial t[(\delta(t) + \frac{j}{\pi t}) * u_k(t)] \exp(-j\omega_k t) \right\|_2^2 \\ & + \left\| y(t) - \textstyle\sum_{k=1}^{K} u_k(t) \right\|_2^2, \end{aligned} \tag{12}$$

where the first term evaluates the bandwidth of the analytic signal associated with each component, and the second term evaluates the data fidelity. K is the total number of decomposition components, where $u = \{u1(t),..., u_K(t)\}$ and $\Omega = \{\omega_1, ..., \omega_K\}$ are the set for all components and their center frequencies, respectively. $\alpha$ is a parameter for balancing the bandwidth constraint and data fidelity.

Once the hyper-parameters are known, the optimization problem in Eqn. (12) can be solved by alternatively or iteratively updating $u_k(t)$ and $\omega_k$ until convergence. To update $u_k$, the subproblem can be written as

$$\begin{aligned} u_k(t) = \underset{u_k(t)}{\operatorname{argmin}} \quad & \left\| \partial t[(\delta(t) + \frac{j}{\pi t}) * u_k(t)] \exp(-j\omega_k t) \right\|_2^2 \\ & + \left\| y(t) - \textstyle\sum_{i=1}^{K} u_i(t) \right\|_2^2. \end{aligned} \tag{13}$$

By using the Parseval theorem, the problem can be rewritten as

$$\mathcal{u}_k(\omega) = \arg\min_{\mathcal{u}_k(\omega)} \quad \alpha\|j\omega[(1+\mathrm{sgn}(\omega+\omega_k))\mathcal{u}_k(\omega)]\|_2^2 + \left\|\mathcal{y}(\omega) - \sum_{i=1}^{K} \mathcal{u}_i(\omega)\right\|_2^2, \qquad (14)$$

where $\mathcal{u}_k(\omega)$ and $\mathcal{y}(\omega)$ are the Fourier transfer of $u_k(t)$ and $y(t)$ respectively. After taking integrals over frequency and performing a change of variable, one can get the updating formula, where

$$\mathcal{u}_k(\omega) = \frac{\mathcal{y}(\omega) - \sum_{i,i\neq k} \mathcal{u}_i(\omega)}{1+2\alpha(\omega-\omega_k)^2}. \qquad (15)$$

The center frequencies $\omega_k$ only appear in the bandwidth constraint and thus the subproblem can be written as

$$\omega_k = \underset{\omega_k}{\mathrm{argmin}} \left\|\partial t[(\delta(t) + \frac{j}{\pi t}) * u_k(t)]\exp(-j\omega_k t)\right\|_2^2. \qquad (16)$$

As before, one can find the optimum in Fourier domain, and have

$$\omega_k = \underset{\omega_k}{\mathrm{argmin}} \int_0^\infty (\omega - \omega_k)^2 |\mathcal{u}_k(\omega)|^2 d\omega. \qquad (17)$$

The minimizer of the above quadratic problem is

$$\omega_k = \frac{\int_0^\infty \omega|\mathcal{u}_k(\omega)|^2 d\omega}{\int_0^\infty |\mathcal{u}_k(\omega)|^2 d\omega}. \qquad (18)$$

FIGS. 15A-15B illustrate the decomposition of a typical one-minute phase signal from the experiment, where the original phase information has been decomposed into 4 components. FIG. 15A is the decomposition result in the time domain; FIG. 15B is the corresponding spectrum of each component. In some embodiments, the first component 1501 reflects the body motion of the human subject; the second component 1502 is the respiration motion; and the third component 1503 is the heartbeat wave. Since the noise has different vibration characteristics as vital signals, it falls into a different mode as well as in the residual 1504 of the decomposition of the signal, as shown in FIGS. 15A-15B.

The decomposition problem can be solved once the hyper-parameters are properly defined. However, it is hard to predefine these hyper-parameters in real applications for heartbeat wave extraction. First, the human motion does not always exist and the human respiration sometimes will have a strong second harmonic component, making it even harder to determine the component number. Furthermore, the hyper-parameter $\alpha$ also influences the decomposition performance. Before discussing how to choose the hyper-parameter, their influence on the decomposition result is disclosed as follows.

For the case that $\alpha$ is too small, i.e., the bandwidth constraint is too loose, when K is too small, the mixing problem will happen so that two signals may merge to a single decomposed component. When K is too large, some of the decomposed components may include noise. For the case that $\alpha$ is too large, i.e., the bandwidth constraint is too tight, when K is too small, some target signals may be discarded in noise. When K is too large, some important parts of the signal may be separated into two or more decomposed components.

In some embodiments of mmHRV, to accurately decompose the signal and get the component of interest, i.e., the heartbeat wave, one may adaptively change the component number K and $\alpha$ for different datasets. Here, a heuristic method is disclosed to change K and $\alpha$ as the iteration proceeds to get proper decomposition result. Since the distance change caused by heartbeat is much smaller than the distance change caused by respiration and human motion, once the component corresponding to the heartbeat is decomposed, the component corresponding to respiration and motion should be decomposed as well, considering the data fidelity constraint in the objective function. Therefore, the algorithm will terminate once one gets the component corresponding to the heartbeat.

HRV Estimation: In some embodiments, once the heartbeat wave is extracted, the exact time corresponding to each heartbeat can be identified by the peaks of the heartbeat wave. To further increase the accuracy, normalization may be performed before peak extraction.

In some embodiments, the envelope of the heartbeat wave is estimated by taking moving average to the absolute value of the heartbeat component, shown as the dashed line in FIG. 16A. One may further perform a moving average filter to the original heartbeat wave to reduce the noise. The normalized wave is the ratio between the filtered heartbeat wave and the estimated envelope. IBIs can thus be derived by calculating the time duration between two adjacent heartbeats. FIG. 16B shows a segment of heartbeat wave and its ECG ground-truth, where the dashed lines show the exact time of each heartbeat from a commercial ECG sensor. The peaks of normalized heartbeat wave match with the ground-truth, where FIG. 16C shows the estimated IBIs and the ECG ground-truth.

The HRV features can be further obtained from the IBI sequence. In some embodiments of mmHRV, one may use the following three metrics to evaluate the HRV. One is the Root Mean Square of Successive Differences (RMSSD), which measures the successive IBI changes, and can be calculated by

$$\mathrm{RMSSD} = \sqrt{\frac{1}{N_{IBI}-1}\sum_{i=2}^{N_{IBI}} (\mathrm{IBI}(i) - \mathrm{IBI}(i-1))^2}, \qquad (19)$$

where $N_{IBI}$ is the total number of IBIs of the measurement. The standard deviation of all the IBIs (SDRR) measures the variation of the IBIs, which can be calculated as

$$\mathrm{SDRR} = \sqrt{\frac{1}{N_{IBI}}\sum_{i=1}^{N_{IBI}IBI} (\mathrm{IBI}(i) - \overline{\mathrm{IBI}})^2}, \qquad (20)$$

where $\overline{\mathrm{IBI}}$ is the empirical mean of the IBIs of each measurement. The metric pNN50 measures the percentage of successive IBI that differ by more than 50 milliseconds (ms), which can be calculated by

$$\mathrm{pNN50} = \frac{\sum_{i=2}^{N_{IBI}} 1\{(\mathrm{IBI}(i)-\mathrm{IBI}(i-1))>50ms\}}{N_{IBI}}, \qquad (21)$$

where 1{·} is the indicator function.

Experiment Evaluation: In some embodiments, one may prototype the mmHRV system by leveraging a commodity mmWave FMCW radar in a typical office of size 3.5 m × 3.2 m. By configuring the 2 Tx antennas and 4 Rx antennas into TDM-MIMO mode, the system can achieve a theoretical azimuth resolution of 15°. The Field of View (FoV) is 100° in the horizontal plane with a radius of about 4m, which is sufficient to cover typical rooms. To get the true heartbeat signal, an ECG sensor is used to collect the ground-truth simultaneously with the mmHRV during the experiment. In total, 11 participants (6 males and 5 females) aging from 20 to 60 are invited to conduct experiments in both LOS and NLOS scenario. The experiments are conducted with a variety of settings including different distances, incidental angles, orientations and blockages between the human subject and the radar.

To further evaluate the performance of the disclosed system, the mmHRV system may be compared with the HRV estimation technique using Band-Pass-Filter-Bank (BPFB). FIG. 17 shows the overall IBI estimation accuracy of the mmHRV and BPFB methods. The experiment includes 11 participants while 15 different experiment settings (e.g., different distances, incidental angle, orientation and blockages) are conducted for each participant. As shown in FIG. 17, BPFB yields about 44ms medium error while the 90-percentile error is about 200ms. The mmHRV achieves a medium error of about 28ms, with the 80ms of the 90-percentile error, which outperforms the BPFB about 60%. To thoroughly evaluate the HRV estimation accuracy, Table I below shows the estimated HRV features in terms of mean IBI, RMSSD, SDRR and pNN50 of 11 participants, where the distance between user and device is about 1m. It is shown that mmHRV can achieve 3.89ms average error of mean IBI, 6.43ms average error of RMSSD, 6.44ms average error of SDRR and 2.52% average error of the pNN50. Correspondingly, the average estimation error of BPFB is 15.33ms of mean IBI, 41.94ms of RMSSD, 32.59ms of SDRR and 12.17% of the pNN50 estimations.

Table I:

| Metrics | Methods | | User ID | | | | | | | | | | |
|---|---|---|---|---|---|---|---|---|---|---|---|---|---|
| | | | 1 | 2 | 3 | 4 | 5 | 6 | 7 | 8 | 9 | 10 | 11 |
| Mean IBI | Value (ms) | ECG | 899.4 | 789.9 | 723.2 | 854.6 | 654.5 | 822.9 | 645.2 | 890.1 | 564.9 | 728.1 | 763.8 |
| | | mmHRV | 906.3 | 790.4 | 725.6 | 848.6 | 652.4 | 828.3 | 644.2 | 888.1 | 574.2 | 722.7 | 762.6 |
| | | BPFB | 881.5 | 784.2 | 781.5 | 842.1 | 676.6 | 821.7 | 651.5 | 878.4 | 579.1 | 719 | 773.5 |
| | Error (ms) | mmHRV | 6.95 | 0.45 | 2.47 | 5.92 | 2.17 | 5.4 | 0.99 | 1.97 | 9.33 | 5.38 | 1.2 |
| | | BPFB | 17.87 | 5.7 | 58.36 | 12.44 | 22.01 | 1.25 | 6.31 | 11.66 | 14.21 | 9.16 | 9.66 |
| RMSSD | Value (ms) | ECG | 38.59 | 10.85 | 37.56 | 31.49 | 34.05 | 35.1 | 16.88 | 27.52 | 5.26 | 23.28 | 31.16 |
| | | mmHRV | 33.52 | 16.53 | 39.08 | 35.26 | 20.29 | 39.72 | 18.14 | 26.06 | 27.8 | 30.52 | 34.92 |
| | | BPFB | 59.34 | 54.26 | 53.83 | 52.94 | 78.57 | 65.63 | 95.09 | 45.56 | 140.36 | 59.61 | 47.92 |
| | Error (ms) | mmHRV | 5.08 | 5.68 | 1.52 | 3.77 | 13.76 | 4.62 | 1.26 | 1.46 | 22.53 | 7.25 | 3.76 |
| | | BPFB | 20.75 | 43.41 | 16.27 | 21.45 | 44.53 | 30.52 | 78.21 | 18.04 | 135.1 | 36.34 | 16.76 |
| SDRR | Value (ms) | ECG | 56.28 | 22.91 | 50.54 | 35.35 | 33.61 | 48.55 | 23.24 | 32.66 | 12.25 | 35.83 | 50.87 |
| | | mmHRV | 43.22 | 27.25 | 53.3 | 45.88 | 33.54 | 48.53 | 25.49 | 37.43 | 38.66 | 37.15 | 45.51 |
| | | BPFB | 71.01 | 47.28 | 110.29 | 58.92 | 69.68 | 55.11 | 67.61 | 50.44 | 118.41 | 47.92 | 63.94 |
| | Error (ms) | mmHRV | 13.07 | 4.34 | 2.76 | 10.53 | 0.07 | 0.02 | 2.24 | 4.78 | 26.42 | 1.31 | 5.36 |
| | | BPFB | 14.72 | 24.37 | 59.74 | 23.57 | 36.07 | 6.55 | 44.37 | 17.78 | 106.16 | 12.09 | 13.07 |
| pnn50 | Value (%) | ECG | 11.54 | 0 | 9.15 | 4.32 | 1.14 | 6.29 | 0.55 | 3.76 | 0 | 0.61 | 4.49 |
| | | mmHRV | 8.46 | 1.33 | 7.93 | 5.76 | 2.2 | 6.99 | 2.17 | 2.26 | 4.83 | 6.71 | 6.41 |
| | | BPFB | 19.4 | 18.54 | 14.57 | 20 | 14.2 | 22.92 | 18.13 | 12.59 | 10.24 | 12.8 | 12.42 |
| | Error (ms) | mmHRV | 3.08 | 1.33 | 1.22 | 1.44 | 1.05 | 0.7 | 1.62 | 1.5 | 4.83 | 6.09 | 1.92 |
| | | BPFB | 7.86 | 18.54 | 5.42 | 15.68 | 13.06 | 16.62 | 17.58 | 8.83 | 10.24 | 12.19 | 7.93 |

FIG. 18 illustrates a flow chart of an exemplary method 1800 for wireless vital monitoring, according to some embodiments of the present disclosure. At operation 1802, a first wireless signal is transmitted through a wireless channel of a venue. At operation 1804, a second wireless signal is received through the wireless channel, wherein the second wireless signal comprises a reflection of the first wireless signal by at least one living being having at least one repetitive motion in the venue. At operation 1806, a time series of channel information (CI) of the wireless channel is obtained based on the second wireless signal, wherein each CI comprises at least one of: a channel state information (CSI), channel impulse response (CIR), channel frequency response (CFR), or received signal strength index (RSSI). At operation 1808, for each of the at least one living being, a vital signal representing all repetitive motions of the living being is generated based on the plurality of TSCI. The vital signal may represent vital signs like heartbeat or breathing. At operation 1810, a heartbeat signal is extracted from the vital signal of each living being. At operation 1812, for each living being in the venue, a heart rate variability is monitored based on the heartbeat signal. The order of the operations in FIG. 18 may be changed according to various embodiments of the present teaching.

In some embodiments, a wireless vital monitoring method includes steps s1 to s8 as described below.

At step s1: capture CSI using multiple transmit (Tx) antenna and multiple receive (Rx) antenna. At step s2: apply beamforming to get directional CSI (e.g. CIR). This may associate direction and distance with CSI. At step s3: determine direction-of-interest (DoI) by detecting object presence in each direction, which includes steps s3a and s3b performed for each direction.

At step s3a: compute magnitude of CSI (e.g. |h(theta, distance)| of CIR) for each time instance, then time-average it over a time window. At step s3b: determine object is present in the direction (and thus the direction is DoI) if the time-averaged magnitude response is greater than a threshold T1, wherein the threshold T1 may be a 2-dimensional CFAR filtering of |h| in theta and distance direction.

At step s4: for each DoI (i.e. the direction where object presence is detected), perform motion detection by classifying object into: (a) static object (e.g. furniture), (b) stationary human (with breathing and heartbeat), and (c) random body motion, which includes steps s4a to s4e.

At step s4a: compute variance (V) of phase of CSI (e.g. phase of h(theta, distance)), over time in a time window. In some embodiments, a larger phase variance means the target is a living being with heartbeat. At step s4b: classify motion as "static object" if V is less than a threshold T2. At step s4c: if V>T2, compute auto-correlation function (ACF) and find a significant feature point (e.g. first peak) PI. At step s4d: classify motion as "stationary human" if V>T2 and PI >T3. At step s4e: classify motion as "random body motion" if V>T2 and P1<T3. In some embodiments, a larger PI means a more periodic feature.

At step s5: determine the number of stationary human subjects and their corresponding vital motions, which includes steps s5a to s5b.

At step s5a: cluster the set of point-of-interest (PoI) (i.e., the (theta, distance) corresponding to stationary human in step s4), wherein the PoIs are clustered without prior knowledge of the cluster number, i.e., non-parametric clustering. The PoIs can be classified based on density-based method (e.g., DBSCAN) or can be classified based on distance (e.g., if the distance between two PoIs > common size of a human body, then they belong to different clusters).

At step s5b: generate the vital motions corresponding to each human subject. When more than one PoI corresponds to a human subject, the corresponding motion can be combined, e.g., by weighted averaging the phase measurement of PoIs, or a dominant PoI may be identified and the vital motion is associated with the dominant tap.

At step s6: for each human subject, extract a heartbeat signal by decomposing the vital signal with a few band-limited signals by either jointly optimizing the decomposition as in step s6a or a successive decomposition as in step s6b.

At step s6a: jointly optimize the decomposition of the raw signal modeled using steps s6a1, s6a2 and s6a3.

At step s6a1: given a default setting of component number K and the parameter $\alpha$ for balancing the bandwidth constraint and data fidelity, alternatively optimize the components and their center frequencies.

At step s6a2: check whether there is a component corresponding to heartbeat by some features, wherein the component corresponds to heartbeat wave if the amplitude of the signal is located in range [T4, T5], and its center frequency is located in range [T6, T7]. In some embodiments, one may also extract breathing/respiration signals at step s6a2.

At step s6a3: if there is a decomposed component corresponding to heartbeat, normalize the heartbeat signal in step s7; otherwise, update the values of component number K and tradeoff factor $\alpha$ and repeat steps s6a1 to s6a3.

At step s6b: successively decompose the raw signal to get the heartbeat wave using steps s6b1, s6b2, and s6b3.

At step s6b1: process the raw signal by removing/suppressing influence of the dominant (larger magnitude) periodic signal (e.g. filter the raw signal, or estimate the dominant periodic signal and subtract it from the raw signal), wherein the dominant periodic signal may be estimated by an operation on the raw signal (e.g. smoothing, low pass filtering, spline interpolation, B-spline, cubic spline interpolation, polynomial fitting, polynomial fitting with order adaptively selected based on the distance/tap, etc.).

At step s6b2: compute characteristics of the next dominant periodic signal based on the processed raw signal. The characteristics may be computed based on frequency transform, trigonometric transform, fast Fourier transform (FFT), wavelet transform, ACF, etc. The characteristics may also be computed by constrained optimization (e.g. minimization of an energy function subjected to a smoothness constraint). The energy function may be energy of frequency (e.g. energy of FFT of dominant-component-removed signal, where the signal may be the fused/clustered signal).

At step s6b3: check whether the component corresponds to heartbeat by some features, wherein the component corresponds to heartbeat wave if the amplitude of the signal is located in range [T4, T5], and its center frequency is located in range [T6, T7]. If it corresponds to the heartbeat, normalize the heartbeat signal in step s7; otherwise, remove the component and then repeat steps s6b2 and s6b3.

In some embodiments, other mode decomposition method can be applied for step s6, where mode can be viewed as a frequency component, a signal, etc. by e.g. ensemble empirical mode decomposition. In some embodiments, instead of using phase information as the input to extract heartbeat, one may also rely on CIR amplitude to extract heartbeat signal/wave.

At step s7: normalize the estimated heartbeat wave by dividing the estimated wave with an envelope of the signal, wherein the envelope can be estimated by an operation on the raw signal (e.g. smoothing, low pass filtering, spline interpolation, B-spline, cubic spline interpolation, polynomial fitting and moving average).

At step s8: identify an exact time of each heartbeat and then calculate the inter-beat intervals to estimate heart rate variability (HRV) and/or other statistics of inter-beat intervals, wherein the exact time of each heartbeat can be identified by several ways, e.g., by identifying the peaks of the heartbeat waves, identifying the zero-crossing points, or finding some feature points after taking continuous wavelet transform.

The following numbered clauses provide implementation examples for wireless vital monitoring.

Clause B1. A system for wireless monitoring, comprising: a transmitter configured for transmitting, using N1 transmit antennas, a first wireless signal through a wireless channel of a venue; a receiver configured for receiving, using N2 receive antennas, a second wireless signal through the wireless channel, wherein N1 and N2 are positive integers, wherein the second wireless signal comprises a reflection of the first wireless signal by at least one living being having at least one repetitive motion in the venue; and a processor configured for: obtaining a plurality of time series of channel information (TSCI) of the wireless channel based on the second wireless signal, wherein each of the plurality of TSCI is associated with a respective transmit antenna of the transmitter and a respective receive antenna of the receiver, generating, for each living being of the at least one living being, a vital signal representing all repetitive motions of the living being based on the plurality of TSCI, extracting, from the vital signal of each living being, a heartbeat signal, and monitoring, for each living being in the venue, a heart rate variability based on the heartbeat signal.

Clause B2. The system of clause B1, wherein: the at least one living being comprises: a human being or an animal; the first wireless signal is carried on a millimeter wave; each object in the venue has a location determined based on a plurality of spatial bins in the venue; each of the plurality of spatial bins is determined by: a direction and a distance range originating from the receiver; and each direction is associated with at least one of: an angle, an azimuth angle, or an elevation angle.

Clause B3. The system of clause B1 or B2, wherein generating the vital signal for each living being comprises: computing a beamforming based on the plurality of TSCI; and computing a set of time series of directional channel information (CI) each associated with a direction based on the beamforming.

Clause B4. The system of clauses B1 to B3, wherein generating the vital signal for each living being further comprises: for each directional CI associated with a respective direction, computing, for each time instance, a CI amplitude based on the directional CI for the respective direction and a distance range to obtain CI amplitudes over time, computing a time average of the CI amplitudes based on a time window, and detecting object presence at the distance range in the respective direction when the time average is greater than a first threshold; and determining a set of direction-of-interest's (DoIs) each comprising a direction in which object presence is detected.

Clause B5. The system of clause B4, wherein the first threshold is determined adaptively to filter the CI amplitudes at the distance range in the respective direction based on a two-dimensional constant false alarm rate (CFAR).

Clause B6. The system of clause B4 or B5, wherein generating the vital signal for each living being further comprises: for each DoI in the set of DoIs and for each distance range, computing a phase variance of a directional CI associated with the DoI over time in a time window, classifying a detected object at the distance range and the DoI as a static object without repetitive motion, when the phase variance is less than a second threshold, and classifying a detected object at the distance range and the DoI as a living being with repetitive motion, when the phase variance is greater than or equal to the second threshold.

Clause B7. The system of any previous B clause, wherein generating the vital signal for each living being further comprises: determining a plurality of target spatial bins for each detected living being, wherein each of the plurality of target spatial bins is determined by a target DoI and a target distance range; and for each target spatial bin, computing an auto-correlation function based on the directional CI associated with the target spatial bin, determining a first peak of the auto-correlation function, classifying a motion of the detected living being at the target spatial bin as a repetitive motion when the first peak is greater than a third threshold, and classifying a motion of the detected living being at the target spatial bin as a random body motion when the first peak is less than or equal to the third threshold.

Clause B8. The system of any previous B clause, wherein generating the vital signal for each living being further comprises: computing a set of point-of-interest's (PoIs), wherein each PoI in the set of PoIs is associated with a detected living being at the PoI and is a target spatial bin in the venue associated with a repetitive motion of the detected living being at the PoI; clustering the set of PoIs to generate at least one PoI cluster with a total cluster number, wherein: the set of PoIs are clustered without prior knowledge of the total cluster number after clustering, and the set of PoIs are clustered based on at least one of: a density related to the set of PoIs, a distance between any two PoIs of the set of PoIs, or a threshold related to a size of a living being; and determining a quantity of target living beings in the venue based on the total cluster number.

Clause B9. The system of any previous B clause, wherein generating the vital signal for each living being further comprises: for each of the at least one PoI cluster, combining PoIs in the PoI cluster to generate a combined PoI based on at least one of: a weighted average of CI phases measured at the PoIs, or a dominant PoI having a highest peak among the first peaks of the auto-correlation functions associated with the PoIs, and generating a vital signal for a target living being corresponding to the PoI cluster based on a CI phase signal corresponding to the combined PoI, wherein the CI phase signal is associated with all repetitive motions of the target living being.

Clause B10. The system of any previous B clause, wherein extracting a heartbeat signal from the vital signal comprises: decomposing, for each living being, the CI phase signal associated with the living being to generate the heartbeat signal based on at least one of: a joint optimization of a decomposition of the CI phase signal, or a successive decomposition of the CI phase signal.

Clause B11. The system of clause B10, wherein the joint optimization comprises: determining a number K that represents a quantity of possible signal components of the CI phase signal, wherein K is larger than or equal to a quantity of living beings in the venue; determining a tradeoff factor for balancing bandwidth constraint and data fidelity; iteratively optimizing, based on the tradeoff factor, K signal components of the CI phase signal and center frequencies of the K signal components, based on an objective function that maximizes spectrum sparsity and data fidelity of the CI phase signal at the same time, until a convergence of the objective function; and concurrently generating K decomposed components of the CI phase signal based on the iteratively optimizing.

Clause B12. The system of clause B 10 or B11, wherein the joint optimization further comprises: determining whether the K decomposed components comprise a heartbeat component, which has an amplitude located within a first value range and has a center frequency located within a second value range, wherein each of the first value range and the second value range is predetermined based on heartbeat statistics.

Clause B13. The system of clauses B10 to B12, wherein the joint optimization further comprises: when there is a heartbeat component in the K decomposed components, estimating an envelope of the heartbeat component based on at least one of: smoothing, low pass filtering, spline interpolation, B-spline, cubic spline interpolation, polynomial fitting, or moving average, and normalizing the heartbeat component by dividing the heartbeat component with the envelope of the heartbeat component to generate a normalized heartbeat signal for the living being.

Clause B14. The system of clauses B10 to B13, wherein the joint optimization further comprises: when there is no heartbeat component in the K decomposed components, updating the number K to generate an updated number K' to represent an updated quantity of possible signal components of the CI phase signal, updating the tradeoff factor to generate an updated tradeoff factor for balancing bandwidth constraint and data fidelity, and iteratively optimizing, based on the updated tradeoff factor, K' signal components of the CI phase signal and center frequencies of the K' signal components, based on the objective function that maximizes spectrum sparsity and data fidelity of the CI phase signal at the same time, until a convergence of the objective function, to concurrently generate K' decomposed components of the CI phase signal.

Clause B15. The system of clause B10, wherein the successive decomposition comprises: estimating a dominant component of the CI phase signal, based on at least one of: smoothing, low pass filtering, spline interpolation, or polynomial fitting; removing the dominant component from the CI phase signal to generate a processed CI phase signal; computing a characteristic of a second dominant component of the CI phase signal based on the processed CI phase signal utilizing at least one of: a frequency transform, a trigonometric transform, a fast Fourier transform (FFT), or a wavelet transform; and determining, based on the characteristic, whether the second dominant component is a heartbeat component, which has an amplitude located within a first value range and has a center frequency located within a second value range, wherein each of the first value range and the second value range is related to heartbeat. The characteristic may also be computed by a minimization of an energy function subject to a smoothness constraint.

Clause B16. The system of clause B10 or B15, wherein the successive decomposition further comprises: when the second dominant component is a heartbeat component, estimating an envelope of the heartbeat component based on at least one of: smoothing, low pass filtering, spline interpolation, B-spline, cubic spline interpolation, polynomial fitting, or moving average, and normalizing the heartbeat component by dividing the heartbeat component with the envelope of the heartbeat component to generate a normalized heartbeat signal for the living being.

Clause B17. The system of clause B10, B15 or B16, wherein the successive decomposition further comprises: when the second dominant component is not a heartbeat component, removing the second dominant component from the CI phase signal to generate an additional processed CI phase signal, computing an additional characteristic of a next dominant component of the CI phase signal based on the additional processed CI phase signal, and determining based on the additional characteristic, whether the next dominant component is a heartbeat component, which has an amplitude located within the first value range and has a center frequency located within the second value range. The additional characteristic may be computed either by a frequency transform or by a minimization of an energy function subject to a smoothness constraint.

Clause B18. The system of clauses B10 to B17, wherein monitoring the heart rate variability further comprises: for each living being, determining a heartbeat time for the living being at each time instance to compute heartbeat times, based on at least one of: identifying peaks of the heartbeat signal, identifying zero-crossing points of the heartbeat signal, or performing continuous wavelet transform on the heartbeat signal; calculating a plurality of inter-beat intervals based on the heartbeat times; and estimating the heart rate variability for the living being based on statistics of the inter-beat intervals.

Clause B19. The system of any previous B clause, wherein extracting a heartbeat signal from the vital signal comprises generating, for each living being, the heartbeat signal based on at least one of: a decomposition of the vital signal based on frequency components of the vital signal; or a decomposition of a CI amplitude signal associated with the living being.

Clause B20. The system of any previous B clause, wherein the transmitter and the receiver are physically coupled to each other.

Clause B21. A wireless device of a wireless monitoring system, comprising: a processor; a memory communicatively coupled to the processor; and a receiver communicatively coupled to the processor, wherein: an additional wireless device of the wireless monitoring system is configured for transmitting a first wireless signal through a wireless channel of a venue, the receiver is configured for receiving a second wireless signal through the wireless channel, the second wireless signal comprises a reflection of the first wireless signal by at least one living being having at least one repetitive motion in the venue, and the processor is configured for: obtaining a time series of channel information (TSCI) of the wireless channel based on the second wireless signal, generating, for each living being of the at least one living being, a vital signal representing all repetitive motions of the living being based on the TSCI, extracting, from the vital signal of each living being, a heartbeat signal, and monitoring, for each living being in the venue, a heart rate variability based on the heartbeat signal.

Clause B22. The wireless device of clause B21, wherein: the at least one living being comprises: a human being or an animal; the first wireless signal is carried on a millimeter wave; each object in the venue has a location determined based on a plurality of spatial bins in the venue; each of the plurality of spatial bins is determined by: a direction and a distance range originating from the receiver; and each direction is associated with at least one of: an angle, an azimuth angle, or an elevation angle.

Clause B23. The wireless device of clause B21 or B22, wherein generating the vital signal for each living being comprises: computing a beamforming based on the TSCI; computing a set of time series of directional channel information (CI) each associated with a direction based on the beamforming; for each directional CI associated with a respective direction, computing, for each time instance, a CI amplitude based on the directional CI for the respective direction and a distance range to obtain CI amplitudes over time, computing a time average of the CI amplitudes based on a time window, and detecting object presence at the distance range in the respective direction when the time average is greater than a first threshold, wherein the first threshold is determined adaptively to filter the CI amplitudes at the distance range in the respective direction based on a two-dimensional constant false alarm rate (CFAR); and determining a set of direction-of-interest's (DoIs) each comprising a direction in which object presence is detected.

Clause B24. The wireless device of clauses B21 to B23, wherein generating the vital signal for each living being further comprises: for each DoI in the set of DoIs and for each distance range, computing a phase variance of a directional CI associated with the DoI over time in a time window, classifying a detected object at the distance range and the DoI as a static object without repetitive motion, when the phase variance is less than a second threshold, and classifying a detected object at the distance range and the DoI as a living being with repetitive motion, when the phase variance is greater than or equal to the second threshold; determining a plurality of target spatial bins for each detected living being, wherein each of the plurality of target spatial bins is determined by a target DoI and a target distance range; and for each target spatial bin, computing an auto-correlation function based on the directional CI associated with the target spatial bin, determining a first peak of the auto-correlation function, classifying a motion of the detected living being at the target spatial bin as a repetitive motion when the first peak is greater than a third threshold, and classifying a motion of the detected living being at the target spatial bin as a random body motion when the first peak is less than or equal to the third threshold.

Clause B25. The wireless device of clauses B21 to B24, wherein generating the vital signal for each living being further comprises: computing a set of point-of-interest's (PoIs), wherein each PoI in the set of PoIs is associated with a detected living being at the PoI and is a target spatial bin in the venue associated with a repetitive motion of the detected living being at the PoI; clustering the set of PoIs to generate at least one PoI cluster with a total cluster number; determining a quantity of target living beings in the venue based on the total cluster number; and for each of the at least one PoI cluster, combining PoIs in the PoI cluster to generate a combined PoI based on at least one of: a weighted average of CI phases measured at the PoIs, or a dominant PoI having a highest peak among the first peaks of the auto-correlation functions associated with the PoIs, and generating a vital signal for a target living being corresponding to the PoI cluster based on a CI phase signal corresponding to the combined PoI, wherein the CI phase signal is associated with all repetitive motions of the target living being.

Clause B26. The wireless device of clauses B21 to B25, wherein extracting a heartbeat signal from the vital signal comprises: decomposing, for each living being, a CI phase signal associated with the living being to generate the heartbeat signal based on a joint optimization of a decomposition of the CI phase signal, wherein the joint optimization comprises: determining a number K that represents a quantity of possible signal components of the CI phase signal, wherein K is larger than or equal to a quantity of living beings in the venue, determining a tradeoff factor for balancing bandwidth constraint and data fidelity, iteratively optimizing, based on the tradeoff factor, K signal components of the CI phase signal and center frequencies of the K signal components, based on an objective function that maximizes spectrum sparsity and data fidelity of the CI phase signal at the same time, until a convergence of the objective function, and concurrently generating K decomposed components of the CI phase signal based on the iteratively optimizing.

Clause B27. The wireless device of clause B26, wherein the joint optimization further comprises: determining whether the K decomposed components comprise a heartbeat component, which has an amplitude located within a first value range and has a center frequency located within a second value range, wherein each of the first value range and the second value range is predetermined based on heartbeat statistics.

Clause B28. The wireless device of clause B26 or B27, wherein the joint optimization further comprises: when there is a heartbeat component in the K decomposed components, estimating an envelope of the heartbeat component based on at least one of: smoothing, low pass filtering, spline interpolation, B-spline, cubic spline interpolation, polynomial fitting, or moving average, and normalizing the heartbeat component by dividing the heartbeat component with the envelope of the heartbeat component to generate a normalized heartbeat signal for the living being.

Clause B29. The wireless device of clauses B26 to B28, wherein the joint optimization further comprises: when there is no heartbeat component in the K decomposed components, updating the number K to generate an updated number K' to represent an updated quantity of possible signal components of the CI phase signal, updating the tradeoff factor to generate an updated tradeoff factor for balancing bandwidth constraint and data fidelity, and iteratively optimizing, based on the updated tradeoff factor, K' signal components of the CI phase signal and center frequencies of the K' signal components, based on the objective function that maximizes spectrum sparsity and data fidelity of the CI phase signal at the same time, until a convergence of the objective function, to concurrently generate K' decomposed components of the CI phase signal.

Clause B30. A method of a wireless monitoring system, comprising: transmitting a first wireless signal through a wireless channel of a venue; receiving a second wireless signal through the wireless channel, wherein the second wireless signal comprises a reflection of the first wireless signal by a plurality of human beings in the venue; obtaining a time series of channel information (TSCI) of the wireless channel based on the second wireless signal, wherein each CI comprises at least one of: a channel state information (CSI), channel impulse response (CIR), channel frequency response (CFR), or received signal strength index (RSSI); generating, for each of the plurality of human beings, a vital signal representing all repetitive motions of the human being based on the TSCI; extracting, from the vital signal of each human being, a heartbeat signal; and simultaneously monitoring, for each of the plurality of human beings, a heart rate variability based on the heartbeat signal.

Automobiles have become a daily necessity in current fast-paced world due to its mobility, convenience and comfortableness. Statistics show that the number of worldwide automobiles on-the-road has reached 1.2 billion by 2015. However, in the meanwhile, road traffic crashes result in about 1.35 million deaths around the world each year and leave between 20 and 50 million people with non-fatal injuries, according to World Health Organization. To reduce the number of road accidents and enhance the driving safety, automobile manufacturers and researchers have been working on more and more Advanced Driver Assistance Systems (ADAS). Among many popular topics in autonomous driving, driver's vital sign monitoring is one of the essential components. Continuously monitoring driver's status makes it possible to allow the ADAS to take control of the automobiles in case of emergency, such as when the driver encounters a sudden heart attack, stroke or fatigue, which can be predicted or indicated by using the driver's Heart Rate Variability (HRV), i.e., the variation of the Inter-Beat Intervals (IBI). HRV, in combination with Heart Rate (HR) and Respiration Rate (RR), has been well established as a good indicator of cardiac arrhythmia, alcohol usage, mental stress and drowsiness, and thus predicts the human alertness well.

Traditional driver vital signs monitoring solutions mainly include two categories: sensor-based methods and vision-based methods. The sensor based methods require a driver to wear physiological sensors such as photoplethysmography (PPG), electrocardiogram (ECG) and electroencephalography (EEG) to monitor vital signs. However, it is cumbersome and uncomfortable to wear these dedicated sensors in daily commute. Moreover, wearing sensors may distract driver's attention, degrading the safety and user experience. As a less intrusive solution, vision-based methods utilize image sequences to detect the vital signs including RR, HR and HRV. However, the main drawbacks such as its poor performance in low-light scenarios and the privacy concerns hinder the wide deployment of the vision-based systems.

With the development of wireless sensing, Radio Frequency (RF) based methods have become one of the most promising candidates. Intuitively, the presence of a human subject will affect the RF propagation, i.e., RF signals reflected off human subjects will be modulated by the body movement including chest movement due to respiration and heartbeat. As a result, RF-based systems can estimate vital signs without any physical contact, while preserving the user privacy and operating robustly regardless of the light conditions. Many work have validated the feasibility of RR, HR and HRV monitoring using RF signal. However, most of these solutions focus on indoor scenarios with stationary human subjects, which cannot deal with the noisy in-car environment with engine vibrations, road vibrations, and human body motion. Therefore, accurate RF-based driver vital sign monitoring needs to be further investigated.

Technically, it is non-trivial to enable RF-based driver vital signs monitoring. First, during driving, the driver exhibits frequent and unpredictable motion (e.g., control the steering wheel, head movement to keep track of the car, and body roaming due to acceleration or brake, etc.), which frequently cause dominant motion larger than respiration and heartbeat, and can easily corrupt the periodic variations induced by vital signals. Therefore, it is hard to distill the minute motion caused by vital signals through the raw RF signal.

To overcome the problem, the present teaching discloses a two-step motion compensation algorithm, according to some embodiments. The reflection profile of the driver stays similar considering the resolution of the system and the size of target. Given such an observation, in the first step, the location change of the driver is compensated based on the cross correlation between consecutive Channel Impulse Response (CIR). After that, the reflections corresponding to the same part of the human body will be aligned in the same range-azimuth bin over time. To further remove the fine motion artifacts and recover the periodicity of vital signals revealed in the phase measurement, in the second step, the motion trend is further estimated by smoothing spline and then eliminated. Second, even after eliminating most of the effect of body motion, it is still challenging to extract individual heartbeats from the compound vital signals. This is because that the distance change caused by heartbeat is an order of magnitude smaller than that caused by respiration, and the heartbeat signal is easily to be submerged. Moreover, these subtle cardiogenic body movements lack sharp peak feature as in ECG signals, making it harder to accurately pinpoint the exact timing of heartbeats for HRV estimation.

To tackle these challenges and reconstruct respiration as well as heartbeat signals from the RF reflections, the present teaching discloses a joint decomposition method by exploring following properties of the vital signals. (1) Both respiration and heartbeat signals are quasi-periodic signals, where the normal frequency of respiration and heartbeat are 6-30 Respiration Per Minute (RPM) and 50-120 Beat Per Minute (BPM), respectively. (2) The reflections from the human chest would occupy different range taps and azimuth angles (known as range-azimuth bins as shown in FIG.

19) considering the range-azimuth resolution of the device and the size of human body. Hence, the vital information contained in multiple range-azimuth bins can be jointly optimized to improve the estimation accuracy. (3) The frequency of vital signals reflected by different parts of human chest (corresponding to different range-azimuth bins) stay the same because the reflections come from the same human subject. However, the distance change caused by respiration and heartbeat can be distinct in different parts of human body due to the physiological structure as shown in FIG. 19. Therefore, for all the range-azimuth bins containing vital signals, one would observe periodic signals with the same frequency but different amplitude in the phase measurement.

Leveraging the aforementioned properties, the disclosed system can jointly optimize the decomposition of the vital signals in different range-azimuth bins as an ensemble of band-limited signals. The respiration and heartbeat signals can be further reconstructed by using the empirical mean of the corresponding component over all range-azimuth bins for RR, HR and IBI estimations.

In some embodiments, the disclosed system may be prototyped using a single Commodity Off-The-Shelf (COTS) millimeter-wave (mmWave) radio, with extensive on-road tests conducted to evaluate its performance. In some embodiments, 4 volunteers (2 males and 2 females) help on the data collection, and the testing route is a cycle of 50.7 miles including local routes and highway with different road conditions. The impact of different factors, including the pavement condition, the device location and user heterogeneity are investigated. Experimental results show that the disclosed system can achieve accurate estimations with the median errors of RR, HR and IBI estimation being 0.16 RPM, 0.82 BPM and 46 ms, respectively. The disclosed system is the first RF-based driver vital sign monitoring system that can achieve accurate HRV estimation with motion artifacts. The disclosed system can estimate the driver's vital signs including RR, HR, and more importantly HRV regardless of motion artifacts, which is not achievable in existing works.

In some embodiments, the disclosed system aims at non-contact driver's vital sign monitoring in practical driving scenarios with inevitable random motions by using a single commodity Frequency-Modulated Continuous Wave (FMCW) radar. According to some embodiments, the pipeline of the system is shown in FIG. 20, which includes two main modules: a vital motion extraction module 2010 and a vital signs estimation module 2020.

A transmitter (Tx) 2001 and a receiver (Rx) 2002 may be used to obtain channel information of a wireless channel based on a wireless signal. The wireless signal may be transmitted by the Tx 2001 and received by the Rx 2002 after reflected by objects and/or human beings through the wireless channel. The wireless signal may be at high frequency band, such as 28GHz, 60GHz, 77GHz. In some embodiments, the Tx 2001 is a Bot as described above and has a structure as shown in FIG. 9; and the Rx 2002 is an Origin as described above and has a structure as shown in FIG. 10.

In the first stage, the vital motion extraction module 2010 extracts the bins containing vital signals from the channel information. A beamforming may be performed on the channel information to get the Channel Impulse Response (CIR) at different range-azimuth bins. Then, the clutter removal is performed to subtract the background reflections. However, vital signals cannot be directly extracted even after background subtraction because the driver's location w.r.t. radar can change over time (e.g., body roaming due to acceleration or brake) during driving. As a result, the vital signals will spread over multiple range bins. Therefore, a motion compensation algorithm is devised to eliminate the effect of large body movement. The location change of the driver is first roughly compensated between consecutive CIRs based on correlation of the CIR amplitude. Then, the subtle motion within the range bin are estimated and eliminated from the CIR phase utilizing smoothing spline. After motion compensation, the range-azimuth bins containing vital signals (a.k.a. vital bins) will show periodic pattern, and the CIR of these bins will be exported for further vital signs estimation.

In the second stage, the vital signs estimation module 2020 estimates drivers' RR, HR and HRV using the vital signals exported by the vital motion extraction module 2010. To enable HRV analysis, heartbeat wave needs to be reconstructed to get the exact time of each heartbeat. However, it is non-trivial to extract the heartbeat signal from the compound vital signals including both respiration and heartbeat movements. To accurately recover the respiration as well as heartbeat signal, the disclosed system can optimize the decomposition of vital signals in all vital bins with multiple band-limited signals concurrently. The extracted respiration and heartbeat signals in all the vital bins are further combined to give an estimate of the respiration and heartbeat wave for RR, HR and IBI estimation.

In some embodiments, the disclosed system is built upon an FMCW radar, which transmits a signal with periodic linearly-increasing frequency ramps, as shown in FIG. 21. A chirp in FIG. 21 is a single transmission and the transmitted signal of the m-th chirp can be expressed as

$$x_T^m(\tau) = A_T \exp\{-j[2\pi f_c \tau + \pi \frac{B}{T_c} \tau^2]\}, \quad (22)$$

where $f_c$ is the chirp starting frequency, $T_c$ is the chirp duration, B is the bandwidth and $A_T$ is the transmitting power. The reflected signal $x_R^m(\tau)$ can be expressed as

$$x_R^m(\tau) = \sum_{p=1}^{P} A_R \exp\{-j[2\pi f_c(\tau - \tau_p) + \pi \frac{B}{T_c}(\tau - \tau_p)^2]\}, \quad (23)$$

where $A_R$ is the amplitude of the receiving signal, $\tau_p$ stands for the round-trip delay of p-th reflecting path and can be denoted as $\tau_p = \frac{2d_p}{c}$, where $d_p$ is the distance between the reflecting object and the device, c is the speed of light, and P denotes the total number of reflecting points in the environment.

Mixing the received signal with a replica of the transmitted signal and following a low-pass filter, the channel information at time instance m can be expressed as

$$h^m(\tau) = \sum_{p=1}^{P} A\exp\{-j(2\pi \frac{B\tau_p}{T_c}\tau + 2\pi f_c \tau_p - \pi \frac{B}{T_c}\tau_p^2)\}, \quad (24)$$

where A denotes the channel gain, $\tau_p$ is in nanosecond for the short-range applications, and the term $\pi \frac{B}{T_c}\tau_p^2$ is negligible. Therefore, the $h^m(\tau)$ can be written as

$$h^m(\tau) = \sum_{p=1}^{P} A\exp\{-j(2\pi \frac{B\tau_p}{T_c}\tau + 2\pi f_c \tau_p)\}, \quad (25)$$

which is a summation of P sinusoidal signals, whose frequency $f_p \triangleq \frac{B\tau_p}{T_c} = \frac{2Bd_p}{cT_c}$ depends on the target's distance. In addition, by leveraging multiple antennas of the chipset to increase angle resolution, the channel information can be further denoted as

$$h^m(\tau, l) = \sum_{p=1}^{P} A\exp\{-2\pi j(f_p\tau + f_c\tau_p + \frac{d_l \sin\theta}{\lambda_c})\}, \quad (26)$$

where $\lambda_c$ denotes the wavelength of the chirp, $d_l$ is the relative distance introduced by the 1-th antenna, $\theta$ is the azimuth angle of the target. This channel information can be converted to CIR by Fast Fourier Transform (FFT) of $h^m(\tau, l)$, a.k.a Range-FFT, which can be denoted as

$$h_{r,l}(m) = \sum_{n=1}^{N} h^m(n, l)\exp\{-j2\pi \frac{rn}{N}\}, \quad (27)$$

where $h_{r,l}(m)$ denotes the CIR of 1-th antenna element and r-th range tap r at time instance m, n denotes the sample index after digitizing the $h^m(\tau,l)$ over fast-time $\tau$, and N is the total number of samples per chirp.

In a real-world setting, extracting vital motions from the RF signal is not trivial. Due to the presence of various clutters in car (e.g., chairs, metal objects, ceilings, etc.), it is hard to filter the RF reflections off human body. Moreover, since body motion will be involved during driving, the periodicity of the reflected signal caused by vital motions can be corrupted, complicating the detection of vital signals.

In some embodiments, to determine the range and the direction of the reflecting objects, the disclosed system employs digital beamforming over all antennas for each range tap. For example, the Bartlett beamformer may be used, where the coefficient of the 1 -th antenna towards azimuth angel $\theta$ is

$$s_l(\theta) = \exp(-2\pi j \frac{d_l \sin\theta}{\lambda_c}), \quad (28)$$

The beamformed CIR corresponding to range r and azimuth angle $\theta$ can be expressed as

$$h(r, \theta, m) = \mathbf{s}^H(\theta)\mathbf{h}_{r,l}(m) + \varepsilon(m), \quad (29)$$

where $s(\theta) = [s_1(\theta), s2(\theta),..., s_L(\theta)]^T$ is the steering vector towards angle 0. $h_{r,l}(m) = [h_{r,1}(m), h_{r,2}(m), ..., h_{r,L}(m)]^T$ is the channel information vector at range tap r. $\varepsilon(m)$ is the additive white Gaussian noise assumed to be Independent and Identically Distributed (I.I.D) for different range-azimuth bins.

In some embodiments, to locate the range-azimuth bins corresponding to the driver and reduce the impact of reflections from static objects in the vehicle, the disclosed system deploys a clutter removal algorithm to subtract the CIR from the background. The reflections from the static object is reasonably assumed to be invariant within a certain period of time, while the reflections from the driver change over time due to human motion (including body motion and motion caused by vital signals). The background profile can be estimated by taking average of the CIR over slow-time, and the calibrated CIR can be denoted as

$$\hat{h}(r,\theta,m) = h(r,\theta,m) - \frac{1}{M}\sum_{i=1}^{M} h(r,\theta,m-i), \quad (30)$$

where M is the number of samples used for clutter removal. FIGS. 22A and 22B show the effect of the background cancellation, where the raw CIR before clutter removal is shown in FIG. 22A, and the corresponding calibrated CIR after clutter removal is shown in FIG. 22B. As shown in FIGS. 22A and 22B, clutter removal reduces the background noise significantly.

In some embodiments, after extracting the dynamic CIR corresponding to the driver, the system can get the range-azimuth bins contributed by the vital signals (a.k.a. vital bins). The vital bins can be easily identified by checking the periodicity of the phase signal if the human subject stays stationary. However, the assumption of the stationary human subject barely holds in the driving scenario. To recover the periodic vital signals from the CIR involving human motion, the disclosed system uses a two-step motion compensation algorithm.

When there is a large body motion, the location of range-azimuth bins corresponding to human subject will change, as shown in FIG. 23, where the human subject sits at around 0.5m away from device at azimuth angle 0°. The human subject sways the body back-and-forth, resulting in the change of reflecting locations. The amplitude of CIR measurement is shown every 15s, for example. The profile of human reflections stays similar, as shown in FIG. 23. Therefore, to remove body movement, the 2-dimensional cross correlation between consecutive CIRs is calculated. Then the CIR at each time instance is circularly shifted to the point corresponding to the maximum cross correlation.

FIG. 24 shows the amplitude of 1-minute CIR before and after body movement compensation. For visualization, the CIR is plotted at azimuth angle 0° over range [0,0.9]m. The upper figure shows 1 minute CIR amplitude at azimuth angle 0° over range [0,0.9]m, where the distance between human subject and device changes over time. The lower figure shows the corresponding CIR amplitude after large body movement compensation, where the range tap of the human subject stays the same. It is shown that after the large body movement compensation, the bins correspond to human subject have been aligned. The 2-D Constant False Alarm Rate (CFAR) detector will be further applied over the CIR after aligning the human subject, and the candidate bins with human subject can be selected as shown in FIGS. 25A and 25B.

Although the candidate range-azimuth bins corresponding to human subject have been aligned and selected in the first step, it is still hard to locate those bins reflected by chest with periodic vital signals. The reason is that the first step can only remove the motion artifacts that are larger than the range-azimuth resolution, however, it cannot deal with the fine movements within the range-azimuth resolution. FIG. 26A shows an example of the unwrapped phase measurement of the candidate range-azimuth bins after large body movement compensation in solid lines, where the slow trend is caused by the fine movements. To recover the periodicity of vital signals, the system may further eliminate the impact of these fine movements.

In some embodiments, let $y_{r,\theta} = [y_{r,\theta}(1), y_{r,\theta}(2), \cdots, y_{r,\theta}(M)]$ to be the unwrapped phase sequence corresponding to the range r and the azimuth angle θ at the observation window, where M is the total number of samples. $[t_1, t_2, \cdots, t_M]$ denotes the time corresponding to each observation. The operation of the fine movement elimination is performed within the same range-azimuth bin over slow time.

In some embodiments, to remove the motion artifacts that have larger distance change and lower frequency compared to the vital motions, the estimation of the phase change caused by motion artifacts can be obtained by

$$\min_{\hat{f}} \sum_{m=1}^{M} \{y(m) - \hat{f}(t_m)\}^2 + \lambda \int \hat{f}''(t)^2 dt, \quad (31)$$

where $\lambda \geq 0$ is a smoothing parameter. The second term evaluates the smoothness of a function, $\hat{f}$ is the estimate of the phase change caused by motion, defined as

$$\hat{f}(t) = \sum_{m=1}^{M} \hat{f}(t_m) f_m(t), \quad (32)$$

where $f_m(t)$ are a set of spline basis function. In some embodiments, B-spline is used as the spline basis. Let $\hat{P} = [\hat{f}(t_1), \cdots, \hat{f}(t_M)]^T$, and the roughness penalty has the form

$$\int \hat{f}''(t)^2 dt = \hat{\mathbf{P}}^T \mathbf{A} \hat{\mathbf{P}}, \quad (33)$$

where the elements of A are $\int f_i''(t) f_j''(t) dt$. Therefore, one can rewrite Eqn. (31) as

$$\min_{\hat{\mathbf{P}}} \{\mathbf{y} - \hat{\mathbf{P}}\}^T \{\mathbf{y} - \hat{\mathbf{P}}\} + \lambda \hat{\mathbf{P}}^T \mathbf{A} \hat{\mathbf{P}}, \quad (34)$$

where the minimizer of Eqn. (34) is obtained as

$$\hat{\mathbf{P}}^* = (\mathbf{I} + \lambda \mathbf{A})^{-1} \mathbf{y}. \quad (35)$$

The estimation of motion can be obtained by

$$\hat{f}(t) = \hat{\mathbf{P}}^{*T} \mathbf{f}(\mathbf{t}), \quad (36)$$

where f(t) is the vector form of the spline basis function. The estimated motion artifacts can then be removed to get the clean phase revealing the vital information. FIGS. 26A to 26C illustrate the effect of fine movement cancellation, where the dashed lines in FIG. 26A show the estimated phase measurement caused by body movement. FIG. 26B shows the phase measurement after the motion artifacts are removed, where the periodicity caused by vital signals appears. The above fine movement elimination is performed over all candidate bins selected by CFAR detector and the cleaned phase of each candidate bin is saved for further analysis.

In some embodiments, after motion compensation, the phase information corresponding to the human chest show periodicity due to the modulation of both respiration and heartbeat, as shown in FIG. 26B. To filter out the bins reflected by other parts of human body (i.e., bins dominated by motion), the system can check the periodicity of the phase signals over slow time by examining their Auto-Correlation Function (ACF). The reason is that when the phase measurement contains vital signals, a peak can be observed at $\tau^*$ in its corresponding ACF, which reveals the time duration of a breathing cycle. FIG. 26C shows an example of the ACF of the phase measurement corresponding to human chest, where the time duration of a breathing cycle is about 3.7s, correspond to 16.1 RPM. The system can check the periodicity over all candidate bins corresponding to the human subject, and those bins whose peak located within the range of normal human RR are identified as vital bins for further analysis.

In some embodiments, the vital bins identified by the previous module can only reflect the compound distance change caused by respiration and heartbeat. To further estimate the vital signs including RR, HR, and HRV, the system can reconstruct the distance change caused by respiration and heartbeat respectively. For simplicity, in the following analysis, the analog form of the signal model is directly used.

In some embodiments, let $y(t) = [y_1(t), y_2(t), ..., y_B(t)]^T$ denote the vector of the phase signals of all the B vital bins. As the phase signal after movement elimination is a mixture of vital signals, one can obtain

$$\mathbf{y}(t) = \mathbf{s}_r(t) + \mathbf{s}_h(t) + \mathbf{n}(t). \quad (37)$$

where $s_r(t)$ and $s_h(t)$ denote the vector of respiration and heartbeat signal respectively, n(t) is the random phase offset introduced by noise, which is independent with the phase change caused by vital signs. To decompose the phase and get the estimate of vital signs, one can leverage the following properties. First, both respiration and heartbeat are quasi-periodic signals, whose periodicity changes slightly over time. Second, the periodicity of signals corresponding to respiration and heartbeat should stay the same in different vital bins since these signals are modulated by the same person. Third, the distance change caused by respiration and heartbeat can be different in different parts of human body due to the physiological structure (i.e., the distance change in different vital bins can be distinct).

The phase signal, therefore, can be decomposed as an ensemble of band-limited signals, denoted as $\{u_k(t)\}_{k=1}^K$, where for each component $u_k(t) = [u_{\{k,1\}}(t), u_{\{k,2\}}(t), ..., u_{\{k,B\}}(t)]^T$, the decomposed signals w.r.t. all vital bins should be compact around the same center frequency $\omega_k$ (corresponding to the property a and b). Moreover, the distance change in different vital bins should be optimized separately (corresponding to property c). The decomposition may be modeled as

$$\min_{u_{k,b}\in\mathcal{U},\omega_k\in\Omega} \quad \alpha\sum_{k=1}^{K}\sum_{b=1}^{B}\left\|\partial t\left[(\delta(t)+\frac{j}{\pi t})*u_{k,b}(t)\right]\exp(-j\omega_k t)\right\|_2^2 + \sum_{b=1}^{B}\left\|y_b(t)-\sum_{k=1}^{K}u_{k,b}(t)\right\|_2^2, \tag{38}$$

where $\mathcal{U}=\{u_{1,1},u_{1,2},\dots,u_{1,B},\dots,u_{K,B}\}$ and $\Omega = \{\omega_1, \dots, \omega_K\}$ denote the set for all components and their center frequencies, respectively. The first term in Eqn. (38) represents the bandwidth constraint, which is measured by the sum of the $L_2$ norm of the gradient of the analytic signal corresponding to each component. The second term is the fidelity constraint, which is evaluated by the quadratic penalty w.r.t. reconstruction. $\alpha$ is a parameter for balancing the bandwidth constraint and data fidelity. The optimization problem in Eqn. (38) can be solved by alternatively updating $\mathcal{U}$ and $\Omega$ until convergence.

The system may then calculate a minimization w.r.t. $u_{k,b}$. In some embodiments, to update the k-th component for vital bin b, the subproblem can be written as

$$u_{k,b}(t) = \arg\min_{u_{k,b}(t)} \alpha\left\|\partial t\left[(\delta(t)+\frac{j}{\pi t})*u_{k,b}(t)\right]\exp(-j\omega_k t)\right\|_2^2 + \left\|y_b(t)-\sum_{i=1}^{K}u_{i,b}(t)\right\|_2^2. \tag{39}$$

By using the Parseval theorem, the problem is equivalent to

$$\mathcal{u}_{k,b}(\omega) = \arg\min_{\mathcal{u}_{k,b}(\omega)} \alpha\left\|j\omega[(1+\mathrm{sgn}(\omega+\omega_k))\mathcal{u}_{k,b}(\omega+\omega_k)]\right\|_2^2 + \left\|\mathcal{y}_b(\omega)-\sum_{i=1}^{K}\mathcal{u}_{i,b}(\omega)\right\|_2^2, \tag{40}$$

where $\mathcal{u}_{k,b}(\omega)$ and $\mathcal{y}_b(\omega)$ are the Fourier transfer of $u_{k,b}(t)$ and $y_b(t)$ respectively. After performing a change of variables $\omega \leftarrow \omega - \omega_k$ in the first term, and using the Hermition symmetry of the real signals in the spectrum for the second term, the above problem can be rewritten as

$$\mathcal{u}_{k,b}(\omega) = \arg\min_{\mathcal{u}_{k,b}(\omega)} \int_0^\infty 4\alpha(\omega-\omega_k)^2|\mathcal{u}_{k,b}(\omega)|^2 + 2|\mathcal{y}_b(\omega)-\sum_{i=1}^{K}\mathcal{u}_{i,b}(\omega)|^2 d\omega. \tag{41}$$

The updated solution can be expressed as

$$\mathcal{u}_{k,b}(\omega) = \frac{\mathcal{y}_b(\omega)-\sum_{i,i\neq k}\mathcal{u}_{i,b}(\omega)}{1+2\alpha(\omega-\omega_k)^2}. \tag{42}$$

The system may then calculate a minimization w.r.t. $\omega_k$. The center frequencies $\omega_k$ only appear in the bandwidth constraint and thus the updating function can be written as

$$\omega_k = \operatorname*{argmin}_{\omega_k}\sum_{b=1}^{B}\left\|\partial t\left[(\delta(t)+\frac{j}{\pi t})*u_{k,b}(t)\right]\exp(-j\omega_k t)\right\|_2^2. \tag{43}$$

As discussed above, one can find the optimum in Fourier domain, and have

$$\omega_k = \operatorname*{argmin}_{\omega_k}\sum_{b=1}^{B}\int_0^\infty(\omega-\omega_k)^2|\mathcal{u}_{k,b}(\omega)|^2 d\omega. \tag{44}$$

The minimizer of the above problem is

$$\omega_k = \frac{\sum_b \int_0^\infty \omega |u_{k,b}(\omega)|^2 d\omega}{\sum_b \int_0^\infty |u_{k,b}(\omega)|^2 d\omega}, \tag{45}$$

FIGS. 27A and 27B show an example of vital signals decomposition, where the time and frequency domain of the original phase as well as the decomposition components are shown in FIG. 27A and FIG. 27B, respectively. The first sub-figure in FIG. 27A shows the phase measurement after motion cancellation. The respiration and heartbeat component are shown in the second and third sub-figures of FIG. 27A, respectively. The fourth sub-figure of FIG. 27A shows the decomposition residue. The information of 3 different vital bins is shown. Although the distance change of different vital bins are distinct, as shown in FIG. 27A, the periodicity of the signal of each component stays the same, as shown in FIG. 27B. In other words, components corresponding to vital signals are perfectly aligned over all vital bins, e.g., the first component represents the distinct displacement cause by respiration over different vital bins, and the second component represents the distinct displacement caused by heartbeat over different vital bins. The residue of the decomposition contains noise including car vibrations, as shown in FIGS. 27A and 27B. In some embodiments, to further reduce the noise impact, the system can reconstruct the vital signals by combining the signals of all vital bins using empirical mean, i.e., $s_{\mathbf{r}}(t) = \frac{1}{B}\sum_b u_{i,b}(t)$ and $s_h(t) = \frac{1}{B}\sum_b u_{j,b}(t)$ where the i-th and j-th components correspond to the respiration signal and heartbeat signal respectively. The RR is estimated by finding the first peak of the ACF of the estimated respiration signal, as shown in FIG. 26C. In addition, the FFT is further performed on the estimated heartbeat signal to get the estimation of HR. Moreover, the exact time of each heartbeat can be further extracted from the reconstructed heartbeat wave to estimate the IBI.

FIGS. 28A to 28C show the estimated vital signs versus their ground-truths of a 2-minute dataset, where a 1-minute window is employed for the time-frequency domain transform (i.e., ACF and FFT). The estimated RR and HR are shown in solid lines in FIG. 28A, which match with the ground-truth, shown as dashed lines in FIG. 28A. FIG. 28B shows a segment of the estimated heartbeat wave, and the ground-truth of the exact time of each heartbeat is marked as vertical dashed lines. The estimated IBIs of the whole data and their corresponding ground-truth are shown in FIG. 28C. Clearly, the disclosed system achieves high accuracy in vital signs estimation, and the Root-Mean-Squared-Error (RMSE) of IBI estimation in FIG. 28C is 40.77ms, corresponding to the 96% relative accuracy.

According to some embodiments, extensive experiments are performed to evaluate the performance of the disclosed system. One can compare the performance with the state-of-art work under different experimental settings. In some embodiments, one can conduct experiments using a COTS mmWave radar, , where the 2 Tx antennas and 4 Rx antennas are configured in TDM-MIMO mode. The device can achieve a theoretical azimuth resolution of 15°, and the field-of-view (FoV) is 100° in horizontal plane, which is large enough to cover the driver. The parameters corresponding to the FMCW radar setting are listed in Table II. The ground truth of heartbeat is captured by a commercial ECG sensor, and the ground truth of breathing is measured by a respiration belt.

Table II: Parameters used

| System Parameters | Value | System Parameters | Value |
|---|---|---|---|
| Starting Frequency | 77 GHz | Max. Range | 1.2 m |
| Slow Time Sampling | 1000 chirps/sec | Range Resolution | 3.75 cm |
| Chirp Duration | 57.14 us | Field-of- View | [-50°, 50°] |

In some embodiments, 4 volunteers (2 males and 2 females) are recruited to help on the data collection including 2 different device locations. All of the participants do not have any cardiac history, and more information about the testers is shown in Table III. The driving route is a cycle of 50.7 miles including local routes and highway, where the road conditions can be referred to GIS Dataset. During the data collection, the driver is driving following their own habits with no further constraints, and a copilot is responsible for collecting data.

Table III: Information of the participants

| Subject ID | 1 | 2 | 3 | 4 |
|---|---|---|---|---|
| Gender | M | M | F | F |
| Height (cm) | 174 | 172 | 160 | 166 |
| Weight (Kg) | 79.8 | 70 | 61.4 | 50 |

(continued)

| Subject ID | 1 | 2 | 3 | 4 |
|---|---|---|---|---|
| BMI | 26.36 | 23.66 | 23.98 | 18.14 |

To further evaluate the performance of the disclosed system, one can compare it with the state-of-art work, $V^2$iFi, which estimates driver's vital signs using the CIR of a UWB radar. With assumption that the distance change caused by vital signals are identical in different vital bins, $V^2$iFi estimates the respiration and heartbeat signal by Multi-Sequence Variational Mode Decomposition (MS-VMD). The $V^2$iFi cannot estimate vital signs when drivers have body motion. For fair comparison, motion compensation disclosed here is also applied to $V^2$iFi to remove the motion artifacts before estimating the vital signals.

FIGS. 30A to 30C depict the overall performance of the disclosed system and $V^2$iFi. The experiments include road tests with different pavement conditions, device locations, as well as the controlled experiments with different motion types, including stationary, head motion, hand motion and back-and-forth torso motion for 4 different users. FIG. 30A plots the empirical Cumulative Distribution Function (CDF) of absolute RR estimation error, where the 90-percentile error for the disclosed system and $V^2$iFi are 0.64 RPM and 0.86 RPM respectively. The performance improvement is more significant for HR estimation, where the disclosed system achieves a median error of 0.82 BPM, and the median error of $V^2$iFi is 5.12 BPM, as shown in FIG. 30B. FIG. 30C shows the performance of IBI estimation for the two systems, where $V^2$iFi yields about 84ms medium error, while the disclosed system achieves a medium error of 46ms, outperforming $V^2$iFi by about 45.2%. The Bland-Altman plot is shown in FIG. 29, where the solid line shows the mean of the difference between the estimation and the ground-truth, and the dashed lines show the +1.96 times of standard deviation of the difference. The estimation is nearly unbiased compared with the ground-truth.

In some embodiments, one can investigate the impact of device location on vital signs estimation. The radar is placed at the top of windshield (denoted as "up"), and under the steering wheel (denoted as "down"). FIGS. 31A to 31C plot the CDF of the absolute error of RR, HR and IBI estimations, where lines marked with squares correspond to the "down" setting, and lines marked with diamonds correspond to the "up" setting.

It is shown that the "down" setting achieves better performance for all estimations. Specifically, for the disclosed system, the median error for RR, HR and IBI estimation are 0.2 RPM, 0.65 BPM and 38 ms respectively for the "down" setting. However, it increases to 0.28 RPM, 1.91 BPM and 56 ms for the "up" setting, corresponding to 40%, 193.85% and 47.37% performance degradation, respectively. One can observe the similar phenomenon in $V^2$iFi, where the median error for all the 3 metrics increases when the device is place as the "up" setting, as shown in dashed lines in FIGS. 31A to 31C. The reason is that when the device is mounted on the windshield, the vital bins mainly correspond to the chest, whereas, for the "down" setting, the vital bins mainly correspond to the lower chest and the abdomen. For the same scenario (e.g., car decelerates due to brake), severer motion will be involved in the upper chest than the abdomen. Therefore, the SNR of vital signals for the "down" setting is larger than the "up" setting. However, compared to the disclosed system, $V^2$iFi yields larger estimation error for all the 3 metrics, because it is less robust to noise.

In some embodiments, as driving involves different kinds of motion of head, hand and body when looking at the side mirror, or controlling the steering wheel, etc., to better understand the impact of different motion types, one can conduct controlled experiments and analyze their corresponding impact, as shown in FIGS. 32A to 32C. During the experiment, drivers are asked to continuously perform specific type of motion in a parked car, including sitting stationary, head motion to check the surroundings, hand motion to operate steering wheel and randomly sway their body back-and-forth to emulate the body motion caused by acceleration and deceleration. Every data collection lasts for 2 minutes for both "up" setting and "down" setting, where 32 sets of data are collected for analysis.

FIG. 32A shows the CDF of RR estimation error with different motion types, where one can see that the median estimation error when driver performs head motion is nearly the same as the stationary case. The performance slightly degrade when the driver performs hand motion, where the median error increase from 0.11 RPM to 0.12 RPM compare to the stationary setting. However, for the large back-and-forth motion, one can observe a severe performance degradation, and its median error of RR estimation is 0.19 RPM, 72.73% worse than the stationary setting. Similar performance degradation can be observed in terms of HR and IBI estimation.

FIG. 32B shows that the median error of HR estimation increases from 0.35 BPM corresponding to the stationary setting to 0.68 BPM and 0.75 BPM when the driver performs hand and back-and-forth motion, respectively. As for IBI estimation, the median error when the driver performs sitting stationary, head motion, hand motion and random back-and-forth motion are 37 ms, 41 ms, 45 ms and 68 ms, respectively, as shown in FIG. 32C.

The estimation performance of $V^2$iFi are also plotted in dashed lines in FIGS. 32A to 32C, where the similar performance degradation can be observed. One can see that $V^2$iFi is more vulnerable to motion artifacts, and the performance degradation of hand and back-and-forth motion is more severe compared to the disclosed system. Specifically, one can see that the median error of HR estimation for the back-and-forth setting is larger than 10 BPM, which is almost useless for driver's HR estimation.

In some embodiments, one can investigate the impact of the window length on HRV calculation. Known that the HRV metrics can be derived from the IBI sequence, FIGS. 33A to 33D show 4 different commonly used HRV metrics with window length ranging from 15s to 120s. The mean of IBI and the standard deviation of the IBIs (SDRR) under different time window are shown in FIG. 33A and FIG. 33B, respectively. The Root-Mean-Square-of-Successive Differences (RMSSD) is shown in FIG. 33C, which can be calculated by

$$\text{RMSSD} = \sqrt{\frac{1}{N_{\text{IBI}}-1}\sum_{i=2}^{N_{\text{IBI}}} (\text{IBI}(i) - \text{IBI}(i-1))^2}, \tag{46}$$

where $N_{IBI}$ is the total number of IBIs in the given time window. FIG. 33D shows the the percentage of successive IBI that differ by more than 50ms (pNN50), which can be calculated by

$$\text{pNN50} = \frac{\sum_{i=2}^{N_{\text{IBI}}} 1\{(\text{IBI}(i)-\text{IBI}(i-1))>50ms\}}{N_{\text{IBI}}}, \tag{47}$$

where 1{·} is the indicator function. As shown in FIG. 33A, the mean of IBI barely changes over the window length. However, the other 3 metrics (i.e., SDRR, RMSSD and pNN50) increase with the window length for both estimation and ground-truth, as shown in FIGS. 33B to 33D. Furthermore, the estimation error of SDRR increases from 6.5ms to 8.7ms when the window length increases from 15s to 120s. Similar performance/trend can be observed in RMSSD, where the estimation error increases from 1.5ms to 4.1ms when the window length increases from 15s to 120s.

FIG. 34 illustrates a flow chart of an exemplary method 3400 for wireless vital sign monitoring, according to some embodiments of the present disclosure. At operation 3402, a wireless signal is transmitted by a transmitter through a wireless channel of a venue. At operation 3404, the wireless signal is received by a receiver through the wireless channel, wherein the wireless channel is being impacted by an object motion of an object in the venue, and wherein the object motion comprises at least one non-periodic body motion and at least one periodic vital-sign motion of the object. At operation 3406, the space around the venue is segmented into a plurality of sectors based on a beamforming and the received wireless signal, wherein each sector is associated with a spatial direction relative to an array of antennas on the transmitter and/or the receiver. At operation 3408, a plurality of time series of channel information (CI) of the wireless channel is obtained based on the beamforming, wherein each time series of CI (TSCI) is associated with a respective sector of the plurality of sectors. At operation 3410, the object motion of the object is isolated in the plurality of TSCI to generate a plurality of isolated TSCI. At operation 3412, the at least one non-periodic body motion of the object is compensated for in the plurality of isolated TSCI to generate a plurality of compensated TSCI. At operation 3414, the at least one periodic vital-sign motion of the object is monitored based on the plurality of compensated TSCI. The order of the operations in FIG. 34 may be changed according to various embodiments of the present teaching.

Thus, the present teaching discloses a novel system that can accurately detect driver's vital signs in the presence of practical driving motions using the reflections of RF signals off the human subject only. This is the first contact-free driver vital sign monitoring system that can detect driver's HRV considering driver's motion artifacts using commercial millimeter-wave (mmWave) radio. The system can detect the driver's vital signs without any prior calibration. A novel two-step motion compensation module is devised, where the motion artifact that is larger than the range-azimuth resolution is first eliminated by using the 2D cross correlation of the CIR. Then the fine motion artifact that is smaller than the range-azimuth bin is compensated by smoothing spline. Vital signals are obtained by jointly decomposing the phase measurements of all the reflections containing vital motions with several band-limited components, where the respiration and heartbeat are the component whose amplitude and center frequency satisfy the typical respiration and heartbeat signals.

For example, to locate the reflections from the driver, the system first performs beamforming to get the CIR with different range-azimuth bins, followed by a clutter removal module to remove the reflection from the background. Then the 2-dimensional correlation between different CIR samples have been used to eliminate large displacement caused by body roaming. Finer motion artifacts are further removed by the smoothing spline, which can accurate estimate motion artifacts without dedicated choose of hyper-parameter as in polynomial fitting. The displacement caused by respiration and heartbeat are then estimated by jointly optimizing the decomposition of vital signals in all vital bins, and the RR, HR and IBI can be extracted from the reconstructed respiration and heartbeat wave.

The system may be prototyped using a commercial mmWave radio to conduct experiments in the real driving scenario to evaluate the performance. Experimental results show that the disclosed system can estimate vital signs accurately with driving motion artifacts, outperforming the state-of-art works.

While average CIR in a time window is used to estimate the reflections from the background for clutter removal in some embodiments, other clutter removal method, such as the CIR difference in slow time domain, can be used in other embodiments. In some embodiments, a detrending method, such as the polynomial fitting, may be used to estimate the fine motion artifacts.

In some embodiments, a wireless vital sign monitoring method includes steps s1 to s8 as described below.

At step s1: capture CSI using multiple transmit (Tx) antenna and multiple receive (Rx) antenna. At step s2: apply beamforming to get directional CSI (e.g. CIR). At step s3: remove clutters to reduce the impact of static reflecting objects, which includes steps s3a and s3b.

At step s3a: compute the background profile at each (theta, distance, time index) by taking average of the CIR over a time window. At step s3b: subtract the background profile from the CIR.

At step s4: determine the point-of-interest (PoI) (i.e., the (theta, distance)) corresponding to the reflections of the drive for each time window, which includes steps s4a to s4c. At step s4a: compensate large body movement which is larger than the range-azimuth resolution, including step s4a1 to calculate 2-dimention (2D) cross correlation between consecutive CIRs and step s4a2 to circularly shift the CIR at each time instance to the point (i.e., (theta, distance)) correspond to the maximum cross correlation. At step s4b: determine subject is present in the direction if the time-averaged magnitude response is greater than a threshold T1, wherein the threshold T1 may be a 2-dimensional CFAR filtering of CIR magnitude |h| in theta and distance direction. At step s4c: compensate fine body motion which is smaller than the range-azimuth resolution, wherein the fine body motion may be estimated by the smoothing spline of the phase measurement corresponding to the reflection off the driver.

At step s5: determine the PoI containing vital motions, including step s5a, for each PoI, compute the ACF of the phase and find significant feature point (e.g. first peak) PI, and step s5b, classify the PoI as the vital bin if PI is greater than a threshold T2.

At step s6: extract the respiration signal and heartbeat signal by jointly decomposing the vital signal in all vital bins with a few band-limited signals, which includes steps s6a to s6c.

At step s6a: given a default setting of component number K and the parameter $\alpha$ for balancing, the bandwidth constraint and data fidelity, alternatively optimize the components and their center frequencies. At step s6b: check whether there is a component corresponding to respiration by some features, wherein the component corresponds to respiration wave if the amplitude of the signal locate in range [T3, T4], and its center frequency should locate in range [T5, T6]. At step s6c: check whether there is a component corresponding to heartbeat by some features, wherein the component corresponds to heartbeat wave if the amplitude of the signal locate in range [T7, T8], and its center frequency should locate in range [T9, T10].

At step s7: reconstruct respiration wave and heartbeat wave by averaging the respiration and heartbeat wave over all the vital bins, wherein the reconstructed respiration and heartbeat signal may be further normalized by using their envelope to reduce the noise.

At step s8: calculate vital signs by using the reconstructed vital signals, which includes steps s8a to s8c. At step s8a: calculate the respiration rate (e.g., first peak location of the ACF). At step s8b: calculate the heart rate (e.g., highest peak of the spectrum in

range [T9, T10]). At step s8c: identify the exact time of each heartbeat and then calculate the inter-beat intervals to estimate heart rate variability (HRV), wherein the exact time of each heartbeat can be identified by several ways, e.g., identify the peaks of the heartbeat waves or identify the zero-crossing points.

The following numbered clauses provide examples for vital sign monitoring based on wireless beamforming.

Clause C1. A method/device/system/software of a wireless beamforming vital sign monitoring system, comprising: receiving a wireless signal by a Type 2 heterogeneous wireless device, wherein the wireless signal is transmitted to the Type 2 device by a Type 1 heterogeneous wireless device through a wireless multipath channel of a venue, wherein at least one of the Type1 device and the Type2 device comprises an array of antennas for the transmission or reception of the wireless signal, wherein the wireless multipath channel is impacted by an object motion of an object in the venue, wherein the object motion comprises a non-periodic body motion of the object and at least one periodic vital-sign motion of the object; segmenting the space into a plurality of sectors based on a beamforming and the received wireless signal using a processor, a memory and a set of instructions, each sector being associated with a spatial direction relative to the array of antenna; obtaining a plurality of time series of channel information (CI) of the wireless multipath channel based on the beamforming, each time series of CI (TSCI) being associated with a respective sector; isolating the object motion of the object in the plurality of TSCI; compensating for the non-periodic body motion of the object in the plurality of the isolated TSCI; monitoring the at least one periodic vital-sign motion of the object based on the plurality of compensated TSCI.

Step s3 may be reflected in the following clause according to some embodiments. Clutter removal to remove effect of background of the venue to isolate the object motion of the object in each TSCI. Clutter removal is achieved by subtracting an average of past few CSI from current CSI. Assuming the object (person) is moving around randomly, the effect of the object motion (especially for the periodic vital-sign motion) tends to cancel itself in the averaging. Thus the averaging is an estimation of the background CSI. The subtraction is a kind of filtering (MA filtering).

Clause C2. The method/device/system/software of the wireless vital sign monitoring system of Clause C1, further comprising: filtering each TSCI to isolate the object motion of the object in the TSCI.

Clause C3. The method/device/system/software of the wireless vital sign monitoring system of Clause C1 or C2, further comprising: wherein the filter is one of: a moving-average (MA) filter, an autoregressive (AR) filter, or an autoregressive-moving-average (ARMA) filter.

Clause C4. The method/device/system/software of the wireless vital sign monitoring system of Clause C2 or C3, further comprising: wherein the filter computes a filtered CI by subtracting a weighted average of a number of past CI from a current CI.

Step s4 includes steps s4a, s4b and s4c, and may be reflected in the following clause according to some embodiments. Step s4a computes cross correlation and shift consecutive CIR to compensate for large body movement. Shifting a first CI w.r.t. a second CI to compensate large body movement during the time duration between the first CI and the second CI.

Clause C5. The method/device/system/software of the wireless vital sign monitoring system of Clauses C2 to C4, further comprising: shifting a first CI of a first TSCI; replace a second CI of a second TSCI with the shifted first CI to compensate for a large non-periodic body motion of the object.

According to some embodiments, the following clauses may reflect computing cross correlation between first CI and second CI, and finding max point to find the shift amount.

Clause C6. The method/device/system/software of the wireless vital sign monitoring system of any previous C Clause, further comprising: wherein both the first CI and the second CI are associated with a common time stamp; determining a third CI of the second TSCI as a reference CI, wherein the third CI is temporally adjacent to the common time stamp of both the first CI and the second CI; for each of the plurality of TSCI: computing a respective cross correlation function between the temporal profile of the reference CI and the temporal profile of a respective CI of the respective TSCI, wherein the respective CI is associated with the common time stamp, computing a respective maximum point of the respective cross correlation function, computing a shift amount; computing a dominant maximum point among all the maximum points; determining the TSCI associated with the dominant maximum point as the first TSCI; shifting the temporal profile of the first CI of the first TSCI by an amount equal to a time shift associated with the dominant maximum point; replacing the second CI of the second TSCI by the shifted first CI.

According to some embodiments, the following Clause may reflect a special case when object body moves in radial direction (or radial body motion, i.e. no change in direction/sector, with change in distance). Only one TSCI is involved.

Clause C7. The method/device/system/software of the wireless vital sign monitoring system of any previous C Clause, further comprising: wherein the first TSCI and the second TSCI are a common TSCI; wherein the first CI is the second CI; wherein each CI comprises a temporal profile; wherein shifting the first CI is to shift the temporal profile of the first CI; computing a cross correlation function between the temporal profile of first CI and the temporal profile of a third CI of the common TSCI, wherein the third CI is temporally adjacent to the first CI; computing a maximum point of the cross correlation function; shifting the temporal profile of the first CI by an amount equal to a time shift associated with the maximum point; replacing the first CI of the first TSCI by the shifted first CI.

Clause C8. The method/device/system/software of the wireless vital sign monitoring system of any previous C Clause, further comprising: shifting the first CI using one of: circular shifting, or non-circular shifting.

Step s4b may be reflected in the following clause according to some embodiments to use smoothing spline to compensate for small body movement.

Clause C9. The method/device/system/software of the wireless vital sign monitoring system of any previous C Clause, further comprising: for each of the plurality of TSCI: computing a magnitude feature of a weighted average of the respective CI of the respective TSCI in a time window, associating the respective TSCI, and a respective associated sector, with the object if the magnitude feature is greater than a threshold.

Step s4c may be reflected in the following clause according to some embodiments to use smoothing spline to compensate for small body movement.

Clause C10. The method/device/system/software of the wireless vital sign monitoring system of nay previous C Clause, further comprising: computing at least one time series of CI feature (CIF), each time series of CIF (TSCIF) associated with a respective TSCI associated with the object with each of its CIF being a feature of a respective CI of the respective TSCI; computing an estimate of a small non-periodic body motion of the object based on a smoothing spline; subtracting the estimate from the TSCIF to compensate for a small non-periodic body motion of the object.

Clause C11. The method/device/system/software of the wireless vital sign monitoring system of Clause C10, further comprising: wherein the feature of a CI comprises at least one of: a phase, a magnitude, a function of phase, a function of magnitude, a function of phase and magnitude, or the CI.

Step s5 may be reflected in the following clause according to some embodiments to identify vital-sign bin/sector.

Clause C12. The method/device/system/software of the wireless vital sign monitoring system of Clause C10 or C11, further comprising: classifying a sector as a vital-sign sector based on the associated TSCIF, wherein the vital-sign sector associated with the at least one periodic vital-sign motion of the object.

Clause C13. The method/device/system/software of the wireless vital sign monitoring system of Clauses C10 to C12, further comprising: computing an autocorrelation function (ACF) of each TSCIF; computing a second feature of the ACF; classifying the sector associated with the TSCI associated with the TSCIF as a vital-sign sector if the second feature exceeds is threshold.

Clause C14. The method/device/system/software of the wireless vital sign monitoring system of Clause C13, further comprising: wherein the second feature comprises at least one of: a maximum point, a local maximum point, a first positive local maximum point, a second local maximum point, a global maximum point, a magnitude of a maximum point, a time associated with a maximum point, a timing associated with a maximum point, a minimum point, a local minimum point, a first positive local minimum point, a second local minimum point, a global minimum point, a magnitude of a minimum point, a time associated with a minimum point, a timing associated with a minimum point, a zero-crossing point, a first positive zero-crossing point, a second positive zero-crossing point, a time associated with a zero-crossing point, a time duration between two zero-crossing points, or a timing associated with a zero-crossing point.

Step s6a may be reflected in the following clause according to some embodiments to decompose phase of TSCI into a sum of breathing signal and a heart-beat signal.

Clause C15. The method/device/system/software of the wireless vital sign monitoring system of Clauses C12 to C14, further comprising: wherein there is at least one vital-sign section; decomposing each TSCIF associated with a respective vital-sign section into at least one respective periodic component, each periodic component corresponding to a periodic vital-sign motion of the object, wherein each periodic component is associated with a respective frequency.

Clause C16. The method/device/system/software of the wireless vital sign monitoring system of Clauses C10 to C15, further comprising: decomposing each TSCIF into the at least one respective periodic component based on an iterative optimization.

Step s6b and s6c may be reflected in the following clause according to some embodiments about amplitude range and frequency range constraint.

Clause C17. The method/device/system/software of the wireless vital sign monitoring system of Clause C15 or C16, further comprising: wherein each of the at least one respective periodic component is constrained to have at least one of: a respective frequency within a respective frequency range, or a respective amplitude within a respective amplitude range.

Clause C18. The method/device/system/software of the wireless vital sign monitoring system of Clauses C15 to C17, further comprising: wherein each of the at least one respective periodic component is associated with a respective likelihood function within the respective frequency range.

Step s7 may be reflected in the following clause according to some embodiments to aggregate multiple vital-sign sector by averaging them.

Clause C19. The method/device/system/software of the wireless vital sign monitoring system of Clauses C15 to C18, further comprising: computing at least one aggregate periodic component, each aggregate periodic component comprising a weighted average of the respective decomposed periodic components associated with the at least one vital-sign sector; monitoring each periodic vital-sign motion by analyzing the corresponding aggregate periodic component.

Step s8 may be reflected in the following clause according to some embodiments to compute various statistics/analytics.

Clause C20. The method/device/system/software of the wireless vital sign monitoring system of any previous C Clause, further comprising: analyzing an aggregate periodic component by computing at least one of: an instantaneous frequency, instantaneous period, instantaneous timing, vital sign timing, maximum, minimum, zero-crossing, instantaneous vital sign beat, instantaneous vital-sign interval, instantaneous beat interval between adjacent beat, average, average frequency, average period, average interval, moving average, statistics, statistics of the frequency, statistics of the period, statistics of the interval, mean, median, mode, variance, standard deviation, variation, derivative, slope, total variation, absolute variation, square variation, spread, dispersion, variability, deviation, absolute deviation, square deviation, total deviation, divergence, range, interquartile range, skewness, kurtosis, L-moment, coefficient of variation, quartile coefficient of dispersion, mean absolute difference, Gini coefficient, relative mean difference, median absolute deviation, average absolute deviation, coefficient of dispersion, entropy, variance-to-mean ratio, maximum-to-minimum ratio, variation measure, regularity measure, similarity measure, likelihood, probability distribution function, histogram, sample distribution, moment generating function, expected value, expected function, correlation, correlation of two CI, correlation of two DI, correlation coefficient, correlation indicator, autocorrelation, a feature of autocorrelation function (ACF), cross correlation, inner product, dot product, outer product, covariance, auto-covariance, cross covariance, discrimination score, similarity score, similarity measure, similarity between two CI, similarity between two CI, similarity between two vectors of CI, similarity between two windows of CI, similarity between two windows of CI with unequal window length, similarity between two DI, similarity between two DI, similarity between two vectors of DI, similarity between two windows of DI, similarity between two windows of DI with unequal window length, distance, distance score, distance measure between two CI, distance measure between two vectors of CI, distance measure between two windows of CI, distance measure between two windows of CI aligned and mapped, distance measure between two windows of CI aligned using dynamic time warping (DTW), distance measure between two DI, distance measure between two vectors of DI, distance measure between two windows of DI, distance measure between two windows of DI aligned and mapped, distance measure between two windows of DI aligned using dynamic time warping (DTW), Euclidean distance, absolute distance, L-1 distance, L-2 distance, L-k distance, weighted distance, graph distance, distance metric, norm, L-1 norm, L-2 norm, L-k norm, location, localization, location coordinate, change in location, position, map position, height, horizontal location, vertical location, distance, displacement, speed, acceleration, rotational speed, rotational acceleration, direction, angle of motion, azimuth, direction of motion, rotation, path, deformation, transformation, shrinking, expanding, positional characteristics, gait, gait cycle, gesture, handwriting, head motion, mouth motion, hand motion, leg motion, body motion, heart motion, internal organ motion, tool motion, machine motion, complex motion, combination of multiple motions, motion trend, repeatedness, periodicity, pseudo-periodicity, impulsiveness, sudden-ness, fall-down occurrence, recurrence, transient event, behavior, transient behavior, period, time trend, temporal profile, temporal characteristics, occurrence, time, timing, starting time, initiating time, ending time, duration, history, motion classification, motion type, change, temporal change, frequency change, CI change, DI change, timing change, gait cycle change, measure of at least one of: repeatedness, periodicity, measure of variability, frequency spectrum, frequency characteristics, frequency, presence, absence, proximity, approaching, receding, object identifier, object composition, mouth-related rate, eye-related rate, walking rate, breathing rate, heart rate, tidal volume, depth of breath, inhale time, exhale time, inhale time to exhale time ratio, airflow rate, heart beat-to-beat interval, heart rate variability, motion detection statistics, motion identification statistics, motion recognition statistics, signal statistics, signal dynamics, anomaly, parameter, motion magnitude, motion phase, motion signal transformation, motion feature, presence of object, absence of object, entrance of object, exit of object, change of object, motion cycle, motion count, gait cycle, motion rhythm, deformation motion, size, length, area, volume, capacity, shape, form, tag, starting/initiating location, ending location, starting/initiating quantity, ending quantity, event occurrence, event statistics, fall-down event, security event, accident event, home event, office event, factory event, warehouse event, manufacturing event, assembly line event, maintenance event, vehicle-related event, navigation event, tracking event, door event, door-open event, door-close event, window event, window-open event, window-close event, repeatable event, one-time event, consumed quantity, unconsumed quantity, state, physical state, health state, well-being state, emotional state, mental state, another event, analytics, or output responses.

The following numbered clauses provide examples for vital sign monitoring based on wireless beamforming.

Clause D1. A system for vital sign monitoring based on wireless beamforming, comprising: a transmitter configured to transmit a wireless signal through a wireless channel of a venue; a receiver configured to receive the wireless signal through the wireless channel that is being impacted by an object motion of an object in the venue, wherein at least one of the transmitter or the receiver comprises an array of antennas used to transmit or receive the wireless signal, the object motion comprises at least one non-periodic body motion of the object and at least one periodic vital-sign motion of the object; and a processor configured for: segmenting space around the venue into a plurality of sectors based on a beamforming and the received wireless signal, wherein each sector of the plurality of sectors is associated with a spatial direction relative to the array of antennas, obtaining a plurality of time series of channel information (CI) of the wireless channel based on the beamforming, wherein each time series of CI (TSCI) of the plurality of TSCI is associated with a respective sector of the plurality of sectors, isolating the object motion of the object in the plurality of TSCI to generate a plurality of isolated TSCI, compensating for the at least one non-periodic body motion of the object in the plurality of isolated TSCI to generate a plurality of compensated TSCI, and monitoring the at least one periodic vital-sign motion of the object based on the plurality of compensated TSCI.

Clause D2. The system of Clause DI, wherein isolating the object motion of the object comprises: filtering each TSCI of the plurality of TSCI based on a filter to isolate the object motion of the object in the TSCI.

Clause D3. The system of Clause DI or D2, wherein the filter is one of: a moving-average (MA) filter, an autoregressive (AR) filter, or an autoregressive-moving-average (ARMA) filter.

Clause D4. The system of Clause D2 or D3, wherein filtering each TSCI comprises: subtracting a weighted average of a number of past CI from a current CI in the TSCI to generate a filtered CI.

Clause D5. The system of any previous D Clause, wherein compensating for the at least one non-periodic body motion of the object comprises: shifting a first CI of a first TSCI in the plurality of isolated TSCI; and replacing a second CI of a second TSCI in the plurality of isolated TSCI with the shifted first CI to compensate for a first non-periodic body motion of the object, when the first non-periodic body motion is larger than a range-azimuth resolution.

Clause D6. The system of any previous D Clause, wherein compensating for the at least one non-periodic body motion of the object further comprises: determining a third CI of the second TSCI as a reference CI, wherein both the first CI and the second CI are associated with a common time stamp, wherein the third CI is temporally adjacent to the common time stamp of both the first CI and the second CI; for each respective TSCI of the plurality of TSCI: computing a respective cross correlation function between a temporal profile of the reference CI and a temporal profile of a respective CI of the respective TSCI, wherein the respective CI is associated with the common time stamp, and computing a respective maximum point of the respective cross correlation function; computing a dominant maximum point among all of the maximum points; determining the TSCI associated with the dominant maximum point as the first TSCI; shifting the first CI of the first TSCI based on shifting a temporal profile of the first CI by an amount equal to a time shift associated with the dominant maximum point; and replacing the second CI of the second TSCI by the shifted first CI.

Clause D7. The system of any previous D Clause, wherein: the first TSCI and the second TSCI are a common TSCI; the first CI is the second CI; each CI comprises a temporal profile; shifting the first CI comprises shifting the temporal profile of the first CI; and compensating for the at least one non-periodic body motion of the object further comprises: computing a cross correlation function between the temporal profile of the first CI and the temporal profile of a third CI of the common TSCI, wherein the third CI is temporally adjacent to the first CI, computing a maximum point of the cross correlation function, shifting the temporal profile of the first CI by an amount equal to a time shift associated with the maximum point, and replacing the first CI of the first TSCI by the shifted first CI.

Clause D8. The system of Clauses D5 to D7, wherein: the first CI is shifted using one of: circular shifting or non-circular shifting; and compensating for the at least one non-periodic body motion of the object comprises: for each respective TSCI of the plurality of TSCI, computing a magnitude feature of a weighted average of a respective CI of the respective TSCI in a time window, and for each respective TSCI of the plurality of TSCI, associating the respective TSCI and a respective associated sector with the object when the magnitude feature is greater than a threshold.

Clause D9. The system of any previous D Clause, wherein compensating for the at least one non-periodic body motion of the object further comprises: computing at least one time series of CI feature (CIF), wherein each respective time series of CIF (TSCIF) of the at least one TSCIF is associated with a corresponding TSCI associated with the object, each CIF of the respective TSCIF is a feature of a respective CI of the corresponding TSCI; computing an estimate of a second non-periodic body motion of the object based on a smoothing spline, wherein the second non-periodic body motion is smaller than the range-azimuth resolution; and subtracting the estimate from the at least one TSCIF to compensate for the second non-periodic body motion of the object.

Clause D10. The system of Clause D8 to D10, wherein the feature of the respective CI comprises at least one of: a phase, a magnitude, a function of phase, a function of magnitude, or a function of phase and magnitude, of the respective CI.

Clause D11. The system of any previous D Clause, wherein monitoring the at least one periodic vital-sign motion of the object comprises: classifying a particular sector among the plurality of sectors as a vital-sign sector based on the at least one TSCIF, wherein the vital-sign sector is associated with the at least one periodic vital-sign motion of the object.

Clause D12. The system of Clause D11, wherein classifying the sector comprises: computing at least one autocorrelation function (ACF) based on the at least one TSCIF, each ACF being an ACF of a respective TSCIF associated with a respective TSCI of a respective sector; computing at least one feature point of the at least one ACF, each feature point being of a respective ACF associated with a respective sector; and classifying the particular sector associated with a particular feature point as the vital-sign sector when the particular feature point exceeds a threshold.

Clause D13. The system of Clause D12, wherein the feature point comprises at least one of: a maximum point, a magnitude of a maximum point, a timing associated with a maximum point, a minimum point, a magnitude of a minimum point, a timing associated with a minimum point, a zero-crossing point, a time duration between two zero-crossing points, or a timing associated with a zero-crossing point.

Clause D14. The system of any previous D Clause, wherein monitoring the at least one periodic vital-sign motion of the object further comprises: decomposing each TSCIF associated with any vital-sign sector into at least one respective periodic component, wherein each periodic component corresponds to a respective periodic vital-sign motion of the object, wherein each periodic component is associated with a respective frequency.

Clause D15. The system of any previous D Clause, wherein each TSCIF is decomposed into the at least one respective periodic component based on an iterative optimization.

Clause D16. The system of Clause D15, wherein each of the at least one respective periodic component is constrained to have at least one of: a respective frequency within a respective frequency range, or a respective amplitude within a respective amplitude range.

Clause D17. The system of Clause D15 or D16, wherein each of the at least one respective periodic component is associated with a respective likelihood function within the respective frequency range.

Clause D18. The system of any previous D Clause, wherein monitoring the at least one periodic vital-sign motion of the object further comprises: computing at least one aggregate periodic component based on the at least one periodic component of a plurality of TSCIF associated with any vital-sign sector, wherein each of the at least one aggregate periodic component comprises a weighted average of the respective periodic components associated with the plurality of TSCIF; and monitoring each periodic vital-sign motion based on analyzing an aggregate periodic component corresponding to the periodic vital-sign motion, wherein the aggregate periodic component is analyzed based on at least one of: an instantaneous frequency, instantaneous period, vital sign timing, average, range, histogram, variance, correlation, variability, deviation, or periodicity.

Clause D19. A method of a vital sign monitoring system, comprising: transmitting, by a transmitter, a wireless signal through a wireless channel of a venue; receiving, by a receiver, the wireless signal through the wireless channel that is being impacted by an object motion of an object in the venue, wherein at least one of the transmitter or the receiver comprises an array of antennas used to transmit or receive the wireless signal, the object motion comprises at least one non-periodic body motion of the object and at least one periodic vital-sign motion of the object; segmenting space around the venue into a plurality of sectors based on a beamforming and the received wireless signal, wherein each sector of the plurality of sectors is associated with a spatial direction relative to the array of antennas; obtaining a plurality of time series of channel information (CI) of the wireless channel based on the beamforming, wherein each time series of CI (TSCI) of the plurality of TSCI is associated with a respective sector of the plurality of sectors; isolating the object motion of the object in the plurality of TSCI to generate a plurality of isolated TSCI; compensating for the at least one non-periodic body motion of the object in the plurality of isolated TSCI to generate a plurality of compensated TSCI; and monitoring the at least one periodic vital-sign motion of the object based on the plurality of compensated TSCI.

Clause D20. A wireless device of a vital sign monitoring system, comprising: a processor; a memory communicatively coupled to the processor; and a receiver communicatively coupled to the processor, wherein: an additional wireless device of the vital sign monitoring system is configured for transmitting a wireless signal through a wireless channel of a venue, the receiver is configured for receiving the wireless signal through the wireless channel that is being impacted by an object motion of an object in the venue, at least one of the transmitter or the receiver comprises an array of antennas used to transmit or receive the wireless signal, the object motion comprises at least one non-periodic body motion of the object and at least one periodic vital-sign motion of the object, and the processor is configured for: segmenting space around the venue into a plurality of sectors based on a beamforming and the received wireless signal, wherein each sector of the plurality of sectors is associated with a spatial direction relative to the array of antennas, obtaining a plurality of time series of channel information (CI) of the wireless channel based on the beamforming, wherein each time series of CI (TSCI) of the plurality of TSCI is associated with a respective sector of the plurality of sectors, isolating the object motion of the object in the plurality of TSCI to generate a plurality of isolated TSCI, compensating for the at least one non-periodic body motion of the object in the plurality of isolated TSCI to generate a plurality of compensated TSCI, and monitoring the at least one periodic vital-sign motion of the object based on the plurality of compensated TSCI.

Speech enhancement and separation have been a long-standing problem, despite recent advances using a single microphone. Although microphones perform well in constrained settings, their performance for speech separation decreases in noisy conditions. The present teaching discloses RadioSES, an audio-radio speech enhancement and separation system that overcomes inherent problems in audio-only systems. By fusing a complementary radio modality, RadioSES can estimate the number of speakers, solve source association problem, separate, and enhance noisy mixture speeches, and improve both intelligibility and perceptual quality. The system can perform millimeter wave sensing to detect and localize speakers and introduce an audio-radio deep learning framework to fuse the separate radio features with the mixed audio features. Extensive experiments using commercial off-the-shelf devices show that RadioSES outperforms a variety of state-of-the-art baselines, with consistent performance gains in different environmental settings. Compared with the audiovisual methods, RadioSES provides similar improvements, along with the benefits of lower computational complexity and being less privacy concerning.

The disclosed RadioSES system addresses the SES problem by jointly leveraging millimeter-wave (mmWave) sensing as an orthogonal radio modality. Compared to cameras, radio devices have lower power, can operate in dark, through-wall settings and are less privacy-invasive. The radio reflections from speakers can not only allow separation of multiple speakers but also capture articulatory motions for SES. The reasons to select mmWave radios are two-fold: On the one hand, more and more smart devices now include an mmWave radar and a microphone, such as Google Soli phone and Nest Hub, Amazon Alexa, etc. and mmWave sensing promises to be more ubiquitous in the future. On the other hand, mmWave sensing has enabled many applications related to motion and vibration, such as heart rate monitoring, measuring machinery and object vibration, or extracting vocal folds vibration. In particular, it has been used to estimate pitch and detect voice activity, reconstruct speech to some extent, as well as enhance speech recognition for a single speaker. Yet no existing work has explored utilizing both modalities for joint SES tasks.

With this motivation, an audio-radio speech enhancement and separation system, RadioSES, is developed to solve the aforementioned problems and improve the overall performance. Building an audio-radio SES system faces multiple challenges. First, in order to solve the number of sources problem, a robust and efficient source detection and tracking method is needed, as the performance of a system can decrease significantly in the event of miss detection. Second, radio signals are usually prone to environmental effects, and their performance can decrease considerably when tested at a new location. Returned signals from the objects are not only affected by vibration, but also from motion, with motion usually being the stronger effect. Third, different from the rich literature in audiovisual deep learning methods, radio modality has not been explored in the context of SES. Designing a suitable and efficient deep learning model for practical applications is non-trivial. Last, deep learning systems require extensive data collection and robust training methods, which is especially challenging for radio signals.

The disclosed RadioSES system can overcome these challenges. As illustrated in FIG. 35, the RadioSES system 3500 can detect, localize, and estimate the number of sources in an environment and improve SES performance even in unseen/challenging conditions. To achieve robust detection and localization, the system can use a computationally efficient pipeline of signal processing that can extract the radio features for speakers separately. In addition, the system can use an audio-radio deep learning framework that takes both audio signals and radio signals as the inputs and outputs separated and enhanced speeches for each of the speakers. Following recent advances in monaural SES, a deep learning module, called RadioSESNet, utilizes adaptive encoders, instead of relying on classical Short-Term Fourier Transform (STFT) representation. The RadioSES system further introduces a variety of techniques learned from audiovisual SES to improve robustness and generalizability of RadioSESNet to unseen environments and users.

In some embodiments, one can evaluate RadioSES using a commercial off-the-shelf (COTS) mmWave radar using synthetic and real-world data. In some embodiments, to boost data collection for training, one can build a data collection platform, and capture 5700 sentences from 19 users. In some embodiments, test results show that the radio modality can complement audio and bring similar improvements to that of video modality while not imposing visual privacy issues. One can extensively test RadioSES in different number of mixtures and a variety of environmental settings. When compared to the state-of-the art audio-only method (e.g., DPRNN-TasNet), RadioSES can bring around 3 dB improvements for separating noisy mixtures, along with benefits of estimating the number of sources and associating output streams. The improvements are not only in terms of SDR, but also of intelligibility and perceptual quality. In some embodiments, the test results indicate that audio-radio methods have a tremendous potential for SES tasks, as they enable a low-complexity, effective, privacy-preserving alternative to audio-only or vision-based methods. As such, RadioSES explores an important step in this direction and will inspire follow-up research.

The present teaching discloses RadioSES, a novel audio-radio system that jointly leverages mmWave radio and audio signals for simultaneous speech enhancement and separation. The present teaching introduces an audio-radio deep learning framework that fuses audio signals and radio signals for multi-modal speech separation and enhancement. The present teaching builds an extensive audio-radio dataset and compares RadioSES's performance in various conditions with state-of-the-art methods. In some examples, RadioSES achieves 3 to 6 dB SiSDR improvements in separating two and three person mixtures, respectively.

In some embodiments, RadioSES requires a device with mmWave sensing capabilities, and a microphone. For example, a monaural microphone records ambient sound, and a mmWave radar is expected to output separate streams for each sound source, where speech signals are investigated. While it is possible to place radar in a separate location, one can assume the radar and microphone are co-located. In some embodiments, one can expect the speaking objects to be in front of the radar. In addition, although radars can sense in NLOS conditions, one can investigate LOS only in some embodiments. For example, the application scenarios of RadioSES can be one or more persons speaking in front of a computer, smart hub, or a phone, with LOS.

In some embodiments, having speaking persons in the field-of-view (FoV), RadioSES detects near stationary bodies and uses the output to estimate and associate sources with the extracted sound signals. Unlike microphone arrays, using mmWave sensing enables to capture individual data streams not only from different azimuth angles, but also from varying distances. After these tasks, an efficient multimodal deep learning module may be used to estimate the clean speech(es), which can be used as clean speech or passed through a speech-to-text engine to convert into commands.

As shown in FIG. 36, the RadioSES system 3600 includes: an mmWave radar, a smart speaker 3611, a first block 3601 for source detection and localization, and a second block 3602 for deep learning module, named RadioSESNet.

In some embodiments, as shown in FIG. 36, the mmWave radar can include a device with a transmitter (Tx) antenna array 3611 and a receiver (Rx) antenna array 3612. In some embodiments, each of the transmitter (Tx) and receiver (Rx) arrays has multiple antennas. To sense sound in the environment, the Tx 3611 can transmit mmWave signals, which may be received by different Rx antennas 3612 sequentially after reflected by sounding or vibrating sources in a venue and other objects in the same venue. In some embodiments, the Tx 3611 is a Bot as described above; and the Rx 3612 is an Origin as described above. While the Tx 3611 and the Rx 3612 are physically coupled to each other in FIG. 36, they may be separated in different devices in other embodiments. In some embodiments, the device including the Tx 3611 and the Rx 3612 serves as the mmWave radar. In other embodiments, the mmWave radar also includes or is coupled to a processor to process the received radar signal at the Rx 3612. In various embodiments, the processor may be physically coupled to the Tx 3611, the Rx 3612, both, or neither.

As shown in FIG. 36, the goal of the radio feature extraction module is to output individual radio streams from sources in the environment. To achieve that, the system can adopt a variety of methods in an efficient pipeline to detect and locate targets. Unlike existing works, RadioSES does not rely on a spectrogram-based metric to localize people in the environment, but utilizes classical, efficient methods to extract the corresponding range-azimuth bins.

In some embodiments, at the channel information module in FIG. 36, RadioSES can work with any type of radar that can report a channel impulse response (CIR), e.g. a frequency modulated continuous wave (FMCW) radar.

When using an FMCW radar, extracting the CIR includes applying an operation called range-FFT. As mmWave devices usually have multiple antennas, one can define the CIR at the m-th antenna $h_m(\tau)$ as:

$$h_m(\tau) = \sum_{r=0}^{R-1} \alpha_{m,r}\, \delta(\tau - \tau_r) + \epsilon(\tau),$$

where R is the number of the CIR range bins, $\delta(\cdot)$ is the Delta function representing the presence of an object at the corresponding location, $\alpha_{m,r}$ and $\tau_r$ denote the complex amplitude and the propagation delay of the r-th range bin, and $\in$ denotes the additive noise, respectively. Here, the range resolution $\Delta R$ can be inferred from the time resolution, $\Delta\tau$, which is inversely proportional to bandwidth (corresponding to 4.26cm for the disclosed device). Therefore, a separate stream from very close targets can be extracted. The CIR is captured repeatedly during sensing, and is time dependent. To simplify CIR equation, one can denote the CIR from m-th antenna, at r-th range bin, at time index t as $h_{m,r}(t)$. As such, $h_{m,r}(t)$ is quantized with respect to time, range bin, and antenna index.

In some embodiments, at the digital beamforming module in FIG. 36, using the individual received streams from each antenna, RadioSES extracts range-azimuth information with classical beamforming. Range-azimuth CIR may be denoted by $h_{r,\theta}(t)$, where $\theta$ represents the azimuth angle. In some examples, the virtual antenna array elements are placed $d = \lambda/2$ apart, where $\lambda$ is the wavelength, and $h_{r,\theta}(t)$ can be given as:

$$h_{r,\theta}(t) = \mathbf{s}^H(\theta)\mathbf{h}_{\mathrm{m,r}}(\mathbf{t}) + \epsilon(t),$$

where $s^H(\theta)$ is the steering vector for angle $\theta$, and $\in$ is the additive noise. The coefficients of the steering vector are:

$$s_m(\theta) = \exp\left(-j2\pi \frac{d\sin\theta}{\lambda}\right),$$

and the channel vector is $h_{m,r}(t) = [h_{1,r}(t), h_{2,r}(t),..., h_{M,r}(t)]$, with M being the total number of antenna elements.

In some embodiments, at the target detection module in FIG. 36, to detect human bodies in the environment, RadioSES first extracts the reflecting objects in the environment. As suggested by the CIR equation, the presence of objects creates strong returned signals, whereas when there is no object, returned signals only include noise. For target detection, one can utilize a classical approach in the radar literature, constant false alarm rate (CFAR) detector, which adaptively estimates the background noise for different bins and thresholds each range-azimuth bin accordingly. In some embodiments, the 2D CFAR window shown in FIG. 37 is denoted with C, and CFAR threshold is denoted with $\gamma$. This window may be applied to the magnitude of the range-azimuth plane, and the corresponding range-azimuth plane is shown in FIG. 38. Therefore, the CFAR detection rule on the range-azimuth plane is given as:

$$B_{r,\theta}^{\mathrm{CFAR}}(t) = \mathbb{1}\{(C \star |h_{r,\theta}|)(t) > \gamma(|h_{r,\theta}(t)|)\},$$

where $\star$ and $\mathbb{1}\{\cdot\}$ denote the convolution operation and indicator function, respectively.

In some embodiments, at the clutter removal module in FIG. 36, previous module extracts a binary map with bins with reflecting objects, which can include static objects. On the other hand, even when a person is stationary, the radar signal still captures a variation at the person's location, due to inherent body motion from breathing and heart rate, a phenomenon used extensively in mmWave based person detection. Therefore, to remove the static objects and detect human bodies, the system can extract the variance at each range-azimuth bin, and use a threshold to identify static objects. One can denote the variance of $h_{r,\theta}(t)$ with $V_{r,\theta}(t)$, where an example can be seen in FIG. 39. Therefore, human detector output is $\mathrm{B}_{\mathrm{r},\theta}^{\mathrm{stat}} \triangleq \mathbb{1}\{\mathrm{V}_{\mathrm{r},\theta}(\mathrm{t}) > \mathrm{H}^{\mathrm{stat}}(\mathrm{r},\theta)\}$. Furthermore, bodies with excessive motion can also be filtered using a similar approach, and the system can reject those by: $\mathrm{B}_{\mathrm{r},\theta}^{\mathrm{mov}} \triangleq \mathbb{1}\{\mathrm{V}_{\mathrm{r},\theta}(\mathrm{t}) < \mathrm{H}^{\mathrm{mov}}(\mathrm{r},\theta)\}$, where $\mathrm{H}^{\mathrm{stat}}(\mathrm{r},\theta) \triangleq \frac{\eta^{\mathrm{stat}}\cos(\theta)}{(1+\mathrm{r}\Delta\mathrm{R})^2}$, $\mathrm{H}^{\mathrm{mov}}(\mathrm{r},\theta) \triangleq \frac{\eta^{\mathrm{mov}}\cos(\theta)}{(1+\mathrm{r}\Delta\mathrm{R})^2}$, $\eta^{stat}$ and $\eta^{mov}$ are empirically found thresholds. The minimum and maximum variances are defined with respect to $(r, \theta)$, in order to accommodate changing reflection energy with respect to angle and distance. The resulting binary detection map, $B_{r,\theta}$ (t) is found by extracting intersection of all binary maps, i.e. $B_{r,\theta}(t) = \{B_{r,\theta}^{CFAR} \cap B_{r,\theta}^{stat} \cap B_{r,\theta}^{mov}\}(t)$ as shown in FIG. 40.

In some embodiments, at the number of speaker estimation module in FIG. 36, each bin of binary detection map, $B_{r,\theta}$ (t) spans ($\Delta$R, $\Delta\theta$) distance in 2D space. Considering the high range and angular resolution, a human body can span multiple bins in B(r, $\theta$). To estimate the number of people, RadioSES clusters binary detection maps using a non-parametric clustering method, DBSCAN. The parameters for DBSCAN are set empirically, and an example clustering is shown in FIG. 41. Furthermore, since the number of people estimation and center extraction is done repeatedly for a window of size W, there is a need to match the locations of bodies at different time indices. The system can use Munkres' algorithm to continuously track the location of users.

In some embodiments, at the radio feature extraction module in FIG. 36, having extracted the number of persons and the corresponding range-azimuth bins, RadioSES extracts the complex radar signals from each person's center directly, following some raw-data based approaches. As there are many range-azimuth bins associated with the same person, RadioSES extracts the median bin for testing, whereas multiple nearby bins are used for training, which helps to boost dataset size and mitigate overfitting. In some examples, output dimensionality of the radar signals is 2 $\times$ 1000 at 16 bits for a 1-second stream, which is lower than the microphone and typical video streams.

Usually, an SES model follows the architecture in FIG. 42A, with an encoder, masker, and a decoder block. Input encoding is multiplied with an estimated mask, which uses a decoder to reconstruct the time-domain signal. STFT may be used as the encoder, with the ideal binary mask being the training objective. The performance can be increased by using more optimal masks; but these still suffer from the fact that STFT-based encoding is not necessarily optimal for speech separation. Methods that replace STFT with adaptive encoders are found to be more optimal.

In some embodiments, the disclosed RadioSES uses the structure in FIG. 42B to realize the RadioSESNet module 3602 in FIG. 36, which introduces radio modality. Radio streams are encoded, and concatenated with the audio stream to estimate the masks. However, this involves a few design choices as follows. Unlike audio signals, radio signals are complex-valued. Both real and imaginary parts of the radio signals change with respect to the motion and vibration. If a spectrogram representation is used as an input, it may not be optimal for neural network, and it usually involves in throwing away some signal content by only extracting amplitude, or half of the spectrogram (e.g. only positive Doppler shifts). Using either the real or imaginary part of the signal or combining both parts optimally with a linear projection also loses important signal content. Based on this, RadioSES may use adaptive front-end for radio streams.

To make RadioSES work with raw radio inputs, the system can apply random rotation in IQ plane. For example, the system can apply a high-pass filter on returned signals to reduce the effect of body motion. The high pass filter is used by the RadioSESNet module 3602 in FIG. 36 to run with raw radar inputs. In some examples, one can select the cutoff frequency of the high pass filter at 90 Hz in order not to filter vocal folds harmonics. Afterward, the radio signals are encoded with an adaptive encoder.

After the encoder, the system can process audio and radio streams separately with individual blocks to exploit long-term dependencies within each modality. To that end, the system processes each modality through an efficient dual-path RNN block (DPRNN). DPRNN blocks do not suffer from limited context, which is a main issue with fully convolutional models. Afterward, the system can combine two modalities via resizing and concatenation on the feature dimension. These models are further processed with DPRNN blocks and 1D decoders before outputs.

An exemplary design of RadioSESNet is shown in FIG. 43. In some examples, the audio encoder of RadioSESNet includes a 1D convolutional layer, with kernel size 16, and number of kernels 256, followed by ReLU nonlinearity and layer normalization. The radio channel of the RadioSESNet uses another 1D convolutional layer, nonlinearity, and normalization, with the same parameters, except that the number of filters being 64, due to the lower sampling rate. Stride size is set to 1/2 of the kernel width, resulting in 50% overlap between convolutional blocks. After the first layer, a second 1D convolution reduces the dimensionality to 64 for audio, and 16 for radio. Each radio stream uses the same encoder block to create an STFT-like representation. One can denote the distorted input audio with $\tilde{a}$, and radio streams with $r_i$, where *i* denotes the $i^{th}$ radio stream. Output of the audio and radio encoders are represented with $\mathbf{X}_\star \in \mathcal{R}^{N_\star \times L_\star}$, with $\star \in (a,r)$, for audio and radio stream, where one can drop the index i for simplicity. Here, $N_\star$ represents the number of features, and $L_\star$ represents the number of time samples of encoded representation.

Both encoded modalities are combined to estimate the masks for each source, as illustrated in the masker of FIG. 43. Each modality passes through individual DPRNN blocks, then fused by vector concatenation, and passes through four more DPRNN blocks before estimating the mask with a 2D convolutional layer, which matches the output with the expected mask number and size.

Table 1: Parameters for the Masker Layer for 2-Mix:

| | | | |
|---|---|---|---|
| Audio | $N_a$ 64 | $K_a$ 128 | $S_a$ 48 |

(continued)

| | | | |
|---|---|---|---|
| Radio | $N_r$ 16 | $K_r$ 16 | $S_r$ 48 |
| Concatenation | $N_c$ *96* | $K_c$ 128 | $K_c$ 48 |

When there are two people speaking at the same time in the venue, there are two radar outputs at the input of FIG. 43. The two radar outputs (e.g. of speaker 1 and of speaker 2) would go through the same adaptive encoder. Alternatively, they can be encoded differently. All the radio DPRNN can be the same for all radio signals and speakers. But the radio DPRNN can be different for different speakers in alternative embodiments.

In other embodiments, the structure in FIG. 43 can be generalized to 3, 4, 5 or more people. In such cases, there will be more radio stream.

In some embodiments, for processing the encoded data, the system can use DPRNN blocks, where an example DPRNN workflow is presented in FIG. 44. DPRNN processing may include reshaping the input data to a 3D representation, through means of extracting overlapping blocks, and concatenating through another dimension, and applying two consecutive RNN layers to different dimensions of the input block. The output of the reshaping operation can be represented as $\widehat{X}_a \in \mathcal{R}^{N_a \times K_a \times S_a}$, with $K_a$ and $S_a$ denoting the block length and number of blocks. The input, output representations $X_r$, $\hat{X}_r$ and dimensionalities $N_r$, $L_r$, $K_r$ and $S_r$ are defined similarly for radio channel, and given in Table 1, whereas the flow for a single DPRNN processing is given in FIG. 44 as well.

In some embodiments, after a suitable reshaping operation, the input blocks are fed to an RNN module, which is operated along the S dimension of the 3D input, followed by a fully connected layer, and layer normalization. After a skip connection in between, a similar operation is repeated through K dimension to capture larger distance relationships between blocks. Each RNN block has depth 1, and fully connected layers are used to match the input size to the output size, which enables to repeat multiple DPRNN blocks without any size mismatches.

In some embodiments, at the output of the masker, a number of masks equal to the number of people are estimated, which is then used to decode the signal to extract time domain audio signals. DPRNN blocks are converted back to a representation similar to the one at the input, by an overlap-add method. The signal is fed through the decoder, which applies a transposed convolution operation. The output is a single channel representation, with the same dimensionality and the same number of filters in the encoder to preserve symmetry, and it is also adaptive.

In some embodiments, in order to train RadioSESNet, the system can use scale-invariant signal-to-distortion (SiSDR) as the loss function between the time-domain signals, which is given by:

$$\mathbf{SiSDR}(\mathbf{a}, \hat{\mathbf{a}}) = 10\log_{10}\left(\frac{\|\frac{\hat{a}^T a}{\|a\|^2} a\|}{\|\frac{\hat{a}^T a}{\|a\|^2} a - \hat{a}\|}\right),$$

where a and $\hat{a}$ denote the target and the estimated sound signals. Use of SiSDR prevents scaling effects to dominate the error calculation, as the amplitude of extracted speech is not of interest. The SiSDR loss has been combined with $L_2$ norm regularization on the weights, where the decay factor is set to $1e^{-6}$. Since a separate model for different numbers of users has been trained, RadioSES switches to the appropriate model by estimating the number of sources.

Complexity and causality are particularly considered in the design.

In some embodiments, RadioSESNet has a compact design, with only 2.1M parameters. Among these, radio stream occupies 320k parameters, which could easily be fit on a small device. Forward pass of a 3-second input with RadioSESNet takes 4ms on a modern GPU with batch processing, which is only 0.4ms slower than the corresponding audio-only method.

In some embodiments, RadioSESNet uses unidirectional LSTMs in the recurrent layers of inter-block processing, whereas intra-blocks rely on BLSTMs which requires having the complete block in S dimension. Therefore, RadioSESNet can work in a causal fashion, with roughly 150ms delay. RadioSES is thus already close to real-time processing.

For experiment and implementation of the disclosed RadioSES system, one can build a data collection platform to obtain large-scale data to train, validate, and evaluate RadioSES. As extracting clean and non-reverberant ground truth samples are important, one can reduce the echo in the room by sound-absorbing pads. In some embodiments, the system can collect clean audio data with a Blue Snowball iCE microphone, sampled at 48 kHz, radar data using a Texas Instruments (TI) IWR1443 mmWave radar, and video data using the front-facing camera of an iPhone 11 Pro. The radar is set to operate with a bandwidth of 3.52 GHz at a sampling rate of 1000 Hz. The system can align the radio signal and audio signal in the time domain using the correlation of their energy. Video data, captured at 1080p and 30 fps, is also collected, although the accompanying audio files are used for training.

In some embodiments, 19 users including native speakers and speakers with different accents are instructed to read phonetically rich sentences from the TIMIT corpus. The users come from a diverse background, where there are 5 native English speakers, along with 9 Chinese, 2 Indian, 2 Turkish, 1 Korean accents. One can remove sentences that are shorter than 25 characters in the dataset. Since the size of TIMIT corpus is limited, 200 common and 100 unique sentences are read by each participant. In total, 2100 different sentences and 5762 unique words are read by participants. The sentences are presented in mixed order, and the dataset includes a lot of pauses, and filler words, in contrast to publicly available datasets, which usually include professional speakers. During data collection, users sit approximately 40cm away from the radio device and read each material at a normal speaking volume while not moving excessively.

In some embodiments, to generate the noisy and mixture sound signals, the system can follow the recipe used in LibriMix with the noise files from WHAM dataset. One can randomly select 13 users for training, and 4 users (2 male, 2 female) for evaluation. Validation set includes remaining two users, and unused speech of the users in the training set. After downsampling all audio files to 8kHz, the system can create synthetic mixtures based on the shortest of the combined files, with a minimum duration constraint of 3-seconds. Each user's recordings are repeated ten times on average, which results in 25,826 utterances (≈30 hours). The gain factors are found by normalizing the loudness of speech and noise signals, and creating noisy mixtures in [-5, 5] dB signal-to-noise rate. The system may create two evaluation sets: i) mixtures from seen users, but unheard sentences, ii) mixtures from unseen users. This helps to explain dependency on seen/unseen users in RadioSES, as different users' radio signals can be different, not only due to their speaking, but also due to their body motion and physical characteristics.

In some embodiments, a multimodal system can fail easily and focus to use a single modality, which is known as mode failure. To prevent this and to further improve robustness, the dataset creation procedure includes the following. First, same-speaker mixtures: the dataset includes same-speaker mixtures, in order to prevent mode failure, which is shown to be effective in the audiovisual domain. Second, multi-microphone mixtures: as the data collection procedure includes two microphones, one can randomly select one when generating each mixture. The evaluation may be done with the better microphone, but this also boosts dataset size multiple folds without collecting more data. Third, clean and noisy mixtures: unlike the LibriMix dataset, one can create both noisy and clean mixtures of multiple speakers and use them to train a single model. Therefore, RadioSES uses a single model, whether an environment is clean or noisy.

In some embodiments, one can implement data collection and raw data processing modules of RadioSES in MATLAB, whereas the deep learning model is implemented in PyTorch, with the help of Asteroid library to follow standard training and evaluation protocols in monoaural SES, and to borrow implementations of existing methods, such as ConvTasNet or DPRNNTasNet. In some embodiments, one can train RadioSESNet and DPRNNTasNet for 60 epochs, using a starting learning rate of $1e^{-3}$, which is halved when the validation loss did not improve for 5 consecutive epochs. Furthermore, the learning rate is scaled by 0.98 every two epochs. An early stopping criterion is set to 15 epochs. To accelerate training, one can use mixed-precision training. Thanks to the low complexity design of RadioSESNet, a single epoch takes roughly 10 minutes to train, with a batch size of 24, using a single NVIDIA RTX 2080S GPU.

As discussed previously, although microphone signals mostly correspond to speech signals, radar signals can be affected by motion, vibration, and environmental factors. Furthermore, it is usually not straightforward to make a multimodal system work easily. To improve the robustness of radio signals, one can implement the following. First, capturing multiple snapshots: since the radio signals from the multiple range-azimuth bins of the same person can change, one can record multiple range-azimuth data in the dataset. In each epoch, one can randomly select a range-azimuth bin for training among 8 candidates, whereas validation and testing use the median bin. This boosts the dataset size significantly without relying on synthetic methods and enables to use a wider range of bins, instead of searching for the most optimal bin. Second, input distortions: the input radio streams are distorted in different ways. These include introducing random rotation, adding noise at different variance levels, replacing some part of the radio signal with zeros (to imitate data loss), or removing some radio signals completely to reduce mode failure.

In some embodiments, one can report the following metrics to evaluate performance of RadioSES: (a) SiSDR: Scale-invariant signal-to-noise ratio, which is an indicator of signal levels, with a normalization factor to prevent scaling of the signals to increase metric unfairly; (b) SIR: Signal-to-interference ratio, which measures the leakage from one person to another when there are multiple speakers, and only reported for SS tasks; (c) STOI: Short time intelligibility metric, correlates with the word error rate, reported from 0 to 1; (d) PESQ: Perceptual evaluation of the sound quality, measured from 0 to 5. Since measuring human perception requires user studies, PESQ is proposed as an alternative, when user studies are not feasible.

Table 2: Results for enhancing single speaker speech

| Evaluation | Seen | | | Unseen | | |
|---|---|---|---|---|---|---|
| Model | SiSDR | STOI | PESQ | SiSDR | STOI | PESQ |
| Input | 3.9 | 0.74 | 1.55 | 3.8 | 0.70 | 1.54 |

(continued)

| Evaluation | Seen | | | Unseen | | |
|---|---|---|---|---|---|---|
| Model | SiSDR | STOI | PESQ | SiSDR | STOI | PESQ |
| WaveVoiceNet | 0.6 | 0.60 | 1.28 | 0.7 | 0.62 | 1.27 |
| ConvTasNet | 14.5 | 0.90 | 2.67 | 13.6 | 0.87 | 2.55 |
| SudoRMRF | 14.0 | 0.88 | 2.32 | 12.2 | 0.84 | 2.04 |
| DPRNNTasNet | 14.2 | 0.89 | 2.62 | 13.0 | 0.86 | 2.46 |
| RadioSES | 14.5 | 0.90 | 2.68 | 13.3 | 0.87 | 2.52 |

Baseline Methods: one can include several radio-only and audio-only methods in the literature for a variety of tasks. First, as a radio-only method, one can implement WaveVoiceNet in WaveEar. This approach uses the radio modality alone to (re)construct sound signals from vocal folds vibration, and assumes no available microphones. It reconstructs magnitude of audio spectrograms and uses Griffin-Lim based phase reconstruction. One can use oracle phase of the clean audio signal instead, which poses an upper limit on its performance.

One can compare performance of RadioSES with other audio-only baselines, to illustrate gains from radio modality, and sustained performance of RadioSES. One can include ConvTasNet, one of the first adaptive-encoder based systems that outperformed STFT-based masks. Second, one can include DPRNNTasNet, which is the audio-only baseline of RadioSES. DPRNNTasNet has shown to outperform ConvTasNet significantly, and can be considered as the state-of-the art. Third, one can use SudoRMRF, which simplifies DPRNNTasNet by replacing the RNN blocks with downsampling and upsampling blocks and is shown to achieve similar performance. Last, one cannot compare with UltraSE, as it uses ultrasound modality, and different speakers and noise dataset. Due to changes in datasets and different sampling rate (16 kHz), it is not possible to copy their results and draw a direct comparison. On the other hand, UltraSE performs similar to ConvTasNet in 2-person mixtures, which has been included as a benchmark in the study.

In speech enhancement, RadioSES brings improvements to the audio-only baseline methods, as shown in Table 2. Since the background signals are statistically different than speech signals, one can see relatively small improvements. This observation is consistent with audiovisual methods (e.g. 0.1 dB improvement), and shows that RadioSES learns to exploit the radio information. On the other hand, results from WaveVoiceNet suggest that, radio modality is not sufficient to (re)construct less-noisy audio, and may not be feasible within the experimental setting. This can be attributed to differences in the hardware, the phonetically rich diverse dataset (5762 unique words), and users. As the results are poor, there is no need to investigate WaveVoiceNet further in the experiments. ConvTasNet performs slightly better, but one can note that, the implementation uses a pretrained ConvTasNet on a much larger dataset. In addition, ConvTasNet is non-causal, and requires 1.5s look-ahead. Despite these drawbacks, RadioSES achieves similar performance as ConvTasNet.

The speech-separation results with RadioSES, along with the previously mentioned baselines are presented in Table 3. For both separating single and noisy speech tasks, RadioSES outperforms a variety of state-of-the-art methods in audio-only domain, including DPRNNTasNet. The DPRNNTasNet implementation achieves 13.5 SiSDR in 2-person clean mixtures, which is close to the reported value in the LibriMix dataset, 16.0. Significant improvements with respect to SIR ratio can be observed in both clean and noisy cases, which can indicate the usefulness of radio channel for separating the mixtures. Furthermore, even though there is more variety in radio inputs (e.g. radio channel inputs are not only affected by the sound, but also by ambient motion and physical characteristics), RadioSES can still generalize better to unseen users, where the basic DPRNNTasNet suffers. RadioSES not only improves signal metrics, but also intelligibility and the perceptual quality metrics (PESQ). The difference between the audio-only baseline becomes larger, especially when the input mixtures are corrupted with noise and when there are multiple people. To that end, one can also train RadioSES with three people mixtures. As shown in Table 4, the improvements from RadioSES are even greater for 3-person mixtures, as radio helps to extract individual streams from each user. Since the performance gains from RadioSES increases with more users, one can expect it to work well for 4 or more users.

Table 3: Evaluation in 2-Person Mixtures (SS)

| | | 2-person mix (clean) | | | | 2-person mix (noisy) | | | |
|---|---|---|---|---|---|---|---|---|---|
| | Model | SiSDR | SIR | STOI | PESQ | SiSDR | SIR | STOI | PESQ |
| Seen | Input | 0.2 | -0.4 | 0.71 | 1.71 | -1.7 | 0.3 | 0.61 | 1.37 |
| | ConvTasNet | 11.3 | 18.5 | 0.87 | 2.53 | 6.1 | 16.8 | 0.77 | 1.78 |
| | SudoRMRF | 10.9 | 15.4 | 0.84 | 2.60 | 4.7 | 16.4 | 0.68 | 1.77 |
| | DPRNN | 13.5 | 21.5 | 0.91 | 2.63 | 8.9 | 20.3 | 0.81 | 1.96 |

(continued)

| | | 2-person mix (clean) | | | | 2-person mix (noisy) | | | |
|---|---|---|---|---|---|---|---|---|---|
| | Model | SiSDR | SIR | STOI | PESQ | SiSDR | SIR | STOI | PESQ |
| | RadioSES | 15.4 | 23.6 | 0.94 | 2.83 | 10.9 | 23.3 | 0.85 | 2.10 |
| Unseen | Input | 0.0 | 0.53 | 0.70 | 1.62 | -1.8 | 0.30 | 0.60 | 1.39 |
| | ConvTasNet | 9.5 | 16.0 | 0.84 | 2.38 | 5.2 | 15.0 | 0.72 | 1.67 |
| | SudoRMRF | 6.2 | 11.5 | 0.76 | 2.13 | 1.0 | 13.0 | 0.60 | 1.39 |
| | DPRNN | 10.8 | 18.1 | 0.86 | 2.38 | 8.9 | 17.3 | 0.75 | 1.83 |
| | RadioSES | 14.5 | 22.3 | 0.92 | 2.70 | 10.3 | 22.5 | 0.83 | 2.05 |

Table 4: Evaluation in 3-Person Mixtures (SS)

| | | 3-person mix (clean) | | | | 3-person mix (noisy) | | | |
|---|---|---|---|---|---|---|---|---|---|
| | Model | SiSDR | SIR | STOI | PESQ | SiSDR | SIR | STOI | PESQ |
| Seen | Input | -3.2 | -2.8 | 0.60 | 1.37 | -4.2 | -2.8 | 0.55 | 1.30 |
| | DPRNN | 7.2 | 14.0 | 0.81 | 1.95 | 4.9 | 15.7 | 0.74 | 1.68 |
| | RadioSES | 11.6 | 19.4 | 0.88 | 2.31 | 9.3 | 19.2 | 0.83 | 1.96 |
| Unseen | Input | -3.2 | -2.8 | 0.58 | 1.37 | -4.2 | -2.8 | 0.54 | 1.31 |
| | DPRNN | 4.2 | 10.2 | 0.73 | 1.72 | 2.6 | 12.5 | 0.66 | 1.55 |
| | RadioSES | 10.7 | 18.2 | 0.86 | 2.21 | 8.6 | 18.2 | 0.81 | 1.90 |

Table 5: Performance with respect to multiple experiments of sources

| Exp | Distance | | | | | | Orientation | | | | | | | | Head Orientation | | | | | |
|---|---|---|---|---|---|---|---|---|---|---|---|---|---|---|---|---|---|---|---|---|
| Case | 50 cm | | 75 cm | | 100 cm | | 0° | | 15° | | 30° | | 45° | | 0° | | 15° | | 30° | |
| Metric | AO | AR | AO | AR | AO | AR | AO | AR | AO | AR | AO | AR | AO | AR | AO | AR | AO | AR | AO | AR |
| SiSDR | 6.3 | 10.9 | 3.8 | 8.6 | 2.3 | 4.3 | 3.8 | 8.6 | 3.6 | 7.8 | 4.4 | 8.3 | 4.2 | 8.2 | 6.3 | 10.9 | 5.6 | 9.8 | 5.4 | 9.3 |
| SIR | 12.5 | 18.3 | 9.9 | 15.6 | 8.7 | 9.8 | 9.9 | 15.6 | 9.6 | 14.8 | 10.6 | 15.1 | 10.2 | 15.6 | 12.5 | 18.3 | 11.7 | 16.8 | 11.5 | 16.3 |
| STOI | 0.83 | 0.93 | 0.79 | 0.90 | 0.74 | 0.81 | 0.79 | 0.90 | 0.78 | 0.89 | 0.79 | 0.89 | 0.78 | 0.88 | 0.83 | 0.93 | 0.80 | 0.90 | 0.79 | 0.89 |
| PESQ | 2.17 | 2.61 | 1.97 | 2.42 | 1.79 | 2.00 | 1.97 | 2.42 | 1.91 | 2.32 | 2.00 | 2.33 | 2.02 | 2.37 | 2.16 | 2.61 | 2.11 | 2.46 | 2.10 | 2.43 |

As mentioned previously, introducing another modality has many benefits, such as guiding the loss function at the beginning of training to solve permutation problem and estimating the number of sources. To that end, in FIG. 45, one can compare the loss values on training and validation sets. As shown in FIG. 45, the audio-radio system has a much steeper learning curve at the beginning, along with a better convergence point.

Furthermore, in FIG. 46, one can compare the output SiSDR of RadioSES with its audio-only baseline. As shown in FIG. 46, the disclosed RadioSES method is superior to the audio-only baseline, and the performance gains are consistent through different input SiSDR levels. To investigate the consistency of audio-radio system over audio, one can plot the differential gain in terms of SiSDR as in FIG. 47 from the radio channel. To characterize the incorrect associations, one can check the amount of samples with $\Delta(DB_i)$ < -3 is 1.03%, indicating correct physical association of sources for 98.97% of the time.

In some embodiments, one can further evaluate the performance of RadioSES in varying settings, conducted in a different location than the original data collection location. Since it is difficult to simulate the extracted radio signals from different environmental scenarios, one can collect data at a variety of settings. For example, to test the effect of distance, one can collect multiple user data at different distances, (e.g. 75cm), and create mixtures from that location. One can normalize input data streams to the same loudness levels for a fair comparison, although minor differences between each setting are inevitable. In order to show improvements, one can present each settings' performance along with the audio-only baseline, and show how RadioSES preserves a better performance in those settings. For presentation, FIG. 45 refers RadioSES as the audio-radio (AR) method, whereas baseline DPRNNTasNet is noted as audio-only (AO) method. As shown in FIG. 45, RadioSES mostly outperforms audio-only baseline with 4dB improvement in the dataset, which includes unseen and same-speaker mixtures. This evaluation is done with clean mixtures for consistency, although one can observe similar gains in noisy mixtures as well.

First, one can evaluate the effect of distance on the signal separation tasks, as illustrated in FIG. 48A. As shown in Table 5, RadioSES can work robustly until the speakers are 1m away from the device, and preserve the gains compared to the audio-only baseline. The performance for both cases decrease, which is due to training dataset being captured from a short distance only. As the distance increases, the received audio signals change due to the room impulse response and microphone nonlinearity, which is a phenomenon used for coarse source distance estimation with microphones recently. In some examples, the performance gains from radio channel does not decrease much from 0.5m and 1m, and the main bottleneck for lower performance is the variety of audio data. A high-performance system can be built by capturing more diverse audio data.

Second, the users are asked to sit 0.75m away from the device, and change their orientation to explore the practical area of sensing, as illustrated in FIG. 48B. The RadioSES can work until 45°, without any performance decrease, as presented in orientation columns of Table 5. The gains from the audio-radio system are consistent (e.g. ~4dB in SiSDR) through each setting, showing the effectiveness in modeling of the radio stream. Furthermore, this observation is consistent with that of distance, as a different deviation angle from the microphone does not create any distance-based nonlinearity, although it reduces the radio-reflection SNR.

Third, the users are asked to sit at 0.5m, and rotate their heads from 0 degrees to 15 and 30 degrees, as shown in FIG. 48C. For example, if a user sits in front of a laptop or monitor, the user would naturally swing head to see different content on the screen, and 30 degrees of head rotation at 0.5m enables to see the entire area of a big screen. Furthermore, if RadioSES is using lip motion, instead of vocal folds vibration, the results are epected to deteriorate quickly. The results are presented in the head orientation column of Table 5, which indicates that RadioSES is robust to changes in head orientation, even though the training procedure does not include explicit head-rotation data.

Fourth, the users are asked to perform a variety of distortions. For example, the users are asked to perform motions in front of the radar while speaking. To have the experiments controlled, the users are asked to move their heads up and down, left-to-right and back-and-forth naturally, as it can happen during speech. Next, data are collected with users wearing a mask, which plays a role as an occlusion. As shown in Table 6, RadioSES is not affected by the head motion. Furthermore, unlike certain visual enhancement methods which lose their advantage with occlusions, RadioSES is robust against wearing a mask, and can preserve the improvements compared to the audio-only method. This is due to the fact that vocal folds vibration are extracted from the body and throat, not from the face.

Table 6: Effect of Motion Distortion

| Exp | Motion Interference | | | | | | Occlusion | |
|---|---|---|---|---|---|---|---|---|
| Case | Back-forth | | Left-right | | Up-down | | Face Mask | |
| Metric | AO | AR | AO | AR | AO | AR | AO | AR |
| SiSDR | 6.2 | 10.5 | 5.7 | 10.3 | 5.6 | 10.4 | 5.4 | 10.5 |
| SIR | 12.3 | 17.8 | 11.6 | 17.6 | 11.6 | 17.6 | 11.6 | 17.2 |
| STOI | 0.81 | 0.91 | 0.81 | 0.91 | 0.80 | 0.91 | 0.82 | 0.92 |

(continued)

| Exp | Motion Interference | | | | | | Occlusion | |
|---|---|---|---|---|---|---|---|---|
| Case | Back-forth | | Left-right | | Up-down | | Face Mask | |
| Metric | AO | AR | AO | AR | AO | AR | AO | AR |
| PESQ | 2.14 | 2.53 | 2.09 | 2.53 | 2.12 | 2.56 | 2.07 | 2.51 |

Table 7: In the Wild Experiment Results

| Case | Speech Enhancement | | | Speech Separation | | |
|---|---|---|---|---|---|---|
| Metric | Clean | AR | Noisy | Clean | AO | AR |
| WER | 14 | 45 | 63 | 20 | 61 | 55 |
| CER | 8 | 32 | 54 | 11 | 50 | 40 |

In an exemplary experiment, multiple users are asked to sit within the same room, to test speech enhancement and separation in the wild. A user is asked to read Rainbow and Arthur passages, while background noises are played from a pair of speakers. Since this experiment does not have the ground truth clean signals, one can only evaluate the performance in terms of word-error-rate, and character-error-rate. To have a fair comparison, the users are asked to read the same material in another quiet environment to capture the performance in that setting. One can use Google's speech-to-text engine without any model adaptation to construct transcripts. As the speakers are not native speakers, and the RadioSES is implemented with telephone-quality speech (8 kHz), overall error-rate is higher. On the other hand, as presented in Table 7, RadioSES can enhance and separate multi-person mixtures and outperform the audio-only baseline for speech separation.

In other exemplary experiments, one can corrupt input signals by adding noise and zero-padding, which helps to gain insight to the performance changes when people are further away, or when there is package loss in the system. These experiments are done with the first 3-seconds of the audio streams, as longer audio streams already require some zero-padding or overlapping block processing. One can add white Gaussian noise to obtain radar data at varying SNRs from 20 to -10 dB levels, and report the performance metrics in Table 8. At larger distance, radio signals are expected to be noisy, and this experiment explores until when the radio signals are still useful. RadioSES outperforms audio baseline, until a radio SNR of -5dB. When the radio signal has further noise, similar performance as the audio baseline is achieved. This experiment indicates that there is great potential for RadioSES at larger distances.

Table 8: Performance for noisy radio inputs

| SiSDR | ∞ | 20 dB | 10 dB | 5 dB | 0 dB | -5 dB | -10dB |
|---|---|---|---|---|---|---|---|
| AO | 7.7 | | | | | | |
| RadioSES | 11.2 | 9.1 | 8.81 | 8.72 | 8.55 | 8.18 | 7.24 |

Table 9: Performance for partial radio inputs

| Metric | AO | AR(%100) AR(%16) | AR(%66) | | AR(%50) | AR(%33) |
|---|---|---|---|---|---|---|
| SiSDR | 7.7 | 11.2 | 9.3 | 8.6 | 7.6 | 7.0 |
| SIR | 18.2 | 21.0 | 20.4 | 19.2 | 17.9 | 17.1 |
| STOI | 0.74 | 0.81 | 0.80 | 0.79 | 0.77 | 0.75 |
| PESQ | 1.92 | 2.20 | 2.13 | 2.10 | 2.03 | 1.98 |

In another experiment, one can zero pad the radio streams to reduce the available radar stream duration and test input radio durations of 2s, 1.5s, 1s, and 0.5s. Such configurations can be used when there are power requirements or package loss in the radio stream. As shown in Table 9, RadioSES can still help with speech separation tasks and improve the performance, compared to the audio-only baseline, when there is at least 1s of signal (i.e., 33%), in terms of perceptual quality. RadioSES system performs better than the audio-only baseline with respect to all inputs after 1.5s of inputs. This indicates that for power-constrained settings, RadioSES can be operated with a duty-cycle less than 33%, and can still bring performance improvements, along with the aforementioned benefits of source association.

Although having speakers outside the FoV of the radar is not the key focus in RadioSES, one can explore the limits of RadioSES in such a mode of operation, by allowing one speaker to be outside the FoV. This setup requires using alternative approaches to estimate the number of speakers, as the radio-based methods will output fewer people. In practice, one may still use radio-based estimation by leveraging temporal information. One can zero pad a radio stream to simulate no information from the outside user, and understand whether RadioSES can benefit from having partial information. One can investigate a single person's missing case, but an extension to two missing people is also possible, with permutation-based methods. As shown in Table 10, RadioSES can still outperform the audio baseline with a large margin, and improve the performance, with missing people. There is not much performance decrease in 2-person noisy mixtures, when one person is outside. For 3-person mixtures, there is more decrease, but the gap between audio-only system is larger, and benefits of having the two other radio signals are clear.

In another experiment, one can train RadioSESNet without several blocks to understand the effect of each component. One can use clean 2-person mixtures for the ablation study. As shown in Table 11, one can remove i) Radio DPRNN blocks ii) Audio DPRNN blocks and iii) High-pass (HP) filter from the mask estimation. In the last case, audio stream is still used to encode the signal, in order not to change the main structure of RadioSES, but is not passed through any DPRNN blocks.

Table 10: Performance for partial detection of sources

| Case | 2-person (noisy) | | | 3-person (noisy) | | |
|---|---|---|---|---|---|---|
| Metric | AO | AR(1) | AR(2) | AO | AR(2) | AR(3) |
| SiSDR | 7.7 | 10.1 | 11.2 | 4.9 | 8.3 | 9.3 |
| SIR | 18.2 | 20.7 | 21.0 | 13.0 | 17.7 | 19.2 |
| STOI | 0.74 | 0.81 | 0.81 | 0.74 | 0.81 | 0.83 |
| PESQ | 1.95 | 2.19 | 2.20 | 1.68 | 1.89 | 1.96 |

Table 11: Ablation Study: Radio modality and HP Filter are essential parts of RadioSESNet, whereas additional radio DPRNN blocks bring extra performance improvements.

| Model | | SiSDR |
|---|---|---|
| RadioSESNet | | 15.4 |
| w/o Radio DPRNN | | 15.2 |
| | w/o Any Radio | 13.5 |
| w/o Audio DPRNN | | 4.8 |
| | w/o HP filter | 0.1 |

In the present teaching, RadioSES is disclosed to improve robustness and performance of SES tasks using radio modality. While one can assume the vibration sources in the field-of-view of radio device to be from vocal folds only, radios can also measure vibration of other sources, such as guitars, or machinery. These vibration sources usually create some sound signature, and they can be used to estimate the sound from each source separately, as done using cameras.

Microphone arrays: in some embodiments, RadioSES uses a single microphone along with an mmWave sensing device. On the other hand, it is also possible for RadioSES to work with a microphone array, and radio modality can still bring further improvements to overall performance. Although beamforming in microphone arrays may indicate that radio modality is unnecessary, it can fail in noisy or reverberant environments. Since RadioSES senses the vibration of the source, it can estimate the direction of the sound for robust beam-steering or can extract the source vibration without any reverberation for further improvement.

Moving Speakers: in some embodiments, RadioSES is designed to track bodies with an inherent assumption that they do not move significantly. This is usually a common constraint in the relevant vital signs monitoring literature (breathing, heart rate), although some recent work started addressing motion for breathing. A more thorough system should support medium and high levels of source motion. To that end, coherent combining of multiple vital sign bins from person point clouds, or deep learning can be some interesting ideas to support multiple moving targets.

Sensing Distance: the experiments indicate that RadioSES can work robustly until the speakers are 1m away from the device, and preserve the gains compared to the audio-only baseline. The performance for both cases decreases, which is due to the training audio dataset being captured from a short distance. However, the performance improvements from RadioSES do not decrease much with the distance. During the experiments, raw signal SNR is still high at large distances (e.g. 2.5m) for people with low pitch (e.g. males). To support all users, one can limit the practical range to 1m, much larger than the range of using ultrasound. Although not much radar signature can be captured from these bodies when they are further away, they can still be robustly detected, (e.g. as in vital sign monitoring), and even the reduced number of high quality radio streams can still help to improve the performance. Moreover, a different hardware can capture vocal folds vibration from 7m, or at 50m. RadioSES can benefit from better hardware significantly, and a more practical system can be built.

Multipath Effects: In the experiments, one can consider cases with multiple sources in front of the radar, and training data assumes perfectly clean radio-streams for each person. However, in challenging conditions, wireless sensing-based systems can have strong multipath effect. Although in mmWave bands, the effect is not as detrimental as 2.4/5 GHz, it can still reduce the performance. This issue may not be in short-range experiments, but it can be a limiting factor for long-range indoor sensing.

In some embodiments, costs for the evaluation board and a single mmWave device can be low. The size of these devices can go as small as 6mm × 6mm to fit in a phone, and the power consumption can be as low as 1mW. Furthermore, RadioSES does not require to run at 100% duty cycle. Based on application, lower power consumption can be achieved. As there are already devices with continuous mmWave sensing capabilities, RadioSES is feasible to be integrated with smart devices.

The disclosed RadioSES is a joint audio-radio speech enhancement and separation system using mmWave sensing. It improves the performance of existing audio-only methods with the help of radio modality and achieves similar improvements as audiovisual systems, with further benefits in computation complexity and privacy. Furthermore, RadioSES can detect the number of sources in the environment, and associate outputs with the physical speaker locations, all being challenging problems in audio-only domain. Real-world experiments show that RadioSES outperforms the state-of-the-art methods considerably, demonstrating the great potential of audio-radio SES.

FIG. 49 illustrates a flow chart of an exemplary method 4900 for radio-assisted signal estimation, according to some embodiments of the present disclosure. In various embodiments, the method 4900 can be performed by the systems disclosed above. At operation 4902, a baseband mixture signal in a venue is obtained. The baseband mixture signal comprises a mixture of a first source signal and an additional signal. The first source signal is generated by a first motion of a first object in the venue. At operation 4904, a radio feature of a radio signal is obtained. The radio signal is transmitted from a transmitter to a receiver in the venue. The received radio signal differs from the transmitted radio signal due to a wireless channel of the venue and at least the first motion of the first object in the venue. At operation 4906, a first adaptive filter is constructed for the baseband mixture signal based on the radio feature. At operation 4908, the baseband mixture signal is filtered using the first adaptive filter to obtain a first output signal. At operation 4910, an estimation of the first source signal is generated based on the first output signal. The order of the operations in FIG. 49 may be changed according to various embodiments of the present teaching.

FIG. 50 illustrates a system 5000 for radio-assisted signal estimation in a venue 5001, according to some embodiments of the present disclosure. As shown in FIG. 50, the system 5000 includes a sensor 5010 configured to obtain a baseband mixture signal 5019 in the venue 5001. In some embodiments, the sensor 5010 may be a microphone (e.g. a microphone on a smart speaker) for acoustic sensing. In some embodiments, the baseband mixture signal 5019 comprises a mixture of a first source signal 5011 and an additional signal 5012. In some embodiments, the first source signal 5011 is generated by a first motion of a first object 5050 in the venue 5001.

As shown in FIG. 50, the system 5000 also includes a transmitter 5020 configured to transmit a first radio signal 5022 through a wireless channel 5040 of the venue 5001; and a receiver 5030 configured to receive a second radio signal 5032 through the wireless channel 5040. In some embodiments, the second radio signal 5032 differs from the first radio signal 5022 due to the wireless channel 5040 and at least the first motion of the first object 5050 in the venue 5001. In some embodiments, the transmitter 5020 may be a wireless transmitter or Bot as shown in FIG. 36. In some embodiments, the receiver 5030 may be a wireless receiver or Origin as shown in FIG. 36.

As shown in FIG. 50, the system 5000 also includes a processor 5035 configured for generating an estimation of the first source signal, based on the baseband mixture signal 5019 from the sensor 5010 and the second radio signal 5032 from the receiver 5030, e.g. following method disclosed in FIG. 49. In some embodiments, the processor 5035 may be a separate device from the receiver 5030. In other embodiments, the processor 5035 may be a device coupled to or integrated with the receiver 5030.

FIG. 51 illustrates a first adaptive filter 5100 in a system, e.g. the system 5000 in FIG. 50, for radio-assisted signal estimation, according to some embodiments of the present disclosure. In other embodiments, the first adaptive filter 5100 may be constructed by the processor 5035 for the baseband mixture signal 5019 based on a radio feature of the second radio signal 5032. The first adaptive filter 5100 may be used to filter the baseband mixture signal 5019 to obtain a first output signal 5109, such that the processor 5035 of the system can generate an estimation of the first source signal 5011 based on the first output signal 5109.

As shown in FIG. 51, the first adaptive filter 5100 includes a first baseband filter 5110, a second baseband filter 5120, a third filter 5130 and a fourth filter 5140. The first baseband filter 5110 may be constructed without using the radio feature of the second radio signal 5032. The second baseband filter 5120 may be constructed based on the radio feature of the second radio signal 5032.

FIG. 52 illustrates a detailed diagram of the first adaptive filter 5100, according to some embodiments of the present disclosure. In this example, as shown in FIG. 52, the first baseband filter 5110 further includes: a first pre-processing module 5211 for processing the baseband mixture signal 5019 in a first signal domain, a first transformation module 5212 for transforming signal from the first signal domain to a first transformed domain, and a first transformed-domain filter 5213 to filter signal in the first transformed domain. In this example, as shown in FIG. 52, the second baseband filter 5120 further includes: a second pre-processing module 5221 for processing the second radio signal 5032 in a second signal domain, a second transformation module 5222 for transforming signal from the second signal domain to a second transformed domain, and a second transformed-domain filter 5223 to filter signal in the second transformed domain.

As shown in FIG. 52, the third filter 5130 in this example comprises: a third pre-processing module 5231 for processing outputs of the first baseband filter 5110 and the second baseband filter 5120, and a third transformed-domain filter 5232 for filtering signal in a third signal domain. As shown in FIG. 52, the fourth filter 5140 in this example comprises: a fourth transformed-domain filter 5241 to filter signal in the first transformed domain based on output of the third transformed-domain filter 5232, a first inverse transformation module 5242 to transform signal from the first transformed domain to the first signal domain, and a post-processing module 5243 to process signal in the first signal domain to generate the first output signal 5109, for estimation of the first source signal 5011.

In some embodiments, a wireless signal (e.g. mmWave, 28 GHz or 60GHz, or radar signal, or UWB signal) between a transmitter and a receiver may be used to assist in signal (e.g. speech) enhancement (e.g. denoising) and/or signal separation, based on channel information (e.g. channel impulse response/CIR, channel frequency response/CFR, and/or channel state information/CSI, RSSI, etc) obtained from the received wireless signal. The transmitter and the receiver may be co-located (e.g. on the same device, or on the same circuit board), or at different locations.

In some embodiments, the transmitter (Type1 device) and/or receiver (Type2 device) may each have an antenna array, distributed antennas. There may be multiple receivers each receiving the wireless signal from the transmitter. There may be multiple transmitters each transmitting a respectively wireless signal to the receiver. There may be multiple transmitters and multiple receivers, each transmitter transmitting a respectively wireless signal to one or more receivers.

In some embodiments, the device implementing the disclosed system may have a commodity wireless networking or communication chip/chipset which may operate in a radar mode. The radar mode may be enabled by attaching an extra antenna array to the chipset. It may use the chip/chipset to transmit the wireless signal using a transmitting radio, and to receive the reflected wireless signal using a receiving radio. The chip may transmit/receive simultaneously or contemporaneously. The chip may switch rapidly between transmit and receive to simulate or mimic "simultaneous" transmit/receive.

In some embodiments, the transmitters and receivers may be in a same venue (e.g. a home, a room, an office, a walkway, a common area, a facility). The transmitters may be physically next to, adjacent to, or at a distance from, the receivers. At least one object or "source" object (e.g. a person, two people or more than two people) may be present in the venue each generating a respective source signal (e.g. speech signal from each person, talking, singing, dialog, one-at-a-time speech, simultaneous two or more people talking). A mixture signal may be obtained (e.g. sound captured by microphone containing simultaneous dialog/singing of two people). The mixture signal may comprise a mixture (e.g. a sum, weighted sum, product, weighted product, etc.) of the signals from the at least one source. The source signal may be generated in the presence of background noise (e.g. two people speaking in a noisy environment, e.g. train station, airport, or a home/office; background noise may be crowd sound, mechanical sound, motor/engine sound, vacuum cleaner/fan/machine/refrigerator/heater/air conditioner).

In some embodiments, the goal of signal separation is to separate more than one source signals (e.g. sound signal) in/from the mixture signal. The goal of signal enhancement is to enhance the individual source signals (e.g. improve intelligibility, improve voice quality, reduce/remove noise).

In some embodiments, a method for radio-assisted signal estimation includes steps s1 to s5 as described below.

At step s1: obtain the mixture signal associated with a sensor (e.g. sound signal captured by a microphone) which comprises a mixture of at least one source signal associated with at least one object in the venue, each source signal from/associated with a respective object in the venue.

At step s2: transmit a wireless signal (e.g. mmWave signal or radar signal, or UWB signal) from Type1 device to Type2 device; obtain more than one time series of channel information (TSCI, with CI being/comprising one or more of CIR, CFR, or CSI, or RSSI, etc.) from received wireless signal, each CI associated with a respective transmitter antenna and a respective receiver antenna. The transmitter antennas and/or receiver antennas may be at known locations. Each CI (e.g. CIR) comprises more than one component, each component being associated with a propagation delay or a range or a range bin. Each component may be a complex number.

At step s3: apply digital beamforming on the TSCI to obtain more than one range-azimuth CIR, each CIR is associated with a range and an azimuth.

At step s4: generate at least one radio signal, CIR Tap(t), each associated with a respective object (speaker) in the venue by the following steps s4a to s4e. At step s4a, CFAR (constant false alarm rate) detector is used for target detection, e.g. target is detected when Tap of CIR > T1. At step s4b, for clutter removal, one may retain a CIP tap if T2<Variance of CIR Tap (over time)<T3, where both T2 and T3 spatially varying w.r.t. {r, theta}. At step s4c, for estimation of number of object (e.g. people), non-parametric clustering (DBSCAN) may be used. At step s4d, continuous tracking of user locations {r, theta} may be performed. At step s4e, for radio feature extraction, CIR tap (a time function) at {r, theta} may be associated with user location {r, thata}, wherein median binning is performed.

At step s5: simultaneous separate and enhance the mixture signal based on a combination of SES and radio feature. The Basic SES may include: an encoder (STFT, or similar), a compute mask (filter), an apply mask (filter), and a decoder (ISTFT, or similar). The front end processing for radio feature (i.e. CIR tap (a time function) at {r, theta}) may include the following steps s5a to s5h. At step s5a, random rotation of CIR tap(t) in IQ plane may be optionally performed. At step s5b, high-pass filter is used to reduce effect of body motion (cutoff freq at 90Hz in order not to filter vocal folds harmonics). At step s5c, an adaptive encoder (STFT-like) is constructed and used.

At step s5d, process audio and radio stream separately with individual blocks to exploit long-term dependency within each modality, where each modality may be processed through dual-path RNN block (DPRNN) - deep learning. After resizing, fuse audio and radio signals by vector concatenation. Then processing with four more DPRNN blocks may be applied before estimating the mask with a 2D convolutional layer. DPRNN processing may include reshaping the input data to a 3D representation, through means of extracting overlapping blocks, concatenating through another dimension, and applying two consecutive RNN layers to different dimensions of input block.

At step s5e, train a mask for each person based on the estimated number of people. At step s5f, apply the mask to audio, e.g. by applying mask1 to extract speaker1's speech and applying mask2 to extract speaker2's speech. At step s5g, a decoder (block-by-block) is applied. At step s5h, overlap and addition are performed.

The following numbered clauses provide implementation examples for sound sensing based on audio and radio signals.

Clause E1. A method/device/system/software of a radio-assisted signal processing system, comprising: obtaining a baseband mixture signal in a venue, wherein the baseband mixture signal comprises a mixture of a first source signal and another signal, wherein a source of the first source signal is a motion of an object in the venue; constructing a first adaptive filter for the baseband mixture signal based on a radio feature of a radio signal transmitted from a Type1 heterogeneous wireless device to a Type2 heterogeneous wireless device in the venue, wherein the received radio signal differs from the transmitted radio signal due to the motion of the object and a wireless multipath channel of the venue; filtering the baseband mixture signal using the first adaptive filter to obtain a first output signal which is an estimation of the first source signal.

In some embodiments, the total processing/filtering may comprise: (1) stage-1 filtering, (2) stage-2 filter and (3) stage-3 filtering. Referring to FIG. 43, stage-1 filter has two parts: one part (called "1a") for filtering the baseband mixture (e.g. sound) signal, and one part (called "1b") for filtering the "radar output" in FIG. 43.

(1a) The stage-la filter corresponds to 3 bottom blocks for processing baseband mixture signal (e.g. sound) in FIG. 43: the "Mic output" block, the "Adaptive Encoder (Audio Feature)" block, and the "Audio Preprocessing (Audio DPRNN(x2))" block. The processing by stage-la filter includes: preprocessing (e.g. nothing, filtering, downsampling); transformation (e.g. transform, convolution, projection) from time domain to transformed domain (e.g. STFT-like domain, frequency domain); and transformed domain processing (e.g. data reorganization or reshaping, Audio DPRNN(x2)).

(1b) The stage-lb filter corresponds to 3 top blocks for processing baseband radio-derived signal (e.g. radar output, signal derived from radio feature (e.g. CSI, CIR, CFR) of radio signal in claim 1) in FIG. 43: the "Radar output" block, the "Adaptive Encoder (Radio Feature)" block, and the "Radio Preprocessing (Radio DPRNN(x2))" block. The processing by stage-1b filter includes: preprocessing (e.g. random phase rotation, high-pass filter) in signal domain (e.g. time domain); transformation (e.g. transform, convolution, projection) from time domain to transformed domain (e.g. STFT-like domain, frequency domain); and transformed domain processing (e.g. data reorganization/reshaping, Radio DPRNN(x2)).

The stage-la filter and the stage-lb filter have similar signal processing elements: namely, the time domain, the transformation, the transformed domain, and the transformed domain processing. The baseband radio-derived signal may comprise a tab of a CIR in a particular {range, azimuth} which correspond to the object. To derive the baseband radio-derived signal, object detection may be performed to compute the particular {range, azimuth}, by performing: beamforming in 2D (or 3D) CIR, CFAR detection (e.g. thresholding, C*h>T1), clutter removal (e.g. thresholding, T2>variance>T3), clustering, number of people estimation, center extraction, and/or radio feature extraction.

(2) The stage-2 filter corresponds to the right half of "Masker" block in FIG. 43: the "Multi-model Masker (Fusion+DPRNN(x4))". The processing by stage-2 filter includes: preprocessing (e.g. fusion of multi-modal data (e.g. sound data + radio data), fusion of output of stage-la and stage-lb filters, data concatenation, transformed domain processing (e.g. data reorganization/reshaping, DPRNN(x4)), generation of transformed domain "target" filter (e.g. "Mask", "Audio Mask 1", "Audio Mask 2") for each of the source signals in the baseband mixture signal (e.g. Audio Mask 1 for first sound signal, Audio Mask 2 for second sound signal).

(3) The stage-3 filter corresponds to last three top blocks in FIG. 43 to generate the first output signal as an estimate of baseband source signal: the "Output (Audio Mask1)" block, the application of the mask (functions as a block, though not shown in block form in FIG. 43), and the "Adaptive Decoder" block. The processing by stage-3 filter includes: filtering the baseband mixture signal using the transformed domain "target" filter, inverse transform, overlap and add to reconstruct time domain output as an estimate of the baseband source signal.

Clause E2. The method/device/system/software of the radio-assisted signal processing system of Clause E1, further comprising: constructing a first baseband stage-1 filter without using the radio feature of the radio signal; constructing a second baseband stage-1 filter based on the radio feature of the radio signal; constructing the first adaptive filter based on the first baseband stage-1 filter, the second baseband stage-1 filter, a stage-2 filter and a stage-3 filter.

Clause E3. The method/device/system/software of the radio-assisted signal processing system of Clause E1 or E2, wherein the second baseband stage-1 filter comprises at least one signal processing element similar to that of the first baseband stage-1 filter.

Clause E4. The method/device/system/software of the radio-assisted signal processing system of any previous E Clause, wherein the first baseband stage-1 filter comprises: a first pre-processing in a first signal domain, a first transformation from the first signal domain to a first transformed domain, a first transformed-domain filter in the first transformed domain; wherein the second baseband stage-1 filter comprises: a second pre-processing in a second signal domain, a second transformation from the second signal domain to a second transformed domain, a second transformed-domain filter in the second transformed domain; wherein the stage-2 filter comprises: a third pre-processing based on outputs of the stage-1 filters, a third transformed-domain filter in a third domain; wherein the stage-3 filter comprises: a fourth transformed-domain filter in the first transformed domain based on output of the third transformed-domain filter, a first inverse transformation from the first transformed domain to the first signal domain, a post-processing in the first signal domain.

Clause E5. The method/device/system/software of the radio-assisted signal processing system of any previous E Clause, wherein at least one of the following pairs of signal processing elements of the first adaptive baseband filter and the second adaptive baseband filter are similar: the pair of the first signal domain and the second signal domain, the pair of the first transformed domain and the second transformed domain, the pair of the first transformation and the second transformation, and the pair of the first transformed-domain filter and the second transformed-domain filter.

Clause E6. The method/device/system/software of the radio-assisted signal processing system of any previous E Clause, wherein at least one of the first pre-processing, the second pre-processing or the third pre-processing comprises at least one of: feature extraction, magnitude computation, phase computation, distance computation, variation computation, norm computation, quantization, vector quantization, histogram, decomposition, projection, orthogonal projection, over-complete projection, eigen-decomposition, singular value decomposition (SVD), principal component analysis (PCA), independent component analysis (ICA), compressive sensing, spectral analysis, transform, low-pass filter, band-pass filter, high-pass filter, linear filter, non-linear filter, finite impulse response (FIR) filter, infinite impulse response (IIR) filter, moving average (MA) filter, autoregressive (AR) filter, ARMA filter, adaptive filter, interpolation, decimation, resampling, subsampling, upsampling, folding, grouping, sorting, re-ordering, permutation, combination, thresholding, clipping, derivative, integration, maximization, minimization, feature extraction, mean filter, weighted mean, median filter, mode filter, rank filter, quartile filter, percentile filter, convolution, time correction, phase correction, magnitude correction, random rotation in IQ plane, random phase shift, normalization, phase cleaning, magnitude cleaning, matched filter, enhancement, restoration, denoising, smoothing, signal conditioning.

Clause E7. The method/device/system/software of the radio-assisted signal processing system of any previous E Clause, wherein at least one of the first signal domain or the second signal domain is a time domain; wherein at least one of the first transformed domain or the second transformed domain is a frequency-like domain.

Clause E8. The method/device/system/software of the radio-assisted signal processing system of any previous E clause, wherein at least one of the first transformation or the second transformation comprises at least one of: a discrete time transform, adaptive transform, encoder, adaptive encoder, trigonometric transform, Fourier transform, sine transform, cosine transform, Hadamard transform, short-time transform, STFT, wavelet transform, fast transform, STFT-like transformation, eigen-decomposition, principal component analysis (PCA), independent component analysis (ICA), singular value decomposition (SVD), time decomposition, frequency decomposition, time-frequency decomposition, compressive sensing, graph-based transform, spectral analysis, matching pursuit, a projection, orthogonal projection, non-orthogonal projection, over-complete projection, a projection into the frequency-like domain, a number of projection filters each associated with a frequency, a number of kernels, a number of convolutional filters, or the number of convolutional filters followed by another number of convolutional filters.

Clause E9. The method/device/system/software of the radio-assisted signal processing system of any previous E Clause, wherein at least one of the first transformed-domain filter, the second transformed-domain filter, the third transformed-domain filter, or the fourth transformed-domain filter comprises at least one of: a linear filter, non-linear filter, finite impulse response filter, infinite impulse response filter, moving average filter, autoregressive filter, adaptive filter, interpolation, decimation, resampling, subsampling, upsampling, reshaping, concatenation, time-domain filtering, frequency domain filtering, layers of interconnected processing nodes with an input layer, processing layers and an output layer, fuzzy logic, radial basis function network, support vector machine, tensor product network, simulated reality, self-organizing map, genetic algorithm, evolutionary algorithm, generative adversarial network, parallel distributed processing, biologically inspired computing, learning network, training, clustering, machine learning, layer normalization, neural network (NN), multiple NN, artificial NN (ANN), feedforward NN, multi-layered perceptron (MLP), transformer based NN, attention based NN, convolutional NN, evolutionary NN, cellular NN, modular NN, recurrent Hopfield network, recurrent NN, RNN, dual path RNN, DPRNN, DPRNN(x2), DPRNN(x4), DPRNN(x8), time-delayed NN, NN with long short-term memory (LSTM), NN with bidirectional long short-term memory (BLSTM), NN with intra-block, NN with inter-block, fully-connected NN, NN with back propagation, deep neural network, or deep learning network.

Clause E10. The method/device/system/software of the radio-assisted signal processing system of any previous E Clause, wherein the first inverse transformation comprises at least one of: the inverse of the first transformation, a transposed convolution operation; wherein the post-processing comprises at least one of: concatenation, overlap-and-add (OLA), synchronized overlap-and-add (SOLA), overlap-and-save, overlap-and-discard, overlap-and-scrap.

Clause E11. The method/device/system/software of the radio-assisted signal processing system of any previous E Clause, further comprising: filtering the baseband mixture signal in the first signal domain using the first baseband stage-1 filter to generate a first intermediate signal in the first transformed domain; filtering a baseband radio-derived signal in the second signal domain using the second baseband stage-1 filter to generate a second intermediate signal in the second transformed domain, wherein the baseband radio-derived signal is derived from the radio signal based on the radio feature; filtering a combination of the first intermediate signal and the second intermediate signal using the stage-2 filter to construct the fourth transformed-domain filter of the stage-3 filter; filtering a transformed baseband mixture signal using the stage-3 filter to obtain the first output signal which is the estimation of the first source signal, wherein the transformed baseband mixture signal is obtained by preprocessing the baseband mixture signal using the first pre-processing followed by transforming from the first signal domain to the first transformed domain using the first transformation.

Clause E12. The method/device/system/software of the radio-assisted signal processing system of clauses E1, E2, E4 or E11, further comprising: wherein the another signal comprises a mixture of a second source signal and yet another signal; constructing a second adaptive filter for the baseband mixture signal based on the radio feature of the radio signal; filtering the baseband mixture signal using the second adaptive filter to obtain a second output signal which is an estimation of the second source signal.

Clause E13. The method/device/system/software of the radio-assisted signal processing system of Clause E12, further comprising: constructing the second adaptive filter based on the first baseband stage-1 filter, the second baseband stage-1 filter, another stage-2 filter and another stage-3 filter.

Clause E14. The method/device/system/software of the radio-assisted signal processing system of Clause E13, further comprising: wherein the another stage-2 filter comprises: another third pre-processing based on outputs of the stage-1 filters, another third transformed-domain filter in the third domain; wherein the another stage-3 filter comprises: another fourth transformed-domain filter in the first transformed domain based on output of the another third transformed-domain filter, the first inverse transformation from the first transformed domain to the first signal domain, the post-processing in the first signal domain.

Clause E15. The method/device/system/software of the radio-assisted signal processing system of Clause E13 or E14, further comprising: filtering another baseband radio-derived signal in the second signal domain using the second baseband stage-1 filter to generate another second intermediate signal in the second transformed domain, wherein the another baseband radio-derived signal is derived from the radio signal based on the radio feature; filtering a combination of the first intermediate signal, and the another second intermediate signal using the another stage-2 filter to construct the another fourth transformed-domain filter of the another stage-3 filter; filtering the transformed baseband mixture signal using the another stage-3 filter to obtain the second output signal which is the estimation of the second source signal.

Clause E16. The method/device/system/software of the radio-assisted signal processing system of Clauses E13 to E15, further comprising: filtering a combination of the first intermediate signal, the second intermediate signal and the another second intermediate signal using the another stage-2 filter to construct the fourth transformed-domain filter of the stage-3 filter and the another fourth transformed-domain filter of the another stage-3 filter.

Clause E17. The method/device/system/software of the radio-assisted signal processing system of any previous E Clause, further comprising: obtaining the radio feature which is a number of time series of channel information (TSCI) of the wireless multipath channel based on the received radio signal using a processor, a memory and a set of instructions, wherein each TSCI is associated with a Tx antenna of the Type1 device and a Rx antenna of the Type2 device, wherein the radio signal comprises a series of sounding signals such that each channel information (CI) is associated with a corresponding sounding signal, wherein each CI comprises at least one of: channel state information (CSI), channel impulse response (CIR), channel frequency response (CFR), or received signal strength index (RSSI); deriving the baseband radio-derived signal from the radio signal based on the number of TSCI.

Clause E18. The method/device/system/software of the radio-assisted signal processing system of any previous E Clause, further comprising: performing beamforming based on the number of TSCI; detecting a location of the object, wherein the location is associated with a range and a direction, determining a particular beamformed TSCI associated with the direction, determining a particular tab of each CI of the particular beamformed TSCI associated with the range, deriving the baseband radio-derived signal from the radio signal based on the particular tab of each CI of the particular beamformed TSCI.

Clause E19. The method/device/system/software of the radio-assisted signal processing system of any previous E Clause, further comprising: wherein the another signal comprises a mixture of a second source signal associated with another object in the venue and yet another signal; detecting another location of the another object, wherein the another location is associated with another range and/or another direction; determining another particular beamformed TSCI associated with the another direction; determining another particular tab of each CI of the another particular beamformed TSCI associated with the another range; deriving another baseband radio-derived signal from the radio signal based on the another particular tab of each CI of the another particular beamformed TSCI; constructing a second adaptive filter for the baseband mixture signal based on the another baseband radio-derived signal; filtering the baseband mixture signal using the second adaptive filter to obtain a second output signal which is an estimation of the second source signal.

Clause E20. The method/device/system/software of the radio-assisted signal processing system of any previous E Clause, further comprising: pre-processing the baseband mixture signal in a first signal domain using a first pre-processing; transforming the baseband mixture signal from the first signal domain to a first transformed domain to obtain a transformed baseband mixture signal using a first transformation; filtering the transformed baseband mixture signal in the first transformed domain to obtain a first intermediate signal using a first transformed-domain filter; pre-processing a baseband radio-derived signal in a second signal domain using a second pre-processing, wherein the baseband radio-derived signal is derived from the radio signal based on the radio feature; transforming the baseband radio-derived signal from the second signal domain to a second transformed domain to obtain a transformed baseband radio-derived signal using a second transformation; filtering the transformed baseband radio-derived signal in the second transformed domain to obtain a second intermediate signal using a second transformed-domain filter; pre-processing the transformed baseband mixture signal and the transformed baseband radio-derived signal to obtain a fused signal in a third transformed domain using a third pre-processing; filtering the fused signal in the third domain to obtain a fourth transformed-domain filter using a third transformed-domain filter; filtering the transformed baseband mixture signal in the first transformed domain to obtain a filtered transformed baseband mixture signal using the fourth transformed-domain filter; transforming the filtered transformed baseband mixture signal from the first transformed domain to the first signal domain using an inverse of the first transformation to obtain the first output signal which is the estimation of the first source signal; post-processing the first output signal.

Clause E21. The method/device/system/software of the radio-assisted signal processing system of any previous E Clause, wherein the first signal domain is a time domain associated with a first sampling rate; wherein the second signal domain is a time domain associated with a second sampling rate.

Clause E22. The method/device/system/software of the radio-assisted signal processing system of any previous E Clause, wherein: the radio signal is at least one of: a radio signal with a carrier frequency larger than 10GHz, a millimeter wave radio signal, a radio signal with a bandwidth larger than 200MHz, a WLAN signal, a WiFi signal, a wireless communication signal, an ultra-wide band (UWB) radio signal, a radar signal.

Clause E23. The method/device/system/software of the radio-assisted signal processing system of any previous E Clause, wherein: the first object is a first person, the first source signal comprises at least one of: a sound, a dialog signal, a speech signal, a singing sound, an activity sound, a musical sound, an instrument sound, a man-made sound, a machine sound, a mechanical sound, a playback sound, a speaker sound, a synthesized sound, an audio signal, a visual signal, a light intensity signal, an image, a video, an imaging, a baseband sound signal captured based on a microphone, an visual signal captured based on a camera sensor, an imaging signal captured based on an imaging sensor, or a baseband sensing signal obtained based on a sensor.

Clause E24. The method/device/system/software of the radio-assisted signal processing system of any previous E Clause, wherein: the another signal comprises at least one of: background noise, a mixture of multiple signals, household sound, mechanical sound, instrument sound, machine sound, vacuum cleaner sound, fan sound, heater sound, air conditioning sound, television sound, radio sound, audio sound, speaker sound, play-backed sound, musical sound, environment sound, air sound, wind sound, traffic sound, tree sound, window sound, door sound, human activity sound, cooking sound, work sound, play sound, tool sound, toy sound, human chattering, laughter, a baseband signal generated a second object, a baseband sound signal associated with a second motion of a second person.

Clause E25. The method/device/system/software of the radio-assisted signal processing system of any previous E Clause, further comprising: processing the first output signal for a task associated with the object.

Clause E26. The method/device/system/software of the radio-assisted signal processing system of any previous E Clause, wherein: the radio signal is obtained using the radio receiver of the Type2 device; the baseband mixture signal is obtained using a sensor of a different kind from the radio receiver.

Clause E27. The method/device/system/software of the radio-assisted signal processing system of any previous E Clause, wherein: the radio signal is obtained using a radio receiver of the Type2 device; the baseband mixture signal is obtained using a sensor which is not a radio receiver.

Clause E28. The method/device/system/software of the radio-assisted signal processing system of any previous E Clause, wherein: the radio signal is obtained using a radio receiver of the Type2 device; the baseband mixture signal is obtained using a sensor which does not comprise a radio component.

Clause E29. The method/device/system/software of the radio-assisted signal processing system of any previous E Clause, wherein: the baseband mixture signal is obtained using a baseband sensor; the radio signal is obtained using a sensor of a different kind from baseband sensor.

Clause E30. The method/device/system/software of the radio-assisted signal processing system of any previous E Clause, wherein: the baseband mixture signal is obtained using a baseband sensor; the radio signal is obtained using a sensor which is not a baseband sensor.

Clause E31. The method/device/system/software of the radio-assisted signal processing system of Clauses E26 to E30, wherein: the first adaptive filter comprises a multimodal fusion of the radio signal and the baseband mixture signal.

A robust voice activity detection (VAD) system, e.g. in an voice interface of a human-machine interaction system, enables the removal of silent and unrelated sound segments, prior to transmission and processing, and therefore helps to reduce the computational complexity and power consumption. A high-performance VAD has many use cases, especially when the voice is transmitted to another human party, such as in meetings and conference calls. Meeting attendees in noisy and interference-prone environments (e.g. people in open workspaces with multiple nearby people) often need to toggle on and off their microphones manually, as the interference creates discomfort to the party listening on the other side. In a challenging environment, such as teleconferencing while driving, the speaker generally needs to interrupt the device through physical interaction (e.g. touch or gesture), which is usually illegal and dangerous. Smart assistants and hands-free systems are also not applicable in such scenarios, as the voice data is already active and transmitted. In other words, the user cannot ask the smart speaker to turn off the microphone, as the microphone is actively transmitting the vocal commands to the other listening party. In a different scenario, a smart speaker may need to be activated only by a particular user and remain deactivated when other interfering speakers are existent. For example, in an open-space environment, the smart speaker of a particular user can be activated by users in other desks. Furthermore, if there are multiple smart assistants within a room, different areas of the room can be assigned for a particular smart device, e.g. to prevent multiple smart assistants to trigger simultaneously. An automated high-performance VAD system that has spatial sensing capabilities would bring tremendous advantages to these practical scenarios, as it would minimize the need for user interaction and improve the quality of the voice calls significantly.

An ideal VAD for practical scenarios should have the following properties. First, the VAD system should be robust against interference and noise. As mentioned previously, an ideal VAD system should be robust to arbitrary background noises and interference signals. The system should be able to combat arbitrary signals to be a practical system. Second, the VAD system should have low computational complexity. Since a VAD system usually runs continuously in the background (either with hotword detection or not), it needs to be computationally efficient, and has low power consumption. Third, the VAD system should have a low detection delay. To enable practical applications, an ideal VAD should be responsive with minimal processing delays. Fourth, in order to focus on the target speaker, an ideal VAD system should be able to selectively extract the voice activity of the desired user.

A next-generation VAD system with these capabilities can be named irVAD, where the name stands for interference and noise resilient voice activity detection system. To develop an irVAD, a system needs to use auxiliary information about the source, as microphones are inherently prone to interference. An irVAD usually cannot be implemented with noise and interference cancellation, as these are computationally complex tasks and they do not provide additional information about the particular source if there are multiple sources. A speaker-conditioned voice activity detection is only triggered based on the speech of a particular speaker. But this requires the collection of a priori data. A system can condition speech on the physical characteristics of the user, e.g. a video of the user can be used to selectively detect voice activity. But this method relies on lip motion, which may not be available, especially when the users are wearing face masks. Furthermore, this requires perfect lighting conditions and can potentially raise privacy concerns.

The present teaching disclosed an irVAD system by exploring a second modality beyond microphones, a radio or radar signal, e.g. mmWave signal. The motivation to use mmWave is multifold. First, unlike microphones that capture ambient sound, mmWave radars can separate sources in the environment with respect to their distance and angle. Second, mmWave signals can be used to measure a side channel of speech, vocal folds vibration, remotely. Third, mmWave signals have large bandwidth and high frequency, which enables precise localization of vibration sources and therefore spatial sensing. Fourth, mmWave radars already exist in some smart devices to perform many interesting sensing applications, such as gesture recognition, breathing and sleep monitoring, and are computationally efficient (deployable within mobile devices) devices.

Using mmWave-based sensing, a system can locate the source of vibration (from vocal folds), with high precision, and use this information to extract VAD as an interference-resilient method. As vocal folds generate the excitation signal for voiced speech, it is a good indicator of individual voice activity. In contrast to other modalities, such as ultrasound, WiFi, and ultrawideband, mmWave can separate sources with high precision. They do not raise privacy concerns as much as cameras. Unlike lidar or infrared, they exist in many devices, which makes mmWave an ideal candidate for irVAD.

An mmWave-based voice-activity detection system can mitigate the aforementioned issues to build an irVAD by a source specific VAD method. Assuming that the vibration source lies in front of the radar (possibly with or without a specific location, such as the driver seat of the car), an mmWave-based system can extract the voice activity of the speaker and control the microphone automatically. The following teaching will illustrate the usefulness of radio modality for VAD through mmWave sensing by disclosing a radio-based VAD system for irVAD, and evaluating it by building multiple silent datasets and using speech datasets. The results are evaluated in different areas, with physically different locations in unconstrained settings, to provide extensive comparisons with audio-based methods.

In some embodiments, for design considerations, VAD is a binary classification task based on time series data as input. The input is usually taken as a window with a short duration (e.g. 32 ms), and an aggregate decision is made for potentially overlapping windows. An automatic VAD should be able to detect the presence of the voice of a particular user with no additional user input and minimal constraints. Minimizing user input eliminates the possibility of assuming a priori user data which requires training. Therefore, speaker-conditioned VAD systems that rely on speaker embedding's or facial data cannot be a solution. Another potential auxiliary information is source location, and the system can activate according to a particular direction. Although microphone arrays enable filtering sources according to their incident angle with the help of beamforming, they cannot distinguish a nearby target user with a background user. Furthermore, beamforming can easily fail in noisy or reverberant conditions. Consequently, it is better that the disclosed system do not rely solely on beamforming. To constrain the VAD on the source vibration, capturing the distance (range) and incident angle (azimuth) of the source with high precision is needed, which is not possible by audio-systems alone without additional further assumptions. In addition to these, a VAD system is required to be robust, computationally efficient, real-time, and responsive, since it is usually a preprocessing block for many applications.

Before explaining how an mmWave-based system can solve the aforementioned limitations, FIGS. 53A-53C illustrate the feasibility and potential of a radio based VAD, where 20-second long audio and radio signals are captured from an environment, where some background noise is played by external speakers, followed by the target and an interfering speaker, respectively.

FIG. 53A illustrates a microphone spectrogram, which has noise (0 to 6s), target speaker (6s to 15s), and interference (15s to 20s). As shown in FIG. 53A, a single microphone captures ambient signal without being able to separate different sources.

FIG. 53B illustrates a radar spectrogram showing activity only during target speech. In FIG. 53B, the radio spectrogram only includes vibration from a particular user and is not affected by background noise or interference.

FIG. 53C further displays detection results from a trained audio-VAD, a reference audio VAD (Silero-VAD), and the disclosed radio-based VAD. As shown in FIG. 53C, only the two audio VAD systems have false alarms, while the disclosed radio-based system does not trigger any false alarm. That is, the two audio VAD systems are triggered when there is interference, whereas a radio-based system is robust against interference and can only be triggered by the target user. A radio-based detection can thus preserve correct decisions even when there is concurrent talking.

In some embodiments of the present teaching, a VAD system includes a smart device that has a microphone and an mmWave radar. One can assume that the target speaker is in the field of view (FoV) of the radar. FIG. 54 illustrates a design of VAD system 5400, which includes a multi-sensor device 5410 (radio and microphone) that detects the activity of the target user (e.g. target speaker) through radio-modality, and activates the microphone for further processing. It is robust against other noise source and interfering source (e.g. interfering speaker).

As illustrated in FIG. 54, the voice activity detection in the VAD system 5400 relies on a radio-based VAD 5420 alone, which runs continuously in the background (potentially along with other sensing applications), and triggers the microphone recording 5430 for further processing after VAD. These further tasks can include speech recognition, speech-to-text conversion, and speaker enhancement, all of which benefit from a robust VAD and are natural extensions of this system.

To achieve irVAD, the radio-based VAD 5420 may include two main modules: a feature extraction module that can ensure speaker conditioning on the source vibration; and a neural network module for the VAD tasks, which is designed to satisfy real-time and low computational complexity requirements.

In some embodiments of the present teaching, a disclosed radio-based VAD (RadioVAD) relies on the raw radar signals, which are complex-valued time-series data, similar to RadioSES. Based on beamforming and frequency modulated carrier wave (FMCW) technique, radars can extract a time-series data from each distance (i.e. range bin) based on some granularity (i.e. range resolution), and from different angles (i.e. azimuth bin) with the resolution depending on the antenna array. One can assume that the range-azimuth plane of the radar signal is available through the appropriate radar processing operations.

Based on the range-azimuth data, the system can perform source detection and localization of the candidate range-azimuth bins. To that extent, the system can use a variance-based detection scheme to find the nearest user to the device, which is a good indicator of presence, due to the body motion caused by breathing and used extensively in the vital-sign monitoring literature for localization. In some embodiments, a time-series data is extracted from the human chest and throat.

The performance of the system can be evaluated using a neural network (NN), which is depicted in FIG. 55. One can select the structure of the neural network for RadioVAD based on the RadioSES. For example, one can use the same NN in RadioSES with minor changes. In some embodiments, the structure of the NN is as follows. First, a time-frequency representation of the input radio (and also audio) signal is obtained with a 1D convolutional layer. Through the overlap-and-concatenate operation, one can obtain a 4D representation of the input. The 4D structure is then passed through BiLSTM, fully connected and normalization layers. These are followed by another set of LSTM, fully connected and normalization layers, and the same structure is repeated 4 times. All these layers preserve the dimensionality of the input. The output is reshaped to match the input data dimensionality through a fully connected layer and overlap and add method. Last, the output is downsampled to VAD sampling rate through averaging.

One can make comparisons between an audio-only baseline and the proposed system, using the same NN, with a variety of datasets to illustrate the feasibility. When using the audio-only system, the sampling rate of the input increases 8 times, which increases the model size and computational complexity. Therefore, even if the audio and radio models match the same performance, a radio-based system has 8 times lower computational complexity due to the lower sampling rate. In terms of comparison, the disclosed radio-based neural network includes 25.8k parameters, which is quite compact. In contrast, the audio-only baseline included 360k parameters, at a sampling rate of 8 kHz. Consequently, achieving similar performance to audio baseline with radio modality indicates a computationally efficient method, and shows great promise of radio modality.

One can investigate a multimodal system to further improve the performance of the system and illustrate the benefits of RadioVAD. In some embodiments, one use the dataset explained in RadioSES, which includes mostly voiced audio and radio files, which had joint audio and radar recordings of 19 users from 5700 sentences. This dataset only includes static users who were allowed to move naturally during speech but not much; therefore, it is not sufficient to evaluate the performance of a VAD. Furthermore, it also lacks silent audio and radar recordings, as the data was cropped with respect to the beginning and end of the sentences.

To overcome these limitations, the system may collect additional data from 11 participants in an experiment area. In some embodiments, the users are asked to sit in the designated area, where they are approximately 0.5m away from the radar. The users work freely in the environment with no further instruction on how they work, except to remain silent. Their work routine included using a separate laptop, and the provided monitor, reading from/writing to paper documents, checking mobile phones, and typing on the keyboard. Each user was asked to work for an hour in the given location. After subtracting the overhead from the data capture, the data from each user ended up around 35 minutes, with the total data being around 6 hours. Furthermore, the system has collected additional radar and audio data in other locations to improve robustness. One dataset includes 30 minutes of new location data to test further generalizability of the system. In addition to these, the system further collects data in more challenging scenarios. Some of these challenging scenarios are driving, moving the device intentionally, and making other motions with mouth, such as whispering or gumming.

In order to generate reference labels, the system may use a high performance off-the-shelf VAD to clean audio files. In some embodiments, the system can extract raw detection decisions from Silero VAD with 32ms-long decision windows and smooth the decisions by setting a minimum speech duration of 0.25s and a minimum silence duration of 0.1s. The system can set onset and offset thresholds as 0.5 and 0.35, and process the data in a causal fashion. In the quiet data setting, one can set all reference labels to zero, as the users are asked to be silent. To train the audio-only system with background noise, the same speech enhancement dataset is created as in RadioSES. Having both noise files and clean audio files corrupted with the noise allows the system to mitigate overfitting issues, as the NN can easily learn to distinguish the environment in a different scenario.

For radio processing, the system may use a variety of preprocessing methods, such as high-pass filtering and random phase rotation of complex-valued signals. Although the decision windows are 32ms long, the system may use longer duration samples to exploit contextual information.

In some embodiments, the training procedure may use a modified F1 score ($F_\beta$) loss between the reference and estimated values. $F_\beta$ score is a modified F1 score, used to balance the cost of precision and recall rates, and is given as:

$$F_\beta = \frac{(1+\beta^2)\mathrm{TP}}{(1+\beta^2)\mathrm{TP} + \beta^2\mathrm{FN} + \mathrm{FP}},$$

where TP, FN, and, FP denote true positive, false negative, and false positive rates, respectively.

For training, the system can utilize different users in the training and test set for both datasets. The performance metrics are provided for the users in the training set (closed condition) to better understand the generalization performance. Since the F1 score is an aggregate metric, one can also provide additional evaluation metrics in experiments.

In some embodiments, the NN model and training process are implemented in PyTorch. The performance of RadioVAD can be evaluated in different scenarios, with respect to a variety of metrics and experiments. The overall performance of RadioVAD will be presented with evaluation of false alarms in a variety of daily scenarios. One can investigate the effect of motion interference and a variety of noise sources, with a comparison of two modalities and a multimodal system.

In some embodiments, one can evaluate the performance with respect to the metrics, such as accuracy, precision, recall, F1-score, and area under curve (AUC). Some of these metrics are given as follows:

$$\text{Precision} = \frac{\text{TP}}{TP + FP},$$

$$\text{Recall} = \frac{\text{TP}}{TP + FN},$$

$$\text{F1-Score} = 2\,\frac{\text{Precision} \cdot \text{Recall}}{\text{Precision} + \text{Recall}}.$$

In some embodiments, one can further evaluate the performance with respect to a variety of environmental factors, such as distance, orientation, occlusions, and arbitrary motion. Moreover, one can provide important metrics, such as detection delay, and investigate the effect of user diversity.

The performance metrics are presented in two test cases in FIG. 56A and FIG. 56B, respectively. In addition to the disclosed RadioVAD, the Audio-VAD baselines, one can also use an off-the-shelf VAD detector, Silero VAD in this setting to provide another baseline. While Silero VAD is trained with larger datasets, the audio baseline is trained in a single (or in a few location) data and may have some overfitting issues to the background.

FIG. 56A shows performance comparison of RadioVAD with Audio VAD and Silero in test set I (with closed condition-seen users); FIG. 56B shows performance comparison of RadioVAD with Audio VAD and Silero in test set II (with open condition-unseen users). In the test set I, one can evaluate the unseen text from the users used during training, whereas test set II only constitutes unseen users. First, one can observe that even though the radar captures secondary information, it can still match the performance of the audio-only method, in terms of accuracy and F1-score closely in the test set I. In test set II, the proposed system outperforms the audio-VAD, showing the promise of radio-based system. In addition, RadioVAD provides much higher performance than Silero VAD, and gives very similar performance to the audio baseline. The audio training pipeline only includes noise files from a particular dataset, and its performance can potentially decrease with a wider variety of noise files. The performance gap between RadioVAD and Silero VAD is clear. In addition, one can observe that RadioVAD outperforms the audio baseline in the unseen condition, which is an indicator of the generalization capabilities of the radio-based VAD method. In summary, using the side-channel information, an mmWave-based system can match and surpass the performance of a microphone based system.

One can also investigate at what conditions, RadioVAD is better than an audio based system, and change the focus to detection delay. For a high-performance VAD system, detection delay is of utmost importance, as this will trigger the capturing of audio signals. More than 85% of the detections of RadioVAD have a delay less than 64ms, and the median detection delay is 0ms. In addition, RadioVAD outperforms Silero VAD, and matches the performance of the audio baseline for most of the time.

One can also conduct additional experiments to test the false alarm rate of RadioVAD during a variety of motion types. To further validate robustness against the motion, one may conduct experiments when the tester is eating, drinking, gumming, or having silent speech. The experiments show that the false alarm rate is low (less than 3%), whereas eating and silent speech may introduce some false alarms. The RadioVAD may use lip motion to some extent, and some of these motions include trigger false alarms due to the opening of the mouth (e.g. silent speech or eating). In some embodiments, the overall false alarm trend of RadioVAD is comparable with that of Silero VAD.

Since radio signals capture the motion (i.e. displacement) of objects in the environment, they are also affected by the relative motion between the device and the sources. In order to test the effect of motion signature, one can conduct the experiments when the tester is holding the device in hand, moving the device in hand, and holding a paper in front of device, speaking with hand gestures, moving the body, and wearing a face mask when speaking. The experiments show that the source or target motion affects the performance of RadioVAD minimally.

One can also investigate the effect of the signal-to-noise ratio on radio signals to better understand RadioVAD in terms of distance and noise robustness. The evaluation may be performed by inspecting the performance metrics with respect to an estimated radio SNR. The evaluation shows that the performance of RadioVAD increases with the higher radio SNR values. In some embodiments, the RadioVAD starts to outperform audio-based VAD when the radio SNR is higher than about 8dB. Furthermore, there is a weak correlation between the performance of audio based performance and Radio SNR, which should be indicative of the speaking strength, but the relationship is very minimal. When Radio SNR is greater than about 8 dB, RadioVAD performs better than audio VAD, along with its aforementioned computational benefits. When the underlying dataset for audio signals have more noise, RadioVAD will be more preferable at lower radio SNR points.

In addition, one can evaluate the performance of RadioVAD with respect to multiple environmental factors. These include testing the system against changes in distance, orientation, and face orientation. One can also evaluate the performance of RadioVAD at varying distances. In some embodiments, RadioVAD may preserve its performance before 75cm.

One can perform experiments with the orientation of the human body in the environment. RadioVAD performs similarly at varying angles. Having a wide field of view is important, and RadioVAD can operate at 45 degree angle without a significant performance reduction.

In a practical scenario, the users do not necessarily look toward the radio device, and they may rotate their heads to look around. As an example, a driver of the car can potentially check the mirrors, or a user can look around a screen to see different materials. Therefore, one may test RadioVAD against head rotation. In some embodiments, small head rotations less than 30 degrees do not affect the performance of RadioVAD, which enables high-performance VAD.

A natural extension of RadioVAD is using the two modalities, by an audio-radio framework. To explore the performance of such system, one can use the same neural network model, but concatenate the radio and audio channels after the encoder layers. At the output, one can map the output directionality to match the dimensionality of the input audio stream and decode the signal accordingly. A high level processing outline is shown in FIG. 57, with similar structures as the NN in FIG. 55, except that FIG. 57 has two inputs, both radio and audio.

In this setting, one can provide and compare the performance metrics of the multimodal system with the proposed system. An audio-radio model further improves the accuracy, precision, recall, and F1 scores. When audio signals are corrupted with varying SNR levels, the performance of RadioVAD matches that of audio-baselines, and surpasses them in some other cases (e.g. Silero VAD). In order to understand this phenomenon better, one can use a predefined SNR value for audio signals and extract the performance metrics. In some embodiments, one can use an audio SNR of -10 dB to +10 dB, and run the audio baseline and Silero VAD. In all cases, RadioVAD performs better than both audio-based approaches when the audio SNR is at 0 dB or lower. On the other hand, recall rates, and f1 score matches that of the audio baseline at 5dB, and accuracy is comparable at 10 dB. Consequently, whenever the audio SNR is lower than 5dB, it becomes more advantageous to use a radio-based VAD system. This is assuming the radio signal SNR distribution is the same as in the dataset, and the matching point can be even higher. For example, radio at higher SNR can match the performance of audio SNR at 10dB or more.

The present teaching explores RadioVAD, an mmWave-based interference-resilient voice activity detector that can be focused on the sound source vibration. Based on the spatial separation capabilities of mmWave-based sensing, a voice activity detector that is robust against interference from other sound sources can be built. Extensive experiments indicate great potential for using mmWave for voice-activity detection with the inherent benefits of mmWave such as low computational complexity, privacy preservation, and occlusion resistance. RadioVAD can combat interference sources significantly, and match the performance of a microphone based VAD.

FIG. 58 illustrates a flow chart of an exemplary method 5800 for radio-based voice activity detection, according to some embodiments of the present disclosure. In various embodiments, the method 5800 can be performed by the systems disclosed above. At operation 5802, a radio signal is obtained. The radio is transmitted from a transmitter to a receiver through a wireless channel of a venue. The wireless channel is impacted by a voice activity of a target voice source in the venue. At operation 5804, a time series of channel information (CI) of the wireless channel is computed based on the radio signal. At operation 5806, the voice activity of the target voice source is detected based on the time series of CI of the wireless channel, without using any signal other than the radio signal. The order of the operations in FIG. 58 may be changed according to various embodiments of the present teaching.

In some embodiments, the present teaching discloses a system for radio-based voice activity detection (VAD). The system can use channel information (CI) obtained from wireless signal (e.g. WiFi signal, mmWave, 28 GHz or 60GHz, or radar signal, or UWB signal) transmitted from a transmitter and received by a receiver to (selectively) detect voice activity of a target (or "selected") user and to output a binary VAD output, which is called "radioVAD" (radio-based VAD), in a venue, even in the presence of voice activity of one or more non-target users, without using voice input (i.e. no microphone input). Based on the radioVAD, a task may be performed, such as activating a user-interface, activating/deactivating a voice interface, toggling on/off a sound input, muting/un-muting a microphone, capturing a sound using the microphone, recording the sound, transmitting/not transmitting a captured sound, removing silent and/or unrelated (e.g. other non-target users) sound segments from captured/recorded/transmitted sound, performing speech recognition, performing another task based on the recognized speech, performing speaking recognition, performing another task based on the recognized speaker, etc.

In some embodiments, the channel information (CI) may be channel impulse response (CIR), channel frequency response (CFR), and/or channel state information (CSI), RSSI, etc. obtained from the received wireless signal. The wireless signal may be an mmWave/UWB/radar signal. The transmitter and the receiver may be co-located (e.g. on the same device, or on the same circuit board such as in the case of a radar), or at different locations.

In some embodiments, transmitter (Type1 device, TX) and/or receiver (Type2 device, RX) may each have an antenna array, or distributed antennas. There may be multiple receivers each receiving the wireless signal from the transmitter. There may be multiple transmitters each transmitting a respectively wireless signal to the receiver. There may be multiple transmitters and multiple receivers, each transmitter transmitting a respectively wireless signal to one or more receivers. The TX/RX device may have a commodity wireless networking or communication chip/chipset which may operate in a radar mode. The radar mode may be enabled by attaching an extra antenna array to the chipset. It may use the chip/chipset to transmit the wireless signal using a transmitting radio, and to receive the reflected wireless signal using a receiving radio. The chip may transmit/receive simultaneously or contemporaneously. The chip may switch rapidly between transmit and receive to simulate or mimic "simultaneous" transmit/receive.

In some embodiments, the transmitters and receivers may be in a same venue (e.g. a home, a room, an office, a walkway, a common area, or a facility). The transmitters may be physically next to, adjacent to, or at a distance from, the receivers. At least one object or "source" object (e.g. a person, two people or more than two people) may be present in the venue each generating a respective source signal (e.g. speech or voice signal from each person, talking, singing, dialog, one-at-a-time speech, simultaneous two or more people talking). A mixture signal may be obtained (e.g. sound captured by microphone containing simultaneous dialog/singing/speech/voice of two people). The mixture signal comprises a mixture (e.g. a sum, weighted sum, product, weighted product, etc.) of the signals from the at least one source. The source signal may be generated in the presence of background noise. For example, two people may speak in a noisy environment, e.g. train station, airport, or a home/office. The background noise may be crowd sound, mechanical sound, motor/engine sound, vacuum cleaner/fan/ machine/refrigerator/heater/air conditioner.

In some embodiments, one goal of voice activity detection (VAD) is to detect presence of speech (e.g. detect voice activity) using wireless (radio) signals only, without using voice signals. The goal of interference-resistant VAD (irVAD) is to detect presence of target speech (e.g. voice activity of target user) in the presence of interference (e.g. voice activity of non-target users) and noise. In some embodiments, the disclosed system can detect voice activity based on radio signal (e.g. based on CI obtained from received radio signal) without using any media input (e.g. input of video, or image/visual, or audio or speech or sound) or any data transmitted in the radio signal by transmitter (Type 1 device).

The following numbered clauses provide implementation examples for radio-based voice activity detection.

Clause F1. A method/device/system/software of a radio-based voice activity detection system, comprising: detecting voice activity based on a radio signal using a processor, a memory and a set of instruction of a device of the system, without using any media signal. The radio signal may transmitted by Type 1 device and received by Type 2 device. TSCI may be obtained in Type 2 device based on received radio signal.

Clause F2. The method/device/system/software of the radio-based voice activity detection system of Clause F1, comprising: wherein the radio signal is a wireless signal transmitted by a Type 1 heterogeneous wireless device of the system in a venue; wherein the radio signal is received by a Type 2 heterogeneous wireless device of the system through a wireless multipath channel of the venue, wherein the wireless multipath channel is impacted by the voice activity in the venue; obtaining a time series of channel information (CI) of the wireless multipath channel based on the received radio signal; detecting the voice activity based on the time series of CI (TSCI) of the wireless multipath channel.

Clause F3. The method/device/system/software of the radio-based voice activity detection system of Clause F1 or F2, further comprising: wherein the radio signal comprises at least one of: a data communication signal, a wireless network signal, a standard compliant signal, a wireless local area network (WLAN) signal, a WiFi signal, an IEEE 802 signal, an IEEE 802.11 signal, an IEEE 802.11bf signal, an IEEE 802.11 directional multi-gigabit (DMG) signal, a wireless communication network signal, a 3GPP signal, a 4G/LTE/5G/6G/7G/8G signal, a wireless sensing signal, a wireless sounding signal, a radar signal, a millimeter wave (mmWave) signal, a UWB signal, or a electromagnetic signal above 40 kHz; wherein the media signal comprises at least one of: a microphone signal, a speech signal, a vocal signal, an audio signal, a signal less than 40kHz, an acoustic signal, an audible signal, a telephone signal, a tele-conferencing signal, an audio-telephony signal, a conference call signal, a visual signal, a video signal, a video-telephony signal, a video-conferencing signal, a media streaming signal, or a multimedia signal.

Clause F4. The method/device/system/software of the radio-based voice activity detection system of Clauses F1 to F3, comprising: wherein the radio signal is a data communication signal; detecting the voice activity without using any data payload communicated in the data communication signal.

Clause F5. The method/device/system/software of the radio-based voice activity detection system of any previous F Clause, comprising: detecting the voice activity without using any media signal data communicated in the data communication signal.

Clause F6. The method/device/system/software of a radio-based voice activity detection system of any previous F Clause, comprising: detecting the voice activity of a target voice source in the venue, wherein the wireless multipath channel is impacted by a voice producing motion of the target voice source.

In some embodiments, the voice-related radio feature (TSRF) is bandlimited. Telephone speech signal may be bandlimited 4kHz. Speech signal may be bandlimited to 7kHz. Audio may be bandlimited to 20kHz. TSRF may be bandlimited to half of sounding frequency, which may be half of 1/10/100/1000/10000/100000/1000000 Hz. To play safe here, TSRF may be bandlimited to 1Mhz. It can be bandlimited to 100kHz, or 10kHz also.

Clause F7. The method/device/system/software of a radio-based voice activity detection system of any previous F Clause, comprising: computing a time series of radio feature (TSRF) based on the TSCI, each radio feature (RF) of the TSRF being computed based on a respective sliding window of the TSCI, wherein the TSRF is a baseband signal bandlimited to 1MHz; detecting the voice activity based on the TSRF.

In some embodiments, the system can detect voice activity by detecting voice-related characteristics (e.g. pitch, harmonics of the pitch, time profile of pitch) that suggests the presence of voice/speech.

Clause F8. The method/device/system/software of a radio-based voice activity detection system of any previous F Clause, comprising: detecting a voice-related characteristics in the TSRF; detecting the voice activity based on the detected voice-related characteristics in the TSRF.

In some embodiments, there are many voice-related characteristics listed here that may be manifested in the TSRF (e.g. pitch, pitch profile, inter-mixed voice/unvoiced speech, etc.). These voice characteristics differentiate voice from non-voice (e.g. machine sound, wind sound, environmental sound, etc.). In particular, voiced speech (e.g. vowel, some consonants, liquid, etc.) has pitch. Unvoiced speech (e.g. most consonants, fricative, plosive) has no pitch. Vowels are voiced sound. Typical human pitch goes between 50Hz and 250Hz.

Clause F9. The method/device/system/software of a radio-based voice activity detection system of any previous F Clause, further comprising: wherein the voice-related characteristics comprises at least one of the following characteristics: a speech feature, a vowel, a consonant, a fricative, an affricate, a plosive, a nasal, an approximant, a liquid, a lateral, bilabial sound, velar sound, alveolar sound, a phone, a phoneme, voiced sound, unvoiced sound, a pitch of voiced speech, a foundation frequency, a voice-related frequency range, at least one harmonics of voiced-speech, at least one formant of speech, a time-varying pitch, a pitch profile, a voice-related time trend of pitch, a tone, a prosodic feature, a sequence of intermittent voiced and unvoiced sound, a musical feature, a speech timing, a speech pacing, a musical timing, a musical pacing, or an environmental sound.

In some embodiments, special voice-related characteristics: instantaneous pitch at a current time, which may be observable in TSRF.

Clause F10. The method/device/system/software of a radio-based voice activity detection system of any previous F Clause, further comprising: detecting an instantaneous pitch associated with a current time instance in the TSRF, wherein an instantaneous fundamental frequency associated with the instantaneous pitch is greater than a lower threshold and less than a upper threshold; detecting the voice activity based on the detected instantaneous pitch.

Human speech often has many harmonics. Some harmonics may be observable in TSRF.

Clause F11. The method/device/system/software of a radio-based voice activity detection system of any previous F Clause, further comprising: detecting at least one instantaneous harmonics of the instantaneous pitch associated with the current time instance in the TSRF, wherein a frequency associated with each respective harmonics is an integer multiple of the instantaneous fundamental frequency of the instantaneous pitch; detecting the voice activity based on the detected instantaneous harmonic of the pitch.

In some embodiments, besides having an instantaneous pitch, human speech has a time-varying pitch. The human speech may use the time-varying pitch to express many things: tones in a tonal language, prosidy, vowel pronunciation, consonant pronunciation, etc. The time-varying pitch comprise pitches within a certain time window.

Clause F12. The method/device/system/software of a radio-based voice activity detection system of any previous F Clause, further comprising: detecting a pitch profile comprising a plurality of instantaneous pitches associated with a plurality of respective time instances in the TSRF, wherein each instantaneous pitch associated with a respective time instance is associated with a respective instantaneous fundamental frequency greater than the lower threshold and less than the upper threshold; detecting a voice-related time trend of the plurality of instantaneous pitches in the pitch profile; detecting the voice activity based on the detected pitch profile and the detected voice-related time trend of the instantaneous pitches.

Clause F13. The method/device/system/software of a radio-based voice activity detection system of any previous F Clause, further comprising: wherein the time trend comprises at least one of: a local continuity of instantaneous pitches, a local continuity of the instantaneous frequencies, a local continuity of frequency of the instantaneous harmonics, a habitual pitch, a long term pitch, a variation of pitch around the habitual pitch, a timing or pacing of pitch change, a fast pitch change within a tone, or a slow pitch change reflecting prosody. The voice-related characteristics may be detected in time domain using neural network.

Clause F14. The method/device/system/software of a radio-based voice activity detection system of any previous F

Clause, further comprising: processing the TSRF with a neural network; detecting the voice-related characteristics based on the neural network processing of the TSFR.

In some embodiments, the voice-related characteristics may be detected in frequency domain, to perform frequency decomposition of TSRF.

Clause F15. The method/device/system/software of a radio-based voice activity detection system of any previous F

Clause, further comprising: computing a frequency decomposition of the TSRF by computing at least one of: a spectrogram, a short-time Fourier transform (STFT), a wavelet transform, a filter-bank representation, a harmonic analysis, a Fourier analysis, a multi-resolution analysis, a time-frequency decomposition, a time-frequency representation, a sonograph, a voiceprint, a voicegram, or a waterfall display; detecting the voice-related characteristics based on the frequency decomposition.

In some embodiments, the voice-related characteristics may be detected in frequency domain using neural network. An optional feature is to apply "overlap and concatenate" on the frequency decomposition to generate input to the neural network.

Clause F16. The method/device/system/software of a radio-based voice activity detection system of any previous F Clause, further comprising: processing the frequency decomposition of the TSRF with a neural network; detecting the voice-related characteristics based on the neural network processing of the frequency decomposition of the TSRF.

In some embodiments, the voice-related characteristics may be detected in frequency domain using some algorithm (i.e. not using neural network). The algorithm may detect the manifestation (e.g. pitch, harmonics, local continuity) of the voice-related characteristics in the TSRF.

Clause F17. The method/device/system/software of a radio-based voice activity detection system of any previous F Clause, further comprising: wherein the voice-related characteristics comprises a pitch of the voice activity; detecting an instantaneous pitch based on the frequency decomposition of the TSRF in a time window associated with the current time instance.

In some embodiments, speech signals may have harmonics in addition to the pitch. Some of the harmonics may be observable in the TSRF.

Clause F18. The method/device/system/software of a radio-based voice activity detection system of any previous F Clause, further comprising: wherein the voice-related characteristics further comprises a harmonics of the pitch of the voice activity; detecting an instantaneous harmonics based on the frequency decomposition of the TSRF in a time window associated with the current time instance.

In some embodiments, identify target voice source based on beamforming. First TSCI are the raw TSCI. Second TSCI are computed based on beamforming performed on the set of raw TSCI.

Clause F19. The method/device/system/software of a radio-based voice activity detection system of any previous F Clause, further comprising: wherein the Type 1 device or Type 2 device has an array of antennas; obtaining a set of raw TSCI of the wireless multipath channel based on the received radio signal, each raw TSCI associated with a respective antenna; obtaining a set of directional TSCI based on a beamforming performed on the set of raw TSCI obtained based on the received radio signal associated with the array of antennas, each directional TSCI associated with a direction relative to the array of antennas, wherein the TSCI is a particular directional TSCI.

Clause F20. The method/device/system/software of the radio-based voice activity detection system of any previous F Clause, further comprising: associating the target voice source with a component of the TSCI; wherein there is at least one non-target voice source undergoing respective asynchronous voice producing motion; wherein the wireless multipath channel is impacted asynchronously by the respective asynchronous voice producing motion of the at least one non-target voice source; associating each non-target voice source with another component of the TSCI or another directional TSCI; rejecting the at least one non-target voice source by computing the TSRF based on the component of the TSCI.

The following numbered clauses provide examples for radio-based voice activity detection.

Clause G1. A system for radio-based voice activity detection, comprising: a transmitter configured to transmit a radio signal through a wireless channel of a venue; a receiver configured to receive the radio signal through the wireless channel, wherein the wireless channel is impacted by a voice activity of a target voice source in the venue; and a processor configured for: computing a time series of channel information (CI) of the wireless channel based on the radio signal, and detecting the voice activity of the target voice source based on the time series of CI (TSCI) of the wireless channel, without using any media signal.

Clause G2. The system of Clause G1, wherein: the radio signal comprises at least one of: a data communication signal, a wireless network signal, a standard compliant signal, a wireless local area network (WLAN) signal, a WiFi signal, an IEEE 802 signal, an IEEE 802.11 signal, an IEEE 802.11bf signal, an IEEE 802.11 directional multi-gigabit (DMG) signal, a wireless communication network signal, a 3GPP signal, a 4G/LTE/5G/6G/7G/8G signal, a wireless sensing signal, a wireless sounding signal, a radar signal, a millimeter wave (mmWave) signal, a UWB signal, or an electromagnetic signal above 40 kHz; and the media signal comprises at least one of: a microphone signal, a speech signal, a vocal signal, an audio signal, a signal less than 40kHz, an acoustic signal, an audible signal, a telephone signal, a tele-conferencing signal, an audio-telephony signal, a conference call signal, a visual signal, a video signal, a video-telephony signal, a video-conferencing signal, a media streaming signal, or a multimedia signal.

Clause G3. The system of Clause G1 or G2, wherein: the radio signal is a data communication signal; and the voice activity is detected without using any data payload communicated in the data communication signal.

Clause G4. The system of any previous G Clause, wherein: the voice activity is detected without using any media signal data communicated in the data communication signal.

Clause G5. The system of any previous G Clause, wherein: the voice activity is associated with a voice producing motion of the target voice source.

Clause G6. The system of any previous G Clause, wherein detecting the voice activity comprises: computing a time series of radio feature (TSRF) based on the TSCI, each radio feature (RF) of the TSRF being computed based on a respective sliding window of the TSCI, wherein the TSRF is a baseband signal bandlimited to 1MHz; and detecting the voice activity based on the TSRF.

Clause G7. The system of any previous G Clause, wherein detecting the voice activity further comprises: detecting a voice-related characteristics in the TSRF; and detecting the voice activity based on the detected voice-related characteristics in the TSRF.

Clause G8. The system of any previous G Clause, wherein the voice-related characteristics comprises at least one of the following characteristics: a speech feature, a vowel, a consonant, a fricative, an affricate, a plosive, a nasal, an approximant, a liquid, a lateral, bilabial sound, velar sound, alveolar sound, a phone, a phoneme, voiced sound, unvoiced sound, a pitch of voiced speech, a foundation frequency, a voice-related frequency range, at least one harmonics of voiced-speech, at least one formant of speech, a time-varying pitch, a pitch profile, a voice-related time trend of pitch, a tone, a prosodic feature, a sequence of intermittent voiced and unvoiced sound, a musical feature, a speech timing, a speech pacing, a musical timing, a musical pacing, or an environmental sound.

Clause G9. The system of any previous G Clause, wherein detecting the voice activity further comprises: detecting an instantaneous pitch associated with a current time instance in the TSRF, wherein an instantaneous fundamental frequency associated with the instantaneous pitch is greater than a lower threshold and less than an upper threshold; and detecting the voice activity based on the detected instantaneous pitch.

Clause G10. The system of any previous G Clause, wherein detecting the voice activity further comprises: detecting at least one instantaneous harmonics of the instantaneous pitch associated with the current time instance in the TSRF, wherein a frequency associated with each respective harmonics is an integer multiple of the instantaneous fundamental frequency associated with the instantaneous pitch; and detecting the voice activity based on the at least one instantaneous harmonics of the instantaneous pitch.

Clause G11. The system of any previous G Clause, wherein detecting the voice activity further comprises: detecting a pitch profile comprising a plurality of instantaneous pitches associated with a plurality of respective time instances in the TSRF, wherein each instantaneous pitch associated with a respective time instance is associated with a respective instantaneous fundamental frequency greater than the lower threshold and less than the upper threshold; detecting a voice-related time trend of the plurality of instantaneous pitches in the pitch profile; and detecting the voice activity based on the pitch profile and the voice-related time trend of the plurality of instantaneous pitches.

Clause G12. The system of any previous G Clause, wherein the voice-related time trend comprises at least one of: a local continuity of instantaneous pitches, a local continuity of the instantaneous fundamental frequencies, a local continuity of frequency of the at least one instantaneous harmonics, a habitual pitch, a long term pitch, a variation of pitch around the habitual pitch, a timing or pacing of pitch change, a fast pitch change within a tone, or a slow pitch change reflecting prosody.

Clause G13. The system of any previous G Clause, wherein the processor is further configured for: processing the TSRF with a neural network; and detecting the voice-related characteristics based on the processing of the TSRF.

Clause G14. The system of any previous G Clause, wherein the processor is further configured for: computing a frequency decomposition of the TSRF by computing at least one of: a spectrogram, a short-time Fourier transform (STFT), a wavelet transform, a filter-bank representation, a harmonic analysis, a Fourier analysis, a multi-resolution analysis, a time-frequency decomposition, a time-frequency representation, a sonograph, a voiceprint, a voicegram, or a waterfall display; and detecting the voice-related characteristics based on the frequency decomposition.

Clause G15. The system of any previous G Clause, wherein detecting the voice-related characteristics comprises: processing the frequency decomposition of the TSRF with a neural network; and detecting the voice-related characteristics based on the processing of the frequency decomposition of the TSRF.

Clause G16. The system of any previous G Clause, wherein: the voice-related characteristics comprises a pitch of the voice activity; and the processor is further configured for detecting an instantaneous pitch based on the frequency decomposition of the TSRF in a time window associated with the current time instance.

Clause G17. The system of any previous G Clause, wherein: the voice-related characteristics further comprises a harmonics of the pitch of the voice activity; and the processor is further configured for detecting an instantaneous harmonics based on the frequency decomposition of the TSRF in a time window associated with the current time instance.

Clause G18. The system of any previous G Clause, wherein: the transmitter or the receiver has an array of antennas; and the processor is further configured for: obtaining a set of raw TSCI of the wireless channel based on the received radio signal, each raw TSCI being associated with a respective antenna in the array of antennas, and obtaining a set of directional TSCI based on a beamforming performed on the set of raw TSCI obtained based on the received radio signal associated with the array of antennas, each directional TSCI being associated with a direction relative to the array of antennas, wherein the TSCI is a particular directional TSCI of the set of directional TSCI.

Clause G19. The system of any previous G Clause, wherein the processor is further configured for: associating the target voice source with a component of the TSCI, wherein there is at least one non-target voice source undergoing respective asynchronous voice producing motion, wherein the wireless channel is impacted asynchronously by the respective asynchronous voice producing motion of the at least one non-target voice source; associating each non-target voice source with a different component of the TSCI or a different directional TSCI; selecting the component of the TSCI; and rejecting the at least one non-target voice source by computing the TSRF based on the selected component of the TSCI.

Clause G20. A method for radio-based voice activity detection, comprising: obtaining a radio signal transmitted from a transmitter to a receiver through a wireless channel of a venue, wherein the wireless channel is impacted by a voice activity of a target voice source in the venue; computing a time series of channel information (CI) of the wireless channel based on the radio signal; and detecting the voice activity of the target voice source based on the time series of CI (TSCI) of the wireless channel, without using any signal other than the radio signal.

Recent advances in RF-based tracking enable high-accuracy trajectory tracking, allowing one to collect high-accuracy trajectories with cost-effective WiFi clients and a single AP for a broad variety of environments. However, leveraging RF-based tracking for high-precision floor plan construction entails great challenges. First, with only a single AP employed, one cannot have global reference information, which is necessary to connect different trajectories and construct a floor plan. Second, with only distance estimation available, there is still need to rely on inaccurate sensors for the direction information. Third, given the imperfect information, it is non-trivial to fuse trajectories collected by a robot to recover a floor plan.

In some embodiments, the present teaching overcomes the above challenges and discloses EZMAP, a novel system to boost the automatic construction of indoor floor plans. In some embodiments, EZMAP employs a commodity home robot, equipped with WiFi and inertial sensors, to roam around the environment and collect data. It requires only a single AP and can map not only public spaces such as malls and office buildings but also home environments that are barely explored by existing works. The reconstructed map represents hallway structure, room layout, as well as their sizes (i.e., corridor widths and room sizes). In some embodiments, EZMAP system uses a novel pipeline to generate such a map in three distinct ways. The EZMAP system may first break down the crowdsourced trajectories of arbitrary lengths into atomic segments, which circumvent the potential accumulative orientation errors and underpin trajectory matching. Second, the EZMAP system can use a robust hierarchical matching scheme to group the atomic segments. Taking advantage of the high-accuracy distance estimation, the EZMAP system may first match the atomic segments by their intrinsic geometric constrains. Then the EZMAP system can further classify them by examining the Received Signal Strength Indicator (RSSI) and magnetic field strength (MFS) information along each segment, providing global information about the actual location. Third, the EZMAP system can utilize a trajectory bundling and fusion technique to robustly embed the clustered segments, i.e., determining their positions relative to each other and thus reconstructing the floor plan.

In some embodiments, the EZMAP system can be implemented using commodity WiFi chipsets and inertial sensors, attached to a robot. The EZMAP system can be deployed at three different scenarios, i.e., a campus store, an office building, and a home. Leveraging a small number of crowdsourcing trajectories, the system generates detailed maps with high accuracy when implemented in an office building, a home environment, and a campus building. With the demonstrated high accuracy yet minimum infrastructure requirement, EZMAP paves the way for many important indoor applications for intelligent buildings and smart homes.

In some embodiments, the present teaching discloses an accurate automatic indoor map reconstruction system that applies to various indoor environments, including private environments such as homes, which do not have numerous WiFi APs. In some embodiments, the present teaching discloses an algorithm that accurately and robustly classifies, bundles, and embeds robot-collected trajectories to reconstruct an indoor map, which works with only one AP. The disclosed system can be implemented on commodity hardware to validate its performance in different buildings, demonstrating outstanding performance in offices, stores, and homes. Different from other systems, the disclosed system employs the increasingly popular home robots for data collection and extends the scope to home environments.

FIG. 59 illustrates an exemplary diagram of a map reconstruction system 5900, according to some embodiments of the present disclosure. As shown in FIG. 59, the map reconstruction system 5900 includes a Trajectory Acquisition module 5910, a Trajectory Segmentation module 5920, a Trajectory Matching module 5930, a Trajectory Bundling module 5940, and a Trajectory Fusing and Shaping module 5950.

In some embodiments, the system can collect trajectories using a tracking device 5905, which may be implemented on a robot, a human-pushed cart, a phone, a tablet, a computer, etc. In some embodiments, the tracking device 5905 may include or serve as a Bot or receiver configured for receiving wireless signals from an Origin/Satellite or transmitter 5906. Trajectories or traces can be acquired by the Trajectory Acquisition module 5910 of the system based on the received wireless signals, which are impacted by the wireless channel and the motion of the tracking device 5905.

In some embodiments, the traces can be collected by humans or robots with one AP deployed in the environment. The traces may contain the distance information obtained by an RF-based Inertial Measurement system and direction information derived from inertial sensors. RSSI of the AP and the MFS are also recorded. Then, the collected trajectories, which could be of arbitrary lengths, are divided into short segments, named as atomic segments, to overcome the potential errors accumulated in orientation while leveraging the accurate distance estimation (Trajectory Segmentation). The atomic segments are then clustered by their intrinsic geometric constraints as well as the accompanying time series of RSSI and MFS (Segment Matching). The clustered segments are then positioned by bundling the long trajectories and thereby inferring their relative positions (Trajectory Bundling). Then all trajectories are fused, taking their original shapes into account, to output the reconstructed map with corridor widths and room sizes (Trajectory Fusion and Shaping).

In some embodiments, the crowdsourcing trajectory tracking is based on RIM, an RF-based Inertial Measurement system, for distance estimation and inertial sensors for orientation estimation. RIM can serve as a sub-system of the disclosed map construction system and is good at estimating the moving distance of wheeled platforms and robots. Leveraging multipath profiles as virtual antennas with a super-resolution virtual antenna alignment algorithm, RIM achieves centimeter accuracy in moving distance over a multipath-rich area. RIM needs only a single AP, and captures Channel State Information (CSI) from data packets for its moving distance calculation. In some embodiments, EZMAP can utilize RIM's algorithm to estimate the moving distance with high accuracy while using inertial sensors to measure the turning angles and heading information, which together shape the geometric properties. For device setup, one can either use a pushing cart or a robot to carry the tracking device. In both cases, the tracking device may include customized hardware with a commodity WiFi chipset and IMU on it. In some embodiments, the EZMAP may include a software solution making no change to hardware.

The acquired trajectories come in various lengths from meters to tens of meters or even longer. In some embodiments, EZMAP prefers long trajectories in general, as they likely cover and connect wider areas and thus contain more information to be uniquely identified. However, long trajectories suffer from large accumulative errors particularly in orientation. To overcome the issues, the system can use the Trajectory Segmentation module 5920 to decompose all collected trajectories into short pieces in a novel form of atomic segments that better preserve the accurate geometric shape information.

In some embodiments, the system can divide each long trajectory into the structures that includes a straight segment with two turns on the two ends, as shown in FIG. 60. One can name such a structure an atomic segment and use it as the basic unit of trajectories in EZMAP. Such atomization can be accomplished by a simple turn detection based on inertial sensor readings. In some embodiments, the EZMAP system may calculate the change rate of angle (the first-order reciprocal of angle to time) of accelerometer and gyroscope data to detect turns.

The design of atomic segment is inspired by the below insights: First, the atomic segments disconnect the original trajectories at each turn, because orientation errors easily accumulate during a turn (as seen in the example in FIG. 61). As a result, it preserves the accurate distance information while resets significant orientation errors before they could accumulate over multiple turns. Second, the two turns are kept as parts of each atomic segment because they provide more information to cluster the segments that have similar lengths. Taking the segments in FIG. 60 as an example, two atomic segments, ending with a left turn and a right turn respectively, can be separated even when they have the same length. Third, considering real-world building layouts, such atomic segments can be frequently obtained. As will be demonstrated later, the global location information (i.e., RSSI and MFS time series) associated with such segments suffice to uniquely cluster and reconnect them, jointly considering the precise geometric shape of the segments.

In some embodiments, some trajectories will not be segmented by the atomization. For example, if the target circles around and moves along a curved pathway, the resulting trajectory would be continuously "turning" and will never be cut. These curved segments are still useful, as they possibly provide information about some open spaces and/or rooms, and will be handled separately.

To generate an accurate floor plan with tracking traces, one needs to accurately estimate the relative position of each trajectory, which is very challenging without global reference or the start point of each trajectory. As discussed before, the system can divide the long trajectories into atomic segments and resort to determining the relative position of each atomic segment. To accurately predict the relative position of each segment, the system can take two steps. In some embodiments, the system may first cluster the atomic segments belongings to the same location. Then the atomic segments in the same cluster are positioned and bundled by inferring its relative position from the long trajectory.

To identify the segments at the same location and distinguish those at different locations, the system can utilize a trajectory matching algorithm at the Trajectory Matching module 5930 to recognize the segments belonging to the same location and distinguish those belonging to different locations by their geometric shapes and time series of RSSI and MFS. An exemplary matching process is shown in FIG. 62.

With the unique structure designed for segments, each atomic segment has a geometric shape which contains the angle information of two turns and distance information of the road section in between. Atomic segments related to same path show similar geometric shapes. Geometric constraints are designed to sort the segments with similar shapes together based on their geometric shape information. Examples of atomic segments belonging to the same path are shown in FIG. 60.

The constraint of geometric shapes has two parts, distance constraint and angle constraint. As for the distance constraint, based on experimental observations and the distance tracking accuracy of the tracking client, one can limit the distance difference between segments on the same path to be no more than 3 m in some embodiments. In other words, if the difference of estimated distances of two road sections is greater than 3m, these two segments are unlikely to be at the same location. As for the angle constraint, considering certain angle errors, the angle difference between the same turn is limited to be no more than 30 degrees in some embodiments. In some embodiments, the atomic segments from the same location with different turnings (left and right) would not be clustered together. But this will not affect the final reconstructed map because they would be recognized and merged together later.

To verify the effectiveness of the geometric constraints, one can conduct a comparative experiment where over 200 atomic segments are used to compare the matching accuracy with and without geometric constraints. The experimental results validate that the trajectory constraint can effectively improve the accuracy of segment matching, which will be shown later.

In some embodiments, the geometric constraints alone are insufficient to support segment matching with high accuracy since it cannot separate the segments from different paths but having similar geometric shapes. Therefore, a robust global reference is crucial to the matching accuracy and the reconstruction performance. In some embodiments, the EZMAP system can leverage the time series of MFS and RSSI along the segment, denoted as ambient properties for global reference information and utilize a robust algorithm to match them.

The magnetic field anomalies caused by ferromagnetic construction materials (e.g., reinforcing steel bars) on the indoor path are generally stationary and unique over time. The time series of MFS are collected when a user or a robot walks along a specific indoor path and are used as the representative feature of the corresponding path. As shown in FIG. 63A, the time series of MFS data along the same indoor path is similar, while those along different indoor paths differ considerably. Similar characteristics also appear on the time series of RSSI values measured for a single AP, as shown in FIG. 63B. Although RSSI values at a single location suffer from very limited space resolution, a series of RSSI along a path provide more distinctive information.

In some embodiments, a cluster can be generated when the similarity value of two atomic segments based on the time series of MFS and RSSI is below a threshold. A similarity vector can be calculated for an atomic segment, measuring the similarity between itself and each existing cluster of atomic segments. For an atomic segment j, dynamic time warping (DTW) can be applied to calculate its RSSI similarity vector $D_{j,R}$ and MFS similarity vector $D_{j,M}$, respectively. $D_{j,R}$ and $D_{j,M}$ are normalized with min-max normalization and summed together to obtain the similarity vector as follows:

$$D_j = \overline{D_{j,R}} + \overline{D_{j,M}} = [d_{j,1}, d_{j,2}, \ldots, d_{j,i}, \ldots, d_{j,N}], \qquad (48)$$

where $d_{j,i}$ denotes the similarity value between atomic segment $j$ to the $i^{th}$ cluster, and $N$ denotes the total number of clusters. If $d_{j,i}$ is below a pre-defined threshold (e.g., 0.2), this atomic segment is clustered to the $i^{th}$ cluster and can be matched with other segments from the $i^{th}$ cluster. If an atomic segment cannot be matched with any existing cluster, the system can generate a new cluster for it. Based on some experimental observations, two segments with a similarity value below 0.2 can be considered matched. As the system has normalized the RSSI vector and MFS vector during fusion, the threshold can be generalized to various environments.

In some corner cases, the threshold may not work well. For example, when: 1) the similarity value of two matched segments (i.e., two segments from the same location) is greater than 0.2, and 2) there are multiple similarity values lower than 0.2 but only one segment should be matched. In the first case, although the segments are mistakenly treated as belonging to two clusters, the system will be able to merge them together. As for the second case, the system will match the target segment with the most similar one, meaning the one with the smallest similarity value. The threshold is tested to be valid in experiments in three scenarios.

For the matched segments within the same cluster, the system can not only know that they should come from the same location but also have the alignment information between each pair of the segments, e.g., the system can have information about how each turn on the atomic segment corresponds to each other on the other matched segments.

After identifying the segments belonging to the same location, to reconstruct an accurate floor plan with these segments, the system may also identify their relative positions. In some embodiments, the system may utilize a trajectory bundling algorithm at the Trajectory Bundling module 5940 to determine the position of atomic segments.

In some embodiments, the long trajectories are bundled leveraging their matched atomic segments. For every two long trajectories that have matched segments, they are bundled in the following two steps: 1) The first turning points of two matched segments are stitched together. 2) The long trajectories are rotated so that the directions of the path section of the two matched atomic segments are consistent. Every long trajectory is bundled to others via aligning their matched segments. After long trajectories are bundled, the system can determine the coordinates of atomic segments by utilizing their spatial relationship on their original long trajectories. An example of trajectory bundling is shown in FIG. 64, where two trajectories are bundled by aligning their matched segments.

While the long trajectory is treated as a rigid body for trajectory bundling, the long trajectories containing large accumulative direction errors, as shown in FIG. 61, can reduce the accuracy of the generated map. As such, the system may adjust the positions of atomic segments on the long trajectory to avoid the impact of accumulated errors on the generated map, e.g. by the Trajectory Fusing and Shaping module 5950.

Although the rough position of atomic segment can be inferred by bundling long trajectories, atomic segments belonging to the same path may have severe coordinate deviation, as shown in FIG. 65, which is undesirable. Therefore, the system can utilize a trajectory fusion algorithm to generate a more accurate and well-shaped map next, e.g. at the Trajectory Fusing and Shaping module 5950.

Leveraging the characteristics of matched segments, the system can fuse matched atomic segments in two steps. First, for matched atomic segments at the same path, the inner-cluster constraints, including angle and positions of their turns, are utilized to update their positions. The system can calculate the medium coordinate of their endpoints at the same turn and update the coordinate of each endpoint by:

$$New_location = \begin{aligned}&(1-b)^{*}cluster_medium_location\\&+b^{*}original_location,\end{aligned} \quad (49)$$

where *b* E (0,1) is a parameter balancing the cluster median location and original location.

Then, the intra-cluster global information can be leveraged to merge the turns that are mistakenly separated, which would happen when atomic segments on the same path are classified into different clusters. DTW can be applied again to merge the mistakenly separated turns. After the trajectories are bundled, the system can search for the turns within a small area. In some embodiments, the system may only apply the DTW on the turns close to each other. If the DTW distance of MFS and RSSI of two turns are below a threshold (e.g. 0.05 according to experiments), the two turns are considered to be at the same location and merged together. Thus, the mistakenly separated segments belonging to the same path are correctly merged.

Then, with the new coordinates of two endpoints, each atomic segment is transformed to its new position by Helmert transformation, a similarity transformation frequently used in geodesy to produce datum transformations between datums. Two dimensional Helmert transformation is performed here. With the knowledge of the original coordinate of two turning points and the updated coordinates of each point on the segments, Helmert Transformation calculates the updated coordinates of each point on the segments. The transformed segments preserve the shape and 2D information of the original atomic segments. FIG. 65 shows an example of trajectory fusion process, where Helmert Transformation is performed on four trajectories.

When segmenting the long trajectories, there are atomic segments without straight path section in between. Instead, they contain curved sections with continuous "turnings". While the straight segments, as discussed before, reflect the major layout of the building, the curved segments also contain useful information about rooms, curved corridors, and open spaces. Therefore, the system can separately process them to add more details to the recovered floor plan.

In some embodiments, the position of the curved segment is deduced from its spatial relationship with straight segments. The system may first find out straight segments that directly connect with the curved segment. Then, the positions of straight segments are utilized to estimate the position of the curved segment and Helmert transformation is then performed on the curved segment. This process is shown in FIG. 66. Without losing the shape information, curved segments are grouped with straight segments to reconstruct the curve corridors, rooms and open spaces. Hence, the map construction algorithm can not only generate straight corridors, but also estimate the location and area of the rooms, making the floor plan more detailed and comprehensive. In some embodiments, to provide a well-shaped floor plan, an alpha-shape method is utilized to extract the contour of trajectories.

In some embodiments, the disclosed system and method are evaluated in three scenarios: Scenario I is an office with an area of 22.0 m $\times$ 36.5 m, which includes corridors, rooms, doors, and elevators as shown in FIG. 67A; Scenario II is the second floor of a townhouse with an area of 5.8 m $\times$ 12.2 m, including a kitchen and a living room as shown in FIG. 67B; and Scenario III is a dining court of a campus building with an area of 49.0 m $\times$ 12.5 m, which includes a dining hall and hallways around it, as shown in FIG. 67C. The goal is to reconstruct the detailed floor plan using the disclosed system.

In Scenario I, the tracking device is installed on a cart pushed by human. Traces are collected on 12 different days in 5 months, with a total of 64 crowdsourcing trajectories collected. Since the different turning directions are considered, there are 18 clusters in this data set. In Scenarios II and III, the tracking device is equipped on a robot, which collects 49 trajectories with 8 clusters over 16 different days in 5 months for Scenario II, and 54 trajectories with 19 clusters on 7 different days in 2 months for Scenario III. One can collect data over a long course to validate the robustness of EZMAP over environmental dynamics, or only obtain a small amount of trajectories to demonstrate the effectiveness of the disclosed system.

In some embodiments, EZMAP employs a commodity home robot to collect data because it is labor-saving and efficient. Moreover, home robots are widely available nowadays. EZMAP is not limited to high-precision robotic tracking based on RIM. The disclosed map construction algorithm is also applicable to high-precision pedestrian tracking.

The results of the three scenarios are presented in FIGS. 68-70, respectively. For all three scenarios, the first subfigure (i.e., FIG. 68A, FIG. 69A, and FIG. 70A) shows the bundled trajectories. The reconstructed floor plan of Scenario I is shown in FIG. 68B, where the accessible area of rooms and open space are well reconstructed with curved trajectories. The reconstructed hallway plan of Scenario I is shown in FIG. 68C, while the ground-truth hallway plan extracted by alpha-shape is shown in FIG. 68D. In Scenario II, the reconstructed floor plan is shown in FIG. 69C, where the open space in Kitchen is well reconstructed by the disclosed system. Without the need of anchor points required by most related works, the disclosed system demonstrates the capability to reconstruct the floor plan for not only large and public areas, but also small and private environments like homes. FIG. 70C shows the well reconstructed floor plan of Scenario III. The result shows the disclosed system can accurately reconstruct the floor plan for a variety of areas with only a single AP.

The matching performance, room size accuracy, hallway shape accuracy and the construction efficiency of EZMAP are evaluated. As for hallway shape and room size, one can compare the disclosed system with SenseWit and CorwdInside.

In some embodiments, the matching performance is evaluated by purity measure and Normalized Mutual Information (NMI) score. The purity score $s_c$ is defined as

$$s_c = \frac{1}{M}\sum_{i=1}^{c} C_i, \qquad (50)$$

where $C_i$ is the number of atomic segments in the largest class inside each cluster, $c$ is the number of clusters and and $M$ is the total number of atomic segments. The NMI is defined as

$$NMI(Y,C) = \frac{2\times I(Y;C)}{[H(Y)+H(C)]}, \qquad (51)$$

where $Y$ denotes class labels, $C$ denotes cluster labels, $H(.)$ is Entropy, and $I(Y;C)$ is Mutual Information between $Y$ and $C$.

In one experiment according to some embodiments, one can collect 206 atomic segments belonging to 18 true clusters, which are clustered into 20 clusters. All the atomic segments belong to the largest class inside the cluster, except for one, resulting in a purity score of 99.51% and a normalized mutual information score of 95.05%.

To justify that the geometric constraint is very beneficial to atomic trajectory matching, a comparison experiment is conducted, where the matching performance is calculated without adopting the geometric constraint. The purity score drops to 87.38%, and the NMI score becomes 81.80%, proving that the geometric constraint can improve the matching performance significantly. On the other hand, it also demonstrates, surprisingly, that a reasonable accuracy of over 80% of segment matching could be achieved by using magnetism and RSSI of a single AP.

The room size is evaluated by the error between the reconstructed room size and ground truth area, which is calculated as:

$$Error = \frac{|reconstructed_room_size - ground_truth_size|}{ground_truth_size}. \qquad (52)$$

One can compare the error of the disclosed system with SenseWit and CrowdInside with the data collected in office. The average error of SenseWit is 31.4%, and that of CrowdInside is 40.6%. The disclosed system has an average error of 36.1%, which is lower than CrowdInside and higher than SenseWit. Without requiring lots of anchor points as SenseWit, the accuracy of the disclosed system is still comparable. The estimation error is mainly due to obstacles like tables, drawers, and chairs in the office, which prevent the cart or robot from walking through the entire room.

One can also evaluate the hallway shape with the same metric as SenseWit, which is shown below,

$$\begin{aligned} \mathcal{P} &= \frac{|S_{gen} \cap S_{true}|}{|S_{gen}|}, \\ \mathcal{R} &= \frac{|S_{gen} \cap S_{true}|}{|S_{true}|}, \\ \mathcal{F} &= 2 * \frac{\mathcal{P} * \mathcal{R}}{\mathcal{P} + \mathcal{R}}, \end{aligned} \quad (53)$$

Where $\mathcal{P}$ is the precision of the hallway shape, $\mathcal{R}$ is the recall, and $\mathcal{F}$ is the harmonic mean of precision and recall. $\mathcal{P}$ is defined as the overlapped area divided by the reconstructed hallway area. $\mathcal{R}$ is defined as the overlapped area divided by the ground-truth hallway area.

The evaluation result is shown in Table 12. The origin point and the orientation of ground truth hallway and the generated hallway are aligned. Table 12 shows that the $\mathcal{P}$ , $\mathcal{R}$ , and $\mathcal{F}$ of the disclosed system are better than CrowdInside and comparable with SenseWit. The precision of the disclosed system is higher than SenseWit, but recall rate is lower than SenseWit, which is because the disclosed system performs multi-trajectory fusion so that the hallway widths are less than the ground truths. SenseWit needs various anchors to achieve comparable performance with the disclosed system, showing that the disclosed system can generate accurate hallway plans for various environments, especially for the private environments without anchor points, such as home.

Table 12: Evaluation Results of Hallway Shape

| | *Disclosed system* | *SenseWit* | *CrowdInside* |
|---|---|---|---|
| $\mathcal{P}$ | 78.18% | 75.3% | 59.5% |
| $\mathcal{R}$ | 75.10% | 82.4% | 47.1% |
| $\mathcal{F}$ | 76.61% | 78.69% | 52.0% |

Different from most crowdsourcing based indoor map construction system, EZMAP can generate hallway map with much fewer trajectories while achieving comparable precision. One can evaluate the construction efficiency of EZMAP in Scenario I. FIGS. 71A-71E show the reconstructed hallway with a number of 24, 35, 46, 52, and 61 trajectories, respectively. The hallway construction accuracy with different number of trajectories is 53.76%, 67.44%, 71.99%, 73.46%, and 76.61%, respectively. The comparison of EZMAP, SenseWit, and CrowdInside is shown in Table 13. SenseWit used over 300 trajectories to cover a campus library with 464 m^2 area, and CrowdInside employed over 150 trajectories to cover an environment with 448 m^2 area. Table 13 shows that the disclosed system EZMAP achieves comparable accuracy to SenseWit with only one fourth of the number of trajectories used in a twice larger environment, and EZMAP is more accurate than CrowdInside with much less trajectories needed. The efficiency evaluation result shows that EZMAP can rapidly reconstruct accurate hallway. In addition, the design of EZMAP employs low-cost home robots, which also saves significant manpower for data collection.

Table 13: Evaluation Results of Construction Efficiency

| | *Disclosed system* | *SenseWit* | *CrowdInside* |
|---|---|---|---|
| Trajectory Number | 61 | 300 | 150 |
| Area | 803 $m^2$ | 464 $m^2$ | 448 $m^2$ |
| Trajectory Number/Area | 0.08 $/m^2$ | 0.65 $/m^2$ | 0.33 $/m^2$ |

In some embodiments, the present teaching discloses EZMAP, a universal automatic floor plan construction system that does not need any prerequisite knowledge of buildings in advance. EZMAP is a high-accuracy and low-cost floor plan reconstruction system leveraging advanced RF-based inertial tracking. The disclosed system combines local information from RF tracking with global contexts from inertial sensing (e.g., magnetic field strength) for an accurate map. EZMAP benefits from centimeter-accuracy indoor tracking using RF signals. It leverages commodity WiFi to estimate accurate moving distances and employs inertial sensors for orientation reckoning. EZMAP then processes crowdsourced trajectories with a novel pipeline of trajectory segmentation, matching, bundling, and shaping, which ultimately reconstructs a floor plan with not only skeletal layouts but also detailed area sizes of straight/curved corridors, open spaces, and rooms. Requiring minimal infrastructure and a small amount of data, EZMAP can scale to a number of various buildings including public malls, offices, as well as home environments.

EZMAP is the first RF-based system that can accurately reconstruct map of private environments. The performance can be validated with commodity WiFi in an office building or home, which shows that the disclosed system can efficiently generate faithful maps for a targeted area. With the ubiquitous deployment of WiFi devices, the disclosed system will advance the development of indoor location-based services.

FIG. 72 illustrates a flow chart of an exemplary method 7200 for map generation, according to some embodiments of the present disclosure. In various embodiments, the method 7200 can be performed by the systems disclosed above. At operation 7202, sensing data and a plurality of trajectories in a venue are obtained. Each trajectory is a time series of spatial coordinates (TSSC) representing a path traversed by a respective object in the venue. Each TSSC is accompanied by at least one respective time series of sensing data (TSSD) collected while the respective object traverses the path in the venue. In some examples, the sensing data and trajectories may be collected using channel information (e.g. CSI, CIR, CFR) captured from received wireless signal (e.g. based on signals according to WiFi, 4G/5G/6G/7G, etc.). In other examples, the sensing data and trajectories may be collected by other means such as radar, sensors, sensors in smart phones, navigation systems, etc.

At operation 7204, each TSSC and its accompanying at least one TSSD are segmented into segments. At operation 7206, the plurality of trajectories are bundled based on similarity measures between pairs of the segments. At operation 7208, the bundled trajectories are fused to generate fused trajectories. At operation 7210, a shape of the fused trajectories is computed. At operation 7212, a map of the venue is generated based on the computed shape. The order of the operations in FIG. 72 may be changed according to various embodiments of the present teaching.

In some embodiments, a 2D (or 3D) map is generated based on a number (e.g. 100, 1000 or more) of 2D (or 3D) trajectories, each together with supplementary sensor information (e.g. RSSI, magnetic field strength/MFS). Each trajectory represents a path of a movement of an object in a venue (e.g. building, home, warehouse, parking lot, etc.). An example of 3D trajectory may be a path of a person as he walks up and down multiple floors of a building/facility.

Some 2D/3D trajectories may be collected using channel information (e.g. CSI, CIR, CFR) captured from received wireless signal (e.g. WiFi, 4G/5G/6G/7G, etc.). They may also be collected by other means such as radar, sensors, sensors in smart phones, navigation systems, etc. They may be crowd sourced.

The 2D/3D map to be generated may include the all reachable areas in the venue. For example, a wide hallway should appear thicker. A narrow hallway should appear thinner. A portion of a hallway obstructed by obstacles (and thus not reachable) should not appear in the 2D/3D map. An accessible room should typically appear roughly as a rectangle, but without the internal space obstructed by obstacles (e.g. wall fixtures, sofa, table, cabinets, stove, fridge, lights, fixtures, boxes, etc.).

An exemplary method comprises the following steps.

The first step is trajectory segmentation. Each trajectory is analyzed and segmented/decomposed into atomic segments. Each atomic segment comprises a straight line segment with two turns at both ends.

The second step is segment matching. The atomic segments and the accompanying sensor information are represented in some space such that clustering can be performed to find dense clusters which correspond to "similar" or "matched" atomic segments of the trajectories.

The third step is trajectory bundling. The various trajectories are "aligned" or "bundled" together based on the matched atomic segments.

The fourth step is trajectory fusing and shaping. The bundled/aligned trajectories are fused and shaped to construct the 2D/3D map.

A "distance" may be defined between two segments. The distance may have several components: a difference between their length, a difference between their two turns at both ends, a difference between their RSSI (matched using DTW), a difference between their MFS (matched using DTW). Some trajectories will not be segmented by atomization, e.g. circles, curved pathway, etc.

In some embodiments, a plurality of trajectories may be obtained, where each trajectory may have different starting points (e.g. one trajectory may start in living room while another may start in bedroom), different starting date/day/time, different paths (e.g. one trajectory may go from living room to dining room while another may go from living room to bedroom), different turns (e.g. , different pace of movement), different stopping/pausing patterns (e.g. the user may stop in living room for 2 hours before going to dining room while the user may pause in living room for a short time, e.g. 1 min, before going to the bedroom).

Each trajectory may be a series of spatial coordinates which together describes a path in/through the venue. The spatial coordinate may be 2-dimensional, or 3-dimensional, etc. The path may be sampled at a first sampling frequency, e.g. 0.01/0.1/1/10/100/1000 sample per second.

Each trajectory may be captured with a respective device. For example, one trajectory may be captured with an iPhone or Android phone carried by a user, while another may be captured with a tablet device (e.g. iPad). Another may be captured using iRobot. Another may be captured using a device installed in a push cart (e.g. janitor’s cart). Another may be captured using wireless signals (e.g. WiFi, 4G/5G/6G/7G/8G, radar signal, etc.) being transmitted from a Type1 device to a Type2 device based on channel information (e.g. CSI, CIR, CFR, RSSI, etc.) derived/captured based on the received wireless signals. The trajectory may be generated using RIM algorithm.

Each trajectory may be accompanied by some sensing data (e.g. RSSI, accelerometer, magnetometer, gyroscope, etc.). Each sensing data (SD) may be sampled at a respective sampling frequency (e.g. RSSI at 100 sample per second, accelerometer at 10 sample per second, magnetometer at 1 sample per second, gyro at 10 sample/sec). The sensing data and the trajectory may share the same time axis (i.e. they are all sampled over time), but their sampling instance (timing) may be different. Thus, time synchronization may be performed to align a trajectory and its accompanying TSSD (e.g. based on time stamps).

Typically in a venue (e.g. a single family house), there may be finite number of possible walkways or path segments. Often a room of "open space" (i.e. empty room, empty hall) may be filled with obstacles (e.g. tables, chairs, tables, desks, shelves, etc.) which restricts the possible walkways or path segments. Thus, typically, same/similar walkways or path segments may appear in different trajectories, at different timing, different order, with different duration, at different paces.

The system may divide each trajectory into a number of segments (segmentation), each with a basically/almost straight path segment and two turns at the two ends of the straight path segment. Then similar segments among the trajectories may be identified/aligned/matched (e.g. based on dynamic time warping applied to accompanying TSSD). A similarity of the trajectories may be analyzed.

Matched segments may need to satisfy some geometric constraint (e.g. length constraint that length difference or length difference percentage must be less than a threshold, or angle constraint that corresponding angle difference must be less than another threshold). For any segment of interest ("current segment"), there may be many candidate segments for matching. A candidate segment may be rejected if it fails to satisfy the geometric constraint. Among the not-rejected candidate segments, the chosen segment (matched segment) for the current segment is the candidate segment with a minimum distance (or matching cost) from the current segment. As the candidate segments and the current segment may have different length/duration with different amount of samples, distance cannot be computed directly. Thus dynamic time warping (DTW) may be applied to align individual samples (of TSSD) such that a normalized distance may be computed (normalized w.r.t. length/amount of samples) between the current segment and each candidate segment.

Then the matched/aligned segments of all the trajectories may be bundled and grouped/joined to form longer segments (e.g. after adjustments/corrections/rotation/translation are applied). Recursively, two adjacent matched segments, or two adjacent "longer joined" segments (e.g. joined segments) each with matched segments, may be combined/grouped/joined to form a longer joined segment, based on some adjustment/correction/rotation/translation so that the matched segments in the two adjacent segments are consistent. The bundling/joining allows individual matched segments to have roughly correct position.

Suppose a segment S1a of a first trajectory T1 is matched to a segment S2a of a second trajectory T2. Suppose further that a segment S1b of T1 is matched to a segment S2b of T2. And S1a and S1b may be neighboring segments in T1 while S2a and S2b may be neighboring segments (e.g. immediately adjacent; or somewhat adjacent, being separated by one or more other segments).

In bundling, S1a and S1b may be combined to form a longer/joined segment, and S2a and S2b may be combined to form a longer/joined segment. Correction/adjustment may be applied to S1a and S1b (e.g. correction to angle in particular) to form a longer, joined segment L1, and also to S2a and S2b to form a longer, joined segment L2, such that L1 is matched to L2. T1 and T2 may be the same trajectory.

Note that although S1a is matched to S2a and S1b is matched to S2b, the unprocessed combined/joined segment of S1a and S1b may NOT match the unprocessed combined/joined segment of S2a and S2b, because the relative angle between S1a and S1b may deviate too much from the relative angle between S2a and S2b. In the bundling process, a turning point between S1a and S1b may be locked/stitched/aligned with a turning point between S2a and S2b. Then S1a and S2a may be adjusted/corrected/rotated/translated/scaled/length adjusted/compensated so that they align. Then segments S1b and S2b may be adjusted/corrected/rotated/translated/scaled/ length adjusted/compensated relative to (or w.r.t) S1a and S2a respectively so that the adjusted S1a and S1b would be consistent w.r.t. to the adjusted S2a and S2b.

Suppose a segment S1a of a long-segment L1a of a first trajectory T1 is matched to a segment S2a of a long-segment L2a of a second trajectory T2. Suppose further that a segment S1b of a long- segment L1b of T1 is matched to a segment S2b of a long-segment L2b of T2. And L1a and L1b may be neighboring long-segments in T1 while L2a and L2b may be neighboring long-segments ("neighboring" may mean immediately adjacent/consecutive, or may mean somewhat adjacent, being separated by one or more other segments) in T2. A long-segment may comprise one or more segments (e.g. consecutive segments, or a few "trains" of consecutive segments).

In bundling, L1a and L1b may be combined to form another long-segment L1c, and L2a and L2b may be combined to form yet another long-segment L2c. Correction/adjustment may be applied to L1a and L1b (e.g. correction to angle in particular) to form L1c, and also to L2a and L2b to form L2c, such that L1c is matched to L2c. T1 and T2 may be the same trajectory.

Although S1a may be matched to S2a, unprocessed L1a may not match unprocessed L2a. Thus some kind of processing of L1a and L2a (e.g. correction or adjustment of angles/lengths) may be needed in order for L1a to match L2a. Similarly, although S1b may be matched to S2b, unprocessed L1b may not match unprocessed L2b. Thus some kind of processing of L1b and L2b (e.g. correction or adjustment of angles/lengths) may be needed in order for L1b to match L2b. Furthermore, unprocessed L1c (combined L1a and L1b) may not match the unprocessed L2c (combined L2a and L2b), because a relative angle between L1a and L1b may deviate too much from a relative angle between L2a and L2b.

In the bundling process, a turning point between L1a and L1b may be locked/stitched/aligned with a turning point between L2a and L2b. Then L1a and L2a may be adjusted/corrected/rotated/length adjusted/compensated so that they align. Then long-segments L1b and L2b may be adjusted/corrected/ rotated/length adjusted/compensated relative to (or w.r.t) long-segments L1a and L2a respectively so that the adjusted L1c would be consistent w.r.t. to the adjusted L2c.

In one example, the long-segments L1a, L1b, L2a and L2b may each be treated as a rigid body such that there may be no "internal" processing/adjustment of segments in each of the four long-segments. But a relative angle between L1a and L1b, and a relative angle between L2a and L2b may be processed/adjusted.

Then matched segments/trajectories may be fused. For a set of matched segments (associated with a path segment), inner-cluster or intra-cluster constraints may be applied to adjust the segment endpoints towards some "cluster centers".

Fine adjustment to individual segments. Consider the coordinate of end points of a group of matched segments. Within the group, there may be zero, one or more than one matched segments from each trajectory. Even though the segments may be matched and somewhat adjusted in the previous bundling step, the segments may not be perfectly aligned (i.e. may not coincide). In particular, instead of coinciding, the endpoints may be close to each other, forming a cluster. Clustering may be applied to find a "center" of the cluster. Then each of the endpoints may be adjusted to move towards/closer to the cluster center. For example, each endpoint may be replaced by a weighted average of the endpoint and the cluster center. When an endpoint of a segment is adjusted, its length is also changed accordingly.

In the fusion of matched segments/trajectories, inter-cluster constraints may be applied to capture/identify/find missed (or mistakenly separated) turns/endpoints. Occasionally some turns that should be matched may be mistakenly separated (i.e. not matched). A turn is a first endpoint of a first segment (e.g. first bundled segment) coinciding with a second endpoint of a second segment (e.g. second bundled segment) adjacent to the first segment. In the bundling stage, the first segment should have been combined/joined with the second segment. But sometimes, they are not combined/joined. To determine if the first endpoint should be matched to the second endpoint, DTW (of TSSD) may be applied to a first section of the first segment near the first endpoint and a second section of the second segment near the second endpoint. For example, the first section may comprise a first percentage (e.g. 50%, 40%, 30%, 20%, 10%, etc.) of the first segment near the first endpoint. Similarly, the second section may comprise a second percentage (e.g. 50%, 40%, 30%, 20%, 10%, etc.) of the second segment near the second endpoint. DTW may be applied to find an optimal matching score (or distance) the first and second sections of one trajectory with the first and second sections of another trajectory. If the optimal matching score is smaller than a threshold, the first endpoint may be matched to the second endpoint.

With updated endpoint locations (e.g. coordinates), each segment may be transformed/adjusted with a transformation (e.g. Helmert transformation, similarity transformation, datum transformation, 2D transformation, 3D transformation, translation, rotation, scaling). Each point of the segment may be transformed/adjusted with the transformation.

Then area shaping may be applied to generate an area/volume based on the trajectories. When segmenting long trajectories, there may be some curved segments without straight path section. Such curved segments may contain useful information about rooms, curved corridors and open space. The position of the curved segments may be deduced from its spatial relationship with straight segments. Then each point on the curved segment may be transformed/adjusted with the transformation (e.g. Helmert transformation). The curved segments may be grouped with straight segments to reconstruct curve corridors, rooms and open spaces. Area shaping may use a procedure comprising at least one of: alpha-shape, alpha complexes/subcomplexes of Delaunay triangulation, convex hull, minimal spanning tree, morphologic operation, or morphological dilation to expand each point on the segments/trajectories to an area/volume.

While a trajectory may be a 1-dimensional line with no width, the map generated by the map generation system may be a 2D area or 3D volume.

The following numbered clauses provide examples for map generation.

Clause H1. A method/device/system/software of a map generation system, comprising: obtaining a plurality of trajectories each with accompanying sensing data in a venue, wherein each trajectory is a time series of spatial coordinates (TSSC) representing a path traversed by a respective object in the venue, wherein each trajectory is accompanied by at least one respective time series of sensing data (TSSD) collected while the respective object traverses the path in the venue; segmenting each TSSC and its accompanying at least one TSSD; computing similarity between pairs of segments based on the accompanying TSSD; bundling the plurality of trajectories based on the computed similarity; fusing the bundled trajectories; computing a shape of the fused trajectories; generating a map of the venue based on the computed shape.

Clause H2. The method/device/system/software of the map generation system of clause H1, comprising: wherein the at least one respective TSSD comprises at least one of: a time series of smart phone sensor data, a time series of portable device sensor data, a time series of accelerometer data, a time series of gyroscope data, a time series of magnetometer data, a time series of Hall sensor data, a time series of global positioning system (GPS) data, a time series of proximity sensor data, a time series of ambient light sensor data, a time series of photometer data, a time series of pedometer data, a time series of barometer data, a time series of thermometer data, a time series of air humidity sensor data, a time series of Geiger counter data, a time series of Soli sensor data, a time series of gravity sensor data, a time series of harmful radiation sensor data, a time series of microphone data, a time series of touchscreen sensor data, a time series of heart rate monitor data, a time series of near field communication (NFC) sensor data, a time series of Bluetooth data, a time series of RFID data, a time series of LiDAR sensor data, a time series of barcode/QR code sensor data, a time series of wireless signal strength, a time series of global positioning data, a time series of magnetic field strength (MFS), or a time series of received signal strength indicator (RSSI).

TSSC and TSSC may be sampled based on the same time axis, and thus may be time synchronized.

Clause H3. The method/device/system/software of the map generation system of clause H1 or H2, comprising: wherein each trajectory is time synchronized with the at least one respective TSSD accompanying the trajectory.

The sampling frequency/period/timing of TSSC and TSSD may be different. The TSSC and/or TSSD may be interpolated so that some or all SD and SC samples may be time aligned.

Clause H4. The method/device/system/software of the map generation system of clauses H1 to H3, comprising: wherein a point of a particular trajectory is associated with a time stamp; computing an interpolated sensing data (SD) at the time stamp to time align with the point based on an interpolation of a particular TSSD accompanying the particular trajectory.

Segmentation may be applied to the TSSC of each trajectory to identify TSSC segments each associated with a straight line segment and two turns. Based on the segmentation of TSSC, the accompanying TSSD may be segmented based on the corresponding time stamp (as TSSC and TSSD may be time synchronized).

Clause H5. The method/device/system/software of the map generation system of any previous H clause, comprising: segmenting the TSSC of each trajectory into a set of TSSC segments, wherein each TSSC segment comprises a straight line segment and two turns at the two ends of the straight line segment; segmenting each TSSD accompanying the trajectory into a set of TSSD segments based on the set of TSSC segments, such that each respective TSSD segment is time synchronized with a respective TSSC segment.

To perform TSSC segmentation, straight line segment may be detected. Or, turns may be detected.

Clause H6. The method/device/system/software of the map generation system of any previous H clause, comprising: detecting at least one of: a straight line segment or a turn in the TSSC of the trajectory; segmenting a TSSC segment of the TSSC based on at least one of: the detected straight line segment, or the detected turn.

Turns may be detected by analyzing/detecting changes of angle/direction provided by TSSD (e.g. accelerometer and/or gyroscope data).

Clause H7. The method/device/system/software of the map generation system of any previous H clause, comprising: detecting the turn in the TSSC based on at least one TSSD accompanying the trajectory.

A turn may be detected if the rate of change of angle/direction exceeds a threshold.

Clause H8. The method/device/system/software of the map generation system of any previous H clause, comprising: computing a rate of a change of an angle of the at least one TSSD; detecting the turn in the TSSC based on the rate of the change of the angle.

Individual segments of the trajectories may be matched (by computing/maximizing similarity measure). To compute similarity measure, dynamic time warping is needed to map samples of two segments of different lengths. The similarity measure may be normalized w.r.t. length, sampling frequency, duration.

Clause H9. The method/device/system/software of the map generation system of any previous H clause, comprising: computing a similarity measure between a first TSSD segment of a first TSSD accompanying a first trajectory and a second TSSD segment of a second TSSD accompanying a second trajectory; matching a first TSSC segment of a first TSSC of the first trajectory with a second TSSC segment of a second TSSC of the second trajectory based on the similarity measure.

Mapping individual sample. Similarity measure may be an aggregation (normalized) of sample-level distance.

Clause H10. The method/device/system/software of the map generation system of any previous H clause, comprising: mapping each sensing data (SD) in the first TSSD segment to at least one SD in the second TSSD segment; computing the similarity measure between the first TSSD segment and the second TSSD segment based on a distance between the SD in the first TSSD segment and each of its mapped SD in the second TSSD segment.

DTW may be used to do the mapping.

Clause H11. The method/device/system/software of the map generation system of any previous H clause, comprising: mapping the SD in the two TSSD segment by performing dynamic time warping (DTW).

Similarity measure may be normalized.

Clause H12. The method/device/system/software of the map generation system of clause H9, comprising: wherein the similarity measure is normalized with respect to at least one of: a length of a TSSD segment, a length of a TSSC segment, a count of SD in the TSSD segment, a count of SC in the TSSC segment, a sampling timing of the TSSD segment, a sampling timing of the TSSC segment, a sampling frequency of the TSSD segment, or a sampling frequency of the TSSC segment.

For each TSSC segment, a set of candidate TSSC segments are identified. Candidate TSSC segment may be rejected if the length difference exceeds a threshold, or if the angle difference exceeds another threshold. Similarity measure may be computed only for non-rejected candidate TSSC segments.

Clause H13. The method/device/system/software of the map generation system of any previous H clause, comprising: wherein each TSSC segment is associated with a length associated with the respective straight line segment and two turning angles associated with the two respective turns; for the first TSSC segment: determining a plurality of candidate TSSC segments, computing a first angle differentiation between a first turning angle of the first TSSC and the first turning angle of each candidate TSSC segment, computing a second angle differentiation between a second turning angle of the first TSSC and the second turning angle of each candidate TSSC segment, rejecting any candidate TSSC segment if the first angle differentiation is larger than a first threshold, or the second angle differentiation is larger than a second threshold, or an aggregate of the first angle differentiation and the second angle differentiation is larger than a third threshold, computing a length differentiation between the length of the first TSSC and the length of each candidate TSSC segment, and rejecting any candidate TSSC segment with a length differentiation larger than a fourth threshold, wherein any differentiation comprises at least one of: a difference, a percentage difference, a ratio, a fraction, a percentage fraction or a combination of the above, wherein the aggregate comprises at least one of: a sum, a linear combination, an average, a weighted average, a geometric mean, or a harmonic mean; wherein the second TSSC segment is a candidate TSSC segment that is not rejected.

Some TSSC segments may be in opposite directions. Thus some additional angle matching is needed to account for the opposite directions.

Clause H14. The method/device/system/software of the map generation system of any previous H clause, comprising: for the first TSSC segment: computing a third angle differentiation between the first turning angle of the first TSSC and the second turning angle of each candidate TSSC segment, computing a fourth angle differentiation between the second turning angle of the first TSSC and the first turning angle of each candidate TSSC segment, and rejecting any candidate TSSC segment if both condition (a) and condition(b) are true, wherein condition (a) is that the first angle differentiation is larger than the first threshold or the second angle differentiation is larger than the second threshold or the aggregate of the first and second angle differentiation is larger than the third threshold, wherein condition (b) is that the third angle differentiation is larger than a fifth threshold or the fourth angle differentiation is larger than a sixth threshold or an aggregate of the third and fourth angle differentiation is larger than a seventh threshold.

Bundling of trajectories may be performed after matching of individual segments of the trajectories.

Clause H15. The method/device/system/software of the map generation system of any previous H clause, comprising: bundling the first trajectory and the second trajectory based on the matched first TSSC segment and the matched second TSSC segment.

Clause H16. The method/device/system/software of the map generation system of any previous H clause, comprising: bundling the first trajectory and the second trajectory based on the respective straight line segment and two turns, of the matched first TSSC segment and the matched second TSSC segment.

In bundling, global geometric property of trajectories may be adjusted. The geometric property may comprise a global (trajectory-to-trajectory) angle of an entire trajectory.

Clause H17. The method/device/system/software of the map generation system of any previous H clause, comprising: bundling the first trajectory and the second trajectory by adjusting a geometric property of the first TSSC and the second TSSC.

Clause H18. The method/device/system/software of the map generation system of clause H17, comprising: wherein the geometric property comprises a length, a scale, an angle, an orientation, a bearing, a relative geometric property, a relative length, a relative scale, or a relative angle.

Global geometric property may be adjusted to increase/maximize some global similarity between trajectories.

Clause H19. The method/device/system/software of the map generation system of clause H17 or H18, comprising: adjusting the geometric property of the first TSSC and the second TSSC to increase a second similarity measure between the first trajectory (i.e. first TSSC) and the second trajectory.

In bundling: Adjust global geometric property may comprise translation, rotation and scaling of trajectories. A point may be locked/aligned/coincided after translation. Then rotation around the point and scaling.

Clause H20. The method/device/system/software of the map generation system of clauses H17 to H19, comprising: adjusting the geometric property of two TSSC by: translating the first TSSC and the second TSSC spatially so that a first point of the matched first TSSC segment of the first trajectory coincides spatially with a second point of the matched second TSSC segment of the second trajectory, rotating and scaling the translated first TSSC around the first point and the translated second TSSC around the second point.

The alignment point may be an end point of the straight line segment of the matched TSSC segment.

Clause H21. The method/device/system/software of the map generation system of clause H20, comprising: wherein the first point comprises one of: a center point, a non-end point or one of the two end points, of the straight line segment of the matched first TSSC segment; wherein the second point comprises one of: a center point, a non-end point or one of the two end points, of the straight line segment of the matched second TSSC segment.

The alignment point may be a turning point between two adjacent matched TSSC segments of a TSSC.

Clause H22. The method/device/system/software of the map generation system of clause H20 or H 21, comprising: wherein the first point is a common end point of the first TSSC segment and a third TSSC segment of the first trajectory; wherein the second point is a common end point of the second TSSC segment and a fourth TSSC segment of the second trajectory; wherein the third TSSC segment is matched to the fourth TSSC segment based on the similarity measure between a third TSSD segment of the first TSSD and a fourth TSSD segment of the second TSSD.

In bundling: Local geometric property between adjacent TSSC segments of a trajectory may be adjusted. Third TSSC segment is adjacent to first TSSC segment in first trajectory. Fourth TSSC segment is adjacent to the second TSSC segment in second trajectory. The second geometric property may comprise a local (segment-to-segment) angle between two adjacent matched segments (TSSC segments).

Clause H23. The method/device/system/software of the map generation system of clause H22, comprising: adjusting a second geometric property between the first TSSC segment and an adjacent third TSSC segment of the first trajectory, and also between of the second TSSC segment and an adjacent fourth TSSC segment of the second trajectory, wherein the third TSSC segment of the first trajectory is matched to the fourth TSSC segment of the second trajectory.

Adjust local geometric property to maximize/increase local similarity measure. Temporary TSSC segments (across two adjacent TSSC segments) may be constructed to capture the local geometric characteristics between the two adjacent TSSC segments. For example, angle between two adjacent straight line segments may be adjusted so that the two adjusted adjacent straight line segments in one trajectory match the two adjusted adjacent straight line segments in another trajectory.

Clause H24. The method/device/system/software of the map generation system of any previous H clause, comprising: computing a third similarity measure between a first temporary TSSC segment of the first TSSC and a second temporary TSSC segment of the second TSSC, wherein the first temporary TSSC segment comprises a sub-segment of the first TSSC segment and an adjoining sub-segment of the third TSSC segment of the first TSSC, wherein the second temporary TSSC segment comprises a sub-segment of the second TSSC segment and an adjoining sub-segment of the fourth TSSC segment of the second TSSC; adjusting the second geometric property to increase the third similarity measure.

Need to align the SC in the temporary TSSC segments to compute the third similarity measure.

Clause H25. The method/device/system/software of the map generation system of clause H24, comprising: mapping each spatial coordinate (SC) in the first temporary TSSC segment to at least one SC in the second temporary TSSC segment; computing the third similarity measure between the first temporary TSSC segment and the second temporary TSSC segment based on a spatial distance between the SC in the first temporary TSSC segment and each of its mapped SC in the second temporary TSSC segment.

Clause H26. The method/device/system/software of the map generation system of clause H25, comprising: mapping the SC in the two temporary TSSC segments by performing dynamic time warping (DTW).

After bundling the trajectories, the bundled trajectories may be fused. Some adjustment are needed. (1) fine adjustment of locations of the matched end-points of the matched segments (move each end point in a cluster of end points towards the cluster center) - (2) identify some not-matched turning points (end points) that should have been matched. Identity non-temporally adjacent matched segments that are spatially adjacent. (Note: temporally adjacent matched segments should be spatially adjacent. But temporally non-adjacent matched segments can also be spatially adjacent when the user re-visit the path or the turning point a second time in the trajectory. This step is to identify them and to mark them as spatially adjacent.)

Clause H27. The method/device/system/software of the map generation system of clauses H17 to H26, comprising: fusing the bundled trajectories by adjusting end points of matched TSSC segments of the bundled trajectories and by identifying spatially adjacent matched segments that are not temporally adjacent.

Fine adjustment of locations of the matched end-points of the matched segments (move each end point in a cluster of end points towards the cluster center).

Clause H28. The method/device/system/software of the map generation system of any previous H clause, comprising: determining a plurality of matched TSSC segments each of a respective trajectory; determining a cluster of matched end points associated the matched TSSC segments; computing a cluster center of the plurality of matched end points; adjusting each matched end points in the cluster towards the cluster center.

Adjust an end point by replacing it by a weighted average of the end point and the cluster center.

Clause H29. The method/device/system/software of the map generation system of clause H28, comprising: replacing each matched end point in the cluster by a weighted average of the matched end point and the cluster center.

Adjust an end point by replacing it by the cluster center.

Clause H30. The method/device/system/software of the map generation system of clause H28 or H29, comprising: replacing each matched end point in the cluster by the cluster center.

The rest of the un-adjusted end-points need to be processed accordingly using Helmert transform.

Clause H31. The method/device/system/software of the map generation system of clause H30, comprising: transforming the non-adjusted end points of the trajectories based on a transformation, wherein the transformation comprises at least one of: Helmert transformation, similarity transformation, datum transformation, 2D transformation, 3D transformation, translation, rotation, or scaling.

Identify some not-matched turning points (end points) that should have been matched. Identity non-temporally adjacent matched segments that are spatially adjacent. (Note: temporally adjacent matched segments should be spatially adjacent. But temporally non-adjacent matched segments can also be spatially adjacent when the user re-visit the path or the turning point a second time in the trajectory. This step is to identify them and to mark them as spatially adjacent.) - Temporally non-adjacent segments may be spatially connected based on TSSD matching.

Clause H32. The method/device/system/software of the map generation system of clauses H27 to H31, comprising: wherein a fifth TSSC segment of the first TSSC and a sixth TSSC segment of the second TSSC are matched; wherein either the fifth TSSC segment is not temporally adjacent to the first TSSC segment in the first TSSC, or the sixth TSSC segment is not temporally adjacent to the second TSSC segment in the second TSSC; constructing a first temporary TSSD segment based on a concatenation of a sub-segment of the first TSSD segment and a sub-segment of a fifth TSSD segment of the first TSSD, wherein the fifth TSSD segment is associated with the fifth TSSC segment; constructing a second temporary TSSD segment based on a concatenation of a sub-segment of the second TSSD segment and a sub-segment of a sixth TSSD segment of the second TSSD, wherein the sixth TSSD segment is associated with the second TSSD segment; computing the similarity measure between the first temporary TSSD segment and the second temporary TSSD segment; computing a spatially adjacent relationship between the first TSSC segment and the temporally non-adjacent fifth TSSC segment in the map; and computing a spatially adjacent relationship between the second TSSC segment and the temporally non-adjacent sixth TSSC segment in the map.

If two temporally non-adjacent segments are spatially adjacent, they should be connected in the map.

Clause H33. The method/device/system/software of the map generation system of clause H31 or H32, comprising: spatially connecting the first TSSC segment and the temporally non-adjacent fifth TSSC segment in the map based on their spatially adjacent relationship; and spatially connecting the second TSSC segment and the temporally non-adjacent sixth TSSC segment in the map based on their spatially adjacent relationship.

Similarity measure may be computed based on DTW.

Clause H34. The method/device/system/software of the map generation system of clauses H31 to H33, comprising: computing the similarity measure between the first temporary TSSD segment and the second temporary TSSD segment based on dynamic time warping (DTW).

Compute shape by expanding each SC on the fused trajectories to give it a width/length, an area or a volume.

Clause H35. The method/device/system/software of the map generation system of clauses H27 to H34, comprising: computing the shape of the fused trajectories by expanding each point of the fused trajectories to an area or a volume.

Compute shape based on alpha-shaping.

Clause H36. The method/device/system/software of the map generation system of any previous H clause, comprising: computing the shape of the fused trajectories based on a shaping, wherein the shaping comprises at least one of: alpha shape, alpha complexes or sub-complexes of Delaunay triangulation, convex hull, minimal spanning tree, morphological operation or morphological dilation.

Include curved segments (i.e. not straight line segments).

Clause H37. The method/device/system/software of the map generation system of clauses H27 to H36, comprising: wherein the fused trajectories comprise curved segments of the trajectories that are not associated with straight line segments.

Curved segments need to be adjusted geometrically.

Clause H38. The method/device/system/software of the map generation system of any previous H clause, comprising: adjusting a geometric property of the curved segments based on their spatial relationship with the TSSC segments associated with straight line segments, wherein the geometric property comprises at least one of: a position, an angle and a size.

Curved segments need to be adjusted geometrically based on translation, rotation and scaling.

Clause H39. The method/device/system/software of the map generation system of any previous H clause, comprising: adjusting a position of a curved segment based on translation, adjusting an angle of the curved segment based on rotation, and adjusting a size of the curved segment based on scaling.

In some embodiments, a system and method are disclosed for non-human recognition, to differentiate motion due to human versus motion due to non-human (e.g. pet, fan, electric appliances, mechanical vibration, or iRobot.) The method may include the following steps.

At step 1, the system may obtain TSCI from wireless signal transmitted by Type1 device and received by Type2 device. Motion may be due to an object (e.g. human, non-human, pet or robot vacuum cleaner).

At step 2, the system may compute an auto-correlation function (ACF) based on the TSCI (e.g. based on a first feature such as magnitude or phase of each CI), and identify a second feature of ACF (e.g. peaks or local max, valleys or local min).

At step 3, the system may compute a number of ACF-based statistics, including ACF peaks mean, ACF valleys mean, ACF peaks interval distance, and ACF valleys interval distance.

The ACF peaks/valleys mean may be computed by computing a number of local max (for ACF peaks mean) or local min (for ACF valleys mean) of ACF for each time instance, computing a "first mean" (e.g. weighted averaging, arithmetic mean, geometric mean, harmonic mean, percentile, median, max, min, mode) of the number of local max/min for the time instance, and then computing a "second mean" (e.g. a "first mean", or filtering, or lowpass filtering) of all the first mean of the time instances in a time window. Alternatively, the ACF peaks/valleys mean may be computed by computing a global max (for ACF peaks mean) or a global min (for ACF valleys mean) of ACF for each time instance, and then computing the "second mean" of all the global max/min of time instances in the time window. This is a special case of (a) in which the "first mean" is max (for ACF peaks mean) or min (for ACF valleys mean).

The ACF peaks/valleys interval distance may be computed by computing the number of local max (for ACF peaks interval distance) or local min (for ACF valleys interval distance) of the ACF for each time instance, computing a number of intervals each being a temporal distance or temporal interval between a pair of neighboring/adjacent local max/min of the ACF for the time instance, computing the "first mean" of the number of intervals for the time instance, and computing the "second mean" (such as weighted averaging, or filtering, or lowpass filtering) of all the first mean of time instances in the time window.

At step 4, the system may compute a motion statistics (MS) based on the ACF (e.g. ACF at a fixed time delay, T1) for each time instance. For example, for T1=1, the MS may be TRRS, inner-product or based on the TSCI. Or, T1=k for TRRS based on TSCI down-sampled at a factor of k. The system can compute some MS-based statistics such as MS mean, and MS variance in a time window.

The motion statistics mean may be computed by computing the "second mean" (such as the first mean, filtering, or lowpass filtering) of all the motion statistics of the time instance in the time window. The motion statistics variance may be computed by computing a "variation measure" (such as variance, standard deviation, variation, derivative, slope, total variation, absolute variation, square variation, spread, dispersion, variability, deviation, absolute deviation, square deviation, total deviation, divergence, range, interquartile range, skewness, kurtosis, L-moment, coefficient of variation, quartile coefficient of dispersion, mean absolute difference, Gini coefficient, relative mean difference, median absolute deviation, average absolute deviation, distance standard deviation, coefficient of dispersion, entropy, variance-to-mean ratio, maximum-to-minimum ratio, variation measure, regularity measure, similarity measure, likelihood, probability distribution function, sample distribution, moment generating function, expected value, expected function) of all the motion statistics of the time instance in the time window.

At step 5, the system may compute a speed of the motion of the object based on ACF of the CSI for a number of time instance. The system may obtain a time series of speed (TSSP). The sampling frequency of TSSP may be lower than that of TSCI. The system can then compute speed-based statistics including a number of gait statistics (e.g. presence of gait, stride cycle time, step cycle time, N-cycle time, stride cycle distance, step cycle distance, N-cycle distance based on the TSSP), and a number of simple speed statistics (e.g. speed mean, speed variation measure, speed percentile, speed histogram).

At step 6, the system may identify peaks (local max), valleys (local min) and/or zero-crossings of the TSSP, and process the peaks, valleys and zero-crossings by removing "false peak", "false valley" and/or "false zero-crossing". False peaks, and false valleys may be removed in pairs (i.e. one false peak may be removed with one adjacent/neighboring false valley together). False zero-crossings may be removed in pairs (i.e. two immediately adjacent zero-crossings may be removed together). The zero-crossings may be computed as zero-crossings of mean-subtracted TSSP.

At step 7, the system may compute a presence of gait behavior of the motion of the object based on the peaks, valleys and/or zero-crossings of the TSSP (or simply the TSSP). For a valid presence of gait behavior, a count of the peaks, valleys or zero-crossings may be upper bounded (by a first threshold) and/or lowered bounded (by a second threshold).

At step 8, if gait behavior is detected (i.e. if gait is present), the system may compute gait statistics (e.g. stride cycle time, step cycle time, stride length, step length). This may include the following sub-steps.

At sub-step 8a, the system can compute a number of "intervals", each being a temporal distance or temporal interval between a pair of neighboring/adjacent peaks or valley or zero-crossing.

At sub-step 8b, the system can compute a number of "interval distance" based on the TSSP corresponding to the number of "intervals", each "interval distance" being the distance traversed by the object in a corresponding interval. An interval distance may be computed as a product of the corresponding interval and a "representative speed" (e.g. "first mean" or "second mean" of speed) in the interval. Alternatively, the interval may be subdivided into more than one disjoint sub-intervals. A subinterval distance may be computed as a product of a sub-interval and a "representative speed" in the sub-interval. The interval distance may be computed as the "first mean" or "second mean" of the more than one corresponding subinterval distance.

At sub-step 8c, the system can compute the stride cycle time as a first "second mean" of the intervals between pairs of immediately adjacent peaks, or a second "second mean" of the intervals between pairs of immediately adjacent valleys, or a third "second mean" of the intervals between pairs of every other zero-crossing (i.e. between first and third zero-crossing, or between k^{th} and (k+2)^{th} zero-crossing) or a weighted average of the first "second mean", the second "second mean" and/or the third "second mean".

At sub-step 8d, the system can compute the step cycle time as half of the stride cycle time. Alternatively, the step cycle time may be computed based on a fourth "second mean" of the intervals between pairs of immediately adjacent zero-crossing (i.e. between first and second zero-crossing, or between k^{th} and (k+1)^{th} zero-crossing).

At sub-step 8e, an N-cycle time may be computed as the interval spanned by N peaks, or N valleys, or 2N zero-crossings.

At sub-step 8f, the system can compute the step cycle distance, stride cycle distance and/or N-cycle distance based on the interval distance. The step cycle distance, stride cycle distance or N-cycle distance may be defined as the distance traversed by the object motion in a step cycle time, a stride cycle time or a N-cycle time. The step cycle distance, stride cycle distance or N-cycle distance may be computed based on a summation of interval distance associated with all intervals in the corresponding step cycle time, stride cycle time or N-cycle time. The step cycle distance, stride cycle distance or N-cycle distance may be averaged over a time period using the first mean or second mean.

At step 9, the system can compute some simple speed statistics (e.g. second mean of speed as defined above, "variation measure" of speed as defined above, speed 25 percentile, speed 50 percentile/median speed, speed 75 percentile) over a time period. A histogram of the speed in the time period may be computed. Any speed percentile or variation measure may be computed based on the histogram.

At step 10, the system can perform a monitoring task based on the ACF-based statistics, the MS-based statistics, the speed-based statistics (including both gait statistics and the simple speed statistics). The monitoring task comprises at least one of: classification/detection/ recognition/identification/presence/ absence/appearance/disappearance/ADL (activity daily living) of at least two of: human, human adult, older human, human children, human baby, non-human, non-human pet, non-human robot, non-human machinery. The task may comprise gesture recognition, tracking, localization, locationing, navigation. The task may be performed based on machine learning, classifier, supervised learning, unsupervised learning, semi-supervised learning, clustering, feature extraction, featuring training, principal component analysis, eigen-decomposition, frequency decomposition, time decomposition, time-frequency decomposition, training, discriminative training, supervised training, unsupervised training, semi-supervised training, neural network, deep learning.

The following numbered clauses provide examples for map generation.

Clause I1. A system for map generation, comprising: a sensor configured to collect sensing data in a venue and obtain a plurality of trajectories, wherein: each trajectory is a time series of spatial coordinates (TSSC) representing a path traversed by a respective object in the venue, each TSSC is accompanied by at least one respective time series of sensing data (TSSD) collected while the respective object traverses the path in the venue; and a processor configured for: segmenting each TSSC and its accompanying at least one TSSD into segments, bundling the plurality of trajectories based on similarity measures between pairs of the segments, fusing the bundled trajectories to generate fused trajectories, computing a shape of the fused trajectories, and generating a map of the venue based on the computed shape

Clause 12. The system of Clause I1, wherein: each trajectory is time synchronized with the at least one respective TSSD accompanying the trajectory; the at least one respective TSSD comprises at least one of: a time series of smart phone sensor data, a time series of portable device sensor data, a time series of accelerometer data, a time series of gyroscope data, a time series of magnetometer data, a time series of wireless signal strength, a time series of global positioning data, a time series of magnetic field strength (MFS), or a time series of received signal strength indicator (RSSI)

Clause 13. The system of Clause I1 or I2, wherein the processor is further configured for: segmenting the TSSC of each trajectory into a set of TSSC segments, wherein each TSSC segment comprises a straight line segment and two turns at two ends of the straight line segment; and segmenting each TSSD accompanying the trajectory into a set of TSSD segments based on the set of TSSC segments, such that each TSSD segment is time synchronized with a respective TSSC segment

Clause I4. The system of Clauses I1 to 13, wherein segmenting the TSSC of each trajectory comprises: detecting a straight line segment and a turn in the TSSC of the trajectory, wherein the turn in the TSSC is detected based on a rate of a change of an angle of the at least one TSSD accompanying the trajectory; and segmenting the TSSC to generate a TSSC segment of the TSSC based on at least one of: the detected straight line segment, or the detected turn

Clause 15. The system of any previous I Clause, wherein the processor is further configured for: computing a similarity measure between a first TSSD segment of a first TSSD accompanying a first trajectory and a second TSSD segment of a second TSSD accompanying a second trajectory; and matching a first TSSC segment of a first TSSC of the first trajectory with a second TSSC segment of a second TSSC of the second trajectory to generate a matched first TSSC segment and a matched second TSSC segment based on the similarity measure

Clause I6. The system of any previous I Clause, wherein the processor is further configured for: mapping each sensing data (SD) in the first TSSD segment to at least one SD in the second TSSD segment based on dynamic time warping (DTW); and computing the similarity measure between the first TSSD segment and the second TSSD segment based on a distance between each SD in the first TSSD segment and each of its mapped SD in the second TSSD segment

Clause 17. The system of any previous I Clause, wherein: the similarity measure is normalized with respect to at least one of: a length of a TSSD segment, a length of a TSSC segment, a count of SD in the TSSD segment, a count of SC in the TSSC segment, a sampling timing of the TSSD segment, a sampling timing of the TSSC segment, a sampling frequency of the TSSD segment, or a sampling frequency of the TSSC segment; and each TSSC segment is associated with a length associated with the respective straight line segment and two turning angles associated with the two respective turns

Clause 18. The system of any previous I Clause, wherein, for the first TSSC segment, the processor is further configured for: determining a plurality of candidate TSSC segments; computing a first angle differentiation between a first turning angle of the first TSSC and a first turning angle of each candidate TSSC segment; computing a second angle differentiation between a second turning angle of the first TSSC and a second turning angle of each candidate TSSC segment; rejecting any candidate TSSC segment when: the first angle differentiation is larger than a first threshold, or the second angle differentiation is larger than a second threshold, or an aggregate of the first angle differentiation and the second angle differentiation is larger than a third threshold; computing a length differentiation between a length of the first TSSC and a length of each candidate TSSC segment; and rejecting any candidate TSSC segment with a length differentiation larger than a fourth threshold, wherein the second TSSC segment is a candidate TSSC segment that is not rejected

Clause I9. The system of any previous I Clause, wherein, for the first TSSC segment, the processor is further configured for: computing a third angle differentiation between the first turning angle of the first TSSC and the second turning angle of each candidate TSSC segment, computing a fourth angle differentiation between the second turning angle of the first TSSC and the first turning angle of each candidate TSSC segment, and rejecting any candidate TSSC segment when: both (a) the first angle differentiation is larger than the first threshold or the second angle differentiation is larger than the second threshold or the aggregate of the first and second angle differentiations is larger than the third threshold, and (b) the third angle differentiation is larger than a fifth threshold or the fourth angle differentiation is larger than a sixth threshold or an aggregate of the third and fourth angle differentiations is larger than a seventh threshold

Clause 110. The system of any previous I Clause, wherein the processor is further configured for: bundling the first trajectory and the second trajectory based on the respective straight line segment and the respective two turns of the matched first TSSC segment and the matched second TSSC segment, wherein the first trajectory and the second trajectory are bundling by adjusting a geometric property of the first TSSC and the second TSSC, to increase a second similarity measure between the first trajectory and the second trajectory

Clause 111. The system of any previous I Clause, wherein adjusting the geometric property of the first TSSC and the second TSSC comprises: translating the first TSSC and the second TSSC spatially such that a first point of the matched first TSSC segment of the first trajectory coincides spatially with a second point of the matched second TSSC segment of the second trajectory; rotating and scaling the translated first TSSC around the first point; and rotating and scaling the translated second TSSC around the second point

Clause 112. The system of any previous I Clause, wherein: the first point is a common end point of the first TSSC segment and a third TSSC segment of the first trajectory; the second point is a common end point of the second TSSC segment and a fourth TSSC segment of the second trajectory; and the third TSSC segment is matched to the fourth TSSC segment based on a similarity measure between a third TSSD segment of the first TSSD and a fourth TSSD segment of the second TSSD

Clause 113. The system of Clause 112, wherein the processor is further configured for: adjusting a first geometric property between the first TSSC segment and the third TSSC segment of the first trajectory; and adjusting a second geometric property between the second TSSC segment and the fourth TSSC segment of the second trajectory

Clause 114. The system of any previous I Clause, wherein the processor is further configured for: computing a third similarity measure between a first temporary TSSC segment of the first TSSC and a second temporary TSSC segment of the second TSSC, wherein the first temporary TSSC segment comprises a sub-segment of the first TSSC segment and an adjoining sub-segment of the third TSSC segment of the first TSSC, the second temporary TSSC segment comprises a sub-segment of the second TSSC segment and an adjoining sub-segment of the fourth TSSC segment of the second TSSC, and the first geometric property and the second geometric property are adjusted to increase the third similarity measure

Clause 115. The system of any previous I Clause, wherein computing the third similarity measure comprises: mapping each spatial coordinate (SC) in the first temporary TSSC segment to at least one SC in the second temporary TSSC segment based on dynamic time warping (DTW); and computing the third similarity measure between the first temporary TSSC segment and the second temporary TSSC segment based on a spatial distance between each SC in the first temporary TSSC segment and each of its mapped SC in the second temporary TSSC segment

Clause 116. The system of any previous I Clause, wherein the processor is further configured for fusing the bundled trajectories based on: adjusting end points of matched TSSC segments of the bundled trajectories; identifying spatially adjacent matched segments that are not temporally adjacent

Clause 117. The system of any previous I Clause, wherein adjusting the end points comprises: determining a plurality of matched TSSC segments each from a respective trajectory; determining a cluster of matched end points associated the plurality of matched TSSC segments; computing a cluster center of the cluster of matched end points; and adjusting each matched end point in the cluster towards the cluster center

Clause 118. The system of any previous I Clause, wherein adjusting the end points further comprises at least one of: replacing each matched end point in the cluster by a weighted average of the matched end point and the cluster center; or replacing each matched end point in the cluster by the cluster center

Clause 119. The system of any previous I Clause, wherein: a fifth TSSC segment of the first TSSC and a sixth TSSC segment of the second TSSC are matched; and either the fifth TSSC segment is not temporally adjacent to the first TSSC segment in the first TSSC, or the sixth TSSC segment is not temporally adjacent to the second TSSC segment in the second TSSC

Clause 120. The system of any previous I Clause, wherein the processor is further configured for: constructing a first temporary TSSD segment based on a concatenation of a sub-segment of the first TSSD segment and a sub-segment of a fifth TSSD segment of the first TSSD, wherein the fifth TSSD segment is associated with the fifth TSSC segment; constructing a second temporary TSSD segment based on a concatenation of a sub-segment of the second TSSD segment and a sub-segment of a sixth TSSD segment of the second TSSD, wherein the sixth TSSD segment is associated with the sixth TSSC segment; computing a similarity measure between the first temporary TSSD segment and the second temporary TSSD segment; computing a first adjacent relationship between the first TSSC segment and the temporally non-adjacent fifth TSSC segment in the map; and computing a second adjacent relationship between the second TSSC segment and the temporally non-adjacent sixth TSSC segment in the map

Clause 121. The system of any previous I Clause, wherein the processor is further configured for: spatially connecting the first TSSC segment and the temporally non-adjacent fifth TSSC segment in the map based on the first spatially adjacent relationship; and spatially connecting the second TSSC segment and the temporally non-adjacent sixth TSSC segment in the map based on the second spatially adjacent relationship

Clause 122. The system of any previous I Clause, wherein the processor is further configured for: computing the shape of the fused trajectories by expanding each point of the fused trajectories to an area or a volume, wherein the fused trajectories comprise curved segments of the plurality of trajectories that are not associated with straight line segments; and adjusting a geometric property of the curved segments based on their spatial relationships with the TSSC segments associated with straight line segments

Clause 123. The system of any previous I Clause, wherein adjusting a geometric property of a curved segment comprises at least one of: adjusting a position of the curved segment based on translation; adjusting an angle of the curved segment based on rotation; and adjusting a size of the curved segment based on scaling

Clause 124. An apparatus for map generation, comprising: a sensor configured to collect sensing data in a venue and obtain a plurality of trajectories, wherein: each trajectory is a time series of spatial coordinates (TSSC) representing a path traversed by a respective object in the venue, each TSSC is accompanied by at least one respective time series of sensing data (TSSD) collected while the respective object traverses the path in the venue; and a processor communicatively coupled to the sensor and configured for: segmenting each TSSC and its accompanying at least one TSSD into segments, bundling the plurality of trajectories based on similarity measures between pairs of the segments, fusing the bundled trajectories to generate fused trajectories, computing a shape of the fused trajectories, and generating a map of the venue based on the computed shape

Clause 125. A method for map generation, comprising: obtaining sensing data and a plurality of trajectories in a venue, wherein: each trajectory is a time series of spatial coordinates (TSSC) representing a path traversed by a respective object in the venue, each TSSC is accompanied by at least one respective time series of sensing data (TSSD) collected while the respective object traverses the path in the venue; segmenting each TSSC and its accompanying at least one TSSD into segments; bundling the plurality of trajectories based on similarity measures between pairs of the segments; fusing the bundled trajectories to generate fused trajectories; computing a shape of the fused trajectories; and generating a map of the venue based on the computed shape.

In some embodiments, a system is disclosed to perform wireless vital sign monitoring, e.g. with ACF enhancement. The system can use a time series of channel information (TSCI) obtained from a wireless signal transmitted from a transmitter to a receiver to detect a vital sign (e.g. breathing or heartbeat or a periodic motion) of an object (e.g. a person, a user, a baby) in a venue based on the TSCI.

The channel information (CI) may be channel impulse response (CIR), channel frequency response (CFR), and/or channel state information (CSI), RSSI, etc., obtained from the received wireless signal. The wireless signal may be an mmWave/UWB/radar signal. The transmitter and the receiver may be co-located (e.g. on the same device, or on the same circuit board such as in the case of radar), or at different locations.

In some embodiments, a vital sign is detected based on a 2 dimensional (2D) decomposition of the TSCI which is enhanced using image processing filtering. To perform construction of 2D decomposition, for each CI, a CI-feature vector is constructed. The CI-feature vector may be constructed by a concatenation of a first basic feature of the CI and a second basic feature of a corresponding denoised CI. The denoised CI may be obtained using some simple denoising, e.g. based on phase(CI_1 - CI_2), where 1 and 2 here refer to two different spatial streams. Any basic feature of a CI may comprise the CI itself, a magnitude of the CI, a phase of the CI, a magnitude of a component of CI, a phase of a component of CI, or a function of any magnitude/phase. The second basic feature of a corresponding denoised CI may comprise a difference between the phases of two temporally adjacent CI.

In some embodiments, the 2D decomposition is performed in a sliding time window of TSCI. The sliding time window is associated with a time stamp or time index. Let the time index of the i^{th} sliding window be i, which is referred to as "real time". A sliding window and the next sliding window may have an overlap (e.g. 10% overlap, 50% overlap, 90% overlap) corresponding to a time difference between two immediately adjacent sliding window time indices (i.e. time difference between time index k and k+1). The size of the sliding time window may be adjusted over time. The size may be smaller at the beginning of the sensing task and may increase over time.

In some embodiments, in each sliding window, a characteristic function is computed based on a transformation. The characteristic function may be a time function (e.g. the transformation may be autocorrelation function (ACF), or autocovariance function, etc.) of the CI-feature in the time window. Let the time index of the ACF (i.e. time shift) be j. The vital sign signal is a periodic signal. In the i^{th} sliding time window, the characteristic (time) function would have a peak (local maximum) at a certain time-shift which corresponds to a period (or frequency) of the periodic vital sign signal. It may also have additional peaks at time-shift being integer multiples of the period.

In some embodiments, a 2D matrix A of size MxN is formed by putting the time function together according to the time index of the sliding time windows. Let a(i,j) be the element of A at the column i and row j, i=1, .., M, j=1,.., N . The i^{th} column of A (i.e. a(i,*)) represents the sliding time function in the i^{th} sliding time window. The index j of a(i,j) is the time-shift of the time function in the i^{th} sliding time window, and a(i,j) is the value of the time function (e.g. ACF) at that time-shift. The 2D matrix A can be treated as an image, with a(I,j) being the image intensity at the (i,j)^{th} pixel.

In some embodiments, as the frequency/period of the vital sign changes very slowly (i.e. almost unchanged within a short time change), the peak (local max) should form a horizontal, or near horizontal line in the image. But without the enhancement described below, the horizontal line(s) may be hardly visible or detectable in real world situations due to noise. With the enhancement, the horizontal line may become more visible or detectable.

In some embodiments, the characteristic function may be a frequency function (e.g. spectrum/power spectral density/short-time Fourier transform/wavelet transform/trigonometric transform/sinusoidal transform/frequency decomposition/ filter-bank) of the CI-feature in the sliding time window. Let the frequency index of the sliding frequency function be j. The vital sign signal is a periodic signal. In the i^{th} sliding time window, the sliding function would have a peak (local maximum) at a certain frequency which corresponds to the fundamental frequency (or period) of the periodic vital sign. There may be additional peaks at the harmonics, which are integer multiples of the fundamental frequency.

In some embodiments, a 2D matrix B of size MxN is formed by putting the frequency function together according to the time index of the sliding time windows. Let b(i,j) be the element of B at the column i and row j, i=1, .., M, j=1,.., N. The i^{th} column of B (i.e. b(i,*)) represents the sliding frequency function in the i^{th} sliding time window. The index j ofb(i,j) is the frequency index of the frequency function in the i^{th} sliding time window, and b(i,j) is the value at that frequency index. Similar to 2D matrix A, the 2D matrix B can be treated as an image, with b(i,j) being the image intensity at the (i,j)^{th} pixel. As the frequency/period of the vital sign changes very slowly (i.e. almost unchanged within a short time change), the peak (local max) should form one or more horizontal, or near horizontal line in the image. But without the enhancement described below, the horizontal line(s) may be hardly visible or detectable in real world situations due to noise. With the enhancement, the horizontal line may become more visible or detectable.

The vital sign may be detected by detecting the horizontal line(s) in the 2D matrix A or B. But due to noise, often the peak(s) and/or the horizontal line(s) may not be obvious. Thus, the system may enhance the 2D decomposition, the peak(s) in particular, by applying image processing filters.

In some embodiments, to perform enhancement of 2D Decomposition, the image processing filter may comprise vertical highpass filter and horizontal lowpass filter. The horizontal lowpass filter may be used to take advantage of the horizontal line structure in the 2D decomposition. The vertical highpass filtering and horizontal lowpass filtering may be performed jointly, or separately. The separate vertical highpass filter, the separate horizontal lowpass filter, and/or the joint vertical highpass horizontal lowpass filter may be linear (e.g. FIR filter) or nonlinear (e.g. median filter, percentile, sorting). Multiple filtering orders are possible, e.g. vertical highpass first, followed by horizontal lowpass; or horizontal lowpass first, then followed by vertical highpass. There may be a succession of filtering, e.g. vertical highpass first, then horizontal lowpass, then vertical highpass; or horizontal lowpass first, then another horizontal lowpass, then vertical highpass.

For the vertical filtering, filtering may be applied to each column, or groups of columns, such as 3 columns or 5 columns or k columns. For one-column filtering (i.e. filtering applied to each column), the system may consider each column of the 2D matrix (A or B) as a time signal x(t) and apply 1-dimensional highpass filter (e.g. [-1 1], or [1 -1], or [-1 2 -1], or [-2 4 -2], or [-1 3 -3 1], or [-1 0 2 0 -1], or [-1 4 -6 4 -1], etc., or a succession of more than one highpass filters each scaled by respective scaling factor) to x(t) to obtain a filtered signal y(t). The filtered signal y(t) may be added back to the time signal to obtain the enhanced column (i.e. x_enhanced(t) = x(t)+y(t)). In one embodiment, the same vertical filtering may be applied to all the columns. In another embodiment, different vertical filter may be applied to different columns. Such vertical filter may be chosen/decided adaptively.

In some embodiments, for 3-column (or k-column in general) vertical filtering, let x(t) be the center column (which is in the center among the 3 columns). The system may apply a 2D high pass filter to the 3 columns (or k columns) to obtain a filtered center column y(t). For example, the system may apply [0 -1 0; 0 2 0; 0 -1 0] which is [-1 2 -1] applied vertically, [-1 0 0; 0 2 0; 0 0 -1] which is [-1 2 -1] applied in diagonal direction, or [0 0 -1; 0 2 0; -1 0 0] which is [-1 2 -1] applied in anti-diagonal direction, or a succession of more than one highpass filter each scaled by respective scaling factor. The diagonal direction is 45 degree to the vertical line. Other angles such as 30 degree, 20 degree, 10 degree are possible. The filtered center column y(t) may be added back to the center column x(t) to obtain the enhanced center column (i.e. x_enhanced(t) = x(t)+y(t), or alternatively x_enhanced(t) = x(t)+alpha*y(t), where alpha is a scaling factor). In one embodiment, the same k-column vertical filtering may be applied to all the columns. In another embodiment, different k-column vertical filtering's may be applied to different columns. Such k-column vertical filter may be chosen/decided adaptively. For example, one column may be processed by 3-column vertical filtering while another column may be processed by 5-column vertical filtering.

In some embodiments, for the horizontal filtering, filtering may be applied to each row, or groups of rows, such as 3 rows or 5 rows or k rows. For one-row filtering (i.e. filtering applied to each row), the system may consider each row of the 2D matrix A or B as a time signal x(t) and apply 1-dimensional lowpass filter (e.g. [1 1]/2, [1 1 1]/3, [1 2 1]/4, [13 3 1]/8, [14 6 4 1]/16, [1 1 1 1 1]/5, 3-point median filter, 5-point median filter, 3-point percentile filter (X %, e.g. 50% for median filter), 3-point mode filter, etc., or a succession of more than one lowpass filters) to x(t) to obtain a filtered signal y(t). The filtered signal y(t) may be added back to the time signal to obtain the enhanced row (i.e. x_enhanced(t) = x(t)+y(t)). In one embodiment, the same horizontal filtering may be applied to all the rows. In another embodiment, different horizontal filters may be applied to different rows. Such horizontal filter may be chosen/decided adaptively.

In some embodiments, for 3-row (or k-row in general) horizontal filtering, let x(t) be the center row (which is in the center among the 3 rows). The system may apply a 2D lowpass filter to the 3 rows (or k rows) to obtain the enhanced center row y(t). For example, the system can apply [0 0 0; 1 2 1; 0 0 0]/4 which is [1 2 1] applied horizontally, [1 0 0; 0 2 0; 0 0 1]/4 which is [1 2 1] applied in diagonal direction, or [0 0 1; 0 2 0; 1 0 0] which is [1 2 1] applied in anti-diagonal direction, or a succession of more than one lowpass filters. The diagonal direction is 45 degree to the horizontal line. Other angles such as 30 degree, 20 degree, 10 degree are possible. In one embodiment, the same k-row horizontal filtering may be applied to all the rows. In another embodiment, different k-row horizontal filters may be applied to different rows. Such k-row horizontal filter may be chosen/decided adaptively. For example, one row may be processed by 3-row horizontal filtering while another row may be processed by 5-row horizontal filtering.

After the vertical highpass and horizontal lowpass filtering, histogram equalization or other histogram operation (e.g., histogram stretching, or contrast stretching) may be applied to a number of consecutive columns (which correspond to a number of consecutive sliding time windows).

Then vital signal detection can be performed based on then enhancement of 2D decomposition. For each time index (and associated sliding time window and corresponding column of the 2D decomposition), the system may make a tentative (not final) decision of whether the vital sign is present, or not. A final decision is made - that the vital sign is present, if vital sign is tentative. Whether vital sign is present or not, there would always be peaks in the enhanced 2D decomposition. So the system can do the following tests to make the tentative decision.

For Test 1, the system can consider a particular time index associated with a particular characteristic function (e.g. time function such as ACF, frequency function such as STFT) which is a particular column of the 2D decomposition. The system can test for presence of vital sign at the particular time by performing dynamic time warping (DTW), e.g. with respect to a normalized distance measure, between the particular characteristic function (e.g. ACF with an unknown vital sign frequency) and a reference characteristic function (e.g. trained ACF with a fixed training vital sign frequency). The DTW may be applied to account for possible mismatch of vital sign frequency between the two characteristic functions. The reference vital sign may be computed in a training phase, based on clustering a number of training characteristic function matched based on DTW, the number of training characteristic function obtained when training subject(s) with vital sign was present. The reference vital sign may also be from empirical vital sign data from adults, child, etc., without training. If the normalized distance measure is larger than a first threshold, the tentative decision is that vital sign is not present (i.e. vital sign not detected). If not, the system can perform Test 2.

For Test 2, let x(t) be the enhanced characteristic function. The system can compute x(t) - b for some value of b (e.g. 0.6, 0.5, 0.4, 0, etc.) and compute a number of zero crossing of x(t) - b (i.e. compute/count how many times x(t) cross the value of b). If the number of zero crossing is greater than a second threshold (probably due to noise), the tentative decision is that vital sign is not present (i.e. vital sign not detected). If not, the system can perform Test 3.

For Test 3, the system can find the peaks (local maximum) of the enhanced characteristic function. For example, the system can compute an inter-peak distance (e.g. adjacent-peak distance such that distance between the first and second peaks, or between second and third peaks, and so on, or a weighted average of the adjacent-peak distances). If the inter-peak distance is in an acceptable range (associated with vital sign, e.g. breathing, or heartbeat), the tentative decision is that vital sign is present (i.e. vital sign detected) and the inter-peak distance is the vital sign period (if the characteristic function is a time function) or vital sign frequency (if characteristic function is a frequency function). If not, vital sign is determined to be not present (i.e. vital sign not detected).

In some embodiments, for the final decision/conclusion at any time, the vital sign is concluded to be present (i.e. vital sign is declared to be detected, as the final decision) if A is larger than a third threshold (e.g. 0.9, 0.5, 0.1). A is the percentage of vital sign being tentatively detected in a certain period of time (e.g. 1 hour, 30 min, 15 min, 5 minute, 3 min, 1 min).

One application of the wireless vital sign monitoring is a wireless based in-car human presence detection system 7300, as illustrated in FIG. 73A and FIG. 73B. The system 7300 includes an access point (AP) device 7310 and a station (STA) device 7320 coupled to a car. The STA device 7320 may serve as a transmitter (or a Bot, or a Type 1 device) to transmit a wireless signal through a wireless channel in the car to the AP device 7310 that serves as a receiver (or an Origin, or a Type 2 device). The received wireless signal differs from the transmitted wireless signal due to the wireless channel that may be impacted by any vital sign motion of a human being (e.g. a child) in the car. As such, the received wireless signal carries information of the vital sign motion, if presence, in the car. In some embodiments, the wireless signal may be a Wi-Fi signal. The detection may be applied to detect any vital sign (e.g. breathing or heartbeat or a periodic motion) of an object (e.g. a person, a user, a baby) in a venue (e.g. a vehicle) based on the channel information obtained from the received wireless signal.

FIG. 73A illustrates a side perspective view of a car including the system 7300; while FIG. 73B illustrates a top perspective view of the car including the system 7300, according to some embodiments of the present disclosure. As shown in FIG. 73A and FIG. 73B, the AP device 7310 may be placed near the gear area or coupled to the dashboard; and the STA device 7320 may be placed on the left side of the rear seat. This setting can generate a full coverage inside the car. In other embodiments, each of the AP device 7310 and the STA device 7320 may be placed at different locations in the car with same detection and operation method applied for wireless vital sign monitoring.

FIG. 74 illustrates a flow chart showing an exemplary method 7400 of a wireless vital sign detection system, e.g. the system 7300 as shown in FIG. 73A and FIG. 73B, according to some embodiments of the present disclosure. As shown in FIG. 74, after the AP and STA antennas are placed in a car and some initial set ups of the system at operation 7410, the STA antenna can transmit a wireless signal, e.g. Wi-Fi signal, through a wireless channel in the car to the AP antenna. The received wireless signal may be used at operation 7420 to capture channel state information (CSI) of the wireless channel in the car. If there is any living object (e.g. child) presence in the car, the captured CSI would include information of a periodic vital sign motion (e.g. breathing or heartbeat) of the living object. In addition, the captured CSI would also include information of any other motion (e.g. non-vital sign movement of the child) in the car.

In the example shown in FIG. 74, the obtained CSI is sent to a breathing engine 7430 for detecting and monitoring breathing in the car; and is also sent to a motion engine 7440 for detecting any non-vital sign motion in the car.

The breathing engine 7430 in this example performs a series of operations 7431-7437. At operation 7431, a phase boosting is applied on the obtained CSI. For example, the breathing engine 7430 can compute a cleaned phase = $\angle H_{m2,n}(f, t) - \angle H_{m1,n}(f, t)$. The cleaned phase may be added as an additional dimension to the CSI vector for phase boosting. In some embodiments, amplitude cleaning is not performed since it would remove the required information.

Then, the breathing engine 7430 may perform (time) window size selection and autocorrelation function (ACF) calculations at operation 7432. Window size selection is critical as it is directly related to initial detection delay. To detect the breathing rates correctly, a considerably long window size is desirable. On the other hand, longer window sizes would cause longer detection delays.

In some embodiments, changing or varying window sizes are used in the breathing engine 7430 to achieve high performance as well as low detection delays. To reduce the initial delay, a small window size is used at the start of the engine 7430. The window size is increased to the required length after the engine 7430 stabilizes.

Then, the breathing engine 7430 may perform enhancement techniques to improve detection at operation 7433. The enhancement techniques may be applied to the ACF spectrum to enhance the breathing traces. These enhancement techniques may be image enhancement techniques including but not limited to: contrast stretching, slicing, histogram equalization, and some algorithms based on the retinex. For child presence detection (CPD) based on motion and breathing detection, image enhancement can also be applied to the ACF to make the peaks of the ACF more prominent, and thus the breathing rate more continuous, and thus the detection rate of CPD can be improved. In some embodiments, image enhancement can also be applied directly to the breathing estimation traces after spectral analysis/frequency components analysis of the time series of channel information. For example, the spectrograms on different transmitter-receiver links can each be enhanced across the different subcarriers and then combined.

In the example shown in FIG. 74, the enhancement techniques include the following. First, peak enhancement is performed on the ACF spectrum. Value k is selected from the experiments, such that enhanced ACF = ACF - k * diff(ACF), where diff(ACF) means the difference between adjacent values of the ACF signal. After enhancement, the signal is normalized to the [0, 1] range. Then, histogram equalization may be performed on the normalized peak enhanced signal.

Then, the breathing engine 7430 may perform false alarm removal at operation 7434. In some embodiments, there are two types of false alarm removal using two techniques. Dynamic time warping (DTW) may be used to measure the similarity between a given breathing signal and extracted breathing signal from the engine 7430. If the extracted breathing signal corresponds to true breathing, the DTW value will be low compared to the false breath. FIG. 75 depicts the DTW value distribution 7500 for true breathing and false breathing, according to some embodiments of the present disclosure. FIG. 75 may be used to determine the DTW threshold value.

In addition, to evaluate the noise level, the number of zero crossings for the extracted signals may be calculated. A high number of zero crossings are expected for false breathing signals as false breathing exhibits higher oscillations than true breathing signals. In some embodiments, extracted breathing signals are in the range of 0-1. Thus, one can consider 0.5 as the 0-th level when calculating the number of zero crossings.

Then, the breathing engine 7430 may perform peak detection at operation 7435. In some embodiments, if the difference between two consecutive peaks is in the range of the breathing cycle, those peaks are considered as the potential peaks to calculate the breathing rate.

Then, the breathing engine 7430 may perform breathing rate calculation at operation 7436. In some embodiments, breathing rate is calculated by the difference between two detected peaks from operation 7435.

Then, the breathing engine 7430 may generate a breathing indicator at operation 7437. In some embodiments, if there are more than two/three/five breathing rates detected in a 10-sec window, it is indicated that a breathing is detected.

The breathing indicator may be sent to the operation child presence detection (CPD) indicator 7450 to determine whether there is a child presence in the car. The CPD indicator 7450 may be generated based on the breathing detection result from the breathing engine 7430, as well as a motion detection result from the motion engine 7440. The motion engine 7440 may perform motion detection operation 7442 based on the obtained CSI, to detect any non-breathing or non-vital motion in the car. In some embodiments, if breathing and/or motion is detected, the system indicates the presence of a baby inside the car.

This Wi-Fi based in-car CPD system utilizing the above described method has a higher performance capability than other Wi-Fi based CPD systems. The approach is robust to severe environmental conditions such as heavy rain, busy traffic around the car. Some image enhancement methods are utilized to increase the detection capabilities, while false alarm removal methods can make the system free of false detections.

In some embodiments, variable window length, instead of fixed window length, is used to calculate the breathing spectrum. Instead of using ACF spectrum directly, the system can employ image enhancement techniques including peak enhancement and histogram equalization to improve the detection performance. During the false alarm removal step, the system may utilize DTW to evaluate structural similarity between true breathing signal and estimated signal, and utilize the number of zero crossing to determine a noise level.

FIG. 76 illustrates an exemplary block diagram of a first wireless device, e.g. a Bot 7600, of a system for radio-assisted signal estimation, according to some embodiments of the present disclosure. The Bot 7600 is an example of a device that can be configured to implement the various methods described herein. As shown in FIG. 76, the Bot 7600 includes a housing 7640 containing a processor 7602, a memory 7604, a transceiver 7610 comprising a transmitter 7612 and receiver 7614, a synchronization controller 7606, a power module 7608, an optional carrier configurator 7620 and a wireless signal generator 7622.

In this embodiment, the processor 7602 controls the general operation of the Bot 7600 and can include one or more processing circuits or modules such as a central processing unit (CPU) and/or any combination of general-purpose microprocessors, microcontrollers, digital signal processors (DSPs), field programmable gate array (FPGAs), programmable logic devices (PLDs), controllers, state machines, gated logic, discrete hardware components, dedicated hardware finite state machines, or any other suitable circuits, devices and/or structures that can perform calculations or other manipulations of data.

The memory 7604, which can include both read-only memory (ROM) and random access memory (RAM), can provide instructions and data to the processor 7602. A portion of the memory 7604 can also include non-volatile random access memory (NVRAM). The processor 7602 typically performs logical and arithmetic operations based on program instructions stored within the memory 7604. The instructions (a.k.a., software) stored in the memory 7604 can be executed by the processor 7602 to perform the methods described herein. The processor 7602 and the memory 7604 together form a processing system that stores and executes software. As used herein, "software" means any type of instructions, whether referred to as software, firmware, middleware, microcode, etc. which can configure a machine or device to perform one or more desired functions or processes. Instructions can include code (e.g., in source code format, binary code format, executable code format, or any other suitable format of code). The instructions, when executed by the one or more processors, cause the processing system to perform the various functions described herein.

The transceiver 7610, which includes the transmitter 7612 and receiver 7614, allows the Bot 7600 to transmit and receive data to and from a remote device (e.g., an Origin or another Bot). An antenna 7650 is typically attached to the housing 7640 and electrically coupled to the transceiver 7610. In various embodiments, the Bot 7600 includes (not shown) multiple transmitters, multiple receivers, and multiple transceivers. In one embodiment, the antenna 7650 is replaced with a multi-antenna array 7650 that can form a plurality of beams each of which points in a distinct direction. The transmitter 7612 can be configured to wirelessly transmit signals having different types or functions, such signals being generated by the processor 7602. Similarly, the receiver 7614 is configured to receive wireless signals having different types or functions, and the processor 7602 is configured to process signals of a plurality of different types.

The Bot 7600 in this example may serve as Bot, Type 1 device, transmitter, or STA in the systems disclosed herein. For example, the wireless signal generator 7622 may generate and transmit, via the transmitter 7612, a wireless signal through a wireless channel in the venue. The received wireless signal would carry channel information of the wireless channel. Because the wireless channel is impacted by any vital sign motion (e.g. breathing, heartbeat, etc.) of an object in the venue, the channel information includes vital sign information from the object. As such, a vital sign can be detected and monitored based on channel information obtained from the received wireless signal. The generation of the wireless signal at the wireless signal generator 7622 may be based on a request for wireless vital sign monitoring from another device, e.g. an Origin, or based on a system pre-configuration. That is, the Bot 7600 may or may not know that the wireless signal transmitted will be used for wireless vital sign monitoring. Other application of wireless sensing, monitoring and tracking can be performed similarly.

The synchronization controller 7606 in this example may be configured to control the operations of the Bot 7600 to be synchronized or un-synchronized with another device, e.g. an Origin or another Bot. In one embodiment, the synchronization controller 7606 may control the Bot 7600 to be synchronized with an Origin that receives the wireless signal transmitted by the Bot 7600. In another embodiment, the synchronization controller 7606 may control the Bot 7600 to transmit the wireless signal asynchronously with other Bots. In another embodiment, each of the Bot 7600 and other Bots may transmit the wireless signals individually and asynchronously.

The carrier configurator 7620 is an optional component in Bot 7600 to configure transmission resources, e.g. time and carrier, for transmitting the wireless signal generated by the wireless signal generator 7622. In one embodiment, each CI of the time series of CI has one or more components each corresponding to a carrier or sub-carrier of the transmission of the wireless signal. The wireless sound sensing may be based on any one or any combination of the components.

The power module 7608 can include a power source such as one or more batteries, and a power regulator, to provide regulated power to each of the above-described modules in FIG. 76. In some embodiments, if the Bot 7600 is coupled to a dedicated external power source (e.g., a wall electrical outlet), the power module 7608 can include a transformer and a power regulator.

The various modules discussed above are coupled together by a bus system 7630. The bus system 7630 can include a data bus and, for example, a power bus, a control signal bus, and/or a status signal bus in addition to the data bus. It is understood that the modules of the Bot 7600 can be operatively coupled to one another using any suitable techniques and mediums.

Although a number of separate modules or components are illustrated in FIG. 76, persons of ordinary skill in the art will understand that one or more of the modules can be combined or commonly implemented. For example, the processor 7602 can implement not only the functionality described above with respect to the processor 7602, but also implement the functionality described above with respect to the wireless signal generator 7622. Conversely, each of the modules illustrated in FIG. 76 can be implemented using a plurality of separate components or elements.

FIG. 77 illustrates an exemplary block diagram of a second wireless device, e.g. an Origin 7700, of a system for radio-assisted signal estimation, according to one embodiment of the present teaching. The Origin 7700 is an example of a device that can be configured to implement the various methods described herein. The Origin 7700 in this example may serve as Origin, receiver, Type 2 device or AP in systems disclosed herein. As shown in FIG. 77, the Origin 7700 includes a housing 7740 containing a processor 7702, a memory 7704, a transceiver 7710 comprising a transmitter 7712 and a receiver 7714, a power module 7708, a synchronization controller 7706, a channel information extractor 7720, and an optional motion detector 7722.

In this embodiment, the processor 7702, the memory 7704, the transceiver 7710 and the power module 7708 work similarly to the processor 7602, the memory 7604, the transceiver 7610 and the power module 7608 in the Bot 7600. An antenna 7750 or a multi-antenna array 7750 is typically attached to the housing 7740 and electrically coupled to the transceiver 7710.

The Origin 7700 may be a second wireless device that has a different type from that of the first wireless device (e.g. the Bot 7600). In particular, the channel information extractor 7720 in the Origin 7700 is configured for receiving the wireless signal through the wireless channel, and obtaining a time series of channel information (CI) of the wireless channel based on the wireless signal. The channel information extractor 7720 may send the extracted CI to the optional motion detector 7722 or to a motion detector outside the Origin 7700 for wireless sound sensing in the venue.

The motion detector 7722 is an optional component in the Origin 7700. In one embodiment, it is within the Origin 7700 as shown in FIG. 77. In another embodiment, it is outside the Origin 7700 and in another device, which may be a Bot, another Origin, a cloud server, a fog server, a local server, and an edge server. The optional motion detector 7722 may be configured for detecting sound information from a vibrating object or source in the venue based on motion information. The motion information may be computed based on the time series of CI by the motion detector 7722 or another motion detector outside the Origin 7700.

The synchronization controller 7706 in this example may be configured to control the operations of the Origin 7700 to be synchronized or un-synchronized with another device, e.g. a Bot, another Origin, or an independent motion detector. In one embodiment, the synchronization controller 7706 may control the Origin 7700 to be synchronized with a Bot that transmits a wireless signal. In another embodiment, the synchronization controller 7706 may control the Origin 7700 to receive the wireless signal asynchronously with other Origins. In another embodiment, each of the Origin 7700 and other Origins may receive the wireless signals individually and asynchronously. In one embodiment, the optional motion detector 7722 or a motion detector outside the Origin 7700 is configured for asynchronously computing respective heterogeneous motion information based on the respective time series of CI.

The various modules discussed above are coupled together by a bus system 7730. The bus system 7730 can include a data bus and, for example, a power bus, a control signal bus, and/or a status signal bus in addition to the data bus. It is understood that the modules of the Origin 7700 can be operatively coupled to one another using any suitable techniques and mediums.

Although a number of separate modules or components are illustrated in FIG. 77, persons of ordinary skill in the art will understand that one or more of the modules can be combined or commonly implemented. For example, the processor 7702 can implement not only the functionality described above with respect to the processor 7702, but also implement the functionality described above with respect to the channel information extractor 7720. Conversely, each of the modules illustrated in FIG. 77 can be implemented using a plurality of separate components or elements.

FIG. 78 illustrates a flow chart of an exemplary method 7800 for radio-assisted signal estimation, according to some embodiments of the present disclosure. In various embodiments, the method 7800 can be performed by the systems disclosed above. At operation 7802, a wireless signal is transmitted through a wireless channel of a venue. At operation 7804, the wireless signal is received through the wireless channel. The received wireless signal differs from the transmitted wireless signal due to the wireless channel that is impacted by a periodic motion of a vital sign of an object in the venue. At operation 7806, a time series of channel information (CI) of the wireless channel is obtained based on the received wireless signal. At operation 7808, a two dimensional (2D) decomposition of the time series of CI (TSCI) is computed. Then the 2D decomposition is enhanced at operation 7810. At operation 7812, the periodic motion of the vital sign is monitored based on the enhanced 2D decomposition. The order of the operations in FIG. 78 may be changed according to various embodiments of the present teaching.

In some embodiments, vital sign detection is based on 2D decomposition of TSCI obtained from wireless sounding signal from a Type 1 device to a Type 2 device.

The following numbered clauses provide examples for wireless vital sign monitoring.

Clause J1. A method/device/system/software of a wireless vital sign monitoring system, comprising: transmitting a wireless signal from a Type1 heterogeneous wireless device of the wireless sensing system through a wireless multipath channel of a venue, wherein the wireless multipath channel is impacted by a vital sign motion of an object in the venue; receiving the wireless signal by a Type2 heterogeneous wireless device of the system through the wireless multipath channel, wherein the received wireless signal differs from the transmitted wireless signal due to the wireless multipath channel of the venue and the periodic vital sign motion of the object; obtaining a time series of channel information (CI) of the wireless multipath channel based on the received wireless signal using a processor, a memory and a set of instructions; computing a two dimensional (2D) decomposition of the time series of CI (TSCI); enhancing the 2D decomposition; monitoring the vital sign motion based on the enhanced 2D decomposition.

Clause J2. The method/device/system/software of the wireless vital sign monitoring system of clause J1, comprising: computing a time series of CI-feature (TSCF) based on the TSCI, each CI-feature (CF) comprising a concatenation of a basic feature of a respective CI and another basic feature of a corresponding denoised CI, wherein the basic feature of any CI comprises at least one of: the CI, a magnitude of the CI, a phase of the CI, a magnitude of a component of the CI (CI-component), a phase of the component of the CI, a function of a magnitude, or a function of a phase, wherein the denoised CI is computed by processing the TSCI with a first denoising filter, the first denoising filter comprising at least one of: a low-pass filter, a bandpass filter, a highpass filter, a weighted sum, a weighted difference, a weighted average, a median filter, a percentile filter, a CI-component-wise denoising filter; computing the 2D decomposition based on the TSCF.

In some embodiments, the system can compute 1D characteristic function for each sliding time window, and construct 2D decomposition based on the 1D characteristic function.

Clause J3. The method/device/system/software of the wireless vital sign monitoring system of clause J1 or J2, comprising: determining a series of sliding time windows, each sliding time window associated with a time stamp; computing a respective one dimensional (1D) characteristic function of the CF of the TSCF in each sliding time window; constructing the 2D decomposition based on the respective 1D characteristic function in each sliding time window.

In some embodiments, the system can compute 1D characteristic function based on a transformation such as ACF or STFT.

Clause J4. The method/device/system/software of the wireless vital sign monitoring system of clauses J1 to J3, comprising: computing the respective 1D characteristic function based on a transformation of the CF of the TSCF in the respective sliding time window, wherein the transformation comprises at least one of: a frequency transformation, a transformation to frequency domain, a short-time Fourier transform (STFT), a wavelet transform, a filter-bank representation, a spectral analysis, a harmonic analysis, a Fourier analysis, a multi-resolution analysis, a time transformation, a transformation to a time-shift domain, an auto-correlation function (ACF), an auto-covariance function, a time-frequency decomposition, a time-frequency representation, or a spectrogram.

In some embodiments, the system can line up and align 1D characteristic function to construct a 2D matrix which is the 2D decomposition.

Clause J5. The method/device/system/software of the wireless vital sign monitoring system of any previous J clause, comprising: constructing a 2D matrix by lining up the 1D characteristic functions in time order in accordance with the associated time stamp and aligning them, wherein the i-th column of the 2D matrix is the 1D characteristic function associated with the i-th sliding time window, wherein the j-th row of the 2D matrix is associated with a time-shift of j time units, wherein the matrix element at i-th column and j-th row is the value of the 1D characteristic function in the i-th sliding time window with a time-shift of j time units, wherein the 2D matrix is the 2D decomposition.

In some embodiments, the system can enhance (process) 2D matrix with vertical highpass filtering and horizontal lowpass filter.

Clause J6. The method/device/system/software of the wireless vital sign monitoring system of any previous J clause, comprising: enhancing the 2D decomposition by applying to each matrix element of the 2D matrix either vertical highpass filtering, or horizontal lowpass filtering, or both.

In some embodiments, the filter may be 2D filter, which may be separable into a 1D vertical filter and a 1D horizontal filter. Different matrix elements may have different filter.

Clause J7. The method/device/system/software of the wireless vital sign monitoring system of any previous J clause, comprising: filtering each matrix element of the 2D matrix with a respective 2D vertical highpass horizontal lowpass filter, wherein the 2D filter is one of: separable or non-separable, wherein the 2D filter is either linear or nonlinear.

In some embodiments, the filter may be separable such that the 2D filter is decomposed into a 1D vertical highpass filter and a 1D horizontal lowpass filter.

Clause J8. The method/device/system/software of the wireless vital sign monitoring system of any previous J clause, comprising: enhancing the 2D decomposition by at least one of: filtering each matrix element of the 2D matrix with a respective vertical highpass filter, or filtering each matrix element of the 2D matrix with a respective horizontal lowpass filter, wherein any filter is either linear or nonlinear.

In some embodiments, same vertical highpass filter is for a whole column/part of a column, or a whole row/part of a row; or same horizontal lowpass filter is for a whole column/part of a column, or a whole row/part of a row.

Clause J9. The method/device/system/software of the wireless vital sign monitoring system of any previous J clause, comprising: enhancing the 2D decomposition by at least one of: filtering part of each column of the 2D matrix with a respective first common vertical highpass filter, or filtering part of each row of the 2D matrix with a respective second common vertical highpass filter, or filtering part of each column of the 2D matrix with a respective first common horizontal lowpass filter, or filtering part of each row of the 2D matrix with a respective second common horizontal lowpass filter.

In some embodiments, vertical highpass filter is a 1D filter applied in either vertical direction or near-vertical direction.

Clause J10. The method/device/system/software of the wireless vital sign monitoring system of any previous J clause, comprising: wherein a vertical highpass filter is a directional 1D highpass filter applied in the vertical direction or in a near-vertical direction.

In some embodiments, horizontal lowpass filter is a 1D filter applied in either horizontal direction or near-horizontal direction.

Clause J11. The method/device/system/software of the wireless vital sign monitoring system of any previous J clause, comprising: wherein a horizontal lowpass filter is a directional 1D lowpass filter applied in the horizontal direction or in a near-horizontal direction.

In some embodiments, there may be a succession of two or more filters, each being either vertical highpass or horizontal lowpass.

Clause J12. The method/device/system/software of the wireless vital sign monitoring system of any previous J clause, comprising: filtering each matrix element of the 2D matrix with a succession of at least two 1D filters, each being either vertical highpass filter or horizontal lowpass filter.

In some embodiments, histogram equalization is performed.

Clause J13. The method/device/system/software of the wireless vital sign monitoring system of any previous J clause, comprising: enhancing the 2D decomposition by applying histogram equalization to a number of consecutive columns of the 2D matrix.

In some embodiments, the system can compute matching score based on DTW. If matching score is larger than a threshold (i.e. bad match), there is no vital sign present.

Clause J14. The method/device/system/software of the wireless vital sign monitoring system of clause J13, comprising: computing a distance score based on dynamic time warping (DTW) between a reference column and a particular column of the enhanced 2D matrix associated with a particular time, wherein the reference column is computed in a training session by clustering a number of training columns matched based on DTW, wherein the training columns are computed based on training TSCI obtain from received training wireless signals transmitted when training objects with vital sign are present; if the distance score is larger than a first threshold, the vital sign motion of the object is tentatively not detected at the particular time.

In some embodiments, the system can compute zero crossing score. If zero crossing score is larger than a threshold (i.e. noisy), there is no vital sign present.

Clause J15. The method/device/system/software of the wireless vital sign monitoring system of any previous J clause, comprising: subtracting a quantity from each matrix element in the particular column of the enhanced 2D matrix; computing a zero-crossing count of the particular column; if the zero-crossing count is larger than a second threshold, the vital sign motion of the object is tentatively not detected at the particular time.

In some embodiments, the system can compute inter-peak distance. If inter-peak distance is not inside acceptable range, there is no vital sign present. If the inter-peak distance is inside acceptable range, vital sign is detected. Period/frequency of detected vital sign may be computed based on the inter-peak distance.

Clause J16. The method/device/system/software of the wireless vital sign monitoring system of any previous J clause, comprising: computing more than one peaks (local maxima) of the particular column of the enhanced 2D matrix; computing an inter-peak distance based on the more than one peaks; If the inter-peak distance is in an acceptable range, the vital sign motion of the object is tentatively detected at the particular time and one of: a frequency or a period, of the vital sign is equal to the inter-peak distance; If the inter-peak distance is not in an acceptable range, the vital sign motion of the object is tentatively not detected at the particular time.

In some embodiments, vital sign is detected if it is tentatively detection at a percentage higher than a threshold.

Clause J17. The method/device/system/software of the wireless vital sign monitoring system of any previous J clause, comprising: detecting the vital sign motion of the object if A is larger than a third threshold, wherein A is the percentage of vital sign being tentatively detected in a period of time.

In some embodiments, the system can detect voice activity based on radio signal (i.e. based on CI obtained from received radio signal) without using any media input (e.g. video, or image/visual, or audio or speech or sound) or any data transmitted in the radio signal by transmitter (Type 1 device).

Clause J18. A method/device/system/software of a wireless vital sign monitoring system, comprising: detecting voice activity based on a radio signal using a processor, a memory and a set of instruction of a device of the system, without using any media signal.

In some embodiments, radio signal is transmitted by Type 1 device and received by Type 2 device. TSCI obtained in Type 2 device based on received radio signal.

Clause J19. The method/device/system/software of the wireless vital sign monitoring system of any previous J clause, comprising: wherein the radio signal is a wireless signal transmitted by a Type 1 heterogeneous wireless device of the system in a venue; wherein the radio signal is received by a Type 2 heterogeneous wireless device of the system through a wireless multipath channel of the venue, wherein the wireless multipath channel is impacted by the voice activity in the venue; obtaining a time series of channel information (CI) of the wireless multipath channel based on the received radio signal; detecting the voice activity based on the time series of CI (TSCI) of the wireless multipath channel.

Clause J20. The method/device/system/software of the wireless vital sign monitoring system of any previous J clause, further comprising: wherein the radio signal comprises at least one of: a data communication signal, a wireless network signal, a standard compliant signal, a wireless local area network (WLAN) signal, a WiFi signal, an IEEE 802 signal, an IEEE 802.11 signal, an IEEE 802.11bf signal, an IEEE 802.11 directional multi-gigabit (DMG) signal, a wireless communication network signal, a 3GPP signal, a 4G/LTE/5G/6G/7G/8G signal, a wireless sensing signal, a wireless sounding signal, a radar signal, a millimeter wave (mmWave) signal, a UWB signal, or a electromagnetic signal above 40 kHz; wherein the media signal comprises at least one of: a microphone signal, a speech signal, a vocal signal, an audio signal, a signal less than 40kHz, an acoustic signal, an audible signal, a telephone signal, a tele-conferencing signal, an audio-telephony signal, a conference call signal, a visual signal, a video signal, a video-telephony signal, a video-conferencing signal, a media streaming signal, or a multimedia signal.

In some embodiments, no data payload in radio signal is used.

Clause J21. The method/device/system/software of the wireless vital sign monitoring system of any previous J clause, comprising: wherein the radio signal is a data communication signal; detecting the voice activity without using any data payload communicated in the data communication signal.

In some embodiments, no media data in radio signal is used.

Clause J22. The method/device/system/software of the wireless vital sign monitoring system of any previous J clause, comprising: detecting the voice activity without using any media signal data communicated in the data communication signal.

In some embodiments, voice activity is produced by target voice source.

Clause J23. The method/device/system/software of a wireless vital sign monitoring system of any previous J clause, comprising: detecting the voice activity of a target voice source in the venue based on the radio signal, wherein the wireless multipath channel is impacted by a voice producing motion of the target voice source.

In some embodiments, the voice-related radio feature (TSRF) is bandlimited. Telephone speech signal may be bandlimited 4kHz. Speech signal may be bandlimited to 7kHz. Audio may be bandlimited to 20kHz. TSRF may be bandlimited to half of sounding frequency, which may be half of 1/10/100/1000/10000/100000/1000000 Hz. To play safe here, TSRF may be bandlimited to 1Mhz. It can be bandlimited to 100kHz, or 10kHz also.

Clause J24. The method/device/system/software of a wireless vital sign monitoring system of any previous J clause, comprising: computing a time series of radio feature (TSRF) based on the TSCI, each radio feature (RF) of the TSRF being computed based on a respective sliding window of the TSCI, wherein the TSRF is a baseband signal bandlimited to 1MHz; detecting the voice activity based on the TSRF.

In some embodiments, the system can detect voice activity by detecting voice-related characteristics (e.g. pitch, harmonics of the pitch, time profile of pitch) that suggests the presence of voice/speech.

Clause J25. The method/device/system/software of a wireless vital sign monitoring system of any previous J clause, comprising: detecting a voice-related characteristics in the TSRF; detecting the voice activity based on the detected voice-related characteristics in the TSRF.

In some embodiments, there are many voice-related characteristics listed here that may be manifested in the TSRF (e.g. pitch, pitch profile, inter-mixed voice/unvoiced speech, etc.). These voice characteristics differentiate voice from non-voice (e.g. machine sound, wind sound, environmental sound, etc.). In particular, voiced speech (e.g. vowel, some consonants, liquid, etc.) has pitch. Unvoiced speech (e.g. most consonants, fricative, plosive) has no pitch. Vowels are voiced sound. Typical human pitch goes between 50Hz and 250Hz.

Clause J26. The method/device/system/software of a wireless vital sign monitoring system of any previous J clause, further comprising: wherein the voice-related characteristics comprises at least one of the following characteristics: a speech feature, a vowel, a consonant, a fricative, an affricate, a plosive, a nasal, an approximant, a liquid, a lateral, bilabial sound, velar sound, alveolar sound, a phone, a phoneme, voiced sound, unvoiced sound, a pitch of voiced speech, a foundation frequency, a voice-related frequency range, at least one harmonics of voiced-speech, at least one formant of speech, a time-varying pitch, a pitch profile, a voice-related time trend of pitch, a tone, a prosodic feature, a sequence of intermittent voiced and unvoiced sound, a musical feature, a speech timing, a speech pacing, a musical timing, a musical pacing, or an environmental sound.

In some embodiments, there is an instantaneous pitch at a current time, which may be observable in TSRF.

Clause J27. The method/device/system/software of a wireless vital sign monitoring system of any previous J clause, further comprising: detecting an instantaneous pitch associated with a current time instance in the TSRF, wherein an instantaneous fundamental frequency associated with the instantaneous pitch is greater than a lower threshold and less than a upper threshold; detecting the voice activity based on the detected instantaneous pitch.

Human speech often has many harmonics. Some harmonics may be observable in TSRF.

Clause J28. The method/device/system/software of a wireless vital sign monitoring system of any previous J clause, further comprising: detecting at least one instantaneous harmonics of the instantaneous pitch associated with the current time instance in the TSRF, wherein a frequency associated with each respective harmonics is an integer multiple of the instantaneous fundamental frequency of the instantaneous pitch; detecting the voice activity based on the detected instantaneous harmonic of the pitch.

Besides having an instantaneous pitch, human speech has a time-varying pitch. The human speech may use the time-varying pitch to express many things: tones in a tonal language, prosidy, vowel pronunciation, consonant pronunciation, etc. The time-varying pitch may comprise pitches within a certain time window.

Clause J29. The method/device/system/software of a wireless vital sign monitoring system of any previous J clause, further comprising: detecting a pitch profile comprising a plurality of instantaneous pitches associated with a plurality of respective time instances in the TSRF, wherein each instantaneous pitch associated with a respective time instance is associated with a respective instantaneous fundamental frequency greater than the lower threshold and less than the upper threshold; detecting a voice-related time trend of the plurality of instantaneous pitches in the pitch profile; detecting the voice activity based on the detected pitch profile and the detected voice-related time trend of the instantaneous pitches.

In some embodiments, there are some linguistically/phonetically meaningful time trends of the time-varying pitch.

Clause J30. The method/device/system/software of a wireless vital sign monitoring system of any previous J clause, further comprising: wherein the time trend comprises at least one of: a local continuity of instantaneous pitches, a local continuity of the instantaneous frequencies, a local continuity of frequency of the instantaneous harmonics, a habitual pitch, a long term pitch, a variation of pitch around the habitual pitch, a timing or pacing of pitch change, a fast pitch change within a tone, or a slow pitch change reflecting prosody.

In some embodiments, the voice-related characteristics may be detected in time domain using neural network.

Clause J31. The method/device/system/software of a wireless vital sign monitoring system of any previous J clause, further comprising: processing the TSRF with a neural network; detecting the voice-related characteristics based on the neural network processing of the TSFR.

In some embodiments, the voice-related characteristics may be detected in frequency domain; and the system can perform frequency decomposition of TSRF.

Clause J32. The method/device/system/software of a wireless vital sign monitoring system of any previous J clause, further comprising: computing a frequency decomposition of the TSRF by computing at least one of: a spectrogram, a short-time Fourier transform (STFT), a wavelet transform, a filter-bank representation, a harmonic analysis, a Fourier analysis, a multi-resolution analysis, a time-frequency decomposition, a time-frequency representation, a sonograph, a voiceprint, a voicegram, or a waterfall display; detecting the voice-related characteristics based on the frequency decomposition.

In some embodiments, the voice-related characteristics may be detected in frequency domain using neural network. An optional feature is to apply "overlap and concatenate" on the frequency decomposition to generate input to the neural network.

Clause J33. The method/device/system/software of a wireless vital sign monitoring system of any previous J clause, further comprising: processing the frequency decomposition of the TSRF with a neural network; detecting the voice-related characteristics based on the neural network processing of the frequency decomposition of the TSRF.

In some embodiments, the voice-related characteristics may be detected in frequency domain using some algorithm (i.e. not using neural network). The algorithm may detect the manifestation (e.g. pitch, harmonics, local continuity) of the voice-related characteristics in the TSRF.

Clause J34. The method/device/system/software of a wireless vital sign monitoring system of any previous J clause, further comprising: wherein the voice-related characteristics comprises a pitch of the voice activity; detecting an instantaneous pitch based on the frequency decomposition of the TSRF in a time window associated with the current time instance.

In some embodiments, speech signals may have harmonics in addition to the pitch. Some of the harmonics may be observable in the TSRF.

Clause J35. The method/device/system/software of a wireless vital sign monitoring system of any previous J clause, further comprising: wherein the voice-related characteristics further comprises a harmonics of the pitch of the voice activity; detecting an instantaneous harmonics based on the frequency decomposition of the TSRF in a time window associated with the current time instance.

In some embodiments, the system can identify target voice source based on beamforming. First TSCI are the raw TSCI. Second TSCI are computed based on beamforming performed on the set of raw TSCI.

Clause J36. The method/device/system/software of a wireless vital sign monitoring system of any previous J clause, further comprising: wherein the Type 1 device or Type 2 device has an array of antennas; obtaining a set of raw TSCI of the wireless multipath channel based on the received radio signal, each raw TSCI associated with a respective antenna; obtaining a set of directional TSCI based on a beamforming performed on the set of raw TSCI obtained based on the received radio signal associated with the array of antennas, each directional TSCI associated with a direction relative to the array of antennas, wherein the TSCI is a particular directional TSCI.

In some embodiments, the system can suppress non-target voice source based on beamforming.

Clause J37. The method/device/system/software of the wireless vital sign monitoring system of any previous J clause, further comprising: associating the target voice source with a component of the TSCI, wherein there is at least one non-target voice source undergoing respective asynchronous voice producing motion, wherein the wireless channel is impacted asynchronously by the respective asynchronous voice producing motion of the at least one non-target voice source; associating each non-target voice source with a different component of the TSCI or a different directional TSCI; selecting the component of the TSCI; and rejecting the at least one non-target voice source by computing the TSRF based on the selected component of the TSCI.

Clause J38. The method/device/system/software of the wireless vital sign monitoring system of any previous J clause, further comprising: detecting the voice activity of the target voice source based on the TSRF computed based on the TSCI; detecting another voice activity of a non-target voice source based on another TSRF computed based on another directional TSCI.

The following numbered clauses provide examples for wireless vital sign monitoring.

Clause K1. A system for wireless vital sign monitoring, comprising: a transmitter configured to transmit a wireless signal through a wireless channel of a venue; a receiver configured to receive the wireless signal through the wireless channel, wherein the received wireless signal differs from the transmitted wireless signal due to the wireless channel that is impacted by a periodic motion of a vital sign of an object in the venue; and a processor configured for: obtaining a time series of channel information (CI) of the wireless channel based on the received wireless signal, computing a two dimensional (2D) decomposition of the time series of CI (TSCI), enhancing the 2D decomposition, and monitoring the periodic motion of the vital sign based on the enhanced 2D decomposition.

Clause K2. The system of Clause K1, wherein the processor is further configured for: computing a time series of CI-feature (TSCF) based on the TSCI, each CI-feature (CF) comprising a concatenation of a basic feature of a respective CI and a basic feature of a corresponding denoised CI, wherein the basic feature of any CI comprises at least one of: the CI, a magnitude of the CI, a phase of the CI, a magnitude of a component of the CI, or a phase of the component of the CI, wherein the denoised CI is computed by processing the TSCI with a first denoising filter, the first denoising filter comprising at least one of: a low-pass filter, a bandpass filter, a highpass filter, a median filter, or a percentile filter; and computing the 2D decomposition based on the TSCF.

Clause K3. The system of Clause K1 or K2, wherein the processor is further configured for: determining a series of sliding time windows, each sliding time window being associated with a time stamp; computing a respective one dimensional (1D) characteristic function of the CF of the TSCF in each sliding time window; and constructing the 2D decomposition based on the respective 1D characteristic function in each sliding time window.

Clause K4. The system of Clauses K1 to K3, wherein: the respective 1D characteristic function is computed based on a transformation of the CF of the TSCF in each sliding time window; and the transformation comprises at least one of: a frequency transformation, a transformation to frequency domain, a short-time Fourier transform (STFT), a wavelet transform, a filter-bank representation, a time transformation, a transformation to a time-shift domain, an auto-correlation function (ACF), an auto-covariance function, or a time-frequency decomposition.

Clause K5. The system of any previous K Clause, wherein the processor is further configured for: constructing a 2D matrix by lining up and aligning the 1D characteristic functions of the sliding time windows in time order according to the associated time stamps, wherein i-th column of the 2D matrix represents the 1D characteristic function associated with i-th sliding time window, j-th row of the 2D matrix is associated with a time-shift of j time units, a matrix element at the i-th column and the j-th row of the 2D matrix is a value of the 1D characteristic function in the i-th sliding time window with a time-shift of j time units, and the 2D matrix is a result of the 2D decomposition.

Clause K6. The system of any previous K Clause, wherein the processor is further configured for: enhancing the 2D decomposition by applying, to each matrix element of the 2D matrix, a vertical highpass filtering, a horizontal lowpass filtering, or both.

Clause K7. The system of any previous K Clause, wherein the processor is further configured for: filtering each matrix element of the 2D matrix with a respective 2D vertical highpass horizontal lowpass filter that is either separable or non-separable, either linear or nonlinear.

Clause K8. The system of any previous K Clause, wherein the processor is further configured for: enhancing the 2D decomposition by filtering each matrix element of the 2D matrix with at least one of: a respective vertical highpass filter or a respective horizontal lowpass filter, wherein each of the respective vertical highpass filter and the respective horizontal lowpass filter is either linear or nonlinear.

Clause K9. The system of any previous K Clause, wherein the processor is further configured for enhancing the 2D decomposition by at least one of: filtering part of each column of the 2D matrix with a respective first common vertical highpass filter; filtering part of each row of the 2D matrix with a respective second common vertical highpass filter; filtering part of each column of the 2D matrix with a respective first common horizontal lowpass filter; or filtering part of each row of the 2D matrix with a respective second common horizontal lowpass filter.

Clause K10. The system of any previous K Clause, wherein: a vertical highpass filter is a directional 1D highpass filter applied in a vertical direction or in a near-vertical direction.

Clause K11. The system of any previous K Clause, wherein: a horizontal lowpass filter is a directional 1D lowpass filter applied in a horizontal direction or in a near-horizontal direction.

Clause K12. The system of any previous K Clause, wherein the processor is further configured for: filtering each matrix element of the 2D matrix with a succession of at least two 1D filters, each being either the vertical highpass filter or the horizontal lowpass filter.

Clause K13. The system of any previous K Clause, wherein the processor is further configured for: enhancing the 2D decomposition by applying histogram equalization to a number of consecutive columns of the 2D matrix to generate an enhanced 2D matrix.

Clause K14. The system of any previous K Clause, wherein the processor is further configured for: computing a distance score based on dynamic time warping (DTW) between a reference column and a particular column of the enhanced 2D matrix associated with a particular time, wherein: the reference column is computed in a training session by clustering a number of training columns matched based on DTW, the training columns are computed based on training TSCI obtained from training wireless signals that are received through the wireless channel when training objects with vital signs are present and impacting the wireless channel, and when the distance score is larger than a first threshold, the periodic motion of the vital sign of the object is tentatively not detected at the particular time.

Clause K15. The system of any previous K Clause, wherein the processor is further configured for: subtracting a quantity from each matrix element in the particular column of the enhanced 2D matrix; and computing a zero-crossing count of the particular column, wherein when the zero-crossing count is larger than a second threshold, the periodic motion of the vital sign of the object is tentatively not detected at the particular time.

Clause K16. The system of any previous K Clause, wherein the processor is further configured for: computing multiple peaks of the particular column of the enhanced 2D matrix; and computing an inter-peak distance based on the multiple peaks.

Clause K17. The system of any previous K Clause, wherein: when the inter-peak distance is in an acceptable range, the periodic motion of the vital sign of the object is tentatively detected at the particular time, and the inter-peak distance is determined to be equal to a frequency or a period of the vital sign; and when the inter-peak distance is not in an acceptable range, the periodic motion of the vital sign of the object is tentatively not detected at the particular time.

Clause K18. The system of any previous K Clause, wherein the processor is further configured for: detecting the vital sign of the object when P is larger than a third threshold, wherein P is a percentage of the periodic motion of the vital sign being tentatively detected in a period of time.

Clause K19. A wireless device of a system for wireless vital sign monitoring, comprising: a processor; a memory communicatively coupled to the processor; and a receiver communicatively coupled to the processor, wherein: an additional wireless device in the system is configured to transmit a wireless signal through a wireless channel of a venue, the receiver is configured to receive the wireless signal through the wireless channel, the received wireless signal differs from the transmitted wireless signal due to the wireless channel that is impacted by a periodic motion of a vital sign of an object in the venue, and the processor is configured for: obtaining a time series of channel information (CI) of the wireless channel based on the received wireless signal, computing a two dimensional (2D) decomposition of the time series of CI (TSCI), enhancing the 2D decomposition, and monitoring the periodic motion of the vital sign based on the enhanced 2D decomposition.

Clause K20. A method for wireless vital sign monitoring, comprising: transmitting a wireless signal through a wireless channel of a venue; receiving the wireless signal through the wireless channel, wherein the received wireless signal differs from the transmitted wireless signal due to the wireless channel that is impacted by a periodic motion of a vital sign of an object in the venue; obtaining a time series of channel information (CI) of the wireless channel based on the received wireless signal; computing a two dimensional (2D) decomposition of the time series of CI (TSCI); enhancing the 2D decomposition; and monitoring the periodic motion of the vital sign based on the enhanced 2D decomposition.

The features described above may be implemented advantageously in one or more computer programs that are executable on a programmable system including at least one programmable processor coupled to receive data and instructions from, and to transmit data and instructions to, a data storage system, at least one input device, and at least one output device. A computer program is a set of instructions that may be used, directly or indirectly, in a computer to perform a certain activity or bring about a certain result. A computer program may be written in any form of programming language (e.g., C, Java), including compiled or interpreted languages, and it may be deployed in any form, including as a stand-alone program or as a module, component, subroutine, a browser-based web application, or other unit suitable for use in a computing environment.

Suitable processors for the execution of a program of instructions include, e.g., both general and special purpose microprocessors, digital signal processors, and the sole processor or one of multiple processors or cores, of any kind of computer. Generally, a processor will receive instructions and data from a read-only memory or a random access memory or both. The essential elements of a computer are a processor for executing instructions and one or more memories for storing instructions and data. Generally, a computer will also include, or be operatively coupled to communicate with, one or more mass storage devices for storing data files; such devices include magnetic disks, such as internal hard disks and removable disks; magneto-optical disks; and optical disks. Storage devices suitable for tangibly embodying computer program instructions and data include all forms of non-volatile memory, including by way of example semiconductor memory devices, such as EPROM, EEPROM, and flash memory devices; magnetic disks such as internal hard disks and removable disks; magneto-optical disks; and CD-ROM and DVD-ROM disks. The processor and the memory may be supplemented by, or incorporated in, ASICs (application-specific integrated circuits).

While the present teaching contains many specific implementation details, these should not be construed as limitations on the scope of the present teaching or of what may be claimed, but rather as descriptions of features specific to particular embodiments of the present teaching. Certain features that are described in this specification in the context of separate embodiments may also be implemented in combination in a single embodiment. Conversely, various features that are described in the context of a single embodiment may also be implemented in multiple embodiments separately or in any suitable sub-combination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the embodiments described above should not be understood as requiring such separation in all embodiments, and it should be understood that the described program components and systems may generally be integrated together in a single software product or packaged into multiple software products.

Particular embodiments of the subject matter have been described. Any combination of the features and architectures described above is intended to be within the scope of the following claims. Other embodiments are also within the scope of the following claims. In some cases, the actions recited in the claims may be performed in a different order and still achieve desirable results. In addition, the processes depicted in the accompanying figures do not necessarily require the particular order shown, or sequential order, to achieve desirable results. In certain implementations, multitasking and parallel processing may be advantageous.

## Claims

**1.** A system for radio-based sleep tracking, comprising:

a transmitter configured to transmit a first wireless signal through a wireless multipath channel in a venue;
a receiver configured to receive a second wireless signal through the wireless multipath channel, wherein the second wireless signal differs from the first wireless signal due to the wireless multipath channel which is impacted by a sleeping motion of an object in the venue; and
a processor configured for:

obtaining a time series of channel information (TSCI) of the wireless multipath channel based on the second wireless signal, wherein each channel information (CI) of the TSCI comprises N1 components, wherein N1 is a positive integer larger than one,
computing N1 component-wise analytics each associated with one of the N1 components of the TSCI,
identifying N2 largest component-wise analytics among the N1 component-wise analytics, wherein N2 is a positive integer less than N1,
computing at least one first motion statistics based on the N2 largest component-wise analytics of the TSCI, and
monitoring the sleeping motion of the object based on the at least one first motion statistics.

**2.** The system of claim 1, wherein the N2 largest component-wise analytics are identified by one of the following:

comparing each of the N1 component-wise analytics to a threshold; or
sorting the N1 component-wise analytics to find the N2 largest component-wise analytics.

**3.** The system of claim 1 or 2, wherein the processor is further configured for:

computing a time series of first motion statistics, each first motion statistics associated with a time stamp;
computing a set of potential body-motion-during sleep (BMS), each potential BMS (PBMS) being a local maximum point or a local peak of the time series of first motion statistics; and
monitoring the sleeping motion of the object based on the set of PBMS.

**4.** The system of claim 3, wherein the processor is further configured for:

performing a BMS test on each PBMS in the set of PBMS and the time series of first motion statistics; and
removing a PBMS from the set of PBMS when the PBMS fails the BMS test, wherein:

the BMS test is performed based on a magnitude feature of each PBMS, wherein the PBMS fails the BMS test when the local peak of a first motion statistics associated of the PBMS has the magnitude feature less than a first threshold, and
the first threshold is computed adaptively based on at least one of: a weighted mean of the magnitude feature of a number of largest first motion statistics in a period of time and a predefined quantity.

**5.** The system of claim 4, wherein:

the BMS test is performed based on a width measure associated with each PBMS, wherein the PBMS fails the BMS test when the width measure is larger than a second threshold; and
the width measure is computed based on:

computing a left drop point which is a nearest point to the left of PBMS in the time series of first motion statistics when the first motion statistics has a magnitude feature below a first target value,
computing a right drop point which is a nearest point to the right of PBMS in the time series of first motion statistics when the first motion statistics has a magnitude feature below a second target value, and
computing the width measure as a time difference between the left drop point and the right drop point.

**6.** The system of claim 5, wherein:

at least one of the first target value or the second target value is an adaptive threshold based on a peak magnitude feature of the first motion statistics at the PBMS; and
the BMS test is performed based on a height measure associated with each PBMS, wherein the PBMS fails the BMS test when the height measure is less than a third threshold.

**7.** The system of claim 6, wherein the height measure is computed based on:

computing at least one of: a left minimum point or a right minimum point, wherein the left minimum point is a first adjacent minimum point to the left of the PBMS in the time series of first motion statistics, wherein the right minimum point is a first adjacent minimum point to the right of the PBMS in the time series of first motion statistics; and
computing the height measure based on at least one of:

a difference of a first magnitude feature of the first motion statistics between the PBMS and the left minimum point,
a difference of the first magnitude feature of the first motion statistics between the PBMS and the right minimum point,
a quotient of a second magnitude feature of the first motion statistics between the PBMS and the left minimum point,
a quotient of the second magnitude feature of the first motion statistics between the PBMS and the right minimum point.

**8.** The system of claim 6 or 7, wherein the processor is further configured for:

computing a prominence measure based on an increasing function of the height measure and a decreasing function of the width measure, wherein the PBMS fails the BMS test when the prominence measure is less than a fourth threshold;
computing a left dropping point which is a point to the left of PBMS in the time series of first motion statistics at a first time difference from the PBMS;
computing a left boundary point which is a point to the left of PBMS in the time series of first motion statistics at a second time difference from the PBMS;
computing a right dropping point which is a point to the right of PBMS in the time series of first motion statistics at a third time difference from the PBMS;
computing a right boundary point which is a point to the right of PBMS in the time series of first motion statistics at a fourth time difference from the PBMS; and
performing the BMS test based on a neighborhood dominance measure computed based on the left dropping point, the left boundary point, the right dropping point and the right boundary point, wherein the PBMS fails the BMS test when the neighborhood dominance measure is larger than a fifth threshold.

**9.** The system of claims 4 to 8, wherein the processor is further configured for:

detecting a presence of the object in a time period around the PBMS based on a first motion statistics in the time period;
performing the BMS test based on the detecting, wherein the PBMS fails the BMS test when presence of the object is not detected in the time period; and
detecting a motion of the object at each time in the time period based on the first motion statistics,
wherein the presence of the object is detected in the time period when the motion of the object is detected at any time during the time period,
wherein the motion of the object is detected at a time when the first motion statistics at that time is larger than a sixth threshold or a second motion statistics at that time computed based on the TSCI is larger than a seventh threshold.

**10.** The system of claims 4 to 9, wherein the processor is further configured for:

detecting a non-sleeping activity of the object in a time period around the PBMS based on the first motion statistics in the time period, wherein the non-sleeping activity of the object is detected in the time period when a percentage of time in the time period at which the motion of the object is detected is larger than an eighth threshold; and
performing the BMS test based on the non-sleeping activity detection of the object, wherein the PBMS fails the BMS test when non-sleeping activity of the object is detected in the time period.

**11.** The system of claim 4 to 10, wherein the processor is further configured for:

performing the BMS test based on a time difference between the PBMS and a neighboring PBMS, wherein the PBMS fails the BMS test when the time difference is less than a ninth threshold; and
merging the PBMS and the neighboring PBMS when the time difference is less than a tenth threshold.

**12.** The system of any previous claim, wherein the processor is further configured for:

computing a time series of sleep likelihood (TSSL) for a period of time, each sleep likelihood (SL) associated with a time, wherein each SL is computed based on a motion intensity at the time and a count of PBMS in a period of time associated with the time;
computing a time series of sleep indicator (TSSI) for the period of time, each sleep indicator (SI) is computed based on a comparison of a respective SL with a threshold; and
monitoring the sleep motion of the object based on the TSSL and the TSSI.

**13.** The system of any previous claim, wherein the processor is further configured for:

computing a time series of testing score (TS) for the period of time, each TS based on the SL, the SI and a penalty for large motion intensity;
partitioning the period of time into a number of non-overlapping time units;
computing a sum of test scores for each time unit;
among the non-overlapping time units, identifying a time unit with the largest sum of test scores, initializing a sleep period as the time unit, and initializing a total testing score (TTS) as the associated largest sum of test scores;
iteratively expanding the sleep period by adding an adjoining incremental time window either to the right or to the left of the sleep period and iteratively updating the TTS by adding the TS associated with the increment time window to TTS;
in each iteration, adding a penalty to the TTS when the sleep period has a duration approaching or exceeding a typical sleep duration of the object;
stopping the iteration based on a stopping criterion; and
monitoring the sleeping motion of the object based on the sleep period, the TTS, the TS, the PBMS and the first motion statistics in the sleep period.

**14.** The system of claim 13, wherein the processor is further configured for:
computing a sleep analytics based on the sleep period, the TTS, the TS, the PBMS and the first motion statistics in the sleep period, wherein:

each of the N1 component-wise analytics is a pair-wise analytics based on a pair of CI of the TCSI, and

each of the N1 component-wise analytics is computed based on respective components of the pair of CI of the TSCI.

**15.** A method for radio-based sleep tracking, comprising:

transmitting a first wireless signal through a wireless multipath channel in a venue;
receiving a second wireless signal through the wireless multipath channel, wherein the second wireless signal differs from the first wireless signal due to the wireless multipath channel which is impacted by a sleeping motion of an object in the venue;
obtaining a time series of channel information (TSCI) of the wireless multipath channel based on the second wireless signal, wherein each channel information (CI) of the TSCI comprises N1 components, wherein N1 is a positive integer larger than one;
computing N1 component-wise analytics each associated with one of the N1 components of the TSCI;
identifying N2 largest component-wise analytics among the N1 component-wise analytics, wherein N2 is a positive integer less than N1;
computing at least one first motion statistics based on the N2 largest component-wise analytics of the TSCI; and
monitoring the sleeping motion of the object based on the at least one first motion statistics.

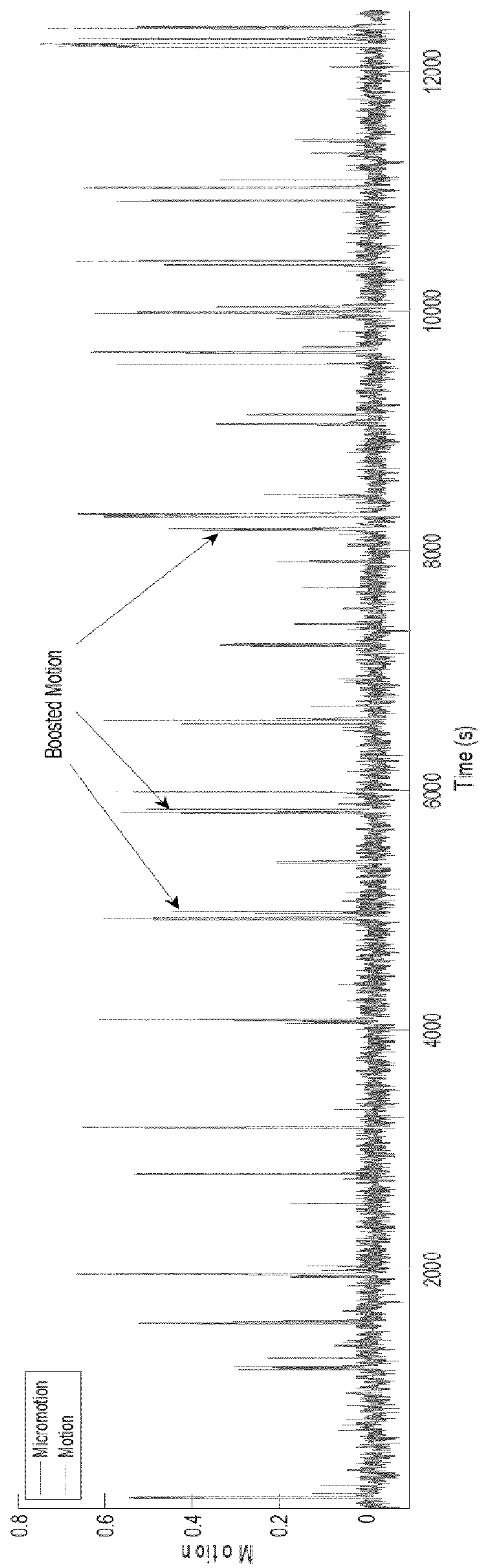

Micromotion
Motion
Boosted Motion
Motion
Time (s)
0
0.2
0.4
0.6
0.8
2000
4000
6000
8000
10000
12000


FIG. 1

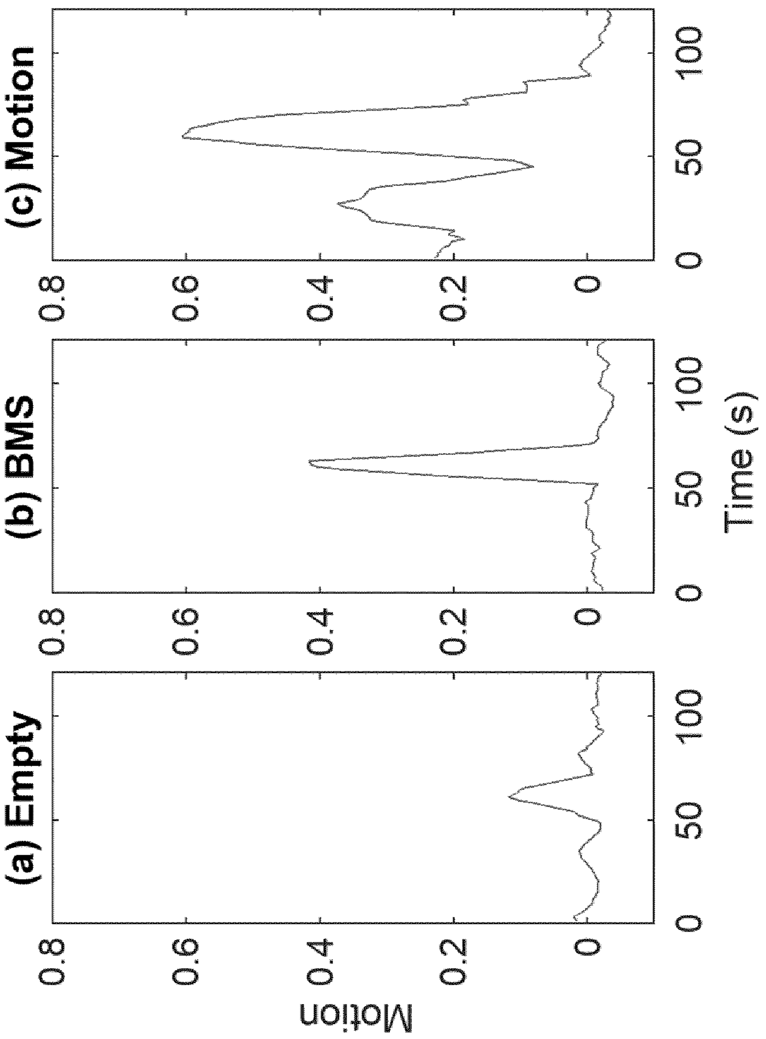


FIG. 2

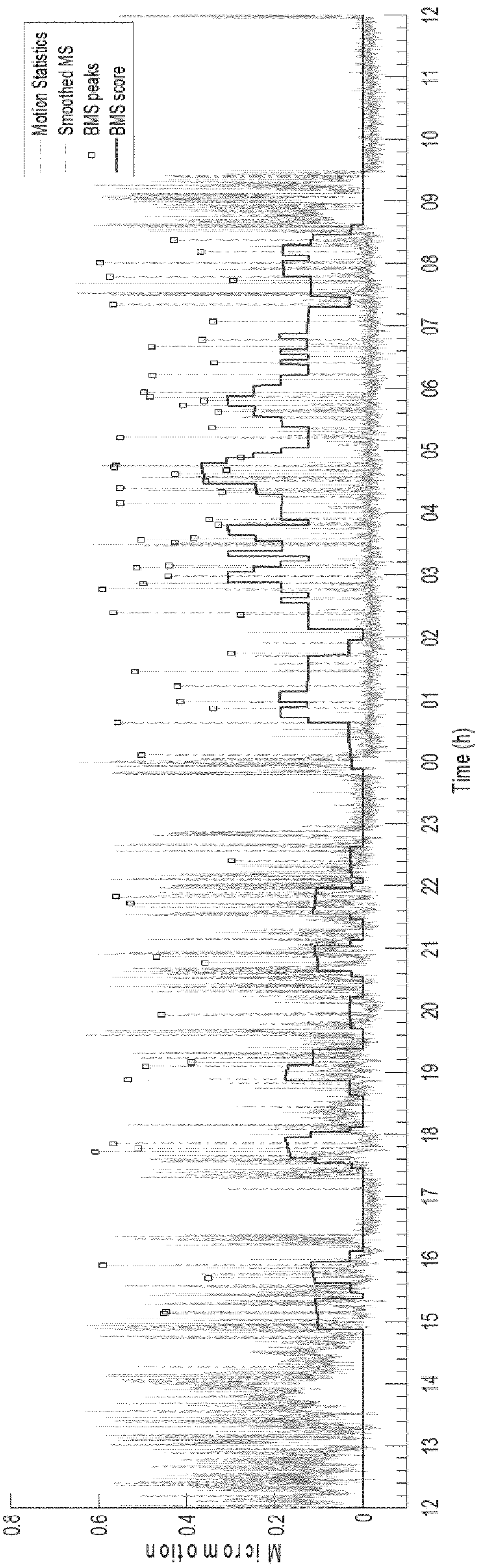
Motion Statistics
Smoothed MS
BMS peaks
BMS score
Micromotion
0.8
0.6
0.4
0.2
0
Time (h)
12 13 14 15 16 17 18 19 20 21 22 23 00 01 02 03 04 05 06 07 08 09 10 11 12

FIG. 3

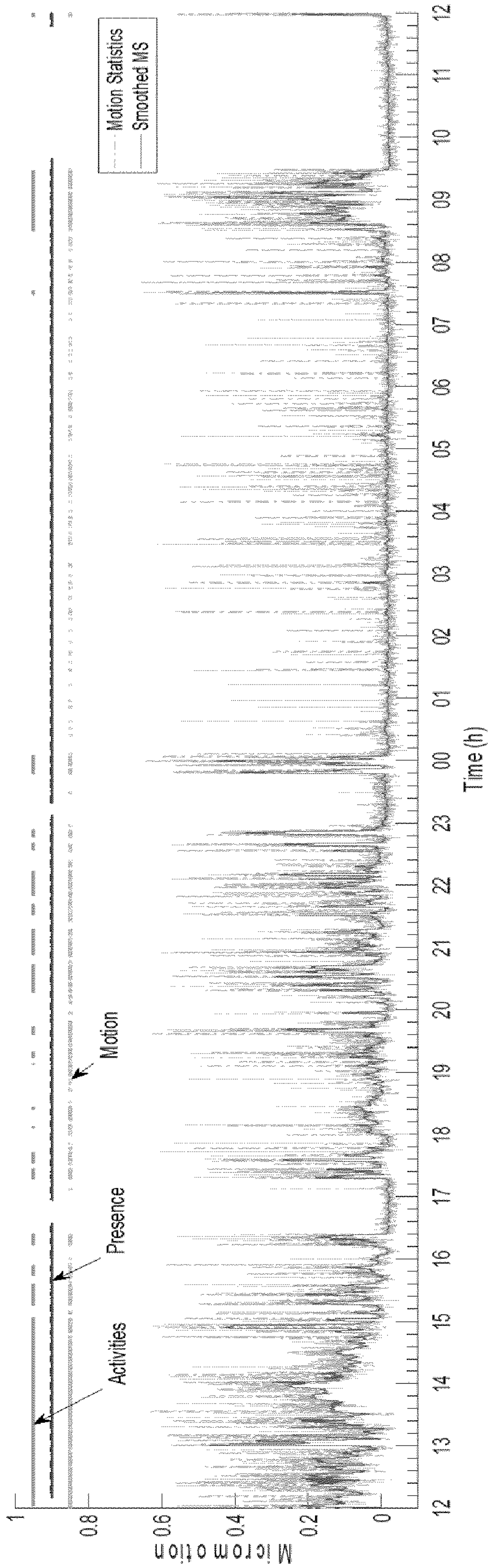
Motion Statistics
Smoothed MS
Motion
Presence
Activities
Micromotion
Time (h)

FIG. 4

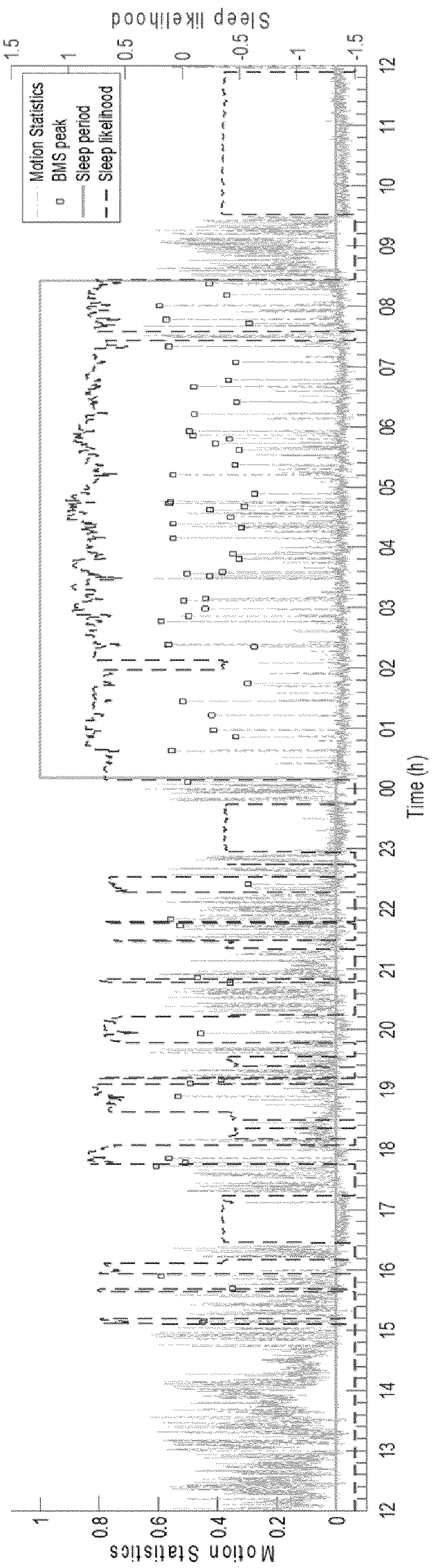

Motion Statistics
BMS peak
Sleep period
Sleep likelihood
Motion Statistics
Sleep likelihood
Time (h)
12
13
14
15
16
17
18
19
20
21
22
23
00
01
02
03
04
05
06
07
08
09
10
11
12
0
0.2
0.4
0.6
0.8
1
-1.5
-1
-0.5
0
0.5
1
1.5


FIG. 5

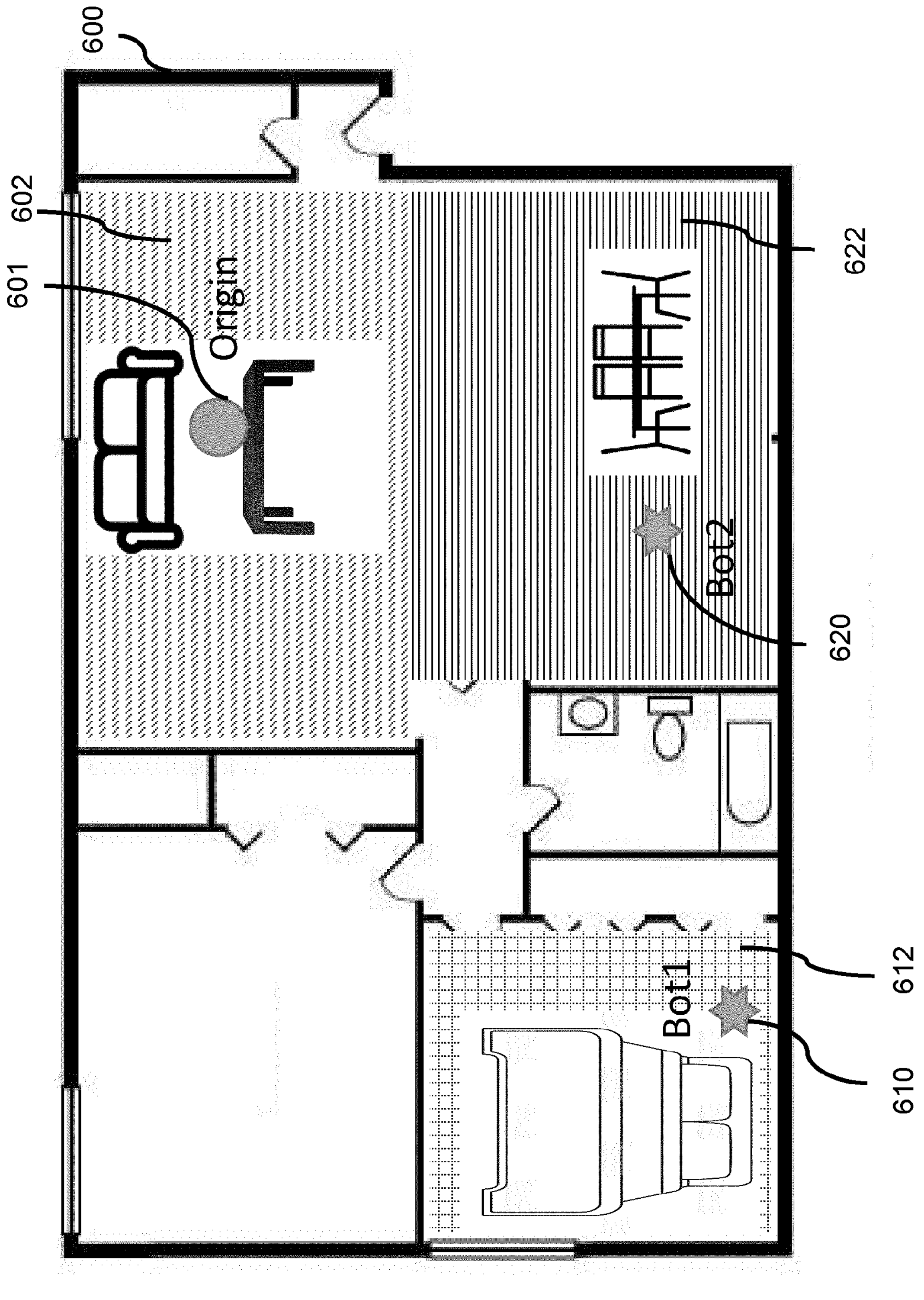


FIG. 6

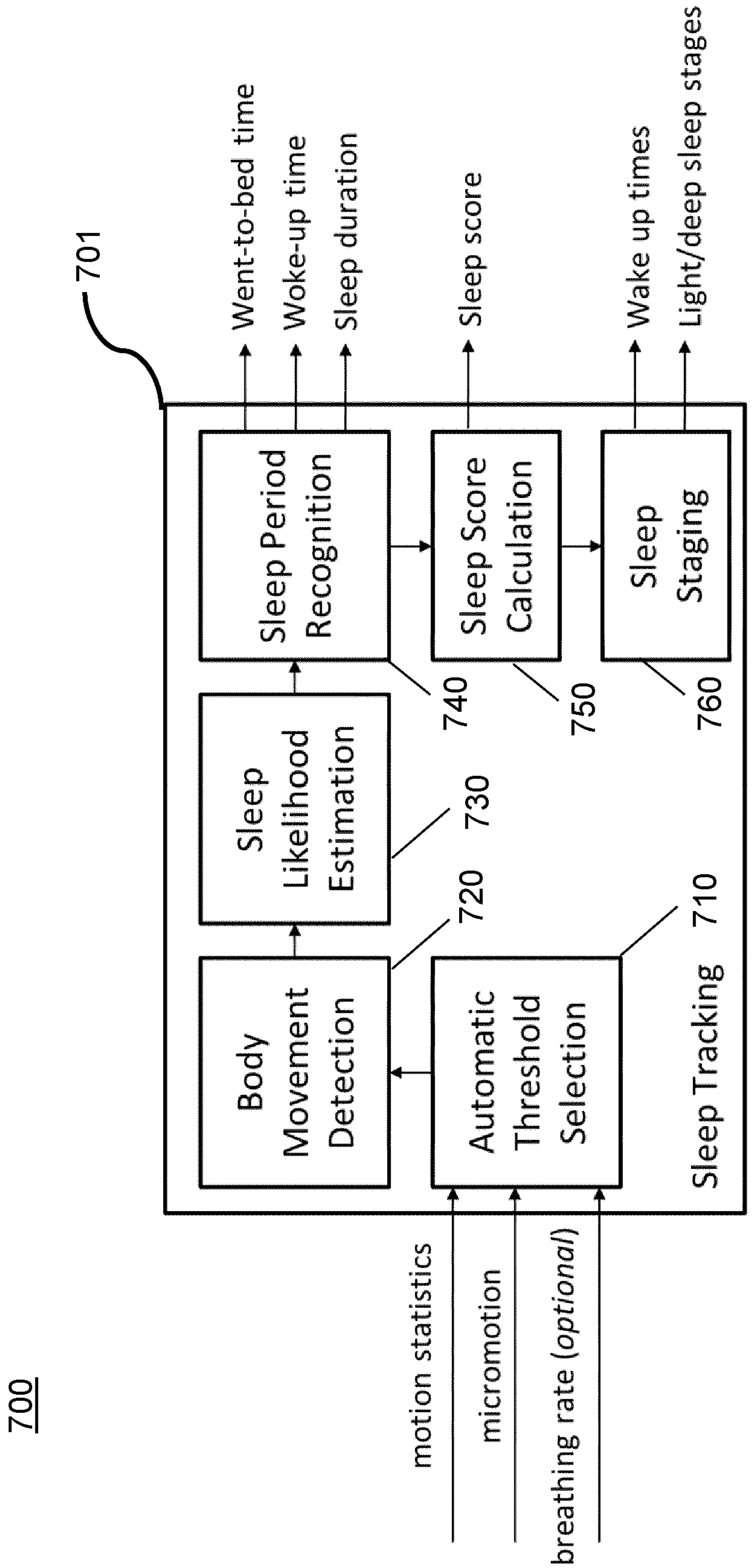
700
701
Went-to-bed time
Woke-up time
Sleep duration
Sleep score
Wake up times
Light/deep sleep stages
Sleep Period Recognition
Sleep Score Calculation
Sleep Staging
740
750
760
Sleep Likelihood Estimation
730
Body Movement Detection
720
Automatic Threshold Selection
710
Sleep Tracking
motion statistics
micromotion
breathing rate (*optional*)

FIG. 7

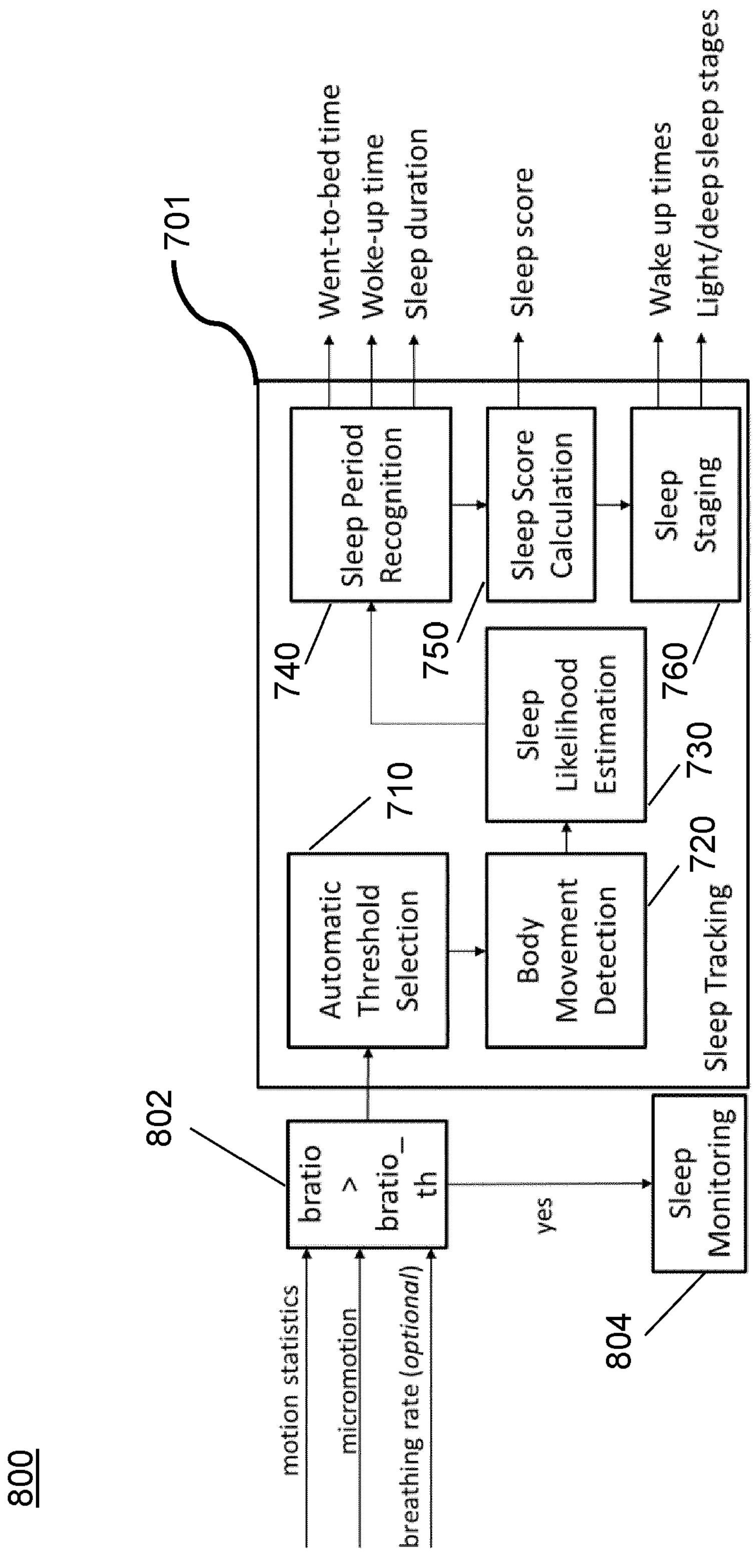
800
motion statistics
micromotion
breathing rate (optional)
bratio > bratio_th
802
yes
Sleep Monitoring
804
Automatic Threshold Selection
710
Body Movement Detection
720
Sleep Likelihood Estimation
730
Sleep Tracking
Sleep Period Recognition
740
Sleep Score Calculation
750
Sleep Staging
760
701
Went-to-bed time
Woke-up time
Sleep duration
Sleep score
Wake up times
Light/deep sleep stages

FIG. 8

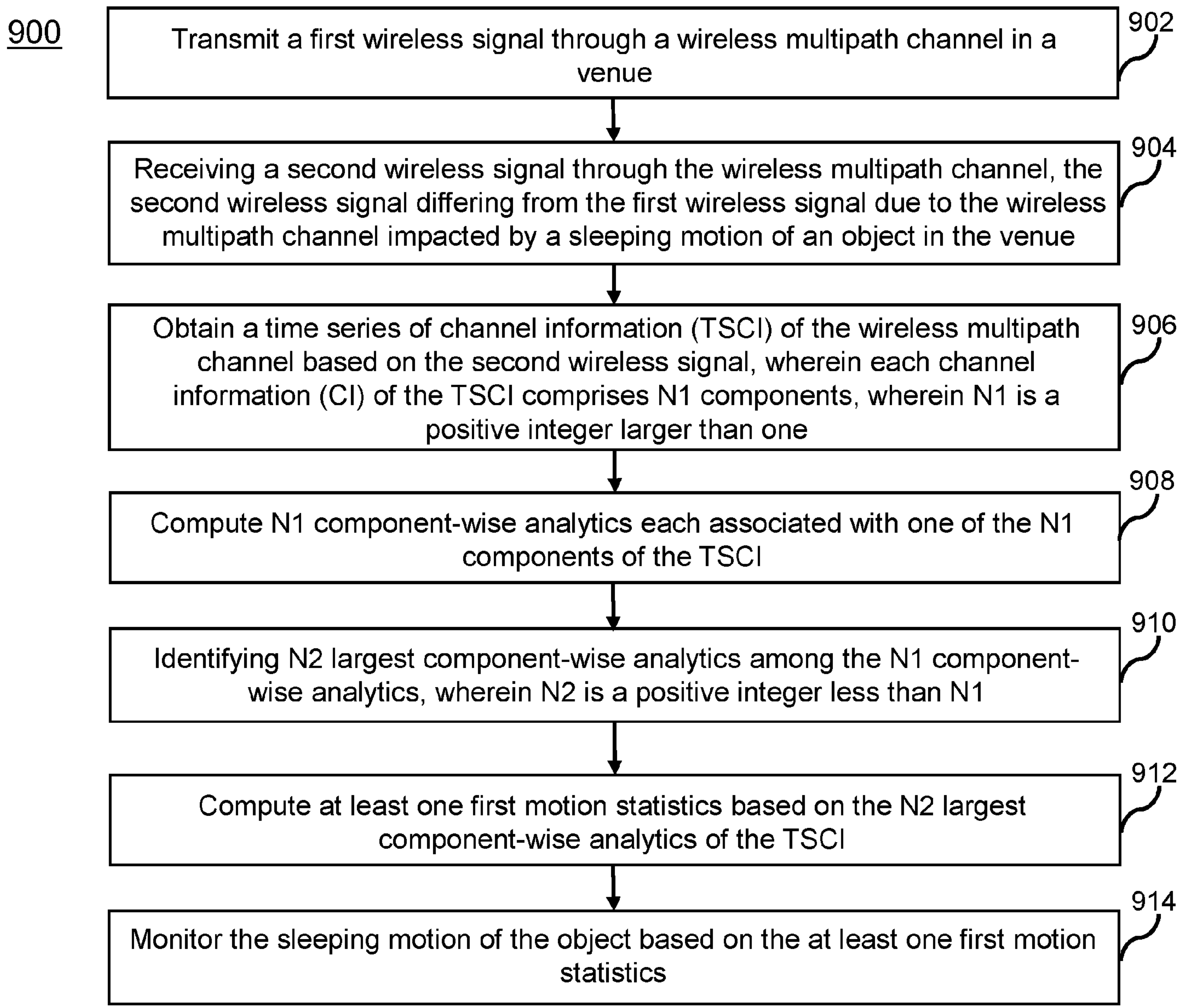
900
Transmit a first wireless signal through a wireless multipath channel in a venue
902
Receiving a second wireless signal through the wireless multipath channel, the second wireless signal differing from the first wireless signal due to the wireless multipath channel impacted by a sleeping motion of an object in the venue
904
Obtain a time series of channel information (TSCI) of the wireless multipath channel based on the second wireless signal, wherein each channel information (CI) of the TSCI comprises N1 components, wherein N1 is a positive integer larger than one
906
Compute N1 component-wise analytics each associated with one of the N1 components of the TSCI
908
Identifying N2 largest component-wise analytics among the N1 component-wise analytics, wherein N2 is a positive integer less than N1
910
Compute at least one first motion statistics based on the N2 largest component-wise analytics of the TSCI
912
Monitor the sleeping motion of the object based on the at least one first motion statistics
914

FIG. 9

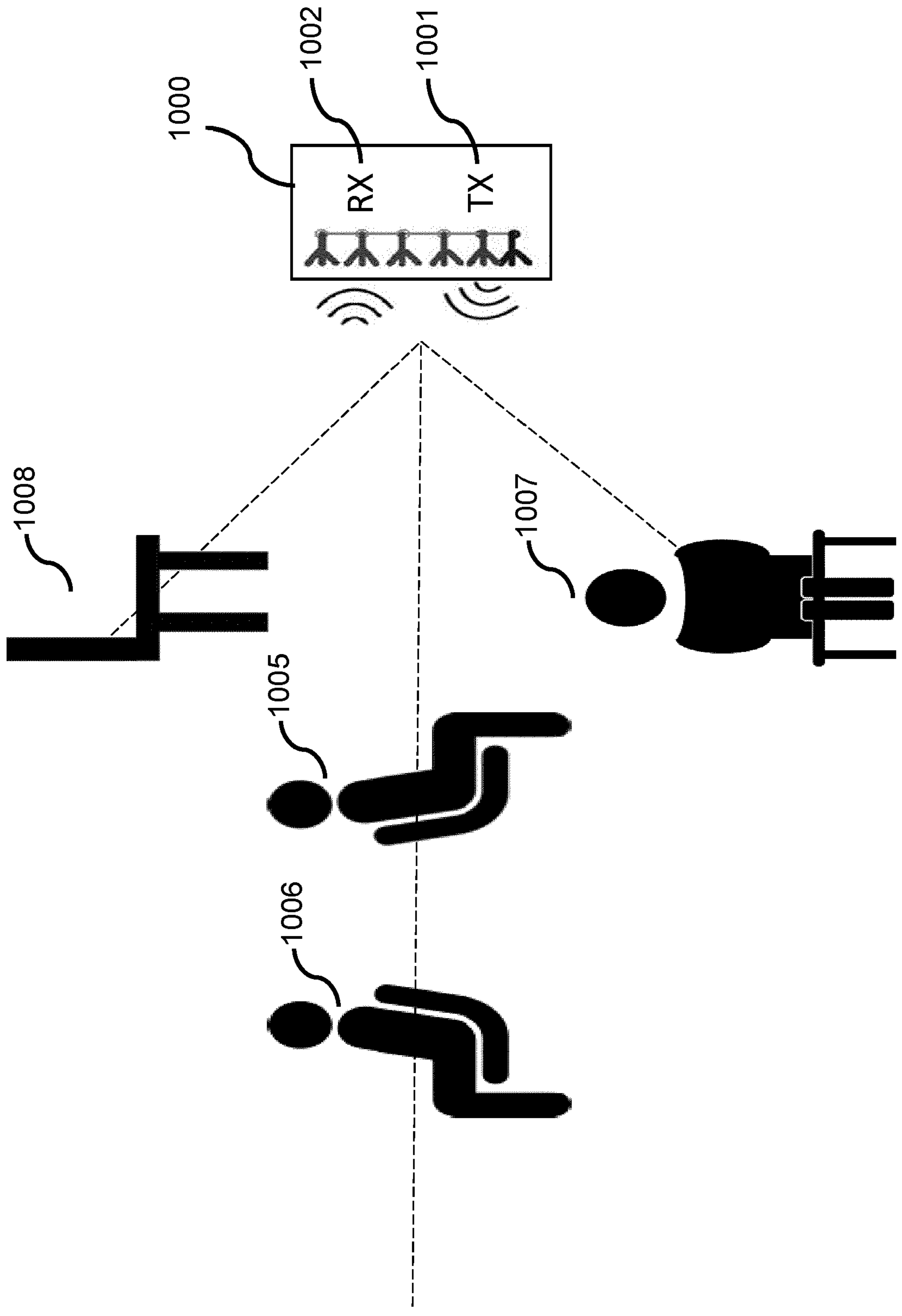


FIG. 10A

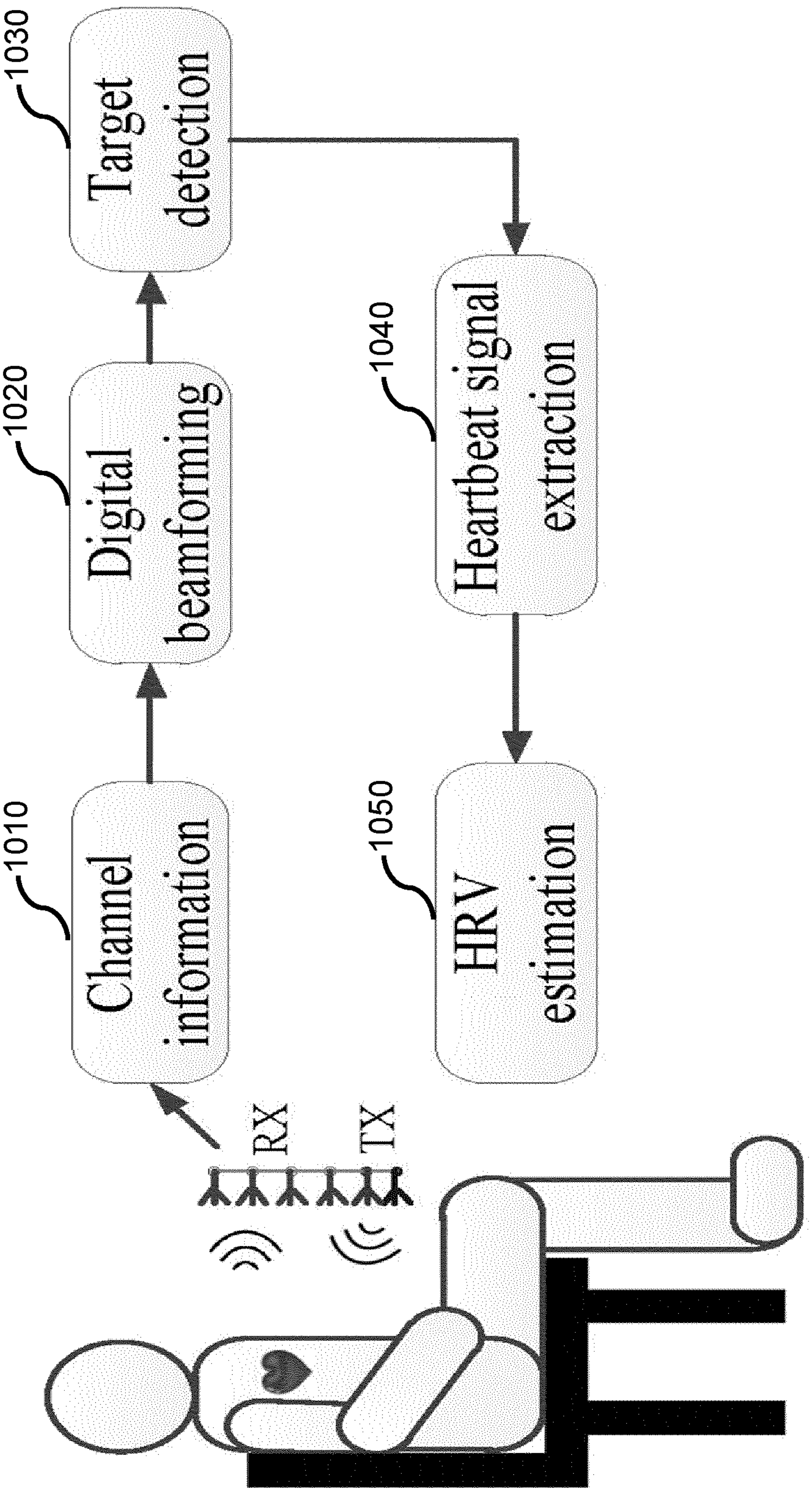
1010
Channel information
1020
Digital beamforming
1030
Target detection
1040
Heartbeat signal extraction
1050
HRV estimation
RX
TX

FIG. 10B

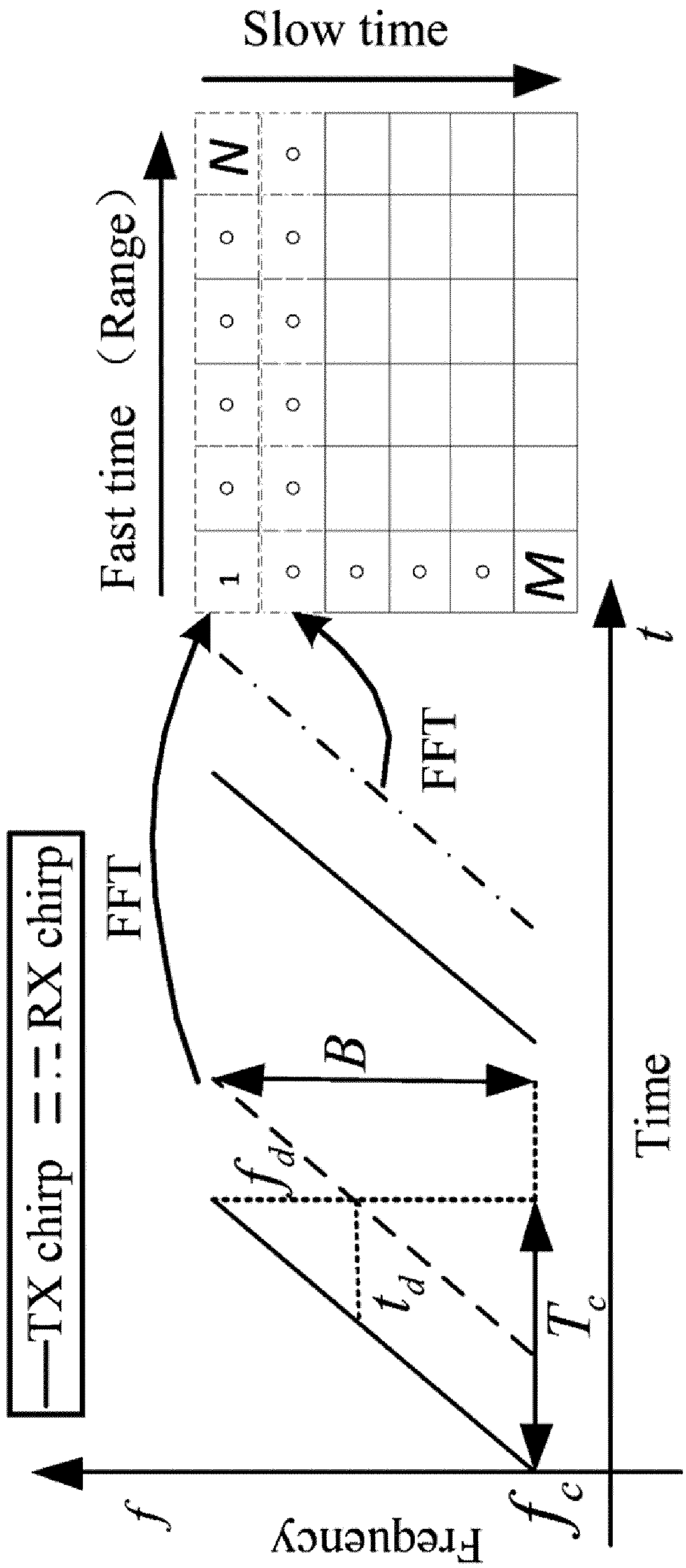
Slow time
Fast time (Range)
N
1
M
TX chirp
RX chirp
FFT
FFT
B
$f_d$
$t_d$
$T_c$
$f_c$
f
Frequency
Time
t

FIG. 11

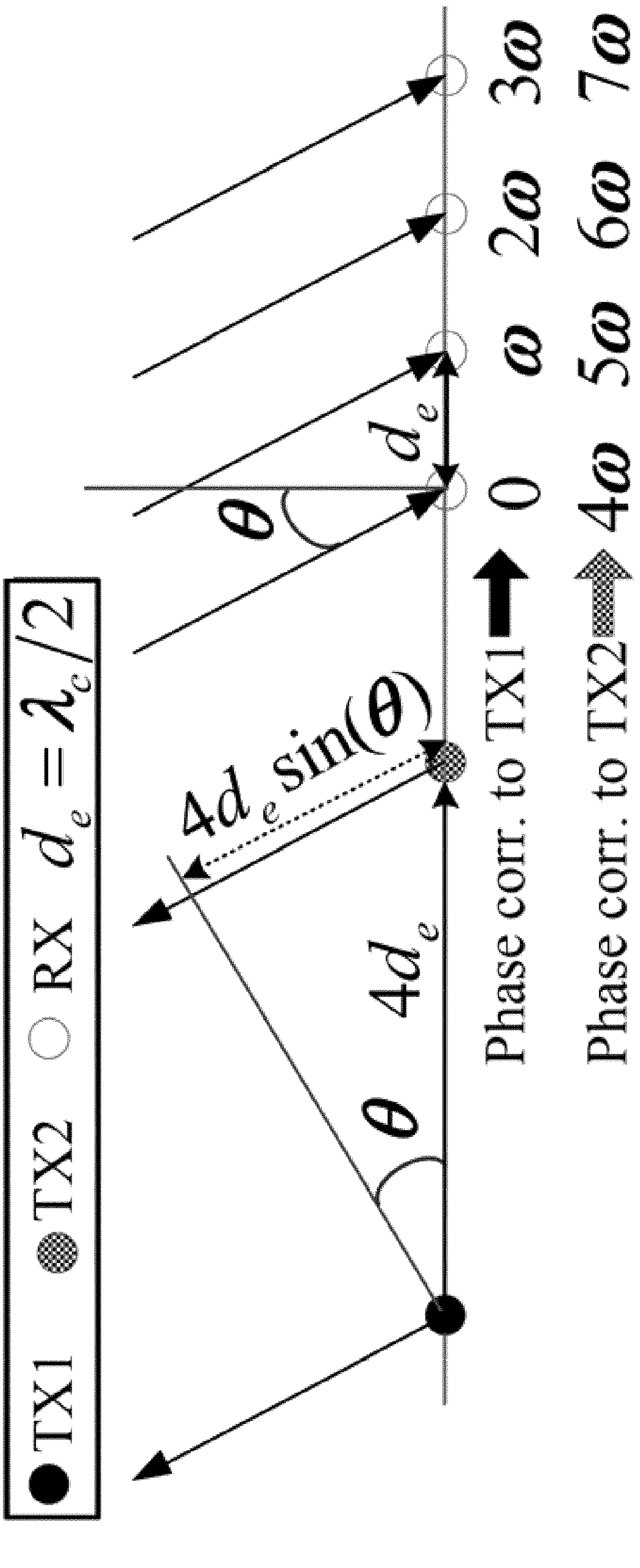
TX1
TX2
RX
$d_e = \lambda_c/2$
$4d_e$
$4d_e \sin(\theta)$
$\theta$
$d_e$
0
$\omega$
$2\omega$
$3\omega$
$4\omega$
$5\omega$
$6\omega$
$7\omega$
Phase corr. to TX1
Phase corr. to TX2

FIG. 12

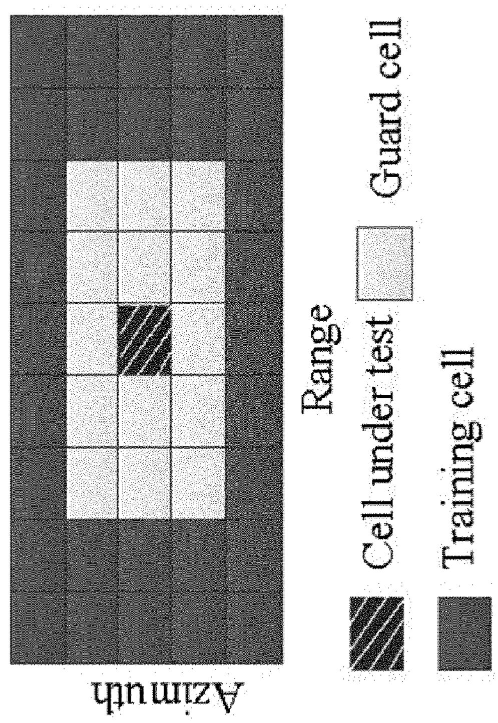


FIG. 13A

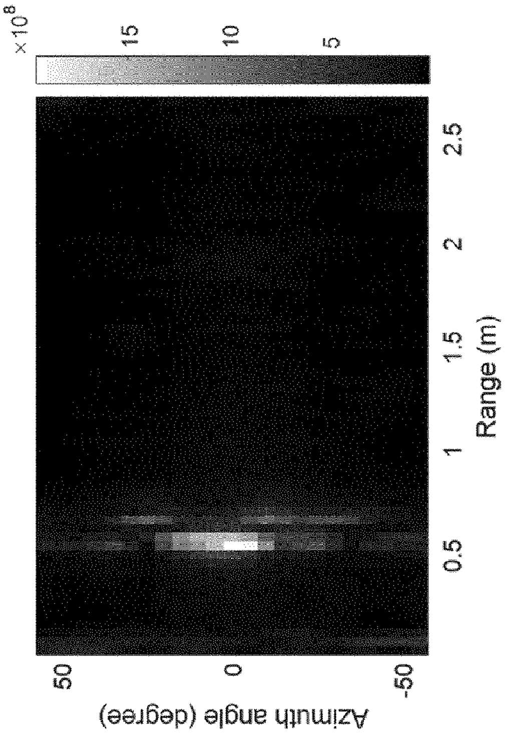


FIG. 13B

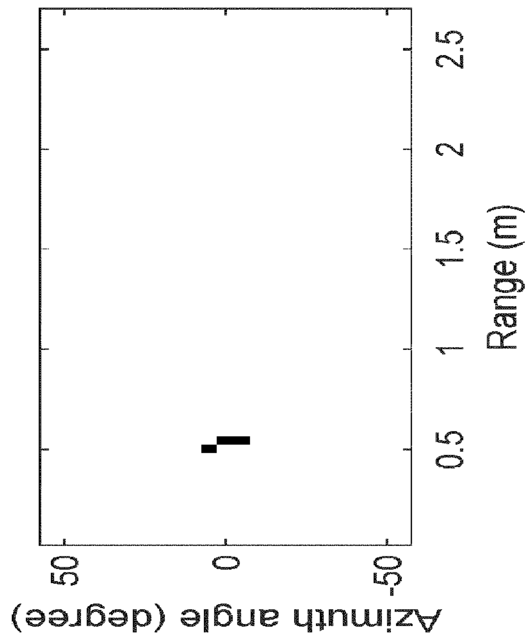


FIG. 13C

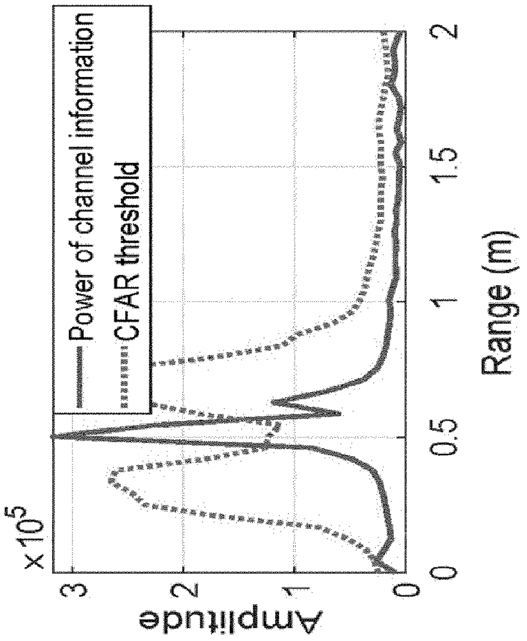


FIG. 13D

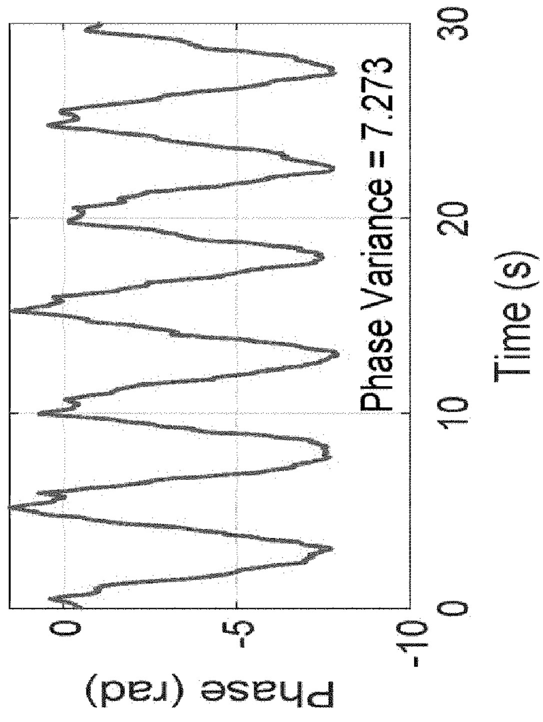


FIG. 14A

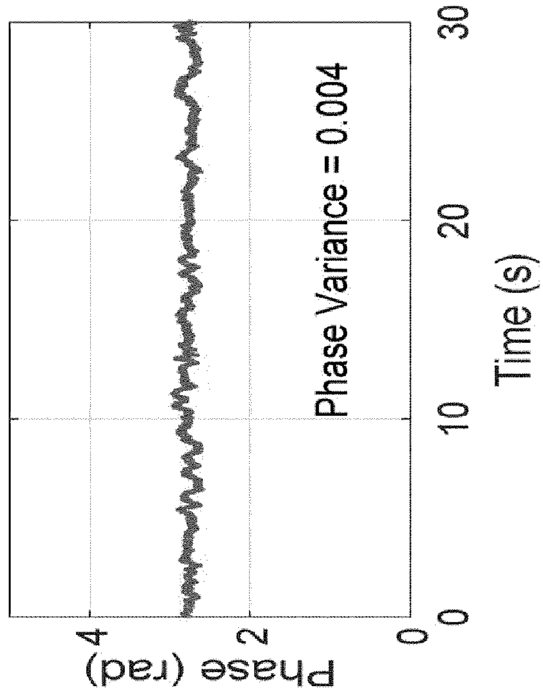


FIG. 14B

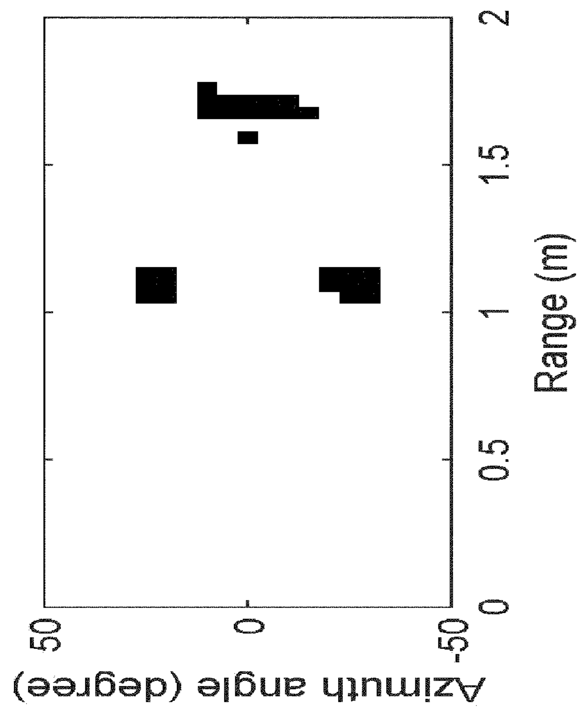


FIG. 14C

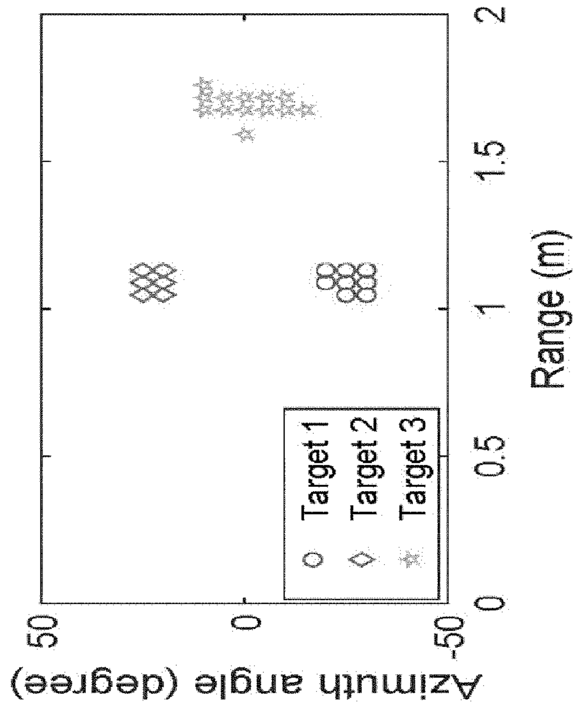


FIG. 14D

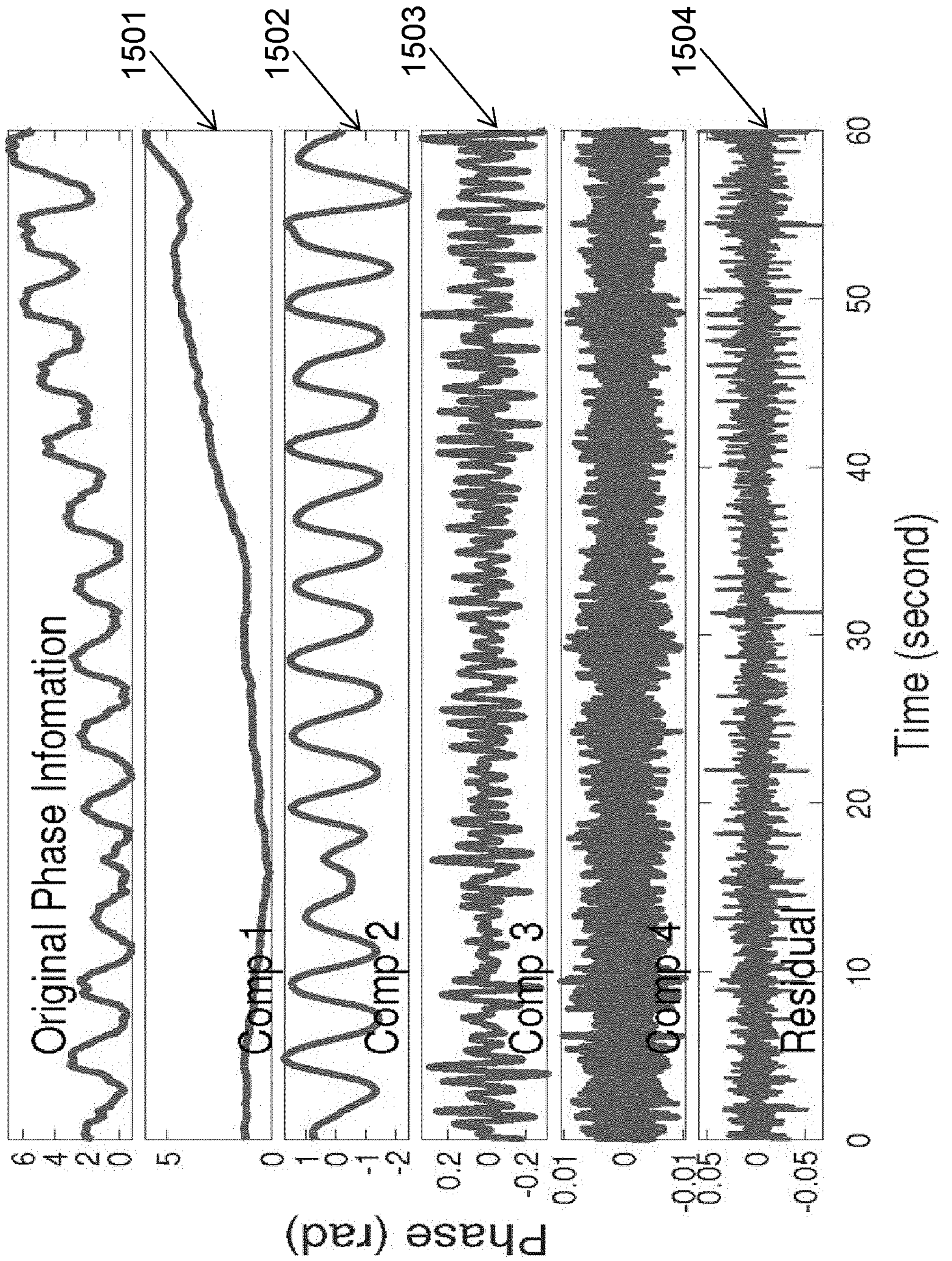

Original Phase Infomation
Comp 1
Comp 2
Comp 3
Comp 4
Residual
1501
1502
1503
1504
Phase (rad)
Time (second)
6
4
2
0
5
0
1
0
-1
-2
0.2
0
-0.2
0.01
0
-0.01
0.05
0
-0.05
10
20
30
40
50
60


FIG. 15A

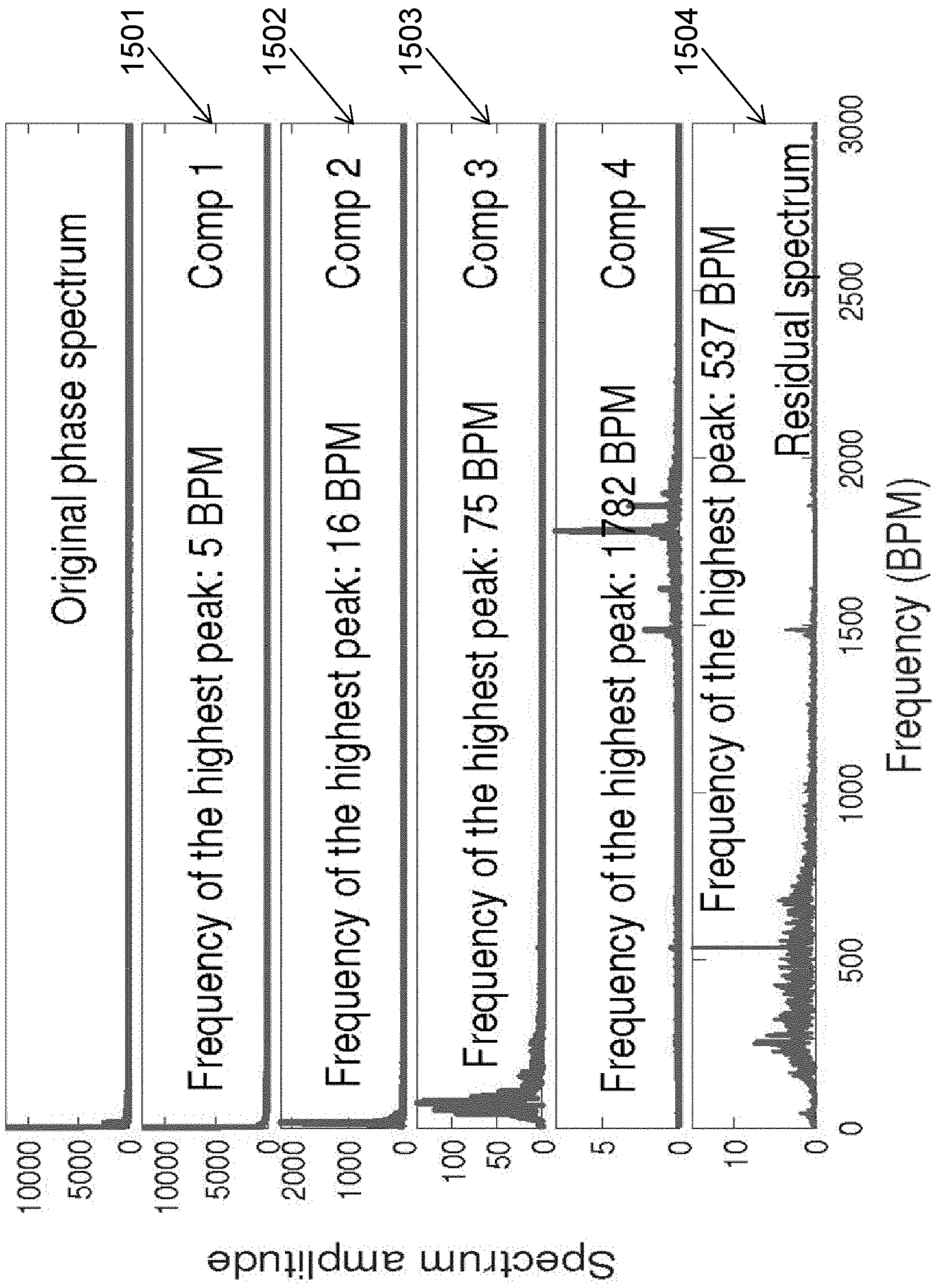
1501
1502
1503
1504
Original phase spectrum
Frequency of the highest peak: 5 BPM
Comp 1
Frequency of the highest peak: 16 BPM
Comp 2
Frequency of the highest peak: 75 BPM
Comp 3
Frequency of the highest peak: 1782 BPM
Comp 4
Frequency of the highest peak: 537 BPM
Residual spectrum
Spectrum amplitude
Frequency (BPM)
0
500
1000
1500
2000
2500
3000
10000
5000
2000
1000
100
50
10
5

FIG. 15B

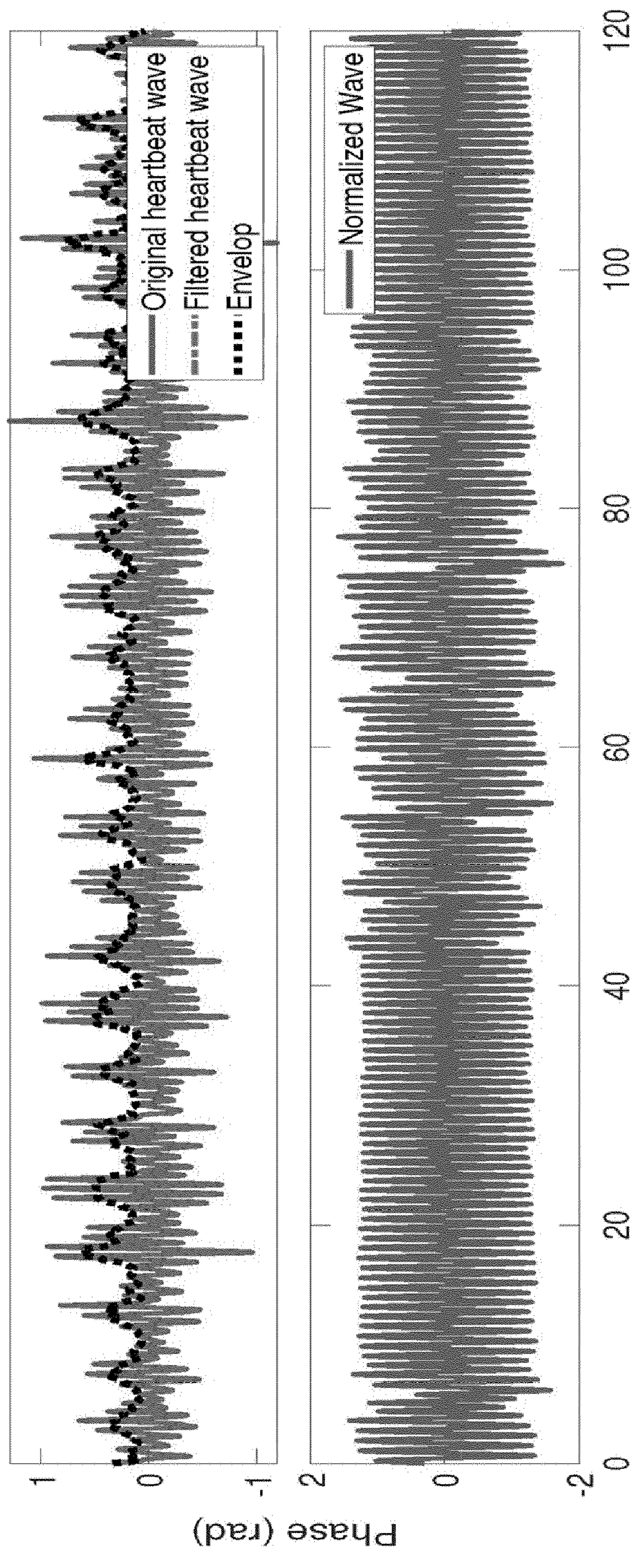
Original heartbeat wave
Filtered heartbeat wave
Envelop
Normalized Wave
Phase (rad)
1
0
-1
2
0
-2
0
20
40
60
80
100
120

FIG. 16A

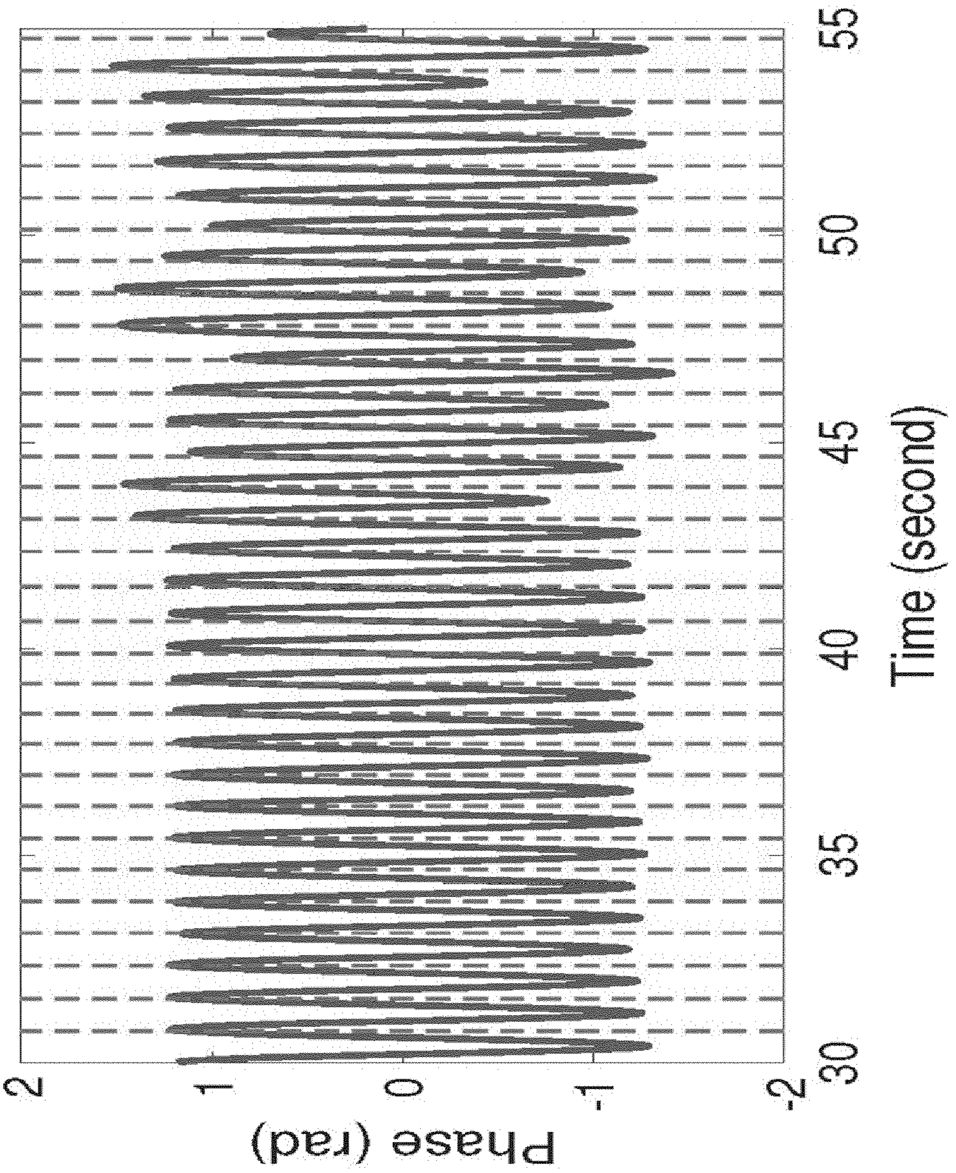


FIG. 16B

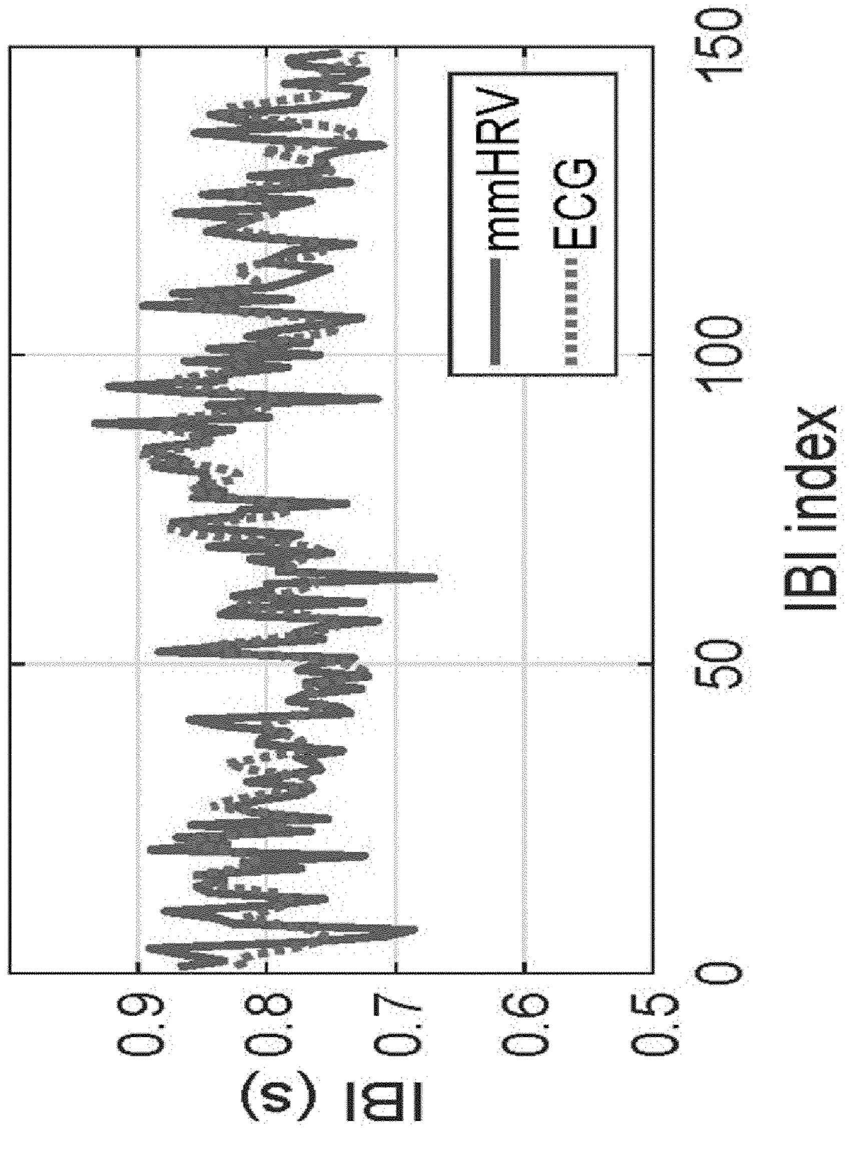


FIG. 16C

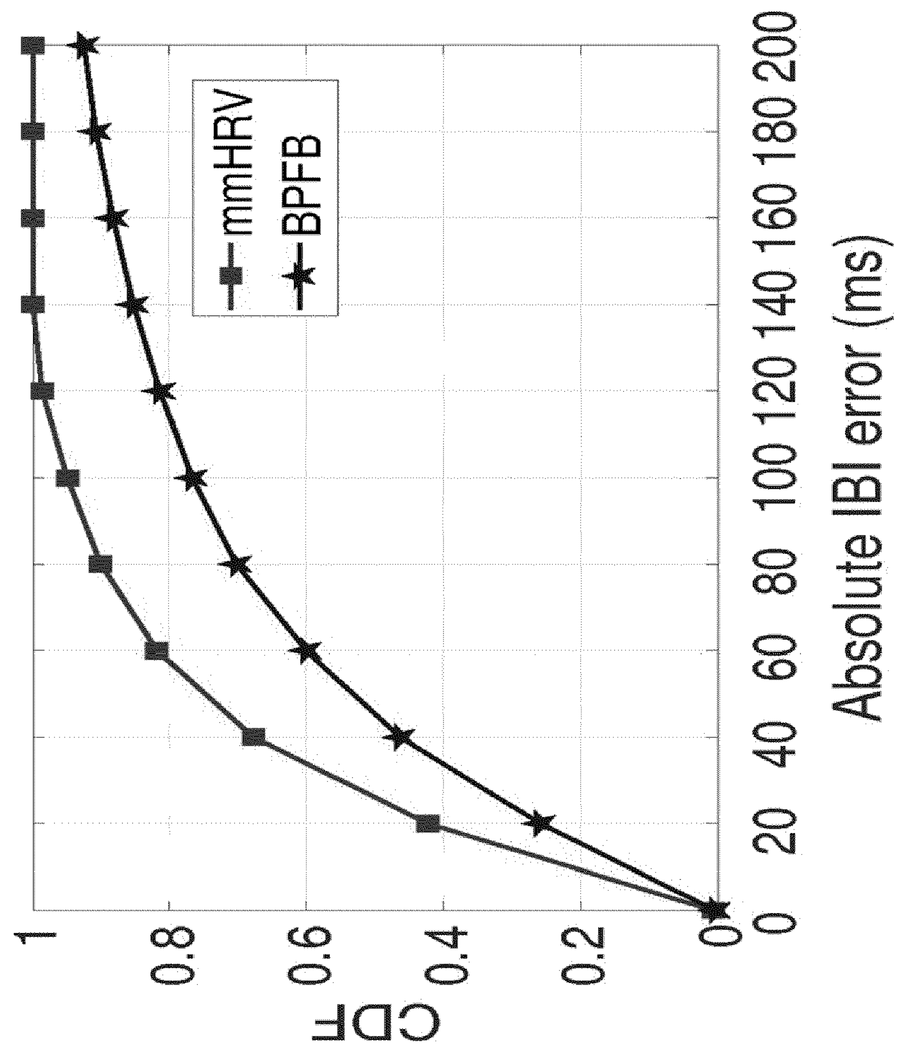


FIG. 17B

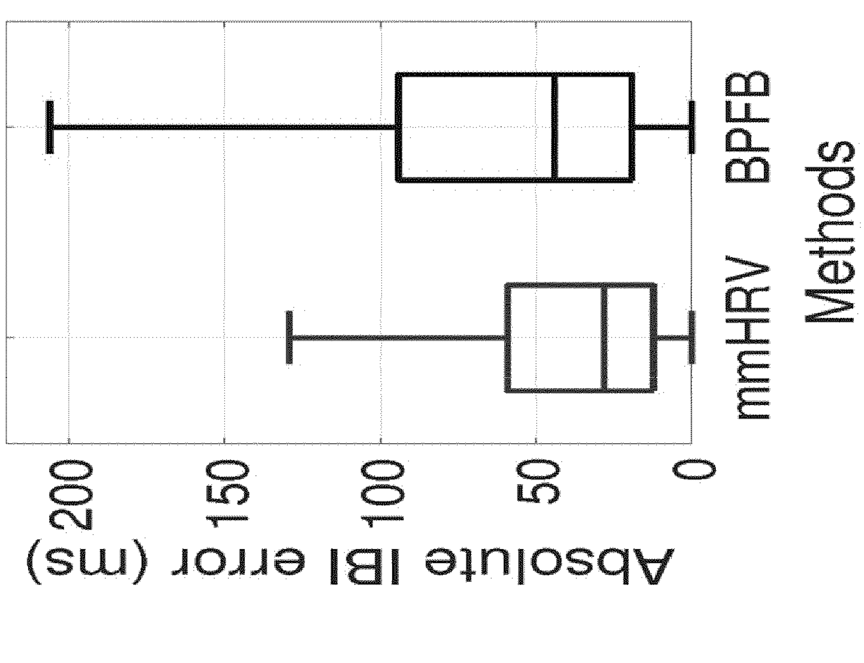


FIG. 17A

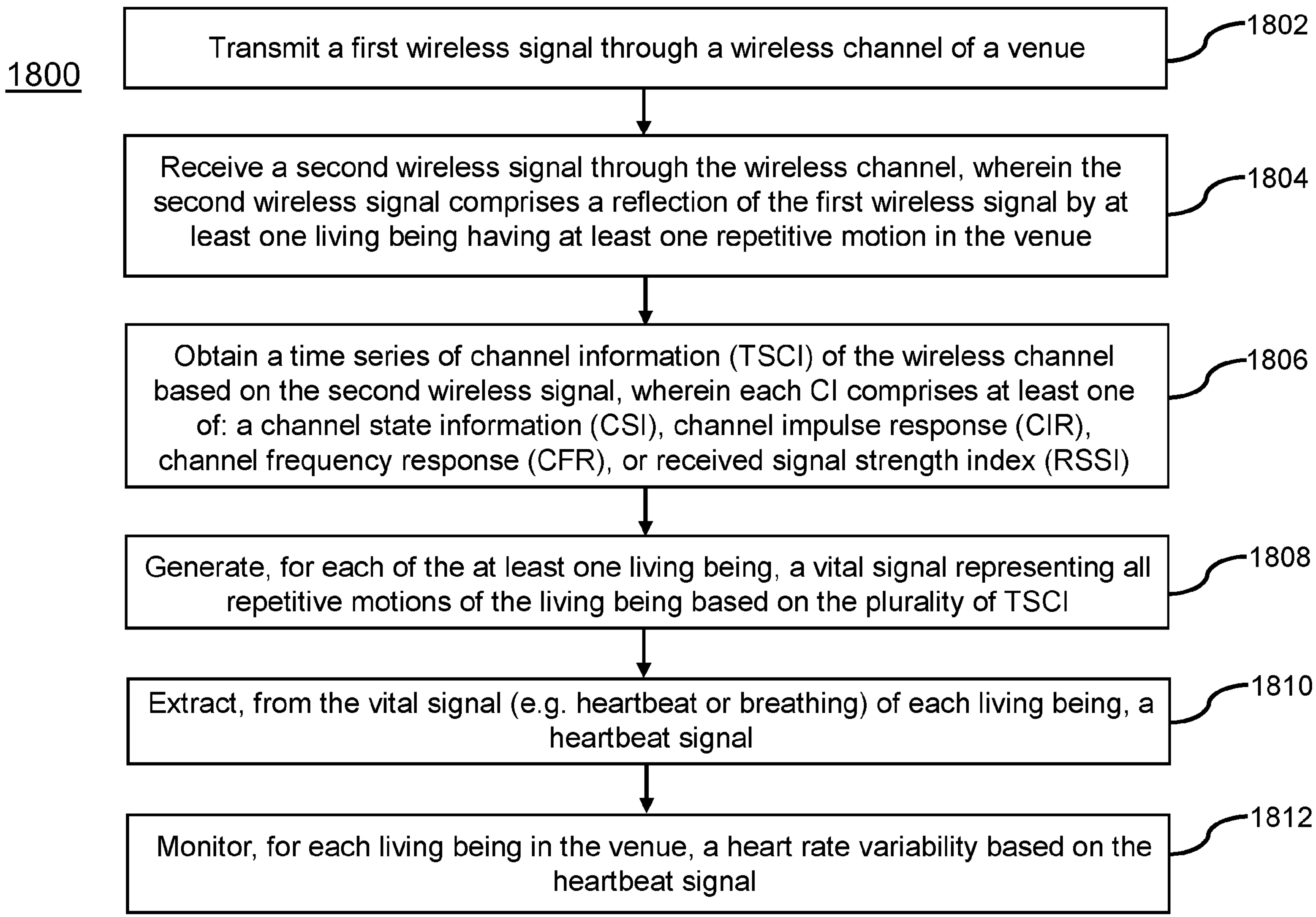
1800
Transmit a first wireless signal through a wireless channel of a venue
1802
Receive a second wireless signal through the wireless channel, wherein the second wireless signal comprises a reflection of the first wireless signal by at least one living being having at least one repetitive motion in the venue
1804
Obtain a time series of channel information (TSCI) of the wireless channel based on the second wireless signal, wherein each CI comprises at least one of: a channel state information (CSI), channel impulse response (CIR), channel frequency response (CFR), or received signal strength index (RSSI)
1806
Generate, for each of the at least one living being, a vital signal representing all repetitive motions of the living being based on the plurality of TSCI
1808
Extract, from the vital signal (e.g. heartbeat or breathing) of each living being, a heartbeat signal
1810
Monitor, for each living being in the venue, a heart rate variability based on the heartbeat signal
1812

FIG. 18

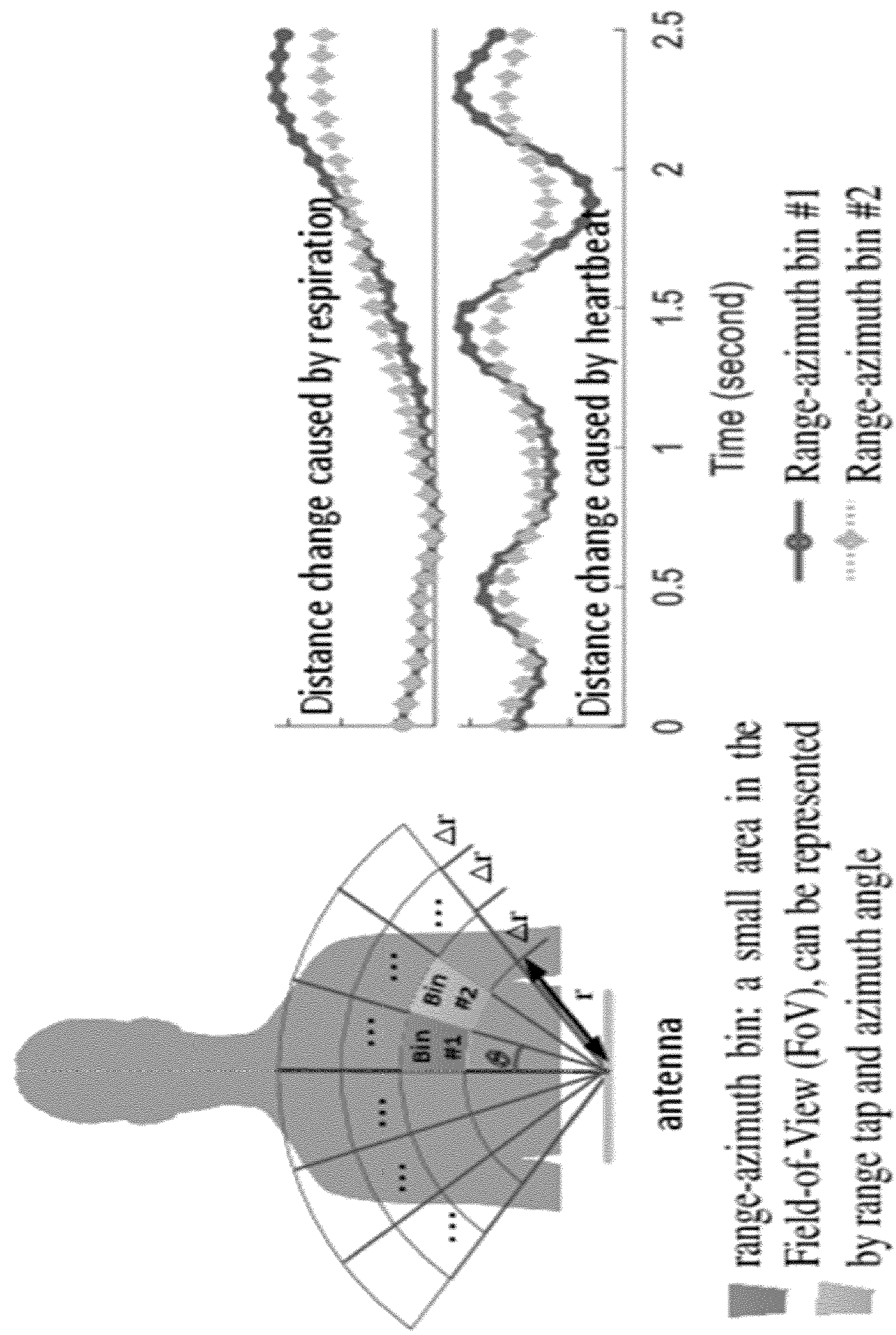
antenna
Bin #1
Bin #2
r
Δr
Δr
Δr
θ
range-azimuth bin: a small area in the Field-of-View (FoV), can be represented by range tap and azimuth angle
Distance change caused by respiration
Distance change caused by heartbeat
0
0.5
1
1.5
2
2.5
Time (second)
Range-azimuth bin #1
Range-azimuth bin #2

FIG. 19

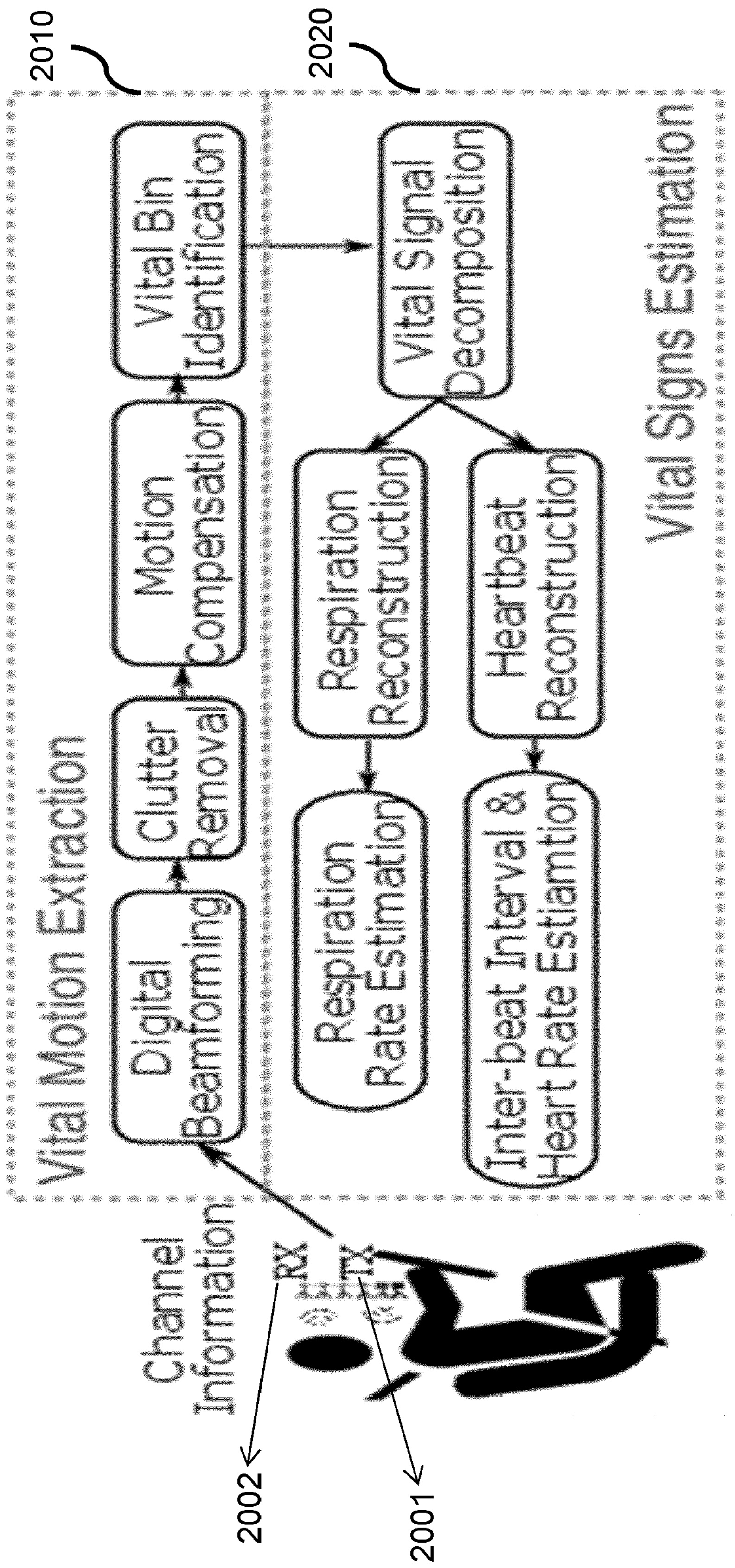
2010
2020
Vital Motion Extraction
Digital Beamforming
Clutter Removal
Motion Compensation
Vital Bin Identification
Vital Signs Estimation
Vital Signal Decomposition
Respiration Reconstruction
Heartbeat Reconstruction
Respiration Rate Estimation
Inter-beat Interval & Heart Rate Estiamtion
Channel Information
RX
TX
2002
2001

FIG. 20

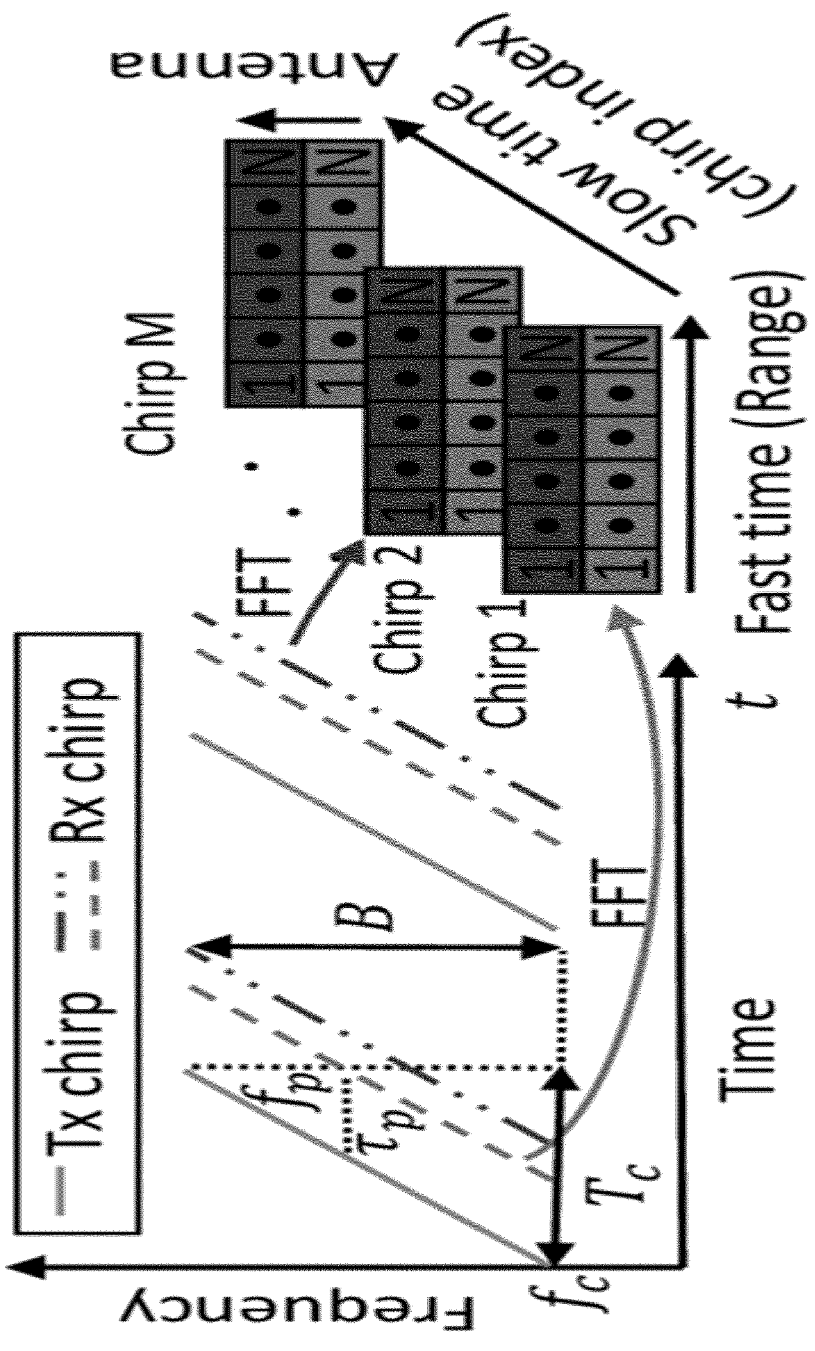
Tx chirp
Rx chirp
Frequency
$f_c$
$T_c$
$\tau_p$
$f_p$
$B$
Time
FFT
$t$
Chirp 1
Chirp 2
Chirp M
FFT
Fast time (Range)
Slow time
(chirp index)
Antenna

FIG. 21

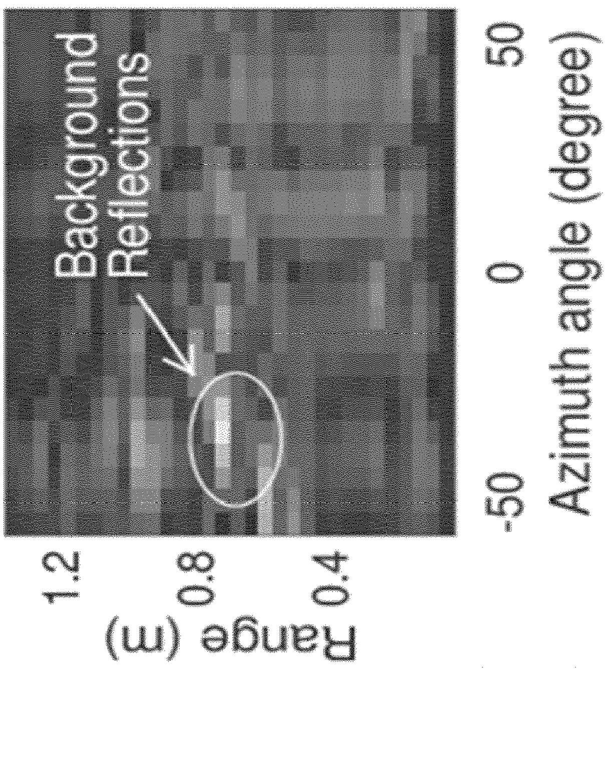


FIG. 22A

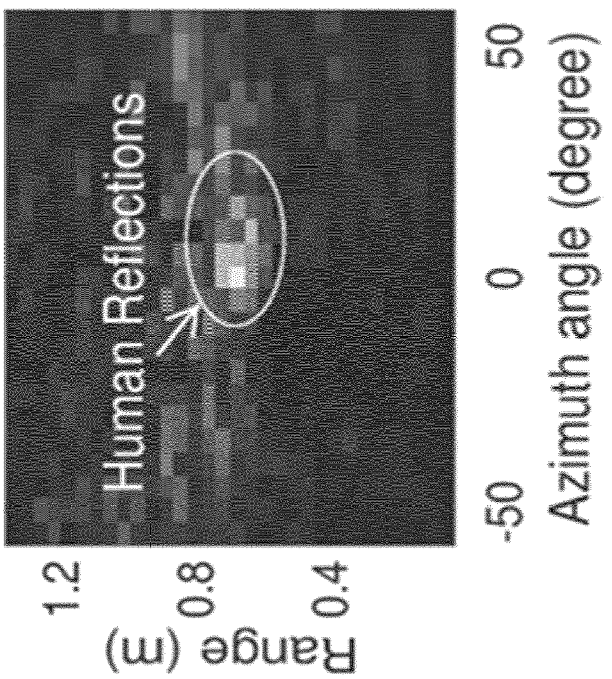


FIG. 22B

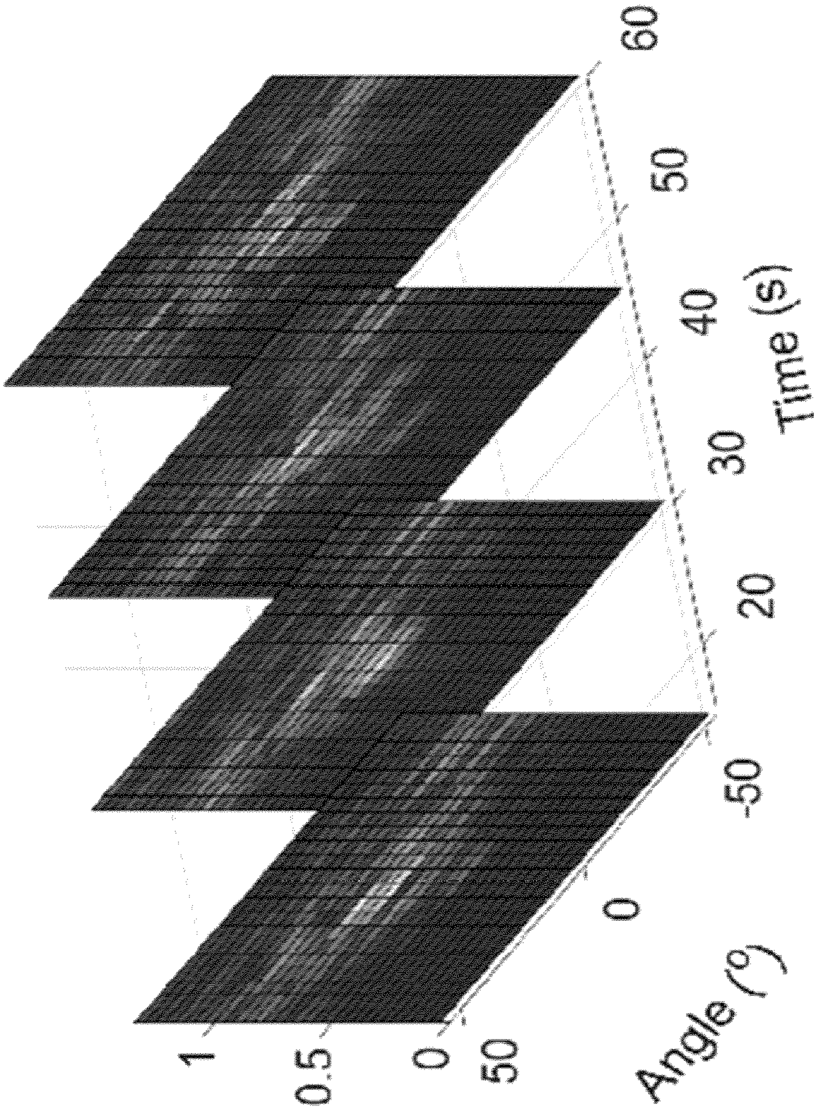


FIG. 23

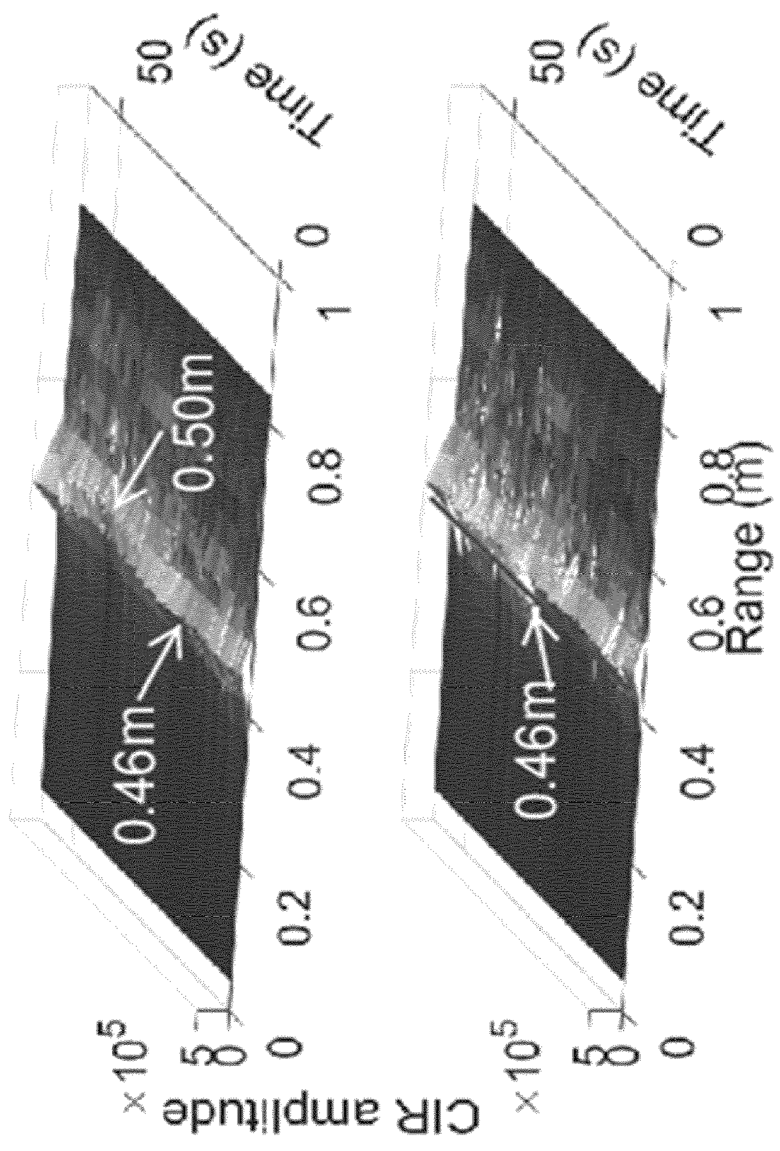


FIG. 24

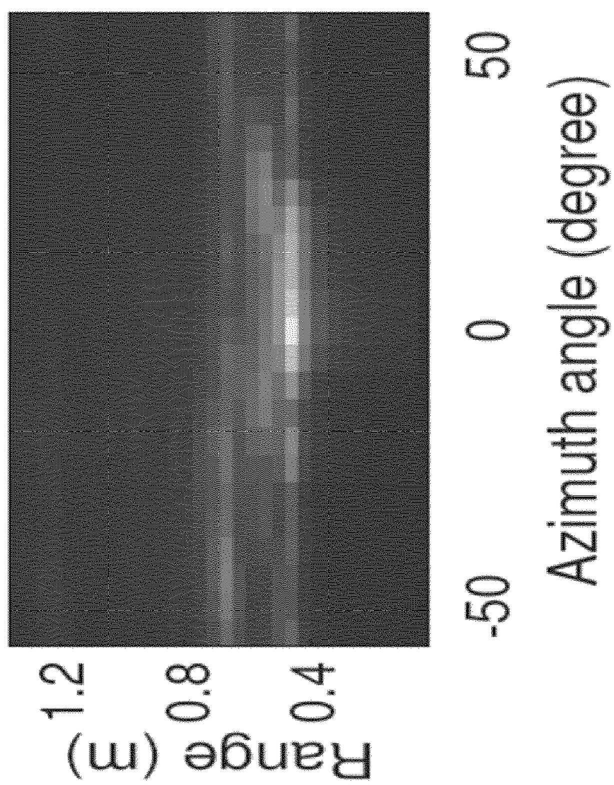


FIG. 25A

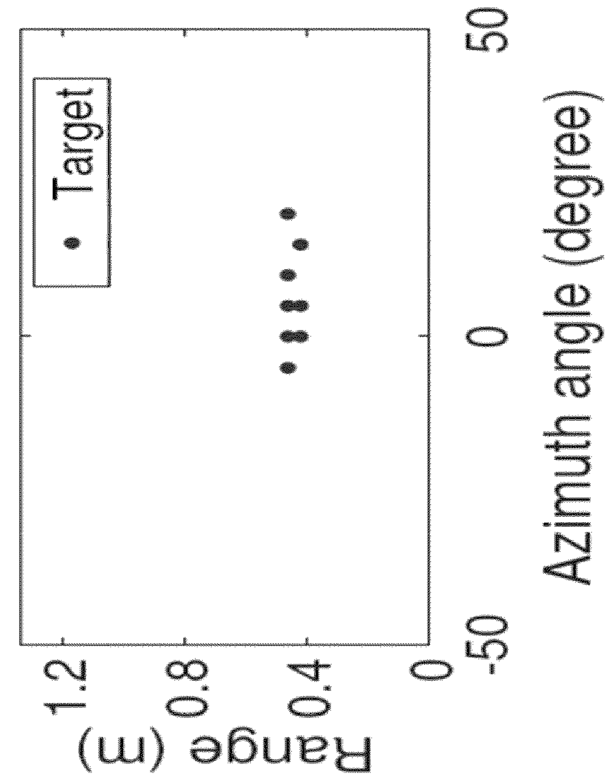


FIG. 25B

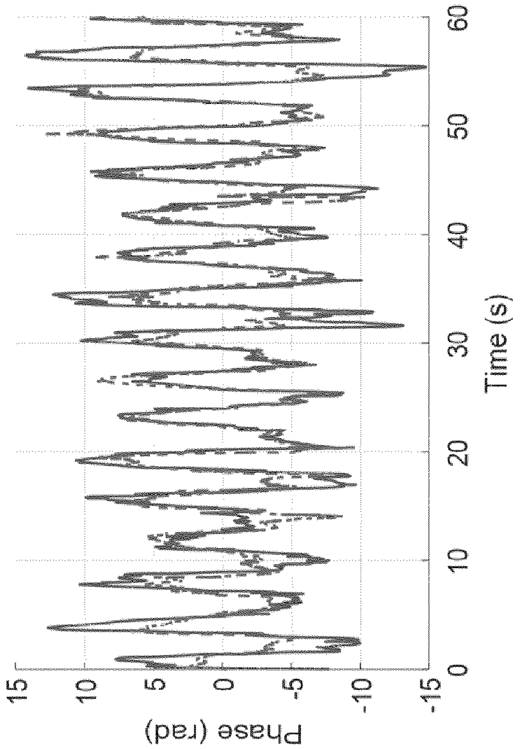


FIG. 26B

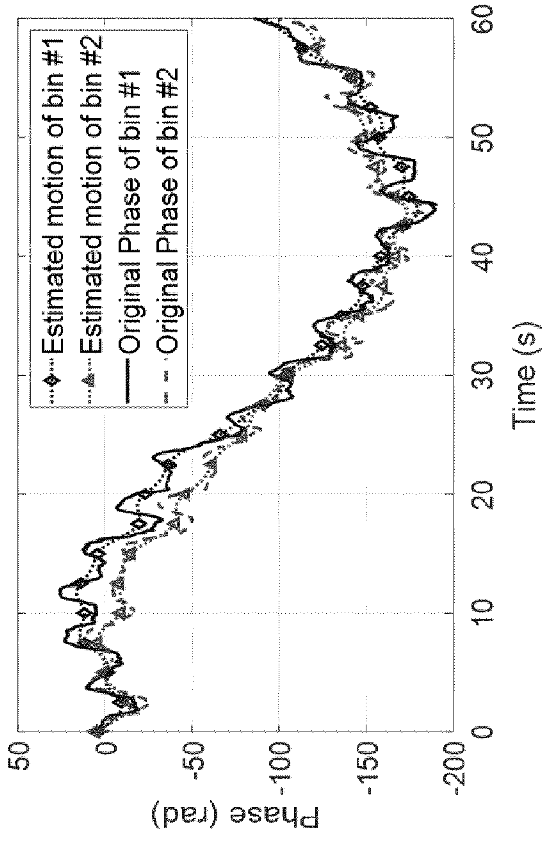


FIG. 26A

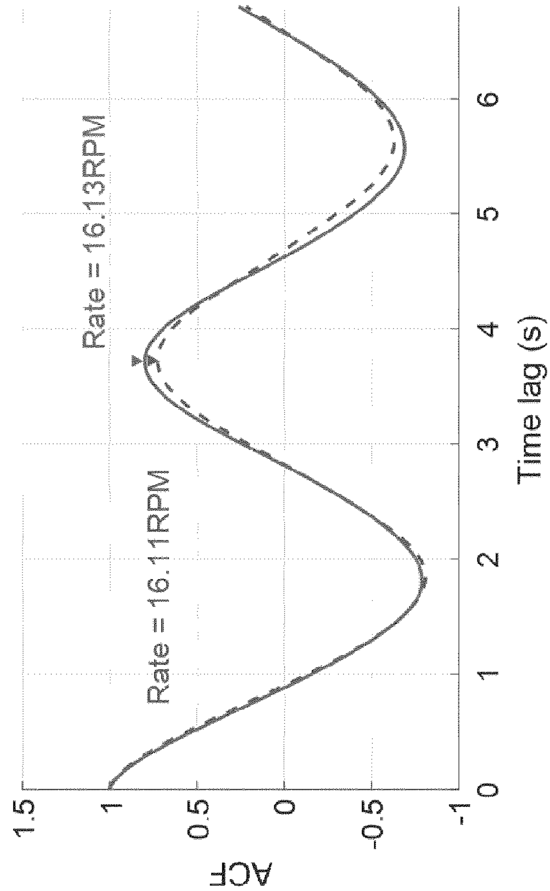


FIG. 26C

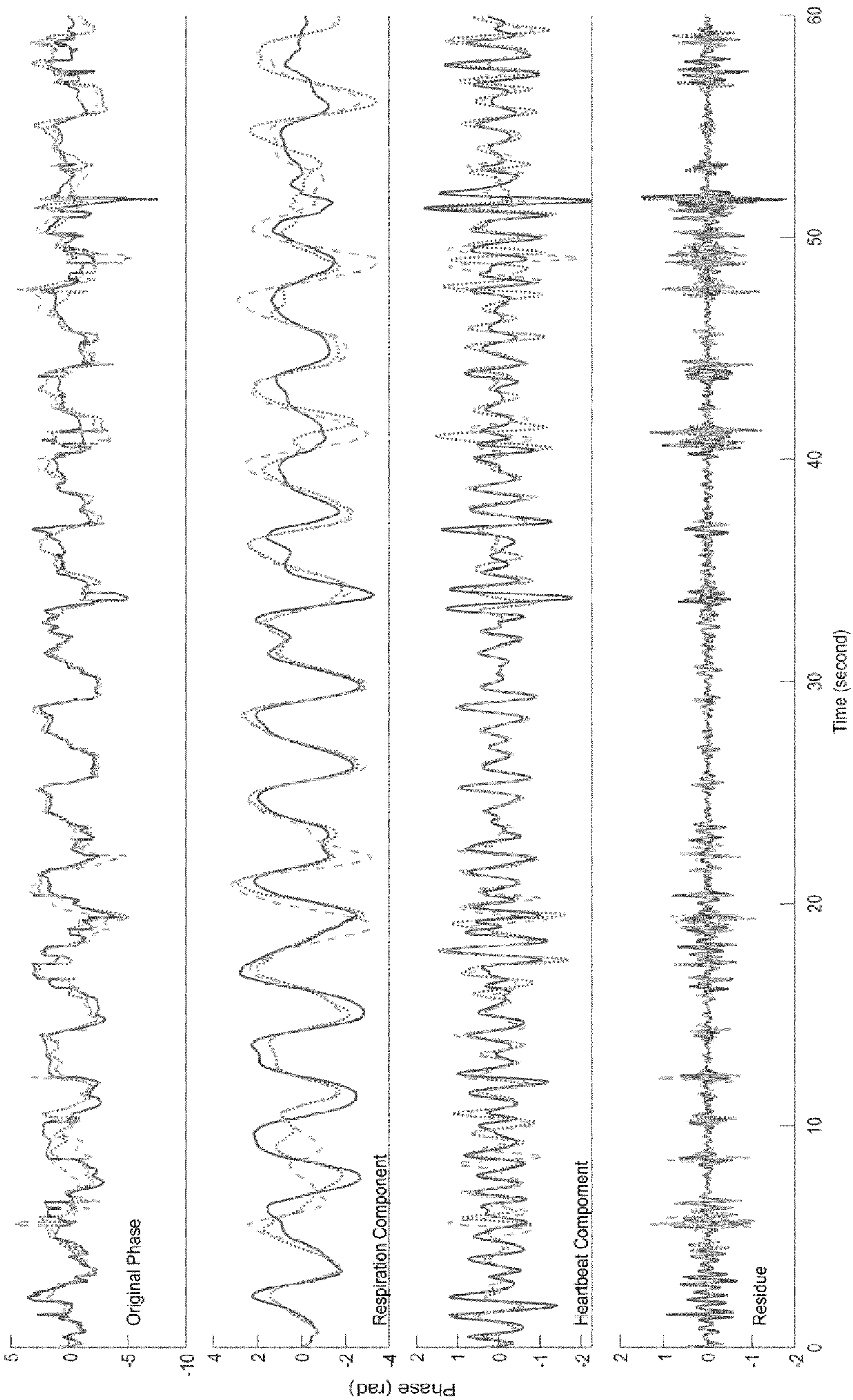
Original Phase
Respiration Component
Heartbeat Component
Residue
Phase (rad)
Time (second)
0
10
20
30
40
50
60

FIG. 27A

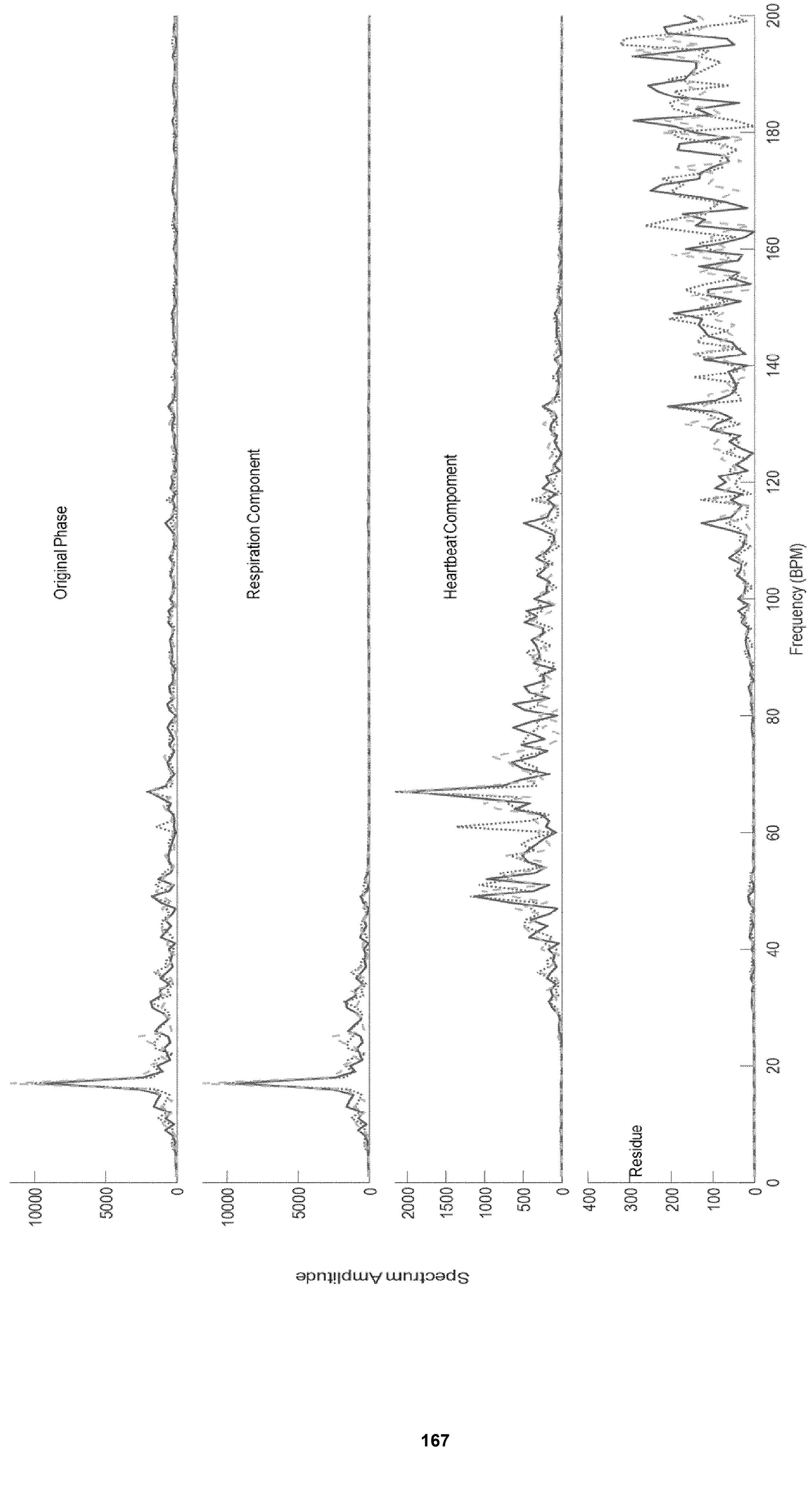
Original Phase
Respiration Component
Heartbeat Component
Residue
Spectrum Amplitude
Frequency (BPM)
10000
5000
0
2000
1500
1000
500
400
300
200
100
0
20
40
60
80
100
120
140
160
180
200

FIG. 27B

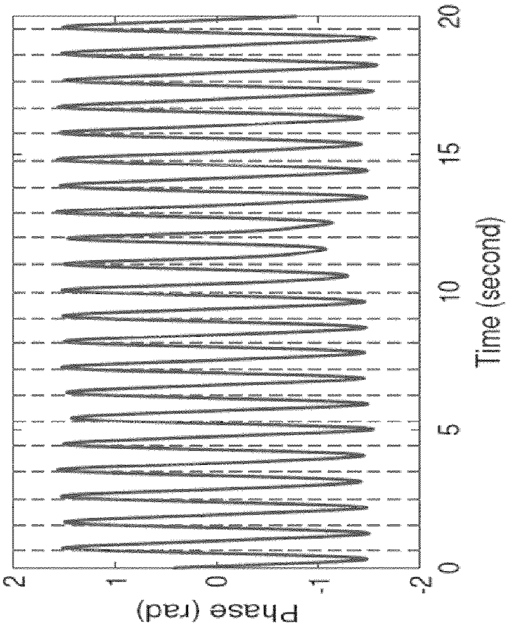


FIG. 28B

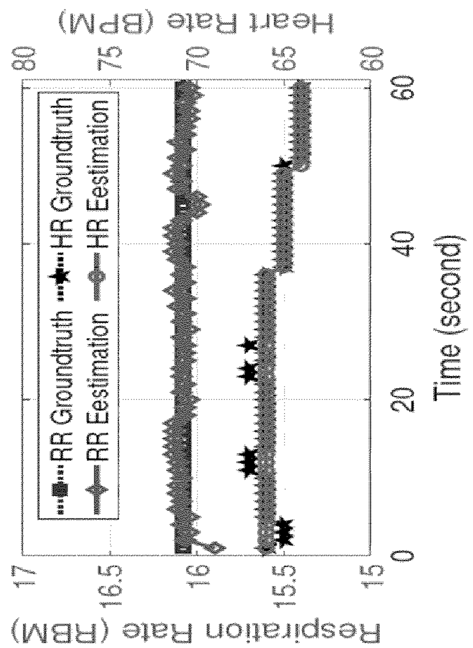


FIG. 28A

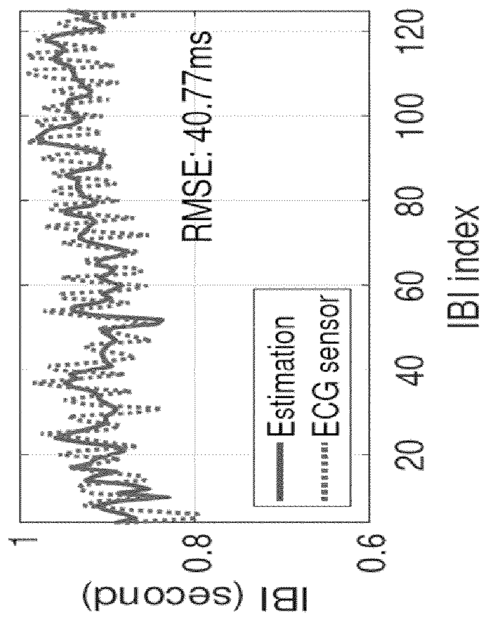


FIG. 28C

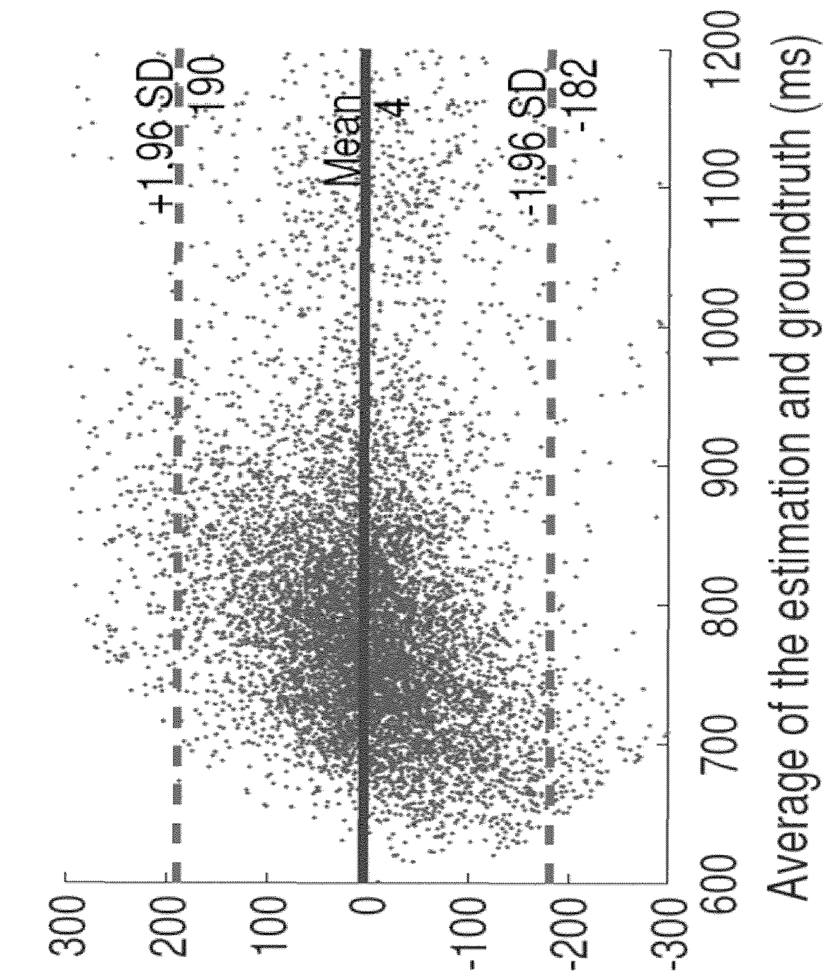
Estimation - groundtruth (ms)
300
200
100
0
-100
-200
-300
600
700
800
900
1000
1100
1200
Average of the estimation and groundtruth (ms)
+1.96 SD
190
Mean
4
-1.96 SD
-182

FIG. 29

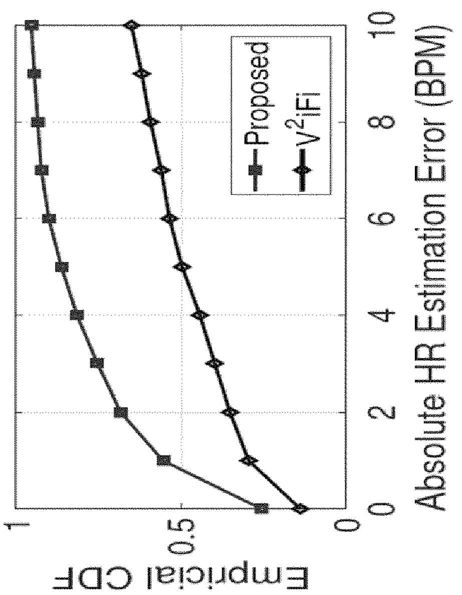


FIG. 30B

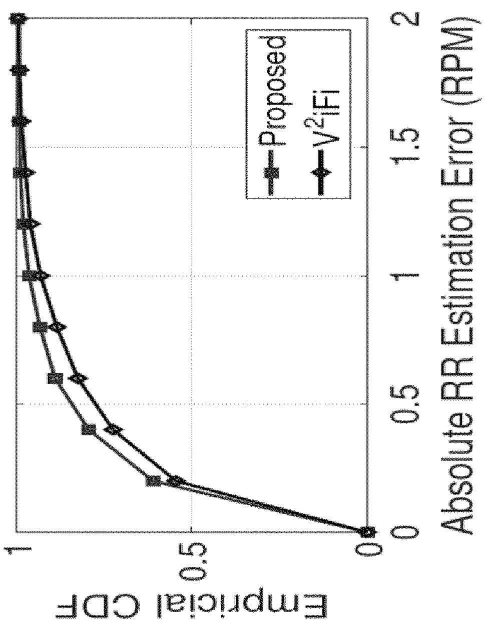


FIG. 30A

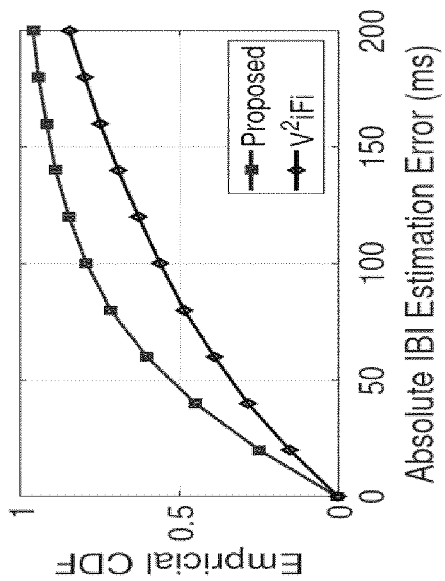


FIG. 30C

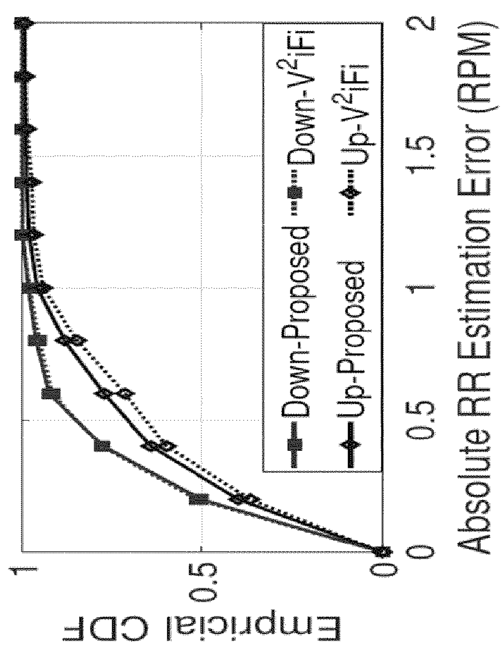


FIG. 31A

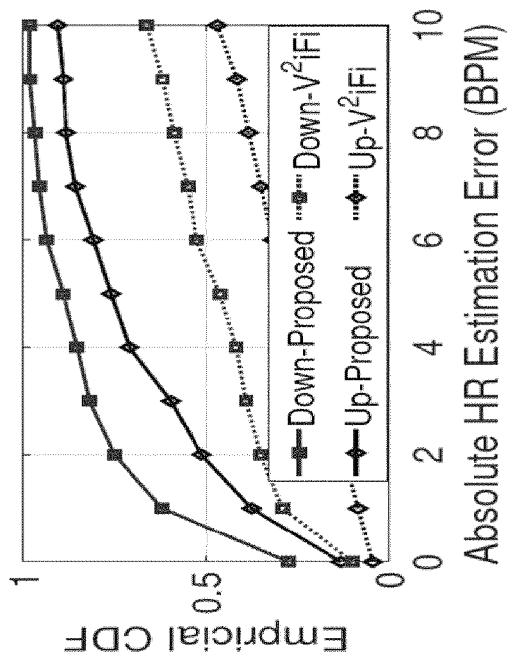


FIG. 31B

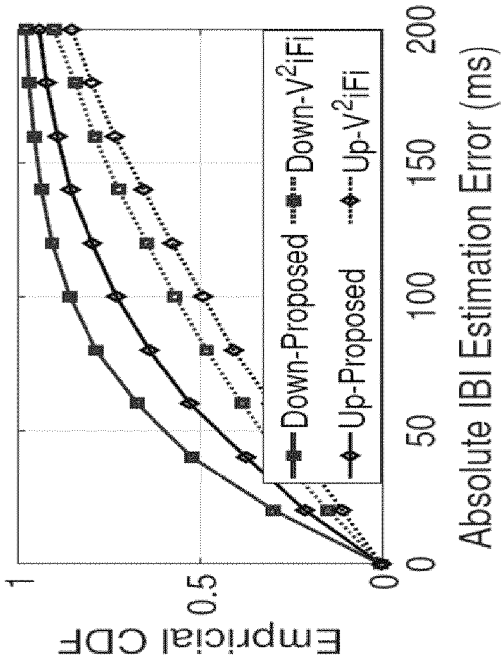


FIG. 31C

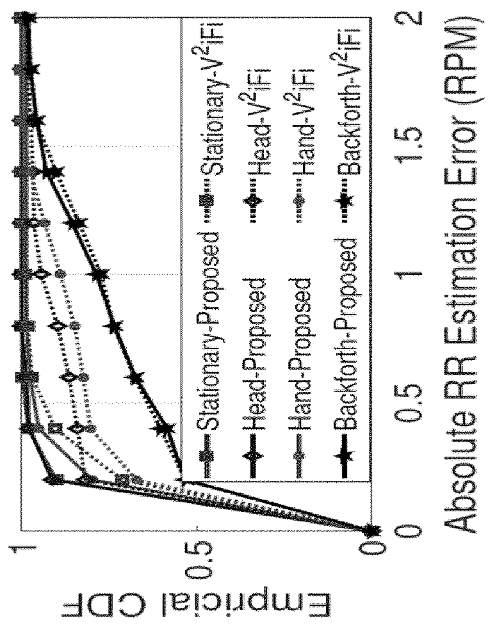


FIG. 32A

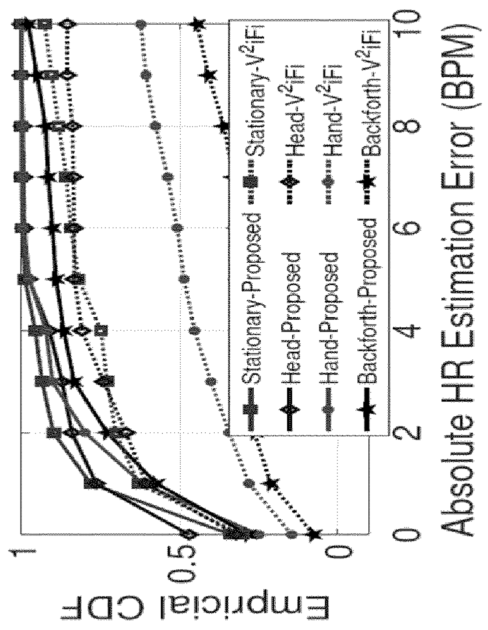


FIG. 32B

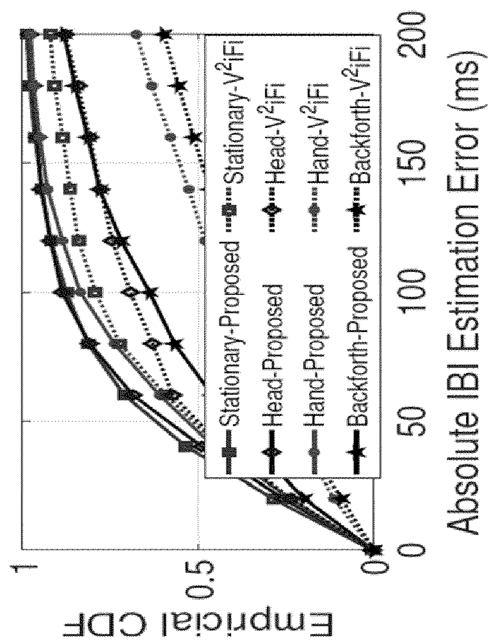


FIG. 32C

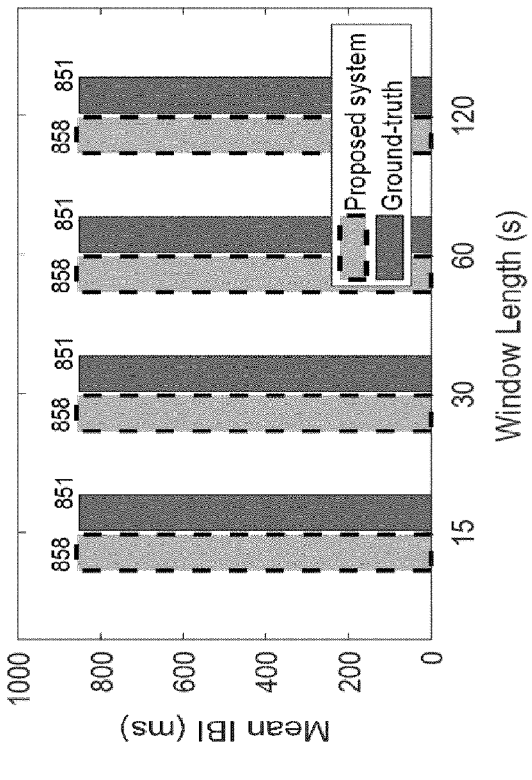


FIG. 33A

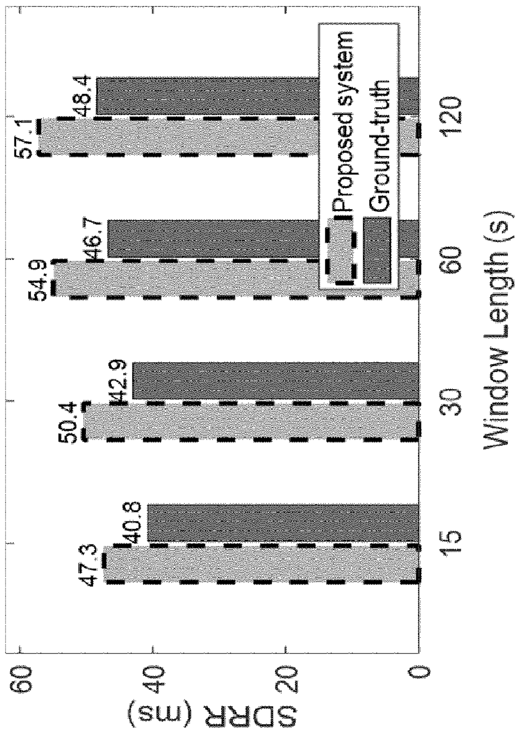


FIG. 33B

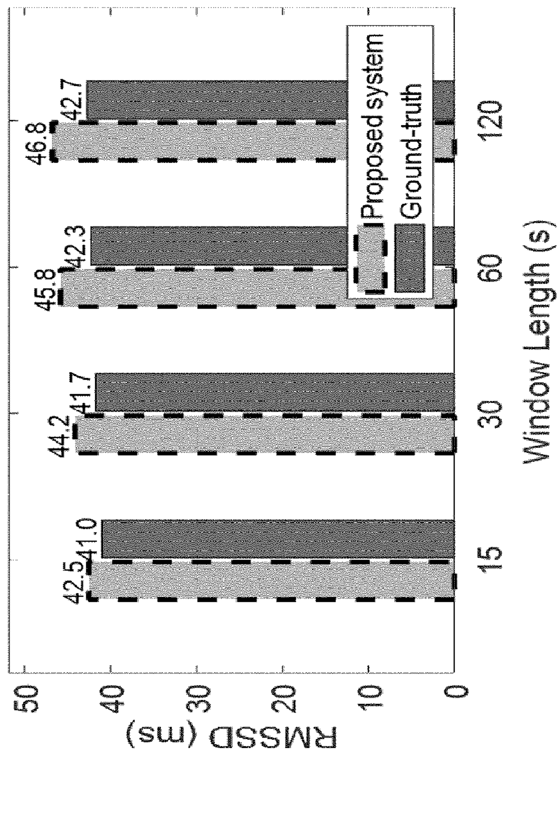


FIG. 33C

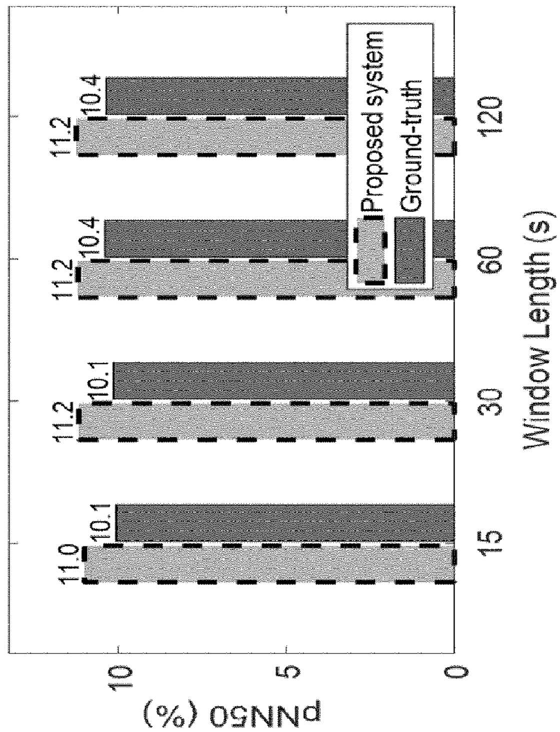


FIG. 33D

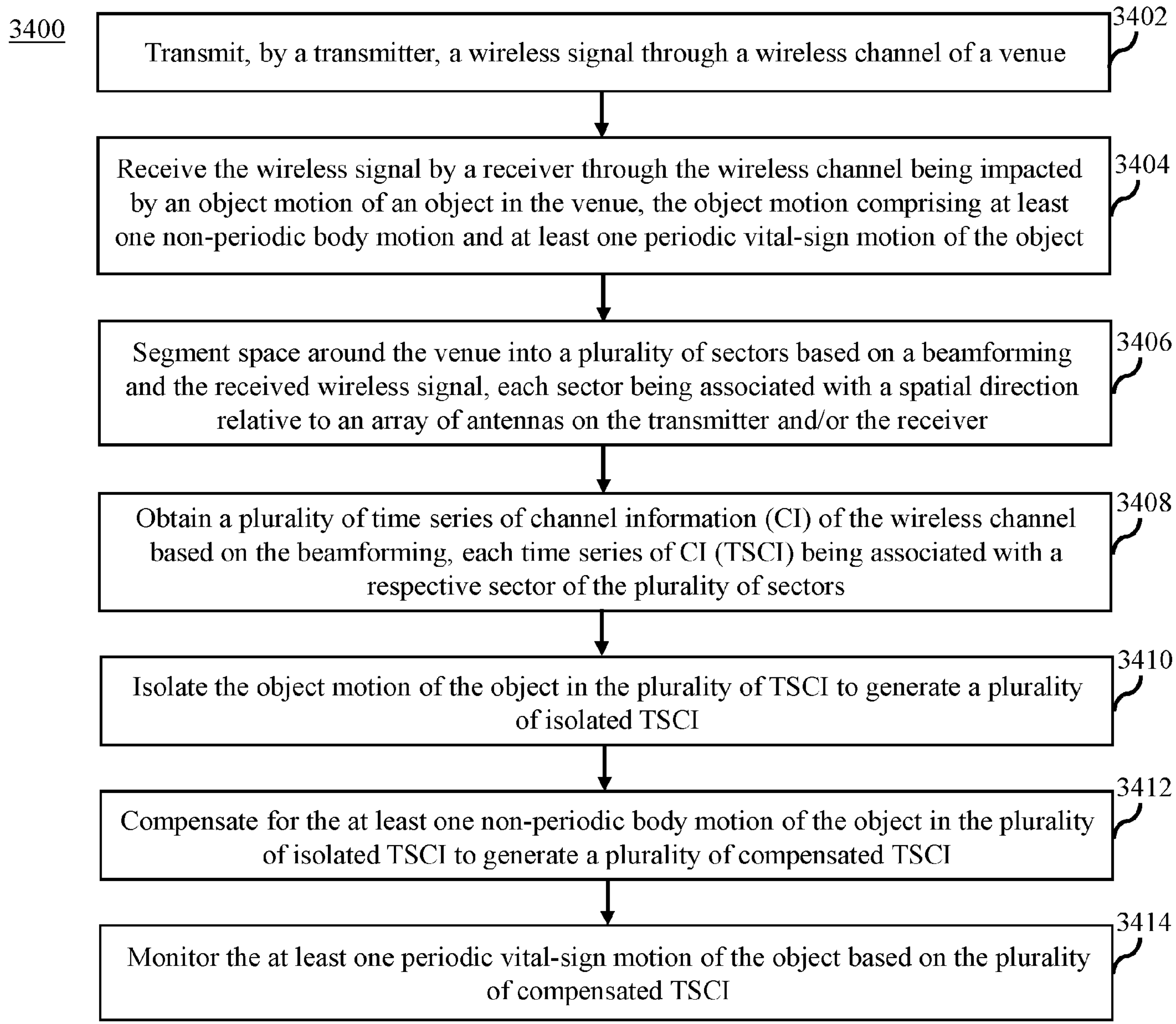
3400
Transmit, by a transmitter, a wireless signal through a wireless channel of a venue
3402
Receive the wireless signal by a receiver through the wireless channel being impacted by an object motion of an object in the venue, the object motion comprising at least one non-periodic body motion and at least one periodic vital-sign motion of the object
3404
Segment space around the venue into a plurality of sectors based on a beamforming and the received wireless signal, each sector being associated with a spatial direction relative to an array of antennas on the transmitter and/or the receiver
3406
Obtain a plurality of time series of channel information (CI) of the wireless channel based on the beamforming, each time series of CI (TSCI) being associated with a respective sector of the plurality of sectors
3408
Isolate the object motion of the object in the plurality of TSCI to generate a plurality of isolated TSCI
3410
Compensate for the at least one non-periodic body motion of the object in the plurality of isolated TSCI to generate a plurality of compensated TSCI
3412
Monitor the at least one periodic vital-sign motion of the object based on the plurality of compensated TSCI
3414

FIG. 34

3500

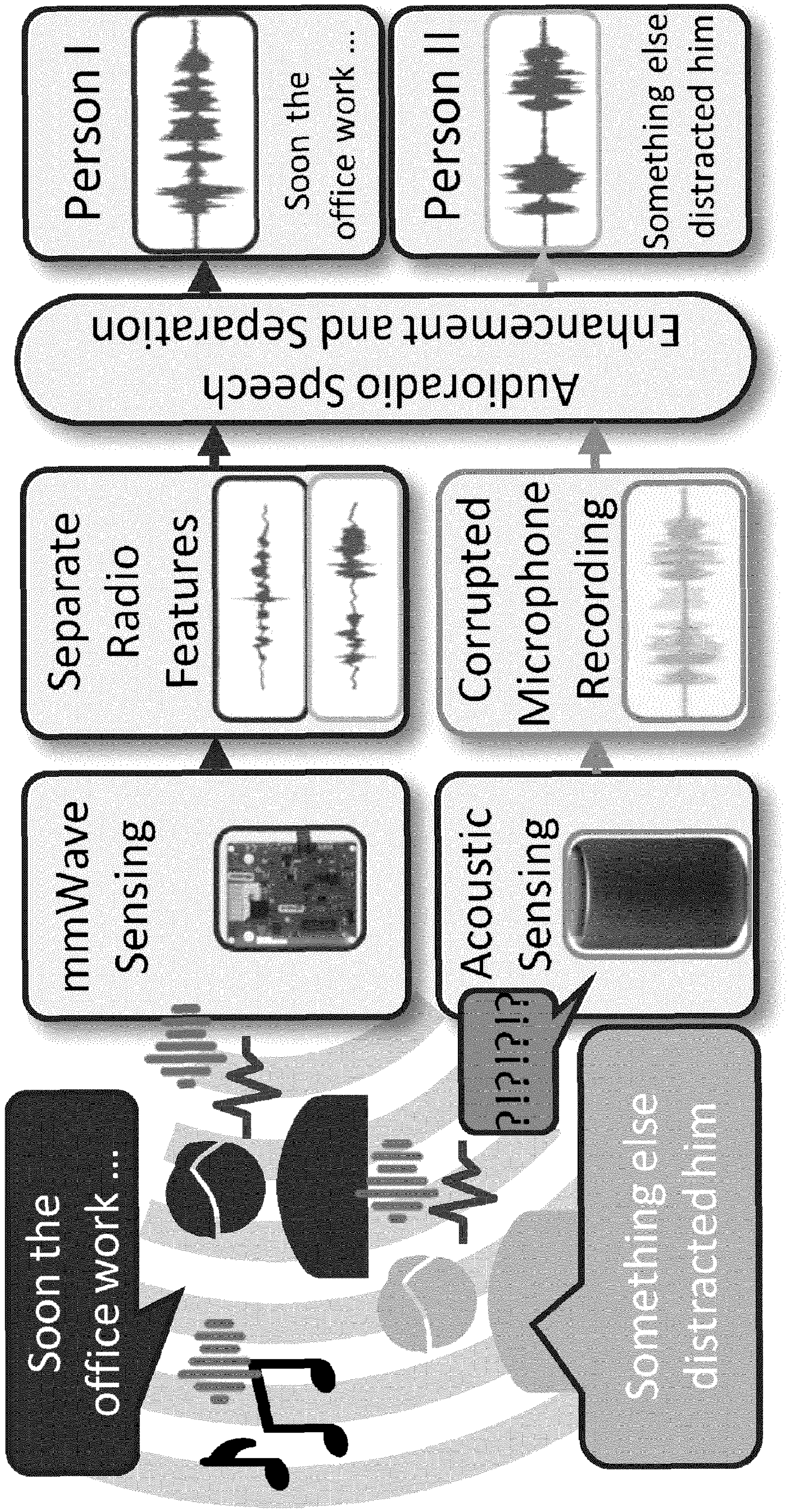
Soon the office work ...
Something else distracted him
mmWave Sensing
Acoustic Sensing
Separate Radio Features
Corrupted Microphone Recording
Audioradio Speech Enhancement and Separation
Person I
Soon the office work ...
Person II
Something else distracted him

FIG. 35

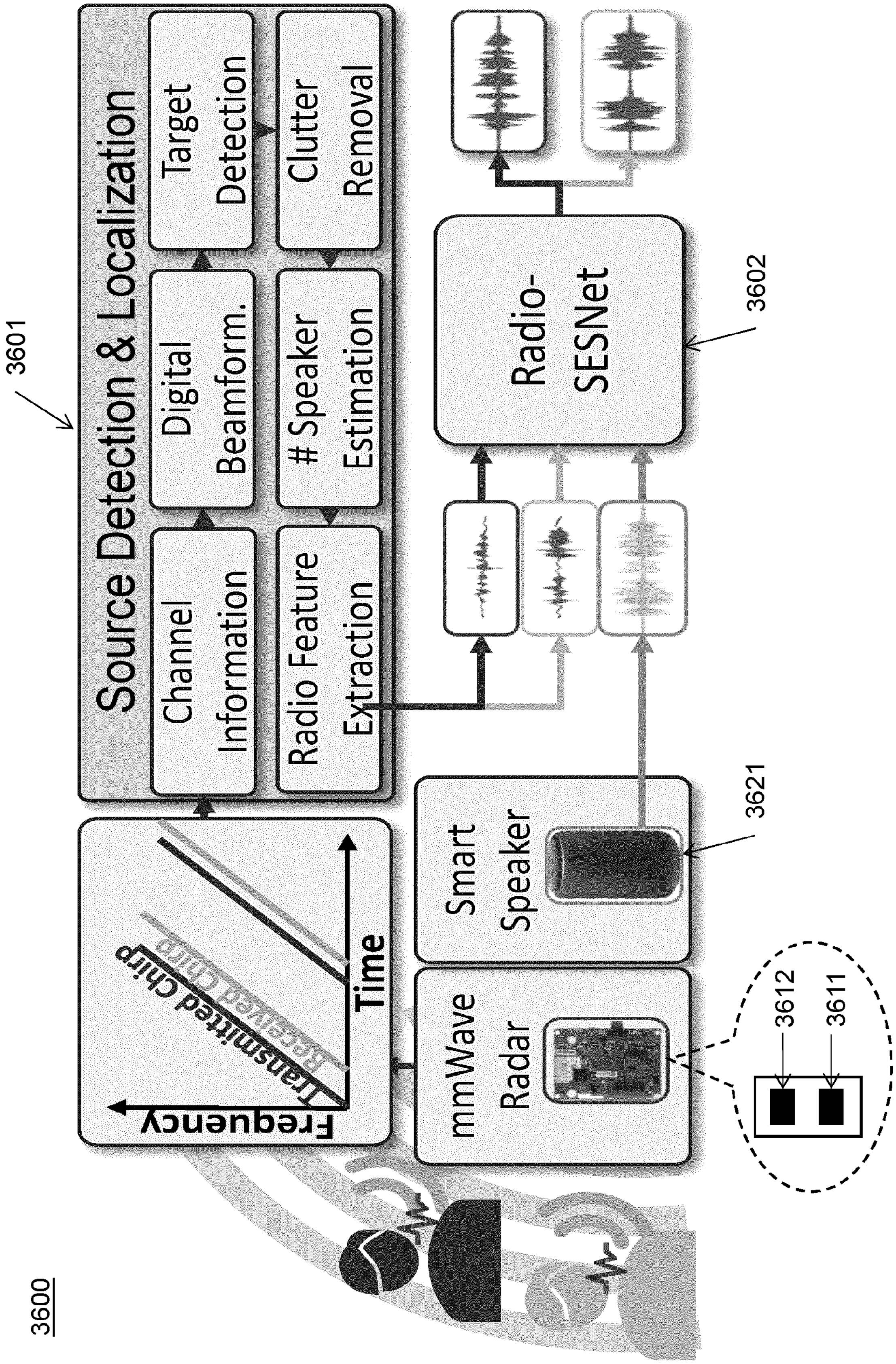
3600
3601
Source Detection & Localization
Channel Information
Digital Beamform.
Target Detection
Radio Feature Extraction
# Speaker Estimation
Clutter Removal
Frequency
Transmitted Chirp
Received Chirp
Time
mmWave Radar
Smart Speaker
Radio-SESNet
3602
3621
3612
3611

FIG. 36

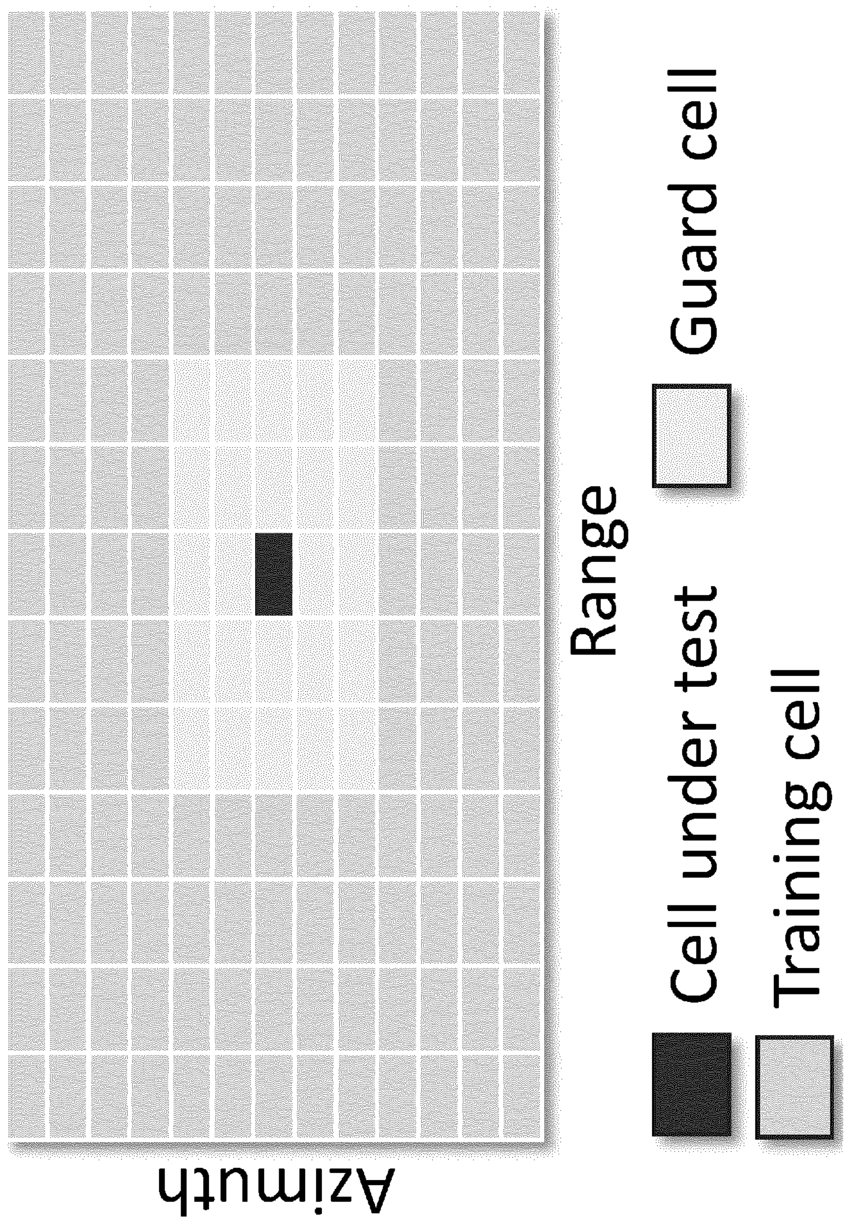


FIG. 37

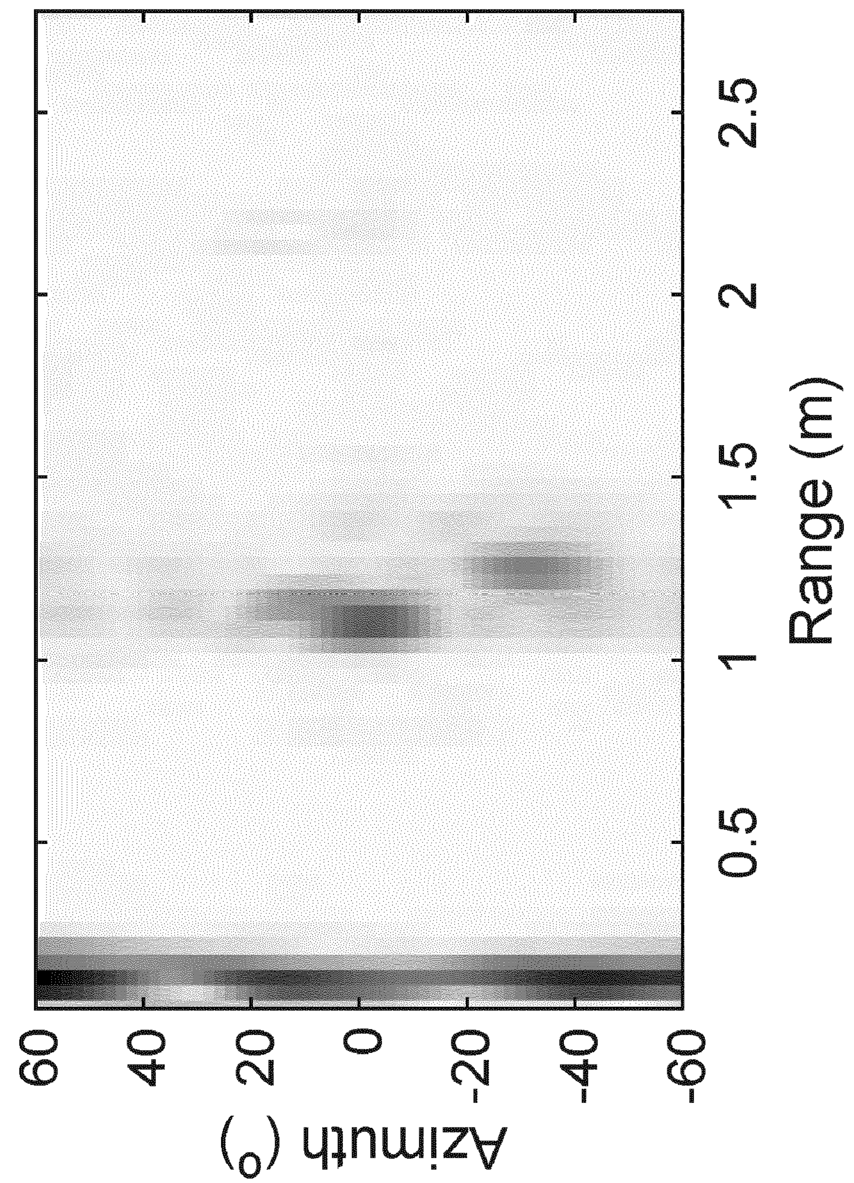


FIG. 38

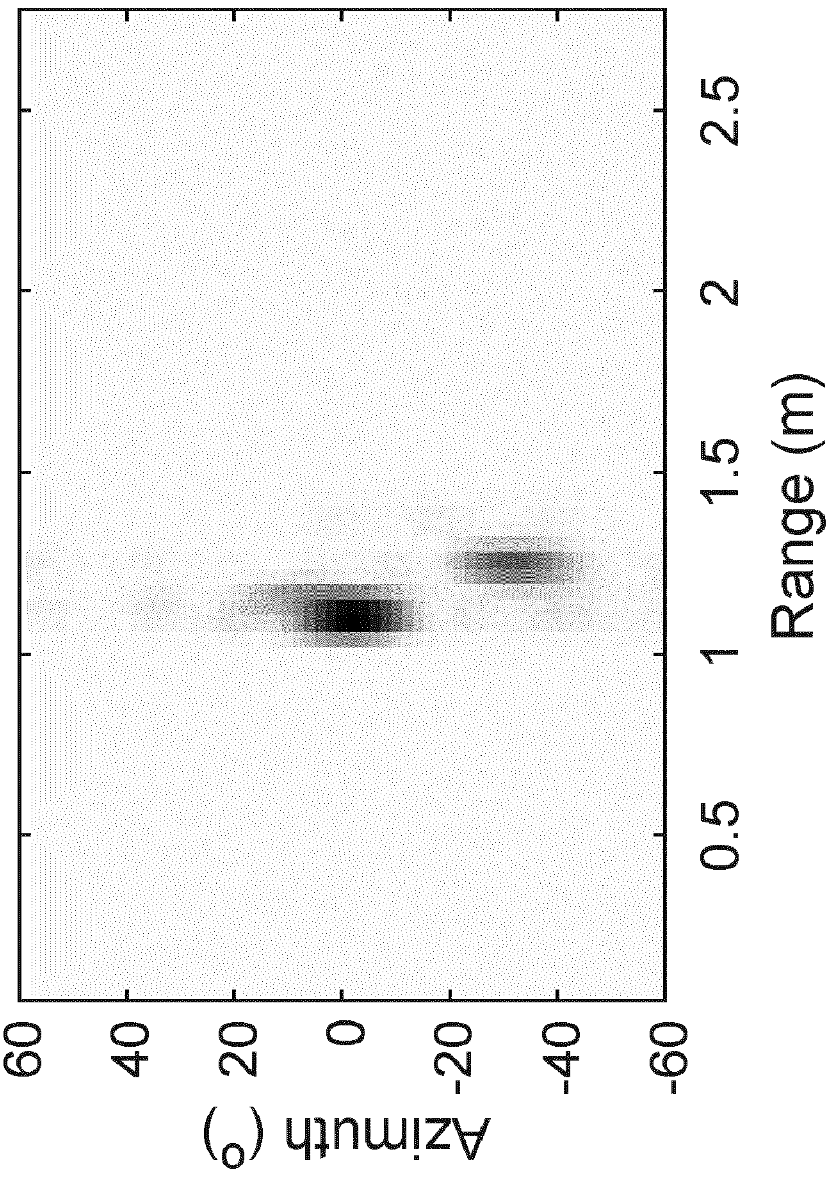


FIG. 39

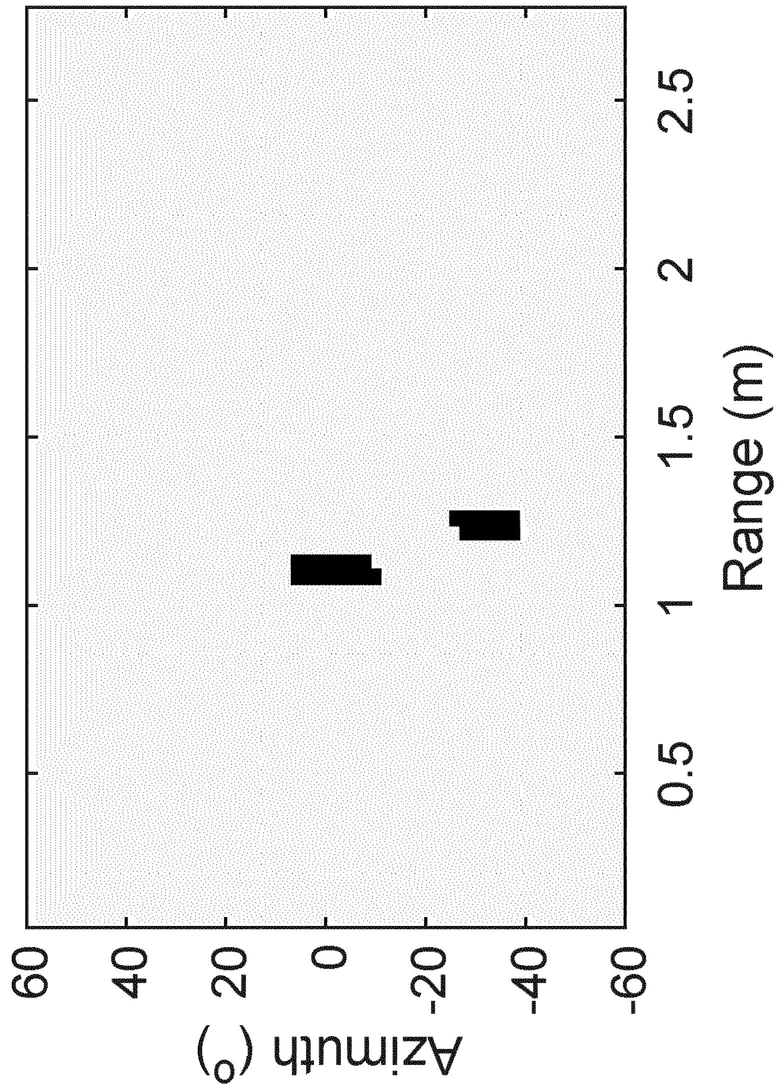


FIG. 40

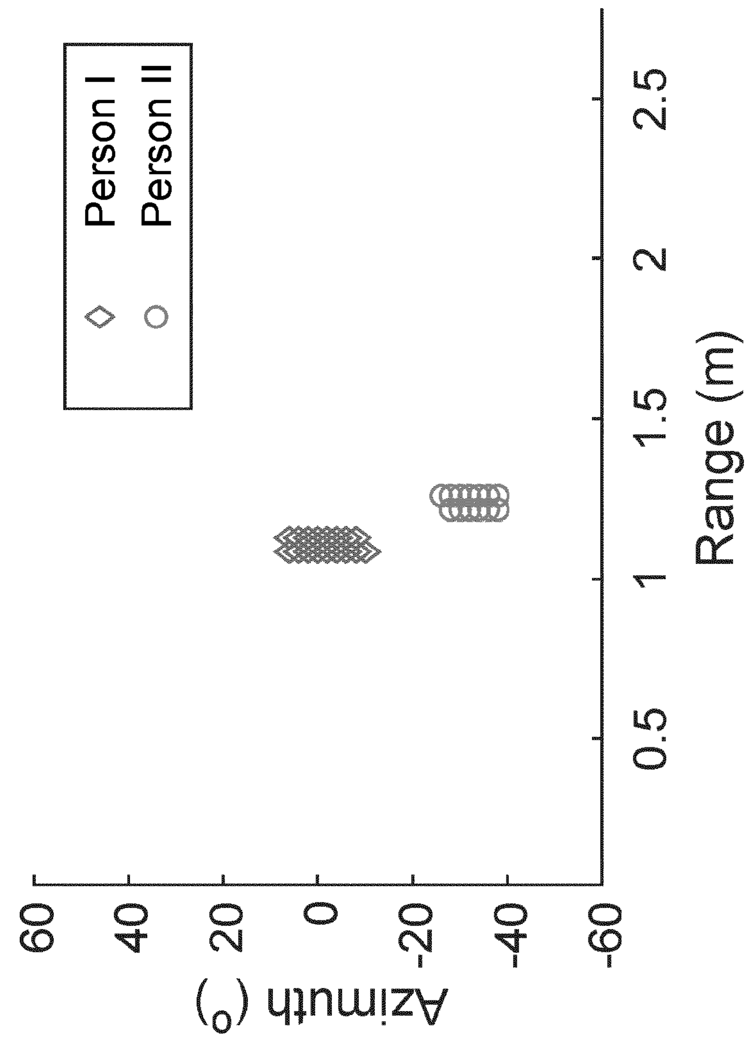


FIG. 41

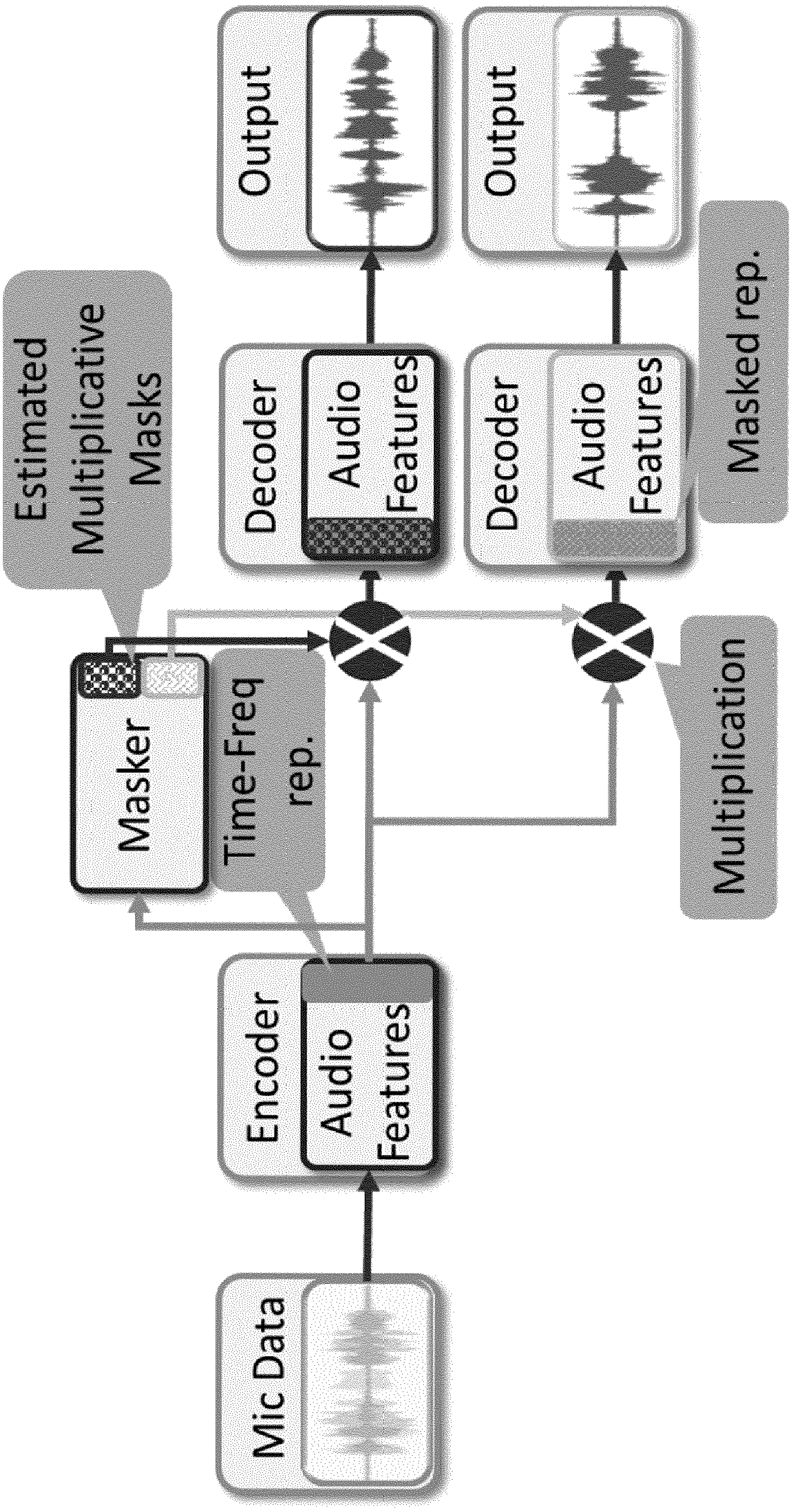
Mic Data
Encoder
Audio Features
Time-Freq rep.
Masker
Estimated Multiplicative Masks
Multiplication
Decoder
Audio Features
Decoder
Audio Features
Masked rep.
Output
Output

FIG. 42A

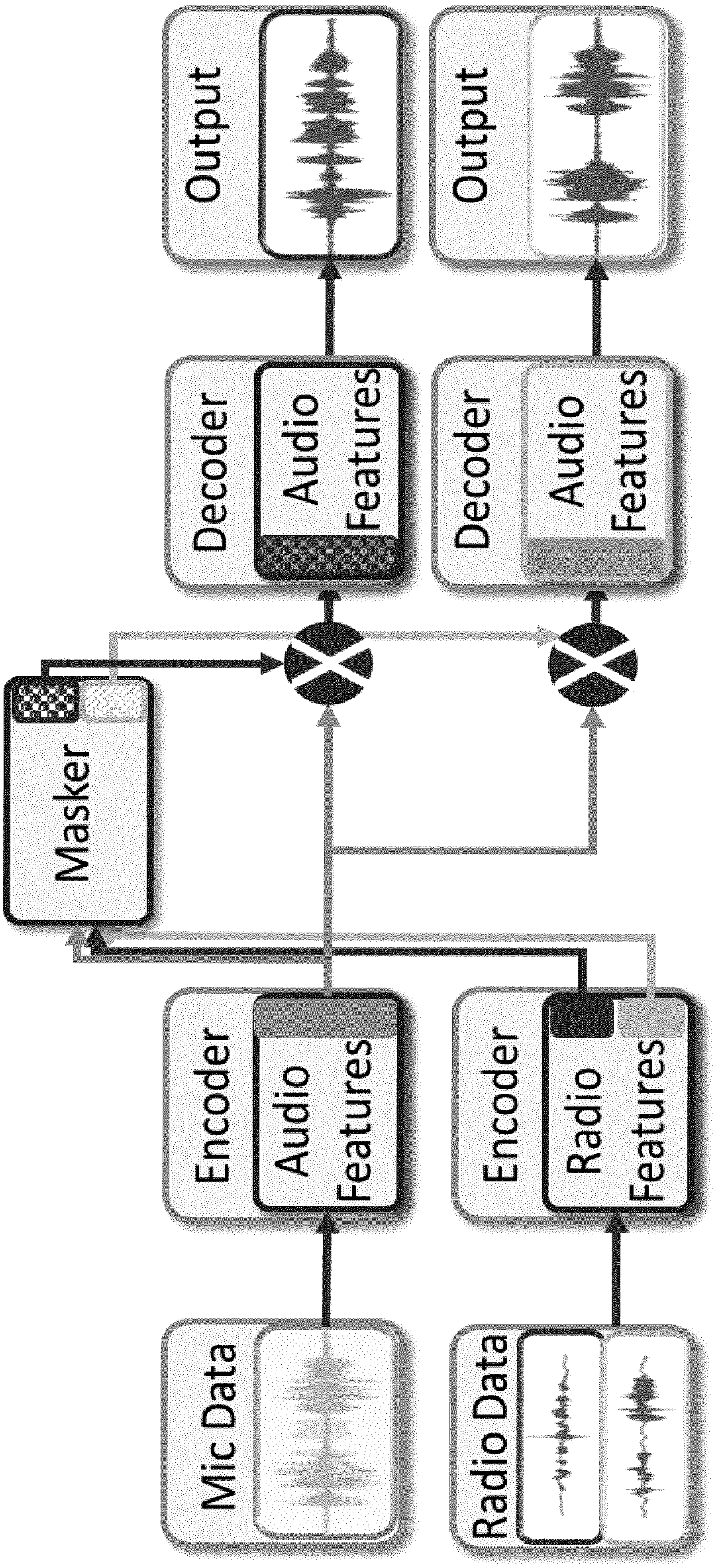
Mic Data
Encoder
Audio Features
Radio Data
Encoder
Radio Features
Masker
Decoder
Audio Features
Output
Decoder
Audio Features
Output

FIG. 42B

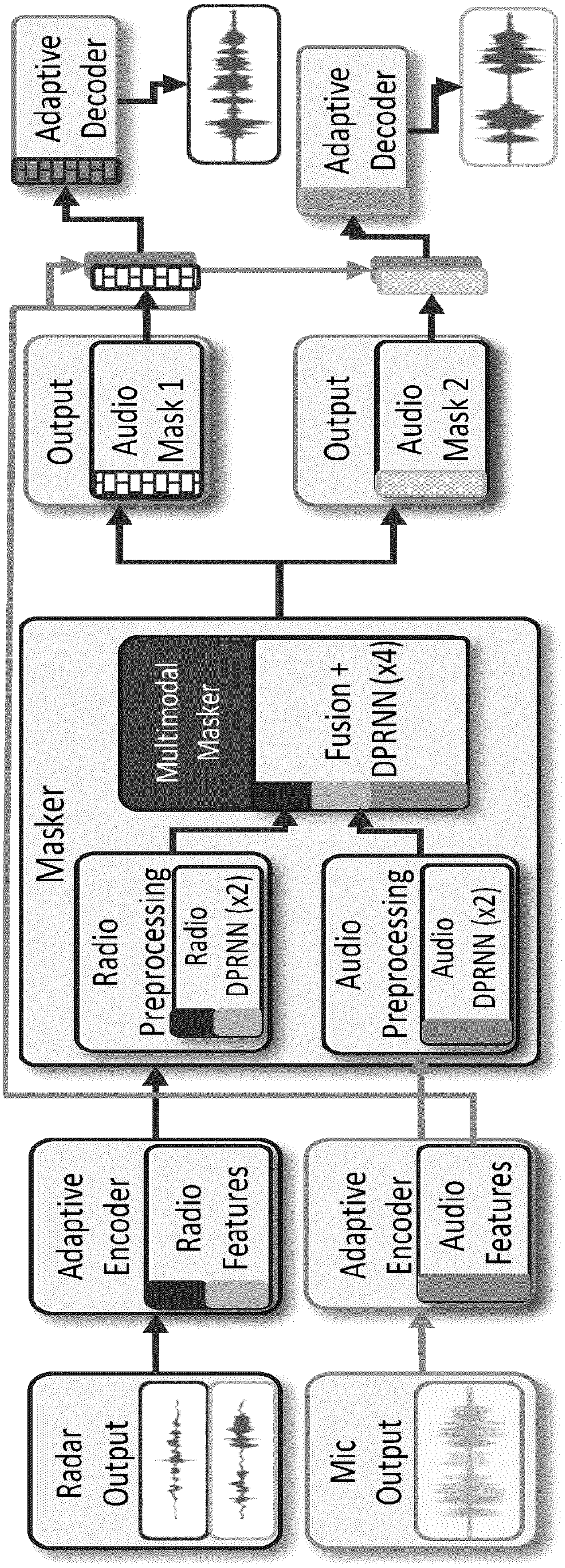
Radar Output
Mic Output
Adaptive Encoder
Radio Features
Adaptive Encoder
Audio Features
Masker
Radio Preprocessing
Radio DPRNN (x2)
Audio Preprocessing
Audio DPRNN (x2)
Multimodal Masker
Fusion + DPRNN (x4)
Output
Audio Mask 1
Output
Audio Mask 2
Adaptive Decoder
Adaptive Decoder

FIG. 43

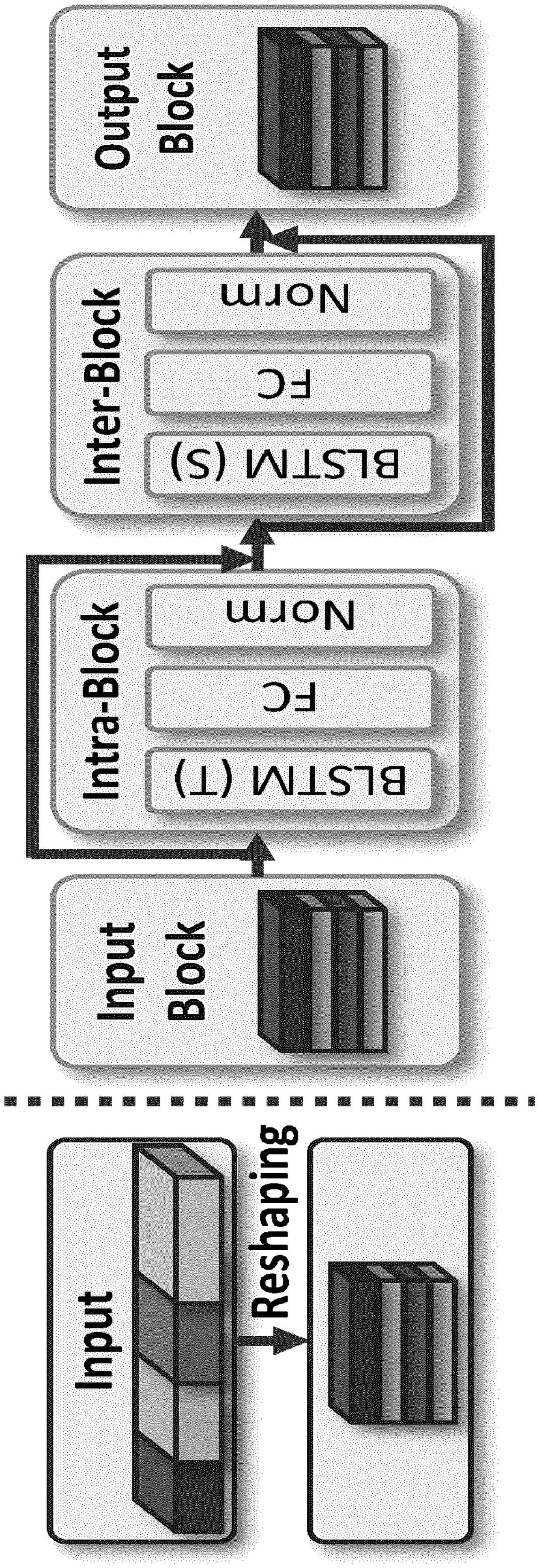
Input
Reshaping
Input Block
Intra-Block
BLSTM (T)
FC
Norm
Inter-Block
BLSTM (S)
FC
Norm
Output Block

FIG. 44

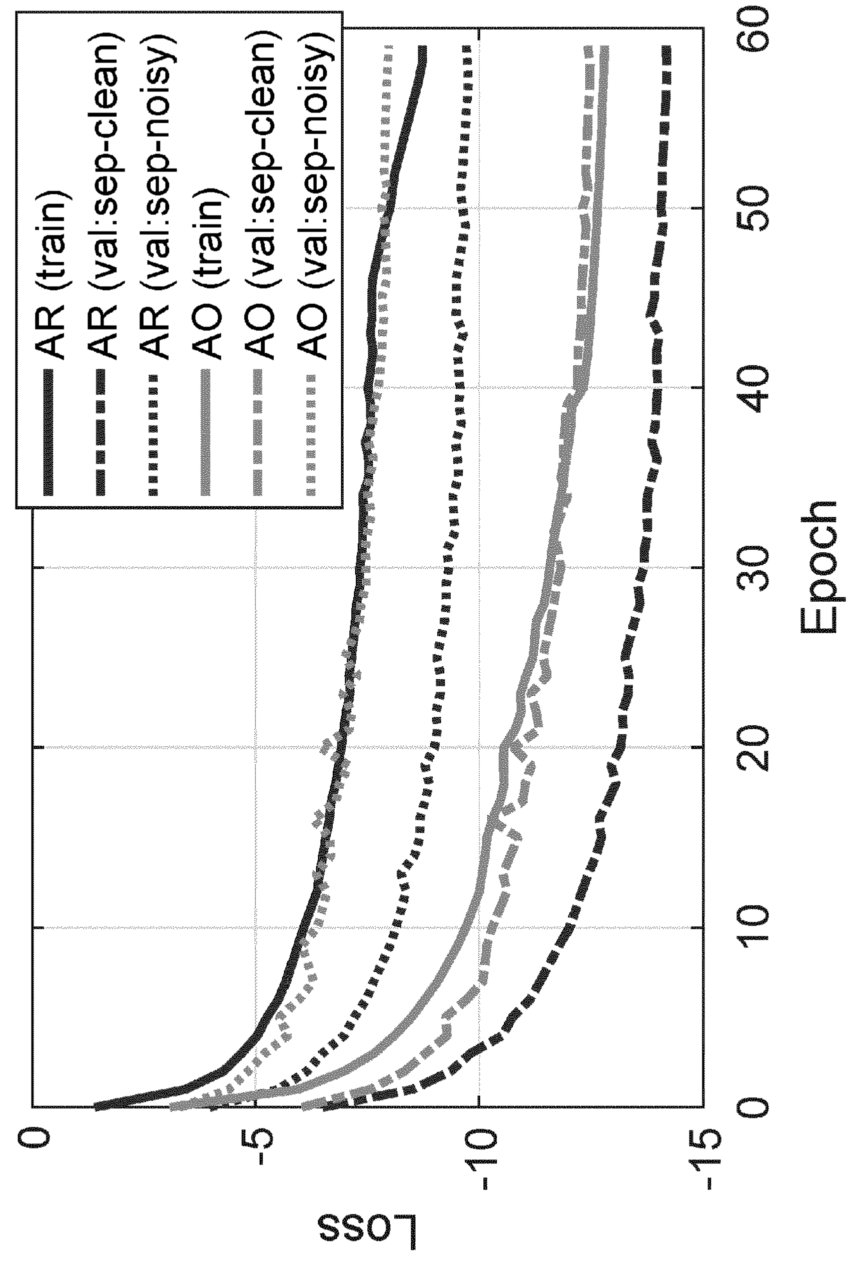
AR (train)
AR (val:sep-clean)
AR (val:sep-noisy)
AO (train)
AO (val:sep-clean)
AO (val:sep-noisy)
0
-5
-10
-15
Loss
0
10
20
30
40
50
60
Epoch

FIG. 45

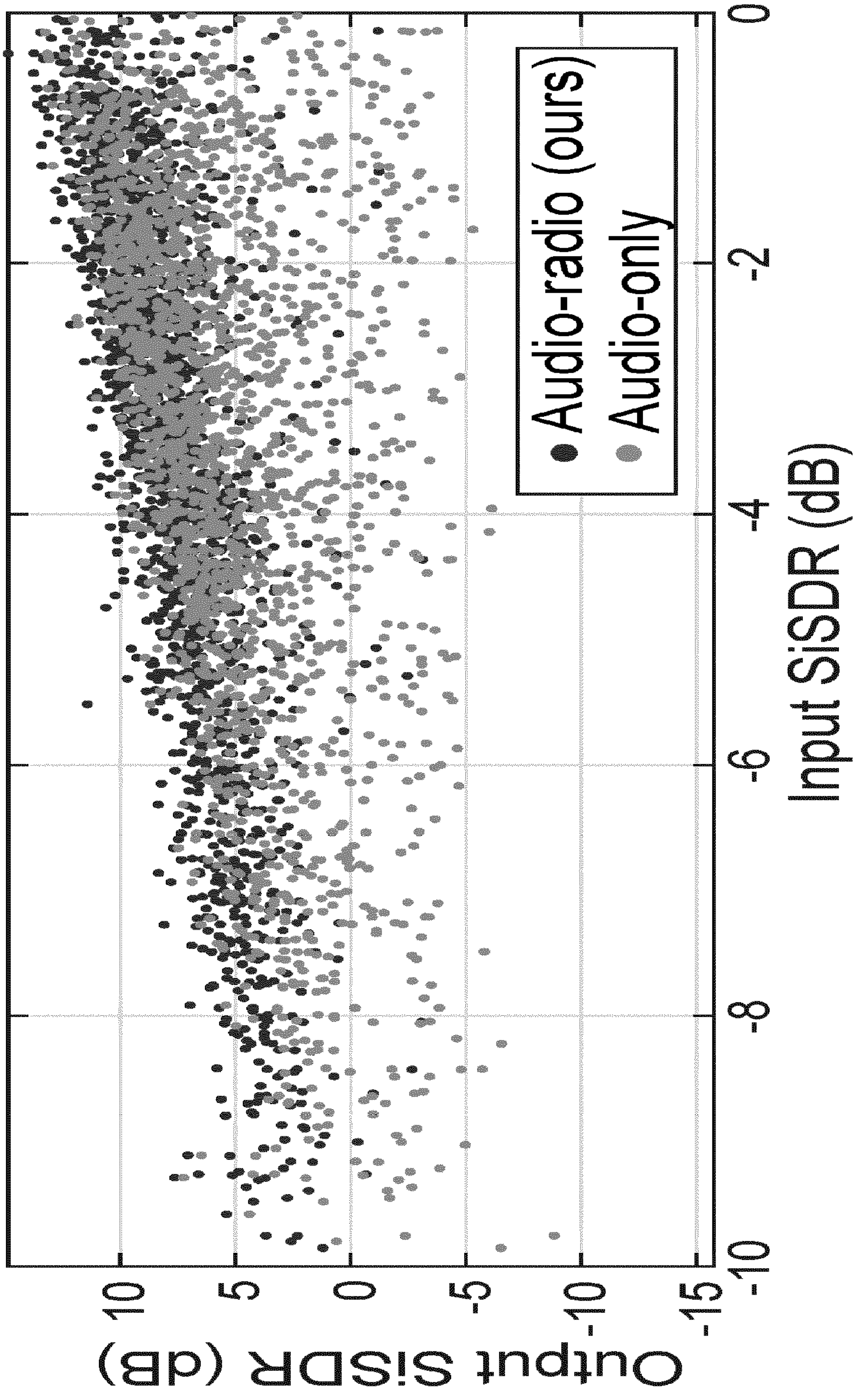
Audio-radio (ours)
Audio-only
Input SiSDR (dB)
Output SiSDR (dB)
0
-2
-4
-6
-8
-10
10
5
0
-5
-10
-15

FIG. 46

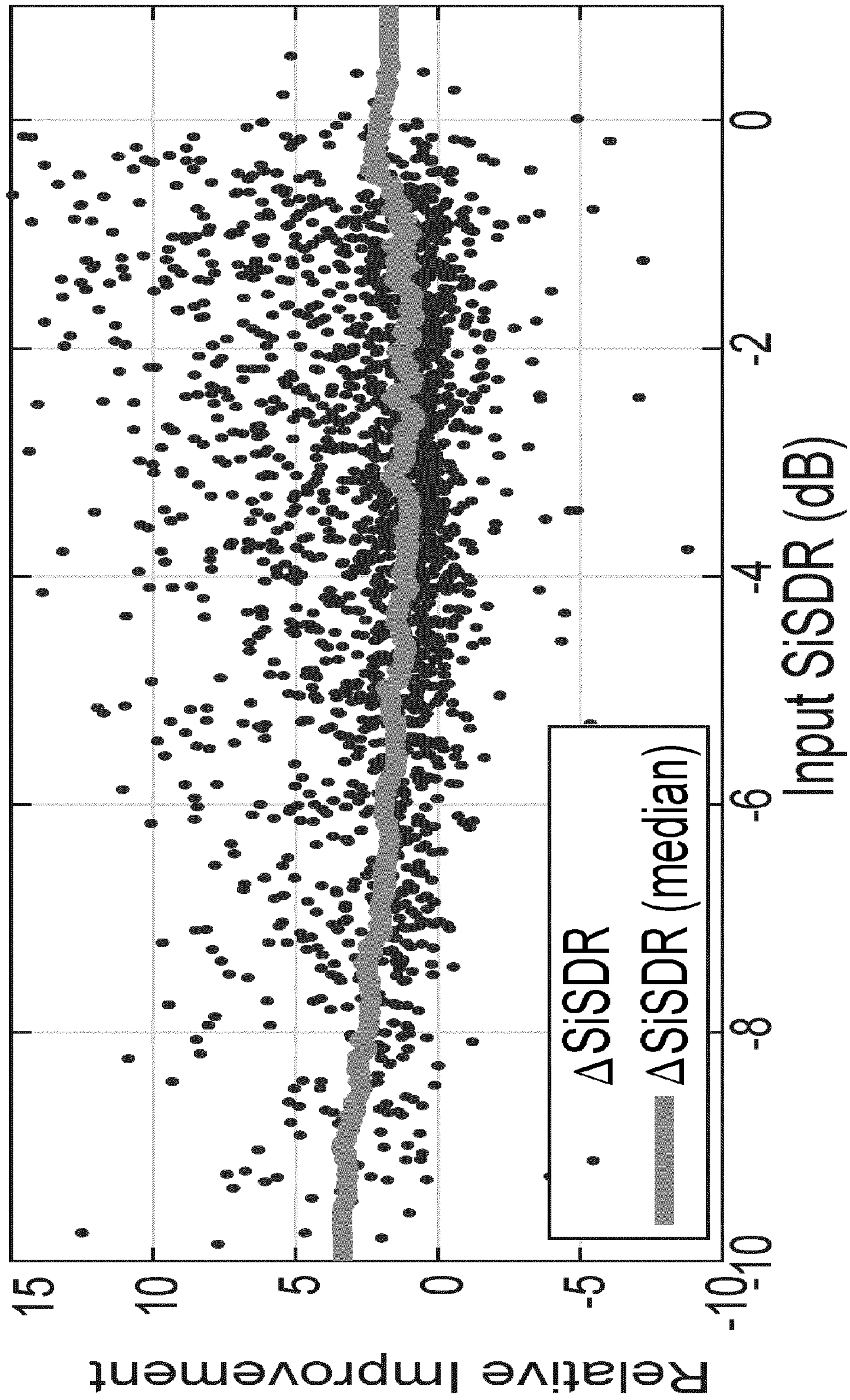

Relative improvement
15
10
5
0
-5
-10
-10
-8
-6
-4
-2
0
Input SiSDR (dB)
ΔSiSDR
ΔSiSDR (median)


FIG. 47

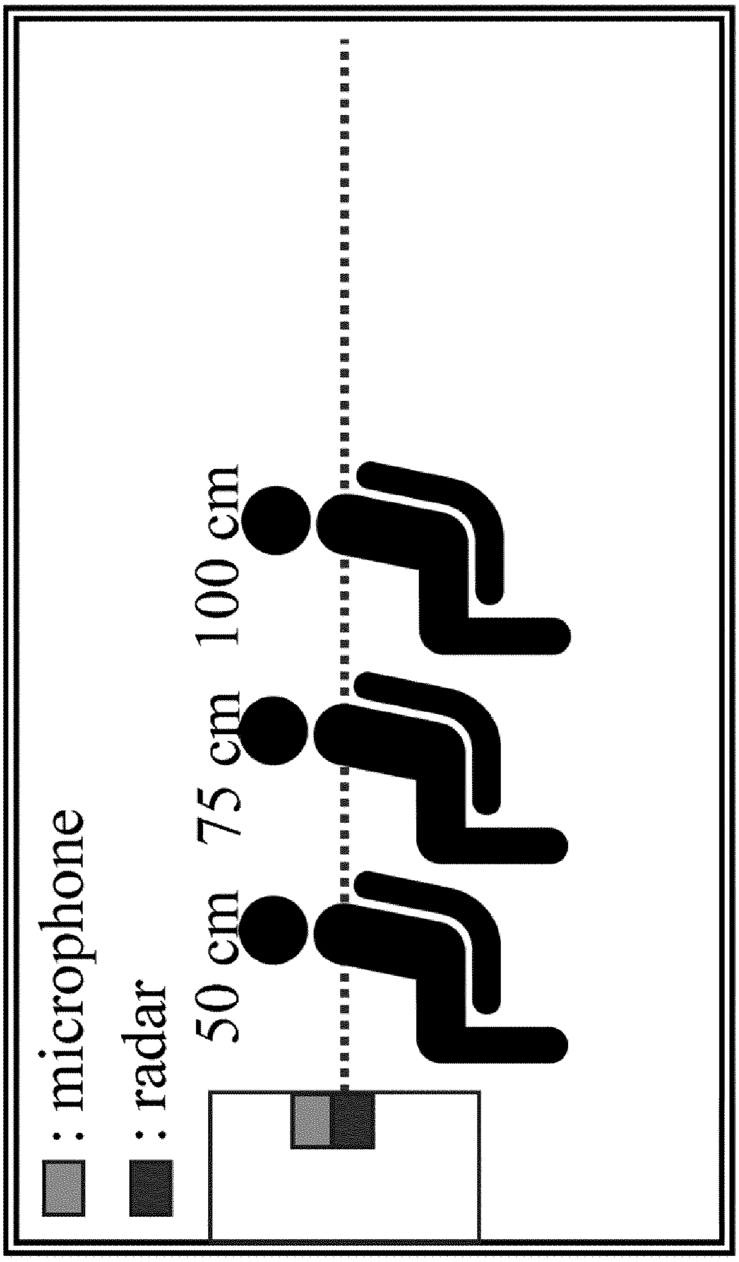


FIG. 48A

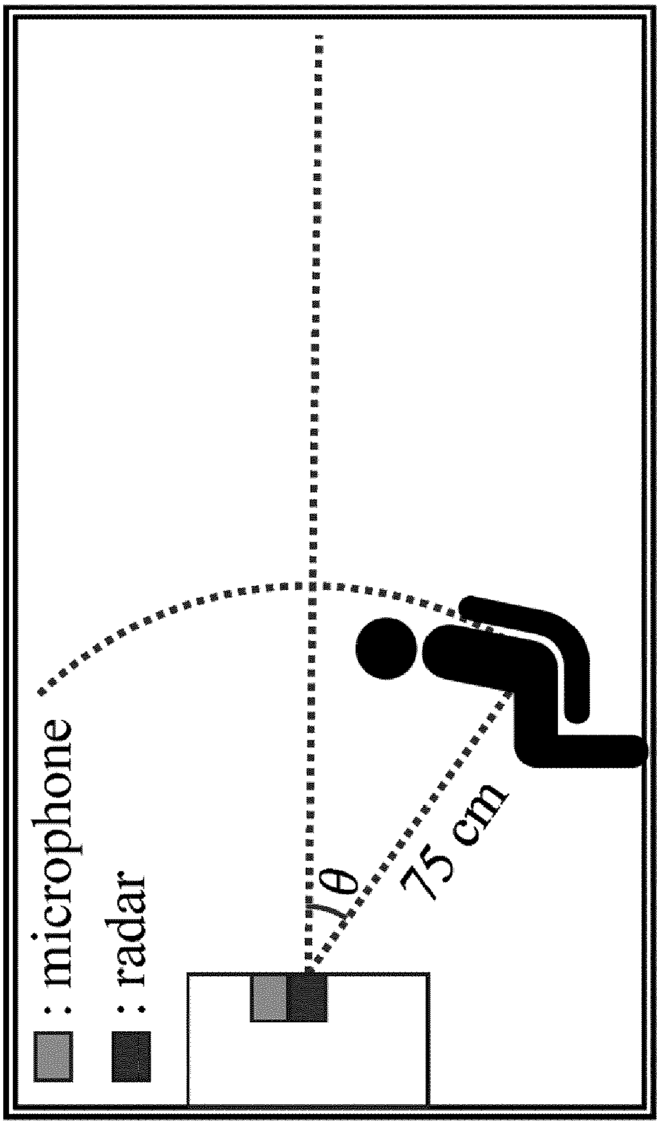


FIG. 48B

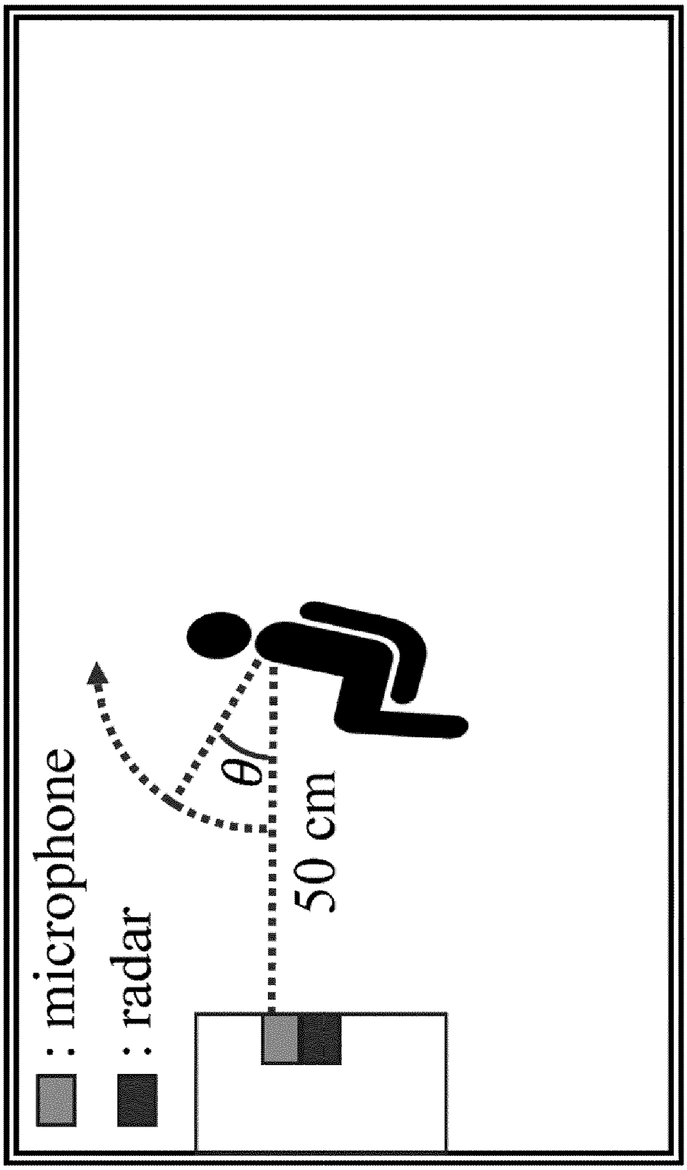


FIG. 48C

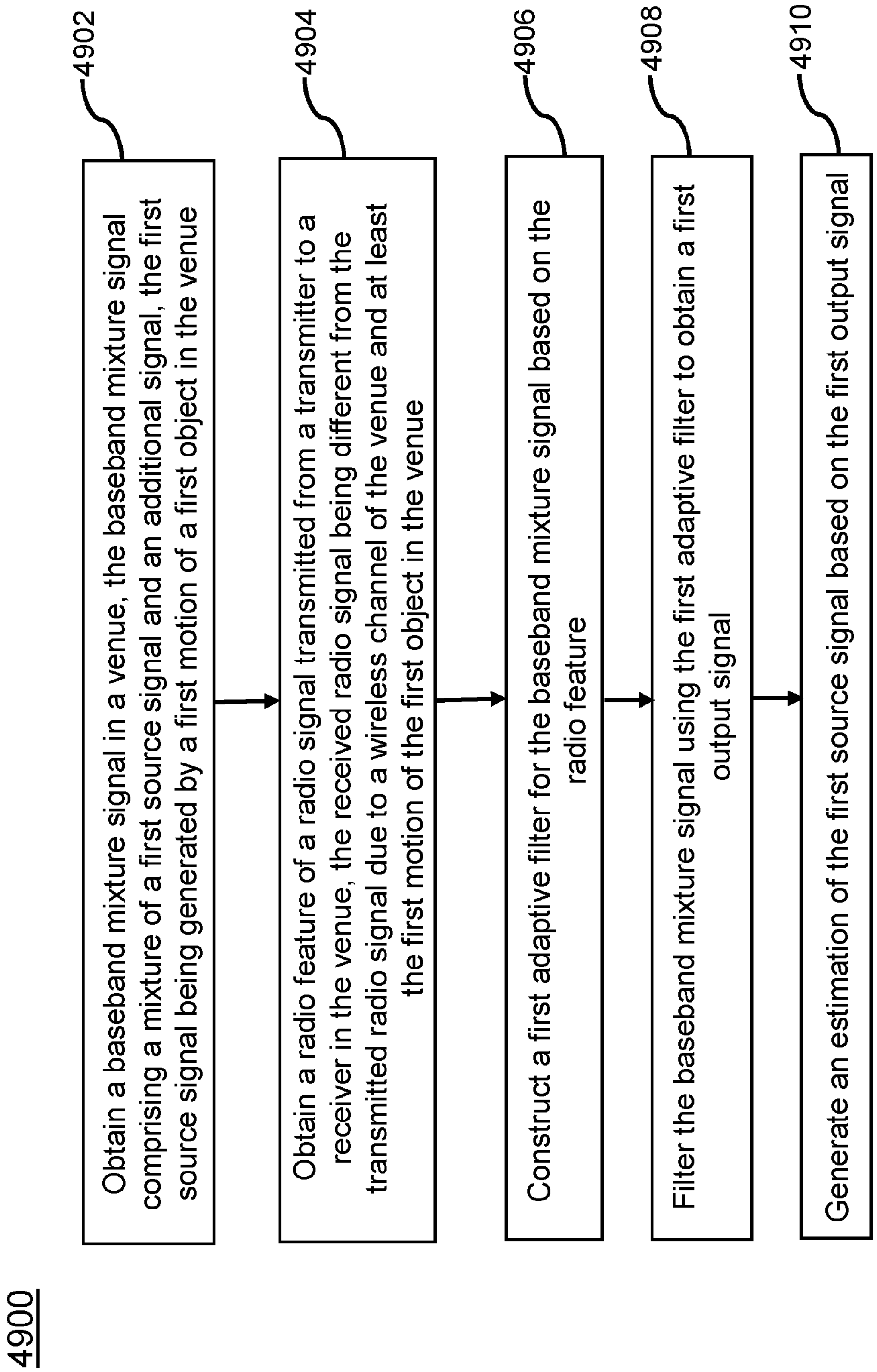
4900
Obtain a baseband mixture signal in a venue, the baseband mixture signal comprising a mixture of a first source signal and an additional signal, the first source signal being generated by a first motion of a first object in the venue
4902
Obtain a radio feature of a radio signal transmitted from a transmitter to a receiver in the venue, the received radio signal being different from the transmitted radio signal due to a wireless channel of the venue and at least the first motion of the first object in the venue
4904
Construct a first adaptive filter for the baseband mixture signal based on the radio feature
4906
Filter the baseband mixture signal using the first adaptive filter to obtain a first output signal
4908
Generate an estimation of the first source signal based on the first output signal
4910

FIG. 49

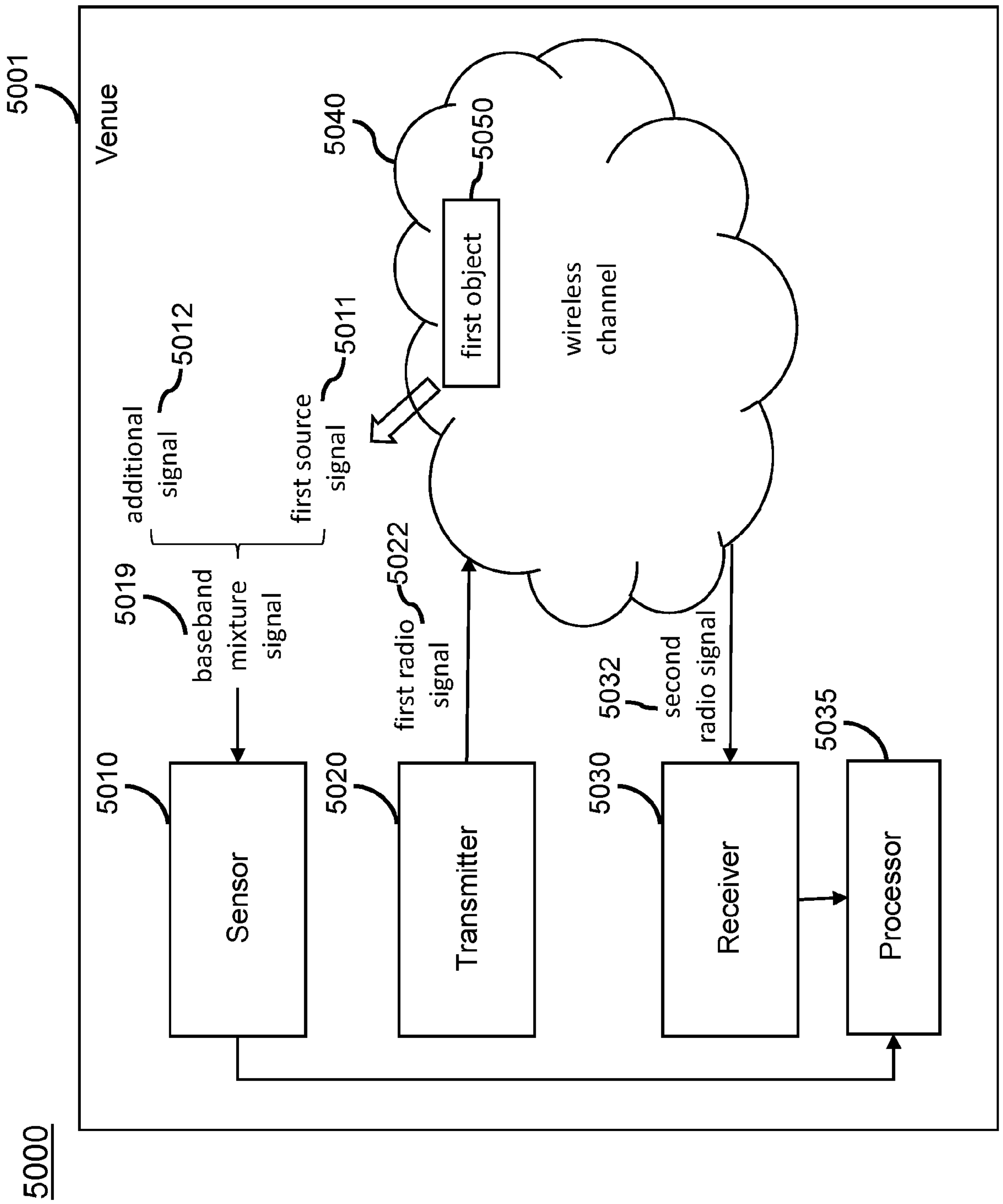
5000
5001
Venue
Sensor
5010
Transmitter
5020
Receiver
5030
Processor
5035
baseband mixture signal
5019
additional signal
5012
first source signal
5011
first radio signal
5022
second radio signal
5032
5040
first object
5050
wireless channel

FIG. 50

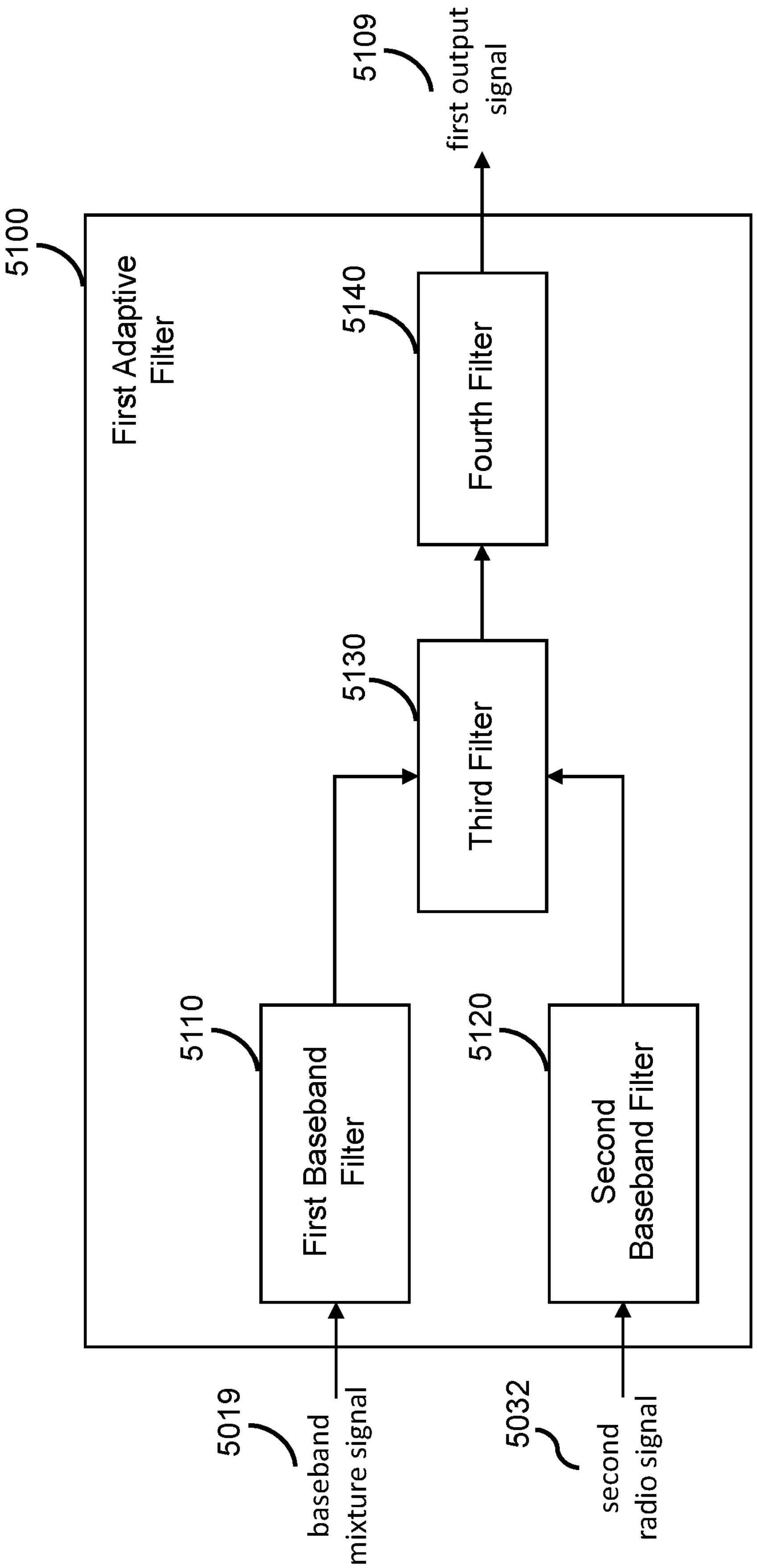
5109
first output signal
5100
First Adaptive Filter
5140
Fourth Filter
5130
Third Filter
5110
First Baseband Filter
5120
Second Baseband Filter
5019
baseband mixture signal
5032
second radio signal

FIG. 51

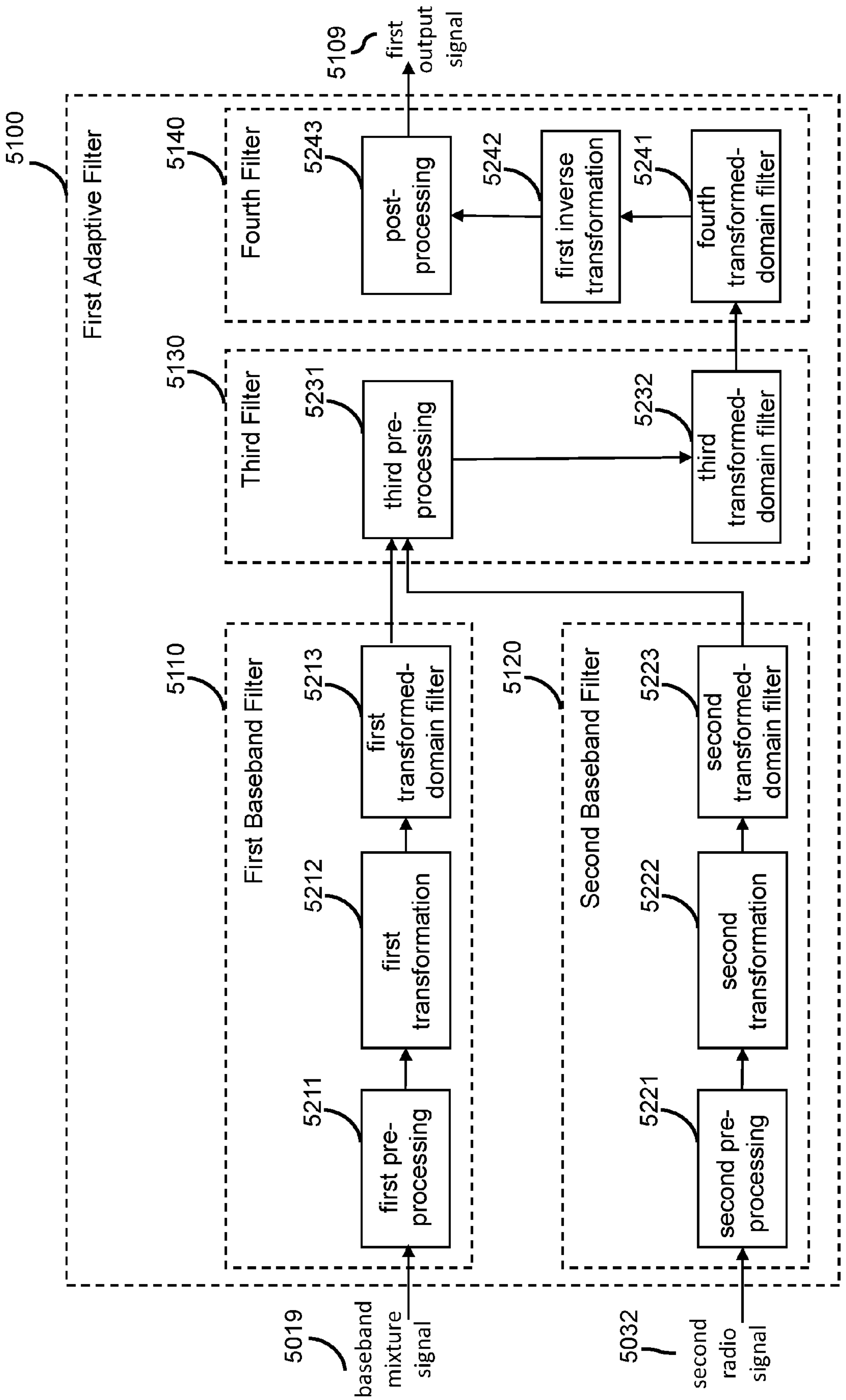
5100
First Adaptive Filter
5109
first output signal
5140
Fourth Filter
5243
post-processing
5242
first inverse transformation
5241
fourth transformed-domain filter
5130
Third Filter
5231
third pre-processing
5232
third transformed-domain filter
5110
First Baseband Filter
5211
first pre-processing
5212
first transformation
5213
first transformed-domain filter
5120
Second Baseband Filter
5221
second pre-processing
5222
second transformation
5223
second transformed-domain filter
5019
baseband mixture signal
5032
second radio signal

FIG. 52

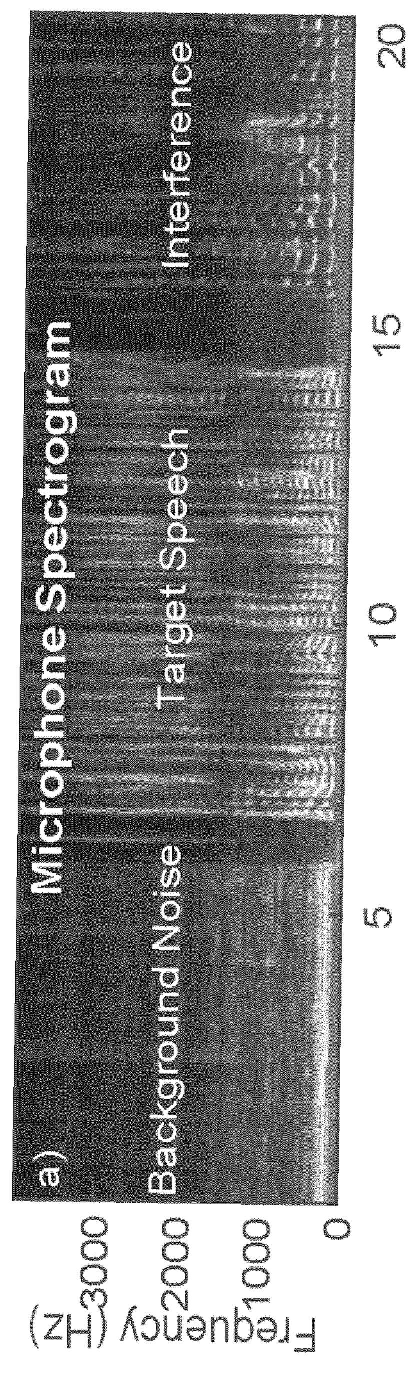
a)
Microphone Spectrogram
Background Noise
Target Speech
Interference
Frequency (Hz)
3000
2000
1000
0
5
10
15
20

FIG. 53A

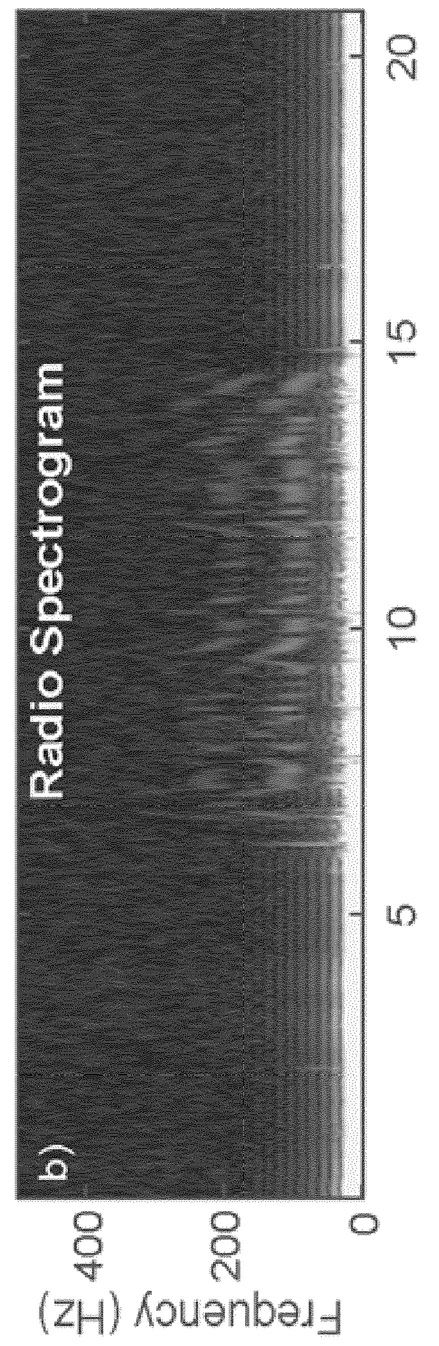


FIG. 53B

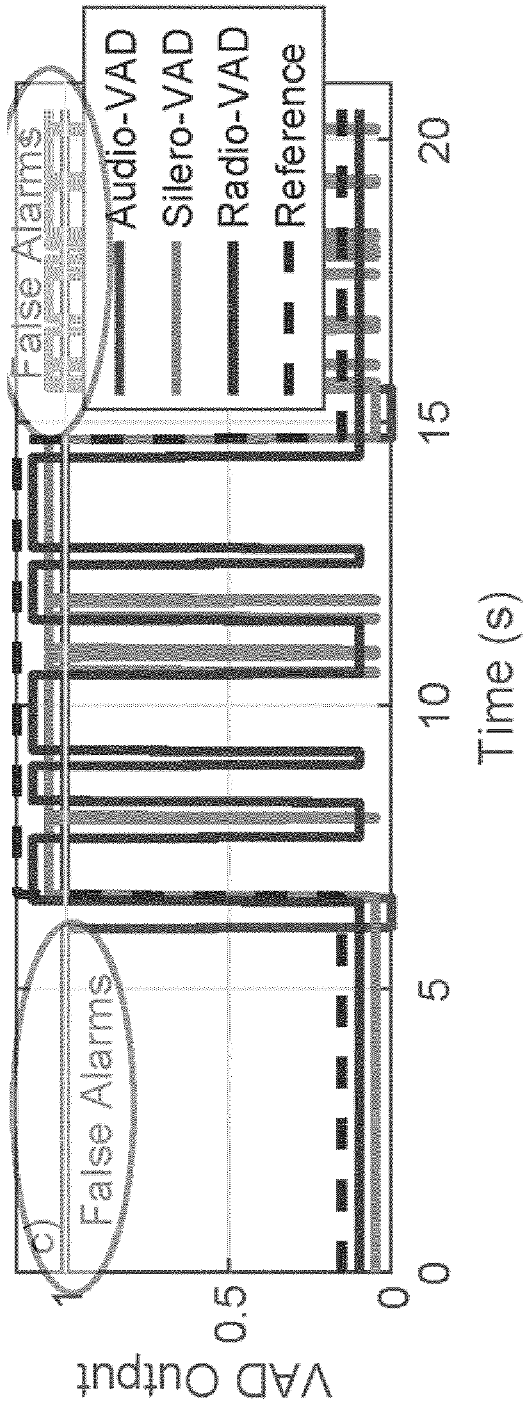
c)
False Alarms
False Alarms
Audio-VAD
Silero-VAD
Radio-VAD
Reference
VAD Output
0
0.5
1
0
5
10
15
20
Time (s)

FIG. 53C

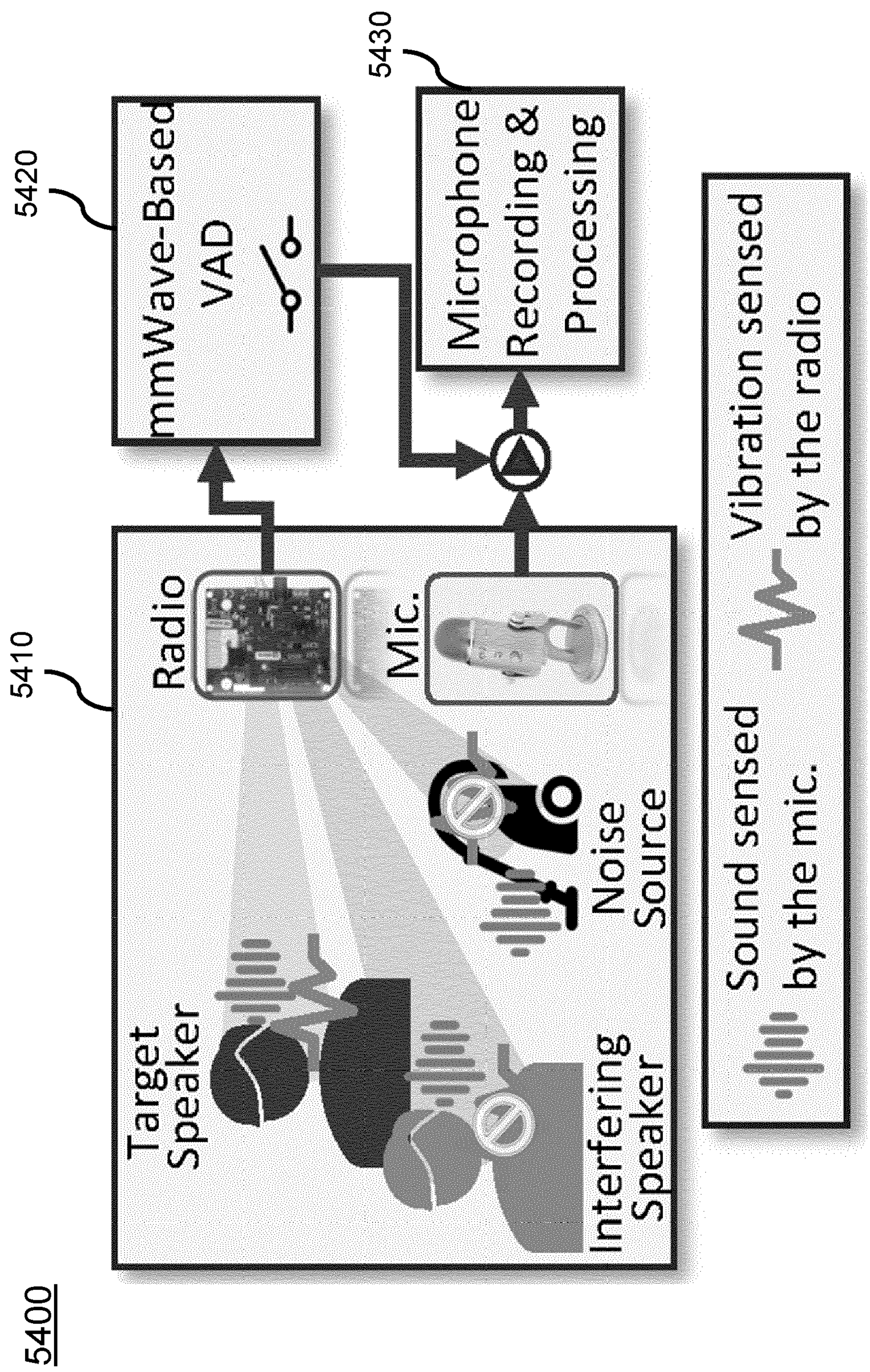
5400
5410
5420
5430
Target Speaker
Interfering Speaker
Noise Source
Radio
Mic.
mmWave-Based VAD
Microphone Recording & Processing
Sound sensed by the mic.
Vibration sensed by the radio

FIG. 54

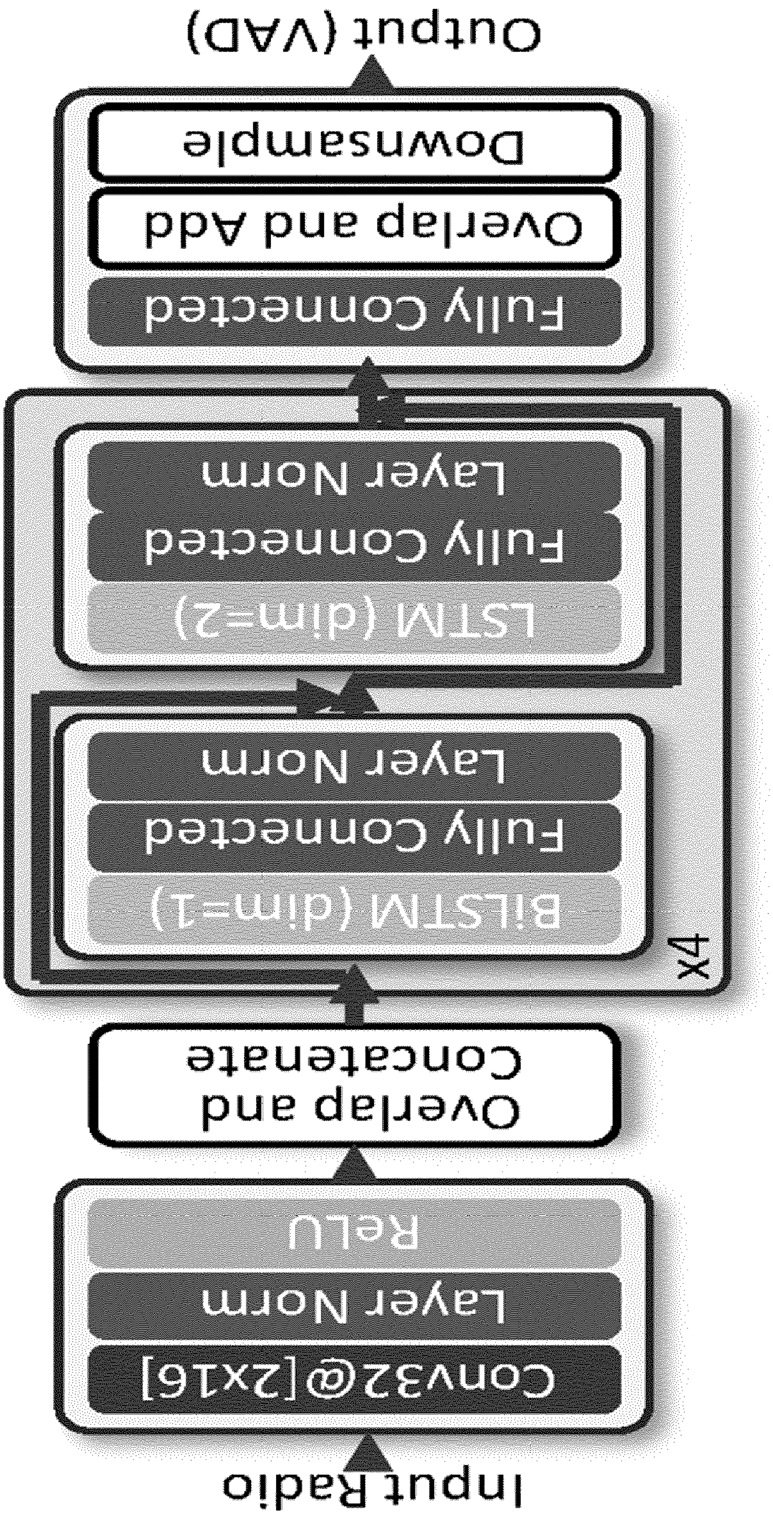
Input Radio
Conv32@[2x16]
Layer Norm
ReLU
Overlap and Concatenate
BiLSTM (dim=1)
Fully Connected
Layer Norm
LSTM (dim=2)
Fully Connected
Layer Norm
x4
Fully Connected
Overlap and Add
Downsample
Output (VAD)

FIG. 55

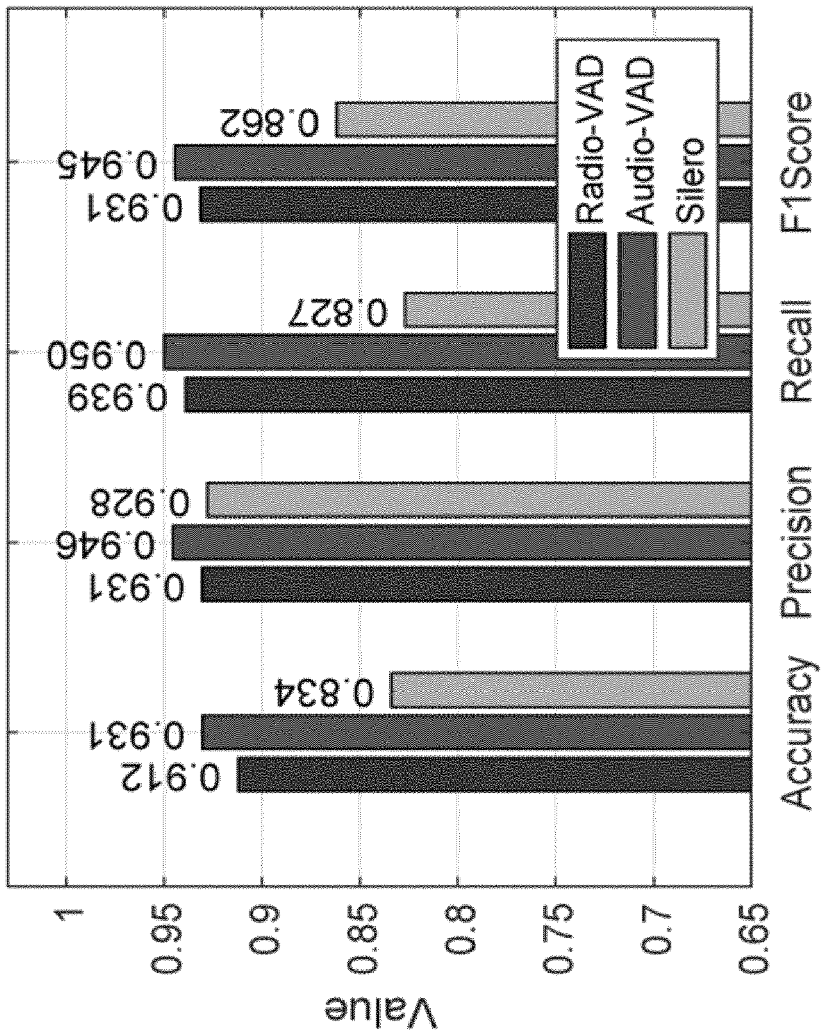
Value
1
0.95
0.9
0.85
0.8
0.75
0.7
0.65
Accuracy
Precision
Recall
F1Score
0.912
0.931
0.834
0.931
0.946
0.928
0.939
0.950
0.827
0.931
0.945
0.862
Radio-VAD
Audio-VAD
Silero

FIG.56A

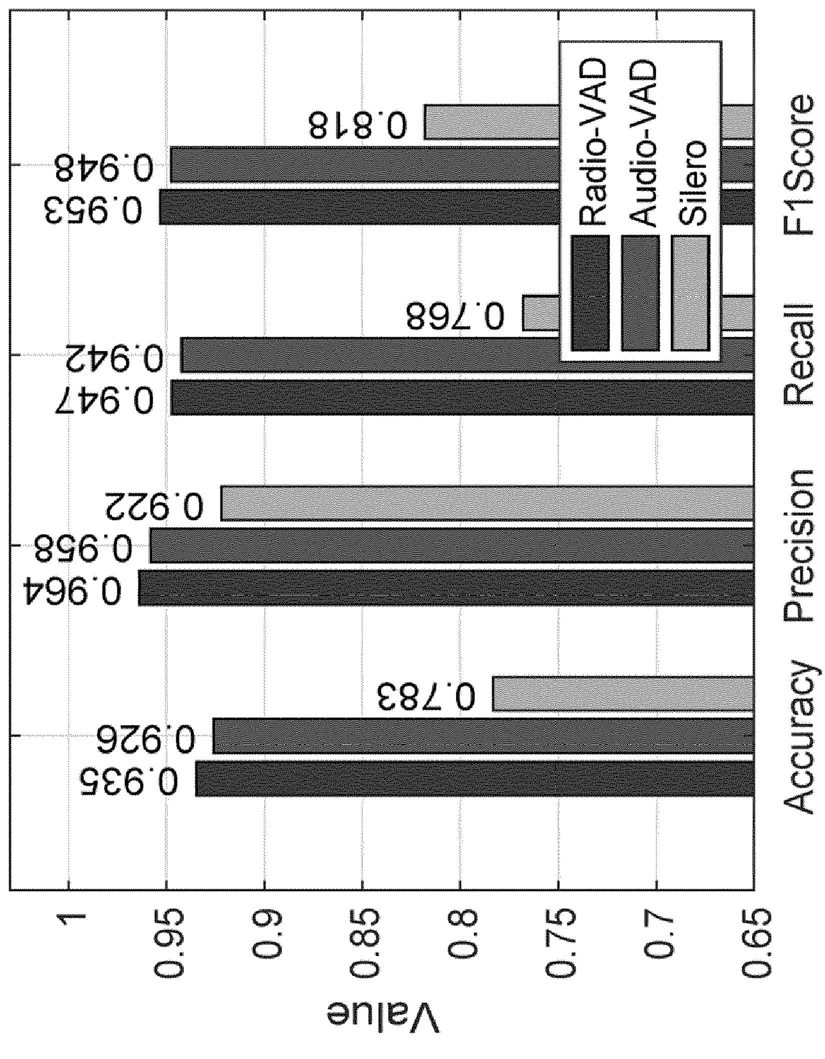
Value
1
0.95
0.9
0.85
0.8
0.75
0.7
0.65
Accuracy
Precision
Recall
F1Score
0.935
0.926
0.783
0.964
0.958
0.922
0.947
0.942
0.768
0.953
0.948
0.818
Radio-VAD
Audio-VAD
Silero

FIG. 56B

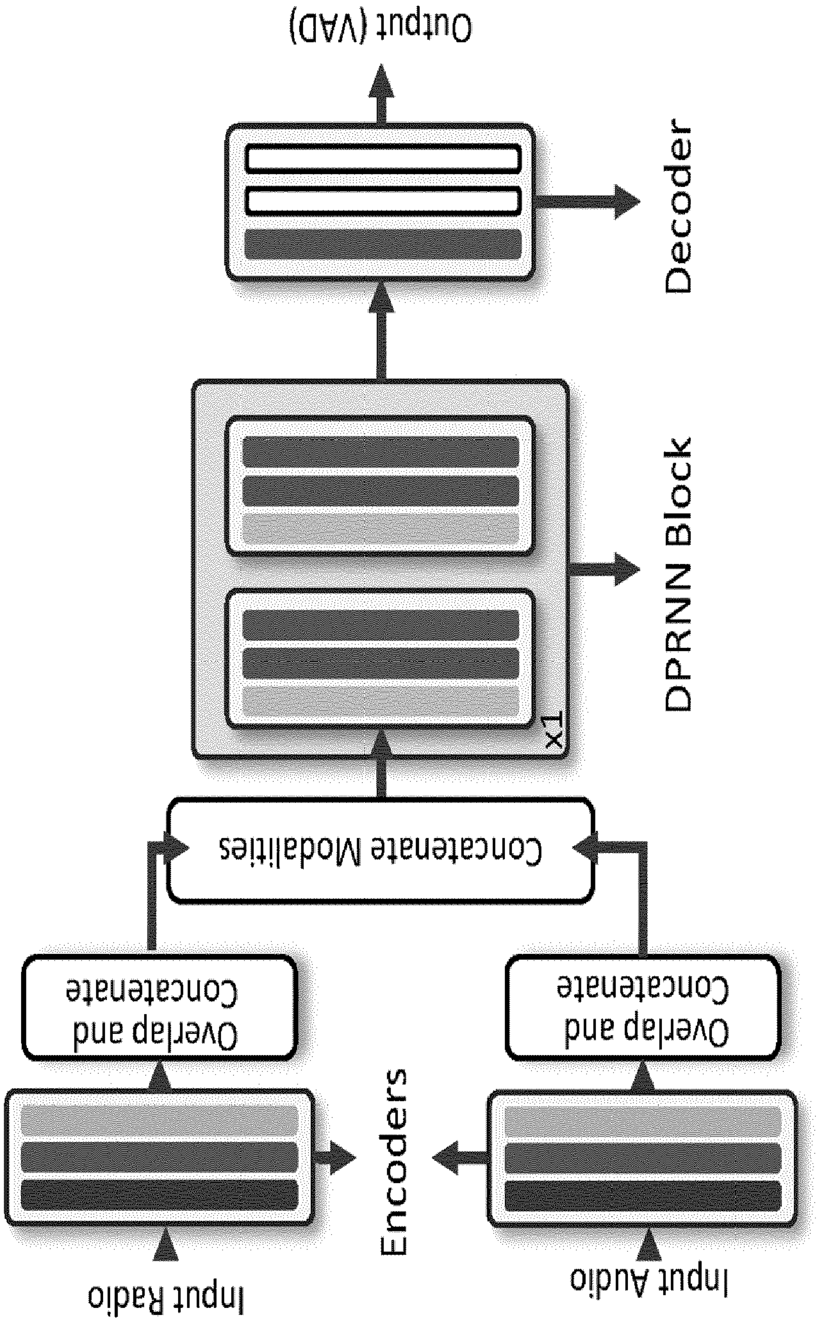

Input Radio
Input Audio
Encoders
Overlap and Concatenate
Overlap and Concatenate
Concatenate Modalities
x1
DPRNN Block
Decoder
Output (VAD)


FIG. 57

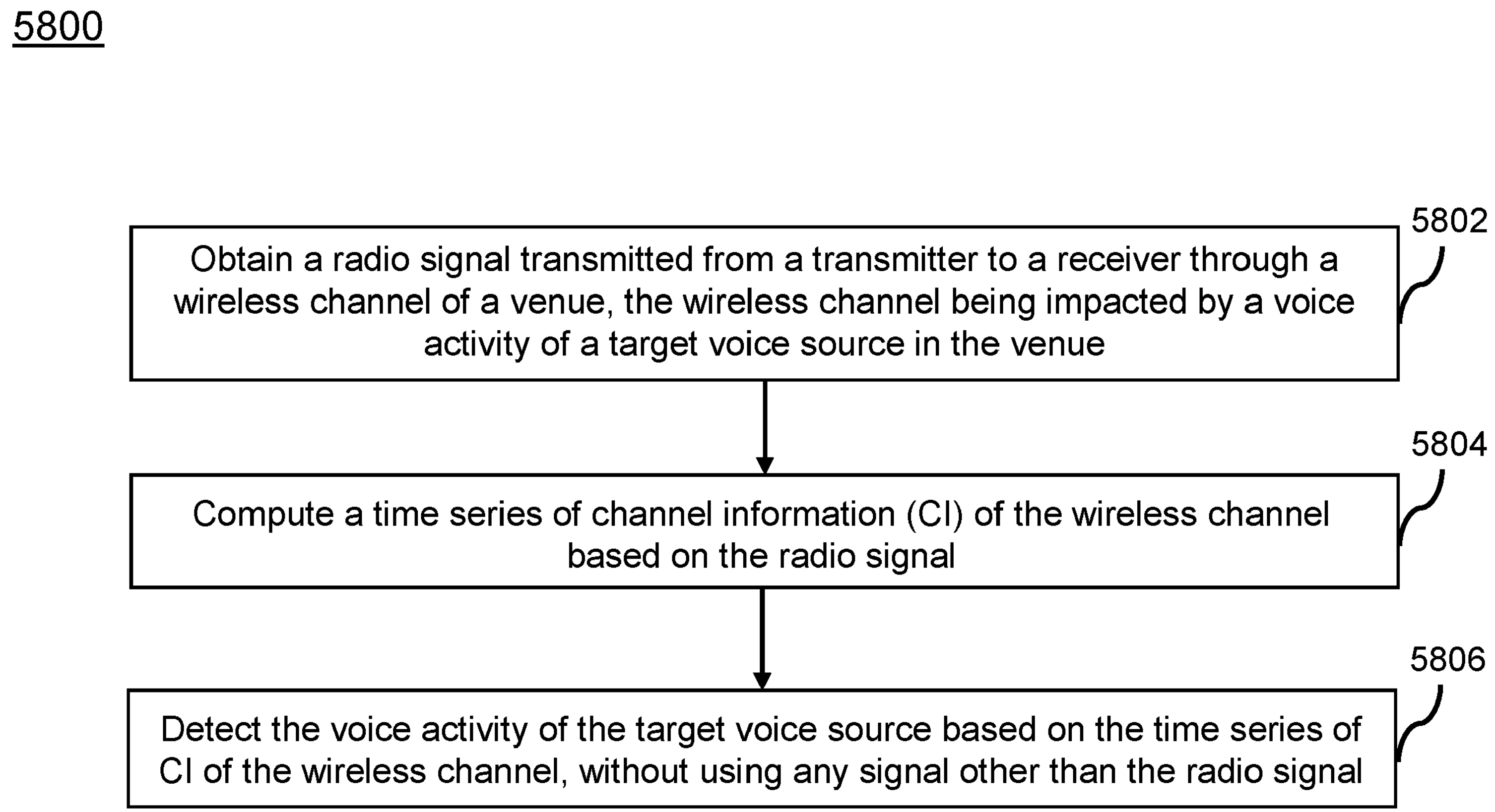
5800
Obtain a radio signal transmitted from a transmitter to a receiver through a wireless channel of a venue, the wireless channel being impacted by a voice activity of a target voice source in the venue
5802
Compute a time series of channel information (CI) of the wireless channel based on the radio signal
5804
Detect the voice activity of the target voice source based on the time series of CI of the wireless channel, without using any signal other than the radio signal
5806

FIG. 58

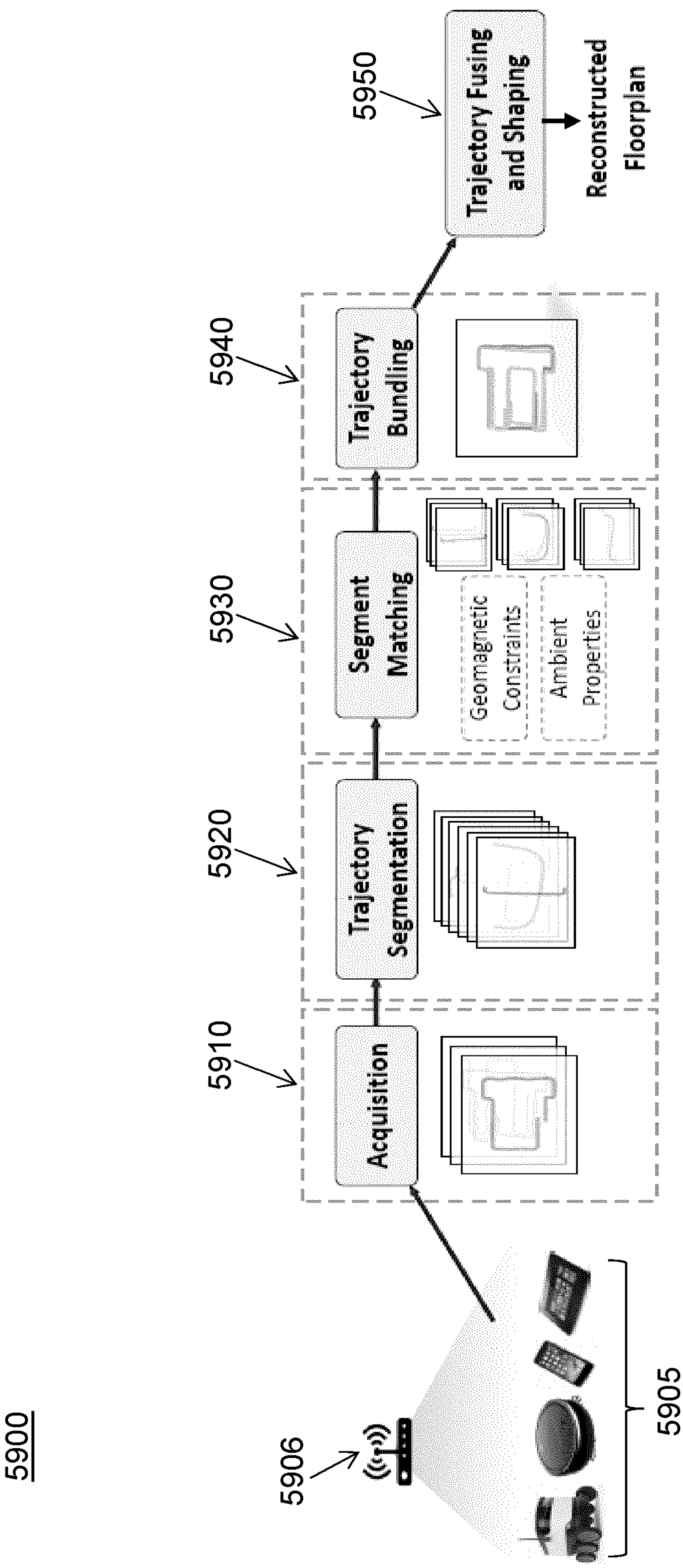
5900
5906
5905
5910
Acquisition
5920
Trajectory Segmentation
5930
Segment Matching
Geomagnetic Constraints
Ambient Properties
5940
Trajectory Bundling
5950
Trajectory Fusing and Shaping
Reconstructed Floorplan

FIG. 59

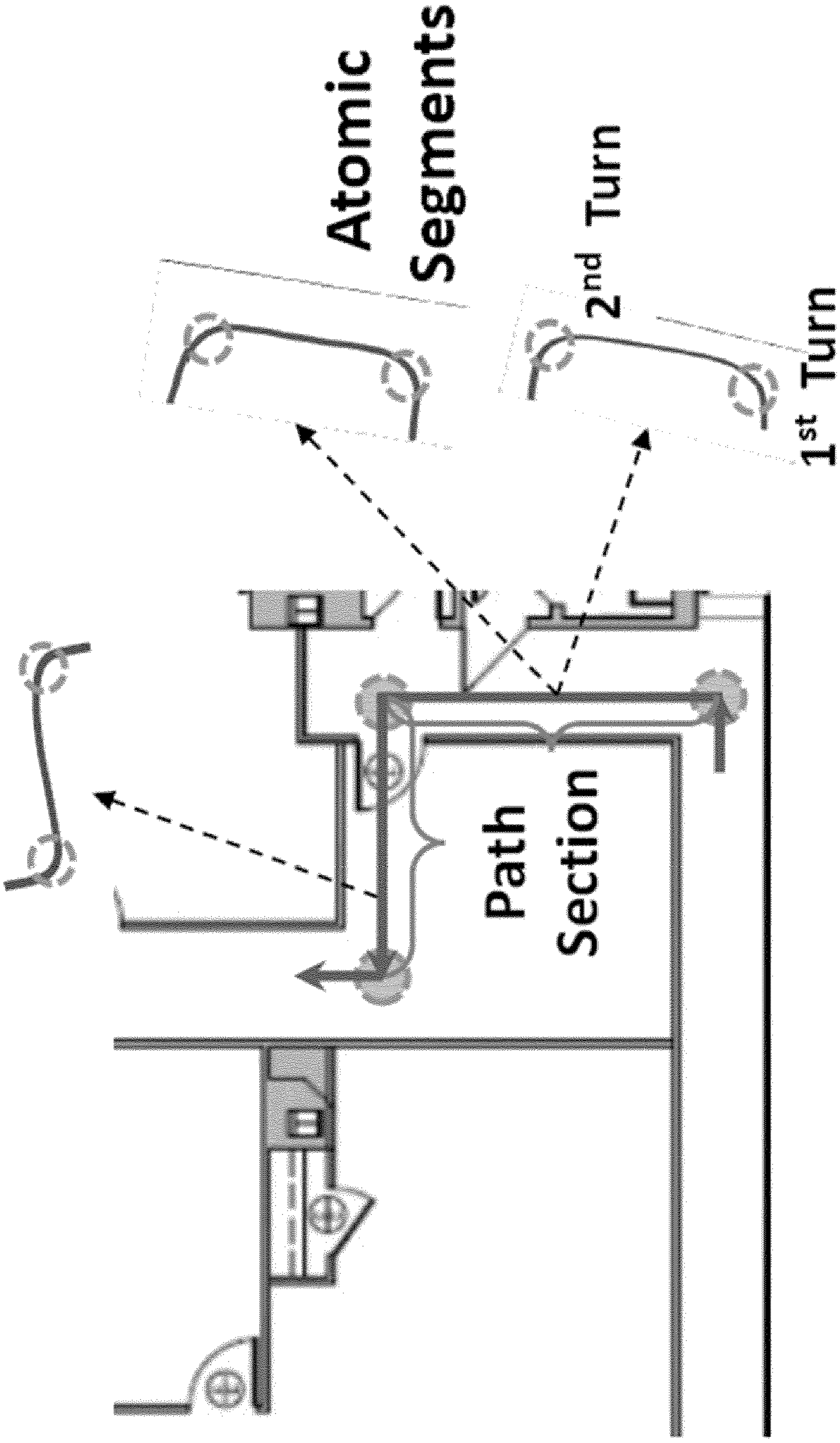


FIG. 60

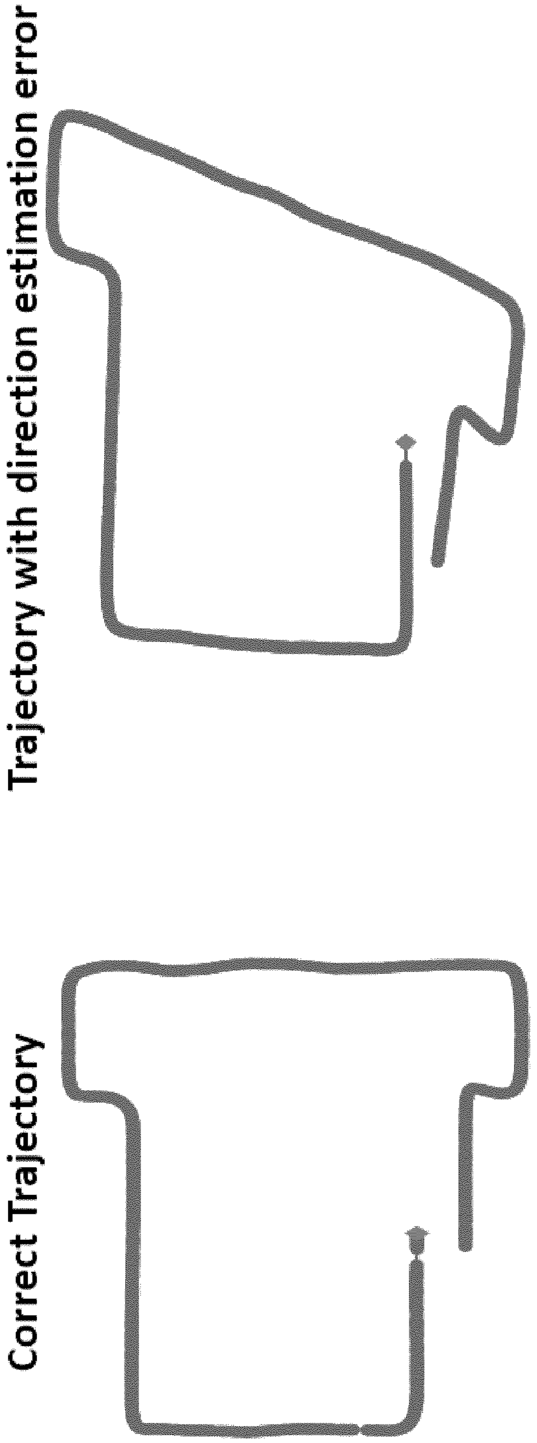
Trajectory with direction estimation error
Correct Trajectory

FIG. 61

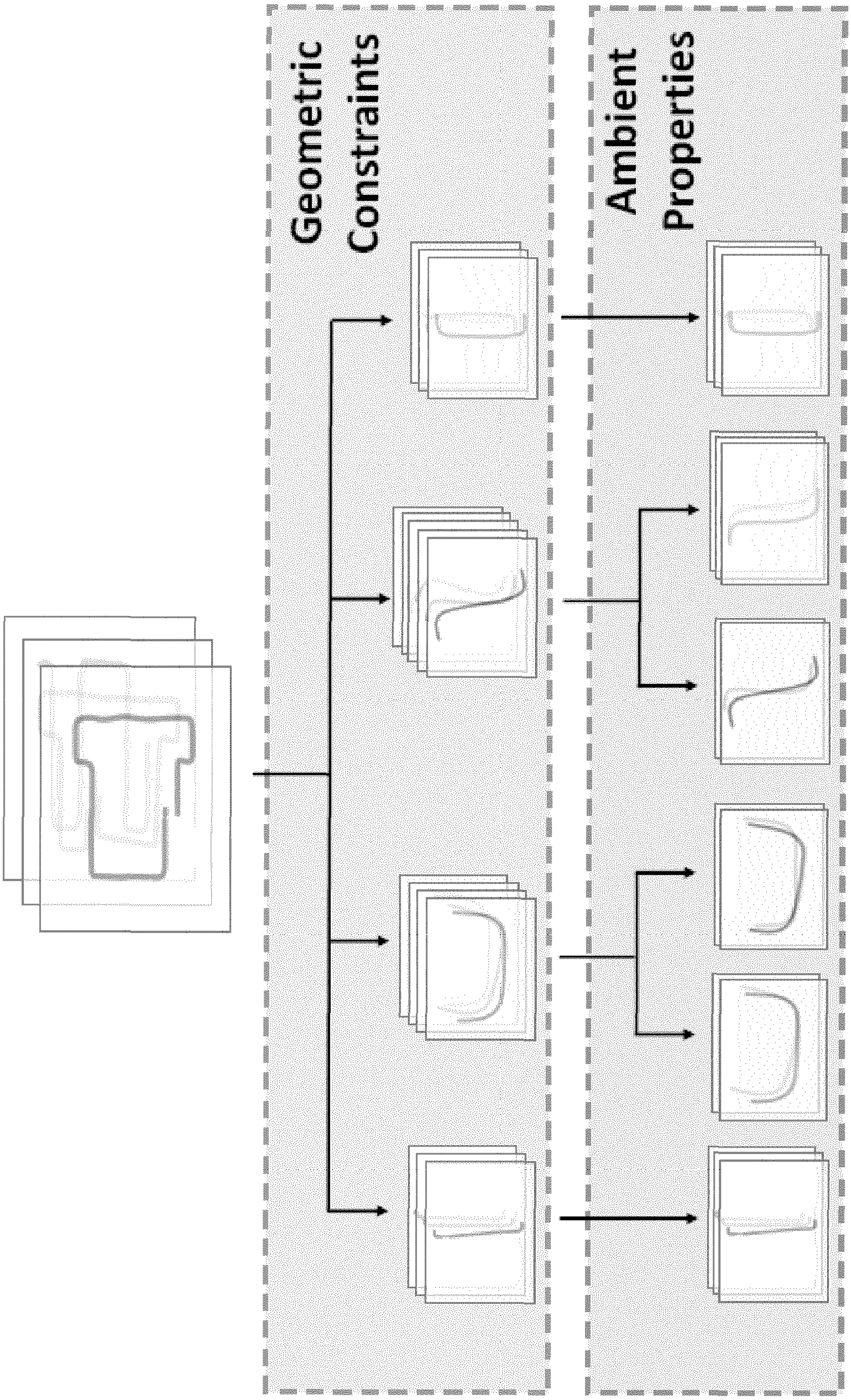


FIG. 62

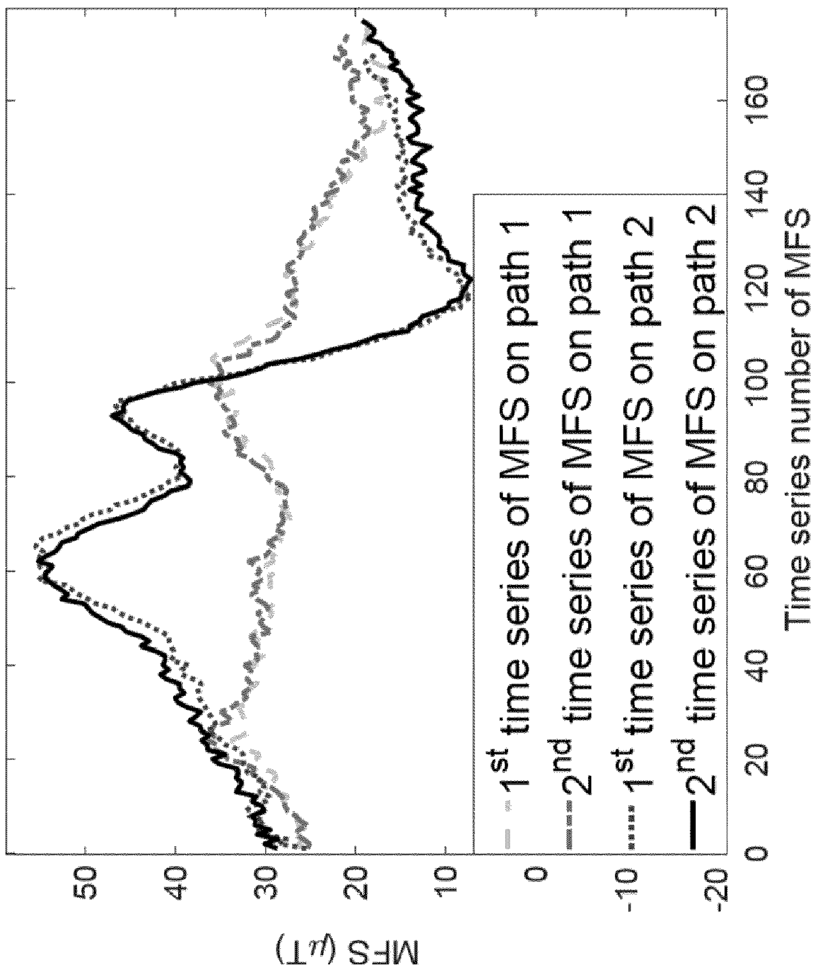
MFS (μT)
50
40
30
20
10
0
-10
-20
0
20
40
60
80
100
120
140
160
Time series number of MFS
1st time series of MFS on path 1
2nd time series of MFS on path 1
1st time series of MFS on path 2
2nd time series of MFS on path 2

FIG. 63A

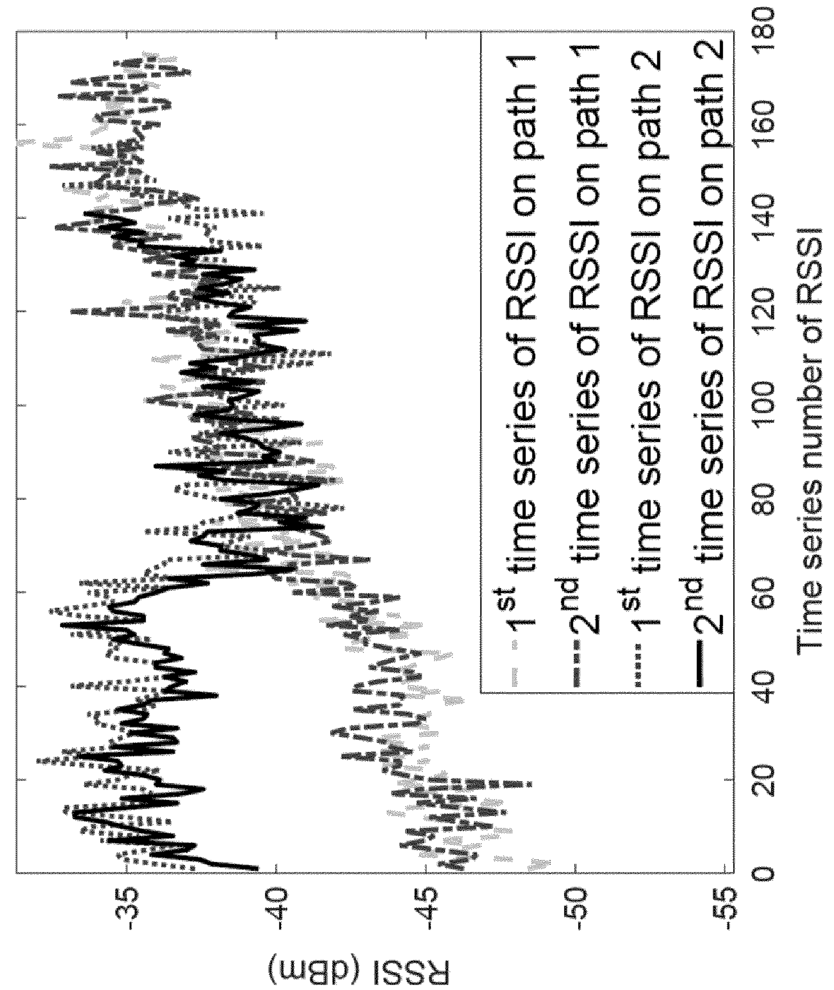
1st time series of RSSI on path 1
2nd time series of RSSI on path 1
1st time series of RSSI on path 2
2nd time series of RSSI on path 2
RSSI (dBm)
Time series number of RSSI
-35
-40
-45
-50
-55
0
20
40
60
80
100
120
140
160
180

FIG. 63B

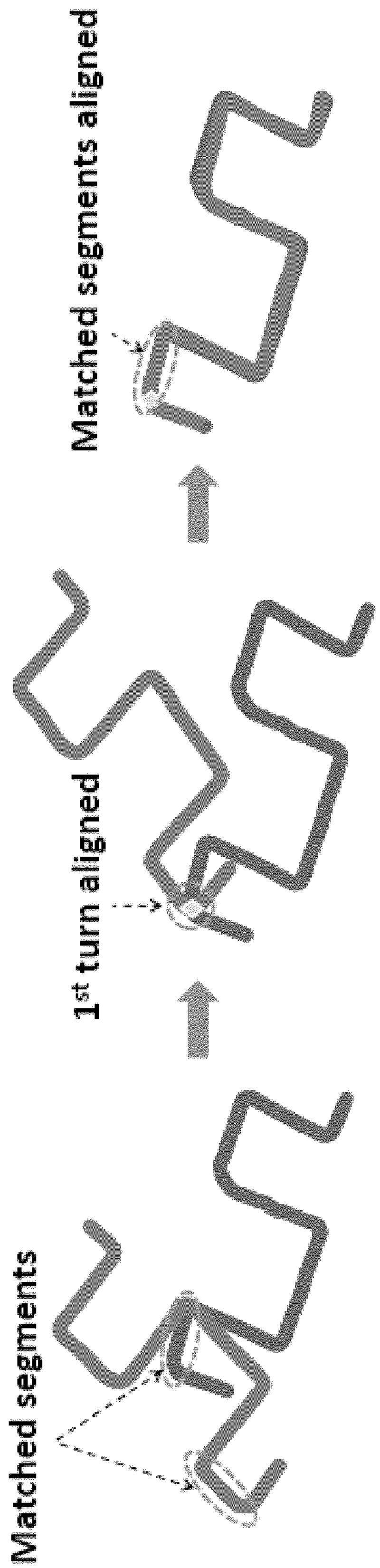

Matched segments
1st turn aligned
Matched segments aligned


FIG. 64

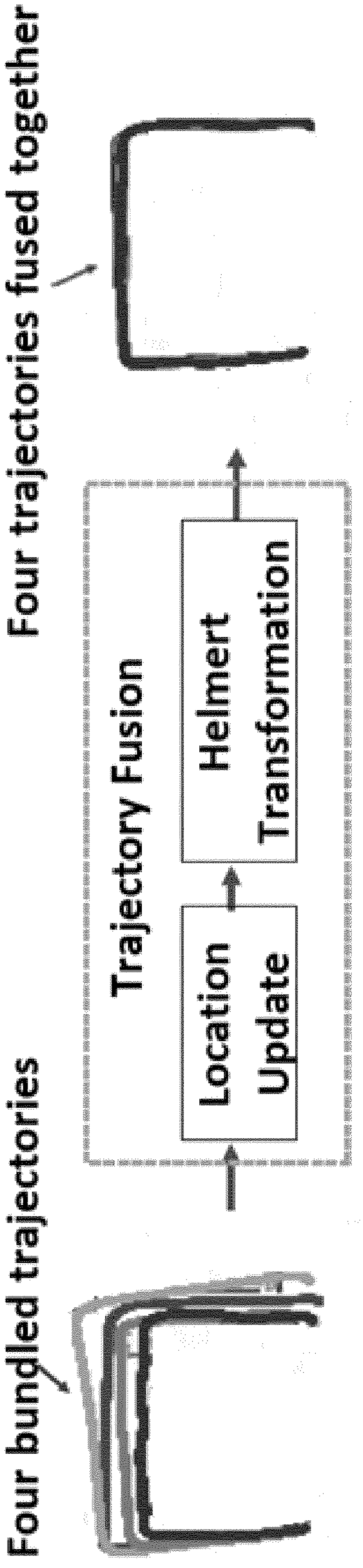
Four bundled trajectories
Trajectory Fusion
Location Update
Helmert Transformation
Four trajectories fused together

FIG. 65

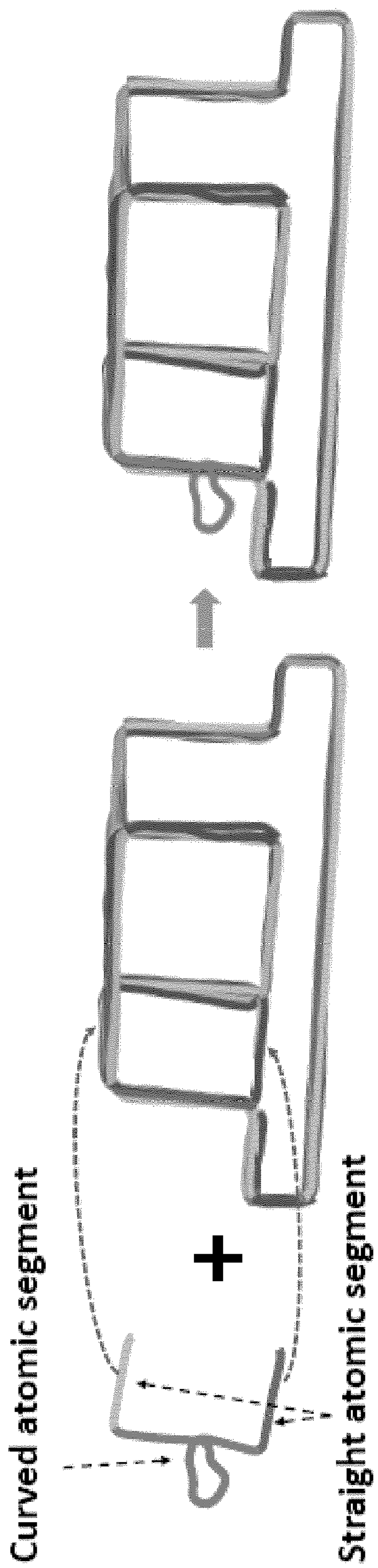


FIG. 66

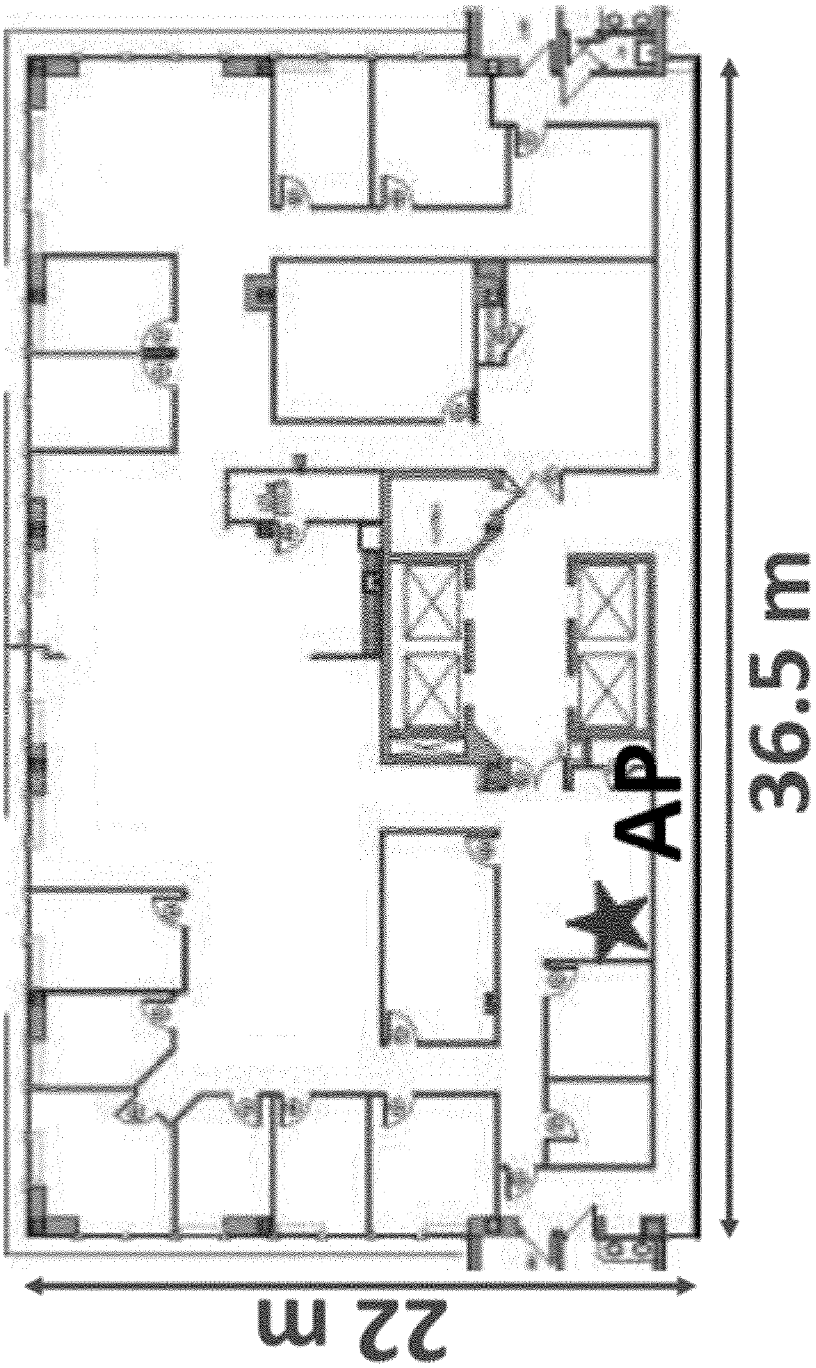


FIG. 67A

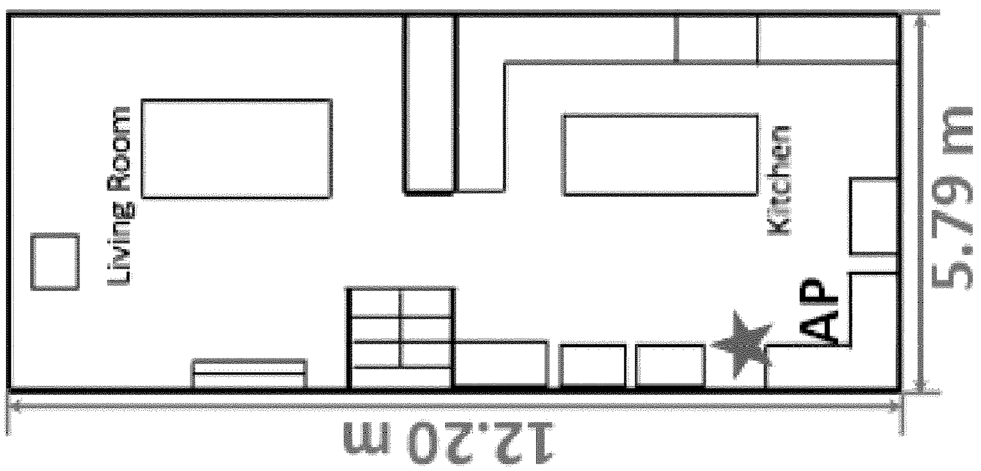


FIG. 67B

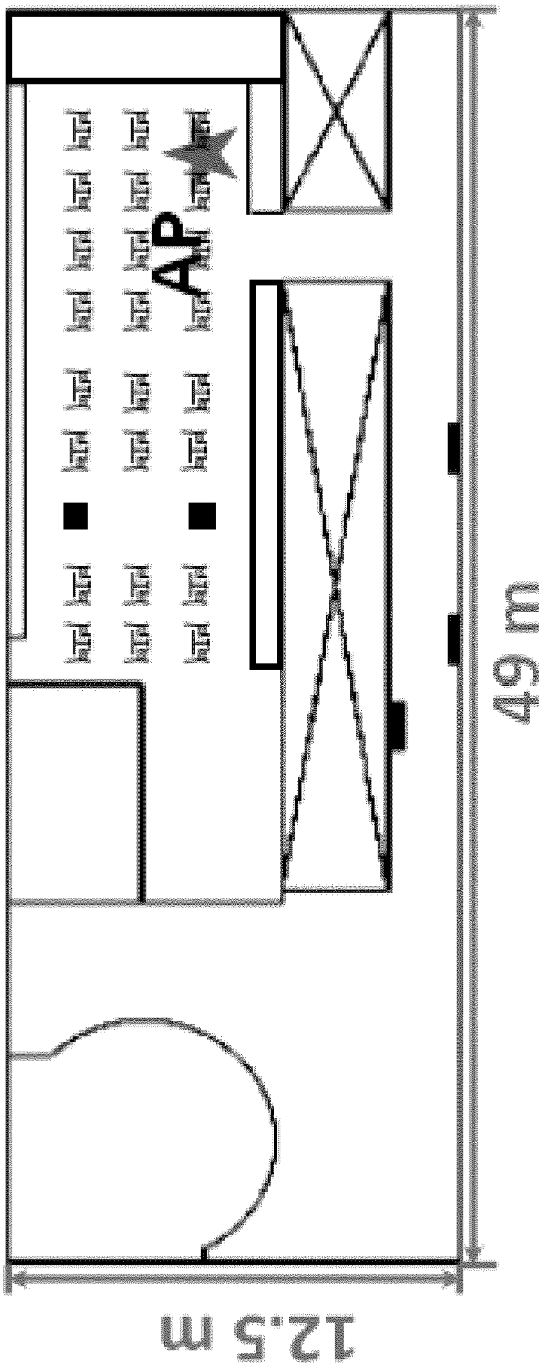


FIG. 67C

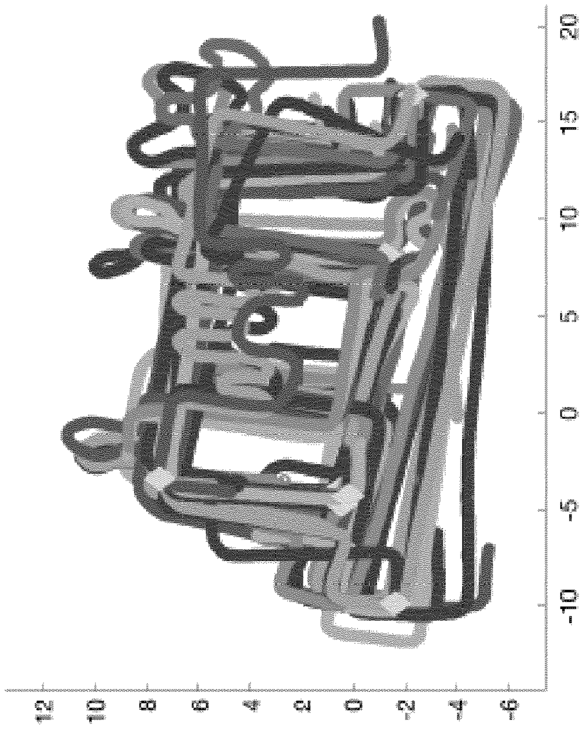

FIG. 68A

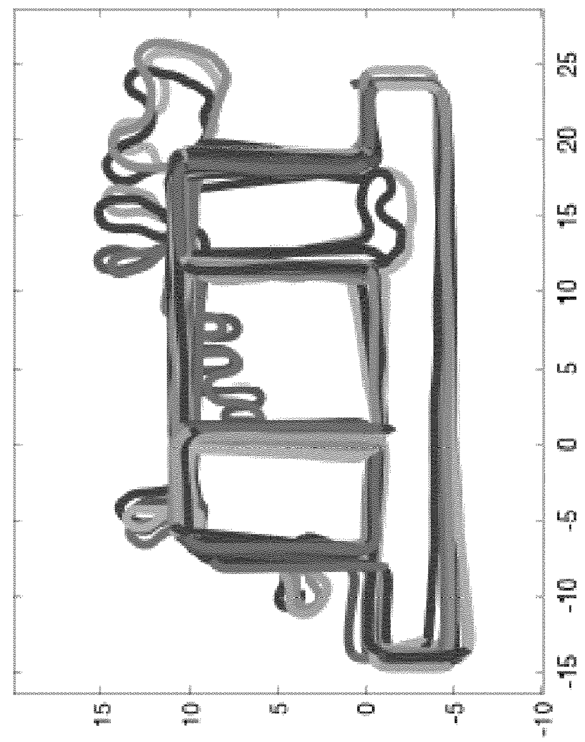

FIG. 68B

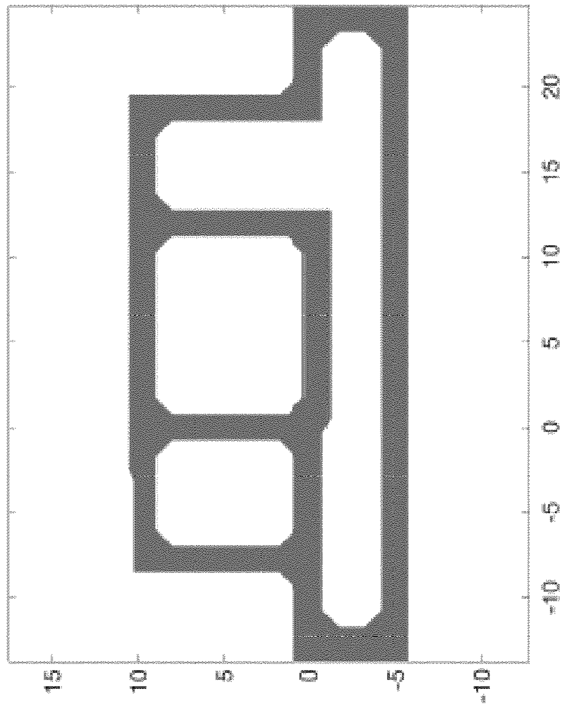

FIG. 68D

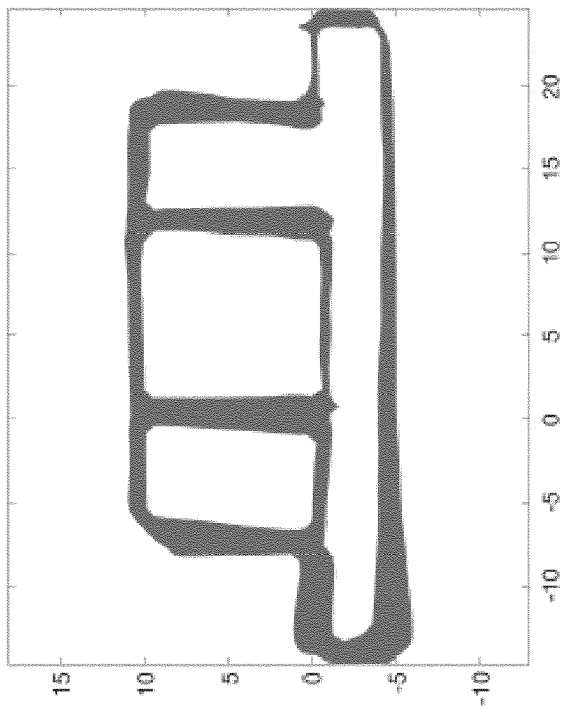

FIG. 68C

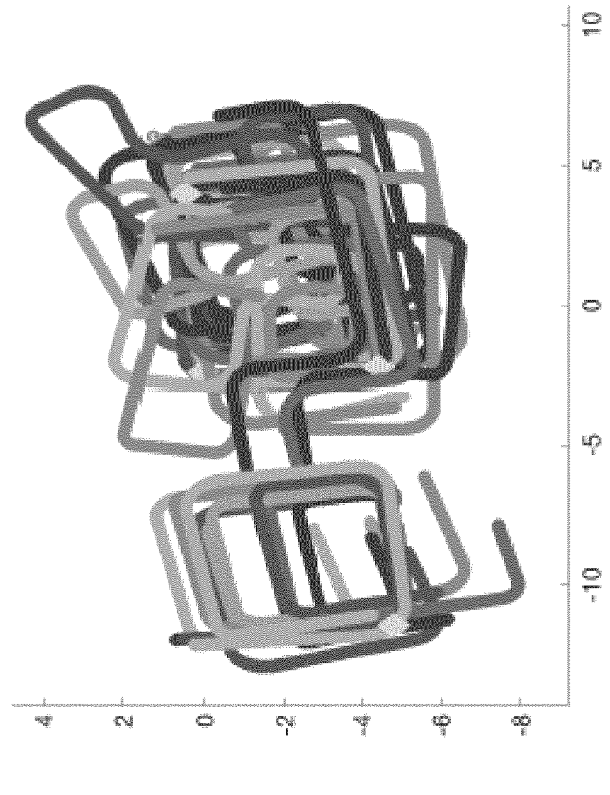


FIG. 69A

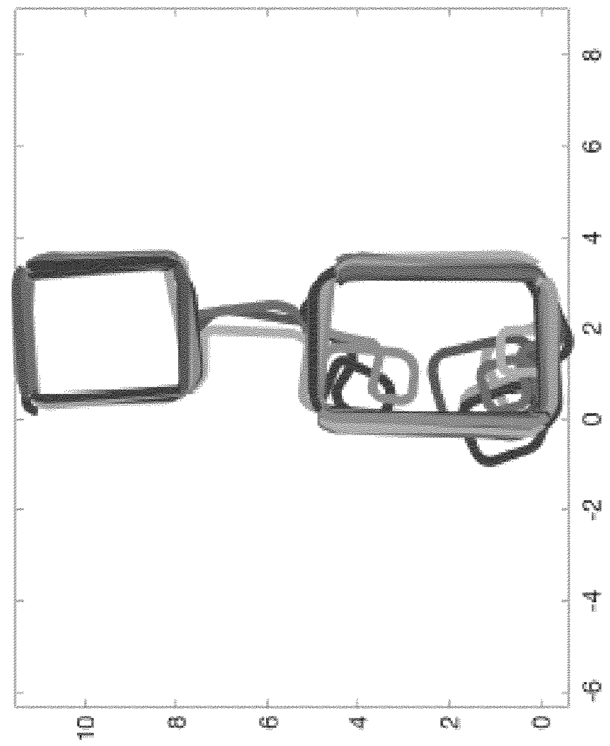


FIG. 69B

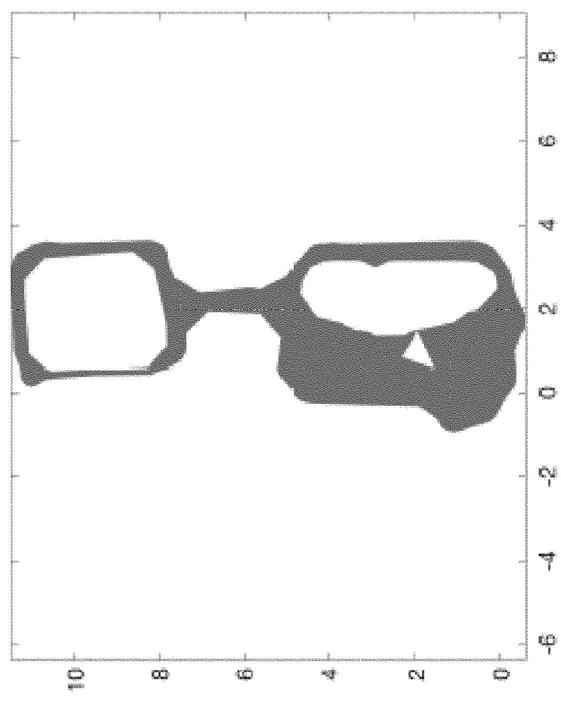


FIG. 69C

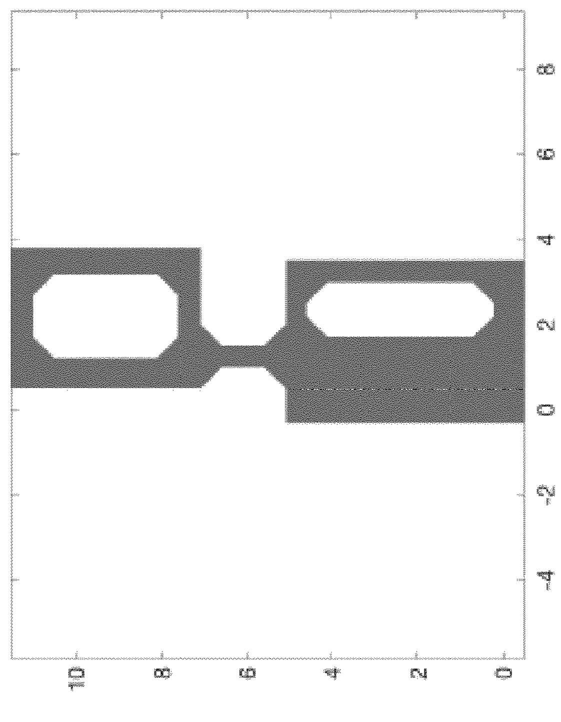


FIG. 69D

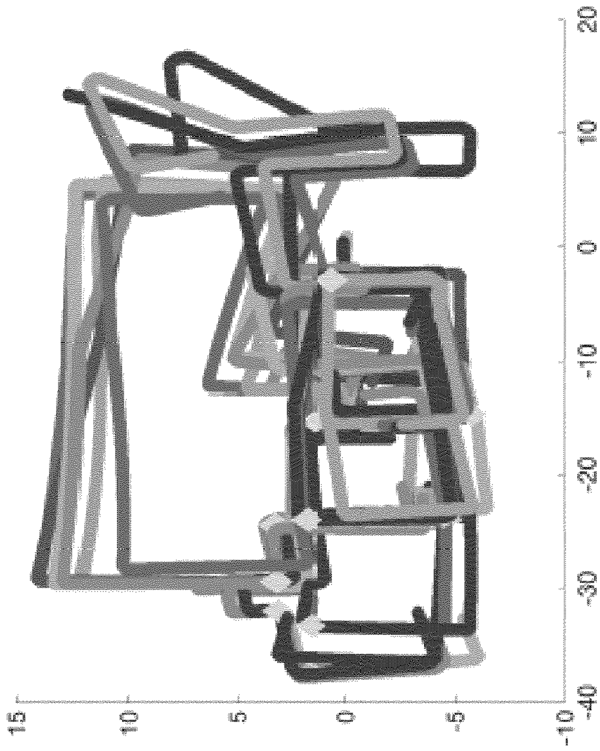

FIG. 70A

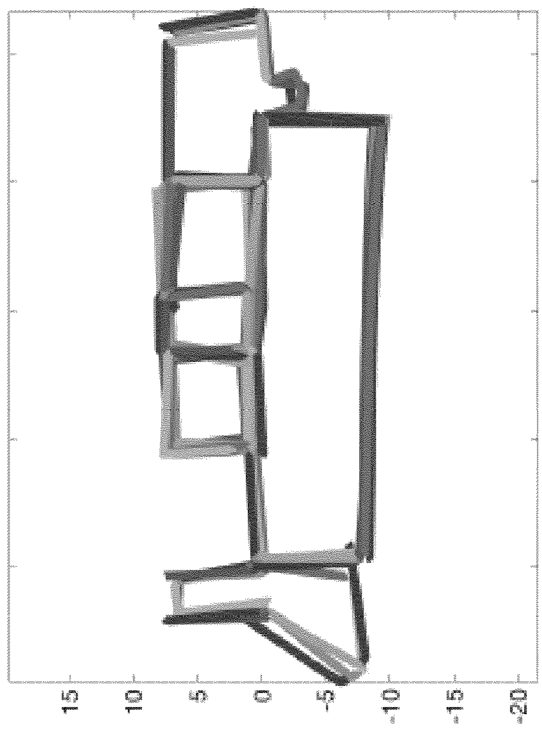

FIG. 70B

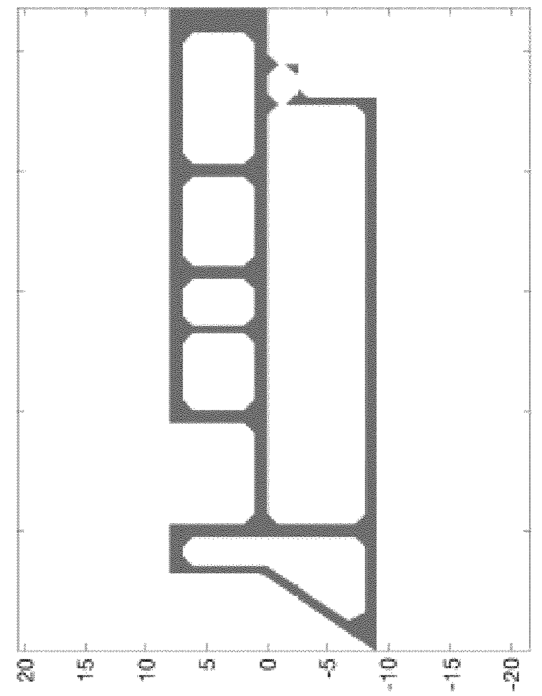


FIG. 70D

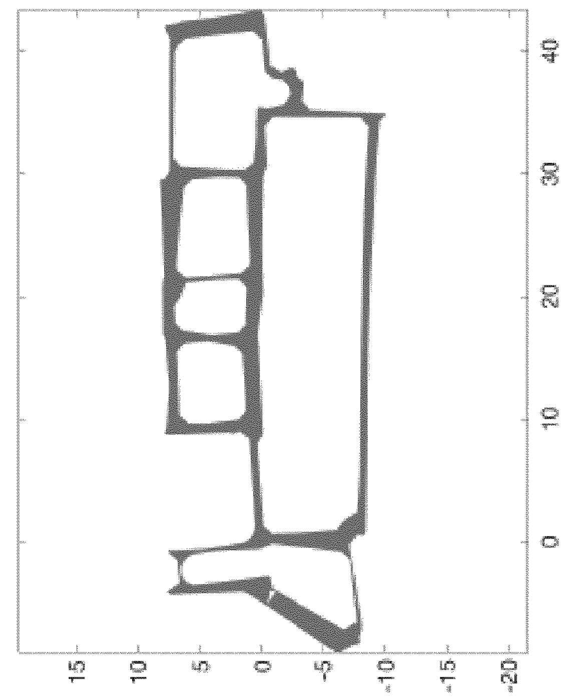


FIG. 70C

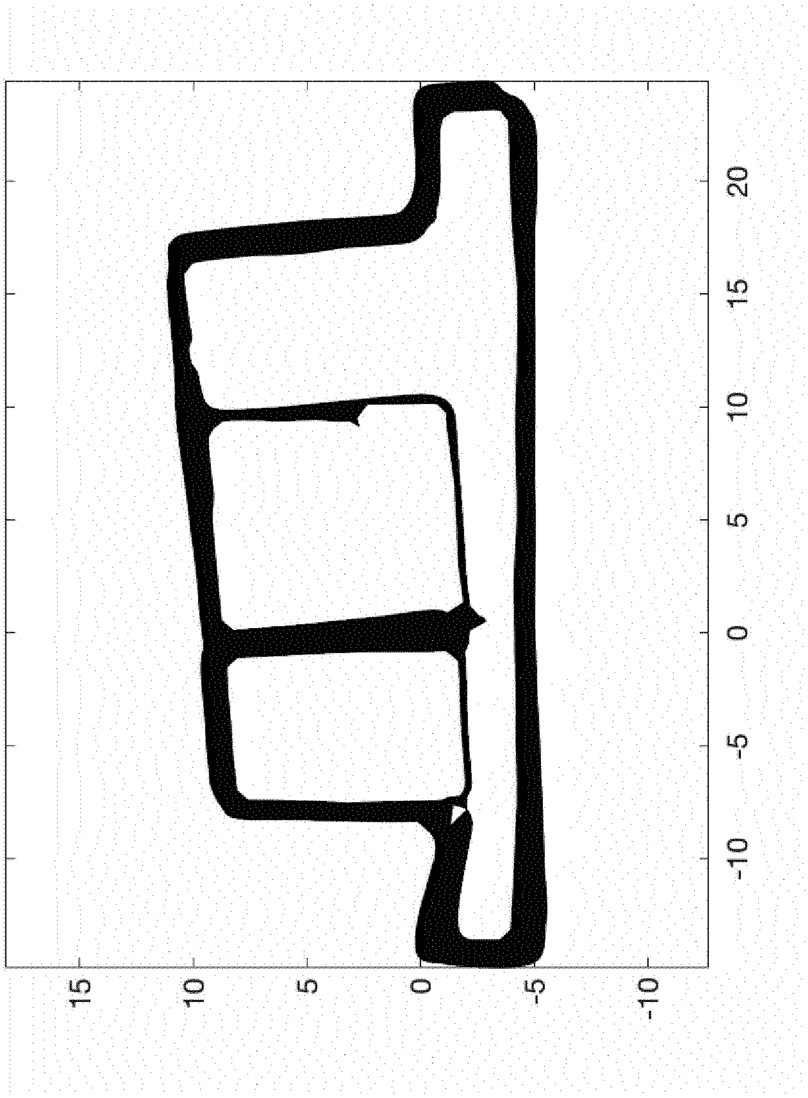


FIG. 71A

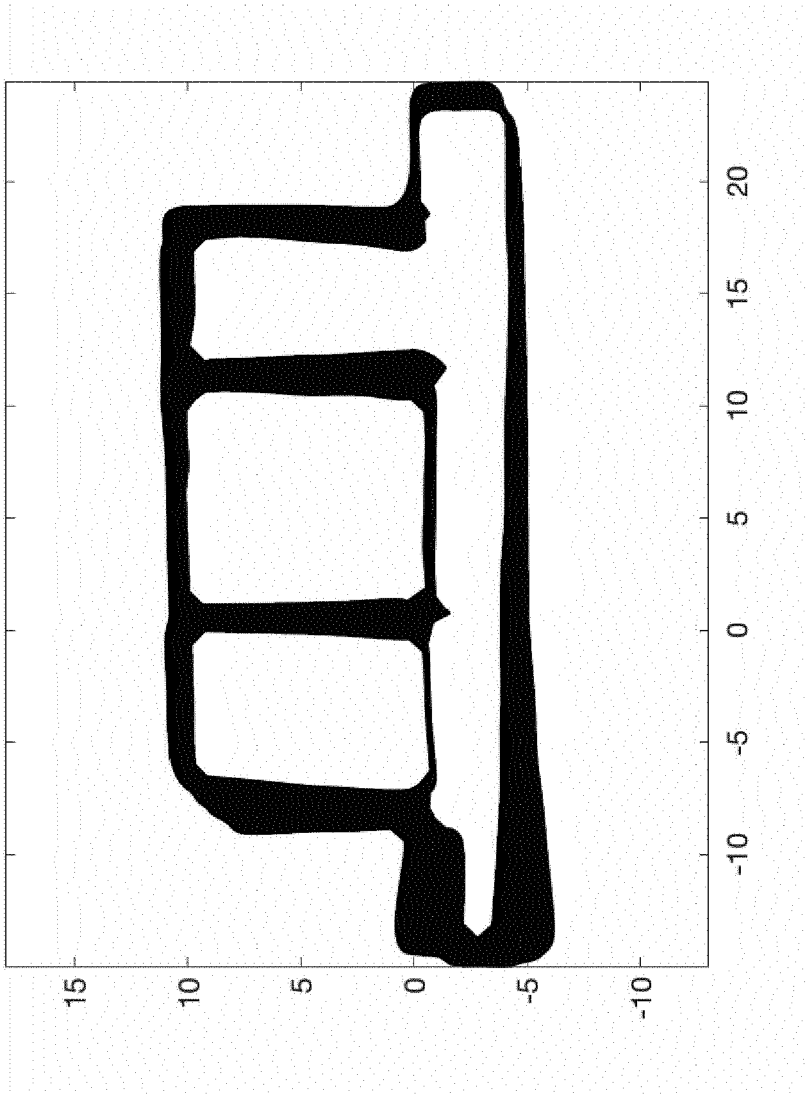


FIG. 71B

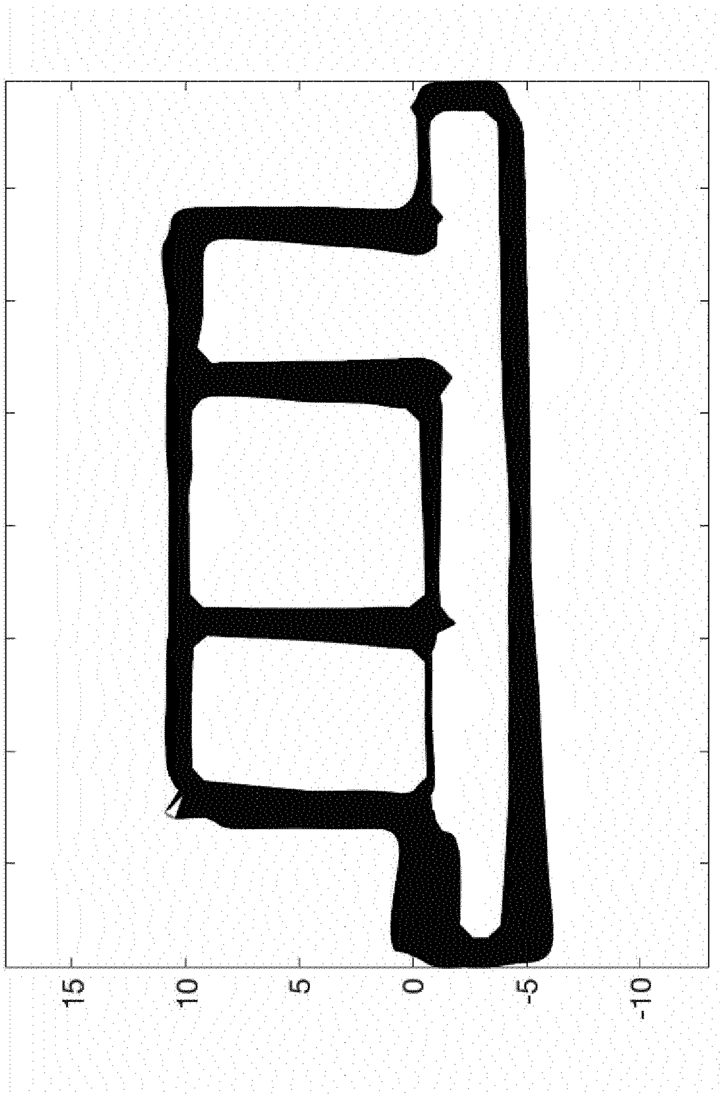


FIG. 71C

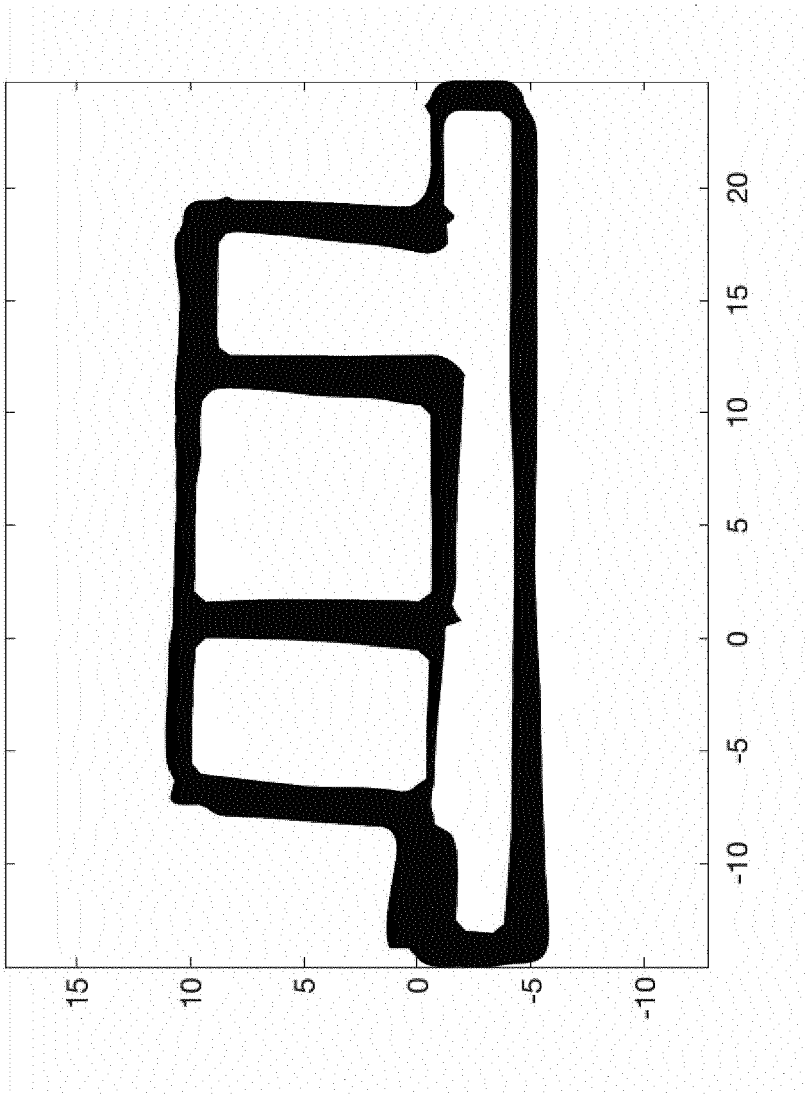


FIG. 71D

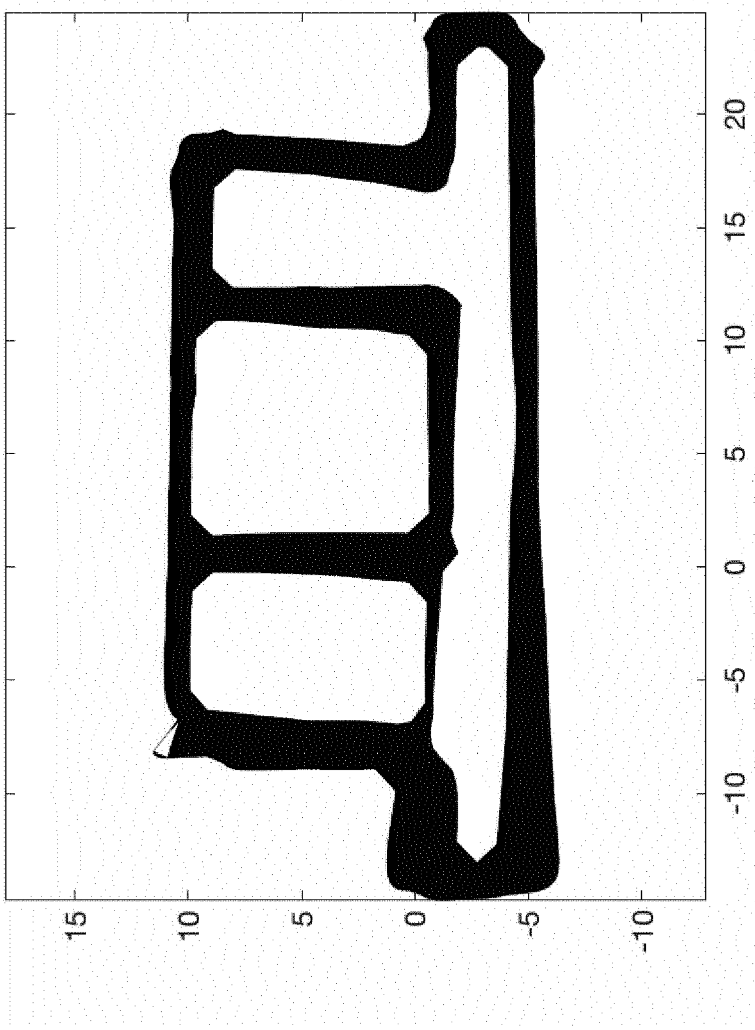


FIG. 71E

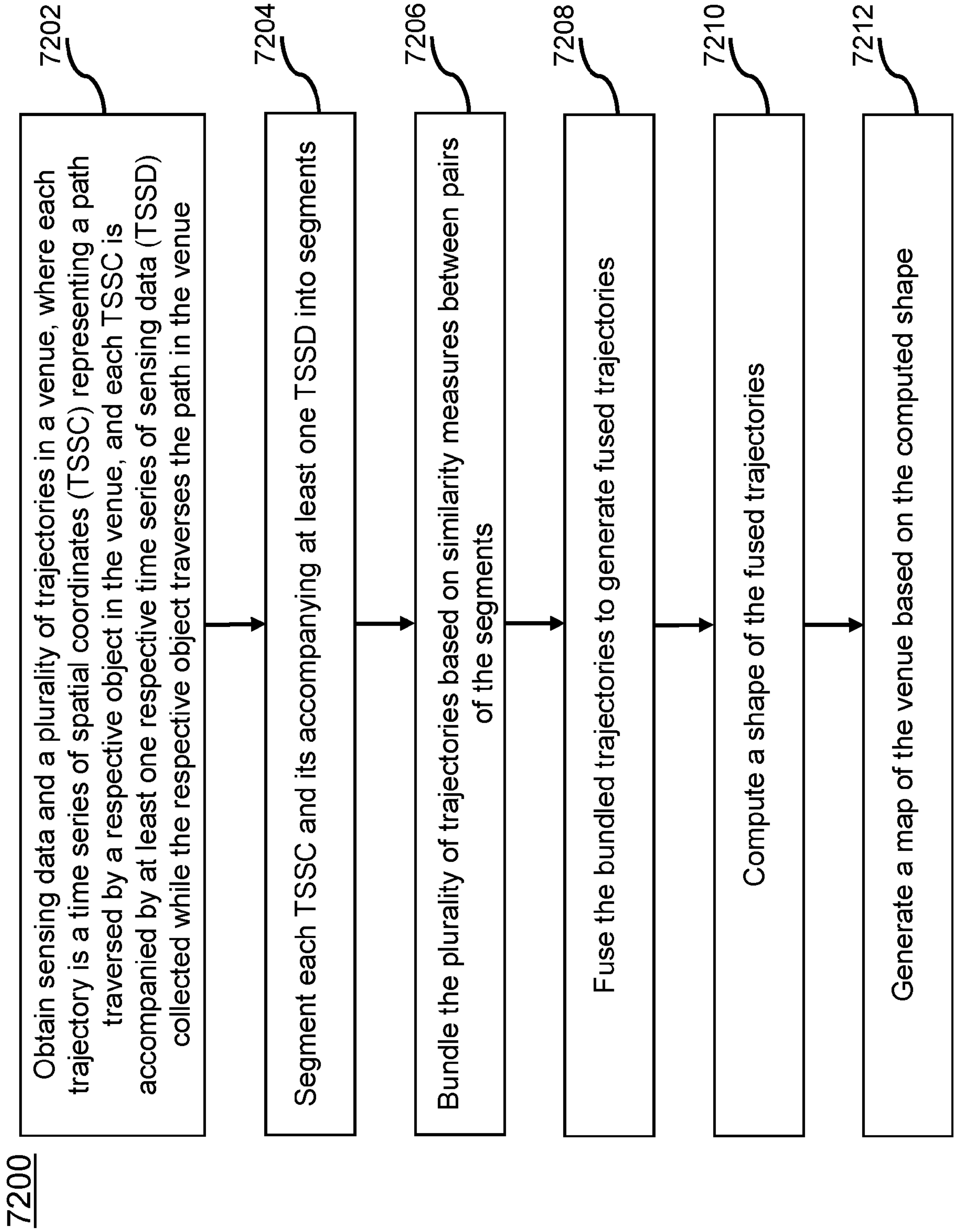
7200
Obtain sensing data and a plurality of trajectories in a venue, where each trajectory is a time series of spatial coordinates (TSSC) representing a path traversed by a respective object in the venue, and each TSSC is accompanied by at least one respective time series of sensing data (TSSD) collected while the respective object traverses the path in the venue
7202
Segment each TSSC and its accompanying at least one TSSD into segments
7204
Bundle the plurality of trajectories based on similarity measures between pairs of the segments
7206
Fuse the bundled trajectories to generate fused trajectories
7208
Compute a shape of the fused trajectories
7210
Generate a map of the venue based on the computed shape
7212

FIG. 72

7300

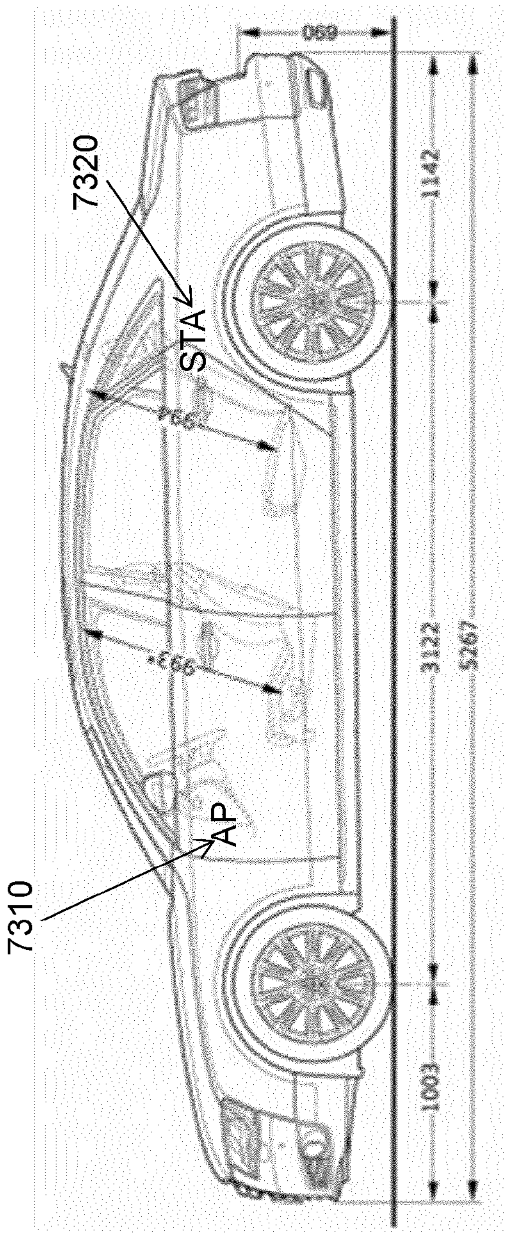


FIG. 73A

7300

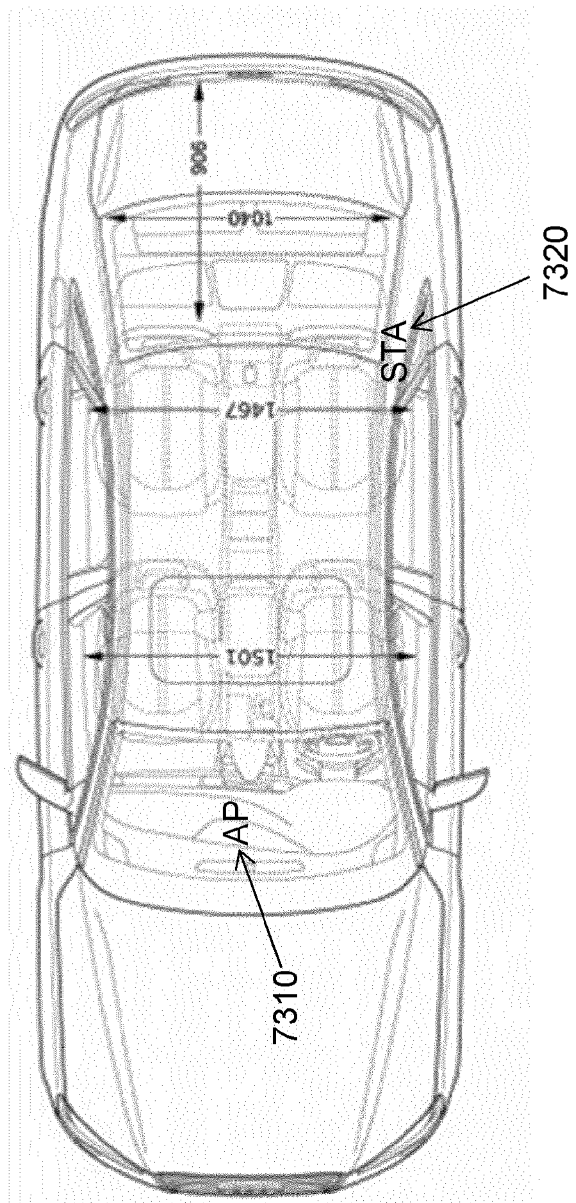


FIG. 73B

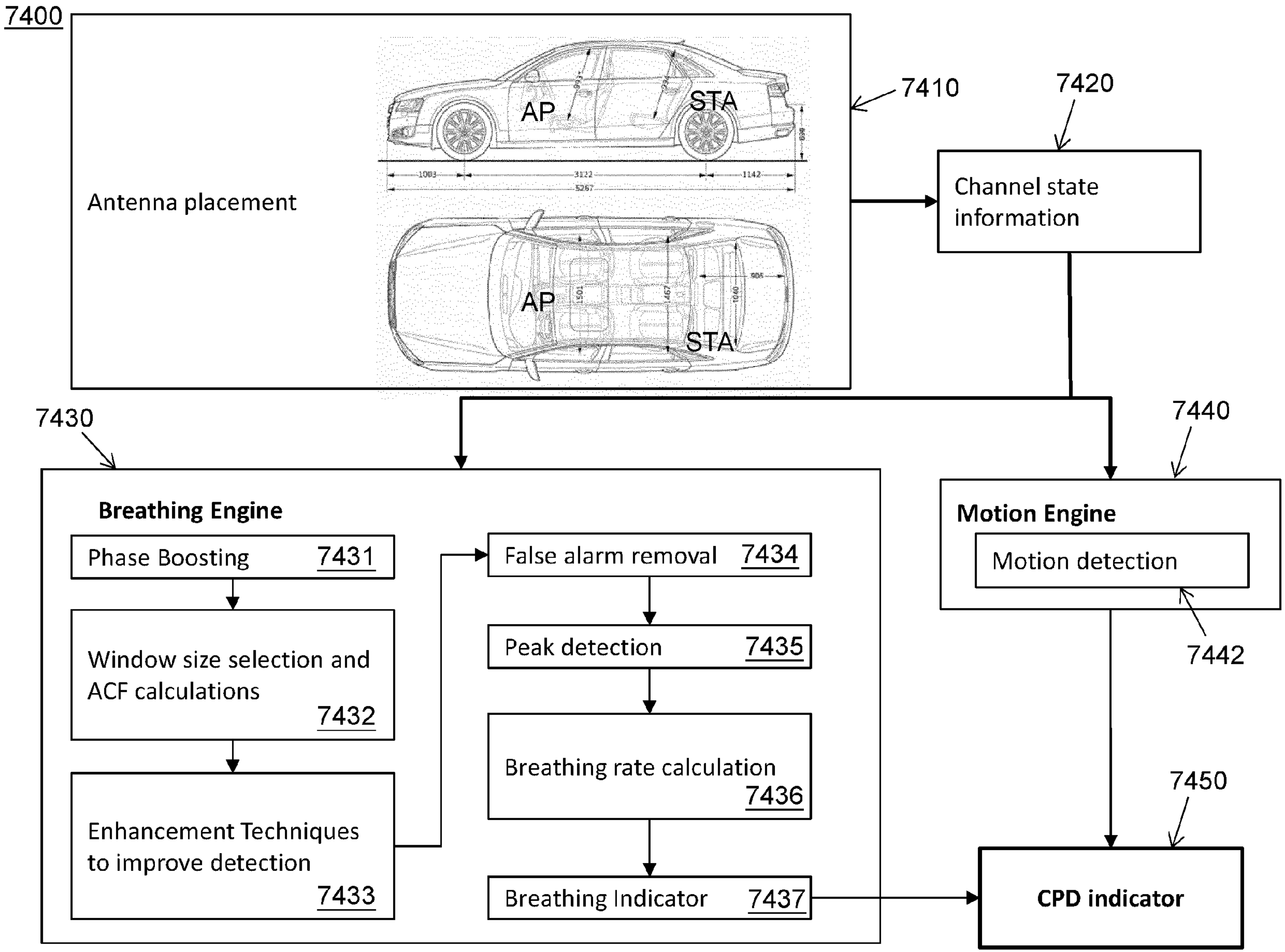
7400
Antenna placement
AP
STA
AP
STA
7410
7420
Channel state information
7430
Breathing Engine
Phase Boosting 7431
Window size selection and ACF calculations 7432
Enhancement Techniques to improve detection 7433
False alarm removal 7434
Peak detection 7435
Breathing rate calculation 7436
Breathing Indicator 7437
7440
Motion Engine
Motion detection
7442
7450
CPD indicator

FIG. 74

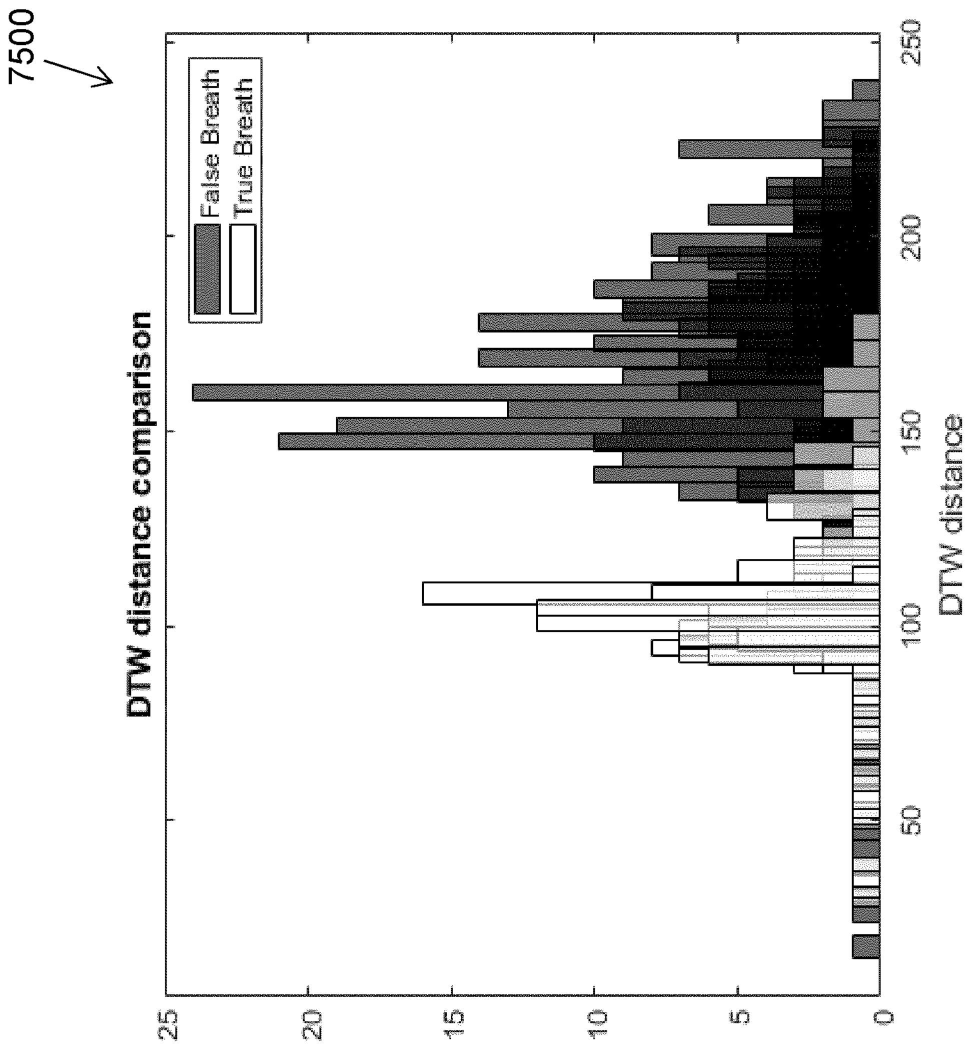
7500
DTW distance comparison
False Breath
True Breath
25
20
15
10
5
0
50
100
150
200
250
DTW distance

FIG. 75

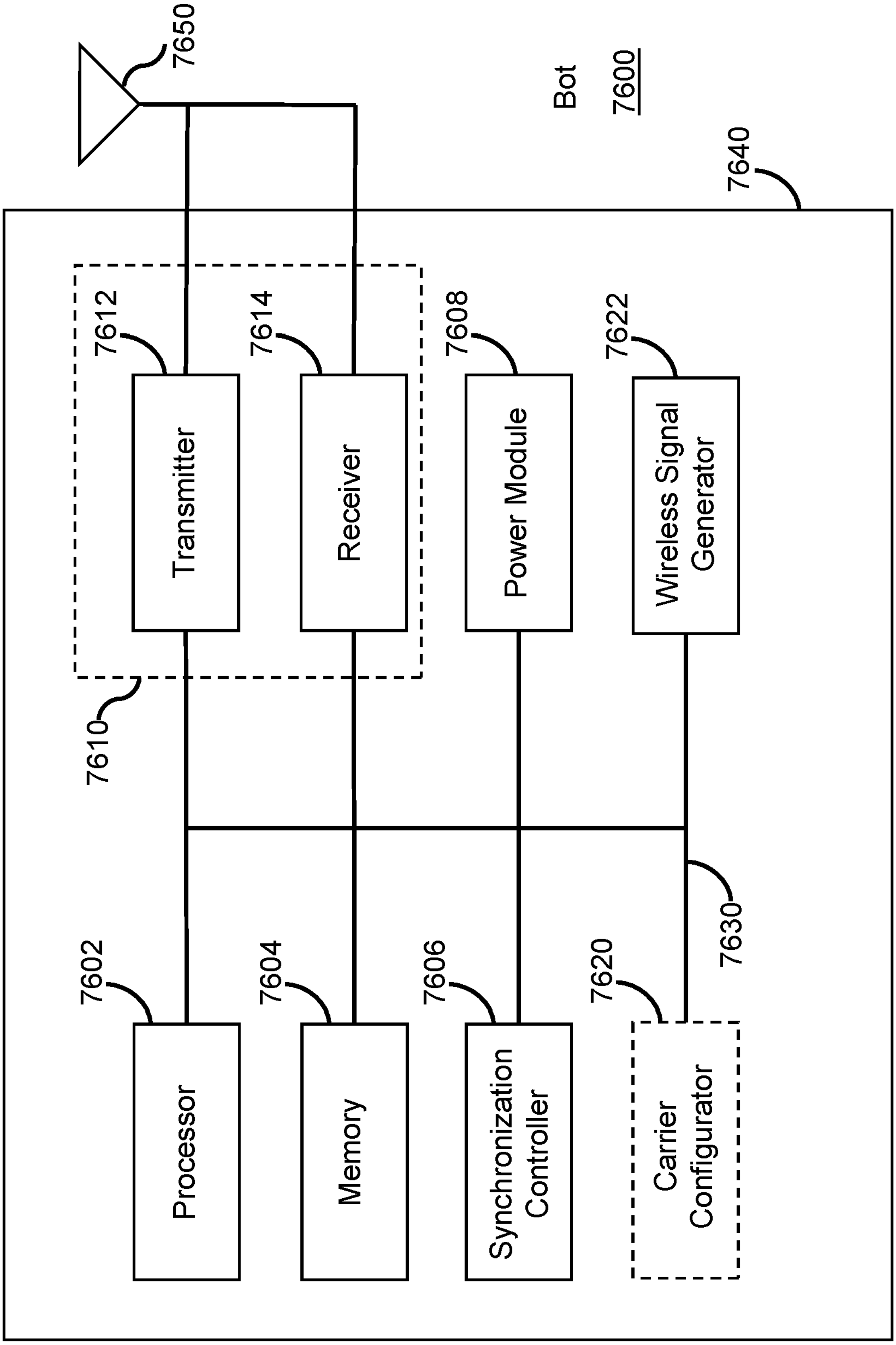
7650
Bot
7600
7640
7612
Transmitter
7614
Receiver
7608
Power Module
7622
Wireless Signal
Generator
7610
7602
Processor
7604
Memory
7606
Synchronization
Controller
7620
Carrier
Configurator
7630

FIG. 76

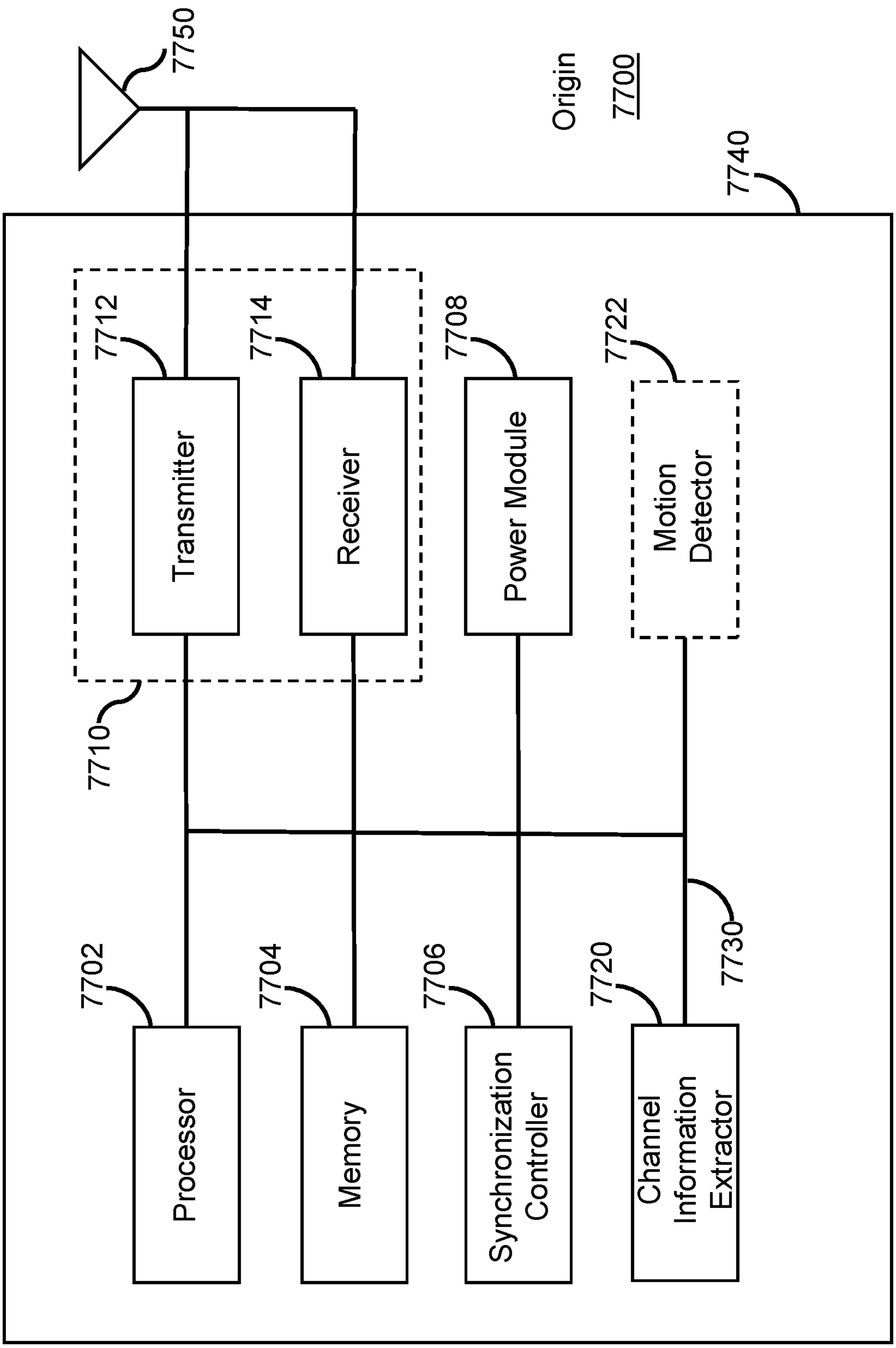
7750
Origin
7700
7740
7712
Transmitter
7714
Receiver
7708
Power Module
7722
Motion Detector
7710
7702
Processor
7704
Memory
7706
Synchronization Controller
7720
Channel Information Extractor
7730

FIG. 77

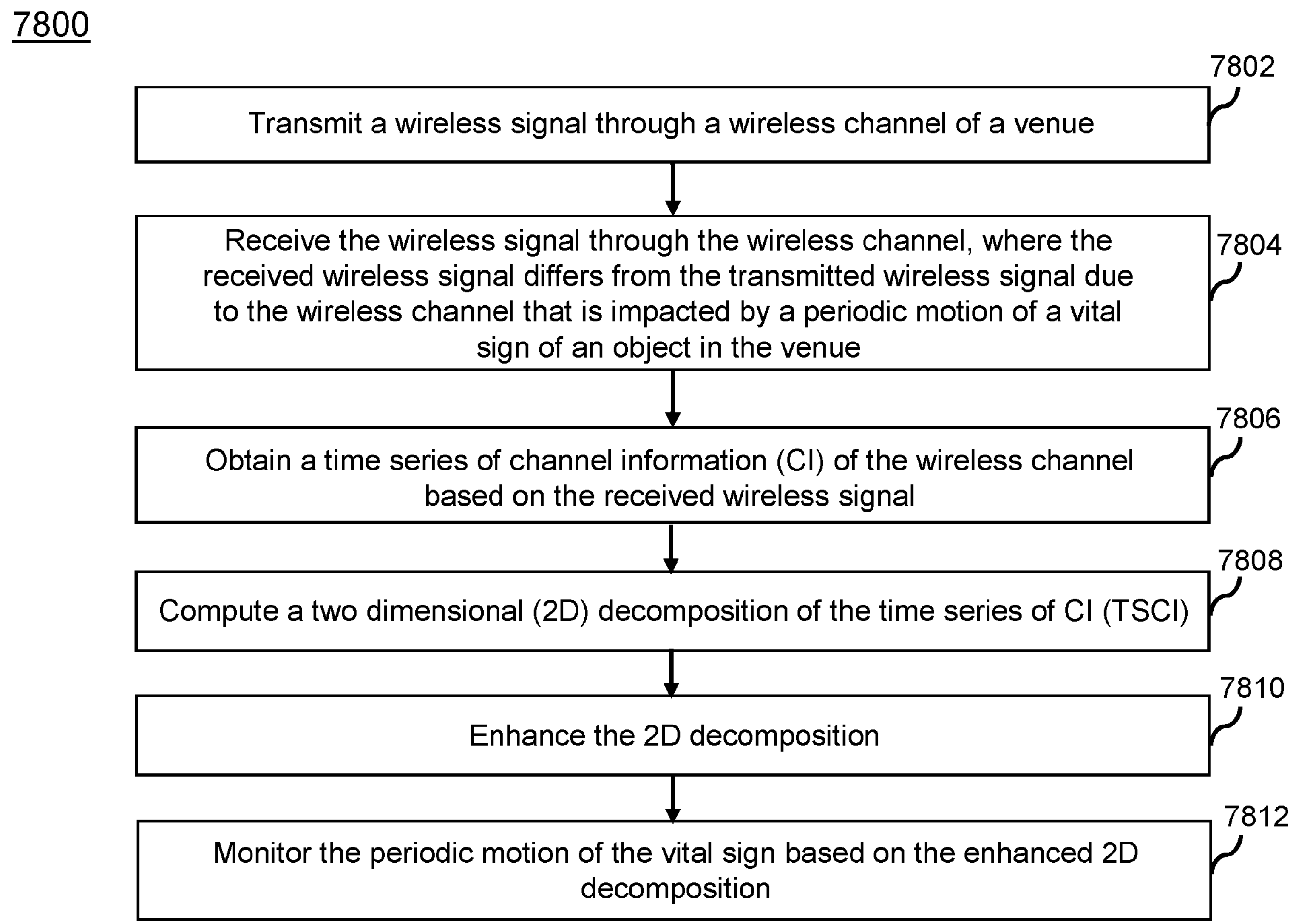
7800
7802
Transmit a wireless signal through a wireless channel of a venue
7804
Receive the wireless signal through the wireless channel, where the received wireless signal differs from the transmitted wireless signal due to the wireless channel that is impacted by a periodic motion of a vital sign of an object in the venue
7806
Obtain a time series of channel information (CI) of the wireless channel based on the received wireless signal
7808
Compute a two dimensional (2D) decomposition of the time series of CI (TSCI)
7810
Enhance the 2D decomposition
7812
Monitor the periodic motion of the vital sign based on the enhanced 2D decomposition

FIG. 78

Europäisches Patentamt
European Patent Office
Office européen des brevets

# EUROPEAN SEARCH REPORT

Application Number
EP 22 20 0097

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/166030 A1 (CHEN CHEN [US] ET AL) 30 May 2019 (2019-05-30) * paragraphs [0429] - [0456], [0468] - [0504]; figures 14-17,20,23A-26 * ----- | 1-15 | INV. A61B5/00 G01S7/41 G01S13/04 G01S13/50 G01S13/66 G01S13/88 ADD. G01S13/46 |
| X | LIU JIAN JLIU28@STEVENS EDU ET AL: "Tracking Vital Signs During Sleep Leveraging Off-the-shelf WiFi", PROCEEDINGS OF THE 34TH ACM SIGMOD-SIGACT-SIGAI SYMPOSIUM ON PRINCIPLES OF DATABASE SYSTEMS, ACMPUB27, NEW YORK, NY, USA, 22 June 2015 (2015-06-22), pages 267-276, XP058511255, DOI: 10.1145/2746285.2746303 ISBN: 978-1-4503-3550-8 * the whole document * ----- | 1-8,11, 15 | |

TECHNICAL FIELDS SEARCHED (IPC)

A61B
G01S

The present search report has been drawn up for all claims

1

| Place of search | Date of completion of the search | Examiner |
|---|---|---|
| Munich | 15 February 2023 | Reeck, Guido |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 20 0097

This annex lists the patent family members relating to the patent documents cited in the above-mentioned European search report. The members are as contained in the European Patent Office EDP file on
The European Patent Office is in no way liable for these particulars which are merely given for the purpose of information.

15-02-2023

| Patent document cited in search report | Publication date | Patent family member(s) | Publication date |
|---|---|---|---|
| US 2019166030 A1 | 30-05-2019 | NONE | |

EPO FORM P0459

For more details about this annex : see Official Journal of the European Patent Office, No. 12/82